# EUROPEAN PATENT SPECIFICATION

(11) **EP 3 118 221 B1**
(45) Date of publication and mention of the grant of the patent: **21.08.2019**
(21) Application number: 16184605.0
(22) Date of filing: 26.02.2008
(51) Int. Cl.: C07K 14/705, G01N 33/574, G01N 33/68, C07K 16/30

(54) **PROTEINS**
PROTEINE
PROTEINES

(30) Priority: 26.02.2007 US 903510 P; 26.02.2007 US 903509 P; 05.06.2007 WO PCT/EP2007/055537; 25.02.2008 WO PCT/GB2008/050124
(43) Date of publication of application: 18.01.2017
(62) Divisional of application: 12152158.7
(73) Proprietor: Oxford BioTherapeutics Ltd, Milton Park Abingdon Oxfordshire OX14 4RZ (GB)
(72) Inventor: Rohlff, Christian, Abingdon, Oxfordshire OX14 4RZ (GB); Stamps, Alasdair, Abingdon, Oxfordshire OX14 4RZ (GB)

(56) References cited:
- WO-A1-2004/074481
- WO-A2-2007/005502
- BÜHRING HANS-JÖRG ET AL: "CDCP1 identifies a broad spectrum of normal and malignant stem/progenitor cell subsets of hematopoietic and nonhematopoietic origin", STEM CELLS, ALPHAMED PRESS, DAYTON, OH, US, vol. 22, no. 3, 1 January 2004 (2004-01-01), pages 334-343, XP002495598, ISSN: 1066-5099, DOI: 10.1634/STEMCELLS.22-3-334
- PERRY ET AL: "Expression of the CUB domain containing protein 1 (CDCP1) gene in colorectal tumour cells", FEBS LETTERS, ELSEVIER, AMSTERDAM, NL, vol. 581, no. 6, 20 February 2007 (2007-02-20), pages 1137-1142, XP005932065, ISSN: 0014-5793, DOI: 10.1016/J.FEBSLET.2007.02.025

### SEQUENCE LISTING

<110> Oxford Genome Sciences (UK) Ltd Rohlff, Christian Stamps, Alasdair
<120> PROTEINS
<130> OGL-P148PC
<140> PCT/GB 08/050125
   <141> 2008-02-25
<150> US 60/903,510
   <151> 2007-02-26
<150> US 60/903,509
   <151> 2007-02-26
<150> PCT/EP07/055537
   <151> 2007-06-05
<160> 1008
<170> PatentIn version 3.3
<210> 1
   <211> 987
   <212> PRT
   <213> Homo Sapiens
<220>
   <221> MISC_FEATURE
   <222> (1)..(987)
   <223> Designated OGTA002 = Swissprot Accession No. P54760, Ephrin type-B receptor 4
<400> 1
<210> 2
   <211> 220
   <212> PRT
   <213> Homo Sapiens
<220>
   <221> MISC_FEATURE
   <222> (1)..(220)
   <223> OGTA009 = Swiss Prot Accession No. 015551, Claudin-3
<400> 2
   Met Ser Met Gly Leu Glu Ile Thr Gly Thr Ala Leu Ala Val Leu Gly
   1               5                   10                  15
   Trp Leu Gly Thr Ile Val Cys Cys Ala Leu Pro Met Trp Arg Val Ser
               20                  25                  30
   Ala Phe Ile Gly Ser Asn Ile Ile Thr Ser Gln Asn Ile Trp Glu Gly
           35                  40                  45
   Leu Trp Met Asn Cys Val Val Gln Ser Thr Gly Gln Met Gln Cys Lys
       50                  55                  60
   Val Tyr Asp Ser Leu Leu Ala Leu Pro Gln Asp Leu Gln Ala Ala Arg
   65                  70                  75                  80
   Ala Leu Ile Val Val Ala Ile Leu Leu Ala Ala Phe Gly Leu Leu Val
                   85                  90                  95
   Ala Leu Val Gly Ala Gln Cys Thr Asn Cys Val Gln Asp Asp Thr Ala
               100                 105                 110
   Lys Ala Lys Ile Thr Ile Val Ala Gly Val Leu Phe Leu Leu Ala Ala
           115                 120                 125
   Leu Leu Thr Leu Val Pro Val Ser Trp Ser Ala Asn Thr Ile Ile Arg
       130                 135                 140
   Asp Phe Tyr Asn Pro Val Val Pro Glu Ala Gln Lys Arg Glu Met Gly
   145                 150                 155                 160
   Ala Gly Leu Tyr Val Gly Trp Ala Ala Ala Ala Leu Gln Leu Leu Gly
                   165                 170                 175
   Gly Ala Leu Leu Cys Cys Ser Cys Pro Pro Arg Glu Lys Lys Tyr Thr
               180                 185                 190
   Ala Thr Lys Val Val Tyr Ser Ala Pro Arg Ser Thr Gly Pro Gly Ala
           195                 200                 205
   Ser Leu Gly Thr Gly Tyr Asp Arg Lys Asp Tyr Val
       210                 215                 220
<210> 3
   <211> 764
   <212> PRT
   <213> Homo Sapiens
<220>
   <221> MISC_FEATURE
   <222> (1)..(764)
   <223> OGTA016 = Swiss Prot Accession No. P01833, Polymeric-immunoglobulin receptor
<400> 3
   Met Leu Leu Phe Val Leu Thr Cys Leu Leu Ala Val Phe Pro Ala Ile
   1               5                   10                  15
   Ser Thr Lys Ser Pro Ile Phe Gly Pro Glu Glu Val Asn Ser Val Glu
               20                  25                  30
   Gly Asn Ser Val Ser Ile Thr Cys Tyr Tyr Pro Pro Thr Ser Val Asn
           35                  40                  45
   Arg His Thr Arg Lys Tyr Trp Cys Arg Gln Gly Ala Arg Gly Gly Cys
       50                  55                  60
   Ile Thr Leu Ile Ser Ser Glu Gly Tyr Val Ser Ser Lys Tyr Ala Gly
   65                  70                  75                  80
   Arg Ala Asn Leu Thr Asn Phe Pro Glu Asn Gly Thr Phe Val Val Asn
                   85                  90                  95
   Ile Ala Gln Leu Ser Gln Asp Asp Ser Gly Arg Tyr Lys Cys Gly Leu
               100                 105                 110
   Gly Ile Asn Ser Arg Gly Leu Ser Phe Asp Val Ser Leu Glu Val Ser
           115                 120                 125
   Gln Gly Pro Gly Leu Leu Asn Asp Thr Lys Val Tyr Thr Val Asp Leu
       130                 135                 140
   Gly Arg Thr Val Thr Ile Asn Cys Pro Phe Lys Thr Glu Asn Ala Gln
   145                 150                 155                 160
   Lys Arg Lys Ser Leu Tyr Lys Gln Ile Gly Leu Tyr Pro Val Leu Val
                   165                 170                 175
   Ile Asp Ser Ser Gly Tyr Val Asn Pro Asn Tyr Thr Gly Arg Ile Arg
               180                 185                 190
   Leu Asp Ile Gln Gly Thr Gly Gln Leu Leu Phe Ser Val Val Ile Asn
           195                 200                 205
   Gln Leu Arg Leu Ser Asp Ala Gly Gln Tyr Leu Cys Gln Ala Gly Asp
       210                 215                 220
   Asp Ser Asn Ser Asn Lys Lys Asn Ala Asp Leu Gln Val Leu Lys Pro
   225                 230                 235                 240
   Glu Pro Glu Leu Val Tyr Glu Asp Leu Arg Gly Ser Val Thr Phe His
                   245                 250                 255
   Cys Ala Leu Gly Pro Glu Val Ala Asn Val Ala Lys Phe Leu Cys Arg
               260                 265                 270
   Gln Ser Ser Gly Glu Asn Cys Asp Val Val Val Asn Thr Leu Gly Lys
           275                 280                 285
   Arg Ala Pro Ala Phe Glu Gly Arg Ile Leu Leu Asn Pro Gln Asp Lys
   290 295 300
   Asp Gly Ser Phe Ser Val Val Ile Thr Gly Leu Arg Lys Glu Asp Ala
   305 310 315 320
   Gly Arg Tyr Leu Cys Gly Ala His Ser Asp Gly Gln Leu Gln Glu Gly
   325 330 335
   Ser Pro Ile Gln Ala Trp Gln Leu Phe Val Asn Glu Glu Ser Thr Ile
   340 345 350
   Pro Arg Ser Pro Thr Val Val Lys Gly Val Ala Gly Ser Ser Val Ala
   355 360 365
   Val Leu Cys Pro Tyr Asn Arg Lys Glu Ser Lys Ser Ile Lys Tyr Trp
   370 375 380
   Cys Leu Trp Glu Gly Ala Gln Asn Gly Arg Cys Pro Leu Leu Val Asp
   385 390 395 400
   Ser Glu Gly Trp Val Lys Ala Gln Tyr Glu Gly Arg Leu Ser Leu Leu
   405 410 415
   Glu Glu Pro Gly Asn Gly Thr Phe Thr Val Ile Leu Asn Gln Leu Thr
   420 425 430
   Ser Arg Asp Ala Gly Phe Tyr Trp Cys Leu Thr Asn Gly Asp Thr Leu
   435 440 445
   Trp Arg Thr Thr Val Glu Ile Lys Ile Ile Glu Gly Glu Pro Asn Leu
   450 455 460
   Lys Val Pro Gly Asn Val Thr Ala Val Leu Gly Glu Thr Leu Lys Val
   465 470 475 480
   Pro Cys His Phe Pro Cys Lys Phe Ser Ser Tyr Glu Lys Tyr Trp Cys
   485 490 495
   Lys Trp Asn Asn Thr Gly Cys Gln Ala Leu Pro Ser Gln Asp Glu Gly
   500 505 510
   Pro Ser Lys Ala Phe Val Asn Cys Asp Glu Asn Ser Arg Leu Val Ser
   515 520 525
   Leu Thr Leu Asn Leu Val Thr Arg Ala Asp Glu Gly Trp Tyr Trp Cys
   530 535 540
   Gly Val Lys Gln Gly His Phe Tyr Gly Glu Thr Ala Ala Val Tyr Val
   545 550 555 560
   Ala Val Glu Glu Arg Lys Ala Ala Gly Ser Arg Asp Val Ser Leu Ala
   565 570 575
   Lys Ala Asp Ala Ala Pro Asp Glu Lys Val Leu Asp Ser Gly Phe Arg
   580 585 590
   Glu Ile Glu Asn Lys Ala Ile Gln Asp Pro Arg Leu Phe Ala Glu Glu
   595 600 605
   Lys Ala Val Ala Asp Thr Arg Asp Gln Ala Asp Gly Ser Arg Ala Ser
   610 615 620
   Val Asp Ser Gly Ser Ser Glu Glu Gln Gly Gly Ser Ser Arg Ala Leu
   625 630 635 640
   Val Ser Thr Leu Val Pro Leu Gly Leu Val Leu Ala Val Gly Ala Val
   645 650 655
   Ala Val Gly Val Ala Arg Ala Arg His Arg Lys Asn Val Asp Arg Val
   660 665 670
   Ser Ile Arg Ser Tyr Arg Thr Asp Ile Ser Met Ser Asp Phe Glu Asn
   675 680 685
   Ser Arg Glu Phe Gly Ala Asn Asp Asn Met Gly Ala Ser Ser Ile Thr
   690 695 700
   Gln Glu Thr Ser Leu Gly Gly Lys Glu Glu Phe Val Ala Thr Thr Glu
   705 710 715 720
   Ser Thr Thr Glu Thr Lys Glu Pro Lys Lys Ala Lys Arg Ser Ser Lys
   725 730 735
   Glu Glu Ala Glu Met Ala Tyr Lys Asp Phe Leu Leu Gln Ser Ser Thr
   740 745 750
   Val Ala Ala Glu Ala Gln Asp Gly Pro Gln Glu Ala
   755 760
<210> 4
   <211> 865
   <212> PRT
   <213> Homo Sapiens
<220>
   <221> MISC_FEATURE
   <222> (1)..(865)
   <223> OGTA028 = Swiss Prot Accession No. Q5T7N2, FLJ10884
<400> 4
   Met Ser Asp Val Ser Thr Ser Val Gln Ser Lys Phe Ala Arg Leu Ala
   1               5                   10                  15
   Lys Lys Lys Glu Asn Ile Thr Tyr Met Lys Arg Glu Gln Leu Thr Glu
               20                  25                  30
   Thr Asp Lys Asp Ile Ala Pro Val Leu Asp Leu Lys Cys Lys Asp Val
           35                  40                  45
   Ser Ala Ile Met Asn Lys Phe Lys Val Leu Met Glu Ile Gln Asp Leu
       50                  55                  60
   Met Phe Glu Glu Met Arg Glu Thr Leu Lys Asn Asp Leu Lys Ala Val
   65                  70                  75                      80
   Leu Gly Gly Lys Ala Thr Ile Pro Glu Val Lys Asn Ser Glu Asn Ser
                   85                  90                  95
   Ser Ser Arg Thr Glu Phe Gln Gln Ile Ile Asn Leu Ala Leu Gln Lys
               100                 105                 110
   Thr Gly Met Val Gly Lys Ile Glu Gly Glu Asn Ser Lys Ile Gly Asp
           115                 120                 125
   Asp Asn Glu Asn Leu Thr Phe Lys Leu Glu Val Asn Glu Leu Ser Gly
       130                 135                 140
   Lys Leu Asp Asn Thr Asn Glu Tyr Asn Ser Asn Asp Gly Lys Lys Leu
   145                 150                 155                     160
   Pro Gln Gly Glu Ser Arg Ser Tyr Glu Val Met Gly Ser Met Glu Glu
                   165                 170                 175
   Thr Leu Cys Asn Ile Asp Asp Arg Asp Gly Asn Arg Asn Val His Leu
               180                 185                 190
   Glu Phe Thr Glu Arg Glu Ser Arg Lys Asp Gly Glu Asp Glu Phe Val
           195                 200                 205
   Lys Glu Met Arg Glu Glu Arg Lys Phe Gln Lys Leu Lys Asn Lys Glu
       210                 215                 220
   Glu Val Leu Lys Ala Ser Arg Glu Glu Lys Val Leu Met Asp Glu Gly
   225 230 235 240
   Ala Val Leu Thr Leu Val Ala Asp Leu Ser Ser Ala Thr Leu Asp Ile
   245 250 255
   Ser Lys Gln Trp Ser Asn Val Phe Asn Ile Leu Arg Glu Asn Asp Phe
   260 265 270
   Glu Pro Lys Phe Leu Cys Glu Val Lys Leu Ala Phe Lys Cys Asp Gly
   275 280 285
   Glu Ile Lys Thr Phe Ser Asp Leu Gln Ser Leu Arg Lys Phe Ala Ser
   290 295 300
   Gln Lys Ser Ser Val Lys Glu Leu Leu Lys Asp Val Leu Pro Gln Lys
   305 310 315 320
   Glu Glu Ile Asn Gln Gly Gly Arg Lys Tyr Gly Ile Gln Glu Lys Arg
   325 330 335
   Asp Lys Thr Leu Ile Asp Ser Lys His Arg Ala Gly Glu Ile Thr Ser
   340 345 350
   Asp Gly Leu Ser Phe Leu Phe Leu Lys Glu Val Lys Val Ala Lys Pro
   355 360 365
   Glu Glu Met Lys Asn Leu Glu Thr Gln Glu Glu Glu Phe Ser Glu Leu
   370 375 380
   Glu Glu Leu Asp Glu Glu Ala Ser Gly Met Glu Asp Asp Glu Asp Thr
   385 390 395 400
   Ser Gly Leu Glu Glu Glu Glu Glu Glu Pro Ser Gly Leu Glu Glu Glu
   405 410 415
   Glu Glu Glu Glu Ala Ser Gly Leu Glu Glu Asp Glu Ala Ser Gly Leu
   420 425 430
   Glu Glu Glu Glu Glu Gln Thr Ser Glu Gln Asp Ser Thr Phe Gln Gly
   435 440 445
   His Thr Leu Val Asp Ala Lys His Glu Val Glu Ile Thr Ser Asp Gly
   450 455 460
   Met Glu Thr Thr Phe Ile Asp Ser Val Glu Asp Ser Glu Ser Glu Glu
   465                 470                 475                 480
   Glu Glu Glu Gly Lys Ser Ser Glu Thr Gly Lys Val Lys Thr Thr Ser
                   485                 490                 495
   Leu Thr Glu Lys Lys Ala Ser Arg Arg Gln Lys Glu Ile Pro Phe Ser
               500                 505                 510
   Tyr Leu Val Gly Asp Ser Gly Lys Lys Lys Leu Val Lys His Gln Val
           515                 520                 525
   Val His Lys Thr Gln Glu Glu Glu Glu Thr Ala Val Pro Thr Ser Gln
       530                 535                 540
   Gly Thr Gly Thr Pro Cys Leu Thr Leu Cys Leu Ala Ser Pro Ser Lys
   545                 550                 555                 560
   Ser Leu Glu Met Ser His Asp Glu His Lys Lys His Ser His Thr Asn
                   565                 570                 575
   Leu Ser Ile Ser Thr Gly Val Thr Lys Leu Lys Lys Thr Glu Glu Lys
               580                 585                 590
   Lys His Arg Thr Leu His Thr Glu Glu Leu Thr Ser Lys Glu Ala Asp
           595                 600                 605
   Leu Thr Glu Glu Thr Glu Glu Asn Leu Arg Ser Ser Val Ile Asn Ser
       610                 615                 620
   Ile Arg Glu Ile Lys Glu Glu Ile Gly Asn Leu Lys Ser Ser His Ser
   625                 630                 635                 640
   Gly Val Leu Glu Ile Glu Asn Ser Val Asp Asp Leu Ser Ser Arg Met
                   645                 650                 655
   Asp Ile Leu Glu Glu Arg Ile Asp Ser Leu Glu Asp Gln Ile Glu Glu
               660                 665                 670
   Phe Ser Lys Asp Thr Met Gln Met Thr Lys Gln Ile Ile Ser Lys Glu
           675                 680                 685
   Arg Gln Arg Asp Ile Glu Glu Arg Ser Arg Ser Cys Asn Ile Arg Leu
       690                 695                 700
   Ile Gly Ile Pro Glu Lys Glu Ser Tyr Glu Asn Arg Ala Glu Asp Ile
   705                 710                 715                 720
   Ile Lys Glu Ile Ile Asp Glu Asn Phe Ala Glu Leu Lys Lys Gly Ser
                   725                 730                 735
   Ser Leu Glu Ile Val Ser Ala Cys Arg Val Pro Ser Lys Ile Asp Glu
               740                 745                 750
   Lys Arg Leu Thr Pro Arg His Ile Leu Val Lys Phe Trp Asn Ser Ser
           755                 760                 765
   Asp Lys Glu Lys Ile Ile Arg Ala Ser Arg Glu Arg Arg Glu Ile Thr
       770                 775                 780
   Tyr Gln Gly Thr Arg Ile Arg Leu Thr Ala Asp Leu Ser Leu Asp Thr
   785                 790                 795                 800
   Leu Asp Ala Arg Ser Lys Trp Ser Asn Val Phe Lys Val Leu Leu Glu
                   805                 810                 815
   Lys Gly Phe Asn Pro Arg Ile Leu Tyr Pro Ala Lys Met Ala Phe Asp
               820                 825                 830
   Phe Arg Gly Lys Thr Lys Val Phe Leu Ser Ile Glu Glu Phe Arg Asp
           835                 840                 845
   Tyr Val Leu His Met Pro Thr Leu Arg Glu Leu Leu Gly Asn Asn Ile
       850                 855                 860
   Pro
   865
<210> 5
   <211> 323
   <212> PRT
   <213> Homo Sapiens
<220>
   <221> MISC_FEATURE
   <222> (1)..(323)
   <223> OGTA037 = Swiss Prot Accession No. Q9HA72, Protein FAM26B
<400> 5
   Met Ala Ala Leu Ile Ala Glu Asn Phe Arg Phe Leu Ser Leu Phe Phe
   1               5                   10                  15
   Lys Ser Lys Asp Val Met Ile Phe Asn Gly Leu Val Ala Leu Gly Thr
               20                  25                  30
   Val Gly Ser Gln Glu Leu Phe Ser Val Val Ala Phe His Cys Pro Cys
           35                  40                  45
   Ser Pro Ala Arg Asn Tyr Leu Tyr Gly Leu Ala Ala Ile Gly Val Pro
       50                  55                  60
   Ala Leu Val Leu Phe Ile Ile Gly Ile Ile Leu Asn Asn His Thr Trp
   65                  70                  75                  80
   Asn Leu Val Ala Glu Cys Gln His Arg Arg Thr Lys Asn Cys Ser Ala
                   85                  90                  95
   Ala Pro Thr Phe Leu Leu Leu Ser Ser Ile Leu Gly Arg Ala Ala Val
               100                 105                 110
   Ala Pro Val Thr Trp Ser Val Ile Ser Leu Leu Arg Gly Glu Ala Tyr
           115                 120                 125
   Val Cys Ala Leu Ser Glu Phe Val Asp Pro Ser Ser Leu Thr Ala Arg
       130                 135                 140
   Glu Glu His Phe Pro Ser Ala His Ala Thr Glu Ile Leu Ala Arg Phe
   145                 150                 155                 160
   Pro Cys Lys Glu Asn Pro Asp Asn Leu Ser Asp Phe Arg Glu Glu Val
                   165                 170                 175
   Ser Arg Arg Leu Arg Tyr Glu Ser Gln Leu Phe Gly Trp Leu Leu Ile
               180                 185                 190
   Gly Val Val Ala Ile Leu Val Phe Leu Thr Lys Cys Leu Lys His Tyr
           195                 200                 205
   Cys Ser Pro Leu Ser Tyr Arg Gln Glu Ala Tyr Trp Ala Gln Tyr Arg
       210                 215                 220
   Ala Asn Glu Asp Gln Leu Phe Gln Arg Thr Ala Glu Val His Ser Arg
   225                 230                 235                 240
   Val Leu Ala Ala Asn Asn Val Arg Arg Phe Phe Gly Phe Val Ala Leu
                   245                 250                 255
   Asn Lys Asp Asp Glu Glu Leu Ile Ala Asn Phe Pro Val Glu Gly Thr
               260                 265                 270
   Gln Pro Arg Pro Gln Trp Asn Ala Ile Thr Gly Val Tyr Leu Tyr Arg
           275                 280                 285
   Glu Asn Gln Gly Leu Pro Leu Tyr Ser Arg Leu His Lys Trp Ala Gln
   290 295 300
   Gly Leu Ala Gly Asn Gly Ala Ala Pro Asp Asn Val Glu Met Ala Leu
   305 310 315 320
   Leu Pro Ser
<210> 6
   <211> 918
   <212> PRT
   <213> Homo Sapiens
<220>
   <221> MISC_FEATURE
   <222> (1)..(918)
   <223> OGTA041 = Swiss Prot Accession No. P40189, Interleukin-6 receptor subunit beta
<400> 6
<210> 7
   <211> 1042
   <212> PRT
   <213> Homo Sapiens
<220>
   <221> MISC_FEATURE
   <222> (1)..(1042)
   <223> OGTA053 = Swiss Prot Accession No. Q9Y4D2, Snl-specific diacylglycerol lipase alpha
<400> 7
   Met Pro Gly Ile Val Val Phe Arg Arg Arg Trp Ser Val Gly Ser Asp
   1 5 10 15
   Asp Leu Val Leu Pro Ala Ile Phe Leu Phe Leu Leu His Thr Thr Trp
   20 25 30
   Phe Val Ile Leu Ser Val Val Leu Phe Gly Leu Val Tyr Asn Pro His
   35 40 45
   Glu Ala Cys Ser Leu Asn Leu Val Asp His Gly Arg Gly Tyr Leu Gly
   50 55 60
   Ile Leu Leu Ser Cys Met Ile Ala Glu Met Ala Ile Ile Trp Leu Ser
   65 70 75 80
   Met Arg Gly Gly Ile Leu Tyr Thr Glu Pro Arg Asp Ser Met Gln Tyr
   85 90 95
   Val Leu Tyr Val Arg Leu Ala Ile Leu Val Ile Glu Phe Ile Tyr Ala
   100 105 110
   Ile Val Gly Ile Val Trp Leu Thr Gln Tyr Tyr Thr Ser Cys Asn Asp
   115 120 125
   Leu Thr Ala Lys Asn Val Thr Leu Gly Met Val Val Cys Asn Trp Val
   130 135 140
   Val Ile Leu Ser Val Cys Ile Thr Val Leu Cys Val Phe Asp Pro Thr
   145 150 155 160
   Gly Arg Thr Phe Val Lys Leu Arg Ala Thr Lys Arg Arg Gln Arg Asn
   165 170 175
   Leu Arg Thr Tyr Asn Leu Arg His Arg Leu Glu Glu Gly Gln Ala Thr
               180                 185                 190
   Ser Trp Ser Arg Arg Leu Lys Val Phe Leu Cys Cys Thr Arg Thr Lys
           195                 200                 205
   Asp Ser Gln Ser Asp Ala Tyr Ser Glu Ile Ala Tyr Leu Phe Ala Glu
       210                 215                 220
   Phe Phe Arg Asp Leu Asp Ile Val Pro Ser Asp Ile Ile Ala Gly Leu
   225                 230                 235                 240
   Val Leu Leu Arg Gln Arg Gln Arg Ala Lys Arg Asn Ala Val Leu Asp
                   245                 250                 255
   Glu Ala Asn Asn Asp Ile Leu Ala Phe Leu Ser Gly Met Pro Val Thr
               260                 265                 270
   Arg Asn Thr Lys Tyr Leu Asp Leu Lys Asn Ser Gln Glu Met Leu Arg
           275                 280                 285
   Tyr Lys Glu Val Cys Tyr Tyr Met Leu Phe Ala Leu Ala Ala Tyr Gly
       290                 295                 300
   Trp Pro Met Tyr Leu Met Arg Lys Pro Ala Cys Gly Leu Cys Gln Leu
   305                 310                 315                 320
   Ala Arg Ser Cys Ser Cys Cys Leu Cys Pro Ala Arg Pro Arg Phe Ala
                   325                 330                 335
   Pro Gly Val Thr Ile Glu Glu Asp Asn Cys Cys Gly Cys Asn Ala Ile
               340                 345                 350
   Ala Ile Arg Arg His Phe Leu Asp Glu Asn Met Thr Ala Val Asp Ile
           355                 360                 365
   Val Tyr Thr Ser Cys His Asp Ala Val Tyr Glu Thr Pro Phe Tyr Val
       370                 375                 380
   Ala Val Asp His Asp Lys Lys Lys Val Val Ile Ser Ile Arg Gly Thr
   385                 390                 395                 400
   Leu Ser Pro Lys Asp Ala Leu Thr Asp Leu Thr Gly Asp Ala Glu Arg
                   405                 410                 415
   Leu Pro Val Glu Gly His His Gly Thr Trp Leu Gly His Lys Gly Met
               420                 425                 430
   Val Leu Ser Ala Glu Tyr Ile Lys Lys Lys Leu Glu Gln Glu Met Val
   435 440 445
   Leu Ser Gln Ala Phe Gly Arg Asp Leu Gly Arg Gly Thr Lys His Tyr
   450 455 460
   Gly Leu Ile Val Val Gly His Ser Leu Gly Ala Gly Thr Ala Ala Ile
   465 470 475 480
   Leu Ser Phe Leu Leu Arg Pro Gln Tyr Pro Thr Leu Lys Cys Phe Ala
   485 490 495
   Tyr Ser Pro Pro Gly Gly Leu Leu Ser Glu Asp Ala Met Glu Tyr Ser
   500 505 510
   Lys Glu Phe Val Thr Ala Val Val Leu Gly Lys Asp Leu Val Pro Arg
   515 520 525
   Ile Gly Leu Ser Gln Leu Glu Gly Phe Arg Arg Gln Leu Leu Asp Val
   530 535 540
   Leu Gln Arg Ser Thr Lys Pro Lys Trp Arg Ile Ile Val Gly Ala Thr
   545 550 555 560
   Lys Cys Ile Pro Lys Ser Glu Leu Pro Glu Glu Val Glu Val Thr Thr
   565 570 575
   Leu Ala Ser Thr Arg Leu Trp Thr His Pro Ser Asp Leu Thr Ile Ala
   580 585 590
   Leu Ser Ala Ser Thr Pro Leu Tyr Pro Pro Gly Arg Ile Ile His Val
   595 600 605
   Val His Asn His Pro Ala Glu Gln Cys Cys Cys Cys Glu Gln Glu Glu
   610 615 620
   Pro Thr Tyr Phe Ala Ile Trp Gly Asp Asn Lys Ala Phe Asn Glu Val
   625 630 635 640
   Ile Ile Ser Pro Ala Met Leu His Glu His Leu Pro Tyr Val Val Met
   645 650 655
   Glu Gly Leu Asn Lys Val Leu Glu Asn Tyr Asn Lys Gly Lys Thr Ala
   660 665 670
   Leu Leu Ser Ala Ala Lys Val Met Val Ser Pro Thr Glu Val Asp Leu
   675 680 685
   Thr Pro Glu Leu Ile Phe Gln Gln Gln Pro Leu Pro Thr Gly Pro Pro
   690 695 700
   Met Pro Thr Gly Leu Ala Leu Glu Leu Pro Thr Ala Asp His Arg Asn
   705 710 715 720
   Ser Ser Val Arg Ser Lys Ser Gln Ser Glu Met Ser Leu Glu Gly Phe
   725 730 735
   Ser Glu Gly Arg Leu Leu Ser Pro Val Val Ala Ala Ala Ala Arg Gln
   740 745 750
   Asp Pro Val Glu Leu Leu Leu Leu Ser Thr Gln Glu Arg Leu Ala Ala
   755 760 765
   Glu Leu Gln Ala Arg Arg Ala Pro Leu Ala Thr Met Glu Ser Leu Ser
   770 775 780
   Asp Thr Glu Ser Leu Tyr Ser Phe Asp Ser Arg Arg Ser Ser Gly Phe
   785 790 795 800
   Arg Ser Ile Arg Gly Ser Pro Ser Leu His Ala Val Leu Glu Arg Asp
   805 810 815
   Glu Gly His Leu Phe Tyr Ile Asp Pro Ala Ile Pro Glu Glu Asn Pro
   820 825 830
   Ser Leu Ser Ser Arg Thr Glu Leu Leu Ala Ala Asp Ser Leu Ser Lys
   835 840 845
   His Ser Gln Asp Thr Gln Pro Leu Glu Ala Ala Leu Gly Ser Gly Gly
   850 855 860
   Val Thr Pro Glu Arg Pro Pro Ser Ala Ala Ala Asn Asp Glu Glu Glu
   865 870 875 880
   Glu Val Gly Gly Gly Gly Gly Gly Pro Ala Ser Arg Gly Glu Leu Ala
   885 890 895
   Leu His Asn Gly Arg Leu Gly Asp Ser Pro Ser Pro Gln Val Leu Glu
   900 905 910
   Phe Ala Glu Phe Ile Asp Ser Leu Phe Asn Leu Asp Ser Lys Ser Ser
   915 920 925
   Ser Phe Gln Asp Leu Tyr Cys Met Val Val Pro Glu Ser Pro Thr Ser
   930 935 940
   Asp Tyr Ala Glu Gly Pro Lys Ser Pro Ser Gln Gln Glu Ile Leu Leu
   945 950 955 960
   Arg Ala Gln Phe Glu Pro Asn Leu Val Pro Lys Pro Pro Arg Leu Phe
   965 970 975
   Ala Gly Ser Ala Asp Pro Ser Ser Gly Ile Ser Leu Ser Pro Ser Phe
   980 985 990
   Pro Leu Ser Ser Ser Gly Glu Leu Met Asp Leu Thr Pro Thr Gly Leu
   995 1000 1005
   Ser Ser Gln Glu Cys Leu Ala Ala Asp Lys Ile Arg Thr Ser Thr
   1010 1015 1020
   Pro Thr Gly His Gly Ala Ser Pro Ala Lys Gln Asp Glu Leu Val
   1025 1030 1035
   Ile Ser Ala Arg
   1040
<210> 8
   <211> 125
   <212> PRT
   <213> Homo Sapiens
<220>
   <221> MISC_FEATURE
   <222> (1)..(125)
   <223> OGTA054 = Swiss Prot Accession No. P13164, Interferon-induced transmembrane protein 1
<400> 8
   Met His Lys Glu Glu His Glu Val Ala Val Leu Gly Ala Pro Pro Ser
   1 5 10 15
   Thr Ile Leu Pro Arg Ser Thr Val Ile Asn Ile His Ser Glu Thr Ser
   20 25 30
   Val Pro Asp His Val Val Trp Ser Leu Phe Asn Thr Leu Phe Leu Asn
   35 40 45
   Trp Cys Cys Leu Gly Phe Ile Ala Phe Ala Tyr Ser Val Lys Ser Arg
   50 55 60
   Asp Arg Lys Met Val Gly Asp Val Thr Gly Ala Gln Ala Tyr Ala Ser
   65 70 75 80
   Thr Ala Lys Cys Leu Asn Ile Trp Ala Leu Ile Leu Gly Ile Leu Met
   85 90 95
   Thr Ile Gly Phe Ile Leu Leu Leu Val Phe Gly Ser Val Thr Val Tyr
   100 105 110
   His Ile Met Leu Gln Ile Ile Gln Glu Lys Arg Gly Tyr
   115 120 125
<210> 9
   <211> 166
   <212> PRT
   <213> Homo Sapiens
<220>
   <221> MISC_FEATURE
   <222> (1)..(166)
   <223> OGTA066 = Swiss Prot Accession No. Q8TD06, Anterior gradient protein 3 homolog
<400> 9
   Met Met Leu His Ser Ala Leu Gly Leu Cys Leu Leu Leu Val Thr Val
   1 5 10 15
   Ser Ser Asn Leu Ala Ile Ala Ile Lys Lys Glu Lys Arg Pro Pro Gln
   20 25 30
   Thr Leu Ser Arg Gly Trp Gly Asp Asp Ile Thr Trp Val Gln Thr Tyr
   35 40 45
   Glu Glu Gly Leu Phe Tyr Ala Gln Lys Ser Lys Lys Pro Leu Met Val
   50 55 60
   Ile His His Leu Glu Asp Cys Gln Tyr Ser Gln Ala Leu Lys Lys Val
   65 70 75 80
   Phe Ala Gln Asn Glu Glu Ile Gln Glu Met Ala Gln Asn Lys Phe Ile
   85 90 95
   Met Leu Asn Leu Met His Glu Thr Thr Asp Lys Asn Leu Ser Pro Asp
   100 105 110
   Gly Gln Tyr Val Pro Arg Ile Met Phe Val Asp Pro Ser Leu Thr Val
   115 120 125
   Arg Ala Asp Ile Ala Gly Arg Tyr Ser Asn Arg Leu Tyr Thr Tyr Glu
   130 135 140
   Pro Arg Asp Leu Pro Leu Leu Ile Glu Asn Met Lys Lys Ala Leu Arg
   145                 150                 155                 160
   Leu Ile Gln Ser Glu Leu
                   165
<210> 10
   <211> 407
   <212> PRT
   <213> Homo Sapiens
<220>
   <221> MISC_FEATURE
   <222> (1)..(407)
   <223> OGTA072 = Swiss Prot Accession No. Q16186, Adhesion-regulating molecule 1
<400> 10
   Met Thr Thr Ser Gly Ala Leu Phe Pro Ser Leu Val Pro Gly Ser Arg
   1               5                   10                  15
   Gly Ala Ser Asn Lys Tyr Leu Val Glu Phe Arg Ala Gly Lys Met Ser
               20                  25                  30
   Leu Lys Gly Thr Thr Val Thr Pro Asp Lys Arg Lys Gly Leu Val Tyr
           35                  40                  45
   Ile Gln Gln Thr Asp Asp Ser Leu Ile His Phe Cys Trp Lys Asp Arg
       50                  55                  60
   Thr Ser Gly Asn Val Glu Asp Asp Leu Ile Ile Phe Pro Asp Asp Cys
   65                  70                  75                  80
   Glu Phe Lys Arg Val Pro Gln Cys Pro Ser Gly Arg Val Tyr Val Leu
                   85                  90                  95
   Lys Phe Lys Ala Gly Ser Lys Arg Leu Phe Phe Trp Met Gln Glu Pro
               100                 105                 110
   Lys Thr Asp Gln Asp Glu Glu His Cys Arg Lys Val Asn Glu Tyr Leu
           115                 120                 125
   Asn Asn Pro Pro Met Pro Gly Ala Leu Gly Ala Ser Gly Ser Ser Gly
       130                 135                 140
   His Glu Leu Ser Ala Leu Gly Gly Glu Gly Gly Leu Gln Ser Leu Leu
   145                 150                 155                 160
   Gly Asn Met Ser His Ser Gln Leu Met Gln Leu Ile Gly Pro Ala Gly
                   165                 170                 175
   Leu Gly Gly Leu Gly Gly Leu Gly Ala Leu Thr Gly Pro Gly Leu Ala
               180                 185                 190
   Ser Leu Leu Gly Ser Ser Gly Pro Pro Gly Ser Ser Ser Ser Ser Ser
           195                 200                 205
   Ser Arg Ser Gln Ser Ala Ala Val Thr Pro Ser Ser Thr Thr Ser Ser
       210                 215                 220
   Thr Arg Ala Thr Pro Ala Pro Ser Ala Pro Ala Ala Ala Ser Ala Thr
   225                 230                 235                 240
   Ser Pro Ser Pro Ala Pro Ser Ser Gly Asn Gly Ala Ser Thr Ala Ala
                   245                 250                 255
   Ser Pro Thr Gln Pro Ile Gln Leu Ser Asp Leu Gln Ser Ile Leu Ala
               260                 265                 270
   Thr Met Asn Val Pro Ala Gly Pro Ala Gly Gly Gln Gln Val Asp Leu
           275                 280                 285
   Ala Ser Val Leu Thr Pro Glu Ile Met Ala Pro Ile Leu Ala Asn Ala
       290                 295                 300
   Asp Val Gln Glu Arg Leu Leu Pro Tyr Leu Pro Ser Gly Glu Ser Leu
   305                 310                 315                 320
   Pro Gln Thr Ala Asp Glu Ile Gln Asn Thr Leu Thr Ser Pro Gln Phe
                   325                 330                 335
   Gln Gln Ala Leu Gly Met Phe Ser Ala Ala Leu Ala Ser Gly Gln Leu
               340                 345                 350
   Gly Pro Leu Met Cys Gln Phe Gly Leu Pro Ala Glu Ala Val Glu Ala
           355                 360                 365
   Ala Asn Lys Gly Asp Val Glu Ala Phe Ala Lys Ala Met Gln Asn Asn
       370                 375                 380
   Ala Lys Pro Glu Gln Lys Glu Gly Asp Thr Lys Asp Lys Lys Asp Glu
   385                 390                 395                 400
   Glu Glu Asp Met Ser Leu Asp
                   405
<210> 11
   <211> 1587
   <212> PRT
   <213> Homo Sapiens
<220>
   <221> MISC_FEATURE
   <222> (1)..(1587)
   <223> OGTA074 = Swiss Prot Accession No. 000508, Latent TGF-beta binding protein-4
<400> 11
   Met Gly Asp Val Lys Ala Leu Leu Phe Val Ala Ala Ala Arg Ala Arg
   1               5                   10                  15
   Arg Leu Gly Gly Ala Ala Ala Ser Glu Ser Leu Ala Val Ser Glu Ala
               20                  25                  30
   Phe Cys Arg Val Arg Ser Cys Gln Pro Lys Lys Cys Ala Gly Pro Gln
           35                  40                  45
   Arg Cys Leu Asn Pro Val Pro Ala Val Pro Ser Pro Ser Pro Ser Val
       50                  55                  60
   Arg Lys Arg Gln Val Ser Leu Asn Trp Gln Pro Leu Thr Leu Gln Glu
   65                  70                  75                  80
   Ala Arg Ala Leu Leu Lys Arg Arg Arg Pro Arg Gly Pro Gly Gly Arg
                   85                  90                  95
   Gly Leu Leu Arg Arg Arg Pro Pro Gln Arg Ala Pro Ala Gly Lys Ala
               100                 105                 110
   Pro Val Leu Cys Pro Leu Ile Cys His Asn Gly Gly Val Cys Val Lys
           115                 120                 125
   Pro Asp Arg Cys Leu Cys Pro Pro Asp Phe Ala Gly Lys Phe Cys Gln
       130                 135                 140
   Leu His Ser Ser Gly Ala Arg Pro Pro Ala Pro Ala Val Pro Gly Leu
   145                 150                 155                 160
   Thr Arg Ser Val Tyr Thr Met Pro Leu Ala Asn His Arg Asp Asp Glu
                   165                 170                 175
   His Gly Val Ala Ser Met Val Ser Val His Val Glu His Pro Gln Glu
               180                 185                 190
   Ala Ser Val Val Val His Gln Val Glu Arg Val Ser Gly Pro Trp Glu
   195 200 205
   Glu Ala Asp Ala Glu Ala Val Ala Arg Ala Glu Ala Ala Ala Arg Ala
   210 215 220
   Glu Ala Ala Ala Pro Tyr Thr Val Leu Ala Gln Ser Ala Pro Arg Glu
   225 230 235 240
   Asp Gly Tyr Ser Asp Ala Ser Gly Phe Gly Tyr Cys Phe Arg Glu Leu
   245 250 255
   Arg Gly Gly Glu Cys Ala Ser Pro Leu Pro Gly Leu Arg Thr Gln Glu
   260 265 270
   Val Cys Cys Arg Gly Ala Gly Leu Ala Trp Gly Val His Asp Cys Gln
   275 280 285
   Leu Cys Ser Glu Arg Leu Gly Asn Ser Glu Arg Val Ser Ala Pro Asp
   290 295 300
   Gly Pro Cys Pro Thr Gly Phe Glu Arg Val Asn Gly Ser Cys Glu Asp
   305 310 315 320
   Val Asp Glu Cys Ala Thr Gly Gly Arg Cys Gln His Gly Glu Cys Ala
   325 330 335
   Asn Thr Arg Gly Gly Tyr Thr Cys Val Cys Pro Asp Gly Phe Leu Leu
   340 345 350
   Asp Ser Ser Arg Ser Ser Cys Ile Ser Gln His Val Ile Ser Glu Ala
   355 360 365
   Lys Gly Pro Cys Phe Arg Val Leu Arg Asp Gly Gly Cys Ser Leu Pro
   370 375 380
   Ile Leu Arg Asn Ile Thr Lys Gln Ile Cys Cys Cys Ser Arg Val Gly
   385 390 395 400
   Lys Ala Trp Gly Arg Gly Cys Gln Leu Cys Pro Pro Phe Gly Ser Glu
   405 410 415
   Gly Phe Arg Glu Ile Cys Pro Ala Gly Pro Gly Tyr His Tyr Ser Ala
   420 425 430
   Ser Asp Leu Arg Tyr Asn Thr Arg Pro Leu Gly Gln Glu Pro Pro Arg
   435 440 445
   Val Ser Leu Ser Gln Pro Arg Thr Leu Pro Ala Thr Ser Arg Pro Ser
       450                 455                 460
   Ala Gly Phe Leu Pro Thr His Arg Leu Glu Pro Arg Pro Glu Pro Arg
   465                 470                 475                 480
   Pro Asp Pro Arg Pro Gly Pro Glu Leu Pro Leu Pro Ser Ile Pro Ala
                   485                 490                 495
   Trp Thr Gly Pro Glu Ile Pro Glu Ser Gly Pro Ser Ser Gly Met Cys
               500                 505                 510
   Gln Arg Asn Pro Gln Val Cys Gly Pro Gly Arg Cys Ile Ser Arg Pro
           515                 520                 525
   Ser Gly Tyr Thr Cys Ala Cys Asp Ser Gly Phe Arg Leu Ser Pro Gln
       530                 535                 540
   Gly Thr Arg Cys Ile Asp Val Asp Glu Cys Arg Arg Val Pro Pro Pro
   545                 550                 555                 560
   Cys Ala Pro Gly Arg Cys Glu Asn Ser Pro Gly Ser Phe Arg Cys Val
                   565                 570                 575
   Cys Gly Pro Gly Phe Arg Ala Gly Pro Arg Ala Ala Glu Cys Leu Asp
               580                 585                 590
   Val Asp Glu Cys His Arg Val Pro Pro Pro Cys Asp Leu Gly Arg Cys
           595                 600                 605
   Glu Asn Thr Pro Gly Ser Phe Leu Cys Val Cys Pro Ala Gly Tyr Gln
       610                 615                 620
   Ala Ala Pro His Gly Ala Ser Cys Gln Asp Val Asp Glu Cys Thr Gln
   625                 630                 635                 640
   Ser Pro Gly Leu Cys Gly Arg Gly Ala Cys Lys Asn Leu Pro Gly Ser
                   645                 650                 655
   Phe Arg Cys Val Cys Pro Ala Gly Phe Arg Gly Ser Ala Cys Glu Glu
               660                 665                 670
   Asp Val Asp Glu Cys Ala Gln Glu Pro Pro Pro Cys Gly Pro Gly Arg
           675                 680                 685
   Cys Asp Asn Thr Ala Gly Ser Phe His Cys Ala Cys Pro Ala Gly Phe
       690                 695                 700
   Arg Ser Arg Gly Pro Gly Ala Pro Cys Gln Asp Val Asp Glu Cys Ala
   705 710 715 720
   Arg Ser Pro Pro Pro Cys Thr Tyr Gly Arg Cys Glu Asn Thr Glu Gly
   725 730 735
   Ser Phe Gln Cys Val Cys Pro Met Gly Phe Gln Pro Asn Thr Ala Gly
   740 745 750
   Ser Glu Cys Glu Asp Val Asp Glu Cys Glu Asn His Leu Ala Cys Pro
   755 760 765
   Gly Gln Glu Cys Val Asn Ser Pro Gly Ser Phe Gln Cys Arg Thr Cys
   770 775 780
   Pro Ser Gly His His Leu His Arg Gly Arg Cys Thr Asp Val Asp Glu
   785 790 795 800
   Cys Ser Ser Gly Ala Pro Pro Cys Gly Pro His Gly His Cys Thr Asn
   805 810 815
   Thr Glu Gly Ser Phe Arg Cys Ser Cys Ala Pro Gly Tyr Arg Ala Pro
   820 825 830
   Ser Gly Arg Pro Gly Pro Cys Ala Asp Val Asn Glu Cys Leu Glu Gly
   835 840 845
   Asp Phe Cys Phe Pro His Gly Glu Cys Leu Asn Thr Asp Gly Ser Phe
   850 855 860
   Ala Cys Thr Cys Ala Pro Gly Tyr Arg Pro Gly Pro Arg Gly Ala Ser
   865 870 875 880
   Cys Leu Asp Val Asp Glu Cys Ser Glu Glu Asp Leu Cys Gln Ser Gly
   885 890 895
   Ile Cys Thr Asn Thr Asp Gly Ser Phe Glu Cys Ile Cys Pro Pro Gly
   900 905 910
   His Arg Ala Gly Pro Asp Leu Ala Ser Cys Leu Asp Val Asp Glu Cys
   915 920 925
   Arg Glu Arg Gly Pro Ala Leu Cys Gly Ser Gln Arg Cys Glu Asn Ser
   930 935 940
   Pro Gly Ser Tyr Arg Cys Val Arg Asp Cys Asp Pro Gly Tyr His Ala
   945 950 955 960
   Gly Pro Glu Gly Thr Cys Asp Asp Val Asp Glu Cys Gln Glu Tyr Gly
   965 970 975
   Pro Glu Ile Cys Gly Ala Gln Arg Cys Glu Asn Thr Pro Gly Ser Tyr
   980 985 990
   Arg Cys Thr Pro Ala Cys Asp Pro Gly Tyr Gln Pro Thr Pro Gly Gly
   995 1000 1005
   Gly Cys Gln Asp Val Asp Glu Cys Arg Asn Arg Ser Phe Cys Gly
   1010 1015 1020
   Ala His Ala Val Cys Gln Asn Leu Pro Gly Ser Phe Gln Cys Leu
   1025 1030 1035
   Cys Asp Gln Gly Tyr Glu Gly Ala Arg Asp Gly Arg His Cys Val
   1040 1045 1050
   Asp Val Asn Glu Cys Glu Thr Leu Gln Gly Val Cys Gly Ala Ala
   1055 1060 1065
   Leu Cys Glu Asn Val Glu Gly Ser Phe Leu Cys Val Cys Pro Asn
   1070 1075 1080
   Ser Pro Glu Glu Phe Asp Pro Met Thr Gly Arg Cys Val Pro Pro
   1085 1090 1095
   Arg Thr Ser Ala Gly Thr Phe Pro Gly Ser Gln Pro Gln Ala Pro
   1100 1105 1110
   Ala Ser Pro Val Leu Pro Ala Arg Pro Pro Pro Pro Pro Leu Pro
   1115 1120 1125
   Arg Arg Pro Ser Thr Pro Arg Gln Gly Pro Val Gly Ser Gly Arg
   1130 1135 1140
   Arg Glu Cys Tyr Phe Asp Thr Ala Ala Pro Asp Ala Cys Asp Asn
   1145 1150 1155
   Ile Leu Ala Arg Asn Val Thr Trp Gln Glu Cys Cys Cys Thr Val
   1160 1165 1170
   Gly Glu Gly Trp Gly Ser Gly Cys Arg Ile Gln Gln Cys Pro Gly
   1175 1180 1185
   Thr Glu Thr Ala Glu Tyr Gln Ser Leu Cys Pro His Gly Arg Gly
   1190 1195 1200
   Tyr Leu Ala Pro Ser Gly Asp Leu Ser Leu Arg Arg Asp Val Asp
   1205 1210 1215
   Glu Cys Gln Leu Phe Arg Asp Gln Val Cys Lys Ser Gly Val Cys
   1220 1225 1230
   Val Asn Thr Ala Pro Gly Tyr Ser Cys Tyr Cys Ser Asn Gly Tyr
   1235 1240 1245
   Tyr Tyr His Thr Gln Arg Leu Glu Cys Ile Asp Asn Asp Glu Cys
   1250 1255 1260
   Ala Asp Glu Glu Pro Ala Cys Glu Gly Gly Arg Cys Val Asn Thr
   1265 1270 1275
   Val Gly Ser Tyr His Cys Thr Cys Glu Pro Pro Leu Val Leu Asp
   1280 1285 1290
   Gly Ser Gln Arg Arg Cys Val Ser Asn Glu Ser Gln Ser Leu Asp
   1295 1300 1305
   Asp Asn Leu Gly Val Cys Trp Gln Glu Val Gly Ala Asp Leu Val
   1310 1315 1320
   Cys Ser His Pro Arg Leu Asp Arg Gln Ala Thr Tyr Thr Glu Cys
   1325 1330 1335
   Cys Cys Leu Tyr Gly Glu Ala Trp Gly Met Asp Cys Ala Leu Cys
   1340 1345 1350
   Pro Ala Gln Asp Ser Asp Asp Phe Glu Ala Leu Cys Asn Val Leu
   1355 1360 1365
   Arg Pro Pro Ala Tyr Ser Pro Pro Arg Pro Gly Gly Phe Gly Leu
   1370 1375 1380
   Pro Tyr Glu Tyr Gly Pro Asp Leu Gly Pro Pro Tyr Gln Gly Leu
   1385 1390 1395
   Pro Tyr Gly Pro Glu Leu Tyr Pro Pro Pro Ala Leu Pro Tyr Asp
   1400 1405 1410
   Pro Tyr Pro Pro Pro Pro Gly Pro Phe Ala Arg Arg Glu Ala Pro
   1415 1420 1425
   Tyr Gly Ala Pro Arg Phe Asp Met Pro Asp Phe Glu Asp Asp Gly
   1430 1435 1440
   Gly Pro Tyr Gly Glu Ser Glu Ala Pro Ala Pro Pro Gly Pro Gly
   1445 1450 1455
   Thr Arg Trp Pro Tyr Arg Ser Arg Asp Thr Arg Arg Ser Phe Pro
   1460 1465 1470
   Glu Pro Glu Glu Pro Pro Glu Gly Gly Ser Tyr Ala Gly Ser Leu
   1475 1480 1485
   Ala Glu Pro Tyr Glu Glu Leu Glu Ala Glu Glu Cys Gly Ile Leu
   1490 1495 1500
   Asp Gly Cys Thr Asn Gly Arg Cys Val Arg Val Pro Glu Gly Phe
   1505 1510 1515
   Thr Cys Arg Cys Phe Asp Gly Tyr Arg Leu Asp Met Thr Arg Met
   1520 1525 1530
   Ala Cys Val Asp Ile Asn Glu Cys Asp Glu Ala Glu Ala Ala Ser
   1535 1540 1545
   Pro Leu Cys Val Asn Ala Arg Cys Leu Asn Thr Asp Gly Ser Phe
   1550 1555 1560
   Arg Cys Ile Cys Arg Pro Gly Phe Ala Pro Thr His Gln Pro His
   1565 1570 1575
   His Cys Ala Pro Ala Arg Pro Arg Ala
   1580 1585
<210> 12
   <211> 1722
   <212> PRT
   <213> Homo Sapiens
<220>
   <221> MISC_FEATURE
   <222> (1)..(1722)
   <223> OGTA076 = Swiss Prot Accession No. 060449, Lymphocyte antigen 75
<400> 12
   Met Arg Thr Gly Trp Ala Thr Pro Arg Arg Pro Ala Gly Leu Leu Met
   1 5 10 15
   Leu Leu Phe Trp Phe Phe Asp Leu Ala Glu Pro Ser Gly Arg Ala Ala
   20 25 30
   Asn Asp Pro Phe Thr Ile Val His Gly Asn Thr Gly Lys Cys Ile Lys
   35 40 45
   Pro Val Tyr Gly Trp Ile Val Ala Asp Asp Cys Asp Glu Thr Glu Asp
   50 55 60
   Lys Leu Trp Lys Trp Val Ser Gln His Arg Leu Phe His Leu His Ser
   65 70 75 80
   Gln Lys Cys Leu Gly Leu Asp Ile Thr Lys Ser Val Asn Glu Leu Arg
   85 90 95
   Met Phe Ser Cys Asp Ser Ser Ala Met Leu Trp Trp Lys Cys Glu His
   100 105 110
   His Ser Leu Tyr Gly Ala Ala Arg Tyr Arg Leu Ala Leu Lys Asp Gly
   115 120 125
   His Gly Thr Ala Ile Ser Asn Ala Ser Asp Val Trp Lys Lys Gly Gly
   130 135 140
   Ser Glu Glu Ser Leu Cys Asp Gln Pro Tyr His Glu Ile Tyr Thr Arg
   145 150 155 160
   Asp Gly Asn Ser Tyr Gly Arg Pro Cys Glu Phe Pro Phe Leu Ile Asp
   165 170 175
   Gly Thr Trp His His Asp Cys Ile Leu Asp Glu Asp His Ser Gly Pro
   180 185 190
   Trp Cys Ala Thr Thr Leu Asn Tyr Glu Tyr Asp Arg Lys Trp Gly Ile
   195 200 205
   Cys Leu Lys Pro Glu Asn Gly Cys Glu Asp Asn Trp Glu Lys Asn Glu
   210 215 220
   Gln Phe Gly Ser Cys Tyr Gln Phe Asn Thr Gln Thr Ala Leu Ser Trp
   225 230 235 240
   Lys Glu Ala Tyr Val Ser Cys Gln Asn Gln Gly Ala Asp Leu Leu Ser
   245 250 255
   Ile Asn Ser Ala Ala Glu Leu Thr Tyr Leu Lys Glu Lys Glu Gly Ile
   260 265 270
   Ala Lys Ile Phe Trp Ile Gly Leu Asn Gln Leu Tyr Ser Ala Arg Gly
           275                 280                 285
   Trp Glu Trp Ser Asp His Lys Pro Leu Asn Phe Leu Asn Trp Asp Pro
       290                 295                 300
   Asp Arg Pro Ser Ala Pro Thr Ile Gly Gly Ser Ser Cys Ala Arg Met
   305                 310                 315                 320
   Asp Ala Glu Ser Gly Leu Trp Gln Ser Phe Ser Cys Glu Ala Gln Leu
                   325                 330                 335
   Pro Tyr Val Cys Arg Lys Pro Leu Asn Asn Thr Val Glu Leu Thr Asp
               340                 345                 350
   Val Trp Thr Tyr Ser Asp Thr Arg Cys Asp Ala Gly Trp Leu Pro Asn
           355                 360                 365
   Asn Gly Phe Cys Tyr Leu Leu Val Asn Glu Ser Asn Ser Trp Asp Lys
       370                 375                 380
   Ala His Ala Lys Cys Lys Ala Phe Ser Ser Asp Leu Ile Ser Ile His
   385                 390                 395                 400
   Ser Leu Ala Asp Val Glu Val Val Val Thr Lys Leu His Asn Glu Asp
                   405                 410                 415
   Ile Lys Glu Glu Val Trp Ile Gly Leu Lys Asn Ile Asn Ile Pro Thr
               420                 425                 430
   Leu Phe Gln Trp Ser Asp Gly Thr Glu Val Thr Leu Thr Tyr Trp Asp
           435                 440                 445
   Glu Asn Glu Pro Asn Val Pro Tyr Asn Lys Thr Pro Asn Cys Val Ser
       450                 455                 460
   Tyr Leu Gly Glu Leu Gly Gln Trp Lys Val Gln Ser Cys Glu Glu Lys
   465                 470                 475                 480
   Leu Lys Tyr Val Cys Lys Arg Lys Gly Glu Lys Leu Asn Asp Ala Ser
                   485                 490                 495
   Ser Asp Lys Met Cys Pro Pro Asp Glu Gly Trp Lys Arg His Gly Glu
               500                 505                 510
   Thr Cys Tyr Lys Ile Tyr Glu Asp Glu Val Pro Phe Gly Thr Asn Cys
           515                 520                 525
   Asn Leu Thr Ile Thr Ser Arg Phe Glu Gln Glu Tyr Leu Asn Asp Leu
       530                 535                 540
   Met Lys Lys Tyr Asp Lys Ser Leu Arg Lys Tyr Phe Trp Thr Gly Leu
   545                 550                 555                 560
   Arg Asp Val Asp Ser Cys Gly Glu Tyr Asn Trp Ala Thr Val Gly Gly
                   565                 570                 575
   Arg Arg Arg Ala Val Thr Phe Ser Asn Trp Asn Phe Leu Glu Pro Ala
               580                 585                 590
   Ser Pro Gly Gly Cys Val Ala Met Ser Thr Gly Lys Ser Val Gly Lys
           595                 600                 605
   Trp Glu Val Lys Asp Cys Arg Ser Phe Lys Ala Leu Ser Ile Cys Lys
       610                 615                 620
   Lys Met Ser Gly Pro Leu Gly Pro Glu Glu Ala Ser Pro Lys Pro Asp
   625                 630                 635                 640
   Asp Pro Cys Pro Glu Gly Trp Gln Ser Phe Pro Ala Ser Leu Ser Cys
                   645                 650                 655
   Tyr Lys Val Phe His Ala Glu Arg Ile Val Arg Lys Arg Asn Trp Glu
               660                 665                 670
   Glu Ala Glu Arg Phe Cys Gln Ala Leu Gly Ala His Leu Ser Ser Phe
           675                 680                 685
   Ser His Val Asp Glu Ile Lys Glu Phe Leu His Phe Leu Thr Asp Gln
       690                 695                 700
   Phe Ser Gly Gln His Trp Leu Trp Ile Gly Leu Asn Lys Arg Ser Pro
   705                 710                 715                 720
   Asp Leu Gln Gly Ser Trp Gln Trp Ser Asp Arg Thr Pro Val Ser Thr
                   725                 730                 735
   Ile Ile Met Pro Asn Glu Phe Gln Gln Asp Tyr Asp Ile Arg Asp Cys
               740                 745                 750
   Ala Ala Val Lys Val Phe His Arg Pro Trp Arg Arg Gly Trp His Phe
           755                 760                 765
   Tyr Asp Asp Arg Glu Phe Ile Tyr Leu Arg Pro Phe Ala Cys Asp Thr
       770                 775                 780
   Lys Leu Glu Trp Val Cys Gln Ile Pro Lys Gly Arg Thr Pro Lys Thr
   785 790 795 800
   Pro Asp Trp Tyr Asn Pro Asp Arg Ala Gly Ile His Gly Pro Pro Leu
   805 810 815
   Ile Ile Glu Gly Ser Glu Tyr Trp Phe Val Ala Asp Leu His Leu Asn
   820 825 830
   Tyr Glu Glu Ala Val Leu Tyr Cys Ala Ser Asn His Ser Phe Leu Ala
   835 840 845
   Thr Ile Thr Ser Phe Val Gly Leu Lys Ala Ile Lys Asn Lys Ile Ala
   850 855 860
   Asn Ile Ser Gly Asp Gly Gln Lys Trp Trp Ile Arg Ile Ser Glu Trp
   865 870 875 880
   Pro Ile Asp Asp His Phe Thr Tyr Ser Arg Tyr Pro Trp His Arg Phe
   885 890 895
   Pro Val Thr Phe Gly Glu Glu Cys Leu Tyr Met Ser Ala Lys Thr Trp
   900 905 910
   Leu Ile Asp Leu Gly Lys Pro Thr Asp Cys Ser Thr Lys Leu Pro Phe
   915 920 925
   Ile Cys Glu Lys Tyr Asn Val Ser Ser Leu Glu Lys Tyr Ser Pro Asp
   930 935 940
   Ser Ala Ala Lys Val Gln Cys Ser Glu Gln Trp Ile Pro Phe Gln Asn
   945 950 955 960
   Lys Cys Phe Leu Lys Ile Lys Pro Val Ser Leu Thr Phe Ser Gln Ala
   965 970 975
   Ser Asp Thr Cys His Ser Tyr Gly Gly Thr Leu Pro Ser Val Leu Ser
   980 985 990
   Gln Ile Glu Gln Asp Phe Ile Thr Ser Leu Leu Pro Asp Met Glu Ala
   995 1000 1005
   Thr Leu Trp Ile Gly Leu Arg Trp Thr Ala Tyr Glu Lys Ile Asn
   1010 1015 1020
   Lys Trp Thr Asp Asn Arg Glu Leu Thr Tyr Ser Asn Phe His Pro
   1025 1030 1035
   Leu Leu Val Ser Gly Arg Leu Arg Ile Pro Glu Asn Phe Phe Glu
   1040 1045 1050
   Glu Glu Ser Arg Tyr His Cys Ala Leu Ile Leu Asn Leu Gln Lys
   1055 1060 1065
   Ser Pro Phe Thr Gly Thr Trp Asn Phe Thr Ser Cys Ser Glu Arg
   1070 1075 1080
   His Phe Val Ser Leu Cys Gln Lys Tyr Ser Glu Val Lys Ser Arg
   1085 1090 1095
   Gln Thr Leu Gln Asn Ala Ser Glu Thr Val Lys Tyr Leu Asn Asn
   1100 1105 1110
   Leu Tyr Lys Ile Ile Pro Lys Thr Leu Thr Trp His Ser Ala Lys
   1115 1120 1125
   Arg Glu Cys Leu Lys Ser Asn Met Gln Leu Val Ser Ile Thr Asp
   1130 1135 1140
   Pro Tyr Gln Gln Ala Phe Leu Ser Val Gln Ala Leu Leu His Asn
   1145 1150 1155
   Ser Ser Leu Trp Ile Gly Leu Phe Ser Gln Asp Asp Glu Leu Asn
   1160 1165 1170
   Phe Gly Trp Ser Asp Gly Lys Arg Leu His Phe Ser Arg Trp Ala
   1175 1180 1185
   Glu Thr Asn Gly Gln Leu Glu Asp Cys Val Val Leu Asp Thr Asp
   1190 1195 1200
   Gly Phe Trp Lys Thr Val Asp Cys Asn Asp Asn Gln Pro Gly Ala
   1205 1210 1215
   Ile Cys Tyr Tyr Ser Gly Asn Glu Thr Glu Lys Glu Val Lys Pro
   1220 1225 1230
   Val Asp Ser Val Lys Cys Pro Ser Pro Val Leu Asn Thr Pro Trp
   1235 1240 1245
   Ile Pro Phe Gln Asn Cys Cys Tyr Asn Phe Ile Ile Thr Lys Asn
   1250 1255 1260
   Arg His Met Ala Thr Thr Gln Asp Glu Val His Thr Lys Cys Gln
   1265 1270 1275
   Lys Leu Asn Pro Lys Ser His Ile Leu Ser Ile Arg Asp Glu Lys
   1280 1285 1290
   Glu Asn Asn Phe Val Leu Glu Gln Leu Leu Tyr Phe Asn Tyr Met
   1295 1300 1305
   Ala Ser Trp Val Met Leu Gly Ile Thr Tyr Arg Asn Asn Ser Leu
   1310 1315 1320
   Met Trp Phe Asp Lys Thr Pro Leu Ser Tyr Thr His Trp Arg Ala
   1325 1330 1335
   Gly Arg Pro Thr Ile Lys Asn Glu Lys Phe Leu Ala Gly Leu Ser
   1340 1345 1350
   Thr Asp Gly Phe Trp Asp Ile Gln Thr Phe Lys Val Ile Glu Glu
   1355 1360 1365
   Ala Val Tyr Phe His Gln His Ser Ile Leu Ala Cys Lys Ile Glu
   1370 1375 1380
   Met Val Asp Tyr Lys Glu Glu His Asn Thr Thr Leu Pro Gln Phe
   1385 1390 1395
   Met Pro Tyr Glu Asp Gly Ile Tyr Ser Val Ile Gln Lys Lys Val
   1400 1405 1410
   Thr Trp Tyr Glu Ala Leu Asn Met Cys Ser Gln Ser Gly Gly His
   1415 1420 1425
   Leu Ala Ser Val His Asn Gln Asn Gly Gln Leu Phe Leu Glu Asp
   1430 1435 1440
   Ile Val Lys Arg Asp Gly Phe Pro Leu Trp Val Gly Leu Ser Ser
   1445 1450 1455
   His Asp Gly Ser Glu Ser Ser Phe Glu Trp Ser Asp Gly Ser Thr
   1460 1465 1470
   Phe Asp Tyr Ile Pro Trp Lys Gly Gln Thr Ser Pro Gly Asn Cys
   1475 1480 1485
   Val Leu Leu Asp Pro Lys Gly Thr Trp Lys His Glu Lys Cys Asn
   1490 1495 1500
   Ser Val Lys Asp Gly Ala Ile Cys Tyr Lys Pro Thr Lys Ser Lys
   1505 1510 1515
   Lys Leu Ser Arg Leu Thr Tyr Ser Ser Arg Cys Pro Ala Ala Lys
   1520 1525 1530
   Glu Asn Gly Ser Arg Trp Ile Gln Tyr Lys Gly His Cys Tyr Lys
   1535 1540 1545
   Ser Asp Gln Ala Leu His Ser Phe Ser Glu Ala Lys Lys Leu Cys
   1550 1555 1560
   Ser Lys His Asp His Ser Ala Thr Ile Val Ser Ile Lys Asp Glu
   1565 1570 1575
   Asp Glu Asn Lys Phe Val Ser Arg Leu Met Arg Glu Asn Asn Asn
   1580 1585 1590
   Ile Thr Met Arg Val Trp Leu Gly Leu Ser Gln His Ser Val Asp
   1595 1600 1605
   Gln Ser Trp Ser Trp Leu Asp Gly Ser Glu Val Thr Phe Val Lys
   1610 1615 1620
   Trp Glu Asn Lys Ser Lys Ser Gly Val Gly Arg Cys Ser Met Leu
   1625 1630 1635
   Ile Ala Ser Asn Glu Thr Trp Lys Lys Val Glu Cys Glu His Gly
   1640 1645 1650
   Phe Gly Arg Val Val Cys Lys Val Pro Leu Gly Pro Asp Tyr Thr
   1655 1660 1665
   Ala Ile Ala Ile Ile Val Ala Thr Leu Ser Ile Leu Val Leu Met
   1670 1675 1680
   Gly Gly Leu Ile Trp Phe Leu Phe Gln Arg His Arg Leu His Leu
   1685 1690 1695
   Ala Gly Phe Ser Ser Val Arg Tyr Ala Gln Gly Val Asn Glu Asp
   1700 1705 1710
   Glu Ile Met Leu Pro Ser Phe His Asp
   1715 1720
<210> 13
   <211> 355
   <212> PRT
   <213> Homo Sapiens
<220>
   <221> MISC_FEATURE
   <222> (1)..(355)
   <223> OGTA085 = Swiss Prot Accession No. Q14318, 38 kDa FK506-binding protein homologue
<400> 13
   Met Gly Gln Pro Pro Ala Glu Glu Ala Glu Gln Pro Gly Ala Leu Ala
   1               5                   10                  15
   Arg Glu Phe Leu Ala Ala Met Glu Pro Glu Pro Ala Pro Ala Pro Ala
               20                  25                  30
   Pro Glu Glu Trp Leu Asp Ile Leu Gly Asn Gly Leu Leu Arg Lys Lys
           35                  40                  45
   Thr Leu Val Pro Gly Pro Pro Gly Ser Ser Arg Pro Val Lys Gly Gln
       50                  55                  60
   Val Val Thr Val His Leu Gln Thr Ser Leu Glu Asn Gly Thr Arg Val
   65                  70                  75                  80
   Gln Glu Glu Pro Glu Leu Val Phe Thr Leu Gly Asp Cys Asp Val Ile
                   85                  90                  95
   Gln Ala Leu Asp Leu Ser Val Pro Leu Met Asp Val Gly Glu Thr Ala
               100                 105                 110
   Met Val Thr Ala Asp Ser Lys Tyr Cys Tyr Gly Pro Gln Gly Arg Ser
           115                 120                 125
   Pro Tyr Ile Pro Pro His Ala Ala Leu Cys Leu Glu Val Thr Leu Lys
       130                 135                 140
   Thr Ala Val Asp Gly Pro Asp Leu Glu Met Leu Thr Gly Gln Glu Arg
   145                 150                 155                 160
   Val Ala Leu Ala Asn Arg Lys Arg Glu Cys Gly Asn Ala His Tyr Gln
                   165                 170                 175
   Arg Ala Asp Phe Val Leu Ala Ala Asn Ser Tyr Asp Leu Ala Ile Lys
               180                 185                 190
   Ala Ile Thr Ser Ser Ala Lys Val Asp Met Thr Phe Glu Glu Glu Ala
           195                 200                 205
   Gln Leu Leu Gln Leu Lys Val Lys Cys Leu Asn Asn Leu Ala Ala Ser
       210                 215                 220
   Gln Leu Lys Leu Asp His Tyr Arg Ala Ala Leu Arg Ser Cys Ser Leu
   225                 230                 235                 240
   Val Leu Glu His Gln Pro Asp Asn Ile Lys Ala Leu Phe Arg Lys Gly
                   245                 250                 255
   Lys Val Leu Ala Gln Gln Gly Glu Tyr Ser Glu Ala Ile Pro Ile Leu
               260                 265                 270
   Arg Ala Ala Leu Lys Leu Glu Pro Ser Asn Lys Thr Ile His Ala Glu
           275                 280                 285
   Leu Ser Lys Leu Val Lys Lys His Ala Ala Gln Arg Ser Thr Glu Thr
       290                 295                 300
   Ala Leu Tyr Arg Lys Met Leu Gly Asn Pro Ser Arg Leu Pro Ala Lys
   305                 310                 315                 320
   Cys Pro Gly Lys Gly Ala Trp Ser Ile Pro Trp Lys Trp Leu Phe Gly
                   325                 330                 335
   Ala Thr Ala Val Ala Leu Gly Gly Val Ala Leu Ser Val Val Ile Ala
               340                 345                 350
   Ala Arg Asn
           355
<210> 14
   <211> 587
   <212> PRT
   <213> Homo Sapiens
<220>
   <221> MISC_FEATURE
   <222> (1)..(587)
   <223> OGTA087 = Swiss Prot Accession No. O94935, KIAA0851 protein
<400> 14
   Met Ala Thr Ala Ala Tyr Glu Gln Leu Lys Leu His Ile Thr Pro Glu
   1               5                   10                  15
   Lys Phe Tyr Val Glu Ala Cys Asp Asp Gly Ala Asp Asp Val Leu Thr
               20                  25                  30
   Ile Asp Arg Val Ser Thr Glu Val Thr Leu Ala Val Lys Lys Asp Val
   35 40 45
   Pro Pro Ser Ala Val Thr Arg Pro Ile Phe Gly Ile Leu Gly Thr Ile
   50 55 60
   His Leu Val Ala Gly Asn Tyr Leu Ile Val Ile Thr Lys Lys Ile Lys
   65 70 75 80
   Val Gly Glu Phe Phe Ser His Val Val Trp Lys Ala Thr Asp Phe Asp
   85 90 95
   Val Leu Ser Tyr Lys Lys Thr Met Leu His Leu Thr Asp Ile Gln Leu
   100 105 110
   Gln Asp Asn Lys Thr Phe Leu Ala Met Leu Asn His Val Leu Asn Val
   115 120 125
   Asp Gly Phe Tyr Phe Ser Thr Thr Tyr Asp Leu Thr His Thr Leu Gln
   130 135 140
   Arg Leu Ser Asn Thr Ser Pro Glu Phe Gln Glu Met Ser Leu Leu Glu
   145 150 155 160
   Arg Ala Asp Gln Arg Phe Val Trp Asn Gly His Leu Leu Arg Glu Leu
   165 170 175
   Ser Ala Gln Pro Glu Val His Arg Phe Ala Leu Pro Val Leu His Gly
   180 185 190
   Phe Ile Thr Met His Ser Cys Ser Ile Asn Gly Lys Tyr Phe Asp Trp
   195 200 205
   Ile Leu Ile Ser Arg Arg Ser Cys Phe Arg Ala Gly Val Arg Tyr Tyr
   210 215 220
   Val Arg Gly Ile Asp Ser Glu Gly His Ala Ala Asn Phe Val Glu Thr
   225 230 235 240
   Glu Gln Ile Val His Tyr Asn Gly Ser Lys Ala Ser Phe Val Gln Thr
   245 250 255
   Arg Gly Ser Ile Pro Val Phe Trp Ser Gln Arg Pro Asn Leu Lys Tyr
   260 265 270
   Lys Pro Leu Pro Gln Ile Ser Lys Val Ala Asn His Met Asp Gly Phe
   275 280 285
   Gln Arg His Phe Asp Ser Gln Val Ile Ile Tyr Gly Lys Gln Val Ile
       290                 295                 300
   Ile Asn Leu Ile Asn Gln Lys Gly Ser Glu Lys Pro Leu Glu Gln Thr
   305                 310                 315                 320
   Phe Ala Thr Met Val Ser Ser Leu Gly Ser Gly Met Met Arg Tyr Ile
                   325                 330                 335
   Ala Phe Asp Phe His Lys Glu Cys Lys Asn Met Arg Trp Asp Arg Leu
               340                 345                 350
   Ser Ile Leu Leu Asp Gln Val Ala Glu Met Gln Asp Glu Leu Ser Tyr
           355                 360                 365
   Phe Leu Val Asp Ser Ala Gly Gln Val Val Ala Asn Gln Glu Gly Val
       370                 375                 380
   Phe Arg Ser Asn Cys Met Asp Cys Leu Asp Arg Thr Asn Val Ile Gln
   385                 390                 395                 400
   Ser Leu Leu Ala Arg Arg Ser Leu Gln Ala Gln Leu Gln Arg Leu Gly
                   405                 410                 415
   Val Leu His Val Gly Gln Lys Leu Glu Glu Gln Asp Glu Phe Glu Lys
               420                 425                 430
   Ile Tyr Lys Asn Ala Trp Ala Asp Asn Ala Asn Ala Cys Ala Lys Gln
           435                 440                 445
   Tyr Ala Gly Thr Gly Ala Leu Lys Thr Asp Phe Thr Arg Thr Gly Lys
       450                 455                 460
   Arg Thr His Leu Gly Leu Ile Met Asp Gly Trp Asn Ser Met Ile Arg
   465                 470                 475                 480
   Tyr Tyr Lys Asn Asn Phe Ser Asp Gly Phe Arg Gln Asp Ser Ile Asp
                   485                 490                 495
   Leu Phe Leu Gly Asn Tyr Ser Val Asp Glu Leu Glu Ser His Ser Pro
               500                 505                 510
   Leu Ser Val Pro Arg Asp Trp Lys Phe Leu Ala Leu Pro Ile Ile Met
           515                 520                 525
   Val Val Ala Phe Ser Met Cys Ile Ile Cys Leu Leu Met Ala Gly Asp
       530                 535                 540
   Thr Trp Thr Glu Thr Leu Ala Tyr Val Leu Phe Trp Gly Val Ala Ser
   545 550 555 560
   Ile Gly Thr Phe Phe Ile Ile Leu Tyr Asn Gly Lys Asp Phe Val Asp
   565 570 575
   Ala Pro Arg Leu Val Gln Lys Glu Lys Ile Asp
   580 585
<210> 15
   <211> 338
   <212> PRT
   <213> Homo Sapiens
<220>
   <221> MISC_FEATURE
   <222> (1)..(338)
   <223> OGTA088 = Swiss Prot Accession No. 015126, Secretory carrier-associated membrane protein 1
<400> 15
   Met Ser Asp Phe Asp Ser Asn Pro Phe Ala Asp Pro Asp Leu Asn Asn
   1 5 10 15
   Pro Phe Lys Asp Pro Ser Val Thr Gln Val Thr Arg Asn Val Pro Pro
   20 25 30
   Gly Leu Asp Glu Tyr Asn Pro Phe Ser Asp Ser Arg Thr Pro Pro Pro
   35 40 45
   Gly Gly Val Lys Met Pro Asn Val Pro Asn Thr Gln Pro Ala Ile Met
   50 55 60
   Lys Pro Thr Glu Glu His Pro Ala Tyr Thr Gln Ile Ala Lys Glu His
   65 70 75 80
   Ala Leu Ala Gln Ala Glu Leu Leu Lys Arg Gln Glu Glu Leu Glu Arg
   85 90 95
   Lys Ala Ala Glu Leu Asp Arg Arg Glu Arg Glu Met Gln Asn Leu Ser
   100 105 110
   Gln His Gly Arg Lys Asn Asn Trp Pro Pro Leu Pro Ser Asn Phe Pro
   115 120 125
   Val Gly Pro Cys Phe Tyr Gln Asp Phe Ser Val Asp Ile Pro Val Glu
   130 135 140
   Phe Gln Lys Thr Val Lys Leu Met Tyr Tyr Leu Trp Met Phe His Ala
   145 150 155 160
   Val Thr Leu Phe Leu Asn Ile Phe Gly Cys Leu Ala Trp Phe Cys Val
   165 170 175
   Asp Ser Ala Arg Ala Val Asp Phe Gly Leu Ser Ile Leu Trp Phe Leu
   180 185 190
   Leu Phe Thr Pro Cys Ser Phe Val Cys Trp Tyr Arg Pro Leu Tyr Gly
   195 200 205
   Ala Phe Arg Ser Asp Ser Ser Phe Arg Phe Phe Val Phe Phe Phe Val
   210 215 220
   Tyr Ile Cys Gln Phe Ala Val His Val Leu Gln Ala Ala Gly Phe His
   225 230 235 240
   Asn Trp Gly Asn Cys Gly Trp Ile Ser Ser Leu Thr Gly Leu Asn Gln
   245 250 255
   Asn Ile Pro Val Gly Ile Met Met Ile Ile Ile Ala Ala Leu Phe Thr
   260 265 270
   Ala Ser Ala Val Ile Ser Leu Val Met Phe Lys Lys Val His Gly Leu
   275 280 285
   Tyr Arg Thr Thr Gly Ala Ser Phe Glu Lys Ala Gln Gln Glu Phe Ala
   290 295 300
   Thr Gly Val Met Ser Asn Lys Thr Val Gln Thr Ala Ala Ala Asn Ala
   305 310 315 320
   Ala Ser Thr Ala Ala Ser Ser Ala Ala Gln Asn Ala Phe Lys Gly Asn
   325 330 335
   Gln Ile
<210> 16
   <211> 1058
   <212> PRT
   <213> Homo Sapiens
<220>
   <221> MISC_FEATURE
   <222> (1)..(1058)
   <223> OGTA089 = Swiss Prot Accession No. Q5T225, Polycystic kidney disease 1-like
<400> 16
   Met Glu Lys Arg Leu Gly Val Lys Pro Asn Pro Ala Ser Trp Ile Leu
   1               5                   10                  15
   Ser Gly Tyr Tyr Trp Gln Thr Ser Ala Lys Trp Leu Arg Ser Leu Tyr
               20                  25                  30
   Leu Phe Tyr Thr Cys Phe Cys Phe Ser Val Leu Trp Leu Ser Thr Asp
           35                  40                  45
   Ala Ser Glu Ser Arg Cys Gln Gln Gly Lys Thr Gln Phe Gly Val Gly
       50                  55                  60
   Leu Arg Ser Gly Gly Glu Asn His Leu Trp Leu Leu Glu Gly Thr Pro
   65                  70                  75                  80
   Ser Leu Gln Ser Cys Trp Ala Ala Cys Cys Gln Asp Ser Ala Cys His
                   85                  90                  95
   Val Phe Trp Trp Leu Glu Gly Met Cys Ile Gln Ala Asp Cys Ser Arg
               100                 105                 110
   Pro Gln Ser Cys Arg Ala Phe Arg Thr His Ser Ser Asn Ser Met Leu
           115                 120                 125
   Val Phe Leu Lys Lys Phe Gln Thr Ala Asp Asp Leu Gly Phe Leu Pro
       130                 135                 140
   Glu Asp Asp Val Pro His Leu Leu Gly Leu Gly Trp Asn Trp Ala Ser
   145                 150                 155                 160
   Trp Arg Gln Ser Pro Pro Arg Ala Ala Leu Arg Pro Ala Val Ser Ser
                   165                 170                 175
   Ser Asp Gln Gln Ser Leu Ile Arg Lys Leu Gln Lys Arg Gly Ser Pro
               180                 185                 190
   Ser Asp Val Val Thr Pro Ile Val Thr Gln His Ser Lys Val Asn Asp
           195                 200                 205
   Ser Asn Glu Leu Gly Gly Leu Thr Thr Ser Gly Ser Ala Glu Val His
       210                 215                 220
   Lys Ala Ile Thr Ile Ser Ser Pro Leu Thr Thr Asp Leu Thr Ala Glu
   225                 230                 235                 240
   Leu Ser Gly Gly Pro Lys Asn Val Ser Val Gln Pro Glu Ile Ser Glu
                   245                 250                 255
   Gly Leu Ala Thr Thr Pro Ser Thr Gln Gln Val Lys Ser Ser Glu Lys
               260                 265                 270
   Thr Gln Ile Ala Val Pro Gln Pro Val Ala Pro Ser Tyr Ser Tyr Ala
           275                 280                 285
   Thr Pro Thr Pro Gln Ala Ser Phe Gln Ser Thr Ser Ala Pro Tyr Pro
       290                 295                 300
   Val Ile Lys Glu Leu Val Val Ser Ala Gly Glu Ser Val Gln Ile Thr
   305                 310                 315                 320
   Leu Pro Lys Asn Glu Val Gln Leu Asn Ala Tyr Val Leu Gln Glu Pro
                   325                 330                 335
   Pro Lys Gly Glu Thr Tyr Thr Tyr Asp Trp Gln Leu Ile Thr His Pro
               340                 345                 350
   Arg Asp Tyr Ser Gly Glu Met Glu Gly Lys His Ser Gln Ile Leu Lys
           355                 360                 365
   Leu Ser Lys Leu Thr Pro Gly Leu Tyr Glu Phe Lys Val Ile Val Glu
       370                 375                 380
   Gly Gln Asn Ala His Gly Glu Gly Tyr Val Asn Val Thr Val Lys Pro
   385                 390                 395                 400
   Glu Pro Arg Lys Asn Arg Pro Pro Ile Ala Ile Val Ser Pro Gln Phe
                   405                 410                 415
   Gln Glu Ile Ser Leu Pro Thr Thr Ser Thr Val Ile Asp Gly Ser Gln
               420                 425                 430
   Ser Thr Asp Asp Asp Lys Ile Val Gln Tyr His Trp Glu Glu Leu Lys
           435                 440                 445
   Gly Pro Leu Arg Glu Glu Lys Ile Ser Glu Asp Thr Ala Ile Leu Lys
       450                 455                 460
   Leu Ser Lys Leu Val Pro Gly Asn Tyr Thr Phe Ser Leu Thr Val Val
   465                 470                 475                 480
   Asp Ser Asp Gly Ala Thr Asn Ser Thr Thr Ala Asn Leu Thr Val Asn
                   485                 490                 495
   Lys Ala Val Asp Tyr Pro Pro Val Ala Asn Ala Gly Pro Asn Gln Val
   500 505 510
   Ile Thr Leu Pro Gln Asn Ser Ile Thr Leu Phe Gly Asn Gln Ser Thr
   515 520 525
   Asp Asp His Gly Ile Thr Ser Tyr Glu Trp Ser Leu Ser Pro Ser Ser
   530 535 540
   Lys Gly Lys Val Val Glu Met Gln Gly Val Arg Thr Pro Thr Leu Gln
   545 550 555 560
   Leu Ser Ala Met Gln Glu Gly Asp Tyr Thr Tyr Gln Leu Thr Val Thr
   565 570 575
   Asp Thr Ile Gly Gln Gln Ala Thr Ala Gln Val Thr Val Ile Val Gln
   580 585 590
   Pro Glu Asn Asn Lys Pro Pro Gln Ala Asp Ala Gly Pro Asp Lys Glu
   595 600 605
   Leu Thr Leu Pro Val Asp Ser Thr Thr Leu Asp Gly Ser Lys Ser Ser
   610 615 620
   Asp Asp Gln Lys Ile Ile Ser Tyr Leu Trp Glu Lys Thr Gln Gly Pro
   625 630 635 640
   Asp Gly Val Gln Leu Glu Asn Ala Asn Ser Ser Val Ala Thr Val Thr
   645 650 655
   Gly Leu Gln Val Gly Thr Tyr Val Phe Thr Leu Thr Val Lys Asp Glu
   660 665 670
   Arg Asn Leu Gln Ser Gln Ser Ser Val Asn Val Ile Val Lys Glu Glu
   675 680 685
   Ile Asn Lys Pro Pro Ile Ala Lys Ile Thr Gly Asn Val Val Ile Thr
   690 695 700
   Leu Pro Thr Ser Thr Ala Glu Leu Asp Gly Ser Lys Ser Ser Asp Asp
   705 710 715 720
   Lys Gly Ile Val Ser Tyr Leu Trp Thr Arg Asp Glu Gly Ser Pro Ala
   725 730 735
   Ala Gly Glu Val Leu Asn His Ser Asp His His Pro Ile Leu Phe Leu
   740 745 750
   Ser Asn Leu Val Glu Gly Thr Tyr Thr Phe His Leu Lys Val Thr Asp
           755                 760                 765
   Ala Lys Gly Glu Ser Asp Thr Asp Arg Thr Thr Val Glu Val Lys Pro
       770                 775                 780
   Asp Pro Arg Lys Asn Asn Leu Val Glu Ile Ile Leu Asp Ile Asn Val
   785                 790                 795                 800
   Ser Gln Leu Thr Glu Arg Leu Lys Gly Met Phe Ile Arg Gln Ile Gly
                   805                 810                 815
   Val Leu Leu Gly Val Leu Asp Ser Asp Ile Ile Val Gln Lys Ile Gln
               820                 825                 830
   Pro Tyr Thr Glu Gln Ser Thr Lys Met Val Phe Phe Val Gln Asn Glu
           835                 840                 845
   Pro Pro His Gln Ile Phe Lys Gly His Glu Val Ala Ala Met Leu Lys
       850                 855                 860
   Ser Glu Leu Arg Lys Gln Lys Ala Asp Phe Leu Ile Phe Arg Ala Leu
   865                 870                 875                 880
   Glu Val Asn Thr Val Thr Cys Gln Leu Asn Cys Ser Asp His Gly His
                   885                 890                 895
   Cys Asp Ser Phe Thr Lys Arg Cys Ile Cys Asp Pro Phe Trp Met Glu
               900                 905                 910
   Asn Phe Ile Lys Val Gln Leu Arg Asp Gly Asp Ser Asn Cys Ala Leu
           915                 920                 925
   Thr Ile Leu Ile Phe Leu Ala Glu Trp Ser Val Leu Tyr Val Ile Ile
       930                 935                 940
   Ala Thr Phe Val Ile Val Val Ala Leu Gly Ile Leu Ser Trp Thr Val
   945                 950                 955                 960
   Ile Cys Cys Cys Lys Arg Gln Lys Gly Lys Pro Lys Arg Lys Ser Lys
                   965                 970                 975
   Tyr Lys Ile Leu Asp Ala Thr Asp Gln Glu Ser Leu Glu Leu Lys Pro
               980                 985                 990
   Thr Ser Arg Ala Gly Ile Lys Gln  Lys Gly Leu Leu Leu  Ser Ser Ser
   995 1000 1005
   Leu Met His Ser Glu Ser Glu Leu Asp Ser Asp Asp Ala Ile Phe
   1010 1015 1020
   Thr Trp Pro Asp Arg Glu Lys Gly Lys Leu Leu His Gly Gln Asn
   1025 1030 1035
   Gly Ser Val Pro Asn Gly Gln Thr Pro Leu Lys Ala Arg Ser Pro
   1040 1045 1050
   Arg Glu Glu Ile Leu
   1055
<210> 17
   <211> 2080
   <212> PRT
   <213> Homo Sapiens
<220>
   <221> MISC_FEATURE
   <222> (1)..(2080)
   <223> OGTA091 = Swiss Prot Accession No. O75923, Dysferlin
<400> 17
<210> 18
   <211> 1117
   <212> PRT
   <213> Homo Sapiens
<220>
   <221> MISC_FEATURE
   <222> (1)..(1117)
   <223> OGTA098 = Swiss Prot Accession No. Q14126, Desmoglein-2
<400> 18
   Met Ala Arg Thr Arg Asp Arg Val Arg Leu Leu Leu Leu Leu Ile Cys
   1 5 10 15
   Phe Asn Val Gly Ser Gly Leu His Leu Gln Val Leu Ser Thr Arg Asn
   20 25 30
   Glu Asn Lys Leu Leu Pro Lys His Pro His Leu Val Arg Gln Lys Arg
   35 40 45
   Ala Trp Ile Thr Ala Pro Val Ala Leu Arg Glu Gly Glu Asp Leu Ser
   50 55 60
   Lys Lys Asn Pro Ile Ala Lys Ile His Ser Asp Leu Ala Glu Glu Arg
   65 70 75 80
   Gly Leu Lys Ile Thr Tyr Lys Tyr Thr Gly Lys Gly Ile Thr Glu Pro
   85 90 95
   Pro Phe Gly Ile Phe Val Phe Asn Lys Asp Thr Gly Glu Leu Asn Val
   100 105 110
   Thr Ser Ile Leu Asp Arg Glu Glu Thr Pro Phe Phe Leu Leu Thr Gly
   115 120 125
   Tyr Ala Leu Asp Ala Arg Gly Asn Asn Val Glu Lys Pro Leu Glu Leu
   130 135 140
   Arg Ile Lys Val Leu Asp Ile Asn Asp Asn Glu Pro Val Phe Thr Gln
   145 150 155 160
   Asp Val Phe Val Gly Ser Val Glu Glu Leu Ser Ala Ala His Thr Leu
   165 170 175
   Val Met Lys Ile Asn Ala Thr Asp Ala Asp Glu Pro Asn Thr Leu Asn
   180 185 190
   Ser Lys Ile Ser Tyr Arg Ile Val Ser Leu Glu Pro Ala Tyr Pro Pro
   195 200 205
   Val Phe Tyr Leu Asn Lys Asp Thr Gly Glu Ile Tyr Thr Thr Ser Val
   210 215 220
   Thr Leu Asp Arg Glu Glu His Ser Ser Tyr Thr Leu Thr Val Glu Ala
   225 230 235 240
   Arg Asp Gly Asn Gly Glu Val Thr Asp Lys Pro Val Lys Gln Ala Gln
   245 250 255
   Val Gln Ile Arg Ile Leu Asp Val Asn Asp Asn Ile Pro Val Val Glu
   260 265 270
   Asn Lys Val Leu Glu Gly Met Val Glu Glu Asn Gln Val Asn Val Glu
   275 280 285
   Val Thr Arg Ile Lys Val Phe Asp Ala Asp Glu Ile Gly Ser Asp Asn
   290 295 300
   Trp Leu Ala Asn Phe Thr Phe Ala Ser Gly Asn Glu Gly Gly Tyr Phe
   305 310 315 320
   His Ile Glu Thr Asp Ala Gln Thr Asn Glu Gly Ile Val Thr Leu Ile
   325 330 335
   Lys Glu Val Asp Tyr Glu Glu Met Lys Asn Leu Asp Phe Ser Val Ile
   340 345 350
   Val Ala Asn Lys Ala Ala Phe His Lys Ser Ile Arg Ser Lys Tyr Lys
   355 360 365
   Pro Thr Pro Ile Pro Ile Lys Val Lys Val Lys Asn Val Lys Glu Gly
   370 375 380
   Ile His Phe Lys Ser Ser Val Ile Ser Ile Tyr Val Ser Glu Ser Met
   385 390 395 400
   Asp Arg Ser Ser Lys Gly Gln Ile Ile Gly Asn Phe Gln Ala Phe Asp
   405 410 415
   Glu Asp Thr Gly Leu Pro Ala His Ala Arg Tyr Val Lys Leu Glu Asp
   420 425 430
   Arg Asp Asn Trp Ile Ser Val Asp Ser Val Thr Ser Glu Ile Lys Leu
           435                 440                 445
   Ala Lys Leu Pro Asp Phe Glu Ser Arg Tyr Val Gln Asn Gly Thr Tyr
       450                 455                 460
   Thr Val Lys Ile Val Ala Ile Ser Glu Asp Tyr Pro Arg Lys Thr Ile
   465                 470                 475                 480
   Thr Gly Thr Val Leu Ile Asn Val Glu Asp Ile Asn Asp Asn Cys Pro
                   485                 490                 495
   Thr Leu Ile Glu Pro Val Gln Thr Ile Cys His Asp Ala Glu Tyr Val
               500                 505                 510
   Asn Val Thr Ala Glu Asp Leu Asp Gly His Pro Asn Ser Gly Pro Phe
           515                 520                 525
   Ser Phe Ser Val Ile Asp Lys Pro Pro Gly Met Ala Glu Lys Trp Lys
       530                 535                 540
   Ile Ala Arg Gln Glu Ser Thr Ser Val Leu Leu Gln Gln Ser Glu Lys
   545                 550                 555                 560
   Lys Leu Gly Arg Ser Glu Ile Gln Phe Leu Ile Ser Asp Asn Gln Gly
                   565                 570                 575
   Phe Ser Cys Pro Glu Lys Gln Val Leu Thr Leu Thr Val Cys Glu Val
               580                 585                 590
   Leu His Gly Ser Gly Cys Arg Glu Ala Gln His Asp Ser Tyr Val Gly
           595                 600                 605
   Leu Gly Pro Ala Ala Ile Ala Leu Met Ile Leu Ala Phe Leu Leu Leu
       610                 615                 620
   Leu Leu Val Pro Leu Leu Leu Leu Met Cys His Cys Gly Lys Gly Ala
   625                 630                 635                 640
   Lys Ala Phe Thr Pro Ile Pro Gly Thr Ile Glu Met Leu His Pro Trp
                   645                 650                 655
   Asn Asn Glu Gly Ala Pro Pro Glu Asp Lys Val Val Pro Ser Phe Leu
               660                 665                 670
   Pro Val Asp Gln Gly Gly Ser Leu Val Gly Arg Asn Gly Val Gly Gly
           675                 680                 685
   Met Ala Lys Glu Ala Thr Met Lys Gly Ser Ser Ser Ala Ser Ile Val
       690                 695                 700
   Lys Gly Gln His Glu Met Ser Glu Met Asp Gly Arg Trp Glu Glu His
   705                 710                 715                 720
   Arg Ser Leu Leu Ser Gly Arg Ala Thr Gln Phe Thr Gly Ala Thr Gly
                   725                 730                 735
   Ala Ile Met Thr Thr Glu Thr Thr Lys Thr Ala Arg Ala Thr Gly Ala
               740                 745                 750
   Ser Arg Asp Met Ala Gly Ala Gln Ala Ala Ala Val Ala Leu Asn Glu
           755                 760                 765
   Glu Phe Leu Arg Asn Tyr Phe Thr Asp Lys Ala Ala Ser Tyr Thr Glu
       770                 775                 780
   Glu Asp Glu Asn His Thr Ala Lys Asp Cys Leu Leu Val Tyr Ser Gln
   785                 790                 795                 800
   Glu Glu Thr Glu Ser Leu Asn Ala Ser Ile Gly Cys Cys Ser Phe Ile
                   805                 810                 815
   Glu Gly Glu Leu Asp Asp Arg Phe Leu Asp Asp Leu Gly Leu Lys Phe
               820                 825                 830
   Lys Thr Leu Ala Glu Val Cys Leu Gly Gln Lys Ile Asp Ile Asn Lys
           835                 840                 845
   Glu Ile Glu Gln Arg Gln Lys Pro Ala Thr Glu Thr Ser Met Asn Thr
       850                 855                 860
   Ala Ser His Ser Leu Cys Glu Gln Thr Met Val Asn Ser Glu Asn Thr
   865                 870                 875                 880
   Tyr Ser Ser Gly Ser Ser Phe Pro Val Pro Lys Ser Leu Gln Glu Ala
                   885                 890                 895
   Asn Ala Glu Lys Val Thr Gln Glu Ile Val Thr Glu Arg Ser Val Ser
               900                 905                 910
   Ser Arg Gln Ala Gln Lys Val Ala Thr Pro Leu Pro Asp Pro Met Ala
           915                 920                 925
   Ser Arg Asn Val Ile Ala Thr Glu Thr Ser Tyr Val Thr Gly Ser Thr
       930                 935                 940
   Met Pro Pro Thr Thr Val Ile Leu Gly Pro Ser Gln Pro Gln Ser Leu
   945 950 955 960
   Ile Val Thr Glu Arg Val Tyr Ala Pro Ala Ser Thr Leu Val Asp Gln
   965 970 975
   Pro Tyr Ala Asn Glu Gly Thr Val Val Val Thr Glu Arg Val Ile Gln
   980 985 990
   Pro His Gly Gly Gly Ser Asn Pro Leu Glu Gly Thr Gln His Leu Gln
   995 1000 1005
   Asp Val Pro Tyr Val Met Val Arg Glu Arg Glu Ser Phe Leu Ala
   1010 1015 1020
   Pro Ser Ser Gly Val Gln Pro Thr Leu Ala Met Pro Asn Ile Ala
   1025 1030 1035
   Val Gly Gln Asn Val Thr Val Thr Glu Arg Val Leu Ala Pro Ala
   1040 1045 1050
   Ser Thr Leu Gln Ser Ser Tyr Gln Ile Pro Thr Glu Asn Ser Met
   1055 1060 1065
   Thr Ala Arg Asn Thr Thr Val Ser Gly Ala Gly Val Pro Gly Pro
   1070 1075 1080
   Leu Pro Asp Phe Gly Leu Glu Glu Ser Gly His Ser Asn Ser Thr
   1085 1090 1095
   Ile Thr Thr Ser Ser Thr Arg Val Thr Lys His Ser Thr Val Gln
   1100 1105 1110
   His Ser Tyr Ser
   1115
<210> 19
   <211> 1912
   <212> PRT
   <213> Homo Sapiens
<220>
   <221> MISC_FEATURE
   <222> (1)..(1912)
   <223> OGTA101 = Swiss Prot Accession No. P23468, Receptor-type tyrosine-protein phosphatase delta
<400> 19
   Met Val His Val Ala Arg Leu Leu Leu Leu Leu Leu Thr Phe Phe Leu
   1               5                   10                  15
   Arg Thr Asp Ala Glu Thr Pro Pro Arg Phe Thr Arg Thr Pro Val Asp
               20                  25                  30
   Gln Thr Gly Val Ser Gly Gly Val Ala Ser Phe Ile Cys Gln Ala Thr
           35                  40                  45
   Gly Asp Pro Arg Pro Lys Ile Val Trp Asn Lys Lys Gly Lys Lys Val
       50                  55                  60
   Ser Asn Gln Arg Phe Glu Val Ile Glu Phe Asp Asp Gly Ser Gly Ser
   65                  70                  75                  80
   Val Leu Arg Ile Gln Pro Leu Arg Thr Pro Arg Asp Glu Ala Ile Tyr
                   85                  90                  95
   Glu Cys Val Ala Ser Asn Asn Val Gly Glu Ile Ser Val Ser Thr Arg
               100                 105                 110
   Leu Thr Val Leu Arg Glu Asp Gln Ile Pro Arg Gly Phe Pro Thr Ile
           115                 120                 125
   Asp Met Gly Pro Gln Leu Lys Val Val Glu Arg Thr Arg Thr Ala Thr
       130                 135                 140
   Met Leu Cys Ala Ala Ser Gly Asn Pro Asp Pro Glu Ile Thr Trp Phe
   145                 150                 155                 160
   Lys Asp Phe Leu Pro Val Asp Thr Ser Asn Asn Asn Gly Arg Ile Lys
                   165                 170                 175
   Gln Leu Arg Ser Glu Ser Ile Gly Gly Thr Pro Ile Arg Gly Ala Leu
               180                 185                 190
   Gln Ile Glu Gln Ser Glu Glu Ser Asp Gln Gly Lys Tyr Glu Cys Val
           195                 200                 205
   Ala Thr Asn Ser Ala Gly Thr Arg Tyr Ser Ala Pro Ala Asn Leu Tyr
       210                 215                 220
   Val Arg Glu Leu Arg Glu Val Arg Arg Val Pro Pro Arg Phe Ser Ile
   225                 230                 235                 240
   Pro Pro Thr Asn His Glu Ile Met Pro Gly Gly Ser Val Asn Ile Thr
                   245                 250                 255
   Cys Val Ala Val Gly Ser Pro Met Pro Tyr Val Lys Trp Met Leu Gly
               260                 265                 270
   Ala Glu Asp Leu Thr Pro Glu Asp Asp Met Pro Ile Gly Arg Asn Val
           275                 280                 285
   Leu Glu Leu Asn Asp Val Arg Gln Ser Ala Asn Tyr Thr Cys Val Ala
       290                 295                 300
   Met Ser Thr Leu Gly Val Ile Glu Ala Ile Ala Gln Ile Thr Val Lys
   305                 310                 315                 320
   Ala Leu Pro Lys Pro Pro Gly Thr Pro Val Val Thr Glu Ser Thr Ala
                   325                 330                 335
   Thr Ser Ile Thr Leu Thr Trp Asp Ser Gly Asn Pro Glu Pro Val Ser
               340                 345                 350
   Tyr Tyr Ile Ile Gln His Lys Pro Lys Asn Ser Glu Glu Leu Tyr Lys
           355                 360                 365
   Glu Ile Asp Gly Val Ala Thr Thr Arg Tyr Ser Val Ala Gly Leu Ser
       370                 375                 380
   Pro Tyr Ser Asp Tyr Glu Phe Arg Val Val Ala Val Asn Asn Ile Gly
   385                 390                 395                 400
   Arg Gly Pro Pro Ser Glu Pro Val Leu Thr Gln Thr Ser Glu Gln Ala
                   405                 410                 415
   Pro Ser Ser Ala Pro Arg Asp Val Gln Ala Arg Met Leu Ser Ser Thr
               420                 425                 430
   Thr Ile Leu Val Gln Trp Lys Glu Pro Glu Glu Pro Asn Gly Gln Ile
           435                 440                 445
   Gln Gly Tyr Arg Val Tyr Tyr Thr Met Asp Pro Thr Gln His Val Asn
       450                 455                 460
   Asn Trp Met Lys His Asn Val Ala Asp Ser Gln Ile Thr Thr Ile Gly
   465                 470                 475                 480
   Asn Leu Val Pro Gln Lys Thr Tyr Ser Val Lys Val Leu Ala Phe Thr
                   485                 490                 495
   Ser Ile Gly Asp Gly Pro Leu Ser Ser Asp Ile Gln Val Ile Thr Gln
   500 505 510
   Thr Gly Val Pro Gly Gln Pro Leu Asn Phe Lys Ala Glu Pro Glu Ser
   515 520 525
   Glu Thr Ser Ile Leu Leu Ser Trp Thr Pro Pro Arg Ser Asp Thr Ile
   530 535 540
   Ala Asn Tyr Glu Leu Val Tyr Lys Asp Gly Glu His Gly Glu Glu Gln
   545 550 555 560
   Arg Ile Thr Ile Glu Pro Gly Thr Ser Tyr Arg Leu Gln Gly Leu Lys
   565 570 575
   Pro Asn Ser Leu Tyr Tyr Phe Arg Leu Ala Ala Arg Ser Pro Gln Gly
   580 585 590
   Leu Gly Ala Ser Thr Ala Glu Ile Ser Ala Arg Thr Met Gln Ser Lys
   595 600 605
   Pro Ser Ala Pro Pro Gln Asp Ile Ser Cys Thr Ser Pro Ser Ser Thr
   610 615 620
   Ser Ile Leu Val Ser Trp Gln Pro Pro Pro Val Glu Lys Gln Asn Gly
   625 630 635 640
   Ile Ile Thr Glu Tyr Ser Ile Lys Tyr Thr Ala Val Asp Gly Glu Asp
   645 650 655
   Asp Lys Pro His Glu Ile Leu Gly Ile Pro Ser Asp Thr Thr Lys Tyr
   660 665 670
   Leu Leu Glu Gln Leu Glu Lys Trp Thr Glu Tyr Arg Ile Thr Val Thr
   675 680 685
   Ala His Thr Asp Val Gly Pro Gly Pro Glu Ser Leu Ser Val Leu Ile
   690 695 700
   Arg Thr Asn Glu Asp Val Pro Ser Gly Pro Pro Arg Lys Val Glu Val
   705 710 715 720
   Glu Ala Val Asn Ser Thr Ser Val Lys Val Ser Trp Arg Ser Pro Val
   725 730 735
   Pro Asn Lys Gln His Gly Gln Ile Arg Gly Tyr Gln Val His Tyr Val
   740 745 750
   Arg Met Glu Asn Gly Glu Pro Lys Gly Gln Pro Met Leu Lys Asp Val
           755                 760                 765
   Met Leu Ala Asp Ala Gln Trp Glu Phe Asp Asp Thr Thr Glu His Asp
       770                 775                 780
   Met Ile Ile Ser Gly Leu Gln Pro Glu Thr Ser Tyr Ser Leu Thr Val
   785                 790                 795                 800
   Thr Ala Tyr Thr Thr Lys Gly Asp Gly Ala Arg Ser Lys Pro Lys Leu
                   805                 810                 815
   Val Ser Thr Thr Gly Ala Val Pro Gly Lys Pro Arg Leu Val Ile Asn
               820                 825                 830
   His Thr Gln Met Asn Thr Ala Leu Ile Gln Trp His Pro Pro Val Asp
           835                 840                 845
   Thr Phe Gly Pro Leu Gln Gly Tyr Arg Leu Lys Phe Gly Arg Lys Asp
       850                 855                 860
   Met Glu Pro Leu Thr Thr Leu Glu Phe Ser Glu Lys Glu Asp His Phe
   865                 870                 875                 880
   Thr Ala Thr Asp Ile His Lys Gly Ala Ser Tyr Val Phe Arg Leu Ser
                   885                 890                 895
   Ala Arg Asn Lys Val Gly Phe Gly Glu Glu Met Val Lys Glu Ile Ser
               900                 905                 910
   Ile Pro Glu Glu Val Pro Thr Gly Phe Pro Gln Asn Leu His Ser Glu
           915                 920                 925
   Gly Thr Thr Ser Thr Ser Val Gln Leu Ser Trp Gln Pro Pro Val Leu
       930                 935                 940
   Ala Glu Arg Asn Gly Ile Ile Thr Lys Tyr Thr Leu Leu Tyr Arg Asp
   945                 950                 955                 960
   Ile Asn Ile Pro Leu Leu Pro Met Glu Gln Leu Ile Val Pro Ala Asp
                   965                 970                 975
   Thr Thr Met Thr Leu Thr Gly Leu Lys Pro Asp Thr Thr Tyr Asp Val
               980                 985                 990
   Lys Val Arg Ala His Thr Ser Lys  Gly Pro Gly Pro Tyr  Ser Pro Ser
           995                 1000                1005
   Val Gln Phe Arg Thr Leu Pro Val Asp Gln Val Phe Ala Lys Asn
   1010 1015 1020
   Phe His Val Lys Ala Val Met Lys Thr Ser Val Leu Leu Ser Trp
   1025 1030 1035
   Glu Ile Pro Glu Asn Tyr Asn Ser Ala Met Pro Phe Lys Ile Leu
   1040 1045 1050
   Tyr Asp Asp Gly Lys Met Val Glu Glu Val Asp Gly Arg Ala Thr
   1055 1060 1065
   Gln Lys Leu Ile Val Asn Leu Lys Pro Glu Lys Ser Tyr Ser Phe
   1070 1075 1080
   Val Leu Thr Asn Arg Gly Asn Ser Ala Gly Gly Leu Gln His Arg
   1085 1090 1095
   Val Thr Ala Lys Thr Ala Pro Asp Val Leu Arg Thr Lys Pro Ala
   1100 1105 1110
   Phe Ile Gly Lys Thr Asn Leu Asp Gly Met Ile Thr Val Gln Leu
   1115 1120 1125
   Pro Glu Val Pro Ala Asn Glu Asn Ile Lys Gly Tyr Tyr Ile Ile
   1130 1135 1140
   Ile Val Pro Leu Lys Lys Ser Arg Gly Lys Phe Ile Lys Pro Trp
   1145 1150 1155
   Glu Ser Pro Asp Glu Met Glu Leu Asp Glu Leu Leu Lys Glu Ile
   1160 1165 1170
   Ser Arg Lys Arg Arg Ser Ile Arg Tyr Gly Arg Glu Val Glu Leu
   1175 1180 1185
   Lys Pro Tyr Ile Ala Ala His Phe Asp Val Leu Pro Thr Glu Phe
   1190 1195 1200
   Thr Leu Gly Asp Asp Lys His Tyr Gly Gly Phe Thr Asn Lys Gln
   1205 1210 1215
   Leu Gln Ser Gly Gln Glu Tyr Val Phe Phe Val Leu Ala Val Met
   1220 1225 1230
   Glu His Ala Glu Ser Lys Met Tyr Ala Thr Ser Pro Tyr Ser Asp
   1235 1240 1245
   Pro Val Val Ser Met Asp Leu Asp Pro Gln Pro Ile Thr Asp Glu
   1250 1255 1260
   Glu Glu Gly Leu Ile Trp Val Val Gly Pro Val Leu Ala Val Val
   1265 1270 1275
   Phe Ile Ile Cys Ile Val Ile Ala Ile Leu Leu Tyr Lys Arg Lys
   1280 1285 1290
   Arg Ala Glu Ser Asp Ser Arg Lys Ser Ser Ile Pro Asn Asn Lys
   1295 1300 1305
   Glu Ile Pro Ser His His Pro Thr Asp Pro Val Glu Leu Arg Arg
   1310 1315 1320
   Leu Asn Phe Gln Thr Pro Gly Met Ala Ser His Pro Pro Ile Pro
   1325 1330 1335
   Ile Leu Glu Leu Ala Asp His Ile Glu Arg Leu Lys Ala Asn Asp
   1340 1345 1350
   Asn Leu Lys Phe Ser Gln Glu Tyr Glu Ser Ile Asp Pro Gly Gln
   1355 1360 1365
   Gln Phe Thr Trp Glu His Ser Asn Leu Glu Val Asn Lys Pro Lys
   1370 1375 1380
   Asn Arg Tyr Ala Asn Val Ile Ala Tyr Asp His Ser Arg Val Leu
   1385 1390 1395
   Leu Ser Ala Ile Glu Gly Ile Pro Gly Ser Asp Tyr Val Asn Ala
   1400 1405 1410
   Asn Tyr Ile Asp Gly Tyr Arg Lys Gln Asn Ala Tyr Ile Ala Thr
   1415 1420 1425
   Gln Gly Ser Leu Pro Glu Thr Phe Gly Asp Phe Trp Arg Met Ile
   1430 1435 1440
   Trp Glu Gln Arg Ser Ala Thr Val Val Met Met Thr Lys Leu Glu
   1445 1450 1455
   Glu Arg Ser Arg Val Lys Cys Asp Gln Tyr Trp Pro Ser Arg Gly
   1460 1465 1470
   Thr Glu Thr His Gly Leu Val Gln Val Thr Leu Leu Asp Thr Val
   1475 1480 1485
   Glu Leu Ala Thr Tyr Cys Val Arg Thr Phe Ala Leu Tyr Lys Asn
   1490 1495 1500
   Gly Ser Ser Glu Lys Arg Glu Val Arg Gln Phe Gln Phe Thr Ala
   1505 1510 1515
   Trp Pro Asp His Gly Val Pro Glu His Pro Thr Pro Phe Leu Ala
   1520 1525 1530
   Phe Leu Arg Arg Val Lys Thr Cys Asn Pro Pro Asp Ala Gly Pro
   1535 1540 1545
   Met Val Val His Cys Ser Ala Gly Val Gly Arg Thr Gly Cys Phe
   1550 1555 1560
   Ile Val Ile Asp Ala Met Leu Glu Arg Ile Lys His Glu Lys Thr
   1565 1570 1575
   Val Asp Ile Tyr Gly His Val Thr Leu Met Arg Ala Gln Arg Asn
   1580 1585 1590
   Tyr Met Val Gln Thr Glu Asp Gln Tyr Ile Phe Ile His Asp Ala
   1595 1600 1605
   Leu Leu Glu Ala Val Thr Cys Gly Asn Thr Glu Val Pro Ala Arg
   1610 1615 1620
   Asn Leu Tyr Ala Tyr Ile Gln Lys Leu Thr Gln Ile Glu Thr Gly
   1625 1630 1635
   Glu Asn Val Thr Gly Met Glu Leu Glu Phe Lys Arg Leu Ala Ser
   1640 1645 1650
   Ser Lys Ala His Thr Ser Arg Phe Ile Ser Ala Asn Leu Pro Cys
   1655 1660 1665
   Asn Lys Phe Lys Asn Arg Leu Val Asn Ile Met Pro Tyr Glu Ser
   1670 1675 1680
   Thr Arg Val Cys Leu Gln Pro Ile Arg Gly Val Glu Gly Ser Asp
   1685 1690 1695
   Tyr Ile Asn Ala Ser Phe Ile Asp Gly Tyr Arg Gln Gln Lys Ala
   1700 1705 1710
   Tyr Ile Ala Thr Gln Gly Pro Leu Ala Glu Thr Thr Glu Asp Phe
   1715 1720 1725
   Trp Arg Met Leu Trp Glu His Asn Ser Thr Ile Val Val Met Leu
   1730 1735 1740
   Thr Lys Leu Arg Glu Met Gly Arg Glu Lys Cys His Gln Tyr Trp
   1745 1750 1755
   Pro Ala Glu Arg Ser Ala Arg Tyr Gln Tyr Phe Val Val Asp Pro
   1760 1765 1770
   Met Ala Glu Tyr Asn Met Pro Gln Tyr Ile Leu Arg Glu Phe Lys
   1775 1780 1785
   Val Thr Asp Ala Arg Asp Gly Gln Ser Arg Thr Val Arg Gln Phe
   1790 1795 1800
   Gln Phe Thr Asp Trp Pro Glu Gln Gly Val Pro Lys Ser Gly Glu
   1805 1810 1815
   Gly Phe Ile Asp Phe Ile Gly Gln Val His Lys Thr Lys Glu Gln
   1820 1825 1830
   Phe Gly Gln Asp Gly Pro Ile Ser Val His Cys Ser Ala Gly Val
   1835 1840 1845
   Gly Arg Thr Gly Val Phe Ile Thr Leu Ser Ile Val Leu Glu Arg
   1850 1855 1860
   Met Arg Tyr Glu Gly Val Val Asp Ile Phe Gln Thr Val Lys Met
   1865 1870 1875
   Leu Arg Thr Gln Arg Pro Ala Met Val Gln Thr Glu Asp Gln Tyr
   1880 1885 1890
   Gln Phe Ser Tyr Arg Ala Ala Leu Glu Tyr Leu Gly Ser Phe Asp
   1895 1900 1905
   His Tyr Ala Thr
   1910
<210> 20
   <211> 819
   <212> PRT
   <213> Homo Sapiens
<220>
   <221> MISC_FEATURE
   <222> (1)..(819)
   <223> OGTA104 = Swiss Prot Accession No. Q13443, ADAM 9
<400> 20
   Met Gly Ser Gly Ala Arg Phe Pro Ser Gly Thr Leu Arg Val Arg Trp
   1               5                   10                  15
   Leu Leu Leu Leu Gly Leu Val Gly Pro Val Leu Gly Ala Ala Arg Pro
               20                  25                  30
   Gly Phe Gln Gln Thr Ser His Leu Ser Ser Tyr Glu Ile Ile Thr Pro
           35                  40                  45
   Trp Arg Leu Thr Arg Glu Arg Arg Glu Ala Pro Arg Pro Tyr Ser Lys
       50                  55                  60
   Gln Val Ser Tyr Val Ile Gln Ala Glu Gly Lys Glu His Ile Ile His
   65                  70                  75                  80
   Leu Glu Arg Asn Lys Asp Leu Leu Pro Glu Asp Phe Val Val Tyr Thr
                   85                  90                  95
   Tyr Asn Lys Glu Gly Thr Leu Ile Thr Asp His Pro Asn Ile Gln Asn
               100                 105                 110
   His Cys His Tyr Arg Gly Tyr Val Glu Gly Val His Asn Ser Ser Ile
           115                 120                 125
   Ala Leu Ser Asp Cys Phe Gly Leu Arg Gly Leu Leu His Leu Glu Asn
       130                 135                 140
   Ala Ser Tyr Gly Ile Glu Pro Leu Gln Asn Ser Ser His Phe Glu His
   145                 150                 155                 160
   Ile Ile Tyr Arg Met Asp Asp Val Tyr Lys Glu Pro Leu Lys Cys Gly
                   165                 170                 175
   Val Ser Asn Lys Asp Ile Glu Lys Glu Thr Ala Lys Asp Glu Glu Glu
               180                 185                 190
   Glu Pro Pro Ser Met Thr Gln Leu Leu Arg Arg Arg Arg Ala Val Leu
           195                 200                 205
   Pro Gln Thr Arg Tyr Val Glu Leu Phe Ile Val Val Asp Lys Glu Arg
       210                 215                 220
   Tyr Asp Met Met Gly Arg Asn Gln Thr Ala Val Arg Glu Glu Met Ile
   225                 230                 235                 240
   Leu Leu Ala Asn Tyr Leu Asp Ser Met Tyr Ile Met Leu Asn Ile Arg
                   245                 250                 255
   Ile Val Leu Val Gly Leu Glu Ile Trp Thr Asn Gly Asn Leu Ile Asn
               260                 265                 270
   Ile Val Gly Gly Ala Gly Asp Val Leu Gly Asn Phe Val Gln Trp Arg
           275                 280                 285
   Glu Lys Phe Leu Ile Thr Arg Arg Arg His Asp Ser Ala Gln Leu Val
       290                 295                 300
   Leu Lys Lys Gly Phe Gly Gly Thr Ala Gly Met Ala Phe Val Gly Thr
   305                 310                 315                 320
   Val Cys Ser Arg Ser His Ala Gly Gly Ile Asn Val Phe Gly Gln Ile
                   325                 330                 335
   Thr Val Glu Thr Phe Ala Ser Ile Val Ala His Glu Leu Gly His Asn
               340                 345                 350
   Leu Gly Met Asn His Asp Asp Gly Arg Asp Cys Ser Cys Gly Ala Lys
           355                 360                 365
   Ser Cys Ile Met Asn Ser Gly Ala Ser Gly Ser Arg Asn Phe Ser Ser
       370                 375                 380
   Cys Ser Ala Glu Asp Phe Glu Lys Leu Thr Leu Asn Lys Gly Gly Asn
   385                 390                 395                 400
   Cys Leu Leu Asn Ile Pro Lys Pro Asp Glu Ala Tyr Ser Ala Pro Ser
                   405                 410                 415
   Cys Gly Asn Lys Leu Val Asp Ala Gly Glu Glu Cys Asp Cys Gly Thr
               420                 425                 430
   Pro Lys Glu Cys Glu Leu Asp Pro Cys Cys Glu Gly Ser Thr Cys Lys
           435                 440                 445
   Leu Lys Ser Phe Ala Glu Cys Ala Tyr Gly Asp Cys Cys Lys Asp Cys
       450                 455                 460
   Arg Phe Leu Pro Gly Gly Thr Leu Cys Arg Gly Lys Thr Ser Glu Cys
   465                 470                 475                 480
   Asp Val Pro Glu Tyr Cys Asn Gly Ser Ser Gln Phe Cys Gln Pro Asp
   485 490 495
   Val Phe Ile Gln Asn Gly Tyr Pro Cys Gln Asn Asn Lys Ala Tyr Cys
   500 505 510
   Tyr Asn Gly Met Cys Gln Tyr Tyr Asp Ala Gln Cys Gln Val Ile Phe
   515 520 525
   Gly Ser Lys Ala Lys Ala Ala Pro Lys Asp Cys Phe Ile Glu Val Asn
   530 535 540
   Ser Lys Gly Asp Arg Phe Gly Asn Cys Gly Phe Ser Gly Asn Glu Tyr
   545 550 555 560
   Lys Lys Cys Ala Thr Gly Asn Ala Leu Cys Gly Lys Leu Gln Cys Glu
   565 570 575
   Asn Val Gln Glu Ile Pro Val Phe Gly Ile Val Pro Ala Ile Ile Gln
   580 585 590
   Thr Pro Ser Arg Gly Thr Lys Cys Trp Gly Val Asp Phe Gln Leu Gly
   595 600 605
   Ser Asp Val Pro Asp Pro Gly Met Val Asn Glu Gly Thr Lys Cys Gly
   610 615 620
   Ala Gly Lys Ile Cys Arg Asn Phe Gln Cys Val Asp Ala Ser Val Leu
   625 630 635 640
   Asn Tyr Asp Cys Asp Val Gln Lys Lys Cys His Gly His Gly Val Cys
   645 650 655
   Asn Ser Asn Lys Asn Cys His Cys Glu Asn Gly Trp Ala Pro Pro Asn
   660 665 670
   Cys Glu Thr Lys Gly Tyr Gly Gly Ser Val Asp Ser Gly Pro Thr Tyr
   675 680 685
   Asn Glu Met Asn Thr Ala Leu Arg Asp Gly Leu Leu Val Phe Phe Phe
   690 695 700
   Leu Ile Val Pro Leu Ile Val Cys Ala Ile Phe Ile Phe Ile Lys Arg
   705 710 715 720
   Asp Gln Leu Trp Arg Ser Tyr Phe Arg Lys Lys Arg Ser Gln Thr Tyr
   725 730 735
   Glu Ser Asp Gly Lys Asn Gln Ala Asn Pro Ser Arg Gln Pro Gly Ser
   740 745 750
   Val Pro Arg His Val Ser Pro Val Thr Pro Pro Arg Glu Val Pro Ile
   755 760 765
   Tyr Ala Asn Arg Phe Ala Val Pro Thr Tyr Ala Ala Lys Gln Pro Gln
   770 775 780
   Gln Phe Pro Ser Arg Pro Pro Pro Pro Gln Pro Lys Val Ser Ser Gln
   785 790 795 800
   Gly Asn Leu Ile Pro Ala Arg Pro Ala Pro Ala Pro Pro Leu Tyr Ser
   805 810 815
   Ser Leu Thr
<210> 21
   <211> 2003
   <212> PRT
   <213> Homo Sapiens
<220>
   <221> MISC_FEATURE
   <222> (1)..(2003)
   <223> OGTA106 = Swiss Prot Accession No. Q99466, Neurogenic locus notch homologue protein 4
<400> 21
   Met Gln Pro Pro Ser Leu Leu Leu Leu Leu Leu Leu Leu Leu Leu Leu
   1 5 10 15
   Cys Val Ser Val Val Arg Pro Arg Gly Leu Leu Cys Gly Ser Phe Pro
   20 25 30
   Glu Pro Cys Ala Asn Gly Gly Thr Cys Leu Ser Leu Ser Leu Gly Gln
   35 40 45
   Gly Thr Cys Gln Cys Ala Pro Gly Phe Leu Gly Glu Thr Cys Gln Phe
   50 55 60
   Pro Asp Pro Cys Gln Asn Ala Gln Leu Cys Gln Asn Gly Gly Ser Cys
   65 70 75 80
   Gln Ala Leu Leu Pro Ala Pro Leu Gly Leu Pro Ser Ser Pro Ser Pro
   85 90 95
   Leu Thr Pro Ser Phe Leu Cys Thr Cys Leu Pro Gly Phe Thr Gly Glu
               100                 105                 110
   Arg Cys Gln Ala Lys Leu Glu Asp Pro Cys Pro Pro Ser Phe Cys Ser
           115                 120                 125
   Lys Arg Gly Arg Cys His Ile Gln Ala Ser Gly Arg Pro Gln Cys Ser
       130                 135                 140
   Cys Met Pro Gly Trp Thr Gly Glu Gln Cys Gln Leu Arg Asp Phe Cys
   145                 150                 155                 160
   Ser Ala Asn Pro Cys Val Asn Gly Gly Val Cys Leu Ala Thr Tyr Pro
                   165                 170                 175
   Gln Ile Gln Cys His Cys Pro Pro Gly Phe Glu Gly His Ala Cys Glu
               180                 185                 190
   Arg Asp Val Asn Glu Cys Phe Gln Asp Pro Gly Pro Cys Pro Lys Gly
           195                 200                 205
   Thr Ser Cys His Asn Thr Leu Gly Ser Phe Gln Cys Leu Cys Pro Val
       210                 215                 220
   Gly Gln Glu Gly Pro Arg Cys Glu Leu Arg Ala Gly Pro Cys Pro Pro
   225                 230                 235                 240
   Arg Gly Cys Ser Asn Gly Gly Thr Cys Gln Leu Met Pro Glu Lys Asp
                   245                 250                 255
   Ser Thr Phe His Leu Cys Leu Cys Pro Pro Gly Phe Ile Gly Pro Asp
               260                 265                 270
   Cys Glu Val Asn Pro Asp Asn Cys Val Ser His Gln Cys Gln Asn Gly
           275                 280                 285
   Gly Thr Cys Gln Asp Gly Leu Asp Thr Tyr Thr Cys Leu Cys Pro Glu
       290                 295                 300
   Thr Trp Thr Gly Trp Asp Cys Ser Glu Asp Val Asp Glu Cys Glu Thr
   305                 310                 315                 320
   Gln Gly Pro Pro His Cys Arg Asn Gly Gly Thr Cys Gln Asn Ser Ala
                   325                 330                 335
   Gly Ser Phe His Cys Val Cys Val Ser Gly Trp Gly Gly Thr Ser Cys
               340                 345                 350
   Glu Glu Asn Leu Asp Asp Cys Ile Ala Ala Thr Cys Ala Pro Gly Ser
   355 360 365
   Thr Cys Ile Asp Arg Val Gly Ser Phe Ser Cys Leu Cys Pro Pro Gly
   370 375 380
   Arg Thr Gly Leu Leu Cys His Leu Glu Asp Met Cys Leu Ser Gln Pro
   385 390 395 400
   Cys His Gly Asp Ala Gln Cys Ser Thr Asn Pro Leu Thr Gly Ser Thr
   405 410 415
   Leu Cys Leu Cys Gln Pro Gly Tyr Ser Gly Pro Thr Cys His Gln Asp
   420 425 430
   Leu Asp Glu Cys Leu Met Ala Gln Gln Gly Pro Ser Pro Cys Glu His
   435 440 445
   Gly Gly Ser Cys Leu Asn Thr Pro Gly Ser Phe Asn Cys Leu Cys Pro
   450 455 460
   Pro Gly Tyr Thr Gly Ser Arg Cys Glu Ala Asp His Asn Glu Cys Leu
   465 470 475 480
   Ser Gln Pro Cys His Pro Gly Ser Thr Cys Leu Asp Leu Leu Ala Thr
   485 490 495
   Phe His Cys Leu Cys Pro Pro Gly Leu Glu Gly Gln Leu Cys Glu Val
   500 505 510
   Glu Thr Asn Glu Cys Ala Ser Ala Pro Cys Leu Asn His Ala Asp Cys
   515 520 525
   His Asp Leu Leu Asn Gly Phe Gln Cys Ile Cys Leu Pro Gly Phe Ser
   530 535 540
   Gly Thr Arg Cys Glu Glu Asp Ile Asp Glu Cys Arg Ser Ser Pro Cys
   545 550 555 560
   Ala Asn Gly Gly Gln Cys Gln Asp Gln Pro Gly Ala Phe His Cys Lys
   565 570 575
   Cys Leu Pro Gly Phe Glu Gly Pro Arg Cys Gln Thr Glu Val Asp Glu
   580 585 590
   Cys Leu Ser Asp Pro Cys Pro Val Gly Ala Ser Cys Leu Asp Leu Pro
   595 600 605
   Gly Ala Phe Phe Cys Leu Cys Pro Ser Gly Phe Thr Gly Gln Leu Cys
       610                 615                 620
   Glu Val Pro Leu Cys Ala Pro Asn Leu Cys Gln Pro Lys Gln Ile Cys
   625                 630                 635                 640
   Lys Asp Gln Lys Asp Lys Ala Asn Cys Leu Cys Pro Asp Gly Ser Pro
                   645                 650                 655
   Gly Cys Ala Pro Pro Glu Asp Asn Cys Thr Cys His His Gly His Cys
               660                 665                 670
   Gln Arg Ser Ser Cys Val Cys Asp Val Gly Trp Thr Gly Pro Glu Cys
           675                 680                 685
   Glu Ala Glu Leu Gly Gly Cys Ile Ser Ala Pro Cys Ala His Gly Gly
       690                 695                 700
   Thr Cys Tyr Pro Gln Pro Ser Gly Tyr Asn Cys Thr Cys Pro Thr Gly
   705                 710                 715                 720
   Tyr Thr Gly Pro Thr Cys Ser Glu Glu Met Thr Ala Cys His Ser Gly
                   725                 730                 735
   Pro Cys Leu Asn Gly Gly Ser Cys Asn Pro Ser Pro Gly Gly Tyr Tyr
               740                 745                 750
   Cys Thr Cys Pro Pro Ser His Thr Gly Pro Gln Cys Gln Thr Ser Thr
           755                 760                 765
   Asp Tyr Cys Val Ser Ala Pro Cys Phe Asn Gly Gly Thr Cys Val Asn
       770                 775                 780
   Arg Pro Gly Thr Phe Ser Cys Leu Cys Ala Met Gly Phe Gln Gly Pro
   785                 790                 795                 800
   Arg Cys Glu Gly Lys Leu Arg Pro Ser Cys Ala Asp Ser Pro Cys Arg
                   805                 810                 815
   Asn Arg Ala Thr Cys Gln Asp Ser Pro Gln Gly Pro Arg Cys Leu Cys
               820                 825                 830
   Pro Thr Gly Tyr Thr Gly Gly Ser Cys Gln Thr Leu Met Asp Leu Cys
           835                 840                 845
   Ala Gln Lys Pro Cys Pro Arg Asn Ser His Cys Leu Gln Thr Gly Pro
   850 855 860
   Ser Phe His Cys Leu Cys Leu Gln Gly Trp Thr Gly Pro Leu Cys Asn
   865 870 875 880
   Leu Pro Leu Ser Ser Cys Gln Lys Ala Ala Leu Ser Gln Gly Ile Asp
   885 890 895
   Val Ser Ser Leu Cys His Asn Gly Gly Leu Cys Val Asp Ser Gly Pro
   900 905 910
   Ser Tyr Phe Cys His Cys Pro Pro Gly Phe Gln Gly Ser Leu Cys Gln
   915 920 925
   Asp His Val Asn Pro Cys Glu Ser Arg Pro Cys Gln Asn Gly Ala Thr
   930 935 940
   Cys Met Ala Gln Pro Ser Gly Tyr Leu Cys Gln Cys Ala Pro Gly Tyr
   945 950 955 960
   Asp Gly Gln Asn Cys Ser Lys Glu Leu Asp Ala Cys Gln Ser Gln Pro
   965 970 975
   Cys His Asn His Gly Thr Cys Thr Pro Lys Pro Gly Gly Phe His Cys
   980 985 990
   Ala Cys Pro Pro Gly Phe Val Gly Leu Arg Cys Glu Gly Asp Val Asp
   995 1000 1005
   Glu Cys Leu Asp Gln Pro Cys His Pro Thr Gly Thr Ala Ala Cys
   1010 1015 1020
   His Ser Leu Ala Asn Ala Phe Tyr Cys Gln Cys Leu Pro Gly His
   1025 1030 1035
   Thr Gly Gln Trp Cys Glu Val Glu Ile Asp Pro Cys His Ser Gln
   1040 1045 1050
   Pro Cys Phe His Gly Gly Thr Cys Glu Ala Thr Ala Gly Ser Pro
   1055 1060 1065
   Leu Gly Phe Ile Cys His Cys Pro Lys Gly Phe Glu Gly Pro Thr
   1070 1075 1080
   Cys Ser His Arg Ala Pro Ser Cys Gly Phe His His Cys His His
   1085 1090 1095
   Gly Gly  Leu Cys Leu Pro Ser  Pro Lys Pro Gly Phe  Pro Pro Arg
       1100                 1105                 1110
   Cys Ala  Cys Leu Ser Gly Tyr  Gly Gly Pro Asp Cys  Leu Thr Pro
       1115                 1120                 1125
   Pro Ala  Pro Lys Gly Cys Gly  Pro Pro Ser Pro Cys  Leu Tyr Asn
       1130                 1135                 1140
   Gly Ser  Cys Ser Glu Thr Thr  Gly Leu Gly Gly Pro  Gly Phe Arg
       1145                 1150                 1155
   Cys Ser  Cys Pro His Ser Ser  Pro Gly Pro Arg Cys  Gln Lys Pro
       1160                 1165                 1170
   Gly Ala  Lys Gly Cys Glu Gly  Arg Ser Gly Asp Gly  Ala Cys Asp
       1175                 1180                 1185
   Ala Gly  Cys Ser Gly Pro Gly  Gly Asn Trp Asp Gly  Gly Asp Cys
       1190                 1195                 1200
   Ser Leu  Gly Val Pro Asp Pro  Trp Lys Gly Cys Pro  Ser His Ser
       1205                 1210                 1215
   Arg Cys  Trp Leu Leu Phe Arg  Asp Gly Gln Cys His  Pro Gln Cys
       1220                 1225                 1230
   Asp Ser  Glu Glu Cys Leu Phe  Asp Gly Tyr Asp Cys  Glu Thr Pro
       1235                 1240                 1245
   Pro Ala  Cys Thr Pro Ala Tyr  Asp Gln Tyr Cys His  Asp His Phe
       1250                 1255                 1260
   His Asn  Gly His Cys Glu Lys  Gly Cys Asn Thr Ala  Glu Cys Gly
       1265                 1270                 1275
   Trp Asp  Gly Gly Asp Cys Arg  Pro Glu Asp Gly Asp  Pro Glu Trp
       1280                 1285                 1290
   Gly Pro  Ser Leu Ala Leu Leu  Val Val Leu Ser Pro  Pro Ala Leu
       1295                 1300                 1305
   Asp Gln  Gln Leu Phe Ala Leu  Ala Arg Val Leu Ser  Leu Thr Leu
       1310                 1315                 1320
   Arg Val  Gly Leu Trp Val Arg  Lys Asp Arg Asp Gly  Arg Asp Met
       1325                 1330                 1335
   Val Tyr Pro Tyr Pro Gly Ala Arg Ala Glu Glu Lys Leu Gly Gly
   1340 1345 1350
   Thr Arg Asp Pro Thr Tyr Gln Glu Arg Ala Ala Pro Gln Thr Gln
   1355 1360 1365
   Pro Leu Gly Lys Glu Thr Asp Ser Leu Ser Ala Gly Phe Val Val
   1370 1375 1380
   Val Met Gly Val Asp Leu Ser Arg Cys Gly Pro Asp His Pro Ala
   1385 1390 1395
   Ser Arg Cys Pro Trp Asp Pro Gly Leu Leu Leu Arg Phe Leu Ala
   1400 1405 1410
   Ala Met Ala Ala Val Gly Ala Leu Glu Pro Leu Leu Pro Gly Pro
   1415 1420 1425
   Leu Leu Ala Val His Pro His Ala Gly Thr Ala Pro Pro Ala Asn
   1430 1435 1440
   Gln Leu Pro Trp Pro Val Leu Cys Ser Pro Val Ala Gly Val Ile
   1445 1450 1455
   Leu Leu Ala Leu Gly Ala Leu Leu Val Leu Gln Leu Ile Arg Arg
   1460 1465 1470
   Arg Arg Arg Glu His Gly Ala Leu Trp Leu Pro Pro Gly Phe Thr
   1475 1480 1485
   Arg Arg Pro Arg Thr Gln Ser Ala Pro His Arg Arg Arg Pro Pro
   1490 1495 1500
   Leu Gly Glu Asp Ser Ile Gly Leu Lys Ala Leu Lys Pro Lys Ala
   1505 1510 1515
   Glu Val Asp Glu Asp Gly Val Val Met Cys Ser Gly Pro Glu Glu
   1520 1525 1530
   Gly Glu Glu Val Gly Gln Ala Glu Glu Thr Gly Pro Pro Ser Thr
   1535 1540 1545
   Cys Gln Leu Trp Ser Leu Ser Gly Gly Cys Gly Ala Leu Pro Gln
   1550 1555 1560
   Ala Ala Met Leu Thr Pro Pro Gln Glu Ser Glu Met Glu Ala Pro
   1565 1570 1575
   Asp Leu Asp Thr Arg Gly Pro Asp Gly Val Thr Pro Leu Met Ser
       1580                1585                1590
   Ala Val Cys Cys Gly Glu Val Gln Ser Gly Thr Phe Gln Gly Ala
       1595                1600                1605
   Trp Leu Gly Cys Pro Glu Pro Trp Glu Pro Leu Leu Asp Gly Gly
       1610                1615                1620
   Ala Cys Pro Gln Ala His Thr Val Gly Thr Gly Glu Thr Pro Leu
       1625                1630                1635
   His Leu Ala Ala Arg Phe Ser Arg Pro Thr Ala Ala Arg Arg Leu
       1640                1645                1650
   Leu Glu Ala Gly Ala Asn Pro Asn Gln Pro Asp Arg Ala Gly Arg
       1655                1660                1665
   Thr Pro Leu His Ala Ala Val Ala Ala Asp Ala Arg Glu Val Cys
       1670                1675                1680
   Gln Leu Leu Leu Arg Ser Arg Gln Thr Ala Val Asp Ala Arg Thr
       1685                1690                1695
   Glu Asp Gly Thr Thr Pro Leu Met Leu Ala Ala Arg Leu Ala Val
       1700                1705                1710
   Glu Asp Leu Val Glu Glu Leu Ile Ala Ala Gln Ala Asp Val Gly
       1715                1720                1725
   Ala Arg Asp Lys Trp Gly Lys Thr Ala Leu His Trp Ala Ala Ala
       1730                1735                1740
   Val Asn Asn Ala Arg Ala Ala Arg Ser Leu Leu Gln Ala Gly Ala
       1745                1750                1755
   Asp Lys Asp Ala Gln Asp Asn Arg Glu Gln Thr Pro Leu Phe Leu
       1760                1765                1770
   Ala Ala Arg Glu Gly Ala Val Glu Val Ala Gln Leu Leu Leu Gly
       1775                1780                1785
   Leu Gly Ala Ala Arg Glu Leu Arg Asp Gln Ala Gly Leu Ala Pro
       1790                1795                1800
   Ala Asp Val Ala His Gln Arg Asn His Trp Asp Leu Leu Thr Leu
           1805                1810                1815
   Leu Glu  Gly Ala Gly Pro Pro  Glu Ala Arg His Lys  Ala Thr Pro
       1820                1825                1830
   Gly Arg  Glu Ala Gly Pro Phe  Pro Arg Ala Arg Thr  Val Ser Val
       1835                1840                1845
   Ser Val  Pro Pro His Gly Gly  Gly Ala Leu Pro Arg  Cys Arg Thr
       1850                1855                1860
   Leu Ser  Ala Gly Ala Gly Pro  Arg Gly Gly Gly Ala  Cys Leu Gln
       1865                1870                1875
   Ala Arg  Thr Trp Ser Val Asp  Leu Ala Ala Arg Gly  Gly Gly Ala
       1880                1885                1890
   Tyr Ser  His Cys Arg Ser Leu  Ser Gly Val Gly Ala  Gly Gly Gly
       1895                1900                1905
   Pro Thr  Pro Arg Gly Arg Arg  Phe Ser Ala Gly Met  Arg Gly Pro
       1910                1915                1920
   Arg Pro  Asn Pro Ala Ile Met  Arg Gly Arg Tyr Gly  Val Ala Ala
       1925                1930                1935
   Gly Arg  Gly Gly Arg Val Ser  Thr Asp Asp Trp Pro  Cys Asp Trp
       1940                1945                1950
   Val Ala  Leu Gly Ala Cys Gly  Ser Ala Ser Asn Ile  Pro Ile Pro
       1955                1960                1965
   Pro Pro  Cys Leu Thr Pro Ser  Pro Glu Arg Gly Ser  Pro Gln Leu
       1970                1975                1980
   Asp Cys  Gly Pro Pro Ala Leu  Gln Glu Met Pro Ile  Asn Gln Gly
       1985                1990                1995
   Gly Glu  Gly Lys Lys
       2000
<210> 22
   <211> 390
   <212> PRT
   <213> Homo Sapiens
<220>
   <221> MISC_FEATURE
   <222> (1)..(390)
   <223> OGTA112 = Swiss Prot Accession No. P48594, Serpin B4
<400> 22
   Met Asn Ser Leu Ser Glu Ala Asn Thr Lys Phe Met Phe Asp Leu Phe
   1               5                   10                  15
   Gln Gln Phe Arg Lys Ser Lys Glu Asn Asn Ile Phe Tyr Ser Pro Ile
               20                  25                  30
   Ser Ile Thr Ser Ala Leu Gly Met Val Leu Leu Gly Ala Lys Asp Asn
           35                  40                  45
   Thr Ala Gln Gln Ile Ser Lys Val Leu His Phe Asp Gln Val Thr Glu
       50                  55                  60
   Asn Thr Thr Glu Lys Ala Ala Thr Tyr His Val Asp Arg Ser Gly Asn
   65                  70                  75                  80
   Val His His Gln Phe Gln Lys Leu Leu Thr Glu Phe Asn Lys Ser Thr
                   85                  90                  95
   Asp Ala Tyr Glu Leu Lys Ile Ala Asn Lys Leu Phe Gly Glu Lys Thr
               100                 105                 110
   Tyr Gln Phe Leu Gln Glu Tyr Leu Asp Ala Ile Lys Lys Phe Tyr Gln
           115                 120                 125
   Thr Ser Val Glu Ser Thr Asp Phe Ala Asn Ala Pro Glu Glu Ser Arg
       130                 135                 140
   Lys Lys Ile Asn Ser Trp Val Glu Ser Gln Thr Asn Glu Lys Ile Lys
   145                 150                 155                 160
   Asn Leu Phe Pro Asp Gly Thr Ile Gly Asn Asp Thr Thr Leu Val Leu
                   165                 170                 175
   Val Asn Ala Ile Tyr Phe Lys Gly Gln Trp Glu Asn Lys Phe Lys Lys
               180                 185                 190
   Glu Asn Thr Lys Glu Glu Lys Phe Trp Pro Asn Lys Asn Thr Tyr Lys
           195                 200                 205
   Ser Val Gln Met Met Arg Gln Tyr Asn Ser Phe Asn Phe Ala Leu Leu
       210                 215                 220
   Glu Asp Val Gln Ala Lys Val Leu Glu Ile Pro Tyr Lys Gly Lys Asp
   225                 230                 235                 240
   Leu Ser Met Ile Val Leu Leu Pro Asn Glu Ile Asp Gly Leu Gln Lys
   245 250 255
   Leu Glu Glu Lys Leu Thr Ala Glu Lys Leu Met Glu Trp Thr Ser Leu
   260 265 270
   Gln Asn Met Arg Glu Thr Cys Val Asp Leu His Leu Pro Arg Phe Lys
   275 280 285
   Met Glu Glu Ser Tyr Asp Leu Lys Asp Thr Leu Arg Thr Met Gly Met
   290 295 300
   Val Asn Ile Phe Asn Gly Asp Ala Asp Leu Ser Gly Met Thr Trp Ser
   305 310 315 320
   His Gly Leu Ser Val Ser Lys Val Leu His Lys Ala Phe Val Glu Val
   325 330 335
   Thr Glu Glu Gly Val Glu Ala Ala Ala Ala Thr Ala Val Val Val Val
   340 345 350
   Glu Leu Ser Ser Pro Ser Thr Asn Glu Glu Phe Cys Cys Asn His Pro
   355 360 365
   Phe Leu Phe Phe Ile Arg Gln Asn Lys Thr Asn Ser Ile Leu Phe Tyr
   370 375 380
   Gly Arg Phe Ser Ser Pro
   385 390
<210> 23
   <211> 400
   <212> PRT
   <213> Homo Sapiens
<220>
   <221> MISC_FEATURE
   <222> (1)..(400)
   <223> OGTA113 = Swiss Prot Accession No. Q9BTV4, Transmembrane protein 43
<400> 23
<210> 24
   <211> 893
   <212> PRT
   <213> Homo Sapiens
<220>
   <221> MISC_FEATURE
   <222> (1)..(893)
   <223> OGTA119 = Swiss Prot Accession No. Q6UKI4, KIAA1228 protein
<400> 24
   Met Thr Pro Pro Ser Arg Ala Glu Ala Gly Val Arg Arg Ser Arg Val
   1 5 10 15
   Pro Ser Glu Gly Arg Trp Arg Gly Ala Glu Pro Pro Gly Ile Ser Ala
   20 25 30
   Ser Thr Gln Pro Ala Ser Ala Gly Arg Ala Ala Arg His Cys Gly Ala
   35 40 45
   Met Ser Gly Ala Arg Gly Glu Gly Pro Glu Ala Gly Ala Gly Gly Ala
   50 55 60
   Gly Gly Arg Ala Ala Pro Glu Asn Pro Gly Gly Val Leu Ser Val Glu
   65 70 75 80
   Leu Pro Gly Leu Leu Ala Gln Leu Ala Arg Ser Phe Ala Leu Leu Leu
                   85                  90                  95
   Pro Val Tyr Ala Leu Gly Tyr Leu Gly Leu Ser Phe Ser Trp Val Leu
               100                 105                 110
   Leu Ala Leu Ala Leu Leu Ala Trp Cys Arg Arg Ser Arg Gly Leu Lys
           115                 120                 125
   Ala Leu Arg Leu Cys Arg Ala Leu Ala Leu Leu Glu Asp Glu Glu Arg
       130                 135                 140
   Val Val Arg Leu Gly Val Arg Ala Cys Asp Leu Pro Ala Trp Val His
   145                 150                 155                 160
   Phe Pro Asp Thr Glu Arg Ala Glu Trp Leu Asn Lys Thr Val Lys His
                   165                 170                 175
   Met Trp Pro Phe Ile Cys Gln Phe Ile Glu Lys Leu Phe Arg Glu Thr
               180                 185                 190
   Ile Glu Pro Ala Val Arg Gly Ala Asn Thr His Leu Ser Thr Phe Ser
           195                 200                 205
   Phe Thr Lys Val Asp Val Gly Gln Gln Pro Leu Arg Ile Asn Gly Val
       210                 215                 220
   Lys Val Tyr Thr Glu Asn Val Asp Lys Arg Gln Ile Ile Leu Asp Leu
   225                 230                 235                 240
   Gln Ile Ser Phe Val Gly Asn Cys Glu Ile Asp Leu Glu Ile Lys Arg
                   245                 250                 255
   Tyr Phe Cys Arg Ala Gly Val Lys Ser Ile Gln Ile His Gly Thr Met
               260                 265                 270
   Arg Val Ile Leu Glu Pro Leu Ile Gly Asp Met Pro Leu Val Gly Ala
           275                 280                 285
   Leu Ser Ile Phe Phe Leu Arg Lys Pro Leu Leu Glu Ile Asn Trp Thr
       290                 295                 300
   Gly Leu Thr Asn Leu Leu Asp Val Pro Gly Leu Asn Gly Leu Ser Asp
   305                 310                 315                 320
   Thr Ile Ile Leu Asp Ile Ile Ser Asn Tyr Leu Val Leu Pro Asn Arg
                   325                 330                 335
   Ile Thr Val Pro Leu Val Ser Glu Val Gln Ile Ala Gln Leu Arg Phe
               340                 345                 350
   Pro Val Pro Lys Gly Val Leu Arg Ile His Phe Ile Glu Ala Gln Asp
           355                 360                 365
   Leu Gln Gly Lys Asp Thr Tyr Leu Lys Gly Leu Val Lys Gly Lys Ser
       370                 375                 380
   Asp Pro Tyr Gly Ile Ile Arg Val Gly Asn Gln Ile Phe Gln Ser Arg
   385                 390                 395                 400
   Val Ile Lys Glu Asn Leu Ser Pro Lys Trp Asn Glu Val Tyr Glu Ala
                   405                 410                 415
   Leu Val Tyr Glu His Pro Gly Gln Glu Leu Glu Ile Glu Leu Phe Asp
               420                 425                 430
   Glu Asp Pro Asp Lys Asp Asp Phe Leu Gly Ser Leu Met Ile Asp Leu
           435                 440                 445
   Ile Glu Val Glu Lys Glu Arg Leu Leu Asp Glu Trp Phe Thr Leu Asp
       450                 455                 460
   Glu Val Pro Lys Gly Lys Leu His Leu Arg Leu Glu Trp Leu Thr Leu
   465                 470                 475                 480
   Met Pro Asn Ala Ser Asn Leu Asp Lys Val Leu Thr Asp Ile Lys Ala
                   485                 490                 495
   Asp Lys Asp Gln Ala Asn Asp Gly Leu Ser Ser Ala Leu Leu Ile Leu
               500                 505                 510
   Tyr Leu Asp Ser Ala Arg Asn Leu Pro Ser Gly Lys Lys Ile Ser Ser
           515                 520                 525
   Asn Pro Asn Pro Val Val Gln Met Ser Val Gly His Lys Ala Gln Glu
       530                 535                 540
   Ser Lys Ile Arg Tyr Lys Thr Asn Glu Pro Val Trp Glu Glu Asn Phe
   545                 550                 555                 560
   Thr Phe Phe Ile His Asn Pro Lys Arg Gln Asp Leu Glu Val Glu Val
                   565                 570                 575
   Arg Asp Glu Gln His Gln Cys Ser Leu Gly Asn Leu Lys Val Pro Leu
               580                 585                 590
   Ser Gln Leu Leu Thr Ser Glu Asp Met Thr Val Ser Gln Arg Phe Gln
   595 600 605
   Leu Ser Asn Ser Gly Pro Asn Ser Thr Ile Lys Met Lys Ile Ala Leu
   610 615 620
   Arg Val Leu His Leu Glu Lys Arg Glu Arg Pro Pro Asp His Gln His
   625 630 635 640
   Ser Ala Gln Val Lys Arg Pro Ser Val Ser Lys Glu Gly Arg Lys Thr
   645 650 655
   Ser Ile Lys Ser His Met Ser Gly Ser Pro Gly Pro Gly Gly Ser Asn
   660 665 670
   Thr Ala Pro Ser Thr Pro Val Ile Gly Gly Ser Asp Lys Pro Gly Met
   675 680 685
   Glu Glu Lys Ala Gln Pro Pro Glu Ala Gly Pro Gln Gly Leu His Asp
   690 695 700
   Leu Gly Arg Ser Ser Ser Ser Leu Leu Ala Ser Pro Gly His Ile Ser
   705 710 715 720
   Val Lys Glu Pro Thr Pro Ser Ile Ala Ser Asp Ile Ser Leu Pro Ile
   725 730 735
   Ala Thr Gln Glu Leu Arg Gln Arg Leu Arg Gln Leu Glu Asn Gly Thr
   740 745 750
   Thr Leu Gly Gln Ser Pro Leu Gly Gln Ile Gln Leu Thr Ile Arg His
   755 760 765
   Ser Ser Gln Arg Asn Lys Leu Ile Val Val Val His Ala Cys Arg Asn
   770 775 780
   Leu Ile Ala Phe Ser Glu Asp Gly Ser Asp Pro Tyr Val Arg Met Tyr
   785 790 795 800
   Leu Leu Pro Asp Lys Arg Arg Ser Gly Arg Arg Lys Thr His Val Ser
   805 810 815
   Lys Lys Thr Leu Asn Pro Val Phe Asp Gln Ser Phe Asp Phe Ser Val
   820 825 830
   Ser Leu Pro Glu Val Gln Arg Arg Thr Leu Asp Val Ala Val Lys Asn
   835 840 845
   Ser Gly Gly Phe Leu Ser Lys Asp Lys Gly Leu Leu Gly Lys Val Leu
   850 855 860
   Val Ala Leu Ala Ser Glu Glu Leu Ala Lys Gly Trp Thr Gln Trp Tyr
   865 870 875 880
   Asp Leu Thr Glu Asp Gly Thr Arg Pro Gln Ala Met Thr
   885 890
<210> 25
   <211> 1085
   <212> PRT
   <213> Homo Sapiens
<220>
   <221> MISC_FEATURE
   <222> (1)..(1085)
   <223> OGTA124 = Swiss Prot Accession No. Q9UP95, Solute carrier family 12 member 4
<400> 25
   Met Pro His Phe Thr Val Val Pro Val Asp Gly Pro Arg Arg Gly Asp
   1 5 10 15
   Tyr Asp Asn Leu Glu Gly Leu Ser Trp Val Asp Tyr Gly Glu Arg Ala
   20 25 30
   Glu Leu Asp Asp Ser Asp Gly His Gly Asn His Arg Glu Ser Ser Pro
   35 40 45
   Phe Leu Ser Pro Leu Glu Ala Ser Arg Gly Ile Asp Tyr Tyr Asp Arg
   50 55 60
   Asn Leu Ala Leu Phe Glu Glu Glu Leu Asp Ile Arg Pro Lys Val Ser
   65 70 75 80
   Ser Leu Leu Gly Lys Leu Val Ser Tyr Thr Asn Leu Thr Gln Gly Ala
   85 90 95
   Lys Glu His Glu Glu Ala Glu Ser Gly Glu Gly Thr Arg Arg Arg Ala
   100 105 110
   Ala Glu Ala Pro Ser Met Gly Thr Leu Met Gly Val Tyr Leu Pro Cys
   115 120 125
   Leu Gln Asn Ile Phe Gly Val Ile Leu Phe Leu Arg Leu Thr Trp Met
   130 135 140
   Val Gly Thr Ala Gly Val Leu Gln Ala Leu Leu Ile Val Leu Ile Cys
   145 150 155 160
   Cys Cys Cys Thr Leu Leu Thr Ala Ile Ser Met Ser Ala Ile Ala Thr
   165 170 175
   Asn Gly Val Val Pro Ala Gly Gly Ser Tyr Phe Met Ile Ser Arg Ser
   180 185 190
   Leu Gly Pro Glu Phe Gly Gly Ala Val Gly Leu Cys Phe Tyr Leu Gly
   195 200 205
   Thr Thr Phe Ala Ala Ala Met Tyr Ile Leu Gly Ala Ile Glu Ile Leu
   210 215 220
   Leu Thr Tyr Ile Ala Pro Pro Ala Ala Ile Phe Tyr Pro Ser Gly Ala
   225 230 235 240
   His Asp Thr Ser Asn Ala Thr Leu Asn Asn Met Arg Val Tyr Gly Thr
   245 250 255
   Ile Phe Leu Thr Phe Met Thr Leu Val Val Phe Val Gly Val Lys Tyr
   260 265 270
   Val Asn Lys Phe Ala Ser Leu Phe Leu Ala Cys Val Ile Ile Ser Ile
   275 280 285
   Leu Ser Ile Tyr Ala Gly Gly Ile Lys Ser Ile Phe Asp Pro Pro Val
   290 295 300
   Phe Pro Val Cys Met Leu Gly Asn Arg Thr Leu Ser Arg Asp Gln Phe
   305 310 315 320
   Asp Ile Cys Ala Lys Thr Ala Val Val Asp Asn Glu Thr Val Ala Thr
   325 330 335
   Gln Leu Trp Ser Phe Phe Cys His Ser Pro Asn Leu Thr Thr Asp Ser
   340 345 350
   Cys Asp Pro Tyr Phe Met Leu Asn Asn Val Thr Glu Ile Pro Gly Ile
   355 360 365
   Pro Gly Ala Ala Ala Gly Val Leu Gln Glu Asn Leu Trp Ser Ala Tyr
   370 375 380
   Leu Glu Lys Gly Asp Ile Val Glu Lys His Gly Leu Pro Ser Ala Asp
   385 390 395 400
   Ala Pro Ser Leu Lys Glu Ser Leu Pro Leu Tyr Val Val Ala Asp Ile
   405 410 415
   Ala Thr Ser Phe Thr Val Leu Val Gly Ile Phe Phe Pro Ser Val Thr
   420 425 430
   Gly Ile Met Ala Gly Ser Asn Arg Ser Gly Asp Leu Arg Asp Ala Gln
   435 440 445
   Lys Ser Ile Pro Val Gly Thr Ile Leu Ala Ile Ile Thr Thr Ser Leu
   450 455 460
   Val Tyr Phe Ser Ser Val Val Leu Phe Gly Ala Cys Ile Glu Gly Val
   465 470 475 480
   Val Leu Arg Asp Lys Tyr Gly Asp Gly Val Ser Arg Asn Leu Val Val
   485 490 495
   Gly Thr Leu Ala Trp Pro Ser Pro Trp Val Ile Val Ile Gly Ser Phe
   500 505 510
   Phe Ser Thr Cys Gly Ala Gly Leu Gln Ser Leu Thr Gly Ala Pro Arg
   515 520 525
   Leu Leu Gln Ala Ile Ala Lys Asp Asn Ile Ile Pro Phe Leu Arg Val
   530 535 540
   Phe Gly His Gly Lys Val Asn Gly Glu Pro Thr Trp Ala Leu Leu Leu
   545 550 555 560
   Thr Ala Leu Ile Ala Glu Leu Gly Ile Leu Ile Ala Ser Leu Asp Met
   565 570 575
   Val Ala Pro Ile Leu Ser Met Phe Phe Leu Met Cys Tyr Leu Phe Val
   580 585 590
   Asn Leu Ala Cys Ala Val Gln Thr Leu Leu Arg Thr Pro Asn Trp Arg
   595 600 605
   Pro Arg Phe Lys Tyr Tyr His Trp Ala Leu Ser Phe Leu Gly Met Ser
   610 615 620
   Leu Cys Leu Ala Leu Met Phe Val Ser Ser Trp Tyr Tyr Ala Leu Val
   625 630 635 640
   Ala Met Leu Ile Ala Gly Met Ile Tyr Lys Tyr Ile Glu Tyr Gln Gly
                   645                 650                 655
   Ala Glu Lys Glu Trp Gly Asp Gly Ile Arg Gly Leu Ser Leu Ser Ala
               660                 665                 670
   Ala Arg Tyr Ala Leu Leu Arg Leu Glu Glu Gly Pro Pro His Thr Lys
           675                 680                 685
   Asn Trp Arg Pro Gln Leu Leu Val Leu Leu Lys Leu Asp Glu Asp Leu
       690                 695                 700
   His Val Lys Tyr Pro Arg Leu Leu Thr Phe Ala Ser Gln Leu Lys Ala
   705                 710                 715                 720
   Gly Lys Gly Leu Thr Ile Val Gly Ser Val Ile Gln Gly Ser Phe Leu
                   725                 730                 735
   Glu Ser Tyr Gly Glu Ala Gln Ala Ala Glu Gln Thr Ile Lys Asn Met
               740                 745                 750
   Met Glu Ile Glu Lys Val Lys Gly Phe Cys Gln Val Val Val Ala Ser
           755                 760                 765
   Lys Val Arg Glu Gly Leu Ala His Leu Ile Gln Ser Cys Gly Leu Gly
       770                 775                 780
   Gly Met Arg His Asn Ser Val Val Leu Gly Trp Pro Tyr Gly Trp Arg
   785                 790                 795                 800
   Gln Ser Glu Asp Pro Arg Ala Trp Lys Thr Phe Ile Asp Thr Val Arg
                   805                 810                 815
   Cys Thr Thr Ala Ala His Leu Ala Leu Leu Val Pro Lys Asn Ile Ala
               820                 825                 830
   Phe Tyr Pro Ser Asn His Glu Arg Tyr Leu Glu Gly His Ile Asp Val
           835                 840                 845
   Trp Trp Ile Val His Asp Gly Gly Met Leu Met Leu Leu Pro Phe Leu
       850                 855                 860
   Leu Arg Gln His Lys Val Trp Arg Lys Cys Arg Met Arg Ile Phe Thr
   865                 870                 875                 880
   Val Ala Gln Met Asp Asp Asn Ser Ile Gln Met Lys Lys Asp Leu Ala
                   885                 890                 895
   Val Phe Leu Tyr His Leu Arg Leu Glu Ala Glu Val Glu Val Val Glu
   900 905 910
   Met His Asn Ser Asp Ile Ser Ala Tyr Thr Tyr Glu Arg Thr Leu Met
   915 920 925
   Met Glu Gln Arg Ser Gln Met Leu Arg Gln Met Arg Leu Thr Lys Thr
   930 935 940
   Glu Arg Glu Arg Glu Ala Gln Leu Val Lys Asp Arg His Ser Ala Leu
   945 950 955 960
   Arg Leu Glu Ser Leu Tyr Ser Asp Glu Glu Asp Glu Ser Ala Val Gly
   965 970 975
   Ala Asp Lys Ile Gln Met Thr Trp Thr Arg Asp Lys Tyr Met Thr Glu
   980 985 990
   Thr Trp Asp Pro Ser His Ala Pro Asp Asn Phe Arg Glu Leu Val His
   995 1000 1005
   Ile Lys Pro Asp Gln Ser Asn Val Arg Arg Met His Thr Ala Val
   1010 1015 1020
   Lys Leu Asn Glu Val Ile Val Thr Arg Ser His Asp Ala Arg Leu
   1025 1030 1035
   Val Leu Leu Asn Met Pro Gly Pro Pro Arg Asn Ser Glu Gly Asp
   1040 1045 1050
   Glu Asn Tyr Met Glu Phe Leu Glu Val Leu Thr Glu Gly Leu Glu
   1055 1060 1065
   Arg Val Leu Leu Val Arg Gly Gly Gly Arg Glu Val Ile Thr Ile
   1070 1075 1080
   Tyr Ser
   1085
<210> 26
   <211> 836
   <212> PRT
   <213> Homo Sapiens
<220>
   <221> MISC_FEATURE
   <222> (1)..(836)
   <223> OGTA126 = Swiss Prot Accession No. Q9H5V8, CUB domain-containing protein 1
<400> 26
<210> 27
   <211> 1038
   <212> PRT
   <213> Homo Sapiens
<220>
   <221> MISC_FEATURE
   <222> (1)..(1038)
   <223> OGTA156 = Swiss Prot Accession No. P13612, Integrin alpha-4
<400> 27
   Met Phe Pro Thr Glu Ser Ala Trp Leu Gly Lys Arg Gly Ala Asn Pro
   1 5 10 15
   Gly Pro Glu Ala Ala Val Arg Glu Thr Val Met Leu Leu Leu Cys Leu
   20 25 30
   Gly Val Pro Thr Gly Arg Pro Tyr Asn Val Asp Thr Glu Ser Ala Leu
   35 40 45
   Leu Tyr Gln Gly Pro His Asn Thr Leu Phe Gly Tyr Ser Val Val Leu
   50 55 60
   His Ser His Gly Ala Asn Arg Trp Leu Leu Val Gly Ala Pro Thr Ala
   65 70 75 80
   Asn Trp Leu Ala Asn Ala Ser Val Ile Asn Pro Gly Ala Ile Tyr Arg
   85 90 95
   Cys Arg Ile Gly Lys Asn Pro Gly Gln Thr Cys Glu Gln Leu Gln Leu
   100 105 110
   Gly Ser Pro Asn Gly Glu Pro Cys Gly Lys Thr Cys Leu Glu Glu Arg
   115 120 125
   Asp Asn Gln Trp Leu Gly Val Thr Leu Ser Arg Gln Pro Gly Glu Asn
   130 135 140
   Gly Ser Ile Val Thr Cys Gly His Arg Trp Lys Asn Ile Phe Tyr Ile
   145 150 155 160
   Lys Asn Glu Asn Lys Leu Pro Thr Gly Gly Cys Tyr Gly Val Pro Pro
   165 170 175
   Asp Leu Arg Thr Glu Leu Ser Lys Arg Ile Ala Pro Cys Tyr Gln Asp
   180 185 190
   Tyr Val Lys Lys Phe Gly Glu Asn Phe Ala Ser Cys Gln Ala Gly Ile
   195 200 205
   Ser Ser Phe Tyr Thr Lys Asp Leu Ile Val Met Gly Ala Pro Gly Ser
   210 215 220
   Ser Tyr Trp Thr Gly Ser Leu Phe Val Tyr Asn Ile Thr Thr Asn Lys
   225 230 235 240
   Tyr Lys Ala Phe Leu Asp Lys Gln Asn Gln Val Lys Phe Gly Ser Tyr
   245 250 255
   Leu Gly Tyr Ser Val Gly Ala Gly His Phe Arg Ser Gln His Thr Thr
   260 265 270
   Glu Val Val Gly Gly Ala Pro Gln His Glu Gln Ile Gly Lys Ala Tyr
   275 280 285
   Ile Phe Ser Ile Asp Glu Lys Glu Leu Asn Ile Leu His Glu Met Lys
   290 295 300
   Gly Lys Lys Leu Gly Ser Tyr Phe Gly Ala Ser Val Cys Ala Val Asp
   305 310 315 320
   Leu Asn Ala Asp Gly Phe Ser Asp Leu Leu Val Gly Ala Pro Met Gln
   325 330 335
   Ser Thr Ile Arg Glu Glu Gly Arg Val Phe Val Tyr Ile Asn Ser Gly
   340 345 350
   Ser Gly Ala Val Met Asn Ala Met Glu Thr Asn Leu Val Gly Ser Asp
           355                 360                 365
   Lys Tyr Ala Ala Arg Phe Gly Glu Ser Ile Val Asn Leu Gly Asp Ile
       370                 375                 380
   Asp Asn Asp Gly Phe Glu Asp Val Ala Ile Gly Ala Pro Gln Glu Asp
   385                 390                 395                 400
   Asp Leu Gln Gly Ala Ile Tyr Ile Tyr Asn Gly Arg Ala Asp Gly Ile
                   405                 410                 415
   Ser Ser Thr Phe Ser Gln Arg Ile Glu Gly Leu Gln Ile Ser Lys Ser
               420                 425                 430
   Leu Ser Met Phe Gly Gln Ser Ile Ser Gly Gln Ile Asp Ala Asp Asn
           435                 440                 445
   Asn Gly Tyr Val Asp Val Ala Val Gly Ala Phe Arg Ser Asp Ser Ala
       450                 455                 460
   Val Leu Leu Arg Thr Arg Pro Val Val Ile Val Asp Ala Ser Leu Ser
   465                 470                 475                 480
   His Pro Glu Ser Val Asn Arg Thr Lys Phe Asp Cys Val Glu Asn Gly
                   485                 490                 495
   Trp Pro Ser Val Cys Ile Asp Leu Thr Leu Cys Phe Ser Tyr Lys Gly
               500                 505                 510
   Lys Glu Val Pro Gly Tyr Ile Val Leu Phe Tyr Asn Met Ser Leu Asp
           515                 520                 525
   Val Asn Arg Lys Ala Glu Ser Pro Pro Arg Phe Tyr Phe Ser Ser Asn
       530                 535                 540
   Gly Thr Ser Asp Val Ile Thr Gly Ser Ile Gln Val Ser Ser Arg Glu
   545                 550                 555                 560
   Ala Asn Cys Arg Thr His Gln Ala Phe Met Arg Lys Asp Val Arg Asp
                   565                 570                 575
   Ile Leu Thr Pro Ile Gln Ile Glu Ala Ala Tyr His Leu Gly Pro His
               580                 585                 590
   Val Ile Ser Lys Arg Ser Thr Glu Glu Phe Pro Pro Leu Gln Pro Ile
   595 600 605
   Leu Gln Gln Lys Lys Glu Lys Asp Ile Met Lys Lys Thr Ile Asn Phe
   610 615 620
   Ala Arg Phe Cys Ala His Glu Asn Cys Ser Ala Asp Leu Gln Val Ser
   625 630 635 640
   Ala Lys Ile Gly Phe Leu Lys Pro His Glu Asn Lys Thr Tyr Leu Ala
   645 650 655
   Val Gly Ser Met Lys Thr Leu Met Leu Asn Val Ser Leu Phe Asn Ala
   660 665 670
   Gly Asp Asp Ala Tyr Glu Thr Thr Leu His Val Lys Leu Pro Val Gly
   675 680 685
   Leu Tyr Phe Ile Lys Ile Leu Glu Leu Glu Glu Lys Gln Ile Asn Cys
   690 695 700
   Glu Val Thr Asp Asn Ser Gly Val Val Gln Leu Asp Cys Ser Ile Gly
   705 710 715 720
   Tyr Ile Tyr Val Asp His Leu Ser Arg Ile Asp Ile Ser Phe Leu Leu
   725 730 735
   Asp Val Ser Ser Leu Ser Arg Ala Glu Glu Asp Leu Ser Ile Thr Val
   740 745 750
   His Ala Thr Cys Glu Asn Glu Glu Glu Met Asp Asn Leu Lys His Ser
   755 760 765
   Arg Val Thr Val Ala Ile Pro Leu Lys Tyr Glu Val Lys Leu Thr Val
   770 775 780
   His Gly Phe Val Asn Pro Thr Ser Phe Val Tyr Gly Ser Asn Asp Glu
   785 790 795 800
   Asn Glu Pro Glu Thr Cys Met Val Glu Lys Met Asn Leu Thr Phe His
   805 810 815
   Val Ile Asn Thr Gly Asn Ser Met Ala Pro Asn Val Ser Val Glu Ile
   820 825 830
   Met Val Pro Asn Ser Phe Ser Pro Gln Thr Asp Lys Leu Phe Asn Ile
   835 840 845
   Leu Asp Val Gln Thr Thr Thr Gly Glu Cys His Phe Glu Asn Tyr Gln
   850 855 860
   Arg Val Cys Ala Leu Glu Gln Gln Lys Ser Ala Met Gln Thr Leu Lys
   865 870 875 880
   Gly Ile Val Arg Phe Leu Ser Lys Thr Asp Lys Arg Leu Leu Tyr Cys
   885 890 895
   Ile Lys Ala Asp Pro His Cys Leu Asn Phe Leu Cys Asn Phe Gly Lys
   900 905 910
   Met Glu Ser Gly Lys Glu Ala Ser Val His Ile Gln Leu Glu Gly Arg
   915 920 925
   Pro Ser Ile Leu Glu Met Asp Glu Thr Ser Ala Leu Lys Phe Glu Ile
   930 935 940
   Arg Ala Thr Gly Phe Pro Glu Pro Asn Pro Arg Val Ile Glu Leu Asn
   945 950 955 960
   Lys Asp Glu Asn Val Ala His Val Leu Leu Glu Gly Leu His His Gln
   965 970 975
   Arg Pro Lys Arg Tyr Phe Thr Ile Val Ile Ile Ser Ser Ser Leu Leu
   980 985 990
   Leu Gly Leu Ile Val Leu Leu Leu Ile Ser Tyr Val Met Trp Lys Ala
   995 1000 1005
   Gly Phe Phe Lys Arg Gln Tyr Lys Ser Ile Leu Gln Glu Glu Asn
   1010 1015 1020
   Arg Arg Asp Ser Trp Ser Tyr Ile Asn Ser Lys Ser Asn Asp Asp
   1025 1030 1035
<210> 28
   <211> 314
   <212> PRT
   <213> Homo Sapiens
<220>
   <221> MISC_FEATURE
   <222> (1)..(314)
   <223> OGTA159 = Swiss Prot Accession No. Q96HY6, Uncharacterized protein C20orf116
<400> 28
   Met Val Ala Pro Val Trp Tyr Leu Val Ala Ala Ala Leu Leu Val Gly
   1               5                   10                  15
   Phe Ile Leu Phe Leu Thr Arg Ser Arg Gly Arg Ala Ala Ser Ala Gly
               20                  25                  30
   Gln Glu Pro Leu His Asn Glu Glu Leu Ala Gly Ala Gly Arg Val Ala
           35                  40                  45
   Gln Pro Gly Pro Leu Glu Pro Glu Glu Pro Arg Ala Gly Gly Arg Pro
       50                  55                  60
   Arg Arg Arg Arg Asp Leu Gly Ser Arg Leu Gln Ala Gln Arg Arg Ala
   65                  70                  75                  80
   Gln Arg Val Ala Trp Ala Glu Ala Asp Glu Asn Glu Glu Glu Ala Val
                   85                  90                  95
   Ile Leu Ala Gln Glu Glu Glu Gly Val Glu Lys Pro Ala Glu Thr His
               100                 105                 110
   Leu Ser Gly Lys Ile Gly Ala Lys Lys Leu Arg Lys Leu Glu Glu Lys
           115                 120                 125
   Gln Ala Arg Lys Ala Gln Arg Glu Ala Glu Glu Ala Glu Arg Glu Glu
       130                 135                 140
   Arg Lys Arg Leu Glu Ser Gln Arg Glu Ala Glu Trp Lys Lys Glu Glu
   145                 150                 155                 160
   Glu Arg Leu Arg Leu Glu Glu Glu Gln Lys Glu Glu Glu Glu Arg Lys
                   165                 170                 175
   Ala Arg Glu Glu Gln Ala Gln Arg Glu His Glu Glu Tyr Leu Lys Leu
               180                 185                 190
   Lys Glu Ala Phe Val Val Glu Glu Glu Gly Val Gly Glu Thr Met Thr
           195                 200                 205
   Glu Glu Gln Ser Gln Ser Phe Leu Thr Glu Phe Ile Asn Tyr Ile Lys
       210                 215                 220
   Gln Ser Lys Val Val Leu Leu Glu Asp Leu Ala Ser Gln Val Gly Leu
   225                 230                 235                 240
   Arg Thr Gln Asp Thr Ile Asn Arg Ile Gln Asp Leu Leu Ala Glu Gly
                   245                 250                 255
   Thr Ile Thr Gly Val Ile Asp Asp Arg Gly Lys Phe Ile Tyr Ile Thr
               260                 265                 270
   Pro Glu Glu Leu Ala Ala Val Ala Asn Phe Ile Arg Gln Arg Gly Arg
           275                 280                 285
   Val Ser Ile Ala Glu Leu Ala Gln Ala Ser Asn Ser Leu Ile Ala Trp
       290                 295                 300
   Gly Arg Glu Ser Pro Ala Gln Ala Pro Ala
   305                 310
<210> 29
   <211> 742
   <212> PRT
   <213> Homo Sapiens
<220>
   <221> MISC_FEATURE
   <222> (1)..(742)
   <223> OGTA168 = Swiss Prot Accession No. Q8WZA4, Collectin placenta 1
<400> 29
   Met Lys Asp Asp Phe Ala Glu Glu Glu Glu Val Gln Ser Phe Gly Tyr
   1               5                   10                  15
   Lys Arg Phe Gly Ile Gln Glu Gly Thr Gln Cys Thr Lys Cys Lys Asn
               20                  25                  30
   Asn Trp Ala Leu Lys Phe Ser Ile Ile Leu Leu Tyr Ile Leu Cys Ala
           35                  40                  45
   Leu Leu Thr Ile Thr Val Ala Ile Leu Gly Tyr Lys Val Val Glu Lys
       50                  55                  60
   Met Asp Asn Val Thr Gly Gly Met Glu Thr Ser Arg Gln Thr Tyr Asp
   65                  70                  75                  80
   Asp Lys Leu Thr Ala Val Glu Ser Asp Leu Lys Lys Leu Gly Asp Gln
                   85                  90                  95
   Thr Gly Lys Lys Ala Ile Ser Thr Asn Ser Glu Leu Ser Thr Phe Arg
               100                 105                 110
   Ser Asp Ile Leu Asp Leu Arg Gln Gln Leu Arg Glu Ile Thr Glu Lys
           115                 120                 125
   Thr Ser Lys Asn Lys Asp Thr Leu Glu Lys Leu Gln Ala Ser Gly Asp
       130                 135                 140
   Ala Leu Val Asp Arg Gln Ser Gln Leu Lys Glu Thr Leu Glu Asn Asn
   145                 150                 155                 160
   Ser Phe Leu Ile Thr Thr Val Asn Lys Thr Leu Gln Ala Tyr Asn Gly
                   165                 170                 175
   Tyr Val Thr Asn Leu Gln Gln Asp Thr Ser Val Leu Gln Gly Asn Leu
               180                 185                 190
   Gln Asn Gln Met Tyr Ser His Asn Val Val Ile Met Asn Leu Asn Asn
           195                 200                 205
   Leu Asn Leu Thr Gln Val Gln Gln Arg Asn Leu Ile Thr Asn Leu Gln
       210                 215                 220
   Arg Ser Val Asp Asp Thr Ser Gln Ala Ile Gln Arg Ile Lys Asn Asp
   225                 230                 235                 240
   Phe Gln Asn Leu Gln Gln Val Phe Leu Gln Ala Lys Lys Asp Thr Asp
                   245                 250                 255
   Trp Leu Lys Glu Lys Val Gln Ser Leu Gln Thr Leu Ala Ala Asn Asn
               260                 265                 270
   Ser Ala Leu Ala Lys Ala Asn Asn Asp Thr Leu Glu Asp Met Asn Ser
           275                 280                 285
   Gln Leu Asn Ser Phe Thr Gly Gln Met Glu Asn Ile Thr Thr Ile Ser
       290                 295                 300
   Gln Ala Asn Glu Gln Asn Leu Lys Asp Leu Gln Asp Leu His Lys Asp
   305                 310                 315                 320
   Ala Glu Asn Arg Thr Ala Ile Lys Phe Asn Gln Leu Glu Glu Arg Phe
                   325                 330                 335
   Gln Leu Phe Glu Thr Asp Ile Val Asn Ile Ile Ser Asn Ile Ser Tyr
               340                 345                 350
   Thr Ala His His Leu Arg Thr Leu Thr Ser Asn Leu Asn Glu Val Arg
           355                 360                 365
   Thr Thr Cys Thr Asp Thr Leu Thr Lys His Thr Asp Asp Leu Thr Ser
       370                 375                 380
   Leu Asn Asn Thr Leu Ala Asn Ile Arg Leu Asp Ser Val Ser Leu Arg
   385                 390                 395                 400
   Met Gln Gln Asp Leu Met Arg Ser Arg Leu Asp Thr Glu Val Ala Asn
                   405                 410                 415
   Leu Ser Val Ile Met Glu Glu Met Lys Leu Val Asp Ser Lys His Gly
               420                 425                 430
   Gln Leu Ile Lys Asn Phe Thr Ile Leu Gln Gly Pro Pro Gly Pro Arg
           435                 440                 445
   Gly Pro Arg Gly Asp Arg Gly Ser Gln Gly Pro Pro Gly Pro Thr Gly
       450                 455                 460
   Asn Lys Gly Gln Lys Gly Glu Lys Gly Glu Pro Gly Pro Pro Gly Pro
   465                 470                 475                 480
   Ala Gly Glu Arg Gly Pro Ile Gly Pro Ala Gly Pro Pro Gly Glu Arg
                   485                 490                 495
   Gly Gly Lys Gly Ser Lys Gly Ser Gln Gly Pro Lys Gly Ser Arg Gly
               500                 505                 510
   Ser Pro Gly Lys Pro Gly Pro Gln Gly Ser Ser Gly Asp Pro Gly Pro
           515                 520                 525
   Pro Gly Pro Pro Gly Lys Glu Gly Leu Pro Gly Pro Gln Gly Pro Pro
       530                 535                 540
   Gly Phe Gln Gly Leu Gln Gly Thr Val Gly Glu Pro Gly Val Pro Gly
   545                 550                 555                 560
   Pro Arg Gly Leu Pro Gly Leu Pro Gly Val Pro Gly Met Pro Gly Pro
                   565                 570                 575
   Lys Gly Pro Pro Gly Pro Pro Gly Pro Ser Gly Ala Val Val Pro Leu
               580                 585                 590
   Ala Leu Gln Asn Glu Pro Thr Pro Ala Pro Glu Asp Asn Gly Cys Pro
           595                 600                 605
   Pro His Trp Lys Asn Phe Thr Asp Lys Cys Tyr Tyr Phe Ser Val Glu
       610                 615                 620
   Lys Glu Ile Phe Glu Asp Ala Lys Leu Phe Cys Glu Asp Lys Ser Ser
   625                 630                 635                 640
   His Leu Val Phe Ile Asn Thr Arg Glu Glu Gln Gln Trp Ile Lys Lys
   645 650 655
   Gln Met Val Gly Arg Glu Ser His Trp Ile Gly Leu Thr Asp Ser Glu
   660 665 670
   Arg Glu Asn Glu Trp Lys Trp Leu Asp Gly Thr Ser Pro Asp Tyr Lys
   675 680 685
   Asn Trp Lys Ala Gly Gln Pro Asp Asn Trp Gly His Gly His Gly Pro
   690 695 700
   Gly Glu Asp Cys Ala Gly Leu Ile Tyr Ala Gly Gln Trp Asn Asp Phe
   705 710 715 720
   Gln Cys Glu Asp Val Asn Asn Phe Ile Cys Glu Lys Asp Arg Glu Thr
   725 730 735
   Val Leu Ser Ser Ala Leu
   740
<210> 30
   <211> 1163
   <212> PRT
   <213> Homo Sapiens
<220>
   <221> MISC_FEATURE
   <222> (1)..(1163)
   <223> OGTA169 = Swiss Prot Accession No. P20702, Integrin alpha-X
<400> 30
   Met Thr Arg Thr Arg Ala Ala Leu Leu Leu Phe Thr Ala Leu Ala Thr
   1               5                   10                  15
   Ser Leu Gly Phe Asn Leu Asp Thr Glu Glu Leu Thr Ala Phe Arg Val
               20                  25                  30
   Asp Ser Ala Gly Phe Gly Asp Ser Val Val Gln Tyr Ala Asn Ser Trp
           35                  40                  45
   Val Val Val Gly Ala Pro Gln Lys Ile Thr Ala Ala Asn Gln Thr Gly
       50                  55                  60
   Gly Leu Tyr Gln Cys Gly Tyr Ser Thr Gly Ala Cys Glu Pro Ile Gly
   65                  70                  75                  80
   Leu Gln Val Pro Pro Glu Ala Val Asn Met Ser Leu Gly Leu Ser Leu
                   85                  90                  95
   Ala Ser Thr Thr Ser Pro Ser Gln Leu Leu Ala Cys Gly Pro Thr Val
               100                 105                 110
   His His Glu Cys Gly Arg Asn Met Tyr Leu Thr Gly Leu Cys Phe Leu
           115                 120                 125
   Leu Gly Pro Thr Gln Leu Thr Gln Arg Leu Pro Val Ser Arg Gln Glu
       130                 135                 140
   Cys Pro Arg Gln Glu Gln Asp Ile Val Phe Leu Ile Asp Gly Ser Gly
   145                 150                 155                 160
   Ser Ile Ser Ser Arg Asn Phe Ala Thr Met Met Asn Phe Val Arg Ala
                   165                 170                 175
   Val Ile Ser Gln Phe Gln Arg Pro Ser Thr Gln Phe Ser Leu Met Gln
               180                 185                 190
   Phe Ser Asn Lys Phe Gln Thr His Phe Thr Phe Glu Glu Phe Arg Arg
           195                 200                 205
   Thr Ser Asn Pro Leu Ser Leu Leu Ala Ser Val His Gln Leu Gln Gly
       210                 215                 220
   Phe Thr Tyr Thr Ala Thr Ala Ile Gln Asn Val Val His Arg Leu Phe
   225                 230                 235                 240
   His Ala Ser Tyr Gly Ala Arg Arg Asp Ala Thr Lys Ile Leu Ile Val
                   245                 250                 255
   Ile Thr Asp Gly Lys Lys Glu Gly Asp Ser Leu Asp Tyr Lys Asp Val
               260                 265                 270
   Ile Pro Met Ala Asp Ala Ala Gly Ile Ile Arg Tyr Ala Ile Gly Val
           275                 280                 285
   Gly Leu Ala Phe Gln Asn Arg Asn Ser Trp Lys Glu Leu Asn Asp Ile
       290                 295                 300
   Ala Ser Lys Pro Ser Gln Glu His Ile Phe Lys Val Glu Asp Phe Asp
   305                 310                 315                 320
   Ala Leu Lys Asp Ile Gln Asn Gln Leu Lys Glu Lys Ile Phe Ala Ile
                   325                 330                 335
   Glu Gly Thr Glu Thr Thr Ser Ser Ser Ser Phe Glu Leu Glu Met Ala
   340 345 350
   Gln Glu Gly Phe Ser Ala Val Phe Thr Pro Asp Gly Pro Val Leu Gly
   355 360 365
   Ala Val Gly Ser Phe Thr Trp Ser Gly Gly Ala Phe Leu Tyr Pro Pro
   370 375 380
   Asn Met Ser Pro Thr Phe Ile Asn Met Ser Gln Glu Asn Val Asp Met
   385 390 395 400
   Arg Asp Ser Tyr Leu Gly Tyr Ser Thr Glu Leu Ala Leu Trp Lys Gly
   405 410 415
   Val Gln Ser Leu Val Leu Gly Ala Pro Arg Tyr Gln His Thr Gly Lys
   420 425 430
   Ala Val Ile Phe Thr Gln Val Ser Arg Gln Trp Arg Met Lys Ala Glu
   435 440 445
   Val Thr Gly Thr Gln Ile Gly Ser Tyr Phe Gly Ala Ser Leu Cys Ser
   450 455 460
   Val Asp Val Asp Thr Asp Gly Ser Thr Asp Leu Val Leu Ile Gly Ala
   465 470 475 480
   Pro His Tyr Tyr Glu Gln Thr Arg Gly Gly Gln Val Ser Val Cys Pro
   485 490 495
   Leu Pro Arg Gly Trp Arg Arg Trp Trp Cys Asp Ala Val Leu Tyr Gly
   500 505 510
   Glu Gln Gly His Pro Trp Gly Arg Phe Gly Ala Ala Leu Thr Val Leu
   515 520 525
   Gly Asp Val Asn Gly Asp Lys Leu Thr Asp Val Val Ile Gly Ala Pro
   530 535 540
   Gly Glu Glu Glu Asn Arg Gly Ala Val Tyr Leu Phe His Gly Val Leu
   545 550 555 560
   Gly Pro Ser Ile Ser Pro Ser His Ser Gln Arg Ile Ala Gly Ser Gln
   565 570 575
   Leu Ser Ser Arg Leu Gln Tyr Phe Gly Gln Ala Leu Ser Gly Gly Gln
   580 585 590
   Asp Leu Thr Gln Asp Gly Leu Val Asp Leu Ala Val Gly Ala Arg Gly
   595 600 605
   Gln Val Leu Leu Leu Arg Thr Arg Pro Val Leu Trp Val Gly Val Ser
   610 615 620
   Met Gln Phe Ile Pro Ala Glu Ile Pro Arg Ser Ala Phe Glu Cys Arg
   625 630 635 640
   Glu Gln Val Val Ser Glu Gln Thr Leu Val Gln Ser Asn Ile Cys Leu
   645 650 655
   Tyr Ile Asp Lys Arg Ser Lys Asn Leu Leu Gly Ser Arg Asp Leu Gln
   660 665 670
   Ser Ser Val Thr Leu Asp Leu Ala Leu Asp Pro Gly Arg Leu Ser Pro
   675 680 685
   Arg Ala Thr Phe Gln Glu Thr Lys Asn Arg Ser Leu Ser Arg Val Arg
   690 695 700
   Val Leu Gly Leu Lys Ala His Cys Glu Asn Phe Asn Leu Leu Leu Pro
   705 710 715 720
   Ser Cys Val Glu Asp Ser Val Thr Pro Ile Thr Leu Arg Leu Asn Phe
   725 730 735
   Thr Leu Val Gly Lys Pro Leu Leu Ala Phe Arg Asn Leu Arg Pro Met
   740 745 750
   Leu Ala Ala Asp Ala Gln Arg Tyr Phe Thr Ala Ser Leu Pro Phe Glu
   755 760 765
   Lys Asn Cys Gly Ala Asp His Ile Cys Gln Asp Asn Leu Gly Ile Ser
   770 775 780
   Phe Ser Phe Pro Gly Leu Lys Ser Leu Leu Val Gly Ser Asn Leu Glu
   785 790 795 800
   Leu Asn Ala Glu Val Met Val Trp Asn Asp Gly Glu Asp Ser Tyr Gly
   805 810 815
   Thr Thr Ile Thr Phe Ser His Pro Ala Gly Leu Ser Tyr Arg Tyr Val
   820 825 830
   Ala Glu Gly Gln Lys Gln Gly Gln Leu Arg Ser Leu His Leu Thr Cys
   835 840 845
   Asp Ser Ala Pro Val Gly Ser Gln Gly Thr Trp Ser Thr Ser Cys Arg
       850                 855                 860
   Ile Asn His Leu Ile Phe Arg Gly Gly Ala Gln Ile Thr Phe Leu Ala
   865                 870                 875                 880
   Thr Phe Asp Val Ser Pro Lys Ala Val Leu Gly Asp Arg Leu Leu Leu
                   885                 890                 895
   Thr Ala Asn Val Ser Ser Glu Asn Asn Thr Pro Arg Thr Ser Lys Thr
               900                 905                 910
   Thr Phe Gln Leu Glu Leu Pro Val Lys Tyr Ala Val Tyr Thr Val Val
           915                 920                 925
   Ser Ser His Glu Gln Phe Thr Lys Tyr Leu Asn Phe Ser Glu Ser Glu
       930                 935                 940
   Glu Lys Glu Ser His Val Ala Met His Arg Tyr Gln Val Asn Asn Leu
   945                 950                 955                 960
   Gly Gln Arg Asp Leu Pro Val Ser Ile Asn Phe Trp Val Pro Val Glu
                   965                 970                 975
   Leu Asn Gln Glu Ala Val Trp Met Asp Val Glu Val Ser His Pro Gln
               980                 985                 990
   Asn Pro Ser Leu Arg Cys Ser Ser  Glu Lys Ile Ala Pro  Pro Ala Ser
           995                 1000                1005
   Asp Phe  Leu Ala His Ile Gln  Lys Asn Pro Val Leu  Asp Cys Ser
       1010                1015                1020
   Ile Ala  Gly Cys Leu Arg Phe  Arg Cys Asp Val Pro  Ser Phe Ser
       1025                1030                1035
   Val Gln  Glu Glu Leu Asp Phe  Thr Leu Lys Gly Asn  Leu Ser Phe
       1040                1045                1050
   Gly Trp  Val Arg Gln Ile Leu  Gln Lys Lys Val Ser  Val Val Ser
       1055                1060                1065
   Val Ala  Glu Ile Thr Phe Asp  Thr Ser Val Tyr Ser  Gln Leu Pro
       1070                1075                1080
   Gly Gln  Glu Ala Phe Met Arg  Ala Gln Thr Thr Thr  Val Leu Glu
       1085                1090                1095
   Lys Tyr Lys Val His Asn Pro Thr Pro Leu Ile Val Gly Ser Ser
   1100 1105 1110
   Ile Gly Gly Leu Leu Leu Leu Ala Leu Ile Thr Ala Val Leu Tyr
   1115 1120 1125
   Lys Val Gly Phe Phe Lys Arg Gln Tyr Lys Glu Met Met Glu Glu
   1130 1135 1140
   Ala Asn Gly Gln Ile Ala Pro Glu Asn Gly Thr Gln Thr Pro Ser
   1145 1150 1155
   Pro Pro Ser Glu Lys
   1160
<210> 31
   <211> 495
   <212> PRT
   <213> Homo Sapiens
<220>
   <221> MISC_FEATURE
   <222> (1)..(495)
   <223> OGTA174 = Swiss Prot Accession No. P06127, T-cell surface glycoprotein CD5
<400> 31
   Met Pro Met Gly Ser Leu Gln Pro Leu Ala Thr Leu Tyr Leu Leu Gly
   1 5 10 15
   Met Leu Val Ala Ser Cys Leu Gly Arg Leu Ser Trp Tyr Asp Pro Asp
   20 25 30
   Phe Gln Ala Arg Leu Thr Arg Ser Asn Ser Lys Cys Gln Gly Gln Leu
   35 40 45
   Glu Val Tyr Leu Lys Asp Gly Trp His Met Val Cys Ser Gln Ser Trp
   50 55 60
   Gly Arg Ser Ser Lys Gln Trp Glu Asp Pro Ser Gln Ala Ser Lys Val
   65 70 75 80
   Cys Gln Arg Leu Asn Cys Gly Val Pro Leu Ser Leu Gly Pro Phe Leu
   85 90 95
   Val Thr Tyr Thr Pro Gln Ser Ser Ile Ile Cys Tyr Gly Gln Leu Gly
   100 105 110
   Ser Phe Ser Asn Cys Ser His Ser Arg Asn Asp Met Cys His Ser Leu
   115 120 125
   Gly Leu Thr Cys Leu Glu Pro Gln Lys Thr Thr Pro Pro Thr Thr Arg
   130 135 140
   Pro Pro Pro Thr Thr Thr Pro Glu Pro Thr Ala Pro Pro Arg Leu Gln
   145 150 155 160
   Leu Val Ala Gln Ser Gly Gly Gln His Cys Ala Gly Val Val Glu Phe
   165 170 175
   Tyr Ser Gly Ser Leu Gly Gly Thr Ile Ser Tyr Glu Ala Gln Asp Lys
   180 185 190
   Thr Gln Asp Leu Glu Asn Phe Leu Cys Asn Asn Leu Gln Cys Gly Ser
   195 200 205
   Phe Leu Lys His Leu Pro Glu Thr Glu Ala Gly Arg Ala Gln Asp Pro
   210 215 220
   Gly Glu Pro Arg Glu His Gln Pro Leu Pro Ile Gln Trp Lys Ile Gln
   225 230 235 240
   Asn Ser Ser Cys Thr Ser Leu Glu His Cys Phe Arg Lys Ile Lys Pro
   245 250 255
   Gln Lys Ser Gly Arg Val Leu Ala Leu Leu Cys Ser Gly Phe Gln Pro
   260 265 270
   Lys Val Gln Ser Arg Leu Val Gly Gly Ser Ser Ile Cys Glu Gly Thr
   275 280 285
   Val Glu Val Arg Gln Gly Ala Gln Trp Ala Ala Leu Cys Asp Ser Ser
   290 295 300
   Ser Ala Arg Ser Ser Leu Arg Trp Glu Glu Val Cys Arg Glu Gln Gln
   305 310 315 320
   Cys Gly Ser Val Asn Ser Tyr Arg Val Leu Asp Ala Gly Asp Pro Thr
   325 330 335
   Ser Arg Gly Leu Phe Cys Pro His Gln Lys Leu Ser Gln Cys His Glu
   340 345 350
   Leu Trp Glu Arg Asn Ser Tyr Cys Lys Lys Val Phe Val Thr Cys Gln
   355 360 365
   Asp Pro Asn Pro Ala Gly Leu Ala Ala Gly Thr Val Ala Ser Ile Ile
       370                 375                 380
   Leu Ala Leu Val Leu Leu Val Val Leu Leu Val Val Cys Gly Pro Leu
   385                 390                 395                 400
   Ala Tyr Lys Lys Leu Val Lys Lys Phe Arg Gln Lys Lys Gln Arg Gln
                   405                 410                 415
   Trp Ile Gly Pro Thr Gly Met Asn Gln Asn Met Ser Phe His Arg Asn
               420                 425                 430
   His Thr Ala Thr Val Arg Ser His Ala Glu Asn Pro Thr Ala Ser His
           435                 440                 445
   Val Asp Asn Glu Tyr Ser Gln Pro Pro Arg Asn Ser Arg Leu Ser Ala
       450                 455                 460
   Tyr Pro Ala Leu Glu Gly Val Leu His Arg Ser Ser Met Gln Pro Asp
   465                 470                 475                 480
   Asn Ser Ser Asp Ser Asp Tyr Asp Leu His Gly Ala Gln Arg Leu
                   485                 490                 495
<210> 32
   <211> 359
   <212> PRT
   <213> Homo Sapiens
<220>
   <221> MISC_FEATURE
   <222> (1)..(359)
   <223> OGTA176 = Swiss Prot Accession No. P21854, B-cell differentiation antigen CD72 homolog
<400> 32
   Met Ala Glu Ala Ile Thr Tyr Ala Asp Leu Arg Phe Val Lys Ala Pro
   1               5                   10                  15
   Leu Lys Lys Ser Ile Ser Ser Arg Leu Gly Gln Asp Pro Gly Ala Asp
               20                  25                  30
   Asp Asp Gly Glu Ile Thr Tyr Glu Asn Val Gln Val Pro Ala Val Leu
           35                  40                  45
   Gly Val Pro Ser Ser Leu Ala Ser Ser Val Leu Gly Asp Lys Ala Ala
       50                  55                  60
   Val Lys Ser Glu Gln Pro Thr Ala Ser Trp Arg Ala Val Thr Ser Pro
   65                  70                  75                  80
   Ala Val Gly Arg Ile Leu Pro Cys Arg Thr Thr Cys Leu Arg Tyr Leu
                   85                  90                  95
   Leu Leu Gly Leu Leu Leu Thr Cys Leu Leu Leu Gly Val Thr Ala Ile
               100                 105                 110
   Cys Leu Gly Val Arg Tyr Leu Gln Val Ser Gln Gln Leu Gln Gln Thr
           115                 120                 125
   Asn Arg Val Leu Glu Val Thr Asn Ser Ser Leu Arg Gln Gln Leu Arg
       130                 135                 140
   Leu Lys Ile Thr Gln Leu Gly Gln Ser Ala Glu Asp Leu Gln Gly Ser
   145                 150                 155                 160
   Arg Arg Glu Leu Ala Gln Ser Gln Glu Ala Leu Gln Val Glu Gln Arg
                   165                 170                 175
   Ala His Gln Ala Ala Glu Gly Gln Leu Gln Ala Cys Gln Ala Asp Arg
               180                 185                 190
   Gln Lys Thr Lys Glu Thr Leu Gln Ser Glu Glu Gln Gln Arg Arg Ala
           195                 200                 205
   Leu Glu Gln Lys Leu Ser Asn Met Glu Asn Arg Leu Lys Pro Phe Phe
       210                 215                 220
   Thr Cys Gly Ser Ala Asp Thr Cys Cys Pro Ser Gly Trp Ile Met His
   225                 230                 235                 240
   Gln Lys Ser Cys Phe Tyr Ile Ser Leu Thr Ser Lys Asn Trp Gln Glu
                   245                 250                 255
   Ser Gln Lys Gln Cys Glu Thr Leu Ser Ser Lys Leu Ala Thr Phe Ser
               260                 265                 270
   Glu Ile Tyr Pro Gln Ser His Ser Tyr Tyr Phe Leu Asn Ser Leu Leu
           275                 280                 285
   Pro Asn Gly Gly Ser Gly Asn Ser Tyr Trp Thr Gly Leu Ser Ser Asn
       290                 295                 300
   Lys Asp Trp Lys Leu Thr Asp Asp Thr Gln Arg Thr Arg Thr Tyr Ala
   305                 310                 315                 320
   Gln Ser Ser Lys Cys Asn Lys Val His Lys Thr Trp Ser Trp Trp Thr
                   325                 330                 335
   Leu Glu Ser Glu Ser Cys Arg Ser Ser Leu Pro Tyr Ile Cys Glu Met
               340                 345                 350
   Thr Ala Phe Arg Phe Pro Asp
           355
<210> 33
   <211> 510
   <212> PRT
   <213> Homo Sapiens
<220>
   <221> MISC_FEATURE
   <222> (1)..(510)
   <223> OGTA177 = Swiss Prot Accession No. P49961, Ectonucleoside triphosphate diphosphohydrolase 1
<400> 33
   Met Glu Asp Thr Lys Glu Ser Asn Val Lys Thr Phe Cys Ser Lys Asn
   1               5                   10                  15
   Ile Leu Ala Ile Leu Gly Phe Ser Ser Ile Ile Ala Val Ile Ala Leu
               20                  25                  30
   Leu Ala Val Gly Leu Thr Gln Asn Lys Ala Leu Pro Glu Asn Val Lys
           35                  40                  45
   Tyr Gly Ile Val Leu Asp Ala Gly Ser Ser His Thr Ser Leu Tyr Ile
       50                  55                  60
   Tyr Lys Trp Pro Ala Glu Lys Glu Asn Asp Thr Gly Val Val His Gln
   65                  70                  75                  80
   Val Glu Glu Cys Arg Val Lys Gly Pro Gly Ile Ser Lys Phe Val Gln
                   85                  90                  95
   Lys Val Asn Glu Ile Gly Ile Tyr Leu Thr Asp Cys Met Glu Arg Ala
               100                 105                 110
   Arg Glu Val Ile Pro Arg Ser Gln His Gln Glu Thr Pro Val Tyr Leu
           115                 120                 125
   Gly Ala Thr Ala Gly Met Arg Leu Leu Arg Met Glu Ser Glu Glu Leu
       130                 135                 140
   Ala Asp Arg Val Leu Asp Val Val Glu Arg Ser Leu Ser Asn Tyr Pro
   145 150 155 160
   Phe Asp Phe Gln Gly Ala Arg Ile Ile Thr Gly Gln Glu Glu Gly Ala
   165 170 175
   Tyr Gly Trp Ile Thr Ile Asn Tyr Leu Leu Gly Lys Phe Ser Gln Lys
   180 185 190
   Thr Arg Trp Phe Ser Ile Val Pro Tyr Glu Thr Asn Asn Gln Glu Thr
   195 200 205
   Phe Gly Ala Leu Asp Leu Gly Gly Ala Ser Thr Gln Val Thr Phe Val
   210 215 220
   Pro Gln Asn Gln Thr Ile Glu Ser Pro Asp Asn Ala Leu Gln Phe Arg
   225 230 235 240
   Leu Tyr Gly Lys Asp Tyr Asn Val Tyr Thr His Ser Phe Leu Cys Tyr
   245 250 255
   Gly Lys Asp Gln Ala Leu Trp Gln Lys Leu Ala Lys Asp Ile Gln Val
   260 265 270
   Ala Ser Asn Glu Ile Leu Arg Asp Pro Cys Phe His Pro Gly Tyr Lys
   275 280 285
   Lys Val Val Asn Val Ser Asp Leu Tyr Lys Thr Pro Cys Thr Lys Arg
   290 295 300
   Phe Glu Met Thr Leu Pro Phe Gln Gln Phe Glu Ile Gln Gly Ile Gly
   305 310 315 320
   Asn Tyr Gln Gln Cys His Gln Ser Ile Leu Glu Leu Phe Asn Thr Ser
   325 330 335
   Tyr Cys Pro Tyr Ser Gln Cys Ala Phe Asn Gly Ile Phe Leu Pro Pro
   340 345 350
   Leu Gln Gly Asp Phe Gly Ala Phe Ser Ala Phe Tyr Phe Val Met Lys
   355 360 365
   Phe Leu Asn Leu Thr Ser Glu Lys Val Ser Gln Glu Lys Val Thr Glu
   370 375 380
   Met Met Lys Lys Phe Cys Ala Gln Pro Trp Glu Glu Ile Lys Thr Ser
   385 390 395 400
   Tyr Ala Gly Val Lys Glu Lys Tyr Leu Ser Glu Tyr Cys Phe Ser Gly
   405 410 415
   Thr Tyr Ile Leu Ser Leu Leu Leu Gln Gly Tyr His Phe Thr Ala Asp
   420 425 430
   Ser Trp Glu His Ile His Phe Ile Gly Lys Ile Gln Gly Ser Asp Ala
   435 440 445
   Gly Trp Thr Leu Gly Tyr Met Leu Asn Leu Thr Asn Met Ile Pro Ala
   450 455 460
   Glu Gln Pro Leu Ser Thr Pro Leu Ser His Ser Thr Tyr Val Phe Leu
   465 470 475 480
   Met Val Leu Phe Ser Leu Val Leu Phe Thr Val Ala Ile Ile Gly Leu
   485 490 495
   Leu Ile Phe His Lys Pro Ser Tyr Phe Trp Lys Asp Met Val
   500 505 510
<210> 34
   <211> 976
   <212> PRT
   <213> Homo Sapiens
<220>
   <221> MISC_FEATURE
   <222> (1)..(976)
   <223> OGTA197 = Swiss Prot Accession No. P29317, Ephrin type-A receptor 2
<400> 34
   Met Glu Leu Gln Ala Ala Arg Ala Cys Phe Ala Leu Leu Trp Gly Cys
   1 5 10 15
   Ala Leu Ala Ala Ala Ala Ala Ala Gln Gly Lys Glu Val Val Leu Leu
   20 25 30
   Asp Phe Ala Ala Ala Gly Gly Glu Leu Gly Trp Leu Thr His Pro Tyr
   35 40 45
   Gly Lys Gly Trp Asp Leu Met Gln Asn Ile Met Asn Asp Met Pro Ile
   50 55 60
   Tyr Met Tyr Ser Val Cys Asn Val Met Ser Gly Asp Gln Asp Asn Trp
   65 70 75 80
   Leu Arg Thr Asn Trp Val Tyr Arg Gly Glu Ala Glu Arg Asn Asn Phe
   85 90 95
   Glu Leu Asn Phe Thr Val Arg Asp Cys Asn Ser Phe Pro Gly Gly Ala
   100 105 110
   Ser Ser Cys Lys Glu Thr Phe Asn Leu Tyr Tyr Ala Glu Ser Asp Leu
   115 120 125
   Asp Tyr Gly Thr Asn Phe Gln Lys Arg Leu Phe Thr Lys Ile Asp Thr
   130 135 140
   Ile Ala Pro Asp Glu Ile Thr Val Ser Ser Asp Phe Glu Ala Arg His
   145 150 155 160
   Val Lys Leu Asn Val Glu Glu Arg Ser Val Gly Pro Leu Thr Arg Lys
   165 170 175
   Gly Phe Tyr Leu Ala Phe Gln Asp Ile Gly Ala Cys Val Ala Leu Leu
   180 185 190
   Ser Val Arg Val Tyr Tyr Lys Lys Cys Pro Glu Leu Leu Gln Gly Leu
   195 200 205
   Ala His Phe Pro Glu Thr Ile Ala Gly Ser Asp Ala Pro Ser Leu Ala
   210 215 220
   Thr Val Ala Gly Thr Cys Val Asp His Ala Val Val Pro Pro Gly Gly
   225 230 235 240
   Glu Glu Pro Arg Met His Cys Ala Val Asp Gly Glu Trp Leu Val Pro
   245 250 255
   Ile Gly Gln Cys Leu Cys Gln Ala Gly Tyr Glu Lys Val Glu Asp Ala
   260 265 270
   Cys Gln Ala Cys Ser Pro Gly Phe Phe Lys Phe Glu Ala Ser Glu Ser
   275 280 285
   Pro Cys Leu Glu Cys Pro Glu His Thr Leu Pro Ser Pro Glu Gly Ala
   290 295 300
   Thr Ser Cys Glu Cys Glu Glu Gly Phe Phe Arg Ala Pro Gln Asp Pro
   305 310 315 320
   Ala Ser Met Pro Cys Thr Arg Pro Pro Ser Ala Pro His Tyr Leu Thr
   325 330 335
   Ala Val Gly Met Gly Ala Lys Val Glu Leu Arg Trp Thr Pro Pro Gln
   340 345 350
   Asp Ser Gly Gly Arg Glu Asp Ile Val Tyr Ser Val Thr Cys Glu Gln
   355 360 365
   Cys Trp Pro Glu Ser Gly Glu Cys Gly Pro Cys Glu Ala Ser Val Arg
   370 375 380
   Tyr Ser Glu Pro Pro His Gly Leu Thr Arg Thr Ser Val Thr Val Ser
   385 390 395 400
   Asp Leu Glu Pro His Met Asn Tyr Thr Phe Thr Val Glu Ala Arg Asn
   405 410 415
   Gly Val Ser Gly Leu Val Thr Ser Arg Ser Phe Arg Thr Ala Ser Val
   420 425 430
   Ser Ile Asn Gln Thr Glu Pro Pro Lys Val Arg Leu Glu Gly Arg Ser
   435 440 445
   Thr Thr Ser Leu Ser Val Ser Trp Ser Ile Pro Pro Pro Gln Gln Ser
   450 455 460
   Arg Val Trp Lys Tyr Glu Val Thr Tyr Arg Lys Lys Gly Asp Ser Asn
   465 470 475 480
   Ser Tyr Asn Val Arg Arg Thr Glu Gly Phe Ser Val Thr Leu Asp Asp
   485 490 495
   Leu Ala Pro Asp Thr Thr Tyr Leu Val Gln Val Gln Ala Leu Thr Gln
   500 505 510
   Glu Gly Gln Gly Ala Gly Ser Lys Val His Glu Phe Gln Thr Leu Ser
   515 520 525
   Pro Glu Gly Ser Gly Asn Leu Ala Val Ile Gly Gly Val Ala Val Gly
   530 535 540
   Val Val Leu Leu Leu Val Leu Ala Gly Val Gly Phe Phe Ile His Arg
   545 550 555 560
   Arg Arg Lys Asn Gln Arg Ala Arg Gln Ser Pro Glu Asp Val Tyr Phe
   565 570 575
   Ser Lys Ser Glu Gln Leu Lys Pro Leu Lys Thr Tyr Val Asp Pro His
   580 585 590
   Thr Tyr Glu Asp Pro Asn Gln Ala Val Leu Lys Phe Thr Thr Glu Ile
   595 600 605
   His Pro Ser Cys Val Thr Arg Gln Lys Val Ile Gly Ala Gly Glu Phe
   610 615 620
   Gly Glu Val Tyr Lys Gly Met Leu Lys Thr Ser Ser Gly Lys Lys Glu
   625 630 635 640
   Val Pro Val Ala Ile Lys Thr Leu Lys Ala Gly Tyr Thr Glu Lys Gln
   645 650 655
   Arg Val Asp Phe Leu Gly Glu Ala Gly Ile Met Gly Gln Phe Ser His
   660 665 670
   His Asn Ile Ile Arg Leu Glu Gly Val Ile Ser Lys Tyr Lys Pro Met
   675 680 685
   Met Ile Ile Thr Glu Tyr Met Glu Asn Gly Ala Leu Asp Lys Phe Leu
   690 695 700
   Arg Glu Lys Asp Gly Glu Phe Ser Val Leu Gln Leu Val Gly Met Leu
   705 710 715 720
   Arg Gly Ile Ala Ala Gly Met Lys Tyr Leu Ala Asn Met Asn Tyr Val
   725 730 735
   His Arg Asp Leu Ala Ala Arg Asn Ile Leu Val Asn Ser Asn Leu Val
   740 745 750
   Cys Lys Val Ser Asp Phe Gly Leu Ser Arg Val Leu Glu Asp Asp Pro
   755 760 765
   Glu Ala Thr Tyr Thr Thr Ser Gly Gly Lys Ile Pro Ile Arg Trp Thr
   770 775 780
   Ala Pro Glu Ala Ile Ser Tyr Arg Lys Phe Thr Ser Ala Ser Asp Val
   785 790 795 800
   Trp Ser Phe Gly Ile Val Met Trp Glu Val Met Thr Tyr Gly Glu Arg
   805 810 815
   Pro Tyr Trp Glu Leu Ser Asn His Glu Val Met Lys Ala Ile Asn Asp
   820 825 830
   Gly Phe Arg Leu Pro Thr Pro Met Asp Cys Pro Ser Ala Ile Tyr Gln
               835                 840                 845
   Leu Met Met Gln Cys Trp Gln Gln Glu Arg Ala Arg Arg Pro Lys Phe
       850                 855                 860
   Ala Asp Ile Val Ser Ile Leu Asp Lys Leu Ile Arg Ala Pro Asp Ser
   865                 870                 875                 880
   Leu Lys Thr Leu Ala Asp Phe Asp Pro Arg Val Ser Ile Arg Leu Pro
                   885                 890                 895
   Ser Thr Ser Gly Ser Glu Gly Val Pro Phe Arg Thr Val Ser Glu Trp
               900                 905                 910
   Leu Glu Ser Ile Lys Met Gln Gln Tyr Thr Glu His Phe Met Ala Ala
           915                 920                 925
   Gly Tyr Thr Ala Ile Glu Lys Val Val Gln Met Thr Asn Asp Asp Ile
       930                 935                 940
   Lys Arg Ile Gly Val Arg Leu Pro Gly His Gln Lys Arg Ile Ala Tyr
   945                 950                 955                 960
   Ser Leu Leu Gly Leu Lys Asp Gln Val Asn Thr Val Gly Ile Pro Ile
                   965                 970                 975
<210> 35
   <211> 829
   <212> PRT
   <213> Homo Sapiens
<220>
   <221> MISC_FEATURE
   <222> (1)..(829)
   <223> OGTA202 = Swiss Prot Accession No. P22223, Cadherin-3
<400> 35
<210> 36
   <211> 790
   <212> PRT
   <213> Homo Sapiens
<220>
   <221> MISC_FEATURE
   <222> (1)..(790)
   <223> OGTA203 = Swiss Prot Accession No. P55285, Cadherin-6
<400> 36
   Met Arg Thr Tyr Arg Tyr Phe Leu Leu Leu Phe Trp Val Gly Gln Pro
   1 5 10 15
   Tyr Pro Thr Leu Ser Thr Pro Leu Ser Lys Arg Thr Ser Gly Phe Pro
   20 25 30
   Ala Lys Lys Arg Ala Leu Glu Leu Ser Gly Asn Ser Lys Asn Glu Leu
   35 40 45
   Asn Arg Ser Lys Arg Ser Trp Met Trp Asn Gln Phe Phe Leu Leu Glu
   50 55 60
   Glu Tyr Thr Gly Ser Asp Tyr Gln Tyr Val Gly Lys Leu His Ser Asp
   65 70 75 80
   Gln Asp Arg Gly Asp Gly Ser Leu Lys Tyr Ile Leu Ser Gly Asp Gly
   85 90 95
   Ala Gly Asp Leu Phe Ile Ile Asn Glu Asn Thr Gly Asp Ile Gln Ala
   100 105 110
   Thr Lys Arg Leu Asp Arg Glu Glu Lys Pro Val Tyr Ile Leu Arg Ala
   115 120 125
   Gln Ala Ile Asn Arg Arg Thr Gly Arg Pro Val Glu Pro Glu Ser Glu
   130 135 140
   Phe Ile Ile Lys Ile His Asp Ile Asn Asp Asn Glu Pro Ile Phe Thr
   145 150 155 160
   Lys Glu Val Tyr Thr Ala Thr Val Pro Glu Met Ser Asp Val Gly Thr
   165 170 175
   Phe Val Val Gln Val Thr Ala Thr Asp Ala Asp Asp Pro Thr Tyr Gly
   180 185 190
   Asn Ser Ala Lys Val Val Tyr Ser Ile Leu Gln Gly Gln Pro Tyr Phe
   195 200 205
   Ser Val Glu Ser Glu Thr Gly Ile Ile Lys Thr Ala Leu Leu Asn Met
   210 215 220
   Asp Arg Glu Asn Arg Glu Gln Tyr Gln Val Val Ile Gln Ala Lys Asp
   225 230 235 240
   Met Gly Gly Gln Met Gly Gly Leu Ser Gly Thr Thr Thr Val Asn Ile
   245 250 255
   Thr Leu Thr Asp Val Asn Asp Asn Pro Pro Arg Phe Pro Gln Ser Thr
   260 265 270
   Tyr Gln Phe Lys Thr Pro Glu Ser Ser Pro Pro Gly Thr Pro Ile Gly
   275 280 285
   Arg Ile Lys Ala Ser Asp Ala Asp Val Gly Glu Asn Ala Glu Ile Glu
   290 295 300
   Tyr Ser Ile Thr Asp Gly Glu Gly Leu Asp Met Phe Asp Val Ile Thr
   305 310 315 320
   Asp Gln Glu Thr Gln Glu Gly Ile Ile Thr Val Lys Lys Leu Leu Asp
   325 330 335
   Phe Glu Lys Lys Lys Val Tyr Thr Leu Lys Val Glu Ala Ser Asn Pro
   340 345 350
   Tyr Val Glu Pro Arg Phe Leu Tyr Leu Gly Pro Phe Lys Asp Ser Ala
   355 360 365
   Thr Val Arg Ile Val Val Glu Asp Val Asp Glu Pro Pro Val Phe Ser
   370 375 380
   Lys Leu Ala Tyr Ile Leu Gln Ile Arg Glu Asp Ala Gln Ile Asn Thr
   385 390 395 400
   Thr Ile Gly Ser Val Thr Ala Gln Asp Pro Asp Ala Ala Arg Asn Pro
   405 410 415
   Val Lys Tyr Ser Val Asp Arg His Thr Asp Met Asp Arg Ile Phe Asn
   420 425 430
   Ile Asp Ser Gly Asn Gly Ser Ile Phe Thr Ser Lys Leu Leu Asp Arg
           435                 440                 445
   Glu Thr Leu Leu Trp His Asn Ile Thr Val Ile Ala Thr Glu Ile Asn
       450                 455                 460
   Asn Pro Lys Gln Ser Ser Arg Val Pro Leu Tyr Ile Lys Val Leu Asp
   465                 470                 475                 480
   Val Asn Asp Asn Ala Pro Glu Phe Ala Glu Phe Tyr Glu Thr Phe Val
                   485                 490                 495
   Cys Glu Lys Ala Lys Ala Asp Gln Leu Ile Gln Thr Leu His Ala Val
               500                 505                 510
   Asp Lys Asp Asp Pro Tyr Ser Gly His Gln Phe Ser Phe Ser Leu Ala
           515                 520                 525
   Pro Glu Ala Ala Ser Gly Ser Asn Phe Thr Ile Gln Asp Asn Lys Asp
       530                 535                 540
   Asn Thr Ala Gly Ile Leu Thr Arg Lys Asn Gly Tyr Asn Arg His Glu
   545                 550                 555                 560
   Met Ser Thr Tyr Leu Leu Pro Val Val Ile Ser Asp Asn Asp Tyr Pro
                   565                 570                 575
   Val Gln Ser Ser Thr Gly Thr Val Thr Val Arg Val Cys Ala Cys Asp
               580                 585                 590
   His His Gly Asn Met Gln Ser Cys His Ala Glu Ala Leu Ile His Pro
           595                 600                 605
   Thr Gly Leu Ser Thr Gly Ala Leu Val Ala Ile Leu Leu Cys Ile Val
       610                 615                 620
   Ile Leu Leu Val Thr Val Val Leu Phe Ala Ala Leu Arg Arg Gln Arg
   625                 630                 635                 640
   Lys Lys Glu Pro Leu Ile Ile Ser Lys Glu Asp Ile Arg Asp Asn Ile
                   645                 650                 655
   Val Ser Tyr Asn Asp Glu Gly Gly Gly Glu Glu Asp Thr Gln Ala Phe
               660                 665                 670
   Asp Ile Gly Thr Leu Arg Asn Pro Glu Ala Ile Glu Asp Asn Lys Leu
           675                 680                 685
   Arg Arg Asp Ile Val Pro Glu Ala Leu Phe Leu Pro Arg Arg Thr Pro
   690 695 700
   Thr Ala Arg Asp Asn Thr Asp Val Arg Asp Phe Ile Asn Gln Arg Leu
   705 710 715 720
   Lys Glu Asn Asp Thr Asp Pro Thr Ala Pro Pro Tyr Asp Ser Leu Ala
   725 730 735
   Thr Tyr Ala Tyr Glu Gly Thr Gly Ser Val Ala Asp Ser Leu Ser Ser
   740 745 750
   Leu Glu Ser Val Thr Thr Asp Ala Asp Gln Asp Tyr Asp Tyr Leu Ser
   755 760 765
   Asp Trp Gly Pro Arg Phe Lys Lys Leu Ala Asp Met Tyr Gly Gly Val
   770 775 780
   Asp Ser Asp Lys Asp Ser
   785 790
<210> 37
   <211> 209
   <212> PRT
   <213> Homo Sapiens
<220>
   <221> MISC_FEATURE
   <222> (1)..(209)
   <223> OGTA206 = Swiss Prot Accession No. O14493, Claudin-4
<400> 37
   Met Ala Ser Met Gly Leu Gln Val Met Gly Ile Ala Leu Ala Val Leu
   1 5 10 15
   Gly Trp Leu Ala Val Met Leu Cys Cys Ala Leu Pro Met Trp Arg Val
   20 25 30
   Thr Ala Phe Ile Gly Ser Asn Ile Val Thr Ser Gln Thr Ile Trp Glu
   35 40 45
   Gly Leu Trp Met Asn Cys Val Val Gln Ser Thr Gly Gln Met Gln Cys
   50 55 60
   Lys Val Tyr Asp Ser Leu Leu Ala Leu Pro Gln Asp Leu Gln Ala Ala
   65 70 75 80
   Arg Ala Leu Val Ile Ile Ser Ile Ile Val Ala Ala Leu Gly Val Leu
   85 90 95
   Leu Ser Val Val Gly Gly Lys Cys Thr Asn Cys Leu Glu Asp Glu Ser
   100 105 110
   Ala Lys Ala Lys Thr Met Ile Val Ala Gly Val Val Phe Leu Leu Ala
   115 120 125
   Gly Leu Met Val Ile Val Pro Val Ser Trp Thr Ala His Asn Ile Ile
   130 135 140
   Gln Asp Phe Tyr Asn Pro Leu Val Ala Ser Gly Gln Lys Arg Glu Met
   145 150 155 160
   Gly Ala Ser Leu Tyr Val Gly Trp Ala Ala Ser Gly Leu Leu Leu Leu
   165 170 175
   Gly Gly Gly Leu Leu Cys Cys Asn Cys Pro Pro Arg Thr Asp Lys Pro
   180 185 190
   Tyr Ser Ala Lys Tyr Ser Ala Ala Arg Ser Ala Ala Ala Ser Asn Tyr
   195 200 205
   Val
<210> 38
   <211> 1821
   <212> PRT
   <213> Homo Sapiens
<220>
   <221> MISC_FEATURE
   <222> (1)..(1821)
   <223> OGTA213 = Swiss Prot Accession No. Q14767, Latent-transforming growth factor beta-binding protein 2
<400> 38
   Met Arg Pro Arg Thr Lys Ala Arg Ser Pro Gly Arg Ala Leu Arg Asn
   1 5 10 15
   Pro Trp Arg Gly Phe Leu Pro Leu Thr Leu Ala Leu Phe Val Gly Ala
   20 25 30
   Gly His Ala Gln Arg Asp Pro Val Gly Arg Tyr Glu Pro Ala Gly Gly
   35 40 45
   Asp Ala Asn Arg Leu Arg Arg Pro Gly Gly Ser Tyr Pro Ala Ala Ala
   50 55 60
   Ala Ala Lys Val Tyr Ser Leu Phe Arg Glu Gln Asp Ala Pro Val Ala
   65 70 75 80
   Gly Leu Gln Pro Val Glu Arg Ala Gln Pro Gly Trp Gly Ser Pro Arg
   85 90 95
   Arg Pro Thr Glu Ala Glu Ala Arg Arg Pro Ser Arg Ala Gln Gln Ser
   100 105 110
   Arg Arg Val Gln Pro Pro Ala Gln Thr Arg Arg Ser Thr Pro Leu Gly
   115 120 125
   Gln Gln Gln Pro Ala Pro Arg Thr Arg Ala Ala Pro Ala Leu Pro Arg
   130 135 140
   Leu Gly Thr Pro Gln Arg Ser Gly Ala Ala Pro Pro Thr Pro Pro Arg
   145 150 155 160
   Gly Arg Leu Thr Gly Arg Asn Val Cys Gly Gly Gln Cys Cys Pro Gly
   165 170 175
   Trp Thr Thr Ala Asn Ser Thr Asn His Cys Ile Lys Pro Val Cys Glu
   180 185 190
   Pro Pro Cys Gln Asn Arg Gly Ser Cys Ser Arg Pro Gln Leu Cys Val
   195 200 205
   Cys Arg Ser Gly Phe Arg Gly Ala Arg Cys Glu Glu Val Ile Pro Asp
   210 215 220
   Glu Glu Phe Asp Pro Gln Asn Ser Arg Leu Ala Pro Arg Arg Trp Ala
   225 230 235 240
   Glu Arg Ser Pro Asn Leu Arg Arg Ser Ser Ala Ala Gly Glu Gly Thr
   245 250 255
   Leu Ala Arg Ala Gln Pro Pro Ala Pro Gln Ser Pro Pro Ala Pro Gln
   260 265 270
   Ser Pro Pro Ala Gly Thr Leu Ser Gly Leu Ser Gln Thr His Pro Ser
   275 280 285
   Gln Gln His Val Gly Leu Ser Arg Thr Val Arg Leu His Pro Thr Ala
   290 295 300
   Thr Ala Ser Ser Gln Leu Ser Ser Asn Ala Leu Pro Pro Gly Pro Gly
   305 310 315 320
   Leu Glu Gln Arg Asp Gly Thr Gln Gln Ala Val Pro Leu Glu His Pro
   325 330 335
   Ser Ser Pro Trp Gly Leu Asn Leu Thr Glu Lys Ile Lys Lys Ile Lys
   340 345 350
   Ile Val Phe Thr Pro Thr Ile Cys Lys Gln Thr Cys Ala Arg Gly His
   355 360 365
   Cys Ala Asn Ser Cys Glu Arg Gly Asp Thr Thr Thr Leu Tyr Ser Gln
   370 375 380
   Gly Gly His Gly His Asp Pro Lys Ser Gly Phe Arg Ile Tyr Phe Cys
   385 390 395 400
   Gln Ile Pro Cys Leu Asn Gly Gly Arg Cys Ile Gly Arg Asp Glu Cys
   405 410 415
   Trp Cys Pro Ala Asn Ser Thr Gly Lys Phe Cys His Leu Pro Ile Pro
   420 425 430
   Gln Pro Asp Arg Glu Pro Pro Gly Arg Gly Ser Arg Pro Arg Ala Leu
   435 440 445
   Leu Glu Ala Pro Leu Lys Gln Ser Thr Phe Thr Leu Pro Leu Ser Asn
   450 455 460
   Gln Leu Ala Ser Val Asn Pro Ser Leu Val Lys Val His Ile His His
   465 470 475 480
   Pro Pro Glu Ala Ser Val Gln Ile His Gln Val Ala Gln Val Arg Gly
   485 490 495
   Gly Val Glu Glu Ala Leu Val Glu Asn Ser Val Glu Thr Arg Pro Pro
   500 505 510
   Pro Trp Leu Pro Ala Ser Pro Gly His Ser Leu Trp Asp Ser Asn Asn
   515 520 525
   Ile Pro Ala Arg Ser Gly Glu Pro Pro Arg Pro Leu Pro Pro Ala Ala
   530 535 540
   Pro Arg Pro Arg Gly Leu Leu Gly Arg Cys Tyr Leu Asn Thr Val Asn
   545 550 555 560
   Gly Gln Cys Ala Asn Pro Leu Leu Glu Leu Thr Thr Gln Glu Asp Cys
   565 570 575
   Cys Gly Ser Val Gly Ala Phe Trp Gly Val Thr Leu Cys Ala Pro Cys
   580 585 590
   Pro Pro Arg Pro Ala Ser Pro Val Ile Glu Asn Gly Gln Leu Glu Cys
   595 600 605
   Pro Gln Gly Tyr Lys Arg Leu Asn Leu Thr His Cys Gln Asp Ile Asn
   610 615 620
   Glu Cys Leu Thr Leu Gly Leu Cys Lys Asp Ala Glu Cys Val Asn Thr
   625 630 635 640
   Arg Gly Ser Tyr Leu Cys Thr Cys Arg Pro Gly Leu Met Leu Asp Pro
   645 650 655
   Ser Arg Ser Arg Cys Val Ser Asp Lys Ala Ile Ser Met Leu Gln Gly
   660 665 670
   Leu Cys Tyr Arg Ser Leu Gly Pro Gly Thr Cys Thr Leu Pro Leu Ala
   675 680 685
   Gln Arg Ile Thr Lys Gln Ile Cys Cys Cys Ser Arg Val Gly Lys Ala
   690 695 700
   Trp Gly Ser Glu Cys Glu Lys Cys Pro Leu Pro Gly Thr Glu Ala Phe
   705 710 715 720
   Arg Glu Ile Cys Pro Ala Gly His Gly Tyr Thr Tyr Ala Ser Ser Asp
   725 730 735
   Ile Arg Leu Ser Met Arg Lys Ala Glu Glu Glu Glu Leu Ala Arg Pro
   740 745 750
   Pro Arg Glu Gln Gly Gln Arg Ser Ser Gly Ala Leu Pro Gly Pro Ala
   755 760 765
   Glu Arg Gln Pro Leu Arg Val Val Thr Asp Thr Trp Leu Glu Ala Gly
   770 775 780
   Thr Ile Pro Asp Lys Gly Asp Ser Gln Ala Gly Gln Val Thr Thr Ser
   785 790 795 800
   Val Thr His Ala Pro Ala Trp Val Thr Gly Asn Ala Thr Thr Pro Pro
   805 810 815
   Met Pro Glu Gln Gly Ile Ala Glu Ile Gln Glu Glu Gln Val Thr Pro
   820 825 830
   Ser Thr Asp Val Leu Val Thr Leu Ser Thr Pro Gly Ile Asp Arg Cys
   835 840 845
   Ala Ala Gly Ala Thr Asn Val Cys Gly Pro Gly Thr Cys Val Asn Leu
   850 855 860
   Pro Asp Gly Tyr Arg Cys Val Cys Ser Pro Gly Tyr Gln Leu His Pro
   865 870 875 880
   Ser Gln Ala Tyr Cys Thr Asp Asp Asn Glu Cys Leu Arg Asp Pro Cys
   885 890 895
   Lys Gly Lys Gly Arg Cys Ile Asn Arg Val Gly Ser Tyr Ser Cys Phe
   900 905 910
   Cys Tyr Pro Gly Tyr Thr Leu Ala Thr Ser Gly Ala Thr Gln Glu Cys
   915 920 925
   Gln Asp Ile Asn Glu Cys Glu Gln Pro Gly Val Cys Ser Gly Gly Gln
   930 935 940
   Cys Thr Asn Thr Glu Gly Ser Tyr His Cys Glu Cys Asp Gln Gly Tyr
   945 950 955 960
   Ile Met Val Arg Lys Gly His Cys Gln Asp Ile Asn Glu Cys Arg His
   965 970 975
   Pro Gly Thr Cys Pro Asp Gly Arg Cys Val Asn Ser Pro Gly Ser Tyr
   980 985 990
   Thr Cys Leu Ala Cys Glu Glu Gly Tyr Arg Gly Gln Ser Gly Ser Cys
   995 1000 1005
   Val Asp Val Asn Glu Cys Leu Thr Pro Gly Val Cys Ala His Gly
   1010 1015 1020
   Lys Cys Thr Asn Leu Glu Gly Ser Phe Arg Cys Ser Cys Glu Gln
   1025 1030 1035
   Gly Tyr Glu Val Thr Ser Asp Glu Lys Gly Cys Gln Asp Val Asp
   1040 1045 1050
   Glu Cys Ala Ser Arg Ala Ser Cys Pro Thr Gly Leu Cys Leu Asn
   1055 1060 1065
   Thr Glu Gly Ser Phe Ala Cys Ser Ala Cys Glu Asn Gly Tyr Trp
   1070 1075 1080
   Val Asn Glu Asp Gly Thr Ala Cys Glu Asp Leu Asp Glu Cys Ala
   1085 1090 1095
   Phe Pro Gly Val Cys Pro Ser Gly Val Cys Thr Asn Thr Ala Gly
   1100 1105 1110
   Ser Phe Ser Cys Lys Asp Cys Asp Gly Gly Tyr Arg Pro Ser Pro
   1115 1120 1125
   Leu Gly Asp Ser Cys Glu Asp Val Asp Glu Cys Glu Asp Pro Gln
   1130 1135 1140
   Ser Ser Cys Leu Gly Gly Glu Cys Lys Asn Thr Val Gly Ser Tyr
   1145 1150 1155
   Gln Cys Leu Cys Pro Gln Gly Phe Gln Leu Ala Asn Gly Thr Val
   1160 1165 1170
   Cys Glu Asp Val Asn Glu Cys Met Gly Glu Glu His Cys Ala Pro
   1175 1180 1185
   His Gly Glu Cys Leu Asn Ser His Gly Ser Phe Phe Cys Leu Cys
   1190 1195 1200
   Ala Pro Gly Phe Val Ser Ala Glu Gly Gly Thr Ser Cys Gln Asp
   1205 1210 1215
   Val Asp Glu Cys Ala Thr Thr Asp Pro Cys Val Gly Gly His Cys
   1220 1225 1230
   Val Asn Thr Glu Gly Ser Phe Asn Cys Leu Cys Glu Thr Gly Phe
   1235 1240 1245
   Gln Pro Ser Pro Glu Ser Gly Glu Cys Val Asp Ile Asp Glu Cys
   1250 1255 1260
   Glu Asp Tyr Gly Asp Pro Val Cys Gly Thr Trp Lys Cys Glu Asn
   1265 1270 1275
   Ser Pro Gly Ser Tyr Arg Cys Val Leu Gly Cys Gln Pro Gly Phe
   1280 1285 1290
   His Met Ala Pro Asn Gly Asp Cys Ile Asp Ile Asp Glu Cys Ala
   1295 1300 1305
   Asn Asp Thr Met Cys Gly Ser His Gly Phe Cys Asp Asn Thr Asp
   1310 1315 1320
   Gly Ser Phe Arg Cys Leu Cys Asp Gln Gly Phe Glu Ile Ser Pro
   1325 1330 1335
   Ser Gly Trp Asp Cys Val Asp Val Asn Glu Cys Glu Leu Met Leu
   1340 1345 1350
   Ala Val Cys Gly Ala Ala Leu Cys Glu Asn Val Glu Gly Ser Phe
   1355 1360 1365
   Leu Cys Leu Cys Ala Ser Asp Leu Glu Glu Tyr Asp Ala Gln Glu
   1370 1375 1380
   Gly His Cys Arg Pro Arg Gly Ala Gly Gly Gln Ser Met Ser Glu
   1385 1390 1395
   Ala Pro Thr Gly Asp His Ala Pro Ala Pro Thr Arg Met Asp Cys
   1400 1405 1410
   Tyr Ser Gly Gln Lys Gly His Ala Pro Cys Ser Ser Val Leu Gly
   1415 1420 1425
   Arg Asn Thr Thr Gln Ala Glu Cys Cys Cys Thr Gln Gly Ala Thr
   1430 1435 1440
   Trp Gly Asp Ala Cys Asp Leu Cys Pro Ser Glu Asp Ser Ala Glu
   1445 1450 1455
   Phe Ser Glu Ile Cys Pro Ser Gly Lys Gly Tyr Ile Pro Val Glu
   1460 1465 1470
   Gly Ala Trp Thr Phe Gly Gln Thr Met Tyr Thr Asp Ala Asp Glu
   1475 1480 1485
   Cys Val Ile Phe Gly Pro Gly Leu Cys Pro Asn Gly Arg Cys Leu
   1490 1495 1500
   Asn Thr Val Pro Gly Tyr Val Cys Leu Cys Asn Pro Gly Phe His
   1505 1510 1515
   Tyr Asp Ala Ser His Lys Lys Cys Glu Asp His Asp Glu Cys Gln
   1520 1525 1530
   Asp Leu  Ala Cys Glu Asn Gly  Glu Cys Val Asn Thr  Glu Gly Ser
       1535                 1540                 1545
   Phe His  Cys Phe Cys Ser Pro  Pro Leu Thr Leu Asp  Leu Ser Gln
       1550                 1555                 1560
   Gln Arg  Cys Met Asn Ser Thr  Ser Ser Thr Glu Asp  Leu Pro Asp
       1565                 1570                 1575
   His Asp  Ile His Met Asp Ile  Cys Trp Lys Lys Val  Thr Asn Asp
       1580                 1585                 1590
   Val Cys  Ser Glu Pro Leu Arg  Gly His Arg Thr Thr  Tyr Thr Glu
       1595                 1600                 1605
   Cys Cys  Cys Gln Asp Gly Lys  Ala Trp Ser Gln Gln  Cys Ala Leu
       1610                 1615                 1620
   Cys Pro  Pro Arg Ser Ser Glu  Val Tyr Ala Gln Leu  Cys Asn Val
       1625                 1630                 1635
   Ala Arg  Ile Glu Ala Glu Arg  Glu Ala Gly Val His  Phe Arg Pro
       1640                 1645                 1650
   Gly Tyr  Glu Tyr Gly Pro Gly  Pro Asp Asp Leu His  Tyr Ser Ile
       1655                 1660                 1665
   Tyr Gly  Pro Asp Gly Ala Pro  Phe Tyr Asn Tyr Leu  Gly Pro Glu
       1670                 1675                 1680
   Asp Thr  Val Pro Glu Pro Ala  Phe Pro Asn Thr Ala  Gly His Ser
       1685                 1690                 1695
   Ala Asp  Arg Thr Pro Ile Leu  Glu Ser Pro Leu Gln  Pro Ser Glu
       1700                 1705                 1710
   Leu Gln  Pro His Tyr Val Ala  Ser His Pro Glu Pro  Pro Ala Gly
       1715                 1720                 1725
   Phe Glu  Gly Leu Gln Ala Glu  Glu Cys Gly Ile Leu  Asn Gly Cys
       1730                 1735                 1740
   Glu Asn  Gly Arg Cys Val Arg  Val Arg Glu Gly Tyr  Thr Cys Asp
       1745                 1750                 1755
   Cys Phe  Glu Gly Phe Gln Leu  Asp Ala Ala His Met  Ala Cys Val
       1760                 1765                 1770
   Asp Val Asn Glu Cys Asp Asp Leu Asn Gly Pro Ala Val Leu Cys
   1775 1780 1785
   Val His Gly Tyr Cys Glu Asn Thr Glu Gly Ser Tyr Arg Cys His
   1790 1795 1800
   Cys Ser Pro Gly Tyr Val Ala Glu Ala Gly Pro Pro His Cys Thr
   1805 1810 1815
   Ala Lys Glu
   1820
<210> 39
   <211> 512
   <212> PRT
   <213> Homo Sapiens
<220>
   <221> MISC_FEATURE
   <222> (1)..(512)
   <223> OGTA214 = Swiss Prot Accession No. Q9H3R2, Mucin-13
<400> 39
   Met Lys Ala Ile Ile His Leu Thr Leu Leu Ala Leu Leu Ser Val Asn
   1               5                   10                  15
   Thr Ala Thr Asn Gln Gly Asn Ser Ala Asp Ala Val Thr Thr Thr Glu
               20                  25                  30
   Thr Ala Thr Ser Gly Pro Thr Val Ala Ala Ala Asp Thr Thr Glu Thr
           35                  40                  45
   Asn Phe Pro Glu Thr Ala Ser Thr Thr Ala Asn Thr Pro Ser Phe Pro
       50                  55                  60
   Thr Ala Thr Ser Pro Ala Pro Pro Ile Ile Ser Thr His Ser Ser Ser
   65                  70                  75                  80
   Thr Ile Pro Thr Pro Ala Pro Pro Ile Ile Ser Thr His Ser Ser Ser
                   85                  90                  95
   Thr Ile Pro Ile Pro Thr Ala Ala Asp Ser Glu Ser Thr Thr Asn Val
               100                 105                 110
   Asn Ser Leu Ala Thr Ser Asp Ile Ile Thr Ala Ser Ser Pro Asn Asp
           115                 120                 125
   Gly Leu Ile Thr Met Val Pro Ser Glu Thr Gln Ser Asn Asn Glu Met
   130 135 140
   Ser Pro Thr Thr Glu Asp Asn Gln Ser Ser Gly Pro Pro Thr Gly Thr
   145 150 155 160
   Ala Leu Leu Glu Thr Ser Thr Leu Asn Ser Thr Gly Pro Ser Asn Pro
   165 170 175
   Cys Gln Asp Asp Pro Cys Ala Asp Asn Ser Leu Cys Val Lys Leu His
   180 185 190
   Asn Thr Ser Phe Cys Leu Cys Leu Glu Gly Tyr Tyr Tyr Asn Ser Ser
   195 200 205
   Thr Cys Lys Lys Gly Lys Val Phe Pro Gly Lys Ile Ser Val Thr Val
   210 215 220
   Ser Glu Thr Phe Asp Pro Glu Glu Lys His Ser Met Ala Tyr Gln Asp
   225 230 235 240
   Leu His Ser Glu Ile Thr Ser Leu Phe Lys Asp Val Phe Gly Thr Ser
   245 250 255
   Val Tyr Gly Gln Thr Val Ile Leu Thr Val Ser Thr Ser Leu Ser Pro
   260 265 270
   Arg Ser Glu Met Arg Ala Asp Asp Lys Phe Val Asn Val Thr Ile Val
   275 280 285
   Thr Ile Leu Ala Glu Thr Thr Ser Asp Asn Glu Lys Thr Val Thr Glu
   290 295 300
   Lys Ile Asn Lys Ala Ile Arg Ser Ser Ser Ser Asn Phe Leu Asn Tyr
   305 310 315 320
   Asp Leu Thr Leu Arg Cys Asp Tyr Tyr Gly Cys Asn Gln Thr Ala Asp
   325 330 335
   Asp Cys Leu Asn Gly Leu Ala Cys Asp Cys Lys Ser Asp Leu Gln Arg
   340 345 350
   Pro Asn Pro Gln Ser Pro Phe Cys Val Ala Ser Ser Leu Lys Cys Pro
   355 360 365
   Asp Ala Cys Asn Ala Gln His Lys Gln Cys Leu Ile Lys Lys Ser Gly
   370 375 380
   Gly Ala Pro Glu Cys Ala Cys Val Pro Gly Tyr Gln Glu Asp Ala Asn
   385 390 395 400
   Gly Asn Cys Gln Lys Cys Ala Phe Gly Tyr Ser Gly Leu Asp Cys Lys
   405 410 415
   Asp Lys Phe Gln Leu Ile Leu Thr Ile Val Gly Thr Ile Ala Gly Ile
   420 425 430
   Val Ile Leu Ser Met Ile Ile Ala Leu Ile Val Thr Ala Arg Ser Asn
   435 440 445
   Asn Lys Thr Lys His Ile Glu Glu Glu Asn Leu Ile Asp Glu Asp Phe
   450 455 460
   Gln Asn Leu Lys Leu Arg Ser Thr Gly Phe Thr Asn Leu Gly Ala Glu
   465 470 475 480
   Gly Ser Val Phe Pro Lys Val Arg Ile Thr Ala Ser Arg Asp Ser Gln
   485 490 495
   Met Gln Asn Pro Tyr Ser Arg His Ser Ser Met Pro Arg Pro Asp Tyr
   500 505 510
<210> 40
   <211> 1212
   <212> PRT
   <213> Homo Sapiens
<220>
   <221> MISC_FEATURE
   <222> (1)..(1212)
   <223> OGTA216 = Swiss Prot Accession No. P55011, Solute carrier family 12 member 2
<400> 40
<210> 41
   <211> 411
   <212> PRT
   <213> Homo Sapiens
<220>
   <221> MISC_FEATURE
   <222> (1)..(411)
   <223> OGTA222 = Swiss Prot Accession No. P16444, Dipeptidase 1
<400> 41
   Met Trp Ser Gly Trp Trp Leu Trp Pro Leu Val Ala Val Cys Thr Ala
   1 5 10 15
   Asp Phe Phe Arg Asp Glu Ala Glu Arg Ile Met Arg Asp Ser Pro Val
   20 25 30
   Ile Asp Gly His Asn Asp Leu Pro Trp Gln Leu Leu Asp Met Phe Asn
   35 40 45
   Asn Arg Leu Gln Asp Glu Arg Ala Asn Leu Thr Thr Leu Ala Gly Thr
       50                  55                  60
   His Thr Asn Ile Pro Lys Leu Arg Ala Gly Phe Val Gly Gly Gln Phe
   65                  70                  75                  80
   Trp Ser Val Tyr Thr Pro Cys Asp Thr Gln Asn Lys Asp Ala Val Arg
                   85                  90                  95
   Arg Thr Leu Glu Gln Met Asp Val Val His Arg Met Cys Arg Met Tyr
               100                 105                 110
   Pro Glu Thr Phe Leu Tyr Val Thr Ser Ser Ala Gly Ile Arg Gln Ala
           115                 120                 125
   Phe Arg Glu Gly Lys Val Ala Ser Leu Ile Gly Val Glu Gly Gly His
       130                 135                 140
   Ser Ile Asp Ser Ser Leu Gly Val Leu Arg Ala Leu Tyr Gln Leu Gly
   145                 150                 155                 160
   Met Arg Tyr Leu Thr Leu Thr His Ser Cys Asn Thr Pro Trp Ala Asp
                   165                 170                 175
   Asn Trp Leu Val Asp Thr Gly Asp Ser Glu Pro Gln Ser Gln Gly Leu
               180                 185                 190
   Ser Pro Phe Gly Gln Arg Val Val Lys Glu Leu Asn Arg Leu Gly Val
           195                 200                 205
   Leu Ile Asp Leu Ala His Val Ser Val Ala Thr Met Lys Ala Thr Leu
       210                 215                 220
   Gln Leu Ser Arg Ala Pro Val Ile Phe Ser His Ser Ser Ala Tyr Ser
   225                 230                 235                 240
   Val Cys Ala Ser Arg Arg Asn Val Pro Asp Asp Val Leu Arg Leu Val
                   245                 250                 255
   Lys Gln Thr Asp Ser Leu Val Met Val Asn Phe Tyr Asn Asn Tyr Ile
               260                 265                 270
   Ser Cys Thr Asn Lys Ala Asn Leu Ser Gln Val Ala Asp His Leu Asp
           275                 280                 285
   His Ile Lys Glu Val Ala Gly Ala Arg Ala Val Gly Phe Gly Gly Asp
       290                 295                 300
   Phe Asp Gly Val Pro Arg Val Pro Glu Gly Leu Glu Asp Val Ser Lys
   305 310 315 320
   Tyr Pro Asp Leu Ile Ala Glu Leu Leu Arg Arg Asn Trp Thr Glu Ala
   325 330 335
   Glu Val Lys Gly Ala Leu Ala Asp Asn Leu Leu Arg Val Phe Glu Ala
   340 345 350
   Val Glu Gln Ala Ser Asn Leu Thr Gln Ala Pro Glu Glu Glu Pro Ile
   355 360 365
   Pro Leu Asp Gln Leu Gly Gly Ser Cys Arg Thr His Tyr Gly Tyr Ser
   370 375 380
   Ser Gly Ala Ser Ser Leu His Arg His Trp Gly Leu Leu Leu Ala Ser
   385 390 395 400
   Leu Ala Pro Leu Val Leu Cys Leu Ser Leu Leu
   405 410
<210> 42
   <211> 739
   <212> PRT
   <213> Homo Sapiens
<220>
   <221> MISC_FEATURE
   <222> (1)..(739)
   <223> OGTA236 = Swiss Prot Accession No. P50443, Sulfate transporter
<400> 42
   Met Ser Ser Glu Ser Lys Glu Gln His Asn Val Ser Pro Arg Asp Ser
   1 5 10 15
   Ala Glu Gly Asn Asp Ser Tyr Pro Ser Gly Ile His Leu Glu Leu Gln
   20 25 30
   Arg Glu Ser Ser Thr Asp Phe Lys Gln Phe Glu Thr Asn Asp Gln Cys
   35 40 45
   Arg Pro Tyr His Arg Ile Leu Ile Glu Arg Gln Glu Lys Ser Asp Thr
   50 55 60
   Asn Phe Lys Glu Phe Val Ile Lys Lys Leu Gln Lys Asn Cys Gln Cys
   65 70 75 80
   Ser Pro Ala Lys Ala Lys Asn Met Ile Leu Gly Phe Leu Pro Val Leu
                   85                  90                  95
   Gln Trp Leu Pro Lys Tyr Asp Leu Lys Lys Asn Ile Leu Gly Asp Val
               100                 105                 110
   Met Ser Gly Leu Ile Val Gly Ile Leu Leu Val Pro Gln Ser Ile Ala
           115                 120                 125
   Tyr Ser Leu Leu Ala Gly Gln Glu Pro Val Tyr Gly Leu Tyr Thr Ser
       130                 135                 140
   Phe Phe Ala Ser Ile Ile Tyr Phe Leu Leu Gly Thr Ser Arg His Ile
   145                 150                 155                 160
   Ser Val Gly Ile Phe Gly Val Leu Cys Leu Met Ile Gly Glu Thr Val
                   165                 170                 175
   Asp Arg Glu Leu Gln Lys Ala Gly Tyr Asp Asn Ala His Ser Ala Pro
               180                 185                 190
   Ser Leu Gly Met Val Ser Asn Gly Ser Thr Leu Leu Asn His Thr Ser
           195                 200                 205
   Asp Arg Ile Cys Asp Lys Ser Cys Tyr Ala Ile Met Val Gly Ser Thr
       210                 215                 220
   Val Thr Phe Ile Ala Gly Val Tyr Gln Val Ala Met Gly Phe Phe Gln
   225                 230                 235                 240
   Val Gly Phe Val Ser Val Tyr Leu Ser Asp Ala Leu Leu Ser Gly Phe
                   245                 250                 255
   Val Thr Gly Ala Ser Phe Thr Ile Leu Thr Ser Gln Ala Lys Tyr Leu
               260                 265                 270
   Leu Gly Leu Asn Leu Pro Arg Thr Asn Gly Val Gly Ser Leu Ile Thr
           275                 280                 285
   Thr Trp Ile His Val Phe Arg Asn Ile His Lys Thr Asn Leu Cys Asp
       290                 295                 300
   Leu Ile Thr Ser Leu Leu Cys Leu Leu Val Leu Leu Pro Thr Lys Glu
   305                 310                 315                 320
   Leu Asn Glu His Phe Lys Ser Lys Leu Lys Ala Pro Ile Pro Ile Glu
                   325                 330                 335
   Leu Val Val Val Val Ala Ala Thr Leu Ala Ser His Phe Gly Lys Leu
   340 345 350
   His Glu Asn Tyr Asn Ser Ser Ile Ala Gly His Ile Pro Thr Gly Phe
   355 360 365
   Met Pro Pro Lys Val Pro Glu Trp Asn Leu Ile Pro Ser Val Ala Val
   370 375 380
   Asp Ala Ile Ala Ile Ser Ile Ile Gly Phe Ala Ile Thr Val Ser Leu
   385 390 395 400
   Ser Glu Met Phe Ala Lys Lys His Gly Tyr Thr Val Lys Ala Asn Gln
   405 410 415
   Glu Met Tyr Ala Ile Gly Phe Cys Asn Ile Ile Pro Ser Phe Phe His
   420 425 430
   Cys Phe Thr Thr Ser Ala Ala Leu Ala Lys Thr Leu Val Lys Glu Ser
   435 440 445
   Thr Gly Cys His Thr Gln Leu Ser Gly Val Val Thr Ala Leu Val Leu
   450 455 460
   Leu Leu Val Leu Leu Val Ile Ala Pro Leu Phe Tyr Ser Leu Gln Lys
   465 470 475 480
   Ser Val Leu Gly Val Ile Thr Ile Val Asn Leu Arg Gly Ala Leu Arg
   485 490 495
   Lys Phe Arg Asp Leu Pro Lys Met Trp Ser Ile Ser Arg Met Asp Thr
   500 505 510
   Val Ile Trp Phe Val Thr Met Leu Ser Ser Ala Leu Leu Ser Thr Glu
   515 520 525
   Ile Gly Leu Leu Val Gly Val Cys Phe Ser Ile Phe Cys Val Ile Leu
   530 535 540
   Arg Thr Gln Lys Pro Lys Ser Ser Leu Leu Gly Leu Val Glu Glu Ser
   545 550 555 560
   Glu Val Phe Glu Ser Val Ser Ala Tyr Lys Asn Leu Gln Thr Lys Pro
   565 570 575
   Gly Ile Lys Ile Phe Arg Phe Val Ala Pro Leu Tyr Tyr Ile Asn Lys
   580 585 590
   Glu Cys Phe Lys Ser Ala Leu Tyr Lys Gln Thr Val Asn Pro Ile Leu
   595 600 605
   Ile Lys Val Ala Trp Lys Lys Ala Ala Lys Arg Lys Ile Lys Glu Lys
   610 615 620
   Val Val Thr Leu Gly Gly Ile Gln Asp Glu Met Ser Val Gln Leu Ser
   625 630 635 640
   His Asp Pro Leu Glu Leu His Thr Ile Val Ile Asp Cys Ser Ala Ile
   645 650 655
   Gln Phe Leu Asp Thr Ala Gly Ile His Thr Leu Lys Glu Val Arg Arg
   660 665 670
   Asp Tyr Glu Ala Ile Gly Ile Gln Val Leu Leu Ala Gln Cys Asn Pro
   675 680 685
   Thr Val Arg Asp Ser Leu Thr Asn Gly Glu Tyr Cys Lys Lys Glu Glu
   690 695 700
   Glu Asn Leu Leu Phe Tyr Ser Val Tyr Glu Ala Met Ala Phe Ala Glu
   705 710 715 720
   Val Ser Lys Asn Gln Lys Gly Val Cys Val Pro Asn Gly Leu Ser Leu
   725 730 735
   Ser Ser Asp
<210> 43
   <211> 802
   <212> PRT
   <213> Homo Sapiens
<220>
   <221> MISC_FEATURE
   <222> (1)..(802)
   <223> OGTA237 = Swiss Prot Accession No. P22455, Fibroblast growth factor receptor 4
<400> 43
   Met Arg Leu Leu Leu Ala Leu Leu Gly Val Leu Leu Ser Val Pro Gly
   1 5 10 15
   Pro Pro Val Leu Ser Leu Glu Ala Ser Glu Glu Val Glu Leu Glu Pro
   20 25 30
   Cys Leu Ala Pro Ser Leu Glu Gln Gln Glu Gln Glu Leu Thr Val Ala
           35                  40                  45
   Leu Gly Gln Pro Val Arg Leu Cys Cys Gly Arg Ala Glu Arg Gly Gly
       50                  55                  60
   His Trp Tyr Lys Glu Gly Ser Arg Leu Ala Pro Ala Gly Arg Val Arg
   65                  70                  75                  80
   Gly Trp Arg Gly Arg Leu Glu Ile Ala Ser Phe Leu Pro Glu Asp Ala
                   85                  90                  95
   Gly Arg Tyr Leu Cys Leu Ala Arg Gly Ser Met Ile Val Leu Gln Asn
               100                 105                 110
   Leu Thr Leu Ile Thr Gly Asp Ser Leu Thr Ser Ser Asn Asp Asp Glu
           115                 120                 125
   Asp Pro Lys Ser His Arg Asp Pro Ser Asn Arg His Ser Tyr Pro Gln
       130                 135                 140
   Gln Ala Pro Tyr Trp Thr His Pro Gln Arg Met Glu Lys Lys Leu His
   145                 150                 155                 160
   Ala Val Pro Ala Gly Asn Thr Val Lys Phe Arg Cys Pro Ala Ala Gly
                   165                 170                 175
   Asn Pro Thr Pro Thr Ile Arg Trp Leu Lys Asp Gly Gln Ala Phe His
               180                 185                 190
   Gly Glu Asn Arg Ile Gly Gly Ile Arg Leu Arg His Gln His Trp Ser
           195                 200                 205
   Leu Val Met Glu Ser Val Val Pro Ser Asp Arg Gly Thr Tyr Thr Cys
       210                 215                 220
   Leu Val Glu Asn Ala Val Gly Ser Ile Arg Tyr Asn Tyr Leu Leu Asp
   225                 230                 235                 240
   Val Leu Glu Arg Ser Pro His Arg Pro Ile Leu Gln Ala Gly Leu Pro
                   245                 250                 255
   Ala Asn Thr Thr Ala Val Val Gly Ser Asp Val Glu Leu Leu Cys Lys
               260                 265                 270
   Val Tyr Ser Asp Ala Gln Pro His Ile Gln Trp Leu Lys His Ile Val
           275                 280                 285
   Ile Asn Gly Ser Ser Phe Gly Ala Asp Gly Phe Pro Tyr Val Gln Val
   290 295 300
   Leu Lys Thr Ala Asp Ile Asn Ser Ser Glu Val Glu Val Leu Tyr Leu
   305 310 315 320
   Arg Asn Val Ser Ala Glu Asp Ala Gly Glu Tyr Thr Cys Leu Ala Gly
   325 330 335
   Asn Ser Ile Gly Leu Ser Tyr Gln Ser Ala Trp Leu Thr Val Leu Pro
   340 345 350
   Glu Glu Asp Pro Thr Trp Thr Ala Ala Ala Pro Glu Ala Arg Tyr Thr
   355 360 365
   Asp Ile Ile Leu Tyr Ala Ser Gly Ser Leu Ala Leu Ala Val Leu Leu
   370 375 380
   Leu Leu Ala Gly Leu Tyr Arg Gly Gln Ala Leu His Gly Arg His Pro
   385 390 395 400
   Arg Pro Pro Ala Thr Val Gln Lys Leu Ser Arg Phe Pro Leu Ala Arg
   405 410 415
   Gln Phe Ser Leu Glu Ser Gly Ser Ser Gly Lys Ser Ser Ser Ser Leu
   420 425 430
   Val Arg Gly Val Arg Leu Ser Ser Ser Gly Pro Ala Leu Leu Ala Gly
   435 440 445
   Leu Val Ser Leu Asp Leu Pro Leu Asp Pro Leu Trp Glu Phe Pro Arg
   450 455 460
   Asp Arg Leu Val Leu Gly Lys Pro Leu Gly Glu Gly Cys Phe Gly Gln
   465 470 475 480
   Val Val Arg Ala Glu Ala Phe Gly Met Asp Pro Ala Arg Pro Asp Gln
   485 490 495
   Ala Ser Thr Val Ala Val Lys Met Leu Lys Asp Asn Ala Ser Asp Lys
   500 505 510
   Asp Leu Ala Asp Leu Val Ser Glu Met Glu Val Met Lys Leu Ile Gly
   515 520 525
   Arg His Lys Asn Ile Ile Asn Leu Leu Gly Val Cys Thr Gln Glu Gly
   530 535 540
   Pro Leu Tyr Val Ile Val Glu Cys Ala Ala Lys Gly Asn Leu Arg Glu
   545 550 555 560
   Phe Leu Arg Ala Arg Arg Pro Pro Gly Pro Asp Leu Ser Pro Asp Gly
   565 570 575
   Pro Arg Ser Ser Glu Gly Pro Leu Ser Phe Pro Val Leu Val Ser Cys
   580 585 590
   Ala Tyr Gln Val Ala Arg Gly Met Gln Tyr Leu Glu Ser Arg Lys Cys
   595 600 605
   Ile His Arg Asp Leu Ala Ala Arg Asn Val Leu Val Thr Glu Asp Asn
   610 615 620
   Val Met Lys Ile Ala Asp Phe Gly Leu Ala Arg Gly Val His His Ile
   625 630 635 640
   Asp Tyr Tyr Lys Lys Thr Ser Asn Gly Arg Leu Pro Val Lys Trp Met
   645 650 655
   Ala Pro Glu Ala Leu Phe Asp Arg Val Tyr Thr His Gln Ser Asp Val
   660 665 670
   Trp Ser Phe Gly Ile Leu Leu Trp Glu Ile Phe Thr Leu Gly Gly Ser
   675 680 685
   Pro Tyr Pro Gly Ile Pro Val Glu Glu Leu Phe Ser Leu Leu Arg Glu
   690 695 700
   Gly His Arg Met Asp Arg Pro Pro His Cys Pro Pro Glu Leu Tyr Gly
   705 710 715 720
   Leu Met Arg Glu Cys Trp His Ala Ala Pro Ser Gln Arg Pro Thr Phe
   725 730 735
   Lys Gln Leu Val Glu Ala Leu Asp Lys Val Leu Leu Ala Val Ser Glu
   740 745 750
   Glu Tyr Leu Asp Leu Arg Leu Thr Phe Gly Pro Tyr Ser Pro Ser Gly
   755 760 765
   Gly Asp Ala Ser Ser Thr Cys Ser Ser Ser Asp Ser Val Phe Ser His
   770 775 780
   Asp Pro Leu Pro Leu Gly Ser Ser Ser Phe Pro Phe Gly Ser Gly Val
   785 790 795 800
   Gln Thr
<210> 44
   <211> 1257
   <212> PRT
   <213> Homo Sapiens
<220>
   <221> MISC_FEATURE
   <222> (1)..(1257)
   <223> OGTA247 = Swiss Prot Accession No. P32004, Neural cell adhesion molecule L1
<400> 44
<210> 45
   <211> 864
   <212> PRT
   <213> Homo Sapiens
<220>
   <221> MISC_FEATURE
   <222> (1)..(864)
   <223> OGTA248 = Swiss Prot Accession No. P29376, Leukocyte tyrosine kinase receptor
<400> 45
<210> 46
   <211> 176
   <212> PRT
   <213> Homo Sapiens
<220>
   <221> MISC_FEATURE
   <222> (1)..(176)
   <223> OGTA249 = Swiss Prot Accession No. Q969L2, Protein MAL2
<400> 46
   Met Ser Ala Gly Gly Ala Ser Val Pro Pro Pro Pro Asn Pro Ala Val
   1 5 10 15
   Ser Phe Pro Pro Pro Arg Val Thr Leu Pro Ala Gly Pro Asp Ile Leu
   20 25 30
   Arg Thr Tyr Ser Gly Ala Phe Val Cys Leu Glu Ile Leu Phe Gly Gly
   35 40 45
   Leu Val Trp Ile Leu Val Ala Ser Ser Asn Val Pro Leu Pro Leu Leu
   50 55 60
   Gln Gly Trp Val Met Phe Val Ser Val Thr Ala Phe Phe Phe Ser Leu
   65 70 75 80
   Leu Phe Leu Gly Met Phe Leu Ser Gly Met Val Ala Gln Ile Asp Ala
   85 90 95
   Asn Trp Asn Phe Leu Asp Phe Ala Tyr His Phe Thr Val Phe Val Phe
   100 105 110
   Tyr Phe Gly Ala Phe Leu Leu Glu Ala Ala Ala Thr Ser Leu His Asp
   115 120 125
   Leu His Cys Asn Thr Thr Ile Thr Gly Gln Pro Leu Leu Ser Asp Asn
   130 135 140
   Gln Tyr Asn Ile Asn Val Ala Ala Ser Ile Phe Ala Phe Met Thr Thr
   145 150 155 160
   Ala Cys Tyr Gly Cys Ser Leu Gly Leu Ala Leu Arg Arg Trp Arg Pro
   165 170 175
<210> 47
   <211> 725
   <212> PRT
   <213> Homo Sapiens
<220>
   <221> MISC_FEATURE
   <222> (1)..(725)
   <223> OGTA257 = Swiss Prot Accession No. Q9H1D0, Transient receptor potential cation channel subfamily V member 6
<400> 47
   Met Gly Leu Ser Leu Pro Lys Glu Lys Gly Leu Ile Leu Cys Leu Trp
   1               5                   10                  15
   Ser Lys Phe Cys Arg Trp Phe Gln Arg Arg Glu Ser Trp Ala Gln Ser
               20                  25                  30
   Arg Asp Glu Gln Asn Leu Leu Gln Gln Lys Arg Ile Trp Glu Ser Pro
           35                  40                  45
   Leu Leu Leu Ala Ala Lys Asp Asn Asp Val Gln Ala Leu Asn Lys Leu
       50                  55                  60
   Leu Lys Tyr Glu Asp Cys Lys Val His Gln Arg Gly Ala Met Gly Glu
   65                  70                  75                  80
   Thr Ala Leu His Ile Ala Ala Leu Tyr Asp Asn Leu Glu Ala Ala Met
                   85                  90                  95
   Val Leu Met Glu Ala Ala Pro Glu Leu Val Phe Glu Pro Met Thr Ser
               100                 105                 110
   Glu Leu Tyr Glu Gly Gln Thr Ala Leu His Ile Ala Val Val Asn Gln
           115                 120                 125
   Asn Met Asn Leu Val Arg Ala Leu Leu Ala Arg Arg Ala Ser Val Ser
       130                 135                 140
   Ala Arg Ala Thr Gly Thr Ala Phe Arg Arg Ser Pro Cys Asn Leu Ile
   145                 150                 155                 160
   Tyr Phe Gly Glu His Pro Leu Ser Phe Ala Ala Cys Val Asn Ser Glu
                   165                 170                 175
   Glu Ile Val Arg Leu Leu Ile Glu His Gly Ala Asp Ile Arg Ala Gln
               180                 185                 190
   Asp Ser Leu Gly Asn Thr Val Leu His Ile Leu Ile Leu Gln Pro Asn
           195                 200                 205
   Lys Thr Phe Ala Cys Gln Met Tyr Asn Leu Leu Leu Ser Tyr Asp Arg
       210                 215                 220
   His Gly Asp His Leu Gln Pro Leu Asp Leu Val Pro Asn His Gln Gly
   225                 230                 235                 240
   Leu Thr Pro Phe Lys Leu Ala Gly Val Glu Gly Asn Thr Val Met Phe
                   245                 250                 255
   Gln His Leu Met Gln Lys Arg Lys His Thr Gln Trp Thr Tyr Gly Pro
               260                 265                 270
   Leu Thr Ser Thr Leu Tyr Asp Leu Thr Glu Ile Asp Ser Ser Gly Asp
           275                 280                 285
   Glu Gln Ser Leu Leu Glu Leu Ile Ile Thr Thr Lys Lys Arg Glu Ala
       290                 295                 300
   Arg Gln Ile Leu Asp Gln Thr Pro Val Lys Glu Leu Val Ser Leu Lys
   305                 310                 315                 320
   Trp Lys Arg Tyr Gly Arg Pro Tyr Phe Cys Met Leu Gly Ala Ile Tyr
                   325                 330                 335
   Leu Leu Tyr Ile Ile Cys Phe Thr Met Cys Cys Ile Tyr Arg Pro Leu
               340                 345                 350
   Lys Pro Arg Thr Asn Asn Arg Thr Ser Pro Arg Asp Asn Thr Leu Leu
           355                 360                 365
   Gln Gln Lys Leu Leu Gln Glu Ala Tyr Met Thr Pro Lys Asp Asp Ile
       370                 375                 380
   Arg Leu Val Gly Glu Leu Val Thr Val Ile Gly Ala Ile Ile Ile Leu
   385                 390                 395                 400
   Leu Val Glu Val Pro Asp Ile Phe Arg Met Gly Val Thr Arg Phe Phe
                   405                 410                 415
   Gly Gln Thr Ile Leu Gly Gly Pro Phe His Val Leu Ile Ile Thr Tyr
               420                 425                 430
   Ala Phe Met Val Leu Val Thr Met Val Met Arg Leu Ile Ser Ala Ser
           435                 440                 445
   Gly Glu Val Val Pro Met Ser Phe Ala Leu Val Leu Gly Trp Cys Asn
   450 455 460
   Val Met Tyr Phe Ala Arg Gly Phe Gln Met Leu Gly Pro Phe Thr Ile
   465 470 475 480
   Met Ile Gln Lys Met Ile Phe Gly Asp Leu Met Arg Phe Cys Trp Leu
   485 490 495
   Met Ala Val Val Ile Leu Gly Phe Ala Ser Ala Phe Tyr Ile Ile Phe
   500 505 510
   Gln Thr Glu Asp Pro Glu Glu Leu Gly His Phe Tyr Asp Tyr Pro Met
   515 520 525
   Ala Leu Phe Ser Thr Phe Glu Leu Phe Leu Thr Ile Ile Asp Gly Pro
   530 535 540
   Ala Asn Tyr Asn Val Asp Leu Pro Phe Met Tyr Ser Ile Thr Tyr Ala
   545 550 555 560
   Ala Phe Ala Ile Ile Ala Thr Leu Leu Met Leu Asn Leu Leu Ile Ala
   565 570 575
   Met Met Gly Asp Thr His Trp Arg Val Ala His Glu Arg Asp Glu Leu
   580 585 590
   Trp Arg Ala Gln Ile Val Ala Thr Thr Val Met Leu Glu Arg Lys Leu
   595 600 605
   Pro Arg Cys Leu Trp Pro Arg Ser Gly Ile Cys Gly Arg Glu Tyr Gly
   610 615 620
   Leu Gly Asp Arg Trp Phe Leu Arg Val Glu Asp Arg Gln Asp Leu Asn
   625 630 635 640
   Arg Gln Arg Ile Gln Arg Tyr Ala Gln Ala Phe His Thr Arg Gly Ser
   645 650 655
   Glu Asp Leu Asp Lys Asp Ser Val Glu Lys Leu Glu Leu Gly Cys Pro
   660 665 670
   Phe Ser Pro His Leu Ser Leu Pro Met Pro Ser Val Ser Arg Ser Thr
   675 680 685
   Ser Arg Ser Ser Ala Asn Trp Glu Arg Leu Arg Gln Gly Thr Leu Arg
   690 695 700
   Arg Asp Leu Arg Gly Ile Ile Asn Arg Gly Leu Glu Asp Gly Glu Ser
   705 710 715 720
   Trp Glu Tyr Gln Ile
   725
<210> 48
   <211> 1898
   <212> PRT
   <213> Homo Sapiens
<220>
   <221> MISC_FEATURE
   <222> (1)..(1898)
   <223> OGTA271 = Swiss Prot Accession No. Q5T021, Protein tyrosine phosphatase, receptor type, F
<400> 48
   Met Ala Pro Glu Pro Ala Pro Gly Arg Thr Met Val Pro Leu Val Pro
   1 5 10 15
   Ala Leu Val Met Leu Gly Leu Val Ala Gly Ala His Gly Asp Ser Lys
   20 25 30
   Pro Val Phe Ile Lys Val Pro Glu Asp Gln Thr Gly Leu Ser Gly Gly
   35 40 45
   Val Ala Ser Phe Val Cys Gln Ala Thr Gly Glu Pro Lys Pro Arg Ile
   50 55 60
   Thr Trp Met Lys Lys Gly Lys Lys Val Ser Ser Gln Arg Phe Glu Val
   65 70 75 80
   Ile Glu Phe Asp Asp Gly Ala Gly Ser Val Leu Arg Ile Gln Pro Leu
   85 90 95
   Arg Val Gln Arg Asp Glu Ala Ile Tyr Glu Cys Thr Ala Thr Asn Ser
   100 105 110
   Leu Gly Glu Ile Asn Thr Ser Ala Lys Leu Ser Val Leu Glu Glu Glu
   115 120 125
   Gln Leu Pro Pro Gly Phe Pro Ser Ile Asp Met Gly Pro Gln Leu Lys
   130 135 140
   Val Val Glu Lys Ala Arg Thr Ala Thr Met Leu Cys Ala Ala Gly Gly
   145 150 155 160
   Asn Pro Asp Pro Glu Ile Ser Trp Phe Lys Asp Phe Leu Pro Val Asp
   165 170 175
   Pro Ala Thr Ser Asn Gly Arg Ile Lys Gln Leu Arg Ser Gly Ala Leu
   180 185 190
   Gln Ile Glu Ser Ser Glu Glu Ser Asp Gln Gly Lys Tyr Glu Cys Val
   195 200 205
   Ala Thr Asn Ser Ala Gly Thr Arg Tyr Ser Ala Pro Ala Asn Leu Tyr
   210 215 220
   Val Arg Val Arg Arg Val Ala Pro Arg Phe Ser Ile Pro Pro Ser Ser
   225 230 235 240
   Gln Glu Val Met Pro Gly Gly Ser Val Asn Leu Thr Cys Val Ala Val
   245 250 255
   Gly Ala Pro Met Pro Tyr Val Lys Trp Met Met Gly Ala Glu Glu Leu
   260 265 270
   Thr Lys Glu Asp Glu Met Pro Val Gly Arg Asn Val Leu Glu Leu Ser
   275 280 285
   Asn Val Val Arg Ser Ala Asn Tyr Thr Cys Val Ala Ile Ser Ser Leu
   290 295 300
   Gly Met Ile Glu Ala Thr Ala Gln Val Thr Val Lys Ala Leu Pro Lys
   305 310 315 320
   Pro Pro Ile Asp Leu Val Val Thr Glu Thr Thr Ala Thr Ser Val Thr
   325 330 335
   Leu Thr Trp Asp Ser Gly Asn Ser Glu Pro Val Thr Tyr Tyr Gly Ile
   340 345 350
   Gln Tyr Arg Ala Ala Gly Thr Glu Gly Pro Phe Gln Glu Val Asp Gly
   355 360 365
   Val Ala Thr Thr Arg Tyr Ser Ile Gly Gly Leu Ser Pro Phe Ser Glu
   370 375 380
   Tyr Ala Phe Arg Val Leu Ala Val Asn Ser Ile Gly Arg Gly Pro Pro
   385 390 395 400
   Ser Glu Ala Val Arg Ala Arg Thr Gly Glu Gln Ala Pro Ser Ser Pro
   405 410 415
   Pro Arg Arg Val Gln Ala Arg Met Leu Ser Ala Ser Thr Met Leu Val
   420 425 430
   Gln Trp Glu Pro Pro Glu Glu Pro Asn Gly Leu Val Arg Gly Tyr Arg
   435 440 445
   Val Tyr Tyr Thr Pro Asp Ser Arg Arg Pro Pro Asn Ala Trp His Lys
   450 455 460
   His Asn Thr Asp Ala Gly Leu Leu Thr Thr Val Gly Ser Leu Leu Pro
   465 470 475 480
   Gly Ile Thr Tyr Ser Leu Arg Val Leu Ala Phe Thr Ala Val Gly Asp
   485 490 495
   Gly Pro Pro Ser Pro Thr Ile Gln Val Lys Thr Gln Gln Gly Val Pro
   500 505 510
   Ala Gln Pro Ala Asp Phe Gln Ala Glu Val Glu Ser Asp Thr Arg Ile
   515 520 525
   Gln Leu Ser Trp Leu Leu Pro Pro Gln Glu Arg Ile Ile Met Tyr Glu
   530 535 540
   Leu Val Tyr Trp Ala Ala Glu Asp Glu Asp Gln Gln His Lys Val Thr
   545 550 555 560
   Phe Asp Pro Thr Ser Ser Tyr Thr Leu Glu Asp Leu Lys Pro Asp Thr
   565 570 575
   Leu Tyr Arg Phe Gln Leu Ala Ala Arg Ser Asp Met Gly Val Gly Val
   580 585 590
   Phe Thr Pro Thr Ile Glu Ala Arg Thr Ala Gln Ser Thr Pro Ser Ala
   595 600 605
   Pro Pro Gln Lys Val Met Cys Val Ser Met Gly Ser Thr Thr Val Arg
   610 615 620
   Val Ser Trp Val Pro Pro Pro Ala Asp Ser Arg Asn Gly Val Ile Thr
   625 630 635 640
   Gln Tyr Ser Val Ala Tyr Glu Ala Val Asp Gly Glu Asp Arg Gly Arg
   645 650 655
   His Val Val Asp Gly Ile Ser Arg Glu His Ser Ser Trp Asp Leu Val
   660 665 670
   Gly Leu Glu Lys Trp Thr Glu Tyr Arg Val Trp Val Arg Ala His Thr
   675 680 685
   Asp Val Gly Pro Gly Pro Glu Ser Ser Pro Val Leu Val Arg Thr Asp
   690 695 700
   Glu Asp Val Pro Ser Gly Pro Pro Arg Lys Val Glu Val Glu Pro Leu
   705 710 715 720
   Asn Ser Thr Ala Val His Val Tyr Trp Lys Leu Pro Val Pro Ser Lys
   725 730 735
   Gln His Gly Gln Ile Arg Gly Tyr Gln Val Thr Tyr Val Arg Leu Glu
   740 745 750
   Asn Gly Glu Pro Arg Gly Leu Pro Ile Ile Gln Asp Val Met Leu Ala
   755 760 765
   Glu Ala Gln Glu Thr Thr Ile Ser Gly Leu Thr Pro Glu Thr Thr Tyr
   770 775 780
   Ser Val Thr Val Ala Ala Tyr Thr Thr Lys Gly Asp Gly Ala Arg Ser
   785 790 795 800
   Lys Pro Lys Ile Val Thr Thr Thr Gly Ala Val Pro Gly Arg Pro Thr
   805 810 815
   Met Met Ile Ser Thr Thr Ala Met Asn Thr Ala Leu Leu Gln Trp His
   820 825 830
   Pro Pro Lys Glu Leu Pro Gly Glu Leu Leu Gly Tyr Arg Leu Gln Tyr
   835 840 845
   Cys Arg Ala Asp Glu Ala Arg Pro Asn Thr Ile Asp Phe Gly Lys Asp
   850 855 860
   Asp Gln His Phe Thr Val Thr Gly Leu His Lys Gly Thr Thr Tyr Ile
   865 870 875 880
   Phe Arg Leu Ala Ala Lys Asn Arg Ala Gly Leu Gly Glu Glu Phe Glu
   885 890 895
   Lys Glu Ile Arg Thr Pro Glu Asp Leu Pro Ser Gly Phe Pro Gln Asn
   900 905 910
   Leu His Val Thr Gly Leu Thr Thr Ser Thr Thr Glu Leu Ala Trp Asp
   915 920 925
   Pro Pro Val Leu Ala Glu Arg Asn Gly Arg Ile Ile Ser Tyr Thr Val
   930 935 940
   Val Phe Arg Asp Ile Asn Ser Gln Gln Glu Leu Gln Asn Ile Thr Thr
   945 950 955 960
   Asp Thr Arg Phe Thr Leu Thr Gly Leu Lys Pro Asp Thr Thr Tyr Asp
   965 970 975
   Ile Lys Val Arg Ala Trp Thr Ser Lys Gly Ser Gly Pro Leu Ser Pro
   980 985 990
   Ser Ile Gln Ser Arg Thr Met Pro Val Glu Gln Val Phe Ala Lys Asn
   995 1000 1005
   Phe Arg Val Ala Ala Ala Met Lys Thr Ser Val Leu Leu Ser Trp
   1010 1015 1020
   Glu Val Pro Asp Ser Tyr Lys Ser Ala Val Pro Phe Lys Ile Leu
   1025 1030 1035
   Tyr Asn Gly Gln Ser Val Glu Val Asp Gly His Ser Met Arg Lys
   1040 1045 1050
   Leu Ile Ala Asp Leu Gln Pro Asn Thr Glu Tyr Ser Phe Val Leu
   1055 1060 1065
   Met Asn Arg Gly Ser Ser Ala Gly Gly Leu Gln His Leu Val Ser
   1070 1075 1080
   Ile Arg Thr Ala Pro Asp Leu Leu Pro His Lys Pro Leu Pro Ala
   1085 1090 1095
   Ser Ala Tyr Ile Glu Asp Gly Arg Phe Asp Leu Ser Met Pro His
   1100 1105 1110
   Val Gln Asp Pro Ser Leu Val Arg Trp Phe Tyr Ile Val Val Val
   1115 1120 1125
   Pro Ile Asp Arg Val Gly Gly Ser Met Leu Thr Pro Arg Trp Ser
   1130 1135 1140
   Thr Pro Glu Glu Leu Glu Leu Asp Glu Leu Leu Glu Ala Ile Glu
   1145 1150 1155
   Gln Gly Gly Glu Glu Gln Arg Arg Arg Arg Arg Gln Ala Glu Arg
   1160 1165 1170
   Leu Lys Pro Tyr Val Ala Ala Gln Leu Asp Val Leu Pro Glu Thr
   1175 1180 1185
   Phe Thr Leu Gly Asp Lys Lys Asn Tyr Arg Gly Phe Tyr Asn Arg
   1190 1195 1200
   Pro Leu Ser Pro Asp Leu Ser Tyr Gln Cys Phe Val Leu Ala Ser
   1205 1210 1215
   Leu Lys Glu Pro Met Asp Gln Lys Arg Tyr Ala Ser Ser Pro Tyr
   1220 1225 1230
   Ser Asp Glu Ile Val Val Gln Val Thr Pro Ala Gln Gln Gln Glu
   1235 1240 1245
   Glu Pro Glu Met Leu Trp Val Thr Gly Pro Val Leu Ala Val Ile
   1250 1255 1260
   Leu Ile Ile Leu Ile Val Ile Ala Ile Leu Leu Phe Lys Arg Lys
   1265 1270 1275
   Arg Thr His Ser Pro Ser Ser Lys Asp Glu Gln Ser Ile Gly Leu
   1280 1285 1290
   Lys Asp Ser Leu Leu Ala His Ser Ser Asp Pro Val Glu Met Arg
   1295 1300 1305
   Arg Leu Asn Tyr Gln Thr Pro Gly Met Arg Asp His Pro Pro Ile
   1310 1315 1320
   Pro Ile Thr Asp Leu Ala Asp Asn Ile Glu Arg Leu Lys Ala Asn
   1325 1330 1335
   Asp Gly Leu Lys Phe Ser Gln Glu Tyr Glu Ser Ile Asp Pro Gly
   1340 1345 1350
   Gln Gln Phe Thr Trp Glu Asn Ser Asn Leu Glu Val Asn Lys Pro
   1355 1360 1365
   Lys Asn Arg Tyr Ala Asn Val Ile Ala Tyr Asp His Ser Arg Val
   1370 1375 1380
   Ile Leu Thr Ser Ile Asp Gly Val Pro Gly Ser Asp Tyr Ile Asn
   1385 1390 1395
   Ala Asn Tyr Ile Asp Gly Tyr Arg Lys Gln Asn Ala Tyr Ile Ala
   1400 1405 1410
   Thr Gln Gly Pro Leu Pro Glu Thr Met Gly Asp Phe Trp Arg Met
   1415 1420 1425
   Val Trp Glu Gln Arg Thr Ala Thr Val Val Met Met Thr Arg Leu
   1430 1435 1440
   Glu Glu Lys Ser Arg Val Lys Cys Asp Gln Tyr Trp Pro Ala Arg
   1445 1450 1455
   Gly Thr Glu Thr Cys Gly Leu Ile Gln Val Thr Leu Leu Asp Thr
   1460 1465 1470
   Val Glu Leu Ala Thr Tyr Thr Val Arg Thr Phe Ala Leu His Lys
   1475 1480 1485
   Ser Gly Ser Ser Glu Lys Arg Glu Leu Arg Gln Phe Gln Phe Met
   1490 1495 1500
   Ala Trp Pro Asp His Gly Val Pro Glu Tyr Pro Thr Pro Ile Leu
   1505 1510 1515
   Ala Phe Leu Arg Arg Val Lys Ala Cys Asn Pro Leu Asp Ala Gly
   1520 1525 1530
   Pro Met Val Val His Cys Ser Ala Gly Val Gly Arg Thr Gly Cys
   1535 1540 1545
   Phe Ile Val Ile Asp Ala Met Leu Glu Arg Met Lys His Glu Lys
   1550 1555 1560
   Thr Val Asp Ile Tyr Gly His Val Thr Cys Met Arg Ser Gln Arg
   1565 1570 1575
   Asn Tyr Met Val Gln Thr Glu Asp Gln Tyr Val Phe Ile His Glu
   1580 1585 1590
   Ala Leu Leu Glu Ala Ala Thr Cys Gly His Thr Glu Val Pro Ala
   1595 1600 1605
   Arg Asn Leu Tyr Ala His Ile Gln Lys Leu Gly Gln Val Pro Pro
   1610 1615 1620
   Gly Glu Ser Val Thr Ala Met Glu Leu Glu Phe Lys Leu Leu Ala
   1625 1630 1635
   Ser Ser  Lys Ala His Thr Ser  Arg Phe Ile Ser Ala  Asn Leu Pro
       1640                 1645                 1650
   Cys Asn  Lys Phe Lys Asn Arg  Leu Val Asn Ile Met  Pro Tyr Glu
       1655                 1660                 1665
   Leu Thr  Arg Val Cys Leu Gln  Pro Ile Arg Gly Val  Glu Gly Ser
       1670                 1675                 1680
   Asp Tyr  Ile Asn Ala Ser Phe  Leu Asp Gly Tyr Arg  Gln Gln Lys
       1685                 1690                 1695
   Ala Tyr  Ile Ala Thr Gln Gly  Pro Leu Ala Glu Ser  Thr Glu Asp
       1700                 1705                 1710
   Phe Trp  Arg Met Leu Trp Glu  His Asn Ser Thr Ile  Ile Val Met
       1715                 1720                 1725
   Leu Thr  Lys Leu Arg Glu Met  Gly Arg Glu Lys Cys  His Gln Tyr
       1730                 1735                 1740
   Trp Pro  Ala Glu Arg Ser Ala  Arg Tyr Gln Tyr Phe  Val Val Asp
       1745                 1750                 1755
   Pro Met  Ala Glu Tyr Asn Met  Pro Gln Tyr Ile Leu  Arg Glu Phe
       1760                 1765                 1770
   Lys Val  Thr Asp Ala Arg Asp  Gly Gln Ser Arg Thr  Ile Arg Gln
       1775                 1780                 1785
   Phe Gln  Phe Thr Asp Trp Pro  Glu Gln Gly Val Pro  Lys Thr Gly
       1790                 1795                 1800
   Glu Gly  Phe Ile Asp Phe Ile  Gly Gln Val His Lys  Thr Lys Glu
       1805                 1810                 1815
   Gln Phe  Gly Gln Asp Gly Pro  Ile Thr Val His Cys  Ser Ala Gly
       1820                 1825                 1830
   Val Gly  Arg Thr Gly Val Phe  Ile Thr Leu Ser Ile  Val Leu Glu
       1835                 1840                 1845
   Arg Met  Arg Tyr Glu Gly Val  Val Asp Met Phe Gln  Thr Val Lys
       1850                 1855                 1860
   Thr Leu  Arg Thr Gln Arg Pro  Ala Met Val Gln Thr  Glu Asp Gln
       1865                 1870                 1875
   Tyr Gln Leu Cys Tyr Arg Ala Ala Leu Glu Tyr Leu Gly Ser Phe
   1880 1885 1890
   Asp His Tyr Ala Thr
   1895
<210> 49
   <211> 22
   <212> PRT
   <213> Homo Sapiens
<400> 49
   Ala Ala Ala Ala Ala Ala Ala Ala Ala Ala Ala Ala Ala Ala Ala Gly
   1 5 10 15
   Ala Gly Ala Gly Ala Lys
   20
<210> 50
   <211> 18
   <212> PRT
   <213> Homo Sapiens
<400> 50
   Ala Ala Gly Thr Glu Gly Pro Phe Gln Glu Val Asp Gly Val Ala Thr
   1 5 10 15
   Thr Arg
<210> 51
   <211> 14
   <212> PRT
   <213> Homo Sapiens
<400> 51
   Ala Ala Leu Glu Tyr Leu Gly Ser Phe Asp His Tyr Ala Thr
   1 5 10
<210> 52
   <211> 15
   <212> PRT
   <213> Homo Sapiens
<400> 52
   Ala Ala Asn Asp Pro Phe Thr Ile Val His Gly Asn Thr Gly Lys
   1 5 10 15
<210> 53
   <211> 19
   <212> PRT
   <213> Homo Sapiens
<400> 53
   Ala Ala Ser Ala Gly Gln Glu Pro Leu His Asn Glu Glu Leu Ala Gly
   1 5 10 15
   Ala Gly Arg
<210> 54
   <211> 8
   <212> PRT
   <213> Homo Sapiens
<400> 54
   Ala Ala Thr Tyr His Val Asp Arg
   1 5
<210> 55
   <211> 20
   <212> PRT
   <213> Homo Sapiens
<400> 55
   Ala Cys Phe Ala Leu Leu Trp Gly Cys Ala Leu Ala Ala Ala Ala Ala
   1 5 10 15
   Ala Gln Gly Lys
   20
<210> 56
   <211> 20
   <212> PRT
   <213> Homo Sapiens
<400> 56
   Ala Cys Asn Pro Leu Asp Ala Gly Pro Met Val Val His Cys Ser Ala
   1 5 10 15
   Gly Val Gly Arg
   20
<210> 57
   <211> 8
   <212> PRT
   <213> Homo Sapiens
<400> 57
   Ala Asp Ala Ala Pro Asp Glu Lys
   1 5
<210> 58
   <211> 13
   <212> PRT
   <213> Homo Sapiens
<400> 58
   Ala Asp Glu Ala Arg Pro Asn Thr Ile Asp Phe Gly Lys
   1               5                   10
<210> 59
   <211> 7
   <212> PRT
   <213> Homo Sapiens
<400> 59
   Ala Asp Phe Leu Ile Phe Arg
   1               5
<210> 60
   <211> 15
   <212> PRT
   <213> Homo Sapiens
<400> 60
   Ala Asp Phe Val Leu Ala Ala Asn Ser Tyr Asp Leu Ala Ile Lys
   1               5                   10                  15
<210> 61
   <211> 11
   <212> PRT
   <213> Homo Sapiens
<400> 61
   Ala Asp Gly Ile Ser Ser Thr Phe Ser Gln Arg
   1               5                   10
<210> 62
   <211> 13
   <212> PRT
   <213> Homo Sapiens
<400> 62
   Ala Glu Leu Asp Asp Ser Asp Gly His Gly Asn His Arg
   1               5                   10
<210> 63
   <211> 17
   <212> PRT
   <213> Homo Sapiens
<400> 63
   Ala Glu Pro Glu Ser Glu Thr Ser Ile Leu Leu Ser Trp Thr Pro Pro
   1 5 10 15
   Arg
<210> 64
   <211> 6
   <212> PRT
   <213> Homo Sapiens
<400> 64
   Ala Glu Trp Leu Asn Lys
   1 5
<210> 65
   <211> 24
   <212> PRT
   <213> Homo Sapiens
<400> 65
   Ala Phe Gly Ala Pro Val Pro Ser Val Gln Trp Leu Asp Glu Asp Gly
   1 5 10 15
   Thr Thr Val Leu Gln Asp Glu Arg
   20
<210> 66
   <211> 9
   <212> PRT
   <213> Homo Sapiens
<400> 66
   Ala Phe Met Ile Ile Gln Glu Gln Arg
   1 5
<210> 67
   <211> 21
   <212> PRT
   <213> Homo Sapiens
<400> 67
   Ala Phe Ser Ser Asp Leu Ile Ser Ile His Ser Leu Ala Asp Val Glu
   1 5 10 15
   Val Val Val Thr Lys
   20
<210> 68
   <211> 10
   <212> PRT
   <213> Homo Sapiens
<400> 68
   Ala Phe Val Asn Cys Asp Glu Asn Ser Arg
   1               5                   10
<210> 69
   <211> 12
   <212> PRT
   <213> Homo Sapiens
<400> 69
   Ala Phe Tyr Ala Pro Val His Ala Asp Asp Leu Arg
   1               5                   10
<210> 70
   <211> 15
   <212> PRT
   <213> Homo Sapiens
<400> 70
   Ala Gly Glu Ile Thr Ser Asp Gly Leu Ser Phe Leu Phe Leu Lys
   1               5                   10                  15
<210> 71
   <211> 9
   <212> PRT
   <213> Homo Sapiens
<400> 71
   Ala Gly Leu Gly Glu Glu Phe Glu Lys
   1               5
<210> 72
   <211> 17
   <212> PRT
   <213> Homo Sapiens
<400> 72
   Ala His Thr Asp Val Gly Pro Gly Pro Glu Ser Ser Pro Val Leu Val
   1               5                   10                  15
   Arg
<210> 73
   <211> 7
   <212> PRT
   <213> Homo Sapiens
<400> 73
   Ala Ile Asn Asp Gly Phe Arg
   1               5
<210> 74
   <211> 12
   <212> PRT
   <213> Homo Sapiens
<400> 74
   Ala Ile Ser Thr Asn Ser Glu Leu Ser Thr Phe Arg
   1 5 10
<210> 75
   <211> 10
   <212> PRT
   <213> Homo Sapiens
<400> 75
   Ala Leu Ala Leu Leu Glu Asp Glu Glu Arg
   1 5 10
<210> 76
   <211> 14
   <212> PRT
   <213> Homo Sapiens
<400> 76
   Ala Leu Gly Arg Pro Gly Pro Pro Phe Asn Ile Thr Pro Arg
   1 5 10
<210> 77
   <211> 8
   <212> PRT
   <213> Homo Sapiens
<400> 77
   Ala Leu Tyr Gln Leu Gly Met Arg
   1 5
<210> 78
   <211> 8
   <212> PRT
   <213> Homo Sapiens
<400> 78
   Ala Met Ala Thr Leu Leu Ser Lys
   1 5
<210> 79
   <211> 26
   <212> PRT
   <213> Homo Sapiens
<400> 79
   Ala Asn Leu Thr Asn Phe Pro Glu Asn Gly Thr Phe Val Val Asn Ile
   1               5                   10                  15
   Ala Gln Leu Ser Gln Asp Asp Ser Gly Arg
               20                  25
<210> 80
   <211> 28
   <212> PRT
   <213> Homo Sapiens
<400> 80
   Ala Pro Gln Asp Pro Ala Ser Met Pro Cys Thr Arg Pro Pro Ser Ala
   1               5                   10                  15
   Pro His Tyr Leu Thr Ala Val Gly Met Gly Ala Lys
               20                  25
<210> 81
   <211> 8
   <212> PRT
   <213> Homo Sapiens
<400> 81
   Ala Gln Asp Pro Gly Glu Pro Arg
   1               5
<210> 82
   <211> 14
   <212> PRT
   <213> Homo Sapiens
<400> 82
   Ala Gln Leu Leu Val Val Gly Ser Pro Gly Pro Val Pro Arg
   1               5                   10
<210> 83
   <211> 16
   <212> PRT
   <213> Homo Sapiens
<400> 83
   Ala Gln Pro Pro Glu Ala Gly Pro Gln Gly Leu His Asp Leu Gly Arg
   1               5                   10                  15
<210> 84
   <211> 13
   <212> PRT
   <213> Homo Sapiens
<400> 84
   Ala Gln Gln Glu Phe Ala Thr Gly Val Met Ser Asn Lys
   1 5 10
<210> 85
   <211> 6
   <212> PRT
   <213> Homo Sapiens
<400> 85
   Ala Gln Tyr Glu Gly Arg
   1 5
<210> 86
   <211> 16
   <212> PRT
   <213> Homo Sapiens
<400> 86
   Ala Ser Val Asp Ser Gly Ser Ser Glu Glu Gln Gly Gly Ser Ser Arg
   1 5 10 15
<210> 87
   <211> 15
   <212> PRT
   <213> Homo Sapiens
<400> 87
   Ala Ser Val Ser Phe Leu Asn Phe Asn Leu Ser Asn Cys Glu Arg
   1 5 10 15
<210> 88
   <211> 10
   <212> PRT
   <213> Homo Sapiens
<400> 88
   Ala Thr Asp Phe Asp Val Leu Ser Tyr Lys
   1 5 10
<210> 89
   <211> 11
   <212> PRT
   <213> Homo Sapiens
<400> 89
   Ala Thr Asp Phe Asp Val Leu Ser Tyr Lys Lys
   1 5 10
<210> 90
   <211> 10
   <212> PRT
   <213> Homo Sapiens
<400> 90
   Ala Thr Gly Phe Pro Glu Pro Asn Pro Arg
   1               5                   10
<210> 91
   <211> 7
   <212> PRT
   <213> Homo Sapiens
<400> 91
   Ala Thr Leu Gln Leu Ser Arg
   1               5
<210> 92
   <211> 8
   <212> PRT
   <213> Homo Sapiens
<400> 92
   Ala Thr Asn Ser Met Ile Asp Arg
   1               5
<210> 93
   <211> 18
   <212> PRT
   <213> Homo Sapiens
<400> 93
   Ala Thr Gln Phe Thr Gly Ala Thr Gly Ala Ile Met Thr Thr Glu Thr
   1               5                   10                  15
   Thr Lys
<210> 94
   <211> 17
   <212> PRT
   <213> Homo Sapiens
<400> 94
   Ala Val Asp Glu Gln Gly Trp Glu Tyr Ser Ile Thr Ile Pro Pro Glu
   1               5                   10                  15
   Arg
<210> 95
   <211> 13
   <212> PRT
   <213> Homo Sapiens
<400> 95
   Ala Val Gly Phe Gly Gly Asp Phe Asp Gly Val Pro Arg
   1 5 10
<210> 96
   <211> 9
   <212> PRT
   <213> Homo Sapiens
<400> 96
   Ala Val Ile Phe Thr Gln Val Ser Arg
   1 5
<210> 97
   <211> 21
   <212> PRT
   <213> Homo Sapiens
<400> 97
   Ala Val Ile Ser Gln Phe Gln Arg Pro Ser Thr Gln Phe Ser Leu Met
   1 5 10 15
   Gln Phe Ser Asn Lys
   20
<210> 98
   <211> 18
   <212> PRT
   <213> Homo Sapiens
<400> 98
   Ala Tyr Ile Ala Thr Gln Gly Pro Leu Ala Glu Ser Thr Glu Asp Phe
   1 5 10 15
   Trp Arg
<210> 99
   <211> 9
   <212> PRT
   <213> Homo Sapiens
<400> 99
   Ala Tyr Ile Phe Ser Ile Asp Glu Lys
   1 5
<210> 100
   <211> 17
   <212> PRT
   <213> Homo Sapiens
<400> 100
   Cys Asp Val Pro Ser Phe Ser Val Gln Glu Glu Leu Asp Phe Thr Leu
   1 5 10 15
   Lys
<210> 101
   <211> 12
   <212> PRT
   <213> Homo Sapiens
<400> 101
   Cys Glu Ala Ser Gly Lys Pro Glu Val Gln Phe Arg
   1 5 10
<210> 102
   <211> 11
   <212> PRT
   <213> Homo Sapiens
<400> 102
   Cys Glu His His Ser Leu Tyr Gly Ala Ala Arg
   1 5 10
<210> 103
   <211> 25
   <212> PRT
   <213> Homo Sapiens
<400> 103
   Cys His Ile Gln Ala Ser Gly Arg Pro Gln Cys Ser Cys Met Pro Gly
   1 5 10 15
   Trp Thr Gly Glu Gln Cys Gln Leu Arg
   20 25
<210> 104
   <211> 7
   <212> PRT
   <213> Homo Sapiens
<400> 104
   Cys Ile Ile Trp Asn Thr Arg
   1 5
<210> 105
   <211> 11
   <212> PRT
   <213> Homo Sapiens
<400> 105
   Cys Leu Ala Glu Asn Ser Leu Gly Ser Ala Arg
   1 5 10
<210> 106
   <211> 13
   <212> PRT
   <213> Homo Sapiens
<400> 106
   Cys Met Leu Asn Ile Trp Gly Val Met Leu Phe Ile Arg
   1 5 10
<210> 107
   <211> 10
   <212> PRT
   <213> Homo Sapiens
<400> 107
   Cys Gln Gly Gln Leu Glu Val Tyr Leu Lys
   1 5 10
<210> 108
   <211> 13
   <212> PRT
   <213> Homo Sapiens
<400> 108
   Cys Ser Met Leu Ile Ala Ser Asn Glu Thr Trp Lys Lys
   1 5 10
<210> 109
   <211> 10
   <212> PRT
   <213> Homo Sapiens
<400> 109
   Asp Ala Thr Gln Ile Thr Gln Gly Pro Arg
   1 5 10
<210> 110
   <211> 11
   <212> PRT
   <213> Homo Sapiens
<400> 110
   Asp Ala Val Val Thr Tyr Thr Ala Glu Ser Lys
   1 5 10
<210> 111
   <211> 12
   <212> PRT
   <213> Homo Sapiens
<400> 111
   Asp Asp Gln His Phe Thr Val Thr Gly Leu His Lys
   1 5 10
<210> 112
   <211> 21
   <212> PRT
   <213> Homo Sapiens
<400> 112
   Asp Glu Ala Ile Tyr Glu Cys Thr Ala Thr Asn Ser Leu Gly Glu Ile
   1 5 10 15
   Asn Thr Ser Ala Lys
   20
<210> 113
   <211> 14
   <212> PRT
   <213> Homo Sapiens
<400> 113
   Asp Glu Glu Glu Glu Pro Pro Ser Met Thr Gln Leu Leu Arg
   1 5 10
<210> 114
   <211> 23
   <212> PRT
   <213> Homo Sapiens
<400> 114
   Asp Glu Gly Pro Ala Ala Ala Gly Asp Gly Leu Gly Arg Pro Leu Gly
   1 5 10 15
   Pro Thr Pro Ser Gln Ser Arg
   20
<210> 115
   <211> 12
   <212> PRT
   <213> Homo Sapiens
<400> 115
   Asp Glu Gln His Gln Cys Ser Leu Gly Asn Leu Lys
   1 5 10
<210> 116
   <211> 20
   <212> PRT
   <213> Homo Sapiens
<400> 116
   Asp Phe Leu Leu Gln Ser Ser Thr Val Ala Ala Glu Ala Gln Asp Gly
   1               5                   10                  15
   Pro Gln Glu Ala
               20
<210> 117
   <211> 13
   <212> PRT
   <213> Homo Sapiens
<400> 117
   Asp Phe Leu Pro Val Asp Pro Ala Thr Ser Asn Gly Arg
   1               5                   10
<210> 118
   <211> 13
   <212> PRT
   <213> Homo Sapiens
<400> 118
   Asp Phe Leu Pro Val Asp Thr Ser Asn Asn Asn Gly Arg
   1               5                   10
<210> 119
   <211> 12
   <212> PRT
   <213> Homo Sapiens
<400> 119
   Asp Phe Tyr Asn Pro Val Val Pro Glu Ala Gln Lys
   1               5                   10
<210> 120
   <211> 13
   <212> PRT
   <213> Homo Sapiens
<400> 120
   Asp Phe Tyr Asn Pro Val Val Pro Glu Ala Gln Lys Arg
   1               5                   10
<210> 121
   <211> 14
   <212> PRT
   <213> Homo Sapiens
<400> 121
   Asp Gly Glu Phe Ser Val Leu Gln Leu Val Gly Met Leu Arg
   1               5                   10
<210> 122
   <211> 16
   <212> PRT
   <213> Homo Sapiens
<400> 122
   Asp Gly His Gly Thr Ala Ile Ser Asn Ala Ser Asp Val Trp Lys Lys
   1 5 10 15
<210> 123
   <211> 12
   <212> PRT
   <213> Homo Sapiens
<400> 123
   Asp Gly Asn Gly Glu Val Thr Asp Lys Pro Val Lys
   1 5 10
<210> 124
   <211> 11
   <212> PRT
   <213> Homo Sapiens
<400> 124
   Asp Gly Pro Glu Phe Thr Phe Thr Thr Pro Lys
   1 5 10
<210> 125
   <211> 12
   <212> PRT
   <213> Homo Sapiens
<400> 125
   Asp Gly Ser Phe Ser Val Val Ile Thr Gly Leu Arg
   1 5 10
<210> 126
   <211> 8
   <212> PRT
   <213> Homo Sapiens
<400> 126
   Asp Gly Val His Phe Lys Pro Lys
   1 5
<210> 127
   <211> 16
   <212> PRT
   <213> Homo Sapiens
<400> 127
   Asp His Pro Pro Ile Pro Ile Thr Asp Leu Ala Asp Asn Ile Glu Arg
   1 5 10 15
<210> 128
   <211> 33
   <212> PRT
   <213> Homo Sapiens
<400> 128
   Asp His Val Val Val Pro Ala Asn Thr Thr Ser Val Ile Leu Ser Gly
   1 5 10 15
   Leu Arg Pro Tyr Ser Ser Tyr His Leu Glu Val Gln Ala Phe Asn Gly
   20 25 30
   Arg
<210> 129
   <211> 16
   <212> PRT
   <213> Homo Sapiens
<400> 129
   Asp Ile Asn Ser Gln Gln Glu Leu Gln Asn Ile Thr Thr Asp Thr Arg
   1 5 10 15
<210> 130
   <211> 7
   <212> PRT
   <213> Homo Sapiens
<400> 130
   Asp Ile Gln Asn Gln Leu Lys
   1 5
<210> 131
   <211> 11
   <212> PRT
   <213> Homo Sapiens
<400> 131
   Asp Ile Gln Val Ala Ser Asn Glu Ile Leu Arg
   1 5 10
<210> 132
   <211> 7
   <212> PRT
   <213> Homo Sapiens
<400> 132
   Asp Leu Ala Ala Met Asp Lys
   1               5
<210> 133
   <211> 10
   <212> PRT
   <213> Homo Sapiens
<400> 133
   Asp Leu Ala Val Phe Leu Tyr His Leu Arg
   1               5                   10
<210> 134
   <211> 14
   <212> PRT
   <213> Homo Sapiens
<400> 134
   Asp Leu Leu Pro Glu Asp Phe Val Val Tyr Thr Tyr Asn Lys
   1               5                   10
<210> 135
   <211> 10
   <212> PRT
   <213> Homo Sapiens
<400> 135
   Asp Leu Pro Leu Leu Ile Glu Asn Met Lys
   1               5                   10
<210> 136
   <211> 9
   <212> PRT
   <213> Homo Sapiens
<400> 136
   Asp Leu Pro Pro Ile Leu Leu Val Arg
   1               5
<210> 137
   <211> 10
   <212> PRT
   <213> Homo Sapiens
<400> 137
   Asp Leu Gln Glu Leu Gly Asp Ser Asp Lys
   1               5                   10
<210> 138
   <211> 16
   <212> PRT
   <213> Homo Sapiens
<400> 138
   Asp Leu Gln Ser Ser Val Thr Leu Asp Leu Ala Leu Asp Pro Gly Arg
   1 5 10 15
<210> 139
   <211> 9
   <212> PRT
   <213> Homo Sapiens
<400> 139
   Asp Leu Ser Gln Met Glu Ala Leu Lys
   1 5
<210> 140
   <211> 10
   <212> PRT
   <213> Homo Sapiens
<400> 140
   Asp Leu Ser Gln Met Glu Ala Leu Lys Arg
   1 5 10
<210> 141
   <211> 10
   <212> PRT
   <213> Homo Sapiens
<400> 141
   Asp Leu Val Glu Pro Trp Val Val Val Arg
   1 5 10
<210> 142
   <211> 18
   <212> PRT
   <213> Homo Sapiens
<400> 142
   Asp Met Ala Gly Ala Gln Ala Ala Ala Val Ala Leu Asn Glu Glu Phe
   1 5 10 15
   Leu Arg
<210> 143
   <211> 13
   <212> PRT
   <213> Homo Sapiens
<400> 143
   Asp Met Glu Pro Leu Thr Thr Leu Glu Phe Ser Glu Lys
   1 5 10
<210> 144
   <211> 25
   <212> PRT
   <213> Homo Sapiens
<400> 144
   Asp Asn Ile Val Ser Tyr Asn Asp Glu Gly Gly Gly Glu Glu Asp Thr
   1 5 10 15
   Gln Ala Phe Asp Ile Gly Thr Leu Arg
   20 25
<210> 145
   <211> 12
   <212> PRT
   <213> Homo Sapiens
<400> 145
   Asp Asn Ile Tyr Pro Ala Phe Gln Met Phe Ala Lys
   1 5 10
<210> 146
   <211> 12
   <212> PRT
   <213> Homo Sapiens
<400> 146
   Asp Asn Met Leu Trp Val Glu Trp Thr Thr Pro Arg
   1 5 10
<210> 147
   <211> 11
   <212> PRT
   <213> Homo Sapiens
<400> 147
   Asp Asn Gln Trp Leu Gly Val Thr Leu Ser Arg
   1 5 10
<210> 148
   <211> 14
   <212> PRT
   <213> Homo Sapiens
<400> 148
   Asp Asn Trp Ile Ser Val Asp Ser Val Thr Ser Glu Ile Lys
   1 5 10
<210> 149
   <211> 9
   <212> PRT
   <213> Homo Sapiens
<400> 149
   Asp Pro Ser Val Thr Gln Val Thr Arg
   1 5
<210> 150
   <211> 7
   <212> PRT
   <213> Homo Sapiens
<400> 150
   Asp Gln Ala Asp Gly Ser Arg
   1 5
<210> 151
   <211> 8
   <212> PRT
   <213> Homo Sapiens
<400> 151
   Asp Gln Phe Asp Ile Cys Ala Lys
   1 5
<210> 152
   <211> 5
   <212> PRT
   <213> Homo Sapiens
<400> 152
   Asp Gln Leu Trp Arg
   1 5
<210> 153
   <211> 10
   <212> PRT
   <213> Homo Sapiens
<400> 153
   Asp Gln Val Ala Cys Leu Thr Phe Phe Lys
   1 5 10
<210> 154
   <211> 10
   <212> PRT
   <213> Homo Sapiens
<400> 154
   Asp Gln Val Asn Thr Val Gly Ile Pro Ile
   1 5 10
<210> 155
   <211> 18
   <212> PRT
   <213> Homo Sapiens
<400> 155
   Asp Ser Phe Ser Asp Pro Tyr Ala Ile Val Ser Phe Leu His Gln Ser
   1 5 10 15
   Gln Lys
<210> 156
   <211> 14
   <212> PRT
   <213> Homo Sapiens
<400> 156
   Asp Ser Leu Leu Ala His Ser Ser Asp Pro Val Glu Met Arg
   1 5 10
<210> 157
   <211> 15
   <212> PRT
   <213> Homo Sapiens
<400> 157
   Asp Ser Leu Leu Ala His Ser Ser Asp Pro Val Glu Met Arg Arg
   1 5 10 15
<210> 158
   <211> 14
   <212> PRT
   <213> Homo Sapiens
<400> 158
   Asp Ser Tyr Leu Gly Tyr Ser Thr Glu Leu Ala Leu Trp Lys
   1 5 10
<210> 159
   <211> 14
   <212> PRT
   <213> Homo Sapiens
<400> 159
   Asp Thr Gly Glu Ile Tyr Thr Thr Ser Val Thr Leu Asp Arg
   1 5 10
<210> 160
   <211> 13
   <212> PRT
   <213> Homo Sapiens
<400> 160
   Asp Thr Gly Glu Leu Asn Val Thr Ser Ile Leu Asp Arg
   1 5 10
<210> 161
   <211> 13
   <212> PRT
   <213> Homo Sapiens
<400> 161
   Asp Val Ile Pro Met Ala Asp Ala Ala Gly Ile Ile Arg
   1 5 10
<210> 162
   <211> 8
   <212> PRT
   <213> Homo Sapiens
<400> 162
   Asp Val Ser Ala Ile Met Asn Lys
   1 5
<210> 163
   <211> 10
   <212> PRT
   <213> Homo Sapiens
<400> 163
   Asp Val Val Val Ser Val Glu Tyr Ser Lys
   1 5 10
<210> 164
   <211> 8
   <212> PRT
   <213> Homo Sapiens
<400> 164
   Asp Trp Leu Gln Ala Asp Met Arg
   1 5
<210> 165
   <211> 10
   <212> PRT
   <213> Homo Sapiens
<400> 165
   Asp Tyr Val Leu His Met Pro Thr Leu Arg
   1 5 10
<210> 166
   <211> 13
   <212> PRT
   <213> Homo Sapiens
<400> 166
   Glu Ala Asp Leu Thr Glu Glu Thr Glu Glu Asn Leu Arg
   1 5 10
<210> 167
   <211> 24
   <212> PRT
   <213> Homo Sapiens
<400> 167
   Glu Ala Ser Val His Ile Gln Leu Glu Gly Arg Pro Ser Ile Leu Glu
   1 5 10 15
   Met Asp Glu Thr Ser Ala Leu Lys
   20
<210> 168
   <211> 21
   <212> PRT
   <213> Homo Sapiens
<400> 168
   Glu Asp Ala Gln Ile Asn Thr Thr Ile Gly Ser Val Thr Ala Gln Asp
   1 5 10 15
   Pro Asp Ala Ala Arg
   20
<210> 169
   <211> 15
   <212> PRT
   <213> Homo Sapiens
<400> 169
   Glu Asp Gly Tyr Ser Asp Ala Ser Gly Phe Gly Tyr Cys Phe Arg
   1 5 10 15
<210> 170
   <211> 13
   <212> PRT
   <213> Homo Sapiens
<400> 170
   Glu Asp Thr Gln Val Asp Ser Glu Ala Arg Pro Met Lys
   1 5 10
<210> 171
   <211> 14
   <212> PRT
   <213> Homo Sapiens
<400> 171
   Glu Glu Phe Val Ala Thr Thr Glu Ser Thr Thr Glu Thr Lys
   1               5                   10
<210> 172
   <211> 12
   <212> PRT
   <213> Homo Sapiens
<400> 172
   Glu Glu Gly Val Phe Thr Val Thr Pro Asp Thr Lys
   1               5                   10
<210> 173
   <211> 18
   <212> PRT
   <213> Homo Sapiens
<400> 173
   Glu Glu His Glu Val Ala Val Leu Gly Ala Pro Pro Ser Thr Ile Leu
   1               5                   10                  15
   Pro Arg
<210> 174
   <211> 13
   <212> PRT
   <213> Homo Sapiens
<400> 174
   Glu Glu His Ser Ser Tyr Thr Leu Thr Val Glu Ala Arg
   1               5                   10
<210> 175
   <211> 7
   <212> PRT
   <213> Homo Sapiens
<400> 175
   Glu Glu Ile Gly Asn Leu Lys
   1               5
<210> 176
   <211> 10
   <212> PRT
   <213> Homo Sapiens
<400> 176
   Glu Glu Ile Asn Lys Pro Pro Ile Ala Lys
   1 5 10
<210> 177
   <211> 8
   <212> PRT
   <213> Homo Sapiens
<400> 177
   Glu Glu Ile Asn Gln Gly Gly Arg
   1 5
<210> 178
   <211> 28
   <212> PRT
   <213> Homo Sapiens
<400> 178
   Glu Glu Leu Gly Val Thr Val Tyr Gln Ser Pro His Ser Gly Ser Phe
   1 5 10 15
   Thr Ile Thr Gly Asn Asn Ser Asn Phe Ala Gln Arg
   20 25
<210> 179
   <211> 20
   <212> PRT
   <213> Homo Sapiens
<400> 179
   Glu Glu Met Ile Leu Leu Ala Asn Tyr Leu Asp Ser Met Tyr Ile Met
   1 5 10 15
   Leu Asn Ile Arg
   20
<210> 180
   <211> 6
   <212> PRT
   <213> Homo Sapiens
<400> 180
   Glu Glu Gln Ala Gln Arg
   1 5
<210> 181
   <211> 5
   <212> PRT
   <213> Homo Sapiens
<400> 181
   Glu Glu Arg Lys Arg
   1 5
<210> 182
   <211> 16
   <212> PRT
   <213> Homo Sapiens
<400> 182
   Glu Glu Thr Pro Phe Phe Leu Leu Thr Gly Tyr Ala Leu Asp Ala Arg
   1 5 10 15
<210> 183
   <211> 13
   <212> PRT
   <213> Homo Sapiens
<400> 183
   Glu Phe Ile Tyr Leu Arg Pro Phe Ala Cys Asp Thr Lys
   1 5 10
<210> 184
   <211> 19
   <212> PRT
   <213> Homo Sapiens
<400> 184
   Glu Phe Leu Ser Glu Ala Ser Ile Met Gly Gln Phe Glu His Pro Asn
   1 5 10 15
   Ile Ile Arg
<210> 185
   <211> 9
   <212> PRT
   <213> Homo Sapiens
<400> 185
   Glu Phe Asn Gln Phe Ala Glu Gly Lys
   1 5
<210> 186
   <211> 14
   <212> PRT
   <213> Homo Sapiens
<400> 186
   Glu Gly Ala Gln Tyr Leu Met Gln Ala Ala Gly Leu Gly Arg
   1 5 10
<210> 187
   <211> 8
   <212> PRT
   <213> Homo Sapiens
<400> 187
   Glu Gly Glu Asp Leu Ser Lys Lys
   1 5
<210> 188
   <211> 16
   <212> PRT
   <213> Homo Sapiens
<400> 188
   Glu Gly Leu Ala His Leu Ile Gln Ser Cys Gly Leu Gly Gly Met Arg
   1 5 10 15
<210> 189
   <211> 9
   <212> PRT
   <213> Homo Sapiens
<400> 189
   Glu Gly Pro Gly Glu Ala Ile Val Arg
   1 5
<210> 190
   <211> 11
   <212> PRT
   <213> Homo Sapiens
<400> 190
   Glu His Ala Leu Ala Gln Ala Glu Leu Leu Lys
   1 5 10
<210> 191
   <211> 12
   <212> PRT
   <213> Homo Sapiens
<400> 191
   Glu His Glu Glu Ala Glu Ser Gly Glu Gly Thr Arg
   1 5 10
<210> 192
   <211> 14
   <212> PRT
   <213> Homo Sapiens
<400> 192
   Glu His Glu Glu Ala Glu Ser Gly Glu Gly Thr Arg Arg Arg
   1 5 10
<210> 193
   <211> 7
   <212> PRT
   <213> Homo Sapiens
<400> 193
   Glu His Glu Glu Tyr Leu Lys
   1 5
<210> 194
   <211> 10
   <212> PRT
   <213> Homo Sapiens
<400> 194
   Glu His Gln Pro Leu Pro Ile Gln Trp Lys
   1 5 10
<210> 195
   <211> 17
   <212> PRT
   <213> Homo Sapiens
<400> 195
   Glu His Ser Ser Thr Ala Asn Ile Ile Val Met Ser Leu Pro Val Ala
   1 5 10 15
   Arg
<210> 196
   <211> 12
   <212> PRT
   <213> Homo Sapiens
<400> 196
   Glu His Ser Ser Trp Asp Leu Val Gly Leu Glu Lys
   1 5 10
<210> 197
   <211> 11
   <212> PRT
   <213> Homo Sapiens
<400> 197
   Glu Ile Ile Asp Glu Asn Phe Ala Glu Leu Lys
   1 5 10
<210> 198
   <211> 14
   <212> PRT
   <213> Homo Sapiens
<400> 198
   Glu Ile Pro Ser His His Pro Thr Asp Pro Val Glu Leu Arg
   1 5 10
<210> 199
   <211> 15
   <212> PRT
   <213> Homo Sapiens
<400> 199
   Glu Ile Pro Ser His His Pro Thr Asp Pro Val Glu Leu Arg Arg
   1 5 10 15
<210> 200
   <211> 8
   <212> PRT
   <213> Homo Sapiens
<400> 200
   Glu Ile Thr Tyr Gln Gly Thr Arg
   1 5
<210> 201
   <211> 18
   <212> PRT
   <213> Homo Sapiens
<400> 201
   Glu Lys Pro Ala Ile His His Ile Pro Gly Phe Glu Val Gln Glu Thr
   1 5 10 15
   Ser Arg
<210> 202
   <211> 14
   <212> PRT
   <213> Homo Sapiens
<400> 202
   Glu Leu Ala Gln Ser Gln Glu Ala Leu Gln Val Glu Gln Arg
   1 5 10
<210> 203
   <211> 8
   <212> PRT
   <213> Homo Sapiens
<400> 203
   Glu Leu Leu Gly Asn Asn Ile Pro
   1 5
<210> 204
   <211> 16
   <212> PRT
   <213> Homo Sapiens
<400> 204
   Glu Leu Asn Asp Ile Ala Ser Lys Pro Ser Gln Glu His Ile Phe Lys
   1 5 10 15
<210> 205
   <211> 9
   <212> PRT
   <213> Homo Sapiens
<400> 205
   Glu Leu Asn Ile Leu His Glu Met Lys
   1 5
<210> 206
   <211> 10
   <212> PRT
   <213> Homo Sapiens
<400> 206
   Glu Leu Pro Gly Glu Leu Leu Gly Tyr Arg
   1 5 10
<210> 207
   <211> 15
   <212> PRT
   <213> Homo Sapiens
<400> 207
   Glu Leu Thr Leu Pro Val Asp Ser Thr Thr Leu Asp Gly Ser Lys
   1 5 10 15
<210> 208
   <211> 15
   <212> PRT
   <213> Homo Sapiens
<400> 208
   Glu Leu Thr Tyr Ser Asn Phe His Pro Leu Leu Val Ser Gly Arg
   1 5 10 15
<210> 209
   <211> 13
   <212> PRT
   <213> Homo Sapiens
<400> 209
   Glu Leu Val His Ile Lys Pro Asp Gln Ser Asn Val Arg
   1 5 10
<210> 210
   <211> 25
   <212> PRT
   <213> Homo Sapiens
<400> 210
   Glu Met Met Glu Glu Ala Asn Gly Gln Ile Ala Pro Glu Asn Gly Thr
   1 5 10 15
   Gln Thr Pro Ser Pro Pro Ser Glu Lys
   20 25
<210> 211
   <211> 10
   <212> PRT
   <213> Homo Sapiens
<400> 211
   Glu Met Gln Asn Leu Ser Gln His Gly Arg
   1 5 10
<210> 212
   <211> 11
   <212> PRT
   <213> Homo Sapiens
<400> 212
   Glu Met Gln Asn Leu Ser Gln His Gly Arg Lys
   1 5 10
<210> 213
   <211> 8
   <212> PRT
   <213> Homo Sapiens
<400> 213
   Glu Met Ser Ile Asp Gln Ala Lys
   1 5
<210> 214
   <211> 13
   <212> PRT
   <213> Homo Sapiens
<400> 214
   Glu Asn Gly Gly Ala Ser His Pro Leu Leu Asp Gln Arg
   1 5 10
<210> 215
   <211> 10
   <212> PRT
   <213> Homo Sapiens
<400> 215
   Glu Asn Pro Asp Asn Leu Ser Asp Phe Arg
   1 5 10
<210> 216
   <211> 17
   <212> PRT
   <213> Homo Sapiens
<400> 216
   Glu Pro Phe Glu Asp Gly Phe Ala Asn Gly Glu Glu Ser Thr Pro Thr
   1 5 10 15
   Arg
<210> 217
   <211> 6
   <212> PRT
   <213> Homo Sapiens
<400> 217
   Glu Pro Ile Asp Leu Arg
   1 5
<210> 218
   <211> 7
   <212> PRT
   <213> Homo Sapiens
<400> 218
   Glu Pro Met Asp Gln Lys Arg
   1 5
<210> 219
   <211> 19
   <212> PRT
   <213> Homo Sapiens
<400> 219
   Glu Gln Phe Gly Gln Asp Gly Pro Ile Ser Val His Cys Ser Ala Gly
   1 5 10 15
   Val Gly Arg
<210> 220
   <211> 19
   <212> PRT
   <213> Homo Sapiens
<400> 220
   Glu Gln Phe Gly Gln Asp Gly Pro Ile Thr Val His Cys Ser Ala Gly
   1               5                   10                  15
   Val Gly Arg
<210> 221
   <211> 10
   <212> PRT
   <213> Homo Sapiens
<400> 221
   Glu Gln Thr Pro Leu Phe Leu Ala Ala Arg
   1               5                   10
<210> 222
   <211> 13
   <212> PRT
   <213> Homo Sapiens
<400> 222
   Glu Arg Pro Pro Asp His Gln His Ser Ala Gln Val Lys
   1               5                   10
<210> 223
   <211> 30
   <212> PRT
   <213> Homo Sapiens
<400> 223
   Glu Ser Phe Leu Ala Pro Ser Ser Gly Val Gln Pro Thr Leu Ala Met
   1               5                   10                  15
   Pro Asn Ile Ala Val Gly Gln Asn Val Thr Val Thr Glu Arg
               20                  25                  30
<210> 224
   <211> 8
   <212> PRT
   <213> Homo Sapiens
<400> 224
   Glu Ser His Val Ala Met His Arg
   1               5
<210> 225
   <211> 12
   <212> PRT
   <213> Homo Sapiens
<400> 225
   Glu Ser His Trp Ile Gly Leu Thr Asp Ser Glu Arg
   1               5                   10
<210> 226
   <211> 8
   <212> PRT
   <213> Homo Sapiens
<400> 226
   Glu Ser Pro Ala Gln Ala Pro Ala
   1               5
<210> 227
   <211> 13
   <212> PRT
   <213> Homo Sapiens
<400> 227
   Glu Ser Ser Pro Phe Leu Ser Pro Leu Glu Ala Ser Arg
   1               5                   10
<210> 228
   <211> 10
   <212> PRT
   <213> Homo Sapiens
<400> 228
   Glu Thr Cys Val Asp Leu His Leu Pro Arg
   1               5                   10
<210> 229
   <211> 19
   <212> PRT
   <213> Homo Sapiens
<400> 229
   Glu Thr Asp Ser Leu Ser Ala Gly Phe Val Val Val Met Gly Val Asp
   1               5                   10                  15
   Leu Ser Arg
<210> 230
   <211> 8
   <212> PRT
   <213> Homo Sapiens
<400> 230
   Glu Thr Ile Glu Pro Ala Val Arg
   1 5
<210> 231
   <211> 10
   <212> PRT
   <213> Homo Sapiens
<400> 231
   Glu Thr Leu Gln Ser Glu Glu Gln Gln Arg
   1 5 10
<210> 232
   <211> 11
   <212> PRT
   <213> Homo Sapiens
<400> 232
   Glu Thr Leu Gln Ser Glu Glu Gln Gln Arg Arg
   1 5 10
<210> 233
   <211> 7
   <212> PRT
   <213> Homo Sapiens
<400> 233
   Glu Val Ile Thr Ile Tyr Ser
   1 5
<210> 234
   <211> 20
   <212> PRT
   <213> Homo Sapiens
<400> 234
   Glu Val Leu Ala Thr Pro Ser Leu Ser Ala Ser Phe Asn Ala Pro Leu
   1 5 10 15
   Leu Asp Thr Lys
   20
<210> 235
   <211> 18
   <212> PRT
   <213> Homo Sapiens
<400> 235
   Glu Val Pro Gly Tyr Ile Val Leu Phe Tyr Asn Met Ser Leu Asp Val
   1 5 10 15
   Asn Arg
<210> 236
   <211> 8
   <212> PRT
   <213> Homo Sapiens
<400> 236
   Glu Val Pro Ile Tyr Ala Asn Arg
   1 5
<210> 237
   <211> 10
   <212> PRT
   <213> Homo Sapiens
<400> 237
   Glu Val Pro Pro Ala Val Ser Asp Ile Arg
   1 5 10
<210> 238
   <211> 7
   <212> PRT
   <213> Homo Sapiens
<400> 238
   Glu Val Pro Val Ala Ile Lys
   1 5
<210> 239
   <211> 23
   <212> PRT
   <213> Homo Sapiens
<400> 239
   Glu Val Val Leu Leu Asp Phe Ala Ala Ala Gly Gly Glu Leu Gly Trp
   1 5 10 15
   Leu Thr His Pro Tyr Gly Lys
   20
<210> 240
   <211> 7
   <212> PRT
   <213> Homo Sapiens
<400> 240
   Glu Trp Gly Asp Gly Ile Arg
   1 5
<210> 241
   <211> 11
   <212> PRT
   <213> Homo Sapiens
<400> 241
   Glu Tyr Ser Ile Glu Glu Ile Glu Ala Gly Arg
   1 5 10
<210> 242
   <211> 10
   <212> PRT
   <213> Homo Sapiens
<400> 242
   Phe Ala Asp Ile Val Ser Ile Leu Asp Lys
   1 5 10
<210> 243
   <211> 21
   <212> PRT
   <213> Homo Sapiens
<400> 243
   Phe Ala Pro Gly Val Thr Ile Glu Glu Asp Asn Cys Cys Gly Cys Asn
   1 5 10 15
   Ala Ile Ala Ile Arg
   20
<210> 244
   <211> 9
   <212> PRT
   <213> Homo Sapiens
<400> 244
   Phe Ala Val Pro Thr Tyr Ala Ala Lys
   1 5
<210> 245
   <211> 19
   <212> PRT
   <213> Homo Sapiens
<400> 245
   Phe Cys Gln Ala Leu Gly Ala His Leu Ser Ser Phe Ser His Val Asp
   1               5                   10                  15
   Glu Ile Lys
<210> 246
   <211> 15
   <212> PRT
   <213> Homo Sapiens
<400> 246
   Phe Asp Leu Ser Met Pro His Val Gln Asp Pro Ser Leu Val Arg
   1               5                   10                  15
<210> 247
   <211> 11
   <212> PRT
   <213> Homo Sapiens
<400> 247
   Phe Glu Gln Glu Tyr Leu Asn Asp Leu Met Lys
   1               5                   10
<210> 248
   <211> 12
   <212> PRT
   <213> Homo Sapiens
<400> 248
   Phe Glu Gln Glu Tyr Leu Asn Asp Leu Met Lys Lys
   1               5                   10
<210> 249
   <211> 15
   <212> PRT
   <213> Homo Sapiens
<400> 249
   Phe Glu Val Ile Glu Phe Asp Asp Gly Ala Gly Ser Val Leu Arg
   1               5                   10                  15
<210> 250
   <211> 8
   <212> PRT
   <213> Homo Sapiens
<400> 250
   Phe Phe Ala Ser Ile Gly Glu Arg
   1               5
<210> 251
   <211> 12
   <212> PRT
   <213> Homo Sapiens
<400> 251
   Phe Phe Pro Tyr Ala Asn Gly Thr Leu Gly Ile Arg
   1 5 10
<210> 252
   <211> 15
   <212> PRT
   <213> Homo Sapiens
<400> 252
   Phe Gly Ala Ala Leu Thr Val Leu Gly Asp Val Asn Gly Asp Lys
   1 5 10 15
<210> 253
   <211> 6
   <212> PRT
   <213> Homo Sapiens
<400> 253
   Phe His Ile Leu Phe Lys
   1 5
<210> 254
   <211> 6
   <212> PRT
   <213> Homo Sapiens
<400> 254
   Phe His Leu Glu Tyr Arg
   1 5
<210> 255
   <211> 18
   <212> PRT
   <213> Homo Sapiens
<400> 255
   Phe Ile Lys Pro Trp Glu Ser Pro Asp Glu Met Glu Leu Asp Glu Leu
   1 5 10 15
   Leu Lys
<210> 256
   <211> 6
   <212> PRT
   <213> Homo Sapiens
<400> 256
   Phe Leu Cys Glu Val Lys
   1 5
<210> 257
   <211> 8
   <212> PRT
   <213> Homo Sapiens
<400> 257
   Phe Leu Asp Asp Leu Gly Leu Lys
   1 5
<210> 258
   <211> 18
   <212> PRT
   <213> Homo Sapiens
<400> 258
   Phe Leu Glu Glu Asn Ser Ser Asp Pro Thr Tyr Thr Ser Ser Leu Gly
   1 5 10 15
   Gly Lys
<210> 259
   <211> 8
   <212> PRT
   <213> Homo Sapiens
<400> 259
   Phe Leu Asn Leu Thr Ser Glu Lys
   1 5
<210> 260
   <211> 9
   <212> PRT
   <213> Homo Sapiens
<400> 260
   Phe Leu Pro Gly Gly Thr Leu Cys Arg
   1 5
<210> 261
   <211> 10
   <212> PRT
   <213> Homo Sapiens
<400> 261
   Phe Met Phe Asp Leu Phe Gln Gln Phe Arg
   1 5 10
<210> 262
   <211> 11
   <212> PRT
   <213> Homo Sapiens
<400> 262
   Phe Met Phe Asp Leu Phe Gln Gln Phe Arg Lys
   1 5 10
<210> 263
   <211> 10
   <212> PRT
   <213> Homo Sapiens
<400> 263
   Phe Pro Gln Val Val Ser Ala Leu Asp Lys
   1 5 10
<210> 264
   <211> 7
   <212> PRT
   <213> Homo Sapiens
<400> 264
   Phe Pro Ser Gly Thr Leu Arg
   1 5
<210> 265
   <211> 15
   <212> PRT
   <213> Homo Sapiens
<400> 265
   Phe Pro Val Thr Phe Gly Glu Glu Cys Leu Tyr Met Ser Ala Lys
   1 5 10 15
<210> 266
   <211> 6
   <212> PRT
   <213> Homo Sapiens
<400> 266
   Phe Gln Gly Ile Tyr Arg
   1 5
<210> 267
   <211> 6
   <212> PRT
   <213> Homo Sapiens
<400> 267
   Phe Gln Leu Ala Ala Arg
   1 5
<210> 268
   <211> 13
   <212> PRT
   <213> Homo Sapiens
<400> 268
   Phe Gln Leu Ser Asn Ser Gly Pro Asn Ser Thr Ile Lys
   1 5 10
<210> 269
   <211> 11
   <212> PRT
   <213> Homo Sapiens
<400> 269
   Phe Gln Thr His Phe Thr Phe Glu Glu Phe Arg
   1 5 10
<210> 270
   <211> 12
   <212> PRT
   <213> Homo Sapiens
<400> 270
   Phe Gln Thr His Phe Thr Phe Glu Glu Phe Arg Arg
   1 5 10
<210> 271
   <211> 12
   <212> PRT
   <213> Homo Sapiens
<400> 271
   Phe Gln Val Asp Leu Val Ser Glu Asn Ala Gly Arg
   1 5 10
<210> 272
   <211> 7
   <212> PRT
   <213> Homo Sapiens
<400> 272
   Phe Ser Asp Val Thr Gly Lys
   1 5
<210> 273
   <211> 16
   <212> PRT
   <213> Homo Sapiens
<400> 273
   Phe Ser Phe Asp Asp Phe Leu Gly Ser Leu Gln Leu Asp Leu Asn Arg
   1               5                   10                  15
<210> 274
   <211> 6
   <212> PRT
   <213> Homo Sapiens
<400> 274
   Phe Ser Ser Tyr Glu Lys
   1               5
<210> 275
   <211> 15
   <212> PRT
   <213> Homo Sapiens
<400> 275
   Phe Thr Leu Thr Gly Leu Lys Pro Asp Thr Thr Tyr Asp Ile Lys
   1               5                   10                  15
<210> 276
   <211> 11
   <212> PRT
   <213> Homo Sapiens
<400> 276
   Phe Thr Met Leu Glu Cys Leu Ser Leu Pro Arg
   1               5                   10
<210> 277
   <211> 7
   <212> PRT
   <213> Homo Sapiens
<400> 277
   Phe Thr Gln Asp Thr Phe Arg
   1               5
<210> 278
   <211> 12
   <212> PRT
   <213> Homo Sapiens
<400> 278
   Phe Thr Thr Glu Ile His Pro Ser Cys Val Thr Arg
   1               5                   10
<210> 279
   <211> 9
   <212> PRT
   <213> Homo Sapiens
<400> 279
   Phe Val Trp Asn Gly His Leu Leu Arg
   1 5
<210> 280
   <211> 19
   <212> PRT
   <213> Homo Sapiens
<400> 280
   Phe Tyr Gln Thr Ser Val Glu Ser Thr Asp Phe Ala Asn Ala Pro Glu
   1 5 10 15
   Glu Ser Arg
<210> 281
   <211> 21
   <212> PRT
   <213> Homo Sapiens
<400> 281
   Phe Tyr Gln Thr Ser Val Glu Ser Thr Asp Phe Ala Asn Ala Pro Glu
   1 5 10 15
   Glu Ser Arg Lys Lys
   20
<210> 282
   <211> 11
   <212> PRT
   <213> Homo Sapiens
<400> 282
   Gly Ala Glu Ala Phe Glu Ile Ala Leu Pro Arg
   1 5 10
<210> 283
   <211> 9
   <212> PRT
   <213> Homo Sapiens
<400> 283
   Gly Ala Leu Ala Asp Asn Leu Leu Arg
   1 5
<210> 284
   <211> 21
   <212> PRT
   <213> Homo Sapiens
<400> 284
   Gly Ala Leu Ile Leu Ser Asn Val Gln Pro Ser Asp Thr Met Val Thr
   1 5 10 15
   Gln Cys Glu Ala Arg
   20
<210> 285
   <211> 13
   <212> PRT
   <213> Homo Sapiens
<400> 285
   Gly Ala Asn Thr His Leu Ser Thr Phe Ser Phe Thr Lys
   1 5 10
<210> 286
   <211> 12
   <212> PRT
   <213> Homo Sapiens
<400> 286
   Gly Ala Pro Cys Thr Thr Pro Pro Ser Ala Pro Arg
   1 5 10
<210> 287
   <211> 21
   <212> PRT
   <213> Homo Sapiens
<400> 287
   Gly Ala Val Tyr Leu Phe His Gly Val Leu Gly Pro Ser Ile Ser Pro
   1 5 10 15
   Ser His Ser Gln Arg
   20
<210> 288
   <211> 10
   <212> PRT
   <213> Homo Sapiens
<400> 288
   Gly Asp Ser Asn Ser Tyr Asn Val Arg Arg
   1 5 10
<210> 289
   <211> 17
   <212> PRT
   <213> Homo Sapiens
<400> 289
   Gly Glu Gly Asn Glu Thr Thr Asn Met Val Ile Thr Trp Lys Pro Leu
   1 5 10 15
   Arg
<210> 290
   <211> 14
   <212> PRT
   <213> Homo Sapiens
<400> 290
   Gly Glu Gly Pro Glu Ala Gly Ala Gly Gly Ala Gly Gly Arg
   1 5 10
<210> 291
   <211> 7
   <212> PRT
   <213> Homo Sapiens
<400> 291
   Gly Glu Gly Val Ala Tyr Arg
   1 5
<210> 292
   <211> 12
   <212> PRT
   <213> Homo Sapiens
<400> 292
   Gly Glu Pro Gly Pro Pro Gly Pro Ala Gly Glu Arg
   1 5 10
<210> 293
   <211> 15
   <212> PRT
   <213> Homo Sapiens
<400> 293
   Gly Glu Thr Tyr Thr Tyr Asp Trp Gln Leu Ile Thr His Pro Arg
   1 5 10 15
<210> 294
   <211> 6
   <212> PRT
   <213> Homo Sapiens
<400> 294
   Gly Phe Phe Gly Tyr Lys
   1 5
<210> 295
   <211> 12
   <212> PRT
   <213> Homo Sapiens
<400> 295
   Gly Phe Pro Thr Ile Asp Met Gly Pro Gln Leu Lys
   1 5 10
<210> 296
   <211> 16
   <212> PRT
   <213> Homo Sapiens
<400> 296
   Gly Gly Ala Gln Ile Thr Phe Leu Ala Thr Phe Asp Val Ser Pro Lys
   1 5 10 15
<210> 297
   <211> 16
   <212> PRT
   <213> Homo Sapiens
<400> 297
   Gly Gly Cys Ile Thr Leu Ile Ser Ser Glu Gly Tyr Val Ser Ser Lys
   1 5 10 15
<210> 298
   <211> 10
   <212> PRT
   <213> Homo Sapiens
<400> 298
   Gly Gly Gly Ala Tyr Tyr Leu Ile Ser Arg
   1 5 10
<210> 299
   <211> 11
   <212> PRT
   <213> Homo Sapiens
<400> 299
   Gly Gly Gln Val Ser Val Cys Pro Leu Pro Arg
   1 5 10
<210> 300
   <211> 7
   <212> PRT
   <213> Homo Sapiens
<400> 300
   Gly Ile Asp Tyr Tyr Asp Arg
   1 5
<210> 301
   <211> 14
   <212> PRT
   <213> Homo Sapiens
<400> 301
   Gly Ile Thr Glu Pro Pro Phe Gly Ile Phe Val Phe Asn Lys
   1 5 10
<210> 302
   <211> 16
   <212> PRT
   <213> Homo Sapiens
<400> 302
   Gly Leu Pro Ser Leu Thr Ser Val Ser Trp Asn Ile Ser Val Pro Arg
   1 5 10 15
<210> 303
   <211> 9
   <212> PRT
   <213> Homo Sapiens
<400> 303
   Gly Asn Leu Ser Phe Gly Trp Val Arg
   1 5
<210> 304
   <211> 11
   <212> PRT
   <213> Homo Sapiens
<400> 304
   Gly Asn Asn Val Glu Lys Pro Leu Glu Leu Arg
   1 5 10
<210> 305
   <211> 10
   <212> PRT
   <213> Homo Sapiens
<400> 305
   Gly Asn Ser Ala Gly Gly Leu Gln His Arg
   1 5 10
<210> 306
   <211> 12
   <212> PRT
   <213> Homo Sapiens
<400> 306
   Gly Pro Gly Pro Tyr Ser Pro Ser Val Gln Phe Arg
   1 5 10
<210> 307
   <211> 12
   <212> PRT
   <213> Homo Sapiens
<400> 307
   Gly Pro Ile Val Pro Leu Asn Val Ala Asp Gln Lys
   1 5 10
<210> 308
   <211> 8
   <212> PRT
   <213> Homo Sapiens
<400> 308
   Gly Pro Pro Ser Glu Ala Val Arg
   1 5
<210> 309
   <211> 21
   <212> PRT
   <213> Homo Sapiens
<400> 309
   Gly Pro Pro Ser Glu Pro Val Leu Thr Gln Thr Ser Glu Gln Ala Pro
   1 5 10 15
   Ser Ser Ala Pro Arg
   20
<210> 310
   <211> 10
   <212> PRT
   <213> Homo Sapiens
<400> 310
   Gly Pro Arg Pro Asn Pro Ala Ile Met Arg
   1 5 10
<210> 311
   <211> 21
   <212> PRT
   <213> Homo Sapiens
<400> 311
   Gly Gln Ile Ile Gly Asn Phe Gln Ala Phe Asp Glu Asp Thr Gly Leu
   1 5 10 15
   Pro Ala His Ala Arg
   20
<210> 312
   <211> 8
   <212> PRT
   <213> Homo Sapiens
<400> 312
   Gly Gln Leu Ser Phe Asn Leu Arg
   1 5
<210> 313
   <211> 17
   <212> PRT
   <213> Homo Sapiens
<400> 313
   Gly Gln Val Val Thr Val His Leu Gln Thr Ser Leu Glu Asn Gly Thr
   1 5 10 15
   Arg
<210> 314
   <211> 23
   <212> PRT
   <213> Homo Sapiens
<400> 314
   Gly Ser Glu Lys Pro Leu Glu Gln Thr Phe Ala Thr Met Val Ser Ser
   1 5 10 15
   Leu Gly Ser Gly Met Met Arg
   20
<210> 315
   <211> 12
   <212> PRT
   <213> Homo Sapiens
<400> 315
   Gly Ser Gly Pro Leu Ser Pro Ser Ile Gln Ser Arg
   1 5 10
<210> 316
   <211> 14
   <212> PRT
   <213> Homo Sapiens
<400> 316
   Gly Ser Ile Pro Val Phe Trp Ser Gln Arg Pro Asn Leu Lys
   1 5 10
<210> 317
   <211> 23
   <212> PRT
   <213> Homo Sapiens
<400> 317
   Gly Ser Pro Gly Lys Pro Gly Pro Gln Gly Ser Ser Gly Asp Pro Gly
   1 5 10 15
   Pro Pro Gly Pro Pro Gly Lys
   20
<210> 318
   <211> 25
   <212> PRT
   <213> Homo Sapiens
<400> 318
   Gly Ser Pro Gln Leu Asp Cys Gly Pro Pro Ala Leu Gln Glu Met Pro
   1 5 10 15
   Ile Asn Gln Gly Gly Glu Gly Lys Lys
   20 25
<210> 319
   <211> 12
   <212> PRT
   <213> Homo Sapiens
<400> 319
   Gly Ser Gln Gly Pro Pro Gly Pro Thr Gly Asn Lys
   1 5 10
<210> 320
   <211> 17
   <212> PRT
   <213> Homo Sapiens
<400> 320
   Gly Ser Gln Pro Ser Gly Glu Leu Leu Ala Ser Phe Glu Leu Ile Gln
   1 5 10 15
   Arg
<210> 321
   <211> 14
   <212> PRT
   <213> Homo Sapiens
<400> 321
   Gly Ser Ser Ala Gly Gly Leu Gln His Leu Val Ser Ile Arg
   1 5 10
<210> 322
   <211> 9
   <212> PRT
   <213> Homo Sapiens
<400> 322
   Gly Ser Ser Ser Ala Ser Ile Val Lys
   1 5
<210> 323
   <211> 18
   <212> PRT
   <213> Homo Sapiens
<400> 323
   Gly Ser Val Thr Phe His Cys Ala Leu Gly Pro Glu Val Ala Asn Val
   1 5 10 15
   Ala Lys
<210> 324
   <211> 7
   <212> PRT
   <213> Homo Sapiens
<400> 324
   Gly Thr Thr Tyr Ile Phe Arg
   1 5
<210> 325
   <211> 17
   <212> PRT
   <213> Homo Sapiens
<400> 325
   Gly Val Glu Gly Ser Asp Tyr Ile Asn Ala Ser Phe Ile Asp Gly Tyr
   1 5 10 15
   Arg
<210> 326
   <211> 17
   <212> PRT
   <213> Homo Sapiens
<400> 326
   Gly Val Glu Gly Ser Asp Tyr Ile Asn Ala Ser Phe Leu Asp Gly Tyr
   1 5 10 15
   Arg
<210> 327
   <211> 11
   <212> PRT
   <213> Homo Sapiens
<400> 327
   Gly Val Gln Ser Leu Val Leu Gly Ala Pro Arg
   1 5 10
<210> 328
   <211> 8
   <212> PRT
   <213> Homo Sapiens
<400> 328
   Gly Trp His Phe Tyr Asp Asp Arg
   1 5
<210> 329
   <211> 10
   <212> PRT
   <213> Homo Sapiens
<400> 329
   Gly Trp Met Ile Gly Phe Glu Glu His Lys
   1 5 10
<210> 330
   <211> 8
   <212> PRT
   <213> Homo Sapiens
<400> 330
   Gly Tyr Asn Val Thr Tyr Trp Arg
   1 5
<210> 331
   <211> 8
   <212> PRT
   <213> Homo Sapiens
<400> 331
   Gly Tyr Gln Val Thr Tyr Val Arg
   1 5
<210> 332
   <211> 11
   <212> PRT
   <213> Homo Sapiens
<400> 332
   His Phe Asp Ser Gln Val Ile Ile Tyr Gly Lys
   1 5 10
<210> 333
   <211> 8
   <212> PRT
   <213> Homo Sapiens
<400> 333
   His Phe Val Ser Leu Cys Gln Lys
   1 5
<210> 334
   <211> 12
   <212> PRT
   <213> Homo Sapiens
<400> 334
   His Gly Leu Pro Ser Ala Asp Ala Pro Ser Leu Lys
   1 5 10
<210> 335
   <211> 11
   <212> PRT
   <213> Homo Sapiens
<400> 335
   His Gly Gln Tyr Leu Ile Gly His Gly Thr Lys
   1 5 10
<210> 336
   <211> 11
   <212> PRT
   <213> Homo Sapiens
<400> 336
   His Ile Trp Pro Asn Val Pro Asp Pro Ser Lys
   1 5 10
<210> 337
   <211> 9
   <212> PRT
   <213> Homo Sapiens
<400> 337
   His Leu Pro Glu Thr Glu Ala Gly Arg
   1 5
<210> 338
   <211> 12
   <212> PRT
   <213> Homo Sapiens
<400> 338
   His Met Ala Thr Thr Gln Asp Glu Val His Thr Lys
   1 5 10
<210> 339
   <211> 12
   <212> PRT
   <213> Homo Sapiens
<400> 339
   His Met Trp Pro Phe Ile Cys Gln Phe Ile Glu Lys
   1 5 10
<210> 340
   <211> 13
   <212> PRT
   <213> Homo Sapiens
<400> 340
   His Asn Ser Val Val Leu Gly Trp Pro Tyr Gly Trp Arg
   1 5 10
<210> 341
   <211> 23
   <212> PRT
   <213> Homo Sapiens
<400> 341
   His Asn Thr Asp Ala Gly Leu Leu Thr Thr Val Gly Ser Leu Leu Pro
   1 5 10 15
   Gly Ile Thr Tyr Ser Leu Arg
   20
<210> 342
   <211> 6
   <212> PRT
   <213> Homo Sapiens
<400> 342
   His Gln Met Ala Val Lys
   1 5
<210> 343
   <211> 6
   <212> PRT
   <213> Homo Sapiens
<400> 343
   His Gln Val Val His Lys
   1 5
<210> 344
   <211> 14
   <212> PRT
   <213> Homo Sapiens
<400> 344
   His Ser His Thr Asn Leu Ser Ile Ser Thr Gly Val Thr Lys
   1 5 10
<210> 345
   <211> 14
   <212> PRT
   <213> Homo Sapiens
<400> 345
   His Val Glu Met Tyr Gln Trp Val Glu Thr Glu Glu Ser Arg
   1 5 10
<210> 346
   <211> 9
   <212> PRT
   <213> Homo Sapiens
<400> 346
   His Val Ser Pro Val Thr Pro Pro Arg
   1 5
<210> 347
   <211> 8
   <212> PRT
   <213> Homo Sapiens
<400> 347
   His Val Val Asp Gly Ile Ser Arg
   1 5
<210> 348
   <211> 7
   <212> PRT
   <213> Homo Sapiens
<400> 348
   His Trp Val Pro Ala Glu Lys
   1 5
<210> 349
   <211> 8
   <212> PRT
   <213> Homo Sapiens
<400> 349
   Ile Ala Asp Phe Gly Leu Ala Arg
   1               5
<210> 350
   <211> 9
   <212> PRT
   <213> Homo Sapiens
<400> 350
   Ile Ala Gly Ser Gln Leu Ser Ser Arg
   1               5
<210> 351
   <211> 10
   <212> PRT
   <213> Homo Sapiens
<400> 351
   Ile Ala Pro Cys Tyr Gln Asp Tyr Val Lys
   1               5                   10
<210> 352
   <211> 14
   <212> PRT
   <213> Homo Sapiens
<400> 352
   Ile Ala Pro Pro Ala Ser Asp Phe Leu Ala His Ile Gln Lys
   1               5                   10
<210> 353
   <211> 7
   <212> PRT
   <213> Homo Sapiens
<400> 353
   Ile Asp Ala Thr Val Val Arg
   1               5
<210> 354
   <211> 17
   <212> PRT
   <213> Homo Sapiens
<400> 354
   Ile Asp Phe Ser Asp Ile Met Val Leu Gly Asp Ile Asn Thr Lys Pro
   1 5 10 15
   Lys
<210> 355
   <211> 18
   <212> PRT
   <213> Homo Sapiens
<400> 355
   Ile Asp Phe Ser Asp Ile Met Val Leu Gly Asp Ile Asn Thr Lys Pro
   1 5 10 15
   Lys Lys
<210> 356
   <211> 6
   <212> PRT
   <213> Homo Sapiens
<400> 356
   Ile Asp His Asp Arg Arg
   1 5
<210> 357
   <211> 5
   <212> PRT
   <213> Homo Sapiens
<400> 357
   Ile Asp His Tyr Arg
   1 5
<210> 358
   <211> 13
   <212> PRT
   <213> Homo Sapiens
<400> 358
   Ile Asp Ser Leu Glu Asp Gln Ile Glu Glu Phe Ser Lys
   1 5 10
<210> 359
   <211> 18
   <212> PRT
   <213> Homo Sapiens
<400> 359
   Ile Asp Thr Ile Ala Pro Asp Glu Ile Thr Val Ser Ser Asp Phe Glu
   1 5 10 15
   Ala Arg
<210> 360
   <211> 15
   <212> PRT
   <213> Homo Sapiens
<400> 360
   Ile Glu Glu Val Ile Gly Ala Gly Glu Phe Gly Glu Val Cys Arg
   1 5 10 15
<210> 361
   <211> 8
   <212> PRT
   <213> Homo Sapiens
<400> 361
   Ile Glu Gly Leu Gln Ile Ser Lys
   1 5
<210> 362
   <211> 7
   <212> PRT
   <213> Homo Sapiens
<400> 362
   Ile Glu Met Val Asp Tyr Lys
   1 5
<210> 363
   <211> 13
   <212> PRT
   <213> Homo Sapiens
<400> 363
   Ile Glu Thr Gln Asn Gln Leu Leu Gly Ile Ala Asp Arg
   1 5 10
<210> 364
   <211> 15
   <212> PRT
   <213> Homo Sapiens
<400> 364
   Ile Phe Thr Val Ala Gln Met Asp Asp Asn Ser Ile Gln Met Lys
   1 5 10 15
<210> 365
   <211> 16
   <212> PRT
   <213> Homo Sapiens
<400> 365
   Ile Phe Thr Val Ala Gln Met Asp Asp Asn Ser Ile Gln Met Lys Lys
   1 5 10 15
<210> 366
   <211> 11
   <212> PRT
   <213> Homo Sapiens
<400> 366
   Ile Gly Asp Asp Asn Glu Asn Leu Thr Phe Lys
   1 5 10
<210> 367
   <211> 11
   <212> PRT
   <213> Homo Sapiens
<400> 367
   Ile Gly Glu Thr Val Val Asp Leu Glu Asn Arg
   1 5 10
<210> 368
   <211> 12
   <212> PRT
   <213> Homo Sapiens
<400> 368
   Ile His Phe Ile Glu Ala Gln Asp Leu Gln Gly Lys
   1 5 10
<210> 369
   <211> 9
   <212> PRT
   <213> Homo Sapiens
<400> 369
   Ile His Ser Asp Leu Ala Glu Glu Arg
   1 5
<210> 370
   <211> 6
   <212> PRT
   <213> Homo Sapiens
<400> 370
   Ile Ile Asp Trp Asp Arg
   1 5
<210> 371
   <211> 9
   <212> PRT
   <213> Homo Sapiens
<400> 371
   Ile Ile Glu Gly Glu Pro Asn Leu Lys
   1 5
<210> 372
   <211> 19
   <212> PRT
   <213> Homo Sapiens
<400> 372
   Ile Ile Met Tyr Glu Leu Val Tyr Trp Ala Ala Glu Asp Glu Asp Gln
   1 5 10 15
   Gln His Lys
<210> 373
   <211> 9
   <212> PRT
   <213> Homo Sapiens
<400> 373
   Ile Leu Ala Ser Val Gln His Met Lys
   1 5
<210> 374
   <211> 17
   <212> **PRT**
   <213> Homo Sapiens
<400> 374
   Ile Leu Asp Ala Thr Asp Gln Glu Ser Leu Glu Leu Lys Pro Thr Ser
   1 5 10 15
   Arg
<210> 375
   <211> 17
   <212> PRT
   <213> Homo Sapiens
<400> 375
   Ile Leu Asp Glu Ser Glu Asp Thr Asp Leu Pro Tyr Pro Pro Pro Gln
   1 5 10 15
   Arg
<210> 376
   <211> 7
   <212> PRT
   <213> Homo Sapiens
<400> 376
   Ile Leu Glu Leu Glu Glu Lys
   1               5
<210> 377
   <211> 8
   <212> PRT
   <213> Homo Sapiens
<400> 377
   Ile Leu Leu Asn Pro Gln Asp Lys
   1               5
<210> 378
   <211> 16
   <212> PRT
   <213> Homo Sapiens
<400> 378
   Ile Leu Tyr Asn Gly Gln Ser Val Glu Val Asp Gly His Ser Met Arg
   1               5                   10                  15
<210> 379
   <211> 17
   <212> PRT
   <213> Homo Sapiens
<400> 379
   Ile Leu Tyr Asn Gly Gln Ser Val Glu Val Asp Gly His Ser Met Arg
   1               5                   10                  15
   Lys
<210> 380
   <211> 11
   <212> PRT
   <213> Homo Sapiens
<400> 380
   Ile Met Phe Val Asp Pro Ser Leu Thr Val Arg
   1               5                   10
<210> 381
   <211> 15
   <212> PRT
   <213> Homo Sapiens
<400> 381
   Ile Asn Ala Thr Asp Ala Asp Glu Pro Asn Thr Leu Asn Ser Lys
   1 5 10 15
<210> 382
   <211> 11
   <212> PRT
   <213> Homo Sapiens
<400> 382
   Ile Asn Gly Ile Pro Val Glu Glu Leu Ala Lys
   1 5 10
<210> 383
   <211> 7
   <212> PRT
   <213> Homo Sapiens
<400> 383
   Ile Asn His Leu Ile Phe Arg
   1 5
<210> 384
   <211> 12
   <212> PRT
   <213> Homo Sapiens
<400> 384
   Ile Asn Ser Trp Val Glu Ser Gln Thr Asn Glu Lys
   1 5 10
<210> 385
   <211> 11
   <212> PRT
   <213> Homo Sapiens
<400> 385
   Ile Pro Glu Asn Phe Phe Glu Glu Glu Ser Arg
   1 5 10
<210> 386
   <211> 12
   <212> PRT
   <213> Homo Sapiens
<400> 386
   Ile Pro Asn Pro His Leu Gly Pro Val Glu Glu Arg
   1 5 10
<210> 387
   <211> 17
   <212> PRT
   <213> Homo Sapiens
<400> 387
   Ile Gln Asp Leu Leu Ala Glu Gly Thr Ile Thr Gly Val Ile Asp Asp
   1 5 10 15
   Arg
<210> 388
   <211> 12
   <212> PRT
   <213> Homo Sapiens
<400> 388
   Ile Gln Leu Ser Trp Leu Leu Pro Pro Gln Glu Arg
   1 5 10
<210> 389
   <211> 7
   <212> PRT
   <213> Homo Sapiens
<400> 389
   Ile Gln Met Thr Trp Thr Arg
   1 5
<210> 390
   <211> 15
   <212> PRT
   <213> Homo Sapiens
<400> 390
   Ile Gln Asn Ser Ser Cys Thr Ser Leu Glu His Cys Phe Arg Lys
   1 5 10 15
<210> 391
   <211> 10
   <212> PRT
   <213> Homo Sapiens
<400> 391
   Ile Gln Pro Tyr Thr Glu Gln Ser Thr Lys
   1 5 10
<210> 392
   <211> 14
   <212> PRT
   <213> Homo Sapiens
<400> 392
   Ile Ser Glu Trp Pro Ile Asp Asp His Phe Thr Tyr Ser Arg
   1 5 10
<210> 393
   <211> 16
   <212> PRT
   <213> Homo Sapiens
<400> 393
   Ile Ser Ser Asn Pro Asn Pro Val Val Gln Met Ser Val Gly His Lys
   1 5 10 15
<210> 394
   <211> 14
   <212> PRT
   <213> Homo Sapiens
<400> 394
   Ile Ser Val Thr Val Ser Glu Thr Phe Asp Pro Glu Glu Lys
   1 5 10
<210> 395
   <211> 10
   <212> PRT
   <213> Homo Sapiens
<400> 395
   Ile Thr Asp Asn Glu Leu Glu Leu Tyr Lys
   1 5 10
<210> 396
   <211> 15
   <212> PRT
   <213> Homo Sapiens
<400> 396
   Ile Thr Gln Leu Gly Gln Ser Ala Glu Asp Leu Gln Gly Ser Arg
   1 5 10 15
<210> 397
   <211> 16
   <212> PRT
   <213> Homo Sapiens
<400> 397
   Ile Thr Gln Leu Gly Gln Ser Ala Glu Asp Leu Gln Gly Ser Arg Arg
   1 5 10 15
<210> 398
   <211> 15
   <212> PRT
   <213> Homo Sapiens
<400> 398
   Ile Thr Val Pro Leu Val Ser Glu Val Gln Ile Ala Gln Leu Arg
   1 5 10 15
<210> 399
   <211> 6
   <212> PRT
   <213> Homo Sapiens
<400> 399
   Ile Thr Trp Met Lys Lys
   1 5
<210> 400
   <211> 10
   <212> PRT
   <213> Homo Sapiens
<400> 400
   Ile Val Ala Ile Ser Glu Asp Tyr Pro Arg
   1 5 10
<210> 401
   <211> 9
   <212> PRT
   <213> Homo Sapiens
<400> 401
   Ile Val Phe Thr Pro Thr Ile Cys Lys
   1 5
<210> 402
   <211> 14
   <212> PRT
   <213> Homo Sapiens
<400> 402
   Ile Val Val Glu Asp Val Asp Glu Pro Pro Val Phe Ser Lys
   1 5 10
<210> 403
   <211> 15
   <212> PRT
   <213> Homo Sapiens
<400> 403
   Ile Tyr Pro Leu Pro Glu Asp Pro Ala Ile Pro Met Pro Pro Arg
   1 5 10 15
<210> 404
   <211> 10
   <212> PRT
   <213> Homo Sapiens
<400> 404
   Ile Tyr Val Val Asp Leu Ser Asn Glu Arg
   1 5 10
<210> 405
   <211> 7
   <212> PRT
   <213> Homo Sapiens
<400> 405
   Lys Ala Ala Glu Leu Asp Arg
   1 5
<210> 406
   <211> 8
   <212> PRT
   <213> Homo Sapiens
<400> 406
   Lys Ala Ala Glu Leu Asp Arg Arg
   1 5
<210> 407
   <211> 7
   <212> PRT
   <213> Homo Sapiens
<400> 407
   Lys Ala Glu Ser Pro Pro Arg
   1 5
<210> 408
   <211> 9
   <212> PRT
   <213> Homo Sapiens
<400> 408
   Lys Asp Trp Leu Gln Ala Asp Met Arg
   1 5
<210> 409
   <211> 9
   <212> PRT
   <213> Homo Sapiens
<400> 409
   Lys Glu Gly Asp Ser Leu Asp Tyr Lys
   1 5
<210> 410
   <211> 15
   <212> PRT
   <213> Homo Sapiens
<400> 410
   Lys Glu Asn Ile Ile Ala Phe Glu Glu Ile Ile Glu Pro Tyr Arg
   1 5 10 15
<210> 411
   <211> 8
   <212> PRT
   <213> Homo Sapiens
<400> 411
   Lys Glu Asn Ile Thr Tyr Met Lys
   1 5
<210> 412
   <211> 9
   <212> PRT
   <213> Homo Sapiens
<400> 412
   Lys Glu Asn Ile Thr Tyr Met Lys Arg
   1 5
<210> 413
   <211> 8
   <212> PRT
   <213> Homo Sapiens
<400> 413
   Lys Glu Ser Cys Val Ala Ile Lys
   1 5
<210> 414
   <211> 8
   <212> PRT
   <213> Homo Sapiens
<400> 414
   Lys Glu Val Pro Val Ala Ile Lys
   1 5
<210> 415
   <211> 13
   <212> PRT
   <213> Homo Sapiens
<400> 415
   Lys Phe Val Pro Gly Cys Phe Val Cys Leu Glu Ser Arg
   1 5 10
<210> 416
   <211> 21
   <212> PRT
   <213> Homo Sapiens
<400> 416
   Lys Phe Tyr Gln Thr Ser Val Glu Ser Thr Asp Phe Ala Asn Ala Pro
   1               5                   10                  15
   Glu Glu Ser Arg Lys
               20
<210> 417
   <211> 10
   <212> PRT
   <213> Homo Sapiens
<400> 417
   Lys Gly Asp Ser Asn Ser Tyr Asn Val Arg
   1               5                   10
<210> 418
   <211> 11
   <212> PRT
   <213> Homo Sapiens
<400> 418
   Lys Gly Asp Ser Asn Ser Tyr Asn Val Arg Arg
   1               5                   10
<210> 419
   <211> 18
   <212> PRT
   <213> Homo Sapiens
<400> 419
   Lys Gly Phe Gly Gly Thr Ala Gly Met Ala Phe Val Gly Thr Val Cys
   1               5                   10                  15
   Ser Arg
<210> 420
   <211> 13
   <212> PRT
   <213> Homo Sapiens
<400> 420
   Lys Ile Asn Ser Trp Val Glu Ser Gln Thr Asn Glu Lys
   1               5                   10
<210> 421
   <211> 9
   <212> PRT
   <213> Homo Sapiens
<400> 421
   Lys Lys Glu Asn Ile Thr Tyr Met Lys
   1 5
<210> 422
   <211> 11
   <212> PRT
   <213> Homo Sapiens
<400> 422
   Lys Lys Gly Asp Ser Asn Ser Tyr Asn Val Arg
   1 5 10
<210> 423
   <211> 8
   <212> PRT
   <213> Homo Sapiens
<400> 423
   Lys Leu Gly Asp Gln Thr Gly Lys
   1 5
<210> 424
   <211> 19
   <212> PRT
   <213> Homo Sapiens
<400> 424
   Lys Leu Ile Ala Asp Leu Gln Pro Asn Thr Glu Tyr Ser Phe Val Leu
   1 5 10 15
   Met Asn Arg
<210> 425
   <211> 8
   <212> PRT
   <213> Homo Sapiens
<400> 425
   Lys Leu Pro Gln Gly Glu Ser Arg
   1 5
<210> 426
   <211> 14
   <212> PRT
   <213> Homo Sapiens
<400> 426
   Lys Leu Pro Ser Arg Pro Pro Pro His Tyr Pro Gly Ile Lys
   1               5                   10
<210> 427
   <211> 8
   <212> PRT
   <213> Homo Sapiens
<400> 427
   Lys Met Leu Gly Asn Pro Ser Arg
   1               5
<210> 428
   <211> 6
   <212> PRT
   <213> Homo Sapiens
<400> 428
   Lys Asn Gly Tyr Asn Arg
   1               5
<210> 429
   <211> 15
   <212> PRT
   <213> Homo Sapiens
<400> 429
   Lys Asn Leu Val Asp Pro Phe Val Glu Val Ser Phe Ala Gly Lys
   1               5                   10                  15
<210> 430
   <211> 18
   <212> PRT
   <213> Homo Sapiens
<400> 430
   Lys Asn Pro Asn Phe Asp Ile Cys Thr Leu Phe Met Glu Val Met Leu
   1               5                   10                  15
   Pro Arg
<210> 431
   <211> 8
   <212> PRT
   <213> Homo Sapiens
<400> 431
   Lys Pro Phe Pro Glu Asp Leu Lys
   1               5
<210> 432
   <211> 19
   <212> PRT
   <213> Homo Sapiens
<400> 432
   Lys Pro Leu Met Val Ile His His Leu Glu Asp Cys Gln Tyr Ser Gln
   1 5 10 15
   Ala Leu Lys
<210> 433
   <211> 21
   <212> PRT
   <213> Homo Sapiens
<400> 433
   Lys Gln Asn Ala Tyr Ile Ala Thr Gln Gly Pro Leu Pro Glu Thr Met
   1 5 10 15
   Gly Asp Phe Trp Arg
   20
<210> 434
   <211> 8
   <212> PRT
   <213> Homo Sapiens
<400> 434
   Lys Arg Ala Glu Ser Asp Ser Arg
   1 5
<210> 435
   <211> 18
   <212> PRT
   <213> Homo Sapiens
<400> 435
   Lys Ser Asp Leu Asp Thr Ser Lys Pro Leu Ser Glu Lys Pro Ile Thr
   1 5 10 15
   His Lys
<210> 436
   <211> 7
   <212> PRT
   <213> Homo Sapiens
<400> 436
   Lys Thr Asp Ala Phe Arg Arg
   1 5
<210> 437
   <211> 6
   <212> PRT
   <213> Homo Sapiens
<400> 437
   Lys Thr Glu Glu Lys Lys
   1 5
<210> 438
   <211> 15
   <212> PRT
   <213> Homo Sapiens
<400> 438
   Lys Thr Met Leu His Leu Thr Asp Ile Gln Leu Gln Asp Asn Lys
   1 5 10 15
<210> 439
   <211> 10
   <212> PRT
   <213> Homo Sapiens
<400> 439
   Lys Val Glu Cys Glu His Gly Phe Gly Arg
   1 5 10
<210> 440
   <211> 16
   <212> PRT
   <213> Homo Sapiens
<400> 440
   Lys Val Phe Ala Gln Asn Glu Glu Ile Gln Glu Met Ala Gln Asn Lys
   1 5 10 15
<210> 441
   <211> 7
   <212> PRT
   <213> Homo Sapiens
<400> 441
   Lys Val His Gly Leu Tyr Arg
   1 5
<210> 442
   <211> 8
   <212> PRT
   <213> Homo Sapiens
<400> 442
   Lys Tyr Phe Trp Thr Gly Leu Arg
   1 5
<210> 443
   <211> 5
   <212> PRT
   <213> Homo Sapiens
<400> 443
   Lys Tyr Trp Cys Arg
   1 5
<210> 444
   <211> 9
   <212> PRT
   <213> Homo Sapiens
<400> 444
   Leu Ala Thr Glu Glu Pro Pro Pro Arg
   1 5
<210> 445
   <211> 16
   <212> PRT
   <213> Homo Sapiens
<400> 445
   Leu Asp Cys Gln Val Gln Gly Arg Pro Gln Pro Glu Val Thr Trp Arg
   1 5 10 15
<210> 446
   <211> 8
   <212> PRT
   <213> Homo Sapiens
<400> 446
   Leu Asp Glu Asp Leu His Val Lys
   1 5
<210> 447
   <211> 5
   <212> PRT
   <213> Homo Sapiens
<400> 447
   Leu Asp His Tyr Arg
   1 5
<210> 448
   <211> 16
   <212> PRT
   <213> Homo Sapiens
<400> 448
   Leu Asp Thr Glu Val Ala Asn Leu Ser Val Ile Met Glu Glu Met Lys
   1 5 10 15
<210> 449
   <211> 10
   <212> PRT
   <213> Homo Sapiens
<400> 449
   Leu Glu Asp Pro His Val Asp Ile Ile Arg
   1 5 10
<210> 450
   <211> 6
   <212> PRT
   <213> Homo Sapiens
<400> 450
   Leu Glu Glu Glu Gln Lys
   1 5
<210> 451
   <211> 9
   <212> PRT
   <213> Homo Sapiens
<400> 451
   Leu Glu Glu Gly Pro Pro His Thr Lys
   1 5
<210> 452
   <211> 11
   <212> PRT
   <213> Homo Sapiens
<400> 452
   Leu Glu Glu Gly Gln Ala Thr Ser Trp Ser Arg
   1 5 10
<210> 453
   <211> 9
   <212> PRT
   <213> Homo Sapiens
<400> 453
   Leu Glu Glu Gln Asp Glu Phe Glu Lys
   1 5
<210> 454
   <211> 7
   <212> PRT
   <213> Homo Sapiens
<400> 454
   Leu Glu Gly Val Ile Ser Lys
   1 5
<210> 455
   <211> 7
   <212> PRT
   <213> Homo Sapiens
<400> 455
   Leu Glu Asn Gly Glu Pro Arg
   1 5
<210> 456
   <211> 7
   <212> PRT
   <213> Homo Sapiens
<400> 456
   Leu Glu Thr Ala Asp Leu Lys
   1 5
<210> 457
   <211> 15
   <212> PRT
   <213> Homo Sapiens
<400> 457
   Leu Glu Trp Leu Thr Leu Met Pro Asn Ala Ser Asn Leu Asp Lys
   1 5 10 15
<210> 458
   <211> 9
   <212> PRT
   <213> Homo Sapiens
<400> 458
   Leu Phe His Ala Ser Tyr Gly Ala Arg
   1 5
<210> 459
   <211> 10
   <212> PRT
   <213> Homo Sapiens
<400> 459
   Leu Phe His Ala Ser Tyr Gly Ala Arg Arg
   1 5 10
<210> 460
   <211> 8
   <212> PRT
   <213> Homo Sapiens
<400> 460
   Leu Phe His Leu His Ser Gln Lys
   1 5
<210> 461
   <211> 8
   <212> PRT
   <213> Homo Sapiens
<400> 461
   Leu Gly Asp Gln Thr Gly Lys Lys
   1 5
<210> 462
   <211> 18
   <212> PRT
   <213> Homo Sapiens
<400> 462
   Leu Gly Gln Val Pro Pro Gly Glu Ser Val Thr Ala Met Glu Leu Glu
   1 5 10 15
   Phe Lys
<210> 463
   <211> 10
   <212> PRT
   <213> Homo Sapiens
<400> 463
   Leu Gly Thr Ala Met Ser His Glu Ile Arg
   1 5 10
<210> 464
   <211> 9
   <212> PRT
   <213> Homo Sapiens
<400> 464
   Leu Gly Val Leu His Val Gly Gln Lys
   1 5
<210> 465
   <211> 6
   <212> PRT
   <213> Homo Sapiens
<400> 465
   Leu His Glu Asp Asp Lys
   1 5
<210> 466
   <211> 7
   <212> PRT
   <213> Homo Sapiens
<400> 466
   Leu His Ile Thr Pro Glu Lys
   1 5
<210> 467
   <211> 18
   <212> PRT
   <213> Homo Sapiens
<400> 467
   Leu Ile Ala Asp Leu Gln Pro Asn Thr Glu Tyr Ser Phe Val Leu Met
   1 5 10 15
   Asn Arg
<210> 468
   <211> 10
   <212> PRT
   <213> Homo Sapiens
<400> 468
   Leu Leu Gln Glu Ala Tyr Met Thr Pro Lys
   1 5 10
<210> 469
   <211> 12
   <212> PRT
   <213> Homo Sapiens
<400> 469
   Leu Leu Ser Asp Lys Pro Gln Asp Phe Gln Ile Arg
   1 5 10
<210> 470
   <211> 11
   <212> PRT
   <213> Homo Sapiens
<400> 470
   Leu Met Glu Trp Thr Ser Leu Gln Asn Met Arg
   1 5 10
<210> 471
   <211> 6
   <212> PRT
   <213> Homo Sapiens
<400> 471
   Leu Asn Glu Leu Leu Lys
   1 5
<210> 472
   <211> 8
   <212> PRT
   <213> Homo Sapiens
<400> 472
   Leu Asn Glu Val Ile Val Thr Arg
   1 5
<210> 473
   <211> 19
   <212> PRT
   <213> Homo Sapiens
<400> 473
   Leu Asn Gly Ser Ser Leu His Leu Glu Trp Ser Ala Pro Leu Glu Ser
   1 5 10 15
   Gly Gly Arg
<210> 474
   <211> 6
   <212> PRT
   <213> Homo Sapiens
<400> 474
   Leu Asn Val Glu Glu Arg
   1 5
<210> 475
   <211> 9
   <212> PRT
   <213> Homo Sapiens
<400> 475
   Leu Asn Tyr Gln Thr Pro Gly Met Arg
   1 5
<210> 476
   <211> 7
   <212> PRT
   <213> Homo Sapiens
<400> 476
   Leu Pro Asp Phe Glu Ser Arg
   1 5
<210> 477
   <211> 20
   <212> PRT
   <213> Homo Sapiens
<400> 477
   Leu Pro Gly Gly Gln Trp Ile Tyr Met Ser Asp Asn Tyr Thr Asp Val
   1 5 10 15
   Asn Gly Glu Lys
   20
<210> 478
   <211> 7
   <212> PRT
   <213> Homo Sapiens
<400> 478
   Leu Pro Gly His Gln Lys Arg
   1 5
<210> 479
   <211> 21
   <212> PRT
   <213> Homo Sapiens
<400> 479
   Leu Pro Pro Pro Pro Asp Cys Pro Thr Ser Leu His Gln Leu Met Leu
   1 5 10 15
   Asp Cys Trp Gln Lys
   20
<210> 480
   <211> 7
   <212> PRT
   <213> Homo Sapiens
<400> 480
   Leu Pro Gln Gly Glu Ser Arg
   1 5
<210> 481
   <211> 13
   <212> PRT
   <213> Homo Sapiens
<400> 481
   Leu Pro Ser Thr Ser Gly Ser Glu Gly Val Pro Phe Arg
   1 5 10
<210> 482
   <211> 14
   <212> PRT
   <213> Homo Sapiens
<400> 482
   Leu Pro Thr Gly Gly Cys Tyr Gly Val Pro Pro Asp Leu Arg
   1 5 10
<210> 483
   <211> 23
   <212> PRT
   <213> Homo Sapiens
<400> 483
   Leu Pro Thr Pro Met Asp Cys Pro Ser Ala Ile Tyr Gln Leu Met Met
   1 5 10 15
   Gln Cys Trp Gln Gln Glu Arg
   20
<210> 484
   <211> 9
   <212> PRT
   <213> Homo Sapiens
<400> 484
   Leu Pro Val Gly Leu Tyr Phe Ile Lys
   1 5
<210> 485
   <211> 11
   <212> PRT
   <213> Homo Sapiens
<400> 485
   Leu Gln Ala Ser Gly Asp Ala Leu Val Asp Arg
   1 5 10
<210> 486
   <211> 22
   <212> PRT
   <213> Homo Sapiens
<400> 486
   Leu Ser Leu Leu Glu Glu Pro Gly Asn Gly Thr Phe Thr Val Ile Leu
   1 5 10 15
   Asn Gln Leu Thr Ser Arg
   20
<210> 487
   <211> 10
   <212> PRT
   <213> Homo Sapiens
<400> 487
   Leu Ser Leu Val Leu Val Pro Ala Gln Lys
   1 5 10
<210> 488
   <211> 16
   <212> PRT
   <213> Homo Sapiens
<400> 488
   Leu Ser Asn Thr Ser Pro Glu Phe Gln Glu Met Ser Leu Leu Glu Arg
   1 5 10 15
<210> 489
   <211> 10
   <212> PRT
   <213> Homo Sapiens
<400> 489
   Leu Ser Pro Tyr Val His Tyr Thr Phe Arg
   1 5 10
<210> 490
   <211> 10
   <212> PRT
   <213> Homo Sapiens
<400> 490
   Leu Ser Gln Cys His Glu Leu Trp Glu Arg
   1 5 10
<210> 491
   <211> 13
   <212> PRT
   <213> Homo Sapiens
<400> 491
   Leu Ser Val Phe Ala Glu Thr Tyr Glu Asn Glu Thr Lys
   1 5 10
<210> 492
   <211> 23
   <212> PRT
   <213> Homo Sapiens
<400> 492
   Leu Ser Val Leu Glu Glu Glu Gln Leu Pro Pro Gly Phe Pro Ser Ile
   1 5 10 15
   Asp Met Gly Pro Gln Leu Lys
   20
<210> 493
   <211> 11
   <212> PRT
   <213> Homo Sapiens
<400> 493
   Leu Ser Trp Tyr Asp Pro Asp Phe Gln Ala Arg
   1 5 10
<210> 494
   <211> 13
   <212> PRT
   <213> Homo Sapiens
<400> 494
   Leu Thr Ala Asp Leu Ser Leu Asp Thr Leu Asp Ala Arg
   1 5 10
<210> 495
   <211> 9
   <212> PRT
   <213> Homo Sapiens
<400> 495
   Leu Thr Ala Val Glu Ser Asp Leu Lys
   1 5
<210> 496
   <211> 15
   <212> PRT
   <213> Homo Sapiens
<400> 496
   Leu Thr Asp Val Val Ile Gly Ala Pro Gly Glu Glu Glu Asn Arg
   1 5 10 15
<210> 497
   <211> 9
   <212> PRT
   <213> Homo Sapiens
<400> 497
   Leu Thr Pro Gly Leu Tyr Glu Phe Lys
   1 5
<210> 498
   <211> 18
   <212> PRT
   <213> Homo Sapiens
<400> 498
   Leu Thr Gln Ile Glu Thr Gly Glu Asn Val Thr Gly Met Glu Leu Glu
   1 5 10 15
   Phe Lys
<210> 499
   <211> 6
   <212> PRT
   <213> Homo Sapiens
<400> 499
   Leu Thr Tyr Ser Ser Arg
   1 5
<210> 500
   <211> 9
   <212> PRT
   <213> Homo Sapiens
<400> 500
   Leu Val His Ile Ile Gly Ala Leu Arg
   1 5
<210> 501
   <211> 11
   <212> PRT
   <213> Homo Sapiens
<400> 501
   Leu Val Leu Leu Asn Met Pro Gly Pro Pro Arg
   1 5 10
<210> 502
   <211> 15
   <212> PRT
   <213> Homo Sapiens
<400> 502
   Leu Val Leu Ser Asp Leu His Leu Leu Thr Gln Ser Gln Val Arg
   1 5 10 15
<210> 503
   <211> 11
   <212> PRT
   <213> Homo Sapiens
<400> 503
   Leu Val Asn Ile Met Pro Tyr Glu Ser Thr Arg
   1 5 10
<210> 504
   <211> 12
   <212> PRT
   <213> Homo Sapiens
<400> 504
   Leu Val Ser Tyr Thr Asn Leu Thr Gln Gly Ala Lys
   1 5 10
<210> 505
   <211> 7
   <212> PRT
   <213> Homo Sapiens
<400> 505
   Leu Trp Met Asn Val Glu Lys
   1 5
<210> 506
   <211> 7
   <212> PRT
   <213> Homo Sapiens
<400> 506
   Leu Tyr Thr Tyr Glu Pro Arg
   1 5
<210> 507
   <211> 6
   <212> PRT
   <213> Homo Sapiens
<400> 507
   Met Ala Phe Asp Phe Arg
   1 5
<210> 508
   <211> 11
   <212> PRT
   <213> Homo Sapiens
<400> 508
   Met Ala Leu His Leu Pro Trp Phe His Pro Arg
   1 5 10
<210> 509
   <211> 9
   <212> PRT
   <213> Homo Sapiens
<400> 509
   Met Ala Pro Glu Pro Ala Pro Gly Arg
   1               5
<210> 510
   <211> 7
   <212> PRT
   <213> Homo Sapiens
<400> 510
   Met Asp Ile Leu Glu Glu Arg
   1               5
<210> 511
   <211> 8
   <212> PRT
   <213> Homo Sapiens
<400> 511
   Met Asp Val Gly Thr Ile Tyr Arg
   1               5
<210> 512
   <211> 7
   <212> PRT
   <213> Homo Sapiens
<400> 512
   Met Glu Leu Gln Ala Ala Arg
   1               5
<210> 513
   <211> 6
   <212> PRT
   <213> Homo Sapiens
<400> 513
   Met Glu Pro Leu Glu Lys
   1               5
<210> 514
   <211> 9
   <212> PRT
   <213> Homo Sapiens
<400> 514
   Met Glu Ser Glu Glu Leu Ala Asp Arg
   1 5
<210> 515
   <211> 12
   <212> PRT
   <213> Homo Sapiens
<400> 515
   Met Phe Glu Leu Thr Cys Thr Leu Pro Leu Glu Lys
   1 5 10
<210> 516
   <211> 17
   <212> PRT
   <213> Homo Sapiens
<400> 516
   Met Gly Gln Pro Pro Ala Glu Glu Ala Glu Gln Pro Gly Ala Leu Ala
   1 5 10 15
   Arg
<210> 517
   <211> 6
   <212> PRT
   <213> Homo Sapiens
<400> 517
   Met His Thr Ala Val Lys
   1 5
<210> 518
   <211> 22
   <212> PRT
   <213> Homo Sapiens
<400> 518
   Met Leu Ser Ala Ser Thr Met Leu Val Gln Trp Glu Pro Pro Glu Glu
   1 5 10 15
   Pro Asn Gly Leu Val Arg
   20
<210> 519
   <211> 15
   <212> PRT
   <213> Homo Sapiens
<400> 519
   Met Leu Trp Glu His Asn Ser Thr Ile Ile Val Met Leu Thr Lys
   1               5                   10                  15
<210> 520
   <211> 15
   <212> PRT
   <213> Homo Sapiens
<400> 520
   Met Leu Trp Glu His Asn Ser Thr Ile Val Val Met Leu Thr Lys
   1               5                   10                  15
<210> 521
   <211> 13
   <212> PRT
   <213> Homo Sapiens
<400> 521
   Met Pro His Phe Thr Val Val Pro Val Asp Gly Pro Arg
   1               5                   10
<210> 522
   <211> 26
   <212> PRT
   <213> Homo Sapiens
<400> 522
   Met Pro Asn Val Pro Asn Thr Gln Pro Ala Ile Met Lys Pro Thr Glu
   1               5                   10                  15
   Glu His Pro Ala Tyr Thr Gln Ile Ala Lys
               20                  25
<210> 523
   <211> 18
   <212> PRT
   <213> Homo Sapiens
<400> 523
   Met Gln Gln Tyr Thr Glu His Phe Met Ala Ala Gly Tyr Thr Ala Ile
   1               5                   10                  15
   Glu Lys
<210> 524
   <211> 22
   <212> PRT
   <213> Homo Sapiens
<400> 524
   Met Ser Ala Gly Gly Ala Ser Val Pro Pro Pro Pro Asn Pro Ala Val
   1               5                   10                  15
   Ser Phe Pro Pro Pro Arg
   20
<210> 525
   <211> 11
   <212> PRT
   <213> Homo Sapiens
<400> 525
   Met Ser Asp Val Ser Thr Ser Val Gln Ser Lys
   1               5                   10
<210> 526
   <211> 16
   <212> PRT
   <213> Homo Sapiens
<400> 526
   Met Thr Thr Ser Gly Ala Leu Phe Pro Ser Leu Val Pro Gly Ser Arg
   1               5                   10                  15
<210> 527
   <211> 8
   <212> PRT
   <213> Homo Sapiens
<400> 527
   Met Val Glu Glu Val Asp Gly Arg
   1               5
<210> 528
   <211> 15
   <212> PRT
   <213> Homo Sapiens
<400> 528
   Met Val Phe Phe Val Gln Asn Glu Pro Pro His Gln Ile Phe Lys
   1               5                   10                  15
<210> 529
   <211> 6
   <212> PRT
   <213> Homo Sapiens
<400> 529
   Met Val Trp Glu Gln Arg
   1               5
<210> 530
   <211> 16
   <212> PRT
   <213> Homo Sapiens
<400> 530
   Met Tyr Pro Glu Thr Phe Leu Tyr Val Thr Ser Ser Ala Gly Ile Arg
   1 5 10 15
<210> 531
   <211> 6
   <212> PRT
   <213> Homo Sapiens
<400> 531
   Met Tyr Tyr Thr His Arg
   1 5
<210> 532
   <211> 7
   <212> PRT
   <213> Homo Sapiens
<400> 532
   Met Tyr Tyr Thr His Arg Arg
   1 5
<210> 533
   <211> 8
   <212> PRT
   <213> Homo Sapiens
<400> 533
   Met Tyr Tyr Thr His Arg Arg Arg
   1 5
<210> 534
   <211> 14
   <212> PRT
   <213> Homo Sapiens
<400> 534
   Asn Asp Phe Gln Asn Leu Gln Gln Val Phe Leu Gln Ala Lys
   1 5 10
<210> 535
   <211> 10
   <212> PRT
   <213> Homo Sapiens
<400> 535
   Asn Phe Ala Thr Met Met Asn Phe Val Arg
   1 5 10
<210> 536
   <211> 8
   <212> PRT
   <213> Homo Sapiens
<400> 536
   Asn Gly Val Gly Gly Met Ala Lys
   1 5
<210> 537
   <211> 19
   <212> PRT
   <213> Homo Sapiens
<400> 537
   Asn Gly Val Ile Thr Gln Tyr Ser Val Ala Tyr Glu Ala Val Asp Gly
   1 5 10 15
   Glu Asp Arg
<210> 538
   <211> 10
   <212> PRT
   <213> Homo Sapiens
<400> 538
   Asn Gly Val Ser Gly Leu Val Thr Ser Arg
   1 5 10
<210> 539
   <211> 11
   <212> PRT
   <213> Homo Sapiens
<400> 539
   Asn Ile Ala Phe Tyr Pro Ser Asn His Glu Arg
   1 5 10
<210> 540
   <211> 6
   <212> PRT
   <213> Homo Sapiens
<400> 540
   Asn Ile Phe Tyr Ile Lys
   1 5
<210> 541
   <211> 14
   <212> PRT
   <213> Homo Sapiens
<400> 541
   Asn Leu Ala Leu Phe Glu Glu Glu Leu Asp Ile Arg Pro Lys
   1 5 10
<210> 542
   <211> 15
   <212> PRT
   <213> Homo Sapiens
<400> 542
   Asn Leu Ile Ala Phe Ser Glu Asp Gly Ser Asp Pro Tyr Val Arg
   1 5 10 15
<210> 543
   <211> 8
   <212> PRT
   <213> Homo Sapiens
<400> 543
   Asn Leu Ile Thr Asn Leu Gln Arg
   1 5
<210> 544
   <211> 13
   <212> PRT
   <213> Homo Sapiens
<400> 544
   Asn Leu Gln Ser Gln Ser Ser Val Asn Val Ile Val Lys
   1 5 10
<210> 545
   <211> 12
   <212> PRT
   <213> Homo Sapiens
<400> 545
   Asn Leu Arg Pro Met Leu Ala Ala Asp Ala Gln Arg
   1 5 10
<210> 546
   <211> 11
   <212> PRT
   <213> Homo Sapiens
<400> 546
   Asn Leu Ser Pro Asp Gly Gln Tyr Val Pro Arg
   1 5 10
<210> 547
   <211> 14
   <212> PRT
   <213> Homo Sapiens
<400> 547
   Asn Leu Val Asp Pro Phe Val Glu Val Ser Phe Ala Gly Lys
   1 5 10
<210> 548
   <211> 19
   <212> PRT
   <213> Homo Sapiens
<400> 548
   Asn Met Tyr Leu Thr Gly Leu Cys Phe Leu Leu Gly Pro Thr Gln Leu
   1 5 10 15
   Thr Gln Arg
<210> 549
   <211> 6
   <212> PRT
   <213> Homo Sapiens
<400> 549
   Asn Asn Glu Pro Leu Arg
   1 5
<210> 550
   <211> 8
   <212> PRT
   <213> Homo Sapiens
<400> 550
   Asn Asn Phe Ser Asp Gly Phe Arg
   1 5
<210> 551
   <211> 17
   <212> PRT
   <213> Homo Sapiens
<400> 551
   Asn Pro Asn Phe Asp Ile Cys Thr Leu Phe Met Glu Val Met Leu Pro
   1 5 10 15
   Arg
<210> 552
   <211> 13
   <212> PRT
   <213> Homo Sapiens
<400> 552
   Asn Pro Val Leu Asp Cys Ser Ile Ala Gly Cys Leu Arg
   1 5 10
<210> 553
   <211> 21
   <212> PRT
   <213> Homo Sapiens
<400> 553
   Asn Ser Glu Gly Asp Glu Asn Tyr Met Glu Phe Leu Glu Val Leu Thr
   1 5 10 15
   Glu Gly Leu Glu Arg
   20
<210> 554
   <211> 6
   <212> PRT
   <213> Homo Sapiens
<400> 554
   Asn Ser Tyr Cys Lys Lys
   1 5
<210> 555
   <211> 15
   <212> PRT
   <213> Homo Sapiens
<400> 555
   Asn Val Gly Leu Met Ile Cys Gly His Val His Met Gly Pro Arg
   1 5 10 15
<210> 556
   <211> 16
   <212> PRT
   <213> Homo Sapiens
<400> 556
   Asn Val Gly Leu Met Ile Cys Gly His Val His Met Gly Pro Arg Arg
   1 5 10 15
<210> 557
   <211> 9
   <212> PRT
   <213> Homo Sapiens
<400> 557
   Asn Val His Leu Glu Phe Thr Glu Arg
   1 5
<210> 558
   <211> 10
   <212> PRT
   <213> Homo Sapiens
<400> 558
   Asn Val Leu Glu Leu Ser Asn Val Val Arg
   1 5 10
<210> 559
   <211> 8
   <212> PRT
   <213> Homo Sapiens
<400> 559
   Asn Val Pro Asp Asp Val Leu Arg
   1 5
<210> 560
   <211> 16
   <212> PRT
   <213> Homo Sapiens
<400> 560
   Asn Val Pro Pro Gly Leu Asp Glu Tyr Asn Pro Phe Ser Asp Ser Arg
   1 5 10 15
<210> 561
   <211> 10
   <212> PRT
   <213> Homo Sapiens
<400> 561
   Asn Val Ser Gly Phe Ser Ile Ala Asn Arg
   1 5 10
<210> 562
   <211> 22
   <212> PRT
   <213> Homo Sapiens
<400> 562
   Asn Val Ser Val Gln Pro Glu Ile Ser Glu Gly Leu Ala Thr Thr Pro
   1 5 10 15
   Ser Thr Gln Gln Val Lys
   20
<210> 563
   <211> 7
   <212> PRT
   <213> Homo Sapiens
<400> 563
   Asn Trp Glu Glu Ala Glu Arg
   1 5
<210> 564
   <211> 7
   <212> PRT
   <213> Homo Sapiens
<400> 564
   Asn Tyr Thr Ile Phe Tyr Arg
   1 5
<210> 565
   <211> 6
   <212> PRT
   <213> Homo Sapiens
<400> 565
   Gln Cys Leu Ile Lys Lys
   1 5
<210> 566
   <211> 6
   <212> PRT
   <213> Homo Sapiens
<400> 566
   Gln Glu Glu Leu Glu Arg
   1 5
<210> 567
   <211> 18
   <212> PRT
   <213> Homo Sapiens
<400> 567
   Gln Glu Gln Asp Ile Val Phe Leu Ile Asp Gly Ser Gly Ser Ile Ser
   1 5 10 15
   Ser Arg
<210> 568
   <211> 13
   <212> PRT
   <213> Homo Sapiens
<400> 568
   Gln Glu Ser Thr Ser Val Leu Leu Gln Gln Ser Glu Lys
   1 5 10
<210> 569
   <211> 14
   <212> PRT
   <213> Homo Sapiens
<400> 569
   Gln Glu Ser Thr Ser Val Leu Leu Gln Gln Ser Glu Lys Lys
   1 5 10
<210> 570
   <211> 16
   <212> PRT
   <213> Homo Sapiens
<400> 570
   Gln Phe His Gln Leu Ala Ala Gln Gly Pro Gln Glu Cys Leu Val Arg
   1 5 10 15
<210> 571
   <211> 24
   <212> PRT
   <213> Homo Sapiens
<400> 571
   Gln Phe Gln Phe Met Ala Trp Pro Asp His Gly Val Pro Glu Tyr Pro
   1 5 10 15
   Thr Pro Ile Leu Ala Phe Leu Arg
   20
<210> 572
   <211> 14
   <212> PRT
   <213> Homo Sapiens
<400> 572
   Gln Phe Gln Phe Thr Asp Trp Pro Glu Gln Gly Val Pro Lys
   1 5 10
<210> 573
   <211> 15
   <212> PRT
   <213> Homo Sapiens
<400> 573
   Gln Gly Ala Gln Trp Ala Ala Leu Cys Asp Ser Ser Ser Ala Arg
   1 5 10 15
<210> 574
   <211> 18
   <212> PRT
   <213> Homo Sapiens
<400> 574
   Gln Gly His Phe Tyr Gly Glu Thr Ala Ala Val Tyr Val Ala Val Glu
   1 5 10 15
   Glu Arg
<210> 575
   <211> 19
   <212> PRT
   <213> Homo Sapiens
<400> 575
   Gln Gly His Phe Tyr Gly Glu Thr Ala Ala Val Tyr Val Ala Val Glu
   1 5 10 15
   Glu Arg Lys
<210> 576
   <211> 12
   <212> PRT
   <213> Homo Sapiens
<400> 576
   Gln Gly Leu Thr Val Ser Phe Ile Pro Tyr Phe Lys
   1 5 10
<210> 577
   <211> 9
   <212> PRT
   <213> Homo Sapiens
<400> 577
   Gln Gly Pro Val Gly Ser Gly Arg Arg
   1 5
<210> 578
   <211> 6
   <212> PRT
   <213> Homo Sapiens
<400> 578
   Gln His Gly Gln Ile Arg
   1 5
<210> 579
   <211> 9
   <212> PRT
   <213> Homo Sapiens
<400> 579
   Gln Ile Phe Gly Phe Glu Ser Asn Lys
   1 5
<210> 580
   <211> 19
   <212> PRT
   <213> Homo Sapiens
<400> 580
   Gln Ile Gly Pro Gly Glu Ser Cys Pro Asp Gly Val Thr His Ser Ile
   1 5 10 15
   Ser Gly Arg
<210> 581
   <211> 20
   <212> PRT
   <213> Homo Sapiens
<400> 581
   Gln Asn Ala Tyr Ile Ala Thr Gln Gly Pro Leu Pro Glu Thr Met Gly
   1 5 10 15
   Asp Phe Trp Arg
   20
<210> 582
   <211> 17
   <212> PRT
   <213> Homo Sapiens
<400> 582
   Gln Asn Cys Ser Gln His Glu Ser Ser Pro Asp Ile Ser His Phe Glu
   1 5 10 15
   Arg
<210> 583
   <211> 27
   <212> PRT
   <213> Homo Sapiens
<400> 583
   Gln Pro Gly Asn Met Ala Gly Asn Phe Asn Leu Ser Leu Gln Gly Cys
   1 5 10 15
   Asp Gln Asp Ala Gln Ser Pro Gly Ile Leu Arg
   20 25
<210> 584
   <211> 7
   <212> PRT
   <213> Homo Sapiens
<400> 584
   Gln Pro Gly Ser Val Pro Arg
   1 5
<210> 585
   <211> 15
   <212> PRT
   <213> Homo Sapiens
<400> 585
   Gln Pro His Tyr Ser Ala Phe Gly Ser Val Gly Glu Trp Leu Arg
   1 5 10 15
<210> 586
   <211> 15
   <212> PRT
   <213> Homo Sapiens
<400> 586
   Gln Pro Gln Gln Phe Pro Ser Arg Pro Pro Pro Pro Gln Pro Lys
   1 5 10 15
<210> 587
   <211> 10
   <212> PRT
   <213> Homo Sapiens
<400> 587
   Gln Ser Pro Glu Asp Val Tyr Phe Ser Lys
   1 5 10
<210> 588
   <211> 16
   <212> PRT
   <213> Homo Sapiens
<400> 588
   Gln Ser Ser Gly Glu Asn Cys Asp Val Val Val Asn Thr Leu Gly Lys
   1 5 10 15
<210> 589
   <211> 17
   <212> PRT
   <213> Homo Sapiens
<400> 589
   Gln Ser Ser Gly Glu Asn Cys Asp Val Val Val Asn Thr Leu Gly Lys
   1 5 10 15
   Arg
<210> 590
   <211> 10
   <212> PRT
   <213> Homo Sapiens
<400> 590
   Gln Val Ile Ile Asn Leu Ile Asn Gln Lys
   1 5 10
<210> 591
   <211> 12
   <212> PRT
   <213> Homo Sapiens
<400> 591
   Gln Val Ser Ser Val Asn Glu Glu Asp Phe Val Arg
   1 5 10
<210> 592
   <211> 10
   <212> PRT
   <213> Homo Sapiens
<400> 592
   Gln Trp Ser Asn Val Phe Asn Ile Leu Arg
   1 5 10
<210> 593
   <211> 9
   <212> PRT
   <213> Homo Sapiens
<400> 593
   Gln Tyr Ala Gly Thr Gly Ala Leu Lys
   1 5
<210> 594
   <211> 16
   <212> PRT
   <213> Homo Sapiens
<400> 594
   Gln Tyr Asn Ser Phe Asn Phe Ala Leu Leu Glu Asp Val Gln Ala Lys
   1 5 10 15
<210> 595
   <211> 8
   <212> PRT
   <213> Homo Sapiens
<400> 595
   Arg Ala Glu Ser Asp Ser Arg Lys
   1 5
<210> 596
   <211> 9
   <212> PRT
   <213> Homo Sapiens
<400> 596
   Arg Ala Met Ala Thr Leu Leu Ser Lys
   1 5
<210> 597
   <211> 8
   <212> PRT
   <213> Homo Sapiens
<400> 597
   Arg Ala Pro Ala Phe Glu Gly Arg
   1 5
<210> 598
   <211> 6
   <212> PRT
   <213> Homo Sapiens
<400> 598
   Arg Asp Leu Gly Ser Arg
   1 5
<210> 599
   <211> 10
   <212> PRT
   <213> Homo Sapiens
<400> 599
   Arg Asp Leu Ser Gln Met Glu Ala Leu Lys
   1 5 10
<210> 600
   <211> 6
   <212> PRT
   <213> Homo Sapiens
<400> 600
   Arg Asp Gln Leu Trp Arg
   1               5
<210> 601
   <211> 9
   <212> PRT
   <213> Homo Sapiens
<400> 601
   Arg Glu Ile Thr Tyr Gln Gly Thr Arg
   1               5
<210> 602
   <211> 9
   <212> PRT
   <213> Homo Sapiens
<400> 602
   Arg Glu Gln Leu Thr Glu Thr Asp Lys
   1               5
<210> 603
   <211> 12
   <212> PRT
   <213> Homo Sapiens
<400> 603
   Arg Gly Asp Phe Phe Tyr His Ser Glu Asn Pro Lys
   1               5                   10
<210> 604
   <211> 8
   <212> PRT
   <213> Homo Sapiens
<400> 604
   Arg His Gly Glu Thr Cys Tyr Lys
   1               5
<210> 605
   <211> 5
   <212> PRT
   <213> Homo Sapiens
<400> 605
   Arg Lys Asn Gln Arg
   1 5
<210> 606
   <211> 10
   <212> PRT
   <213> Homo Sapiens
<400> 606
   Arg Leu Asn Tyr Gln Thr Pro Gly Met Arg
   1 5 10
<210> 607
   <211> 7
   <212> PRT
   <213> Homo Sapiens
<400> 607
   Arg Met Glu Pro Leu Glu Lys
   1 5
<210> 608
   <211> 14
   <212> PRT
   <213> Homo Sapiens
<400> 608
   Arg Pro Asp Thr Ser Phe Leu Trp Phe Thr Ser Pro Tyr Lys
   1 5 10
<210> 609
   <211> 8
   <212> PRT
   <213> Homo Sapiens
<400> 609
   Arg Pro Pro Asn Ala Trp His Lys
   1 5
<210> 610
   <211> 8
   <212> PRT
   <213> Homo Sapiens
<400> 610
   Arg Pro Pro Gln Thr Leu Ser Arg
   1 5
<210> 611
   <211> 9
   <212> PRT
   <213> Homo Sapiens
<400> 611
   Arg Gln Asp Leu Glu Val Glu Val Arg
   1 5
<210> 612
   <211> 7
   <212> PRT
   <213> Homo Sapiens
<400> 612
   Arg Gln Glu Glu Leu Glu Arg
   1 5
<210> 613
   <211> 8
   <212> PRT
   <213> Homo Sapiens
<400> 613
   Arg Gln Glu Glu Leu Glu Arg Lys
   1 5
<210> 614
   <211> 6
   <212> PRT
   <213> Homo Sapiens
<400> 614
   Arg Arg Gln Ala Glu Arg
   1 5
<210> 615
   <211> 16
   <212> PRT
   <213> Homo Sapiens
<400> 615
   Arg Thr His Leu Gly Leu Ile Met Asp Gly Trp Asn Ser Met Ile Arg
   1 5 10 15
<210> 616
   <211> 8
   <212> PRT
   <213> Homo Sapiens
<400> 616
   Arg Thr His Ser Pro Ser Ser Lys
   1 5
<210> 617
   <211> 11
   <212> PRT
   <213> Homo Sapiens
<400> 617
   Arg Thr Leu Glu Gln Met Asp Val Val His Arg
   1 5 10
<210> 618
   <211> 6
   <212> PRT
   <213> Homo Sapiens
<400> 618
   Arg Thr Pro Thr Ala Arg
   1 5
<210> 619
   <211> 24
   <212> PRT
   <213> Homo Sapiens
<400> 619
   Ser Ala Asn Tyr Thr Cys Val Ala Ile Ser Ser Leu Gly Met Ile Glu
   1 5 10 15
   Ala Thr Ala Gln Val Thr Val Lys
   20
<210> 620
   <211> 14
   <212> PRT
   <213> Homo Sapiens
<400> 620
   Ser Cys Ser Leu Val Leu Glu His Gln Pro Asp Asn Ile Lys
   1 5 10
<210> 621
   <211> 15
   <212> PRT
   <213> Homo Sapiens
<400> 621
   Ser Asp Met Gly Val Gly Val Phe Thr Pro Thr Ile Glu Ala Arg
   1 5 10 15
<210> 622
   <211> 8
   <212> PRT
   <213> Homo Sapiens
<400> 622
   Ser Asp Pro Tyr Gly Ile Ile Arg
   1 5
<210> 623
   <211> 12
   <212> PRT
   <213> Homo Sapiens
<400> 623
   Ser Asp Gln Ala Leu His Ser Phe Ser Glu Ala Lys
   1 5 10
<210> 624
   <211> 8
   <212> PRT
   <213> Homo Sapiens
<400> 624
   Ser Asp Ser Ala Val Leu Leu Arg
   1 5
<210> 625
   <211> 12
   <212> PRT
   <213> Homo Sapiens
<400> 625
   Ser Asp Thr Ile Ala Asn Tyr Glu Leu Val Tyr Lys
   1 5 10
<210> 626
   <211> 13
   <212> PRT
   <213> Homo Sapiens
<400> 626
   Ser Glu Ile Phe Asn Glu Asn Phe Gly Pro Asp Phe Arg
   1 5 10
<210> 627
   <211> 7
   <212> PRT
   <213> Homo Sapiens
<400> 627
   Ser Glu Ile Ile Asn Ser Lys
   1 5
<210> 628
   <211> 18
   <212> PRT
   <213> Homo Sapiens
<400> 628
   Ser Glu Ile Gln Phe Leu Ile Ser Asp Asn Gln Gly Phe Ser Cys Pro
   1               5                   10                  15
   Glu Lys
<210> 629
   <211> 8
   <212> PRT
   <213> Homo Sapiens
<400> 629
   Ser Glu Gln Leu Lys Pro Leu Lys
   1               5
<210> 630
   <211> 9
   <212> PRT
   <213> Homo Sapiens
<400> 630
   Ser Glu Gln Pro Thr Ala Ser Trp Arg
   1               5
<210> 631
   <211> 16
   <212> PRT
   <213> Homo Sapiens
<400> 631
   Ser Gly Ala Leu Gln Ile Glu Ser Ser Glu Glu Ser Asp Gln Gly Lys
   1               5                   10                  15
<210> 632
   <211> 14
   <212> PRT
   <213> Homo Sapiens
<400> 632
   Ser Gly Glu Gly Phe Ile Asp Phe Ile Gly Gln Val His Lys
   1               5                   10
<210> 633
   <211> 9
   <212> PRT
   <213> Homo Sapiens
<400> 633
   Ser Gly Val Val Cys Gln Thr Gly Arg
   1               5
<210> 634
   <211> 20
   <212> PRT
   <213> Homo Sapiens
<400> 634
   Ser His Ala Glu Asn Pro Thr Ala Ser His Val Asp Asn Glu Tyr Ser
   1 5 10 15
   Gln Pro Pro Arg
   20
<210> 635
   <211> 12
   <212> PRT
   <213> Homo Sapiens
<400> 635
   Ser His Leu Gln Asn Tyr Thr Val Asn Ala Thr Lys
   1 5 10
<210> 636
   <211> 16
   <212> PRT
   <213> Homo Sapiens
<400> 636
   Ser Ile Phe Asp Pro Pro Val Phe Pro Val Cys Met Leu Gly Asn Arg
   1 5 10 15
<210> 637
   <211> 8
   <212> PRT
   <213> Homo Sapiens
<400> 637
   Ser Ile Leu Gln Glu Glu Asn Arg
   1 5
<210> 638
   <211> 9
   <212> PRT
   <213> Homo Sapiens
<400> 638
   Ser Ile Gln Ile His Gly Thr Met Arg
   1 5
<210> 639
   <211> 11
   <212> PRT
   <213> Homo Sapiens
<400> 639
   Ser Leu Glu Met Ser His Asp Glu His Lys Lys
   1 5 10
<210> 640
   <211> 11
   <212> PRT
   <213> Homo Sapiens
<400> 640
   Ser Leu Gly Gly Glu Gly Asn Phe Asn Trp Arg
   1 5 10
<210> 641
   <211> 22
   <212> PRT
   <213> Homo Sapiens
<400> 641
   Ser Leu His Leu Thr Cys Asp Ser Ala Pro Val Gly Ser Gln Gly Thr
   1 5 10 15
   Trp Ser Thr Ser Cys Arg
   20
<210> 642
   <211> 14
   <212> PRT
   <213> Homo Sapiens
<400> 642
   Ser Leu Ser Gly Val Gly Ala Gly Gly Gly Pro Thr Pro Arg
   1 5 10
<210> 643
   <211> 13
   <212> PRT
   <213> Homo Sapiens
<400> 643
   Ser Leu Ser Asn Tyr Pro Phe Asp Phe Gln Gly Ala Arg
   1 5 10
<210> 644
   <211> 13
   <212> PRT
   <213> Homo Sapiens
<400> 644
   Ser Pro Asp Leu Gln Gly Ser Trp Gln Trp Ser Asp Arg
   1 5 10
<210> 645
   <211> 9
   <212> PRT
   <213> Homo Sapiens
<400> 645
   Ser Pro Gly Trp Arg Pro Ala Phe Lys
   1 5
<210> 646
   <211> 18
   <212> PRT
   <213> Homo Sapiens
<400> 646
   Ser Gln Ala Lys Pro Gly Thr Pro Gly Gly Thr Gly Gly Pro Ala Pro
   1 5 10 15
   Gln Tyr
<210> 647
   <211> 17
   <212> PRT
   <213> Homo Sapiens
<400> 647
   Ser Gln His Gln Glu Thr Pro Val Tyr Leu Gly Ala Thr Ala Gly Met
   1 5 10 15
   Arg
<210> 648
   <211> 19
   <212> PRT
   <213> Homo Sapiens
<400> 648
   Ser Gln His Thr Thr Glu Val Val Gly Gly Ala Pro Gln His Glu Gln
   1 5 10 15
   Ile Gly Lys
<210> 649
   <211> 19
   <212> PRT
   <213> Homo Sapiens
<400> 649
   Ser Ser His Ser Gly Val Leu Glu Ile Glu Asn Ser Val Asp Asp Leu
   1 5 10 15
   Ser Ser Arg
<210> 650
   <211> 13
   <212> PRT
   <213> Homo Sapiens
<400> 650
   Ser Ser Leu Pro Tyr Ile Cys Glu Met Thr Ala Phe Arg
   1 5 10
<210> 651
   <211> 14
   <212> PRT
   <213> Homo Sapiens
<400> 651
   Ser Ser Ser Ser Asn Phe Leu Asn Tyr Asp Leu Thr Leu Arg
   1 5 10
<210> 652
   <211> 8
   <212> PRT
   <213> Homo Sapiens
<400> 652
   Ser Ser Val Ile Asn Ser Ile Arg
   1 5
<210> 653
   <211> 14
   <212> PRT
   <213> Homo Sapiens
<400> 653
   Ser Ser Val Ile Ser Ile Tyr Val Ser Glu Ser Met Asp Arg
   1 5 10
<210> 654
   <211> 15
   <212> PRT
   <213> Homo Sapiens
<400> 654
   Ser Thr Glu Glu Phe Pro Pro Leu Gln Pro Ile Leu Gln Gln Lys
   1 5 10 15
<210> 655
   <211> 8
   <212> PRT
   <213> Homo Sapiens
<400> 655
   Ser Thr Glu Thr Ala Leu Tyr Arg
   1 5
<210> 656
   <211> 9
   <212> PRT
   <213> Homo Sapiens
<400> 656
   Ser Thr Glu Thr Ala Leu Tyr Arg Lys
   1 5
<210> 657
   <211> 14
   <212> PRT
   <213> Homo Sapiens
<400> 657
   Ser Thr Gly Pro Gly Ala Ser Leu Gly Thr Gly Tyr Asp Arg
   1 5 10
<210> 658
   <211> 15
   <212> PRT
   <213> Homo Sapiens
<400> 658
   Ser Thr Gly Pro Gly Ala Ser Leu Gly Thr Gly Tyr Asp Arg Lys
   1 5 10 15
<210> 659
   <211> 6
   <212> PRT
   <213> Homo Sapiens
<400> 659
   Ser Thr Ile Glu Lys Lys
   1 5
<210> 660
   <211> 18
   <212> PRT
   <213> Homo Sapiens
<400> 660
   Ser Thr Thr Ser Leu Ser Val Ser Trp Ser Ile Pro Pro Pro Gln Gln
   1               5                   10                  15
   Ser Arg
<210> 661
   <211> 11
   <212> PRT
   <213> Homo Sapiens
<400> 661
   Ser Val Asp Asp Thr Ser Gln Ala Ile Gln Arg
   1               5                   10
<210> 662
   <211> 7
   <212> PRT
   <213> Homo Sapiens
<400> 662
   Ser Val Gly Pro Leu Thr Arg
   1               5
<210> 663
   <211> 8
   <212> PRT
   <213> Homo Sapiens
<400> 663
   Ser Val Gly Pro Leu Thr Arg Lys
   1               5
<210> 664
   <211> 12
   <212> PRT
   <213> Homo Sapiens
<400> 664
   Ser Val Leu Gly Val Ile Thr Ile Val Asn Leu Arg
   1               5                   10
<210> 665
   <211> 6
   <212> PRT
   <213> Homo Sapiens
<400> 665
   Ser Val Gln Met Met Arg
   1               5
<210> 666
   <211> 5
   <212> PRT
   <213> Homo Sapiens
<400> 666
   Ser Tyr Phe Arg Lys
   1 5
<210> 667
   <211> 27
   <212> PRT
   <213> Homo Sapiens
<400> 667
   Ser Tyr Gln Leu Ala Asn Ile Ser Ser Pro Ser Leu Val Val Glu Cys
   1 5 10 15
   Gly Gly Gln Thr Val Gln Ser Cys Val Ile Arg
   20 25
<210> 668
   <211> 9
   <212> PRT
   <213> Homo Sapiens
<400> 668
   Ser Tyr Ser Phe Val Leu Thr Asn Arg
   1 5
<210> 669
   <211> 8
   <212> PRT
   <213> Homo Sapiens
<400> 669
   Thr Ala Leu Leu Asn Met Asp Arg
   1 5
<210> 670
   <211> 22
   <212> PRT
   <213> Homo Sapiens
<400> 670
   Thr Ala Asn Pro Gln Trp Asn Gln Asn Ile Thr Leu Pro Ala Met Phe
   1 5 10 15
   Pro Ser Met Cys Glu Lys
   20
<210> 671
   <211> 21
   <212> PRT
   <213> Homo Sapiens
<400> 671
   Thr Ala Pro Asp Leu Leu Pro His Lys Pro Leu Pro Ala Ser Ala Tyr
   1 5 10 15
   Ile Glu Asp Gly Arg
   20
<210> 672
   <211> 12
   <212> PRT
   <213> Homo Sapiens
<400> 672
   Thr Ala Gln Ser Thr Pro Ser Ala Pro Pro Gln Lys
   1 5 10
<210> 673
   <211> 13
   <212> PRT
   <213> Homo Sapiens
<400> 673
   Thr Ala Ser Val Ser Ile Asn Gln Thr Glu Pro Pro Lys
   1 5 10
<210> 674
   <211> 20
   <212> PRT
   <213> Homo Sapiens
<400> 674
   Thr Ala Thr Met Leu Cys Ala Ala Gly Gly Asn Pro Asp Pro Glu Ile
   1 5 10 15
   Ser Trp Phe Lys
   20
<210> 675
   <211> 20
   <212> PRT
   <213> Homo Sapiens
<400> 675
   Thr Ala Thr Met Leu Cys Ala Ala Ser Gly Asn Pro Asp Pro Glu Ile
   1 5 10 15
   Thr Trp Phe Lys
   20
<210> 676
   <211> 9
   <212> PRT
   <213> Homo Sapiens
<400> 676
   Thr Ala Thr Val Val Met Met Thr Arg
   1 5
<210> 677
   <211> 16
   <212> PRT
   <213> Homo Sapiens
<400> 677
   Thr Ala Val Asp Gly Pro Asp Leu Glu Met Leu Thr Gly Gln Glu Arg
   1 5 10 15
<210> 678
   <211> 6
   <212> PRT
   <213> Homo Sapiens
<400> 678
   Thr Cys Leu Glu Glu Arg
   1 5
<210> 679
   <211> 13
   <212> PRT
   <213> Homo Sapiens
<400> 679
   Thr Cys Ser Ser Asn Leu Thr Leu Thr Ser Gly Ser Lys
   1 5 10
<210> 680
   <211> 7
   <212> PRT
   <213> Homo Sapiens
<400> 680
   Thr Asp Ala Phe Arg Arg Arg
   1 5
<210> 681
   <211> 9
   <212> PRT
   <213> Homo Sapiens
<400> 681
   Thr Asp Ala Leu Leu Gly Glu Phe Arg
   1               5
<210> 682
   <211> 11
   <212> PRT
   <213> Homo Sapiens
<400> 682
   Thr Asp Glu Asp Val Pro Ser Gly Pro Pro Arg
   1               5                   10
<210> 683
   <211> 12
   <212> PRT
   <213> Homo Sapiens
<400> 683
   Thr Asp Glu Asp Val Pro Ser Gly Pro Pro Arg Lys
   1               5                   10
<210> 684
   <211> 12
   <212> PRT
   <213> Homo Sapiens
<400> 684
   Thr Asp Ile Ser Met Ser Asp Phe Glu Asn Ser Arg
   1               5                   10
<210> 685
   <211> 9
   <212> PRT
   <213> Homo Sapiens
<400> 685
   Thr Asp Gln Asp Glu Glu His Cys Arg
   1               5
<210> 686
   <211> 6
   <212> PRT
   <213> Homo Sapiens
<400> 686
   Thr Asp Val His Tyr Arg
   1               5
<210> 687
   <211> 13
   <212> PRT
   <213> Homo Sapiens
<400> 687
   Thr Glu Phe Gln Gln Ile Ile Asn Leu Ala Leu Gln Lys
   1 5 10
<210> 688
   <211> 12
   <212> PRT
   <213> Homo Sapiens
<400> 688
   Thr Phe Gly His Asn Thr Met Asp Ala Val Pro Arg
   1 5 10
<210> 689
   <211> 7
   <212> PRT
   <213> Homo Sapiens
<400> 689
   Thr Phe Ile Asp Thr Val Arg
   1 5
<210> 690
   <211> 9
   <212> PRT
   <213> Homo Sapiens
<400> 690
   Thr Phe Ser Asp Leu Gln Ser Leu Arg
   1 5
<210> 691
   <211> 10
   <212> PRT
   <213> Homo Sapiens
<400> 691
   Thr Phe Ser Asp Leu Gln Ser Leu Arg Lys
   1 5 10
<210> 692
   <211> 13
   <212> PRT
   <213> Homo Sapiens
<400> 692
   Thr Gly Cys Phe Ile Val Ile Asp Ala Met Leu Glu Arg
   1 5 10
<210> 693
   <211> 14
   <212> PRT
   <213> Homo Sapiens
<400> 693
   Thr Gly Glu Gly Phe Ile Asp Phe Ile Gly Gln Val His Lys
   1 5 10
<210> 694
   <211> 11
   <212> PRT
   <213> Homo Sapiens
<400> 694
   Thr Gly Glu Gln Ala Pro Ser Ser Pro Pro Arg
   1 5 10
<210> 695
   <211> 12
   <212> PRT
   <213> Homo Sapiens
<400> 695
   Thr Gly Glu Gln Ala Pro Ser Ser Pro Pro Arg Arg
   1 5 10
<210> 696
   <211> 15
   <212> PRT
   <213> Homo Sapiens
<400> 696
   Thr His Leu Gly Leu Ile Met Asp Gly Trp Asn Ser Met Ile Arg
   1 5 10 15
<210> 697
   <211> 7
   <212> PRT
   <213> Homo Sapiens
<400> 697
   Thr His Gln Ala Phe Met Arg
   1 5
<210> 698
   <211> 8
   <212> PRT
   <213> Homo Sapiens
<400> 698
   Thr His Gln Ala Phe Met Arg Lys
   1 5
<210> 699
   <211> 12
   <212> PRT
   <213> Homo Sapiens
<400> 699
   Thr His Ser Ser Asn Ser Met Leu Val Phe Leu Lys
   1 5 10
<210> 700
   <211> 8
   <212> PRT
   <213> Homo Sapiens
<400> 700
   Thr Ile His Ala Glu Leu Ser Lys
   1 5
<210> 701
   <211> 6
   <212> PRT
   <213> Homo Sapiens
<400> 701
   Thr Ile Asn Phe Ala Arg
   1 5
<210> 702
   <211> 8
   <212> PRT
   <213> Homo Sapiens
<400> 702
   Thr Leu Ala Asp Phe Asp Pro Arg
   1 5
<210> 703
   <211> 10
   <212> PRT
   <213> Homo Sapiens
<400> 703
   Thr Leu Glu Gln Met Asp Val Val His Arg
   1 5 10
<210> 704
   <211> 10
   <212> PRT
   <213> Homo Sapiens
<400> 704
   Thr Leu His Thr Glu Glu Leu Thr Ser Lys
   1 5 10
<210> 705
   <211> 9
   <212> PRT
   <213> Homo Sapiens
<400> 705
   Thr Leu Ser Ala Gly Ala Gly Pro Arg
   1 5
<210> 706
   <211> 27
   <212> PRT
   <213> Homo Sapiens
<400> 706
   Thr Met Gly Met Val Asn Ile Phe Asn Gly Asp Ala Asp Leu Ser Gly
   1 5 10 15
   Met Thr Trp Ser His Gly Leu Ser Val Ser Lys
   20 25
<210> 707
   <211> 14
   <212> PRT
   <213> Homo Sapiens
<400> 707
   Thr Met Leu His Leu Thr Asp Ile Gln Leu Gln Asp Asn Lys
   1 5 10
<210> 708
   <211> 10
   <212> PRT
   <213> Homo Sapiens
<400> 708
   Thr Met Pro Val Glu Gln Val Phe Ala Lys
   1 5 10
<210> 709
   <211> 12
   <212> PRT
   <213> Homo Sapiens
<400> 709
   Thr Asn Glu Asp Val Pro Ser Gly Pro Pro Arg Lys
   1 5 10
<210> 710
   <211> 18
   <212> PRT
   <213> Homo Sapiens
<400> 710
   Thr Asn Glu Pro Val Trp Glu Glu Asn Phe Thr Phe Phe Ile His Asn
   1 5 10 15
   Pro Lys
<210> 711
   <211> 8
   <212> PRT
   <213> Homo Sapiens
<400> 711
   Thr Asn His Phe Thr Ile Pro Lys
   1 5
<210> 712
   <211> 14
   <212> PRT
   <213> Homo Sapiens
<400> 712
   Thr Asn Gln Glu Val Leu Asp Phe Val Val Gly Gly Gly Arg
   1 5 10
<210> 713
   <211> 9
   <212> PRT
   <213> Homo Sapiens
<400> 713
   Thr Asn Ser Ile Leu Phe Tyr Gly Arg
   1 5
<210> 714
   <211> 10
   <212> PRT
   <213> Homo Sapiens
<400> 714
   Thr Asn Val Ile Gln Ser Leu Leu Ala Arg
   1 5 10
<210> 715
   <211> 6
   <212> PRT
   <213> Homo Sapiens
<400> 715
   Thr Asn Trp Val Tyr Arg
   1 5
<210> 716
   <211> 13
   <212> PRT
   <213> Homo Sapiens
<400> 716
   Thr Pro Glu Ser Ser Pro Pro Gly Thr Pro Ile Gly Arg
   1 5 10
<210> 717
   <211> 12
   <212> PRT
   <213> Homo Sapiens
<400> 717
   Thr Pro Leu His Ala Ala Val Ala Ala Asp Ala Arg
   1 5 10
<210> 718
   <211> 9
   <212> PRT
   <213> Homo Sapiens
<400> 718
   Thr Pro Leu Ser Tyr Thr His Trp Arg
   1 5
<210> 719
   <211> 6
   <212> PRT
   <213> Homo Sapiens
<400> 719
   Thr Pro Asn Trp Asp Arg
   1 5
<210> 720
   <211> 7
   <212> PRT
   <213> Homo Sapiens
<400> 720
   Thr Pro Asn Trp Arg Pro Arg
   1 5
<210> 721
   <211> 8
   <212> PRT
   <213> Homo Sapiens
<400> 721
   Thr Pro Pro Pro Gly Gly Val Lys
   1 5
<210> 722
   <211> 19
   <212> PRT
   <213> Homo Sapiens
<400> 722
   Thr Pro Val Ser Thr Ile Ile Met Pro Asn Glu Phe Gln Gln Asp Tyr
   1 5 10 15
   Asp Ile Arg
<210> 723
   <211> 19
   <212> PRT
   <213> Homo Sapiens
<400> 723
   Thr Gln Asp Leu Glu Asn Phe Leu Cys Asn Asn Leu Gln Cys Gly Ser
   1 5 10 15
   Phe Leu Lys
<210> 724
   <211> 15
   <212> PRT
   <213> Homo Sapiens
<400> 724
   Thr Gln Glu Glu Thr Glu Asp Pro Ser Val Ile Gly Glu Phe Lys
   1 5 10 15
<210> 725
   <211> 21
   <212> PRT
   <213> Homo Sapiens
<400> 725
   Thr Gln Gln Gly Val Pro Ala Gln Pro Ala Asp Phe Gln Ala Glu Val
   1               5                   10                  15
   Glu Ser Asp Thr Arg
               20
<210> 726
   <211> 18
   <212> PRT
   <213> Homo Sapiens
<400> 726
   Thr Gln Arg Pro Ala Met Val Gln Thr Glu Asp Gln Tyr Gln Leu Cys
   1               5                   10                  15
   Tyr Arg
<210> 727
   <211> 8
   <212> PRT
   <213> Homo Sapiens
<400> 727
   Thr Ser Gly Phe Pro Ala Lys Lys
   1               5
<210> 728
   <211> 16
   <212> PRT
   <213> Homo Sapiens
<400> 728
   Thr Ser Leu Cys Val Leu Gly Pro Gly Asp Glu Ala Pro Leu Glu Arg
   1               5                   10                  15
<210> 729
   <211> 17
   <212> PRT
   <213> Homo Sapiens
<400> 729
   Thr Ser Leu Cys Val Leu Gly Pro Gly Asp Glu Ala Pro Leu Glu Arg
   1               5                   10                  15
   Lys
<210> 730
   <211> 10
   <212> PRT
   <213> Homo Sapiens
<400> 730
   Thr Ser Ser Gln Pro Gly Phe Leu Glu Arg
   1 5 10
<210> 731
   <211> 20
   <212> PRT
   <213> Homo Sapiens
<400> 731
   Thr Ser Val Leu Leu Ser Trp Glu Ile Pro Glu Asn Tyr Asn Ser Ala
   1 5 10 15
   Met Pro Phe Lys
   20
<210> 732
   <211> 14
   <212> PRT
   <213> Homo Sapiens
<400> 732
   Thr Ser Val Leu Leu Ser Trp Glu Val Pro Asp Ser Tyr Lys
   1 5 10
<210> 733
   <211> 21
   <212> PRT
   <213> Homo Sapiens
<400> 733
   Thr Ser Val Thr Val Ser Asp Leu Glu Pro His Met Asn Tyr Thr Phe
   1 5 10 15
   Thr Val Glu Ala Arg
   20
<210> 734
   <211> 21
   <212> PRT
   <213> Homo Sapiens
<400> 734
   Thr Thr Pro Pro Thr Thr Arg Pro Pro Pro Thr Thr Thr Pro Glu Pro
   1 5 10 15
   Thr Ala Pro Pro Arg
   20
<210> 735
   <211> 12
   <212> PRT
   <213> Homo Sapiens
<400> 735
   Thr Val Asp Ile Tyr Gly His Val Thr Cys Met Arg
   1 5 10
<210> 736
   <211> 12
   <212> PRT
   <213> Homo Sapiens
<400> 736
   Thr Val Asp Ile Tyr Gly His Val Thr Leu Met Arg
   1 5 10
<210> 737
   <211> 23
   <212> PRT
   <213> Homo Sapiens
<400> 737
   Thr Val Gln Thr Ala Ala Ala Asn Ala Ala Ser Thr Ala Ala Ser Ser
   1 5 10 15
   Ala Ala Gln Asn Ala Phe Lys
   20
<210> 738
   <211> 10
   <212> PRT
   <213> Homo Sapiens
<400> 738
   Thr Val Ser Glu Trp Leu Glu Ser Ile Lys
   1 5 10
<210> 739
   <211> 9
   <212> PRT
   <213> Homo Sapiens
<400> 739
   Thr Tyr Glu Val Cys Asp Val Gln Arg
   1 5
<210> 740
   <211> 9
   <212> PRT
   <213> Homo Sapiens
<400> 740
   Thr Tyr Leu Ala Val Gly Ser Met Lys
   1 5
<210> 741
   <211> 13
   <212> PRT
   <213> Homo Sapiens
<400> 741
   Thr Tyr Gln Phe Leu Gln Glu Tyr Leu Asp Ala Ile Lys
   1 5 10
<210> 742
   <211> 17
   <212> PRT
   <213> Homo Sapiens
<400> 742
   Thr Tyr Val Asp Pro His Thr Tyr Glu Asp Pro Asn Gln Ala Val Leu
   1 5 10 15
   Lys
<210> 743
   <211> 8
   <212> PRT
   <213> Homo Sapiens
<400> 743
   Val Ala Lys Pro Glu Glu Met Lys
   1 5
<210> 744
   <211> 10
   <212> PRT
   <213> Homo Sapiens
<400> 744
   Val Ala Asn His Met Asp Gly Phe Gln Arg
   1 5 10
<210> 745
   <211> 13
   <212> PRT
   <213> Homo Sapiens
<400> 745
   Val Ala Gln Pro Gly Pro Leu Glu Pro Glu Glu Pro Arg
   1 5 10
<210> 746
   <211> 12
   <212> PRT
   <213> Homo Sapiens
<400> 746
   Val Ala Thr Pro Leu Pro Asp Pro Met Ala Ser Arg
   1 5 10
<210> 747
   <211> 20
   <212> PRT
   <213> Homo Sapiens
<400> 747
   Val Asp Phe Leu Gly Glu Ala Gly Ile Met Gly Gln Phe Ser His His
   1 5 10 15
   Asn Ile Ile Arg
   20
<210> 748
   <211> 15
   <212> PRT
   <213> Homo Sapiens
<400> 748
   Val Asp Met Thr Phe Glu Glu Glu Ala Gln Leu Leu Gln Leu Lys
   1 5 10 15
<210> 749
   <211> 11
   <212> PRT
   <213> Homo Sapiens
<400> 749
   Val Asp Thr Val Ala Ala Glu His Leu Thr Arg
   1 5 10
<210> 750
   <211> 9
   <212> PRT
   <213> Homo Sapiens
<400> 750
   Val Asp Val Gly Gln Gln Pro Leu Arg
   1 5
<210> 751
   <211> 11
   <212> PRT
   <213> Homo Sapiens
<400> 751
   Val Glu Ala Ser Asn Pro Tyr Val Glu Pro Arg
   1 5 10
<210> 752
   <211> 8
   <212> PRT
   <213> Homo Sapiens
<400> 752
   Val Glu Asp Phe Asp Ala Leu Lys
   1 5
<210> 753
   <211> 11
   <212> PRT
   <213> Homo Sapiens
<400> 753
   Val Glu Asp Leu Pro Ala Asp Asp Ile Leu Arg
   1 5 10
<210> 754
   <211> 20
   <212> PRT
   <213> Homo Sapiens
<400> 754
   Val Glu Leu Pro Gly Thr Ala Val Pro Ser Val Pro Glu Asp Ala Ala
   1 5 10 15
   Pro Ala Ser Arg
   20
<210> 755
   <211> 12
   <212> PRT
   <213> Homo Sapiens
<400> 755
   Val Glu Val Glu Ala Val Asn Ser Thr Ser Val Lys
   1 5 10
<210> 756
   <211> 16
   <212> PRT
   <213> Homo Sapiens
<400> 756
   Val Glu Val Glu Pro Leu Asn Ser Thr Ala Val His Val Tyr Trp Lys
   1 5 10 15
<210> 757
   <211> 16
   <212> PRT
   <213> Homo Sapiens
<400> 757
   Val Glu Tyr Tyr Ile Pro Gly Ser Thr Thr Asn Pro Glu Val Phe Lys
   1 5 10 15
<210> 758
   <211> 15
   <212> PRT
   <213> Homo Sapiens
<400> 758
   Val Phe Ala Gln Asn Glu Glu Ile Gln Glu Met Ala Gln Asn Lys
   1 5 10 15
<210> 759
   <211> 6
   <212> PRT
   <213> Homo Sapiens
<400> 759
   Val Phe His Ala Glu Arg
   1 5
<210> 760
   <211> 7
   <212> PRT
   <213> Homo Sapiens
<400> 760
   Val Phe His Arg Pro Trp Arg
   1 5
<210> 761
   <211> 9
   <212> PRT
   <213> Homo Sapiens
<400> 761
   Val Phe Leu Ser Ile Glu Glu Phe Arg
   1 5
<210> 762
   <211> 10
   <212> PRT
   <213> Homo Sapiens
<400> 762
   Val Gly Glu Glu Asp Asp Gly Glu Tyr Arg
   1 5 10
<210> 763
   <211> 9
   <212> PRT
   <213> Homo Sapiens
<400> 763
   Val Gly Phe Gly Glu Glu Met Val Lys
   1 5
<210> 764
   <211> 9
   <212> PRT
   <213> Homo Sapiens
<400> 764
   Val Gly Gly Ser Met Leu Thr Pro Arg
   1 5
<210> 765
   <211> 9
   <212> PRT
   <213> Homo Sapiens
<400> 765
   Val Gly Asn Gln Ile Phe Gln Ser Arg
   1 5
<210> 766
   <211> 6
   <212> PRT
   <213> Homo Sapiens
<400> 766
   Val His Gly Leu Tyr Arg
   1 5
<210> 767
   <211> 17
   <212> PRT
   <213> Homo Sapiens
<400> 767
   Val Ile Glu Glu Ala Val Tyr Phe His Gln His Ser Ile Leu Ala Cys
   1 5 10 15
   Lys
<210> 768
   <211> 6
   <212> PRT
   <213> Homo Sapiens
<400> 768
   Val Ile Glu Leu Asn Lys
   1 5
<210> 769
   <211> 12
   <212> PRT
   <213> Homo Sapiens
<400> 769
   Val Ile Gly Ala Gly Glu Phe Gly Glu Val Tyr Lys
   1 5 10
<210> 770
   <211> 19
   <212> PRT
   <213> Homo Sapiens
<400> 770
   Val Leu Ala Phe Thr Ala Val Gly Asp Gly Pro Pro Ser Pro Thr Ile
   1 5 10 15
   Gln Val Lys
<210> 771
   <211> 12
   <212> PRT
   <213> Homo Sapiens
<400> 771
   Val Leu Ala Leu Leu Cys Ser Gly Phe Gln Pro Lys
   1 5 10
<210> 772
   <211> 23
   <212> PRT
   <213> Homo Sapiens
<400> 772
   Val Leu Ala Pro Ala Ser Thr Leu Gln Ser Ser Tyr Gln Ile Pro Thr
   1 5 10 15
   Glu Asn Ser Met Thr Ala Arg
   20
<210> 773
   <211> 16
   <212> PRT
   <213> Homo Sapiens
<400> 773
   Val Leu Ala Gln Gln Gly Glu Tyr Ser Glu Ala Ile Pro Ile Leu Arg
   1 5 10 15
<210> 774
   <211> 9
   <212> PRT
   <213> Homo Sapiens
<400> 774
   Val Leu Ala Val Asn Ser Ile Gly Arg
   1 5
<210> 775
   <211> 10
   <212> PRT
   <213> Homo Sapiens
<400> 775
   Val Leu Asp Ala Gly Asp Pro Thr Ser Arg
   1 5 10
<210> 776
   <211> 7
   <212> PRT
   <213> Homo Sapiens
<400> 776
   Val Leu Asp Ser Gly Phe Arg
   1 5
<210> 777
   <211> 16
   <212> PRT
   <213> Homo Sapiens
<400> 777
   Val Leu Glu Asp Asp Pro Glu Ala Thr Tyr Thr Thr Ser Gly Gly Lys
   1 5 10 15
<210> 778
   <211> 17
   <212> PRT
   <213> Homo Sapiens
<400> 778
   Val Leu Glu Gly Met Val Glu Glu Asn Gln Val Asn Val Glu Val Thr
   1 5 10 15
   Arg
<210> 779
   <211> 7
   <212> PRT
   <213> Homo Sapiens
<400> 779
   Val Leu Glu Ile Pro Tyr Lys
   1 5
<210> 780
   <211> 7
   <212> PRT
   <213> Homo Sapiens
<400> 780
   Val Leu Glu Asn Tyr Asn Lys
   1 5
<210> 781
   <211> 14
   <212> PRT
   <213> Homo Sapiens
<400> 781
   Val Leu His Phe Asp Gln Val Thr Glu Asn Thr Thr Glu Lys
   1 5 10
<210> 782
   <211> 24
   <212> PRT
   <213> Homo Sapiens
<400> 782
   Val Leu Met Asp Glu Gly Ala Val Leu Thr Leu Val Ala Asp Leu Ser
   1 5 10 15
   Ser Ala Thr Leu Asp Ile Ser Lys
   20
<210> 783
   <211> 14
   <212> PRT
   <213> Homo Sapiens
<400> 783
   Val Leu Met Glu Ile Gln Asp Leu Met Phe Glu Glu Met Arg
   1 5 10
<210> 784
   <211> 12
   <212> PRT
   <213> Homo Sapiens
<400> 784
   Val Leu Val Ala Leu Ala Ser Glu Glu Leu Ala Lys
   1 5 10
<210> 785
   <211> 12
   <212> PRT
   <213> Homo Sapiens
<400> 785
   Val Met Cys Val Ser Met Gly Ser Thr Thr Val Arg
   1 5 10
<210> 786
   <211> 20
   <212> PRT
   <213> Homo Sapiens
<400> 786
   Val Asn Asp Ser Asn Glu Leu Gly Gly Leu Thr Thr Ser Gly Ser Ala
   1 5 10 15
   Glu Val His Lys
   20
<210> 787
   <211> 10
   <212> PRT
   <213> Homo Sapiens
<400> 787
   Val Pro Ala His Gln Val Leu Phe Ser Arg
   1 5 10
<210> 788
   <211> 8
   <212> PRT
   <213> Homo Sapiens
<400> 788
   Val Pro Cys His Phe Pro Cys Lys
   1 5
<210> 789
   <211> 26
   <212> PRT
   <213> Homo Sapiens
<400> 789
   Val Pro Glu Asp Gln Thr Gly Leu Ser Gly Gly Val Ala Ser Phe Val
   1               5                   10                  15
   Cys Gln Ala Thr Gly Glu Pro Lys Pro Arg
               20                  25
<210> 790
   <211> 8
   <212> PRT
   <213> Homo Sapiens
<400> 790
   Val Pro Glu Gly Phe Thr Cys Arg
   1               5
<210> 791
   <211> 10
   <212> PRT
   <213> Homo Sapiens
<400> 791
   Val Pro Glu Gly Leu Glu Asp Val Ser Lys
   1               5                   10
<210> 792
   <211> 17
   <212> PRT
   <213> Homo Sapiens
<400> 792
   Val Pro Leu Ser Gln Leu Leu Thr Ser Glu Asp Met Thr Val Ser Gln
   1               5                   10                  15
   Arg
<210> 793
   <211> 13
   <212> PRT
   <213> Homo Sapiens
<400> 793
   Val Gln Cys Ser Glu Gln Trp Ile Pro Phe Gln Asn Lys
   1               5                   10
<210> 794
   <211> 7
   <212> PRT
   <213> Homo Sapiens
<400> 794
   Val Gln Val Ile Glu Gly Arg
   1 5
<210> 795
   <211> 9
   <212> PRT
   <213> Homo Sapiens
<400> 795
   Val Gln Trp Arg Pro Gln Gly Thr Arg
   1 5
<210> 796
   <211> 8
   <212> PRT
   <213> Homo Sapiens
<400> 796
   Val Ser Asp Phe Gly Leu Ser Arg
   1 5
<210> 797
   <211> 10
   <212> PRT
   <213> Homo Sapiens
<400> 797
   Val Ser Thr Glu Val Thr Leu Ala Val Lys
   1 5 10
<210> 798
   <211> 15
   <212> PRT
   <213> Homo Sapiens
<400> 798
   Val Ser Trp Ser Pro Ala Glu Asp His Asn Ala Pro Ile Glu Lys
   1 5 10 15
<210> 799
   <211> 11
   <212> PRT
   <213> Homo Sapiens
<400> 799
   Val Ser Trp Val Pro Pro Pro Ala Asp Ser Arg
   1 5 10
<210> 800
   <211> 8
   <212> PRT
   <213> Homo Sapiens
<400> 800
   Val Thr Ala Ala Gly Gln Thr Lys
   1 5
<210> 801
   <211> 6
   <212> PRT
   <213> Homo Sapiens
<400> 801
   Val Thr Glu Met Met Lys
   1 5
<210> 802
   <211> 7
   <212> PRT
   <213> Homo Sapiens
<400> 802
   Val Thr Glu Met Met Lys Lys
   1 5
<210> 803
   <211> 21
   <212> PRT
   <213> Homo Sapiens
<400> 803
   Val Thr Phe Asp Pro Thr Ser Ser Tyr Thr Leu Glu Asp Leu Lys Pro
   1 5 10 15
   Asp Thr Leu Tyr Arg
   20
<210> 804
   <211> 20
   <212> PRT
   <213> Homo Sapiens
<400> 804
   Val Thr Phe Thr Cys Gln Ala Ser Phe Asp Pro Ser Leu Gln Pro Ser
   1 5 10 15
   Ile Thr Trp Arg
   20
<210> 805
   <211> 11
   <212> PRT
   <213> Homo Sapiens
<400> 805
   Val Thr Leu Pro Ala Gly Pro Asp Leu Leu Arg
   1 5 10
<210> 806
   <211> 9
   <212> PRT
   <213> Homo Sapiens
<400> 806
   Val Thr Gln Glu Ile Val Thr Glu Arg
   1 5
<210> 807
   <211> 8
   <212> PRT
   <213> Homo Sapiens
<400> 807
   Val Thr Val Ala Ile Pro Leu Lys
   1 5
<210> 808
   <211> 8
   <212> PRT
   <213> Homo Sapiens
<400> 808
   Val Thr Tyr Gln Asn His Asn Lys
   1 5
<210> 809
   <211> 8
   <212> PRT
   <213> Homo Sapiens
<400> 809
   Val Val Glu Met Gln Gly Val Arg
   1 5
<210> 810
   <211> 10
   <212> PRT
   <213> Homo Sapiens
<400> 810
   Val Val Phe Gln Ile Trp Asp Asn Asp Lys
   1 5 10
<210> 811
   <211> 14
   <212> PRT
   <213> Homo Sapiens
<400> 811
   Val Val Leu Leu Glu Asp Leu Ala Ser Gln Val Gly Leu Arg
   1 5 10
<210> 812
   <211> 17
   <212> PRT
   <213> Homo Sapiens
<400> 812
   Val Val Pro Ser Phe Leu Pro Val Asp Gln Gly Gly Ser Leu Val Gly
   1 5 10 15
   Arg
<210> 813
   <211> 10
   <212> PRT
   <213> Homo Sapiens
<400> 813
   Val Val Gln Met Thr Asn Asp Asp Ile Lys
   1 5 10
<210> 814
   <211> 11
   <212> PRT
   <213> Homo Sapiens
<400> 814
   Val Val Gln Met Thr Asn Asp Asp Ile Lys Arg
   1 5 10
<210> 815
   <211> 7
   <212> PRT
   <213> Homo Sapiens
<400> 815
   Val Val Tyr Ser Ala Pro Arg
   1 5
<210> 816
   <211> 24
   <212> PRT
   <213> Homo Sapiens
<400> 816
   Val Tyr Ala Pro Ala Ser Thr Leu Val Asp Gln Pro Tyr Ala Asn Glu
   1               5                   10                  15
   Gly Thr Val Val Val Thr Glu Arg
               20
<210> 817
   <211> 16
   <212> PRT
   <213> Homo Sapiens
<400> 817
   Val Tyr Asp Ser Leu Leu Ala Leu Pro Gln Asp Leu Gln Ala Ala Arg
   1               5                   10                  15
<210> 818
   <211> 16
   <212> PRT
   <213> Homo Sapiens
<400> 818
   Val Tyr Ile Asp Pro Phe Thr Tyr Glu Asp Pro Asn Glu Ala Val Arg
   1               5                   10                  15
<210> 819
   <211> 8
   <212> PRT
   <213> Homo Sapiens
<400> 819
   Val Tyr Thr Glu Asn Val Asp Lys
   1               5
<210> 820
   <211> 9
   <212> PRT
   <213> Homo Sapiens
<400> 820
   Val Tyr Thr Glu Asn Val Asp Lys Arg
   1               5
<210> 821
   <211> 8
   <212> PRT
   <213> Homo Sapiens
<400> 821
   Val Tyr Thr Val Asp Leu Gly Arg
   1 5
<210> 822
   <211> 5
   <212> PRT
   <213> Homo Sapiens
<400> 822
   Val Tyr Tyr Lys Lys
   1 5
<210> 823
   <211> 8
   <212> PRT
   <213> Homo Sapiens
<400> 823
   Val Tyr Tyr Thr Pro Asp Ser Arg
   1 5
<210> 824
   <211> 12
   <212> PRT
   <213> Homo Sapiens
<400> 824
   Trp Glu Asp Glu Glu Trp Ser Thr Asp Leu Asn Arg
   1 5 10
<210> 825
   <211> 5
   <212> PRT
   <213> Homo Sapiens
<400> 825
   Trp Glu Glu His Arg
   1 5
<210> 826
   <211> 6
   <212> PRT
   <213> Homo Sapiens
<400> 826
   Trp Glu Glu Val Cys Arg
   1 5
<210> 827
   <211> 15
   <212> PRT
   <213> Homo Sapiens
<400> 827
   Trp Leu Leu Leu Ser Asp Pro Asp Asp Phe Ser Ala Gly Ala Arg
   1               5                   10                  15
<210> 828
   <211> 12
   <212> PRT
   <213> Homo Sapiens
<400> 828
   Trp Leu Arg Pro Ser Gly Pro Met Pro Ala Asp Arg
   1               5                   10
<210> 829
   <211> 12
   <212> PRT
   <213> Homo Sapiens
<400> 829
   Trp Met Asp Trp Asn Ala Pro Gln Val Gln Tyr Arg
   1               5                   10
<210> 830
   <211> 10
   <212> PRT
   <213> Homo Sapiens
<400> 830
   Trp Met Met Gly Ala Glu Glu Leu Thr Lys
   1               5                   10
<210> 831
   <211> 10
   <212> PRT
   <213> Homo Sapiens
<400> 831
   Trp Arg Pro Val Asp Leu Ala Gln Val Lys
   1               5                   10
<210> 832
   <211> 6
   <212> PRT
   <213> Homo Sapiens
<400> 832
   Trp Ser Asn Val Phe Lys
   1               5
<210> 833
   <211> 10
   <212> PRT
   <213> Homo Sapiens
<400> 833
   Trp Thr Ala Pro Glu Ala Ile Ala Phe Arg
   1 5 10
<210> 834
   <211> 10
   <212> PRT
   <213> Homo Sapiens
<400> 834
   Trp Thr Ala Pro Glu Ala Ile Ser Tyr Arg
   1 5 10
<210> 835
   <211> 11
   <212> PRT
   <213> Homo Sapiens
<400> 835
   Trp Thr Ala Pro Glu Ala Ile Ser Tyr Arg Lys
   1 5 10
<210> 836
   <211> 5
   <212> PRT
   <213> Homo Sapiens
<400> 836
   Trp Thr Glu Tyr Arg
   1 5
<210> 837
   <211> 10
   <212> PRT
   <213> Homo Sapiens
<400> 837
   Trp Thr Pro Pro Gln Asp Ser Gly Gly Arg
   1 5 10
<210> 838
   <211> 6
   <212> PRT
   <213> Homo Sapiens
<400> 838
   Trp Val Ser Gln His Arg
   1 5
<210> 839
   <211> 12
   <212> PRT
   <213> Homo Sapiens
<400> 839
   Tyr Ala Ile Gly Val Gly Leu Ala Phe Gln Asn Arg
   1 5 10
<210> 840
   <211> 11
   <212> PRT
   <213> Homo Sapiens
<400> 840
   Tyr Ala Asn Val Ile Ala Tyr Asp His Ser Arg
   1 5 10
<210> 841
   <211> 15
   <212> PRT
   <213> Homo Sapiens
<400> 841
   Tyr Ala Val Tyr Thr Val Val Ser Ser His Glu Gln Phe Thr Lys
   1 5 10 15
<210> 842
   <211> 8
   <212> PRT
   <213> Homo Sapiens
<400> 842
   Tyr Asp Ile Glu Phe Glu Asp Lys
   1 5
<210> 843
   <211> 12
   <212> PRT
   <213> Homo Sapiens
<400> 843
   Tyr Glu Gly Val Val Asp Ile Phe Gln Thr Val Lys
   1 5 10
<210> 844
   <211> 12
   <212> PRT
   <213> Homo Sapiens
<400> 844
   Tyr Glu Gly Val Val Asp Met Phe Gln Thr Val Lys
   1 5 10
<210> 845
   <211> 6
   <212> PRT
   <213> Homo Sapiens
<400> 845
   Tyr Glu Val Thr Tyr Arg
   1 5
<210> 846
   <211> 7
   <212> PRT
   <213> Homo Sapiens
<400> 846
   Tyr Glu Val Thr Tyr Arg Lys
   1 5
<210> 847
   <211> 8
   <212> PRT
   <213> Homo Sapiens
<400> 847
   Tyr Glu Val Thr Tyr Arg Lys Lys
   1 5
<210> 848
   <211> 9
   <212> PRT
   <213> Homo Sapiens
<400> 848
   Tyr Phe Asp Trp Ile Leu Ile Ser Arg
   1 5
<210> 849
   <211> 10
   <212> PRT
   <213> Homo Sapiens
<400> 849
   Tyr Phe Asp Trp Ile Leu Ile Ser Arg Arg
   1 5 10
<210> 850
   <211> 7
   <212> PRT
   <213> Homo Sapiens
<400> 850
   Tyr Phe Ile Glu Asp Gly Arg
   1               5
<210> 851
   <211> 7
   <212> PRT
   <213> Homo Sapiens
<400> 851
   Tyr Phe Trp Thr Gly Leu Arg
   1               5
<210> 852
   <211> 6
   <212> PRT
   <213> Homo Sapiens
<400> 852
   Tyr Gly Ile Gln Glu Lys
   1               5
<210> 853
   <211> 18
   <212> PRT
   <213> Homo Sapiens
<400> 853
   Tyr Gly Ile Val Leu Asp Ala Gly Ser Ser His Thr Ser Leu Tyr Ile
   1               5                   10                  15
   Tyr Lys
<210> 854
   <211> 8
   <212> PRT
   <213> Homo Sapiens
<400> 854
   Tyr Ile Ala Phe Asp Phe His Lys
   1               5
<210> 855
   <211> 9
   <212> PRT
   <213> Homo Sapiens
<400> 855
   Tyr Ile Glu Tyr Gln Gly Ala Glu Lys
   1               5
<210> 856
   <211> 9
   <212> PRT
   <213> Homo Sapiens
<400> 856
   Tyr Lys Pro Leu Pro Gln Ile Ser Lys
   1 5
<210> 857
   <211> 18
   <212> PRT
   <213> Homo Sapiens
<400> 857
   Tyr Lys Pro Met Met Ile Ile Thr Glu Tyr Met Glu Asn Gly Ala Leu
   1 5 10 15
   Asp Lys
<210> 858
   <211> 9
   <212> PRT
   <213> Homo Sapiens
<400> 858
   Tyr Lys Pro Thr Pro Ile Pro Ile Lys
   1 5
<210> 859
   <211> 10
   <212> PRT
   <213> Homo Sapiens
<400> 859
   Tyr Leu Ala Glu Met Ser Tyr Val His Arg
   1 5 10
<210> 860
   <211> 10
   <212> PRT
   <213> Homo Sapiens
<400> 860
   Tyr Leu Ala Asn Met Asn Tyr Val His Arg
   1 5 10
<210> 861
   <211> 32
   <212> PRT
   <213> Homo Sapiens
<400> 861
   Tyr Leu Cys Gly Ala His Ser Asp Gly Gln Leu Gln Glu Gly Ser Pro
   1               5                   10                  15
   Ile Gln Ala Trp Gln Leu Phe Val Asn Glu Glu Ser Thr Ile Pro Arg
               20                  25                  30
<210> 862
   <211> 13
   <212> PRT
   <213> Homo Sapiens
<400> 862
   Tyr Leu Gln Val Ser Gln Gln Leu Gln Gln Thr Asn Arg
   1               5                   10
<210> 863
   <211> 16
   <212> PRT
   <213> Homo Sapiens
<400> 863
   Tyr Met Thr Glu Thr Trp Asp Pro Ser His Ala Pro Asp Asn Phe Arg
   1               5                   10                  15
<210> 864
   <211> 10
   <212> PRT
   <213> Homo Sapiens
<400> 864
   Tyr Pro Asp Leu Ile Ala Glu Leu Leu Arg
   1               5                   10
<210> 865
   <211> 8
   <212> PRT
   <213> Homo Sapiens
<400> 865
   Tyr Pro Glu Val Gly Asp Leu Arg
   1               5
<210> 866
   <211> 5
   <212> PRT
   <213> Homo Sapiens
<400> 866
   Tyr Pro Trp His Arg
   1               5
<210> 867
   <211> 6
   <212> PRT
   <213> Homo Sapiens
<400> 867
   Tyr Gln His Thr Gly Lys
   1 5
<210> 868
   <211> 9
   <212> PRT
   <213> Homo Sapiens
<400> 868
   Tyr Gln Val Asn Asn Leu Gly Gln Arg
   1 5
<210> 869
   <211> 20
   <212> PRT
   <213> Homo Sapiens
<400> 869
   Tyr Gln Tyr Phe Val Val Asp Pro Met Ala Glu Tyr Asn Met Pro Gln
   1 5 10 15
   Tyr Ile Leu Arg
   20
<210> 870
   <211> 10
   <212> PRT
   <213> Homo Sapiens
<400> 870
   Tyr Ser Ala Pro Ala Asn Leu Tyr Val Arg
   1 5 10
<210> 871
   <211> 10
   <212> PRT
   <213> Homo Sapiens
<400> 871
   Tyr Ser Glu Pro Pro His Gly Leu Thr Arg
   1 5 10
<210> 872
   <211> 15
   <212> PRT
   <213> Homo Sapiens
<400> 872
   Tyr Ser Ile Gly Gly Leu Ser Pro Phe Ser Glu Tyr Ala Phe Arg
   1               5                   10                  15
<210> 873
   <211> 15
   <212> PRT
   <213> Homo Sapiens
<400> 873
   Tyr Ser Val Ala Gly Leu Ser Pro Tyr Ser Asp Tyr Glu Phe Arg
   1               5                   10                  15
<210> 874
   <211> 8
   <212> **PRT**
   <213> Homo Sapiens
<400> 874
   Tyr Ser Tyr Asn Thr Glu Trp Arg
   1               5
<210> 875
   <211> 6
   <212> PRT
   <213> Homo Sapiens
<400> 875
   Tyr Val Cys Lys Arg Lys
   1               5
<210> 876
   <211> 10
   <212> PRT
   <213> Homo Sapiens
<400> 876
   Tyr Val Gln Asn Gly Thr Tyr Thr Val Lys
   1               5                   10
<210> 877
   <211> 19
   <212> PRT
   <213> Homo Sapiens
<400> 877
   Ala Ala Gly Ser Arg Asp Val Ser Leu Ala Lys Ala Asp Ala Ala Pro
   1               5                   10                  15
   Asp Glu Lys
<210> 878
   <211> 20
   <212> PRT
   <213> Homo Sapiens
<400> 878
   Ala Glu Ala Phe Gly Met Asp Pro Ala Arg Pro Asp Gln Ala Ser Thr
   1 5 10 15
   Val Ala Val Lys
   20
<210> 879
   <211> 6
   <212> PRT
   <213> Homo Sapiens
<400> 879
   Ala Glu Asp Ile Ile Lys
   1 5
<210> 880
   <211> 7
   <212> PRT
   <213> Homo Sapiens
<400> 880
   Ala Glu Leu Arg Gly Leu Lys
   1 5
<210> 881
   <211> 20
   <212> PRT
   <213> Homo Sapiens
<400> 881
   Ala Gly Phe Val Gly Gly Gln Phe Trp Ser Val Tyr Thr Pro Cys Asp
   1 5 10 15
   Thr Gln Asn Lys
   20
<210> 882
   <211> 14
   <212> PRT
   <213> Homo Sapiens
<400> 882
   Ala Asn Leu Ser Gln Val Ala Asp His Leu Asp His Ile Lys
   1 5 10
<210> 883
   <211> 7
   <212> PRT
   <213> Homo Sapiens
<400> 883
   Ala Arg Glu Val Ile Pro Arg
   1 5
<210> 884
   <211> 8
   <212> PRT
   <213> Homo Sapiens
<400> 884
   Ala Thr Gly Thr Ala Phe Arg Arg
   1 5
<210> 885
   <211> 6
   <212> PRT
   <213> Homo Sapiens
<400> 885
   Ala Val Leu Gly Asp Arg
   1 5
<210> 886
   <211> 9
   <212> PRT
   <213> Homo Sapiens
<400> 886
   Ala Val Thr Ser Pro Ala Val Gly Arg
   1 5
<210> 887
   <211> 10
   <212> PRT
   <213> Homo Sapiens
<400> 887
   Cys Gly Val Ser Asn Lys Asp Ile Glu Lys
   1 5 10
<210> 888
   <211> 17
   <212> PRT
   <213> Homo Sapiens
<400> 888
   Cys Leu Asn Pro Val Pro Ala Val Pro Ser Pro Ser Pro Ser Val Arg
   1 5 10 15
   Lys
<210> 889
   <211> 11
   <212> PRT
   <213> Homo Sapiens
<400> 889
   Asp Cys Lys Ile Arg Val Phe Ile Gly Gly Lys
   1 5 10
<210> 890
   <211> 9
   <212> PRT
   <213> Homo Sapiens
<400> 890
   Asp Ile Ala Pro Val Leu Asp Leu Lys
   1 5
<210> 891
   <211> 11
   <212> PRT
   <213> Homo Sapiens
<400> 891
   Asp Ile Met Lys Lys Thr Ile Asn Phe Ala Arg
   1 5 10
<210> 892
   <211> 9
   <212> PRT
   <213> Homo Sapiens
<400> 892
   Asp Pro Cys Phe His Pro Gly Tyr Lys
   1 5
<210> 893
   <211> 5
   <212> PRT
   <213> Homo Sapiens
<400> 893
   Asp Pro Glu Leu Arg
   1 5
<210> 894
   <211> 22
   <212> PRT
   <213> Homo Sapiens
<400> 894
   Asp Ser Pro Val Ile Asp Gly His Asn Asp Leu Pro Trp Gln Leu Leu
   1               5                   10                  15
   Asp Met Phe Asn Asn Arg
               20
<210> 895
   <211> 10
   <212> PRT
   <213> Homo Sapiens
<400> 895
   Asp Ser Gln Met Gln Asn Pro Tyr Ser Arg
   1               5                   10
<210> 896
   <211> 5
   <212> PRT
   <213> Homo Sapiens
<400> 896
   Asp Thr Tyr Leu Lys
   1               5
<210> 897
   <211> 6
   <212> PRT
   <213> Homo Sapiens
<400> 897
   Asp Val Leu Pro Gln Lys
   1               5
<210> 898
   <211> 5
   <212> PRT
   <213> Homo Sapiens
<400> 898
   Glu Gly Ile Ala Lys
   1               5
<210> 899
   <211> 8
   <212> PRT
   <213> Homo Sapiens
<400> 899
   Glu Ile Asp Val Ser Tyr Val Lys
   1 5
<210> 900
   <211> 9
   <212> PRT
   <213> Homo Sapiens
<400> 900
   Glu Leu Asn Glu His Phe Lys Ser Lys
   1 5
<210> 901
   <211> 6
   <212> PRT
   <213> Homo Sapiens
<400> 901
   Glu Leu Arg Glu Val Arg
   1 5
<210> 902
   <211> 6
   <212> PRT
   <213> Homo Sapiens
<400> 902
   Glu Leu Val Ser Leu Lys
   1 5
<210> 903
   <211> 7
   <212> PRT
   <213> Homo Sapiens
<400> 903
   Glu Gln Asp Ile Ala Asp Lys
   1 5
<210> 904
   <211> 7
   <212> PRT
   <213> Homo Sapiens
<400> 904
   Glu Gln Leu Thr Val Ile Arg
   1 5
<210> 905
   <211> 8
   <212> PRT
   <213> Homo Sapiens
<400> 905
   Glu Arg Glu Ala Gln Leu Val Lys
   1 5
<210> 906
   <211> 5
   <212> PRT
   <213> Homo Sapiens
<400> 906
   Glu Arg Met Phe Arg
   1 5
<210> 907
   <211> 6
   <212> PRT
   <213> Homo Sapiens
<400> 907
   Glu Ser Lys Ser Ile Lys
   1 5
<210> 908
   <211> 8
   <212> PRT
   <213> Homo Sapiens
<400> 908
   Glu Val Cys Gln Leu Leu Leu Arg
   1 5
<210> 909
   <211> 5
   <212> PRT
   <213> Homo Sapiens
<400> 909
   Glu Val Ile Pro Arg
   1 5
<210> 910
   <211> 7
   <212> PRT
   <213> Homo Sapiens
<400> 910
   Phe Leu Ile Thr Arg Arg Arg
   1 5
<210> 911
   <211> 13
   <212> PRT
   <213> Homo Sapiens
<400> 911
   Phe Leu Asn Leu Thr Ser Glu Lys Val Ser Gln Glu Lys
   1 5 10
<210> 912
   <211> 9
   <212> PRT
   <213> Homo Sapiens
<400> 912
   Phe Ser Asp Val Thr Gly Lys Ile Lys
   1 5
<210> 913
   <211> 9
   <212> PRT
   <213> Homo Sapiens
<400> 913
   Gly His Glu Val Ala Ala Met Leu Lys
   1 5
<210> 914
   <211> 14
   <212> PRT
   <213> Homo Sapiens
<400> 914
   Gly Asn Ser Ala Gly Gly Leu Gln His Arg Val Thr Ala Lys
   1 5 10
<210> 915
   <211> 6
   <212> PRT
   <213> Homo Sapiens
<400> 915
   Gly Pro Gly Ile Ser Lys
   1 5
<210> 916
   <211> 18
   <212> PRT
   <213> Homo Sapiens
<400> 916
   Gly Thr Leu Ser Pro Lys Asp Ala Leu Thr Asp Leu Thr Gly Asp Ala
   1 5 10 15
   Glu Arg
<210> 917
   <211> 16
   <212> PRT
   <213> Homo Sapiens
<400> 917
   His Ile Glu Glu Glu Asn Leu Ile Asp Glu Asp Phe Gln Asn Leu Lys
   1 5 10 15
<210> 918
   <211> 11
   <212> PRT
   <213> Homo Sapiens
<400> 918
   His Ser Arg Val Thr Val Ala Ile Pro Leu Lys
   1 5 10
<210> 919
   <211> 5
   <212> PRT
   <213> Homo Sapiens
<400> 919
   Ile Asp Glu Lys Arg
   1 5
<210> 920
   <211> 7
   <212> PRT
   <213> Homo Sapiens
<400> 920
   Ile Phe Gln Ala Leu Cys Lys
   1 5
<210> 921
   <211> 4
   <212> PRT
   <213> Homo Sapiens
<400> 921
   Ile Ile Pro Lys
   1
<210> 922
   <211> 5
   <212> PRT
   <213> Homo Sapiens
<400> 922
   Ile Lys Gln Leu Arg
   1 5
<210> 923
   <211> 15
   <212> PRT
   <213> Homo Sapiens
<400> 923
   Ile Leu Tyr Asp Asp Gly Lys Met Val Glu Glu Val Asp Gly Arg
   1 5 10 15
<210> 924
   <211> 15
   <212> PRT
   <213> Homo Sapiens
<400> 924
   Ile Arg Thr Ser Thr Pro Thr Gly His Gly Ala Ser Pro Ala Lys
   1 5 10 15
<210> 925
   <211> 7
   <212> PRT
   <213> Homo Sapiens
<400> 925
   Ile Ser Tyr Arg Ile Leu Arg
   1 5
<210> 926
   <211> 5
   <212> PRT
   <213> Homo Sapiens
<400> 926
   Ile Tyr Ile Val Arg
   1 5
<210> 927
   <211> 11
   <212> PRT
   <213> Homo Sapiens
<400> 927
   Lys Cys Ala Gln Leu Thr Val Asn Leu Thr Arg
   1 5 10
<210> 928
   <211> 8
   <212> PRT
   <213> Homo Sapiens
<400> 928
   Lys Arg Ser Ala Pro Thr Ser Arg
   1               5
<210> 929
   <211> 4
   <212> PRT
   <213> Homo Sapiens
<400> 929
   Leu Ala Leu Lys
   1
<210> 930
   <211> 10
   <212> PRT
   <213> Homo Sapiens
<400> 930
   Leu Ala Ser Ser Lys Ala His Thr Ser Arg
   1               5                   10
<210> 931
   <211> 11
   <212> PRT
   <213> Homo Sapiens
<400> 931
   Leu Glu Asp Pro His Val Asp Ile Ile Arg Arg
   1               5                   10
<210> 932
   <211> 5
   <212> PRT
   <213> Homo Sapiens
<400> 932
   Leu Phe Gly Glu Lys
   1               5
<210> 933
   <211> 9
   <212> PRT
   <213> Homo Sapiens
<400> 933
   Leu Lys Asn Lys Glu Glu Val Leu Lys
   1 5
<210> 934
   <211> 6
   <212> PRT
   <213> Homo Sapiens
<400> 934
   Leu Leu Asp Phe Glu Lys
   1 5
<210> 935
   <211> 17
   <212> PRT
   <213> Homo Sapiens
<400> 935
   Leu Leu His Gly Gln Asn Gly Ser Val Pro Asn Gly Gln Thr Pro Leu
   1 5 10 15
   Lys
<210> 936
   <211> 10
   <212> PRT
   <213> Homo Sapiens
<400> 936
   Leu Leu Ile Glu His Gly Ala Asp Ile Arg
   1 5 10
<210> 937
   <211> 7
   <212> PRT
   <213> Homo Sapiens
<400> 937
   Leu Leu Gln Ala Ile Ala Lys
   1 5
<210> 938
   <211> 5
   <212> PRT
   <213> Homo Sapiens
<400> 938
   Leu Leu Ser Asp Lys
   1 5
<210> 939
   <211> 15
   <212> PRT
   <213> Homo Sapiens
<400> 939
   Leu Leu Ser Asp Pro Asn Tyr Gly Val His Leu Pro Ala Val Lys
   1 5 10 15
<210> 940
   <211> 7
   <212> PRT
   <213> Homo Sapiens
<400> 940
   Leu Leu Val Pro Lys Asp Arg
   1 5
<210> 941
   <211> 4
   <212> PRT
   <213> Homo Sapiens
<400> 941
   Leu Asn Pro Lys
   1
<210> 942
   <211> 13
   <212> PRT
   <213> Homo Sapiens
<400> 942
   Leu Asn Val Glu Glu Arg Ser Val Gly Pro Leu Thr Arg
   1 5 10
<210> 943
   <211> 24
   <212> PRT
   <213> Homo Sapiens
<400> 943
   Leu Gln Cys Glu Asn Val Gln Glu Ile Pro Val Phe Gly Ile Val Pro
   1 5 10 15
   Ala Ile Ile Gln Thr Pro Ser Arg
   20
<210> 944
   <211> 7
   <212> PRT
   <213> Homo Sapiens
<400> 944
   Leu Arg Gln Gly Thr Leu Arg
   1 5
<210> 945
   <211> 7
   <212> PRT
   <213> Homo Sapiens
<400> 945
   Leu Arg Thr Ser Ala Ile Arg
   1 5
<210> 946
   <211> 6
   <212> PRT
   <213> Homo Sapiens
<400> 946
   Leu Ser Ala Arg Asn Lys
   1 5
<210> 947
   <211> 20
   <212> PRT
   <213> Homo Sapiens
<400> 947
   Leu Ser Asp Ala Gly Gln Tyr Leu Cys Gln Ala Gly Asp Asp Ser Asn
   1 5 10 15
   Ser Asn Lys Lys
   20
<210> 948
   <211> 9
   <212> PRT
   <213> Homo Sapiens
<400> 948
   Leu Ser Gly Val Glu Asp His Val Lys
   1 5
<210> 949
   <211> 5
   <212> PRT
   <213> Homo Sapiens
<400> 949
   Leu Thr Val Leu Arg
   1 5
<210> 950
   <211> 9
   <212> PRT
   <213> Homo Sapiens
<400> 950
   Leu Thr Trp Thr Asn Pro Ser Ile Lys
   1 5
<210> 951
   <211> 5
   <212> PRT
   <213> Homo Sapiens
<400> 951
   Leu Val Leu Gly Lys
   1 5
<210> 952
   <211> 14
   <212> PRT
   <213> Homo Sapiens
<400> 952
   Met Arg Tyr Glu Gly Val Val Asp Ile Phe Gln Thr Val Lys
   1 5 10
<210> 953
   <211> 13
   <212> PRT
   <213> Homo Sapiens
<400> 953
   Met Thr Pro Pro Ser Arg Ala Glu Ala Gly Val Arg Arg
   1 5 10
<210> 954
   <211> 16
   <212> PRT
   <213> Homo Sapiens
<400> 954
   Met Val Gly Asp Val Thr Gly Ala Gln Ala Tyr Ala Ser Thr Ala Lys
   1 5 10 15
<210> 955
   <211> 19
   <212> PRT
   <213> Homo Sapiens
<400> 955
   Asn Ala Asp Leu Gln Val Leu Lys Pro Glu Pro Glu Leu Val Tyr Glu
   1 5 10 15
   Asp Leu Arg
<210> 956
   <211> 18
   <212> PRT
   <213> Homo Sapiens
<400> 956
   Asn Asn Leu Val Glu Ile Ile Leu Asp Ile Asn Val Ser Gln Leu Thr
   1 5 10 15
   Glu Arg
<210> 957
   <211> 5
   <212> PRT
   <213> Homo Sapiens
<400> 957
   Asn Pro Ile Ala Lys
   1 5
<210> 958
   <211> 6
   <212> PRT
   <213> Homo Sapiens
<400> 958
   Asn Pro Val Asp Val Lys
   1 5
<210> 959
   <211> 6
   <212> PRT
   <213> Homo Sapiens
<400> 959
   Asn Gln Thr Ala Val Arg
   1 5
<210> 960
   <211> 14
   <212> PRT
   <213> Homo Sapiens
<400> 960
   Asn Arg Ala Gly Leu Gly Glu Glu Phe Glu Lys Glu Ile Arg
   1 5 10
<210> 961
   <211> 8
   <212> PRT
   <213> Homo Sapiens
<400> 961
   Asn Arg Ser Leu Ser Arg Val Arg
   1 5
<210> 962
   <211> 9
   <212> PRT
   <213> Homo Sapiens
<400> 962
   Gln Ile Leu Asp Gln Thr Pro Val Lys
   1 5
<210> 963
   <211> 8
   <212> PRT
   <213> Homo Sapiens
<400> 963
   Gln Leu Val Glu Ala Leu Asp Lys
   1 5
<210> 964
   <211> 18
   <212> PRT
   <213> Homo Sapiens
<400> 964
   Gln Pro His Tyr Ser Ala Phe Gly Ser Val Gly Glu Trp Leu Arg Ala
   1 5 10 15
   Ile Lys
<210> 965
   <211> 11
   <212> PRT
   <213> Homo Sapiens
<400> 965
   Arg Asp Leu Gly Ser Arg Leu Gln Ala Gln Arg
   1 5 10
<210> 966
   <211> 11
   <212> PRT
   <213> Homo Sapiens
<400> 966
   Arg Arg Asp Leu Ser Gln Met Glu Ala Leu Lys
   1 5 10
<210> 967
   <211> 12
   <212> PRT
   <213> Homo Sapiens
<400> 967
   Arg Ser Ser Ala Ala Gly Glu Gly Thr Leu Ala Arg
   1 5 10
<210> 968
   <211> 8
   <212> PRT
   <213> Homo Sapiens
<400> 968
   Ser Ala Ala Ala Ser Asn Tyr Val
   1 5
<210> 969
   <211> 19
   <212> PRT
   <213> Homo Sapiens
<400> 969
   Ser Asp Leu Gln Arg Pro Asn Pro Gln Ser Pro Phe Cys Val Ala Ser
   1 5 10 15
   Ser Leu Lys
<210> 970
   <211> 9
   <212> PRT
   <213> Homo Sapiens
<400> 970
   Ser Leu Gln Glu Ala Asn Ala Glu Lys
   1 5
<210> 971
   <211> 20
   <212> PRT
   <213> Homo Sapiens
<400> 971
   Ser Gln His Gln Glu Thr Pro Val Tyr Leu Gly Ala Thr Ala Gly Met
   1 5 10 15
   Arg Leu Leu Arg
   20
<210> 972
   <211> 9
   <212> PRT
   <213> Homo Sapiens
<400> 972
   Ser Gln Thr Tyr Glu Ser Asp Gly Lys
   1 5
<210> 973
   <211> 8
   <212> PRT
   <213> Homo Sapiens
<400> 973
   Ser Ser Val Lys Glu Leu Leu Lys
   1 5
<210> 974
   <211> 16
   <212> PRT
   <213> Homo Sapiens
<400> 974
   Ser Thr Gly Phe Thr Asn Leu Gly Ala Glu Gly Ser Val Phe Pro Lys
   1 5 10 15
<210> 975
   <211> 16
   <212> PRT
   <213> Homo Sapiens
<400> 975
   Ser Thr Gly Thr Ile Ser Val Ile Ser Ser Gly Leu Asp Arg Glu Lys
   1 5 10 15
<210> 976
   <211> 11
   <212> PRT
   <213> Homo Sapiens
<400> 976
   Thr Ala Ile Glu Ile Leu Ala Trp Gly Leu Arg
   1 5 10
<210> 977
   <211> 9
   <212> PRT
   <213> Homo Sapiens
<400> 977
   Thr Ala Arg Ala Thr Gly Ala Ser Arg
   1 5
<210> 978
   <211> 8
   <212> PRT
   <213> Homo Sapiens
<400> 978
   Thr Ala Thr Gln Pro Leu Leu Lys
   1 5
<210> 979
   <211> 13
   <212> PRT
   <213> Homo Sapiens
<400> 979
   Thr Ala Thr Val Val Met Met Thr Arg Leu Glu Glu Lys
   1 5 10
<210> 980
   <211> 11
   <212> PRT
   <213> Homo Sapiens
<400> 980
   Thr Glu Leu Leu Ala Ala Asp Ser Leu Ser Lys
   1 5 10
<210> 981
   <211> 14
   <212> PRT
   <213> Homo Sapiens
<400> 981
   Thr Gly Arg Pro Val Glu Pro Glu Ser Glu Phe Ile Ile Lys
   1 5 10
<210> 982
   <211> 10
   <212> PRT
   <213> Homo Sapiens
<400> 982
   Thr Leu Ala Glu Val Cys Leu Gly Gln Lys
   1 5 10
<210> 983
   <211> 9
   <212> PRT
   <213> Homo Sapiens
<400> 983
   Thr Leu Arg Leu Gly Pro Leu Ser Lys
   1 5
<210> 984
   <211> 8
   <212> PRT
   <213> Homo Sapiens
<400> 984
   Thr Asn Gly Thr Gly Arg Val Arg
   1 5
<210> 985
   <211> 13
   <212> PRT
   <213> Homo Sapiens
<400> 985
   Thr Ser Lys Thr Thr Phe Gln Leu Glu Leu Pro Val Lys
   1 5 10
<210> 986
   <211> 9
   <212> PRT
   <213> Homo Sapiens
<400> 986
   Thr Ser Asn Gly Arg Leu Pro Val Lys
   1 5
<210> 987
   <211> 9
   <212> PRT
   <213> Homo Sapiens
<400> 987
   Thr Val Thr Ile Asn Cys Pro Phe Lys
   1 5
<210> 988
   <211> 21
   <212> PRT
   <213> Homo Sapiens
<400> 988
   Val Ala Ser Leu Ile Gly Val Glu Gly Gly His Ser Ile Asp Ser Ser
   1 5 10 15
   Leu Gly Val Leu Arg
   20
<210> 989
   <211> 19
   <212> PRT
   <213> Homo Sapiens
<400> 989
   Val Glu Ala Ser Asn Pro Tyr Val Glu Pro Arg Phe Leu Tyr Leu Gly
   1 5 10 15
   Pro Phe Lys
<210> 990
   <211> 5
   <212> PRT
   <213> Homo Sapiens
<400> 990
   Val Phe Pro Gly Lys
   1 5
<210> 991
   <211> 10
   <212> PRT
   <213> Homo Sapiens
<400> 991
   Val Lys Cys Asp Gln Tyr Trp Pro Ala Arg
   1 5 10
<210> 992
   <211> 10
   <212> PRT
   <213> Homo Sapiens
<400> 992
   Val Lys Thr Thr Ser Leu Thr Glu Lys Lys
   1 5 10
<210> 993
   <211> 8
   <212> PRT
   <213> Homo Sapiens
<400> 993
   Val Leu Ala Ala Asn Asn Val Arg
   1 5
<210> 994
   <211> 7
   <212> PRT
   <213> Homo Sapiens
<400> 994
   Val Leu Asp Val Val Glu Arg
   1 5
<210> 995
   <211> 5
   <212> PRT
   <213> Homo Sapiens
<400> 995
   Val Leu Leu Glu Lys
   1 5
<210> 996
   <211> 6
   <212> PRT
   <213> Homo Sapiens
<400> 996
   Val Leu Thr Asp Ile Lys
   1 5
<210> 997
   <211> 11
   <212> PRT
   <213> Homo Sapiens
<400> 997
   Val Pro Ala His Gln Val Leu Phe Ser Arg Arg
   1 5 10
<210> 998
   <211> 9
   <212> PRT
   <213> Homo Sapiens
<400> 998
   Val Gln Ala Val Asn Ser Gln Gly Lys
   1 5
<210> 999
   <211> 11
   <212> PRT
   <213> Homo Sapiens
<400> 999
   Val Thr Ala Ala Gly Gln Thr Lys Arg Thr Arg
   1 5 10
<210> 1000
   <211> 6
   <212> PRT
   <213> Homo Sapiens
<400> 1000
   Val Thr Ala Ile Asn Lys
   1 5
<210> 1001
   <211> 11
   <212> PRT
   <213> Homo Sapiens
<400> 1001
   Val Thr Leu Pro Ala Gly Pro Asp Ile Leu Arg
   1 5 10
<210> 1002
   <211> 6
   <212> PRT
   <213> Homo Sapiens
<400> 1002
   Val Val Ile Ser Ile Arg
   1 5
<210> 1003
   <211> 9
   <212> PRT
   <213> Homo Sapiens
<400> 1003
   Val Val Asn Val Ser Asp Leu Tyr Lys
   1 5
<210> 1004
   <211> 10
   <212> PRT
   <213> Homo Sapiens
<400> 1004
   Tyr Phe Thr Ala Ser Leu Pro Phe Glu Lys
   1 5 10
<210> 1005
   <211> 22
   <212> PRT
   <213> Homo Sapiens
<400> 1005
   Tyr Gly Pro Gly Glu Pro Ser Pro Val Ser Glu Thr Val Val Thr Pro
   1               5                   10                  15
   Glu Ala Ala Pro Glu Lys
               20
<210> 1006
   <211> 7
   <212> PRT
   <213> Homo Sapiens
<400> 1006
   Tyr Leu Asn Asn Leu Tyr Lys
   1               5
<210> 1007
   <211> 14
   <212> PRT
   <213> Homo Sapiens
<400> 1007
   Ile Gly Thr Phe Cys Ser Asn Gly Thr Val Ser Arg Ile Lys
   1               5                   10
<210> 1008
   <211> 16
   <212> PRT
   <213> Homo Sapiens
<400> 1008
   Gln Thr Pro Ala Asp Gly Glu Ala Ser Gly Glu Ser Glu Pro Ala Lys
   1               5                   10                  15

## Description

### INTRODUCTION

The present invention relates to the identification of a membrane protein associated with breast cancer which hasutility as a marker for breast cancer and which also forms a biological target against which antibodies such as therapeutic antibodies (or other affinity reagents) or other pharmaceutical agents can be made.

### BACKGROUND OF THE INVENTION

### Breast Cancer

Globally, breast cancer is both the most common cancer (10% of all cancer cases) and the leading cause of cancer death (6% of cancer deaths) in women. Global incidence of breast cancer is over 1 million cases per year, with about 400,000 deaths. Women in North America have the highest rate of breast cancer in the world (over 200,000 new cases per year, with about 40,000 deaths). The chance of developing invasive breast cancer at some time in a woman's life is about 1 in 8. Breast cancer incidence increases with age, rising sharply after age 40. In the USA, about 77% of invasive breast cancers occur in women over age 50. It has been estimated that approximately US$8.1 billion is spent in the USA each year on treating breast cancer.

### Breast Cancer Diagnosis

Early diagnosis improves the likelihood that treatment will be successful. Screening methods such as mammograms, clinical breast examinations and breast self-examinations are useful in detecting breast cancer. Current diagnostic methods include breast ultrasound, ductogram, full-field digital mammography (FFDM), scintimammography and MRI. A biopsy (fine needle aspiration biopsy, core biopsy or surgical biopsy) is then performed to confirm the presence of breast cancer. Imaging tests such as a chest x-ray, bone scan, CT, MRI and PET are used to detect if the breast cancer has spread.

### Breast Cancer Staging

Breast cancer is staged using the American Joint Committee on Cancer (AJCC) TNM system - Stage 0 - Stage IV. Ductal carcinoma in situ (DCIS), a non-invasive cancer which accounts for 20% of new breast cancer cases is Stage 0. Nearly all women diagnosed at this early stage of breast cancer can be cured. Infiltrating (invasive) ductal carcinoma (IDC), which accounts for 80% of invasive breast cancer and infiltrating (invasive) lobular carcinoma (ILC), which accounts for 5% of invasive breast cancers are more severe Stage I-IV cancers and can metastasize.

### Breast Cancer Treatment

Breast-conserving surgery (lumpectomy) or mastectomy are the usual treatments for breast cancer. For stage I or II breast cancer, breast-conserving surgery is as effective as mastectomy. Patients can then undergo reconstructive surgery. Axillary lymph node sampling and removal or sentinel lymph node biopsy (SLNB) is performed to see if the cancer has spread to the lymph nodes.

Neoadjuvant chemotherapy can be given before surgery to shrink large cancers. Adjuvant chemotherapy after surgery reduces the risk of breast cancer recurrence. Chemotherapy can also be used as the main treatment for women whose cancer has spread outside the breast and underarm area. Chemotherapeutic agents used include anthracyclines (e.g. methotrexate, fluorouracil, doxorubicin, epirubicin), taxanes (e.g. paclitaxel, docetaxel, vinorelbine) and alkylating agents (e.g. cyclophosphamide).

Radiation therapy (usually external beam radiation but sometimes brachytherapy) is given once chemotherapy is complete.

Hormone therapy with selective estrogen receptor modulators (e.g. tamoxifen) can be given to women with estrogen receptor positive breast cancers. Taking tamoxifen after surgery for 5 years can reduce recurrence by about 50% in women with early breast cancer. Aromatase inhibitors such as exemestane, letrozole or anastrozole can also be used.

Women with HER2 positive cancers (about 1/3 of breast cancers) can be given biological response modifiers such as trastuzumab (Herceptin). Clinical trials have shown that adding trastuzumab to chemotherapy lowers the recurrence rate and death rate over chemotherapy alone after surgery in women with HER2 positive early breast cancers.

### Breast Cancer Survival by Stage

Patients diagnosed with breast cancer between 1995 and 1998 had a 5 year relative survival rate of 100% for stage 0 and I, 92% for stage IIA, 81% for stage IIB, 67% for stage IIIA, 54% for stage IIIB and 20% for stage IV.

### Therapeutic Challenges

The major challenges in treatment of breast cancer is to improve early detection rates, to find new non-invasive markers that can be used to follow disease progression and identify relapse, and to find improved and less toxic therapies, especially for more advanced disease where 5 year survival is still poor. There is a great need to identify targets which are more specific to the cancer cells, e.g. ones which are expressed on the surface of the tumour cells so that they can be attacked by promising new approaches like immunotherapeutics and targeted toxins.

### SUMMARY OF THE INVENTION

The present invention provides an isolated monoclonal antibody capable of immuno-specific binding to CUB domain-containing protein 1 (CDCP1), or a fragment or derivative thereof which comprises the antigen binding domain, for use in treating breast cancer wherein the antibody or fragment or derivative thereof is conjugated to a cytotoxic moiety or a drug moiety The present discloure also provides methods and compositions for:
- therapy of, or
- drug development for, or
- screening, diagnosis and prognosis for, or
- monitoring the effectiveness of treatment for

B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma (including patient stratification).

We have used mass spectrometry to identify peptides generated by gel electrophoresis or tagging with iTRAQ reagents and tryptic digest of membrane proteins extracted from lymphoid, breast, cervical, colorectal, gastric, brain, liver, lung, skin, neuronal, osteoblast, ovarian, pancreatic, prostate, kidney and eye cancer tissue samples. Peptide sequences were compared to existing protein and cDNA databases and the corresponding gene sequences identified. The proteins of the invention have not been previously reported to originate from lymphocytic, breast, cervical, colorectal, gastric, brain, liver, lung, skin, neuronal, osteoblast, ovarian, pancreatic, prostate, kidney or eye cancer cell membranes and represent proteins of new therapeutic and/or diagnostic value.

A first example of the disclosure provides methods of treating B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma, comprising administering to a patient a therapeutically effective amount of a compound that modulates (e.g., upregulates or downregulates) or complements the expression or the biological activity (or both) of one or more of the proteins of the invention in patients having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma, in order to ((a) prevent the onset or development of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma; (b) prevent the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma; or (c) ameliorate the symptoms of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

According to a second example of the disclosure we provide a method of detecting, diagnosing and/or screening for or monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence or level of the proteins of the discloure, or one or more fragments thereof, or the presence or level of nucleic acid encoding the proteins of the discloure or the presence or level of the activity of the proteins of the discloure or which comprises detecting a change in the level thereof in said subject.

According to a third example of the disclosure we provide a method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a candidate subject which comprises detecting the presence of the proteins of the disclosure, or one or more fragments thereof, or the presence of nucleic acid encoding the proteins of the discloure or the presence of the activity of the proteins of the discloure in said candidate subject, in which either (a) the presence of an elevated level of the proteins of the discloure or said one or more fragments thereof or an elevated level of nucleic acid encoding the proteins of the discloure or the presence of an elevated level of the activity of the proteins of the discloure in the candidate subject as compared with the level in a healthy subject or (b) the presence of a detectable level of the proteins of the discloure or said one or more fragments thereof or a detectable level of nucleic acid encoding the proteins of the discloure or the presence of a detectable level of the activity of the proteins of the discloure in the candidate subject as compared with a corresponding undetectable level in a healthy subject indicates the presence of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in said subject.

According to a fourth example of the discloure we provide a method of monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a subject or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy which comprises detecting the presence of the proteins of the invention, or one or more fragments thereof, or the presence of nucleic acid encoding the proteins of the discloure or the presence of the activity of the proteins of the invention in said candidate subject at a first time point and at a later time point, the presence of an elevated or lowered level of the proteins of the discloure or said one or more fragments thereof or an elevated or lowered level of nucleic acid encoding the proteins of the discloure or the presence of an elevated or lowered level of the activity of the proteins of the discloure in the subject at the later time point as compared with the level in the subject at said first time point, indicating the progression or regression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or indicating the effect or non-effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in said subject.

The presence of the proteins of the discloure, or one or more fragments thereof, or the presence of nucleic acid encoding the proteins of the discloure or the presence of the activity of the proteins of the discloure may, for example, be detected by analysis of a biological sample obtained from said subject.

The method of discloure may typically include the step of obtaining a biological sample for analysis from said subject. One or more examples the methods of the discloure do not include the step of obtaining a biological sample from a subject.

One example of the discloure provides monoclonal and polyclonal antibodies or other affinity reagents such as an Affibodies capable of immunospecific binding to the proteins of the discloure and compositions comprising same, for example for use in treatment or prophylaxis, such for the treatment or prophylaxis of cancer, particularly a cancer described herein.

The biological sample used can be from any source such as a serum sample or a tissue sample, e.g. lymphoid, breast, cervical, colorectal, gastric, brain, liver, lung, skin, neuronal, osteoblast, ovarian, pancreatic, prostate, kidney or eye tissue. For instance, when looking for evidence of metastatic breast cancer, cervical cancer, colorectal cancer, gastric cancer, hepatocellular carcinoma, lung cancer, melanoma, neuroblastoma, osteosarcoma, ovarian cancer, pancreatic cancer, prostate cancer, renal cell cancer and retinoblastoma, one would look at major sites of breast cancer, cervical cancer, colorectal cancer, gastric cancer, hepatocellular carcinoma, lung cancer, melanoma, neuroblastoma, osteosarcoma, ovarian cancer, pancreatic cancer, prostate cancer, renal cell cancer and retinoblastoma metastasis, e.g. the liver, the lungs and bones for breast cancer; the bladder and the rectum for cervical cancer; the liver, the peritoneal cavity, the pelvis, the retroperitoneum and the lungs for colorectal cancer; the liver, the lungs, the brain and bones for gastric cancer; the lungs and bones for hepatocellular carcinoma; the brain, the liver, the bones and adrenal glands for lung cancer; the lungs, the brain and bones for melanoma; the liver and bones for neuroblastoma; the lungs and other bones for osteosarcoma; the abdomen for ovarian cancer; the liver for pancreatic cancer; the bladder, the rectum and bones for prostate cancer; the lungs, the liver and bones for renal cell cancer and the brain and bones for retinoblastoma.

Alternatively the presence of the proteins of the discloure, or one or more fragments thereof, or the presence of nucleic acid encoding the proteins of the discloure or the presence of the activity of the proteins of the discloure may be detected by analysis in situ.

In certain examples, methods of diagnosis described herein may be at least partly, or wholly, performed *in vitro.*

Suitably the presence of the proteins of the discloure, or one or more fragments thereof, or the presence of nucleic acid encoding the proteins of the discloure or the presence of the activity of the proteins of the discloure is detected quantitatively.

For example, quantitatively detecting may comprise:
(a) contacting a biological sample with an affinity reagent that is specific for the proteins of the discloure, said affinity reagent optionally being conjugated to a detectable label; and
(b) detecting whether binding has occurred between the affinity reagent and at least one species in the sample, said detection being performed either directly or indirectly.

Alternatively the presence of the proteins of the discloure, or one or more fragments thereof, or the presence of nucleic acid encoding the proteins of the discloure or the presence of the activity of the proteins of the discloure may be detected quantitatively by means involving use of an imaging technology.

In another example, the method of the discloure involves use of immunohistochemistry on tissue sections in order to determine the presence of the proteins of the discloure, or one or more fragments thereof, or the presence of nucleic acid encoding the proteins of the discloure or the presence of the activity of the proteins of the discloure, and thereby to localise B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma cells.

In one example the presence of the proteins of the discloure or one or more epitope-containing fragments thereof is detected, for example using an affinity reagent capable of specific binding to the proteins of the discloure or one or more fragments thereof, such as an antibody.

In another example the activity of the proteins of the discloure is detected.

According to another example of the discloure there is provided a method of detecting, diagnosing and/or screening for or monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence or level of antibodies capable of immunospecific binding to the proteins of the discloure, or one or more epitope-containing fragments thereof or which comprises detecting a change in the level thereof in said subject.

According to another example of the discloure there is also provided a method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a subject which comprises detecting the presence of antibodies capable of immunospecific binding to the proteins of the discloure, or one or more epitope-containing fragments thereof in said subject, in which (a) the presence of an elevated level of antibodies capable of immunospecific binding to the proteins of the discloure or said one or more epitope-containing fragments thereof in said subject as compared with the level in a healthy subject or (b) the presence of a detectable level of antibodies capable of immunospecific binding to the proteins of the discloure or said one or more epitope-containing fragments thereof in said subject as compared with a corresponding undetectable level in a healthy subject indicates the presence of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in said subject.

One particular method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma comprises:
(a) bringing into contact with a biological sample to be tested the proteins of the discloure, or one or more epitope-containing fragments thereof; and
(b) detecting the presence of antibodies in the subject capable of immunospecific binding to the proteins of the discloure, or one or more epitope-containing fragments thereof

According to another example of the discloure there is provided a method of monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence of antibodies capable of immunospecific binding to the proteins of the discloure, or one or more epitope-containing fragments thereof in said subject at a first time point and at a later time point, the presence of an elevated or lowered level of antibodies capable of immunospecific binding to the proteins of the discloure, or one or more epitope-containing fragments thereof in said subject at the later time point as compared with the level in said subject at said first time point, indicating the progression or regression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or the effect or non-effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in said subject.

The presence of antibodies capable of immunospecific binding to the proteins of the discloure, or one or more epitope-containing fragments thereof is typically detected by analysis of a biological sample obtained from said subject (exemplary biological samples are mentioned above, e.g. the sample is a sample of lymphoid, breast, cervical, colorectal, gastric, brain, liver, lung, skin, neuronal, osteoblast, ovarian, pancreatic, prostate, kidney or eye tissue, or else a sample of blood or saliva).

The method typically includes the step of obtaining said biological sample for analysis from said subject.

The antibodies that may be detected include IgA, IgM and IgG antibodies.

In any of the above methods, the level that may be detected in the candidate subject who has B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma is 2 or more fold higher than the level in the healthy subject.

Also described in the discloure is an agent capable of specific binding to the proteins of the discloure, or a fragment thereof, or a hybridising agent capable of hybridizing to nucleic acid encoding the proteins of the discloure or an agent capable of detecting the activity of the proteins of the discloure for use in screening for, detecting and/or diagnosing disease, such as cancer, and especially B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

Also described are the proteins of the discloure, or fragments thereof for use in screening for, detecting and/or diagnosing disease such as cancer, and especially B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

Also described are affinity reagents capable of specific binding to the proteins of the discloure or fragments thereof, for example an affinity reagent which contains or is conjugated to a detectable label or contains or is conjugated to a therapeutic moiety such as a cytotoxic moiety. The affinity reagent may, for example, be an antibody.

Also described are hybridizing agents capable of hybridizing to nucleic acid encoding the proteins of the discloure, for example, a hybridizing agent which contains or is conjugated to a detectable label. One example of a hybridizing agent is an inhibitory RNA (RNAi). Other examples include anti-sense oligonucleotides and ribozymes.

The discloure also provides kits containing the proteins of the discloure and/or one or more fragments thereof or containing one or more aforementioned affinity reagents and/or hybridizing agents or containing one or more agents capable of detecting the activity of the proteins of the discloure together with instructions for their use in an aforementioned method. The kit may further contain reagents capable of detecting and reporting the binding of said affinity reagents and/or hybridizing agents to their binding partners.

Also described are pharmaceutical compositions comprising a therapeutically effective amount of affinity reagents capable of specific binding to the proteins of the discloure or a fragment thereof.

Another example of the discloure is a pharmaceutically acceptable diluent or carrier and a pharmaceutical composition comprising one or more affinity reagents or hybridizing reagents as aforesaid and a pharmaceutically acceptable diluent or carrier.

In one example the cancer to be detected, prevented or treated is B-cell non-Hodgkin's lymphoma.

In one embodiment the cancer to be detected, prevented or treated is breast cancer.

In one example the cancer to be detected, prevented or treated is cervical cancer.

In one example the cancer to be detected, prevented or treated is colorectal cancer.

In one example the cancer to be detected, prevented or treated is gastric cancer.

In one example the cancer to be detected, prevented or treated is glioblastoma.

In one example the cancer to be detected, prevented or treated is hepatocellular carcinoma.

In one example the cancer to be detected, prevented or treated is lung cancer.

In one example the cancer to be detected, prevented or treated is lymphoid leukaemia (particularly acute T-cell leukaemia and chronic lymphocytic leukaemia).

In one example the cancer to be detected, prevented or treated is melanoma.

In one example the cancer to be detected, prevented or treated is neuroblastoma.

In one example the cancer to be detected, prevented or treated is osteosarcoma.

In another example the cancer to be detected, prevented or treated is ovarian cancer.

In another example the cancer to be detected, prevented or treated is pancreatic cancer. In another example the cancer to be detected, prevented or treated is prostate cancer.

In another example the cancer to be detected, prevented or treated is renal cell cancer. In another example the cancer to be detected, prevented or treated is retinoblastoma. Other aspects of the present invention are set out below and in the claims herein.

### BRIEF DESCRIPTION OF THE FIGURES

Figure 1 shows the amino acid sequences of the proteins of the disclosure. The tryptics detected experimentally by mass spectrometry are highlighted - mass match peptides are shown in bold, tandem peptides are underlined.
Figures 2 and 4 show so-called Box Plot data for OGTA066, as described in Examples 3 and 4.
Figure 3 shows ROC curve data for OGTA066, as described in Example 4.

### DETAILED DESCRIPTION OF THE INVENTION

The invention encompasses the administration of therapeutic compositions to a mammalian subject to treat or prevent breast cancer. Also described is the administration of therapeutic compositions to a mammalian subject to treat or prevent B-cell non-Hodgkin's lymphoma, , cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma.

The disclosure also encompasses clinical screening, diagnosis and prognosis in a mammalian subject for identifying patients most likely to respond to a particular therapeutic treatment, for monitoring the results of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma therapy, for drug screening and drug development.
The mammalian subject may be a non-human mammal, for example a human, such as a human adult, i.e. a human subject at least 21 (more preferably at least 35, at least 50, at least 60, at least 70, or at least 80) years old. For clarity of disclosure, and not by way of limitation, the disclosure will be described with respect to the analysis of lymphoid, breast, cervical, colorectal, gastric, brain, liver, lung, skin, neuronal, osteoblast, ovarian, pancreatic, prostate, kidney and eye tissue. However, as one skilled in the art will appreciate, the assays and techniques described below can be applied to other types of patient samples, including body fluids (*e.g.* blood, urine or saliva), a tissue sample from a patient at risk of having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma (*e.g.* a biopsy such as a bone marrow, breast, cervical, liver, stomach, brain, lung, skin, bone, ovarian, pancreatic, prostate or kidney biopsy) or homogenate thereof.

The methods and compositions of the present disclosure are specially suited for screening, diagnosis and prognosis of a living subject, but may also be used for postmortem diagnosis in a subject, for example, to identify family members at risk of developing the same disease.

A relevant cancer as used herein refers to B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and/or retinoblastoma.

### Proteins of the invention

In one example, one-dimensional electrophoresis or isobaric tags for relative and absolute quantification (iTRAQ) or other appropriate methods are used to analyse relevant cancer tissue samples from a subject, such as a living subject, in order to measure the expression of the proteins of the disclosure for screening or diagnosis of a relevant cancer, to determine the prognosis of a patient with same, to monitor the effectiveness of therapy for same, or for drug development in relation to same.

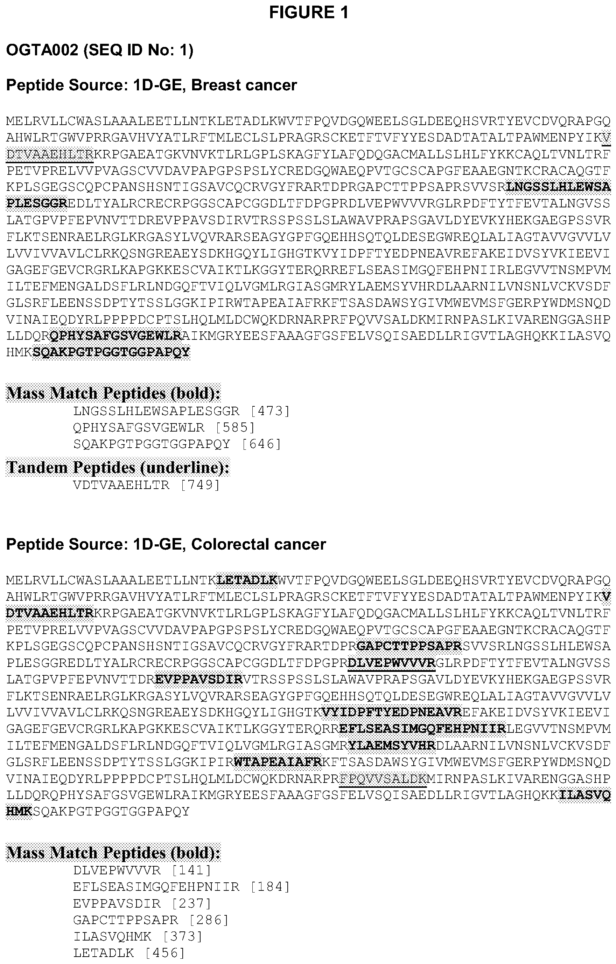

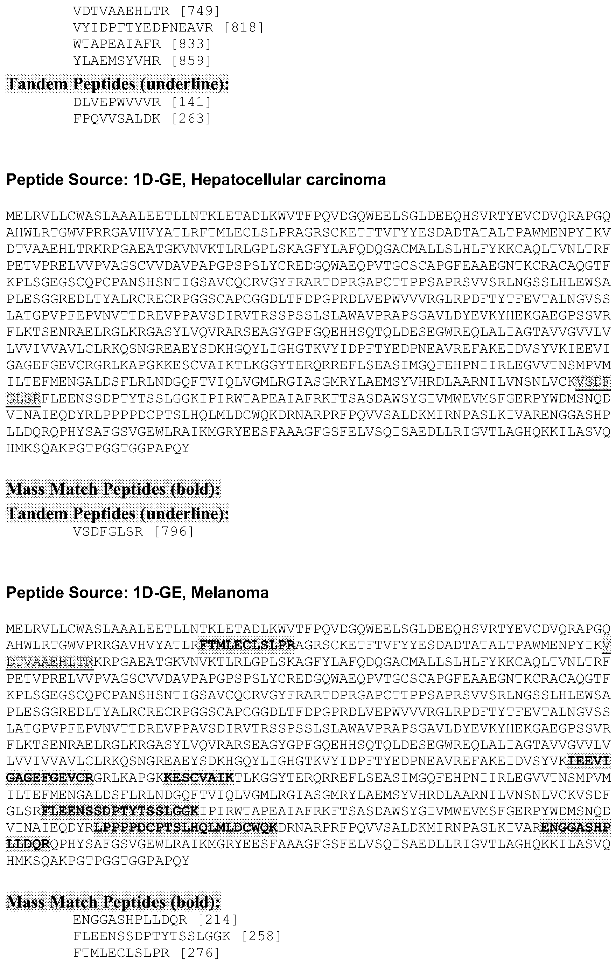

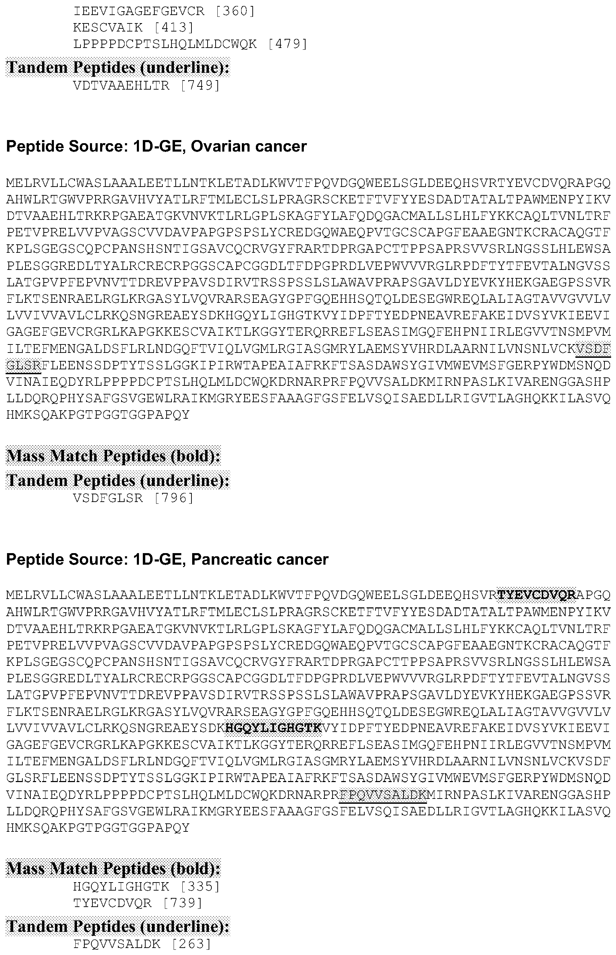

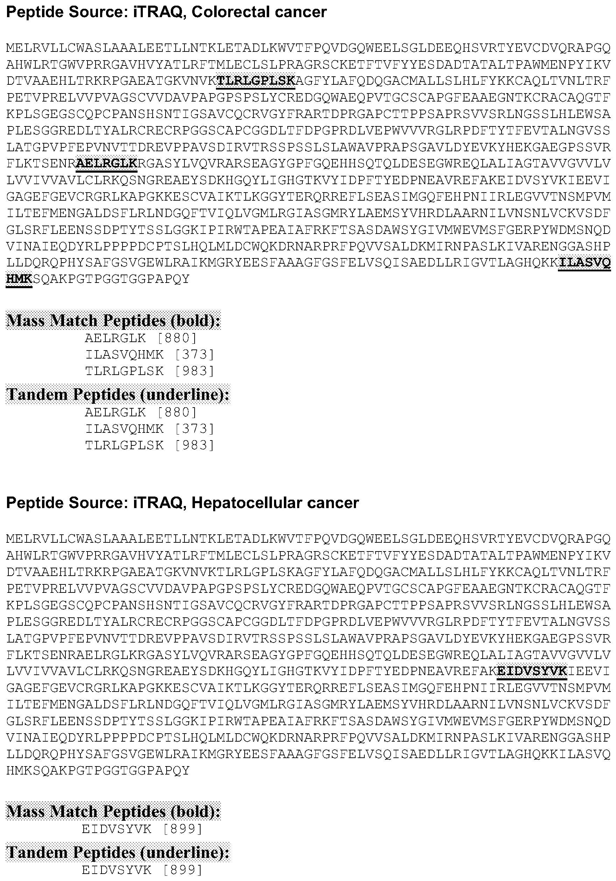

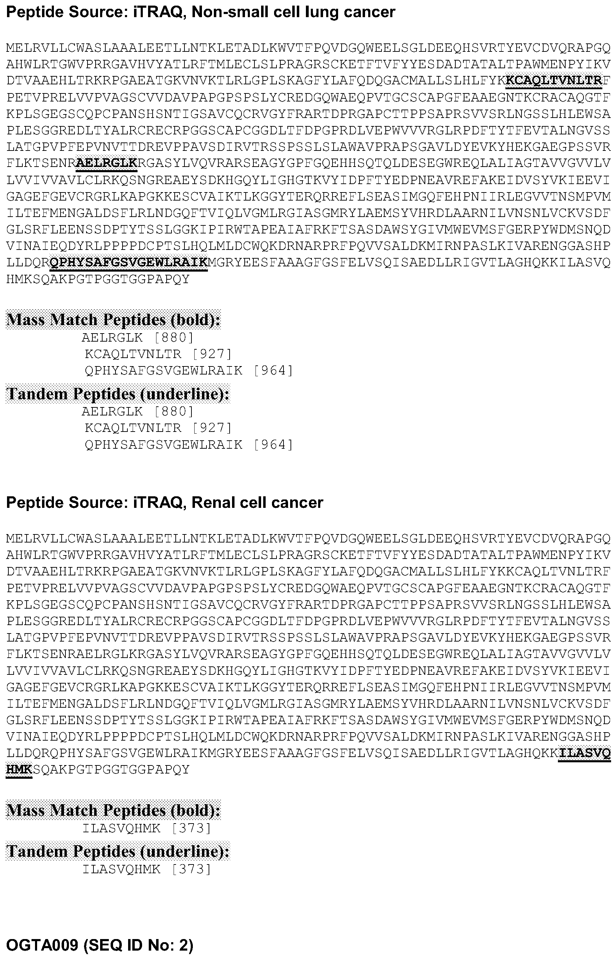

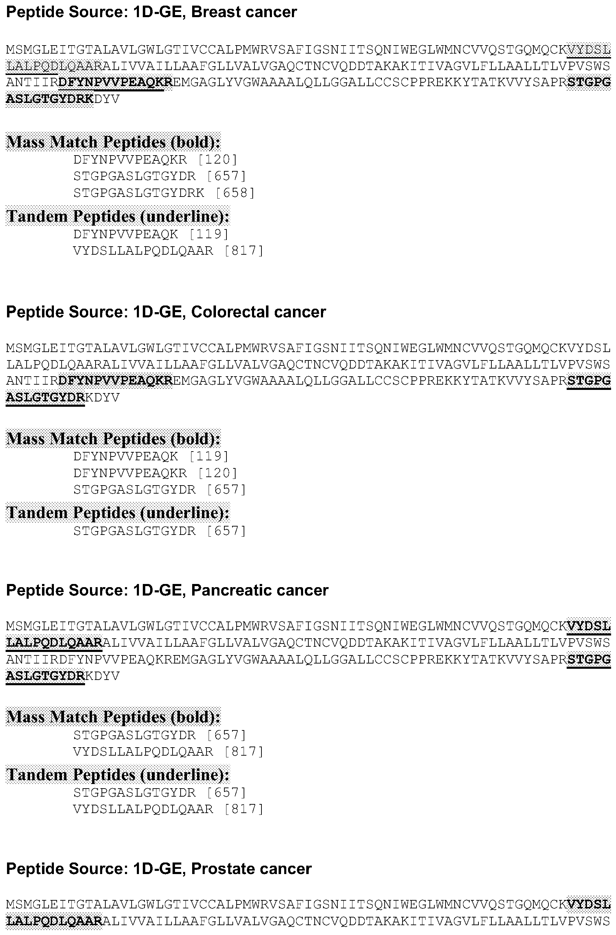

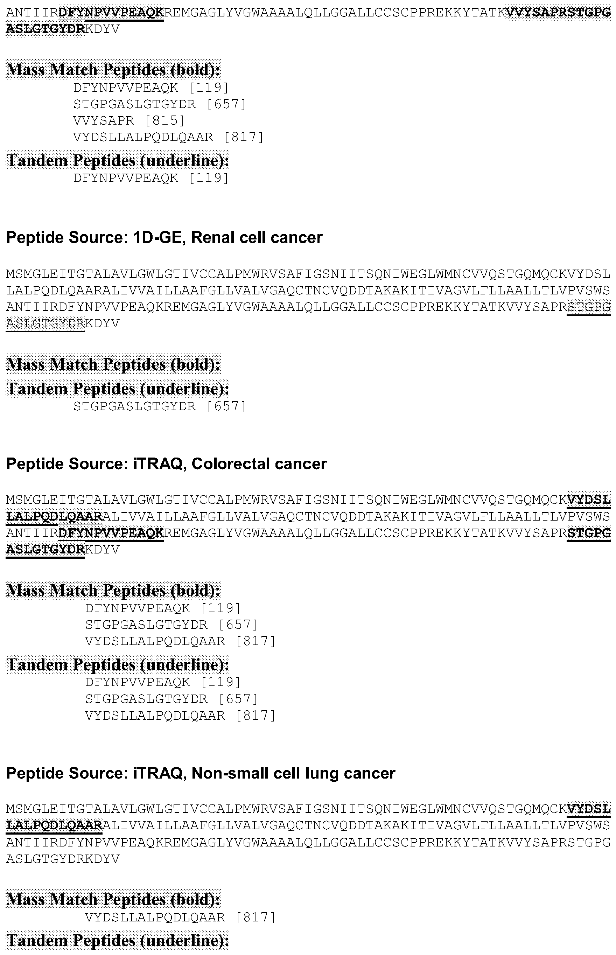

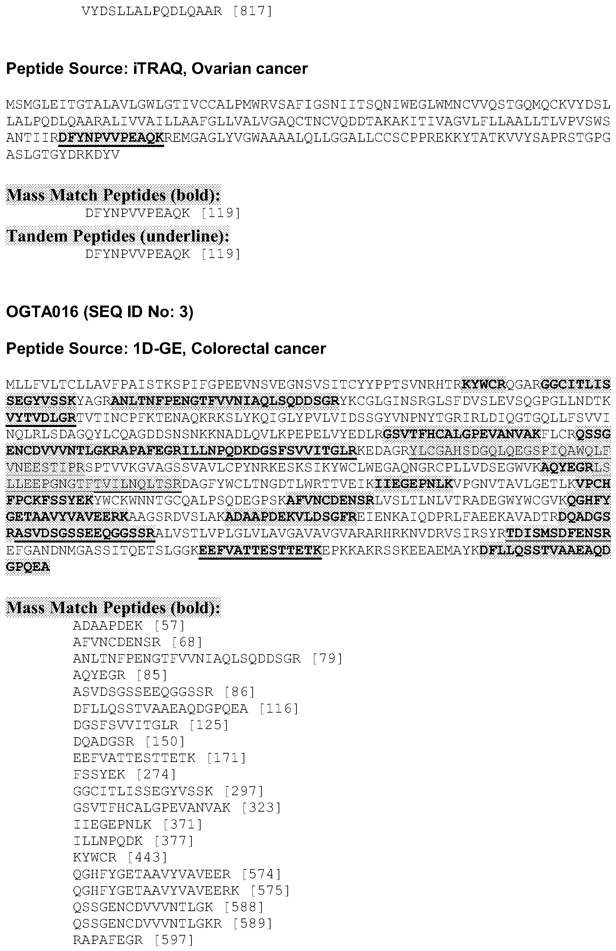

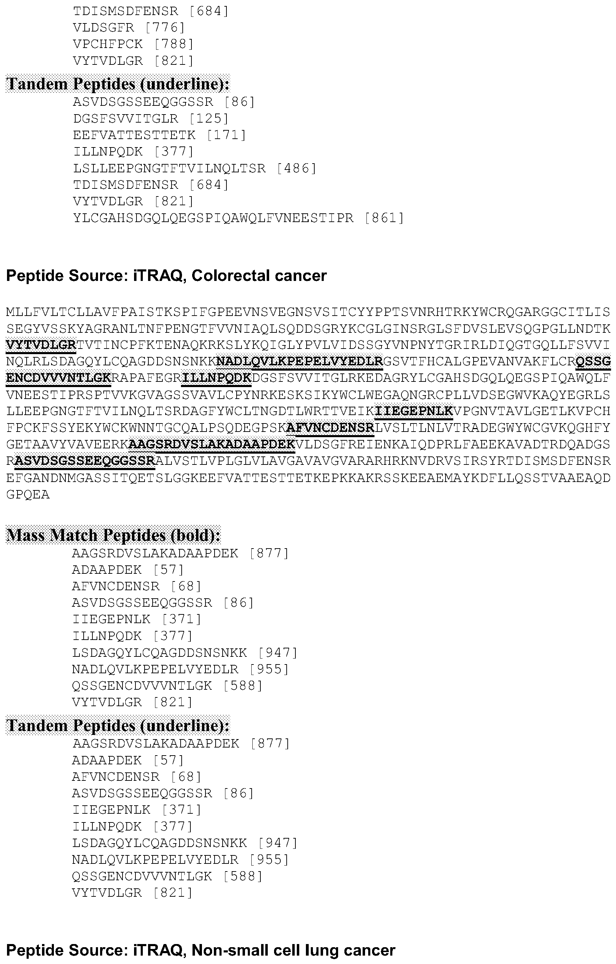

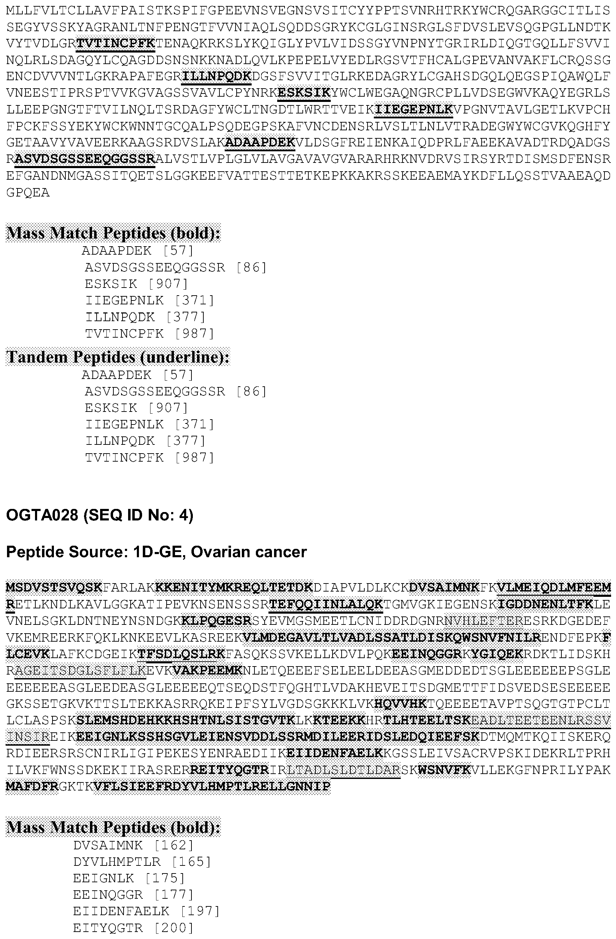

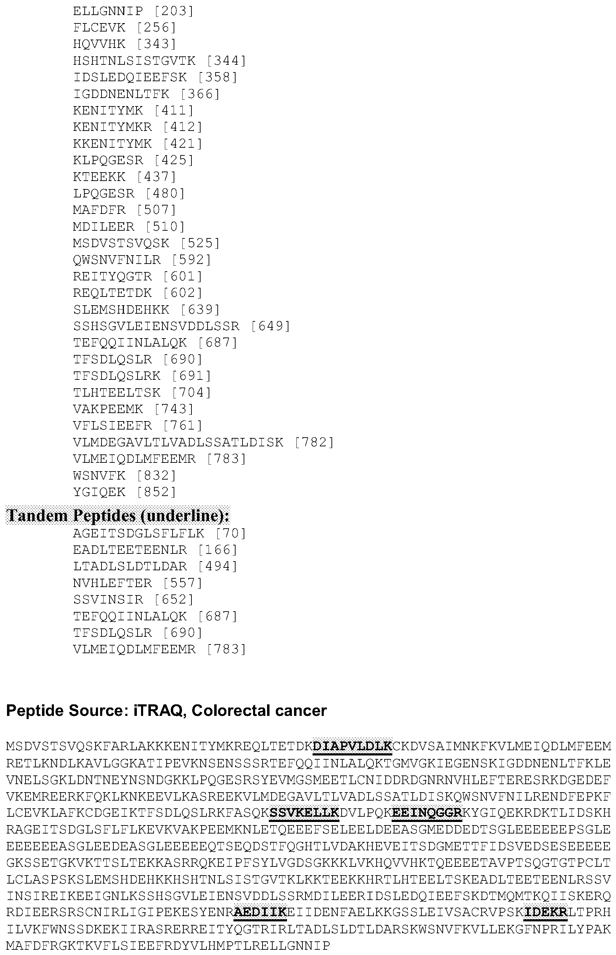

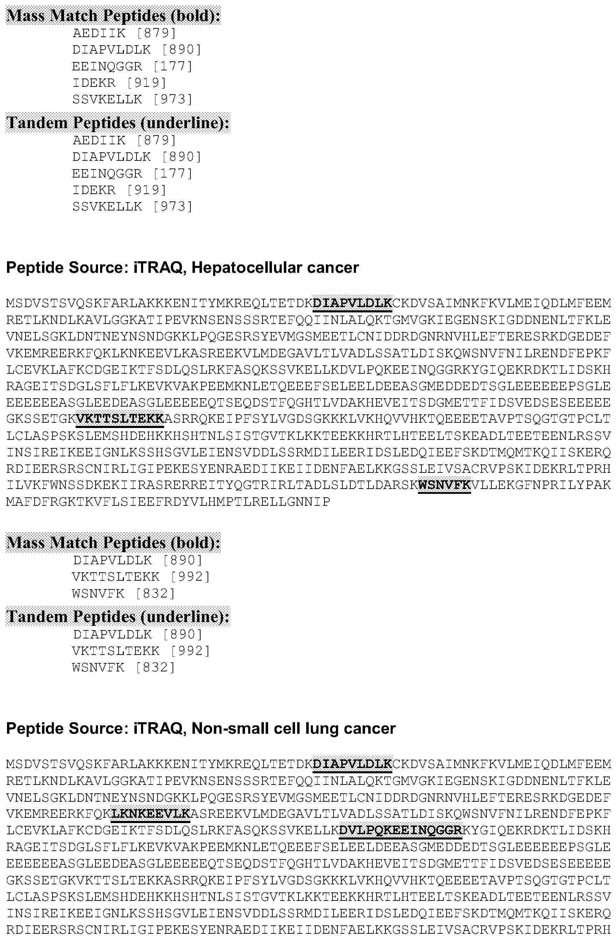

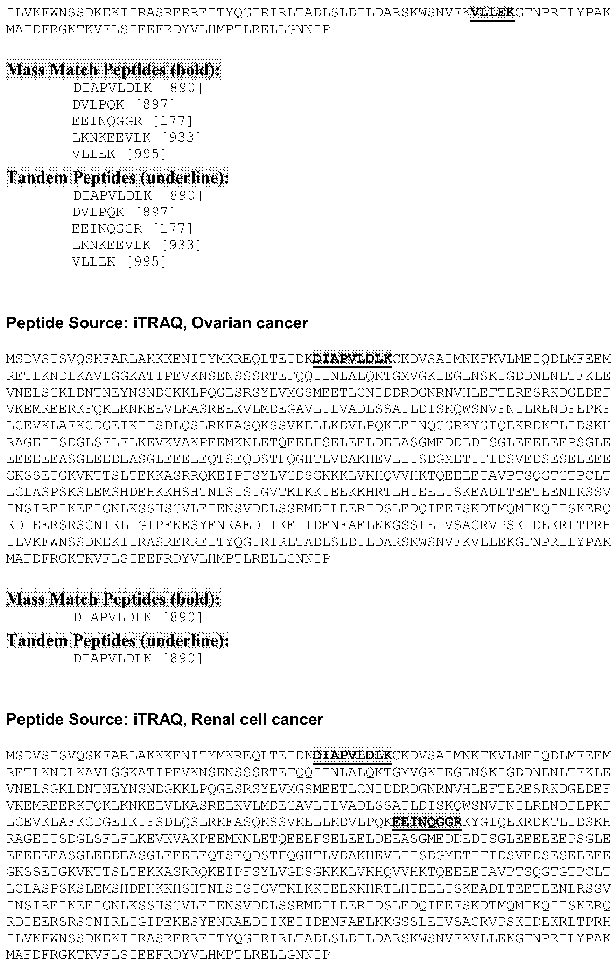

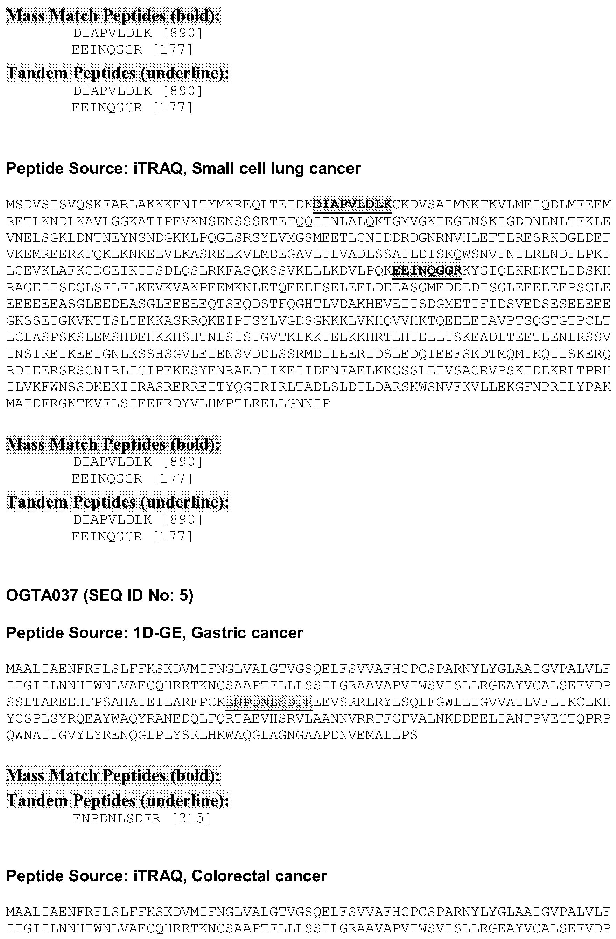

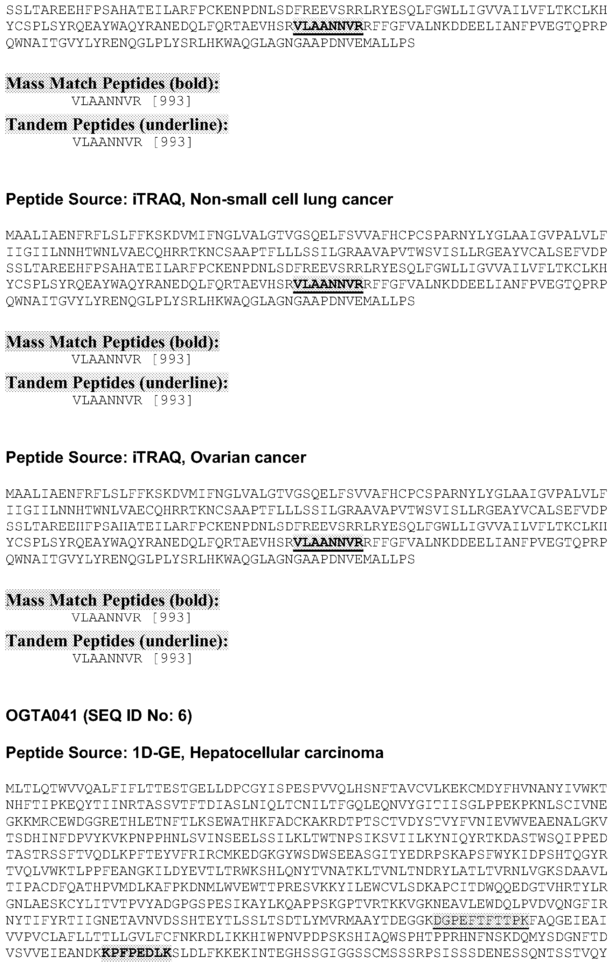

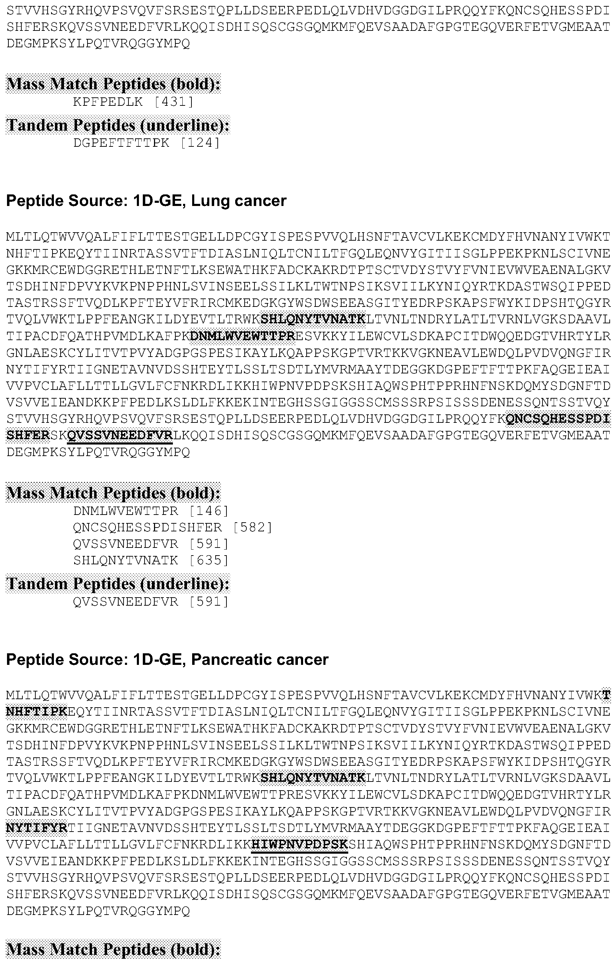

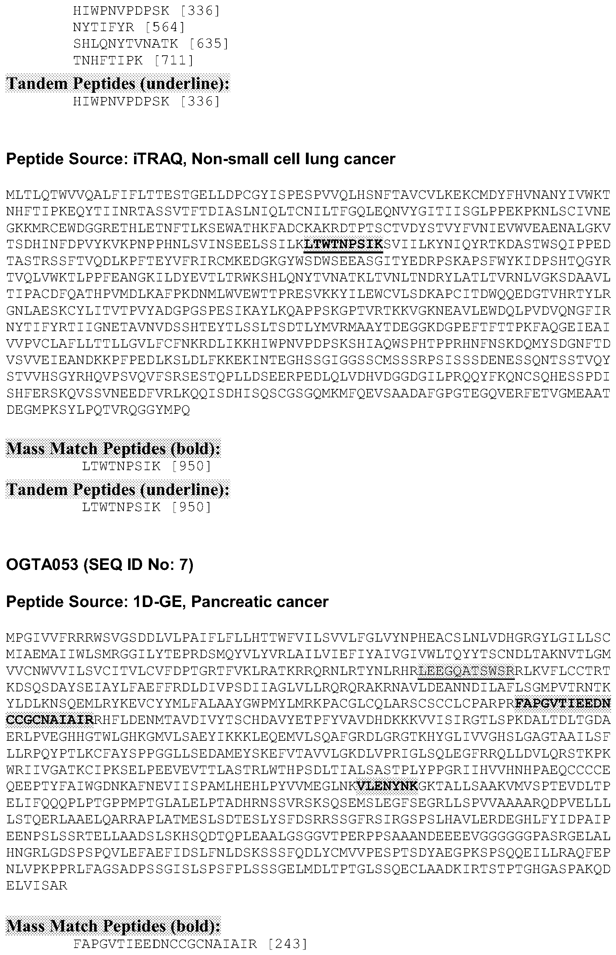

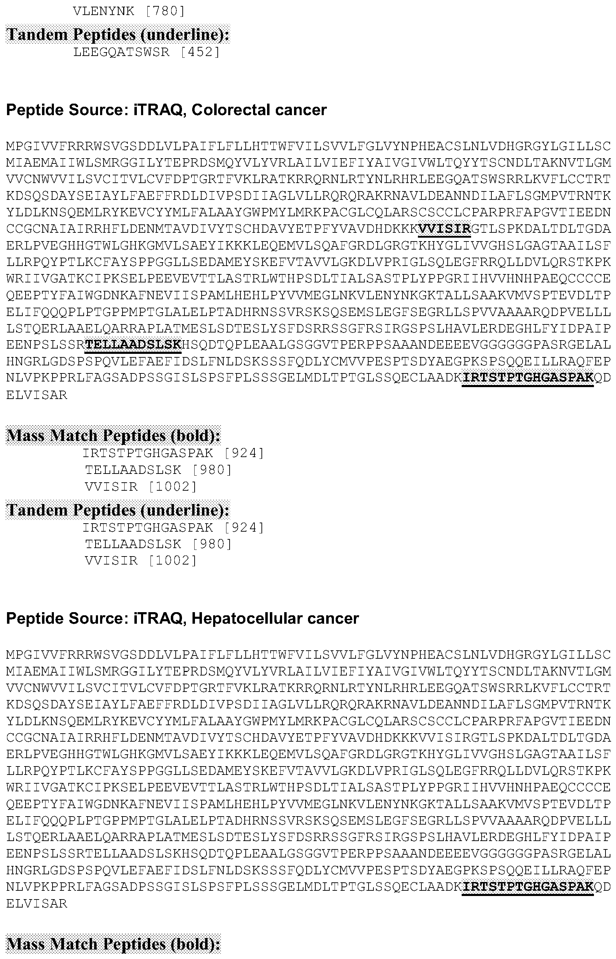

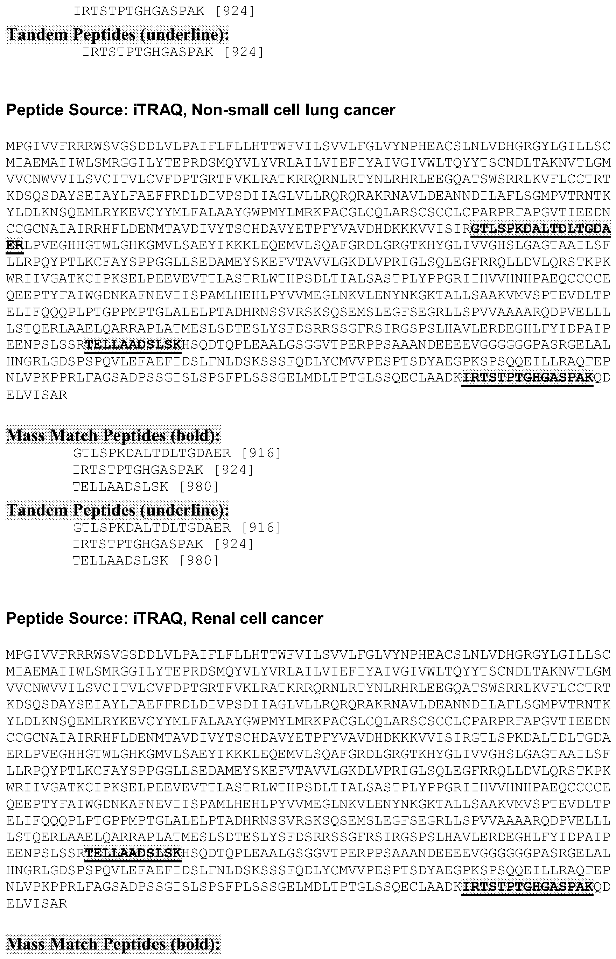

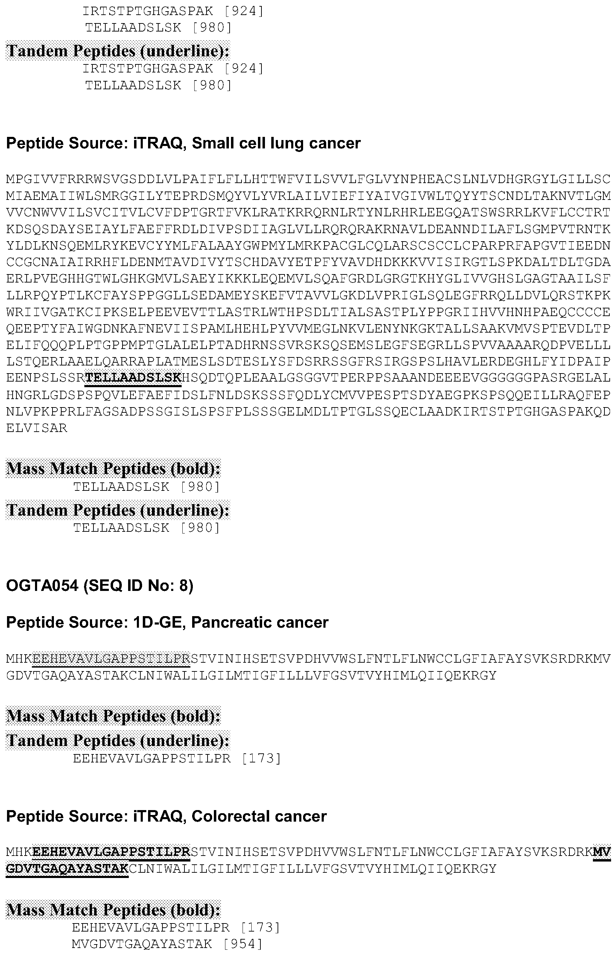

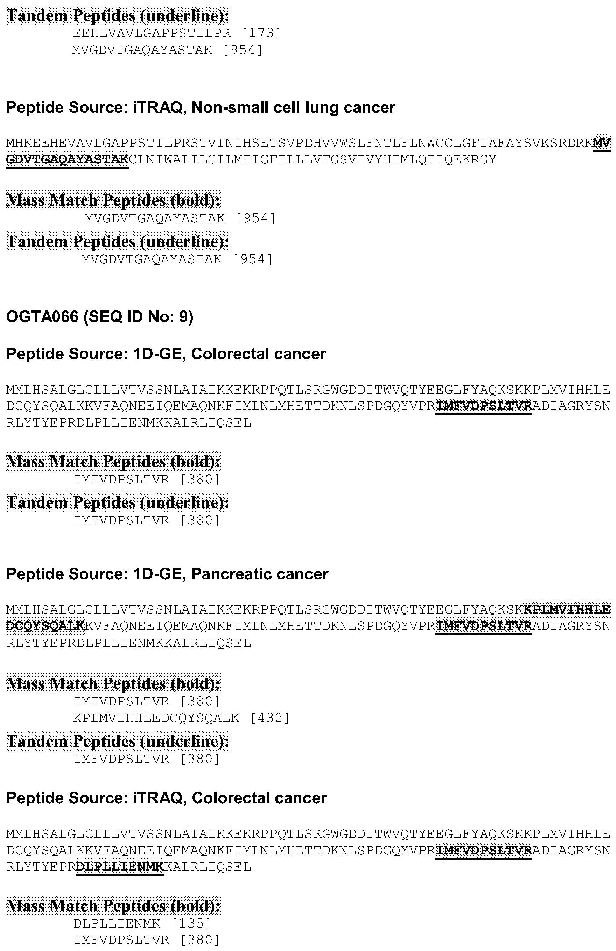

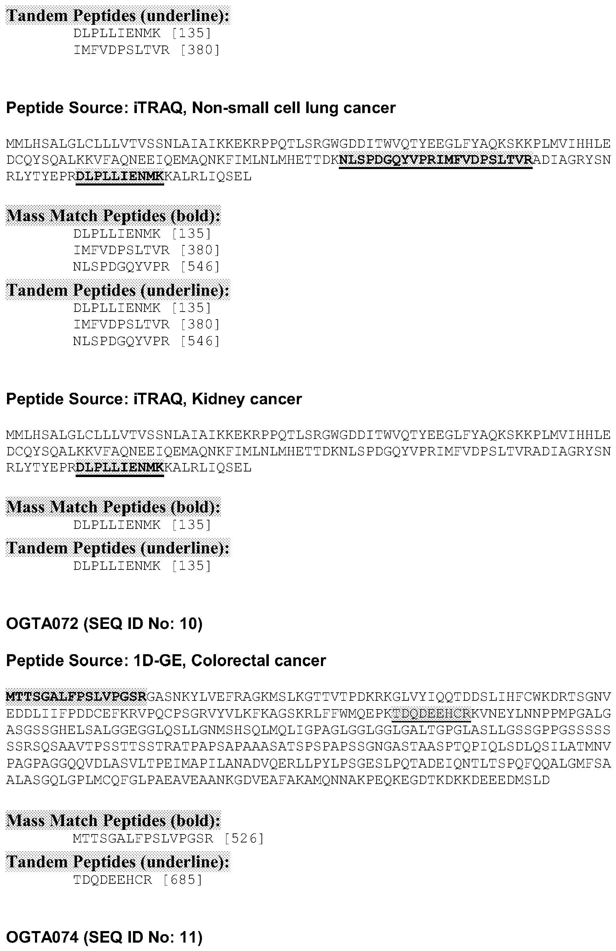

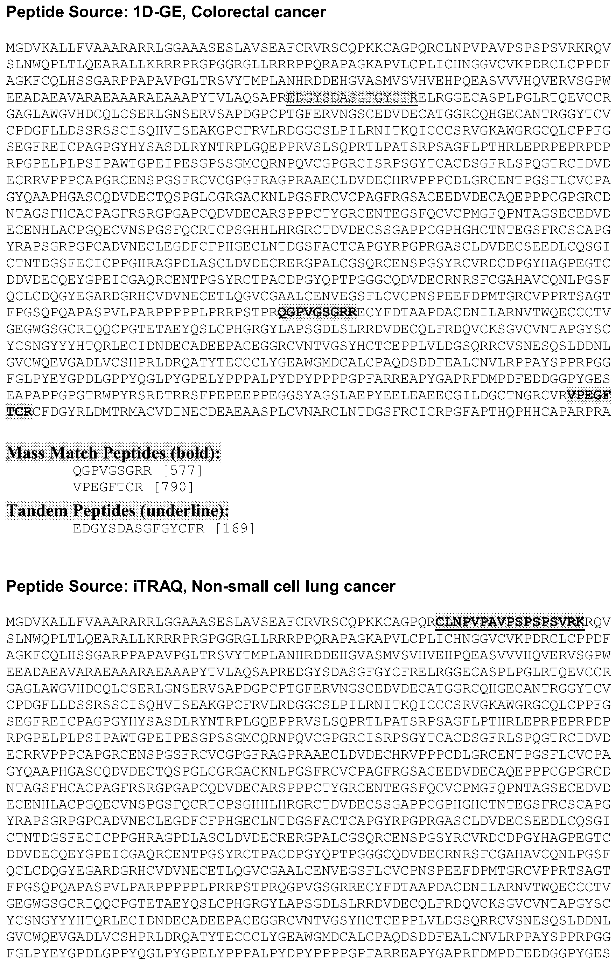

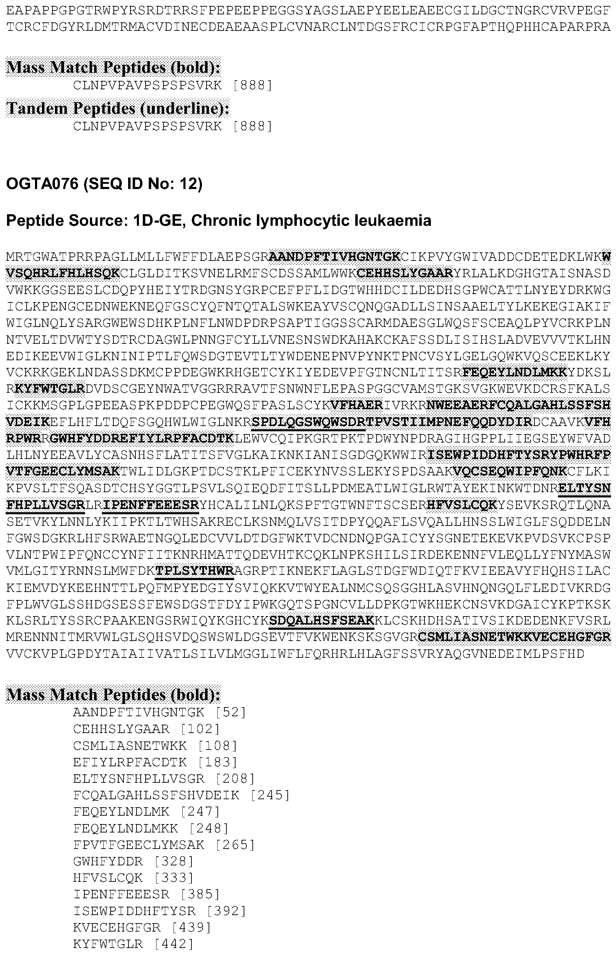

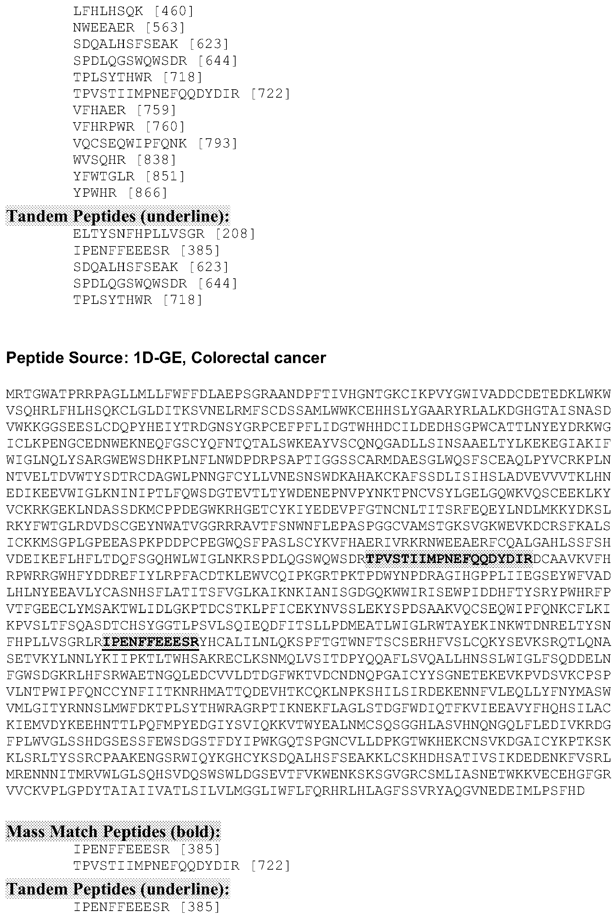

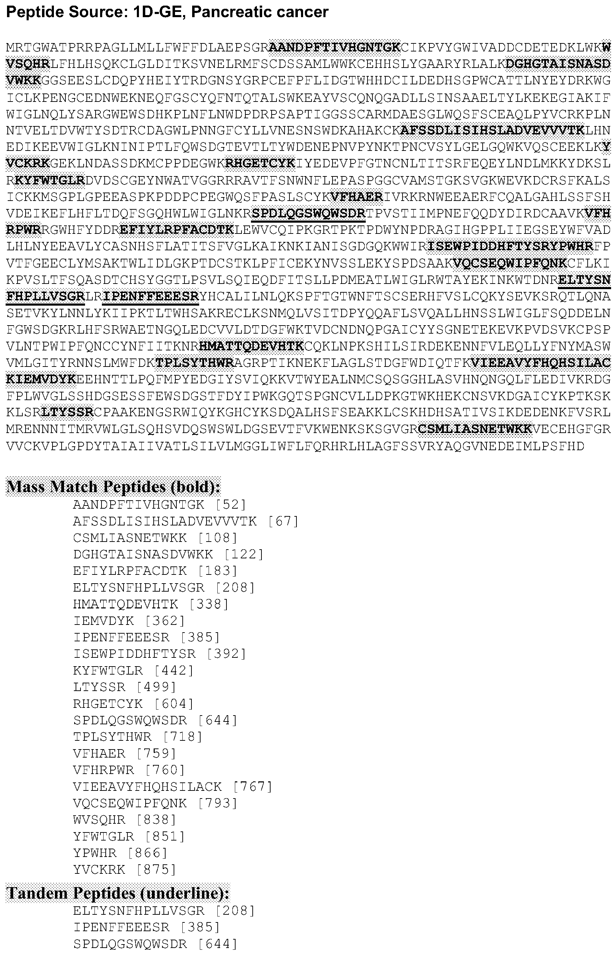

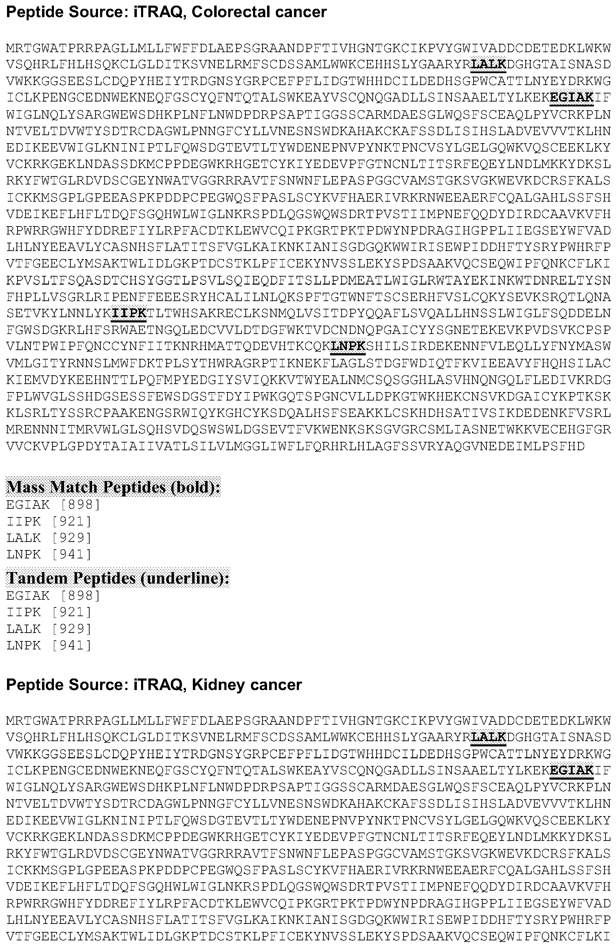

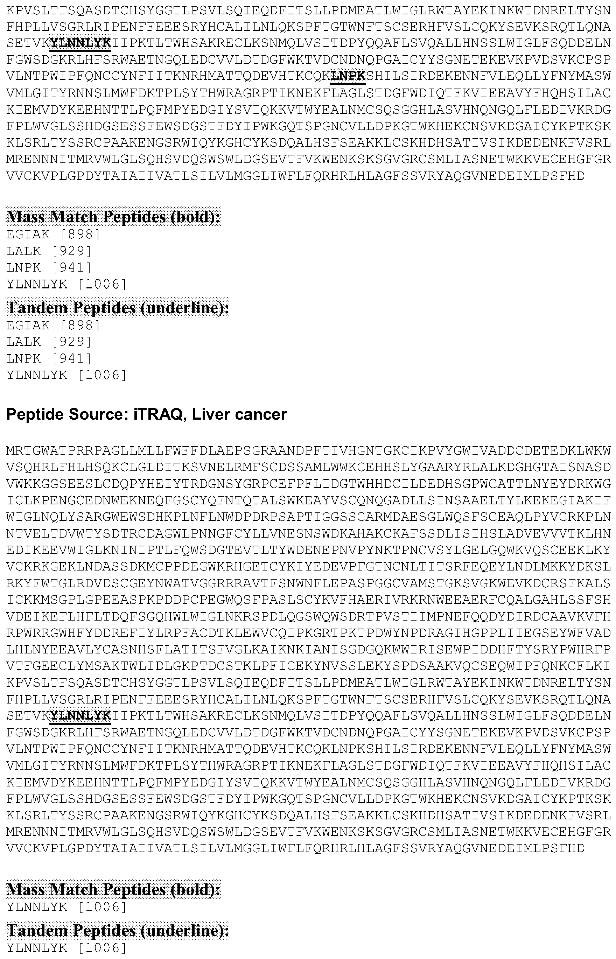

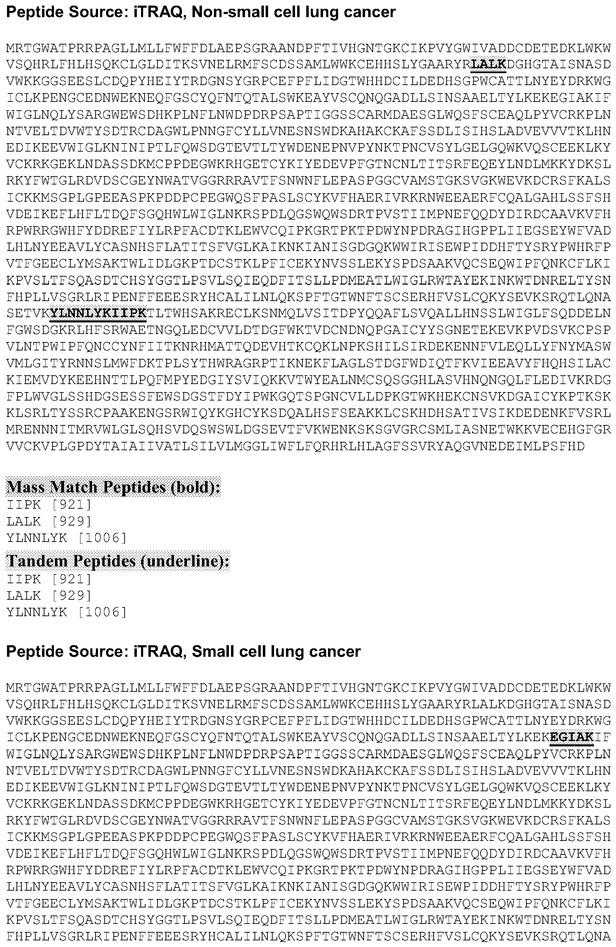

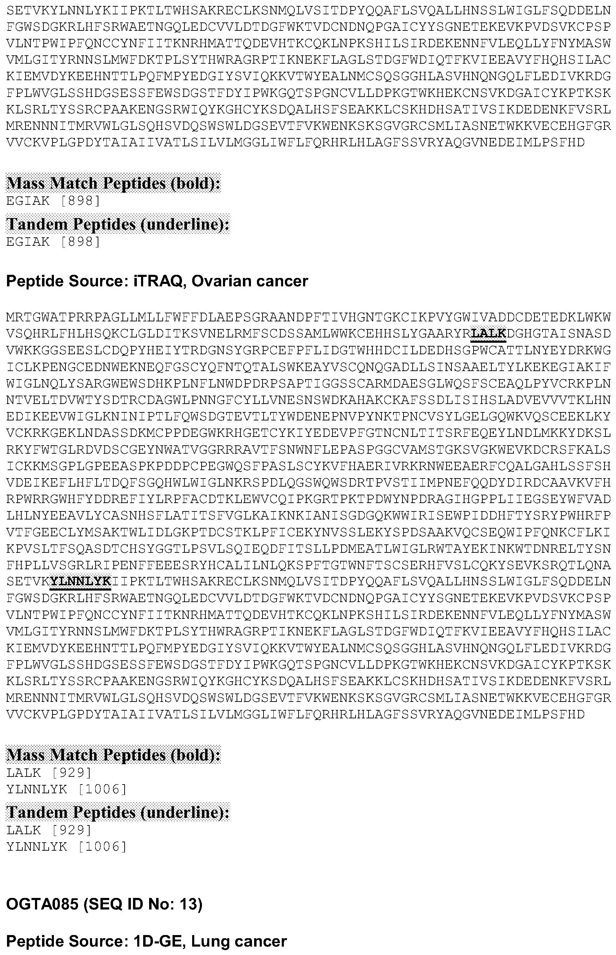

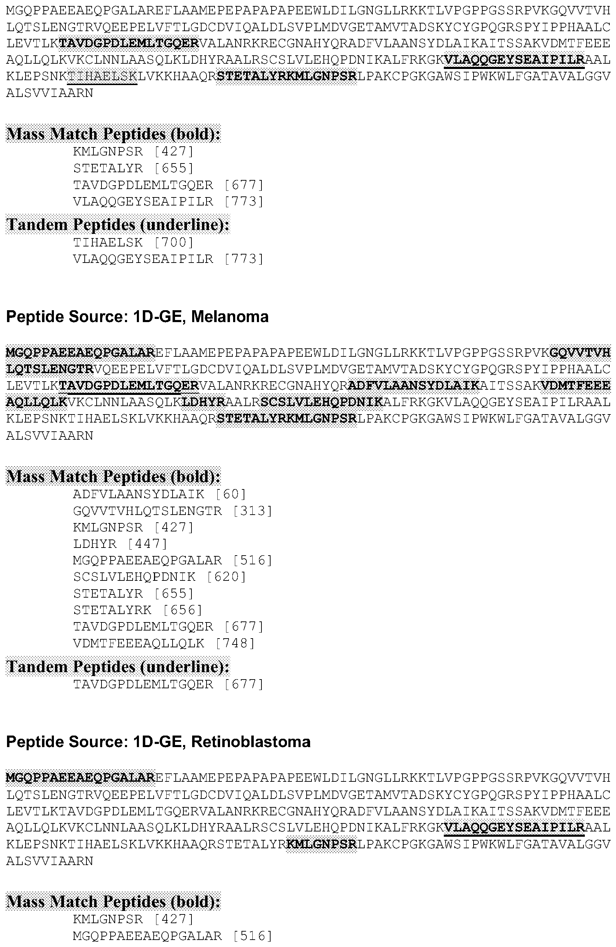

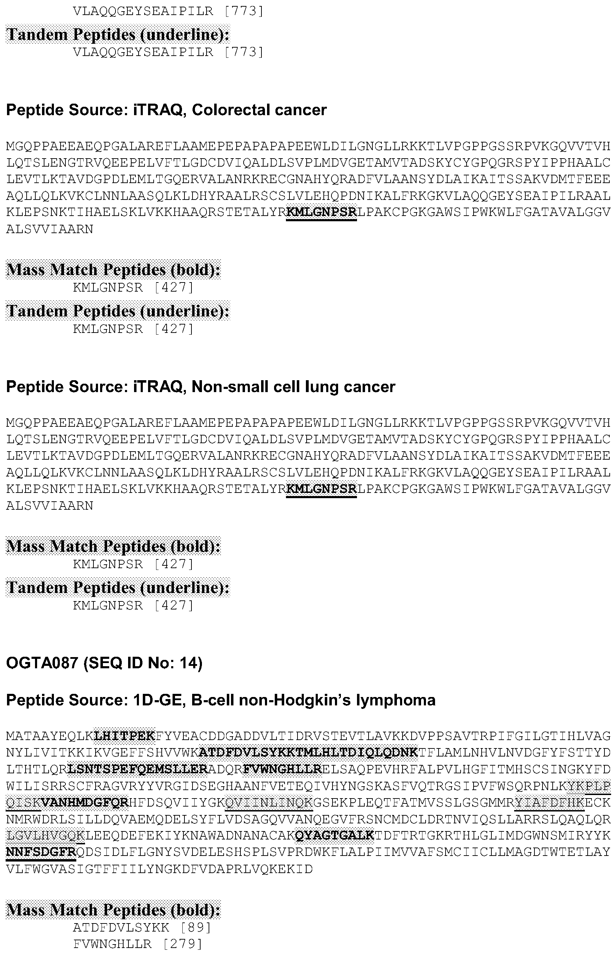

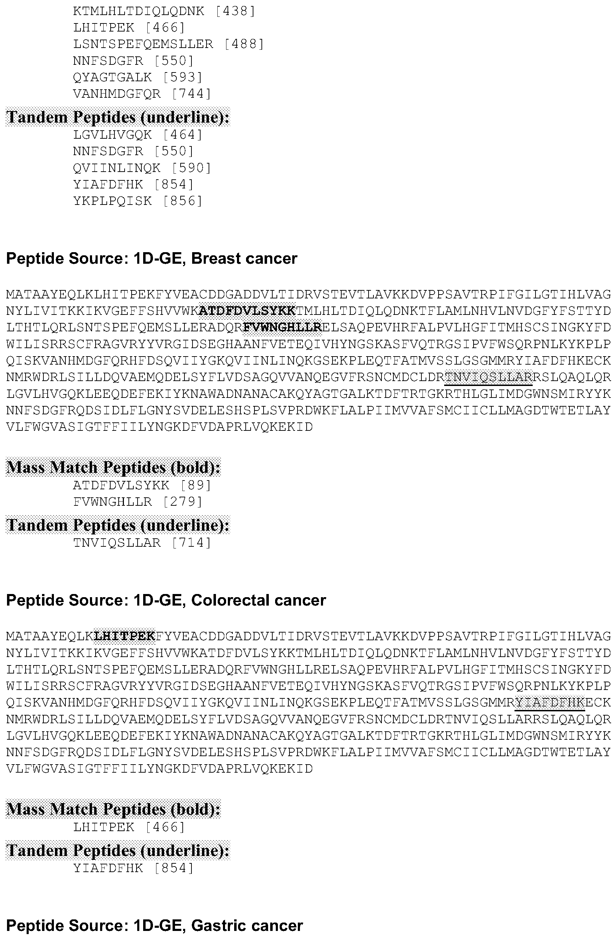

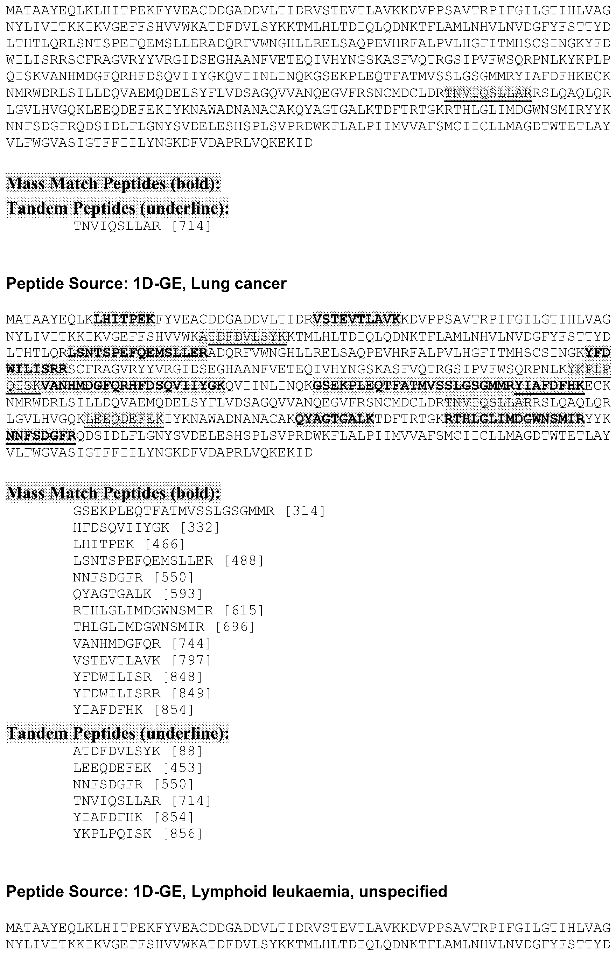

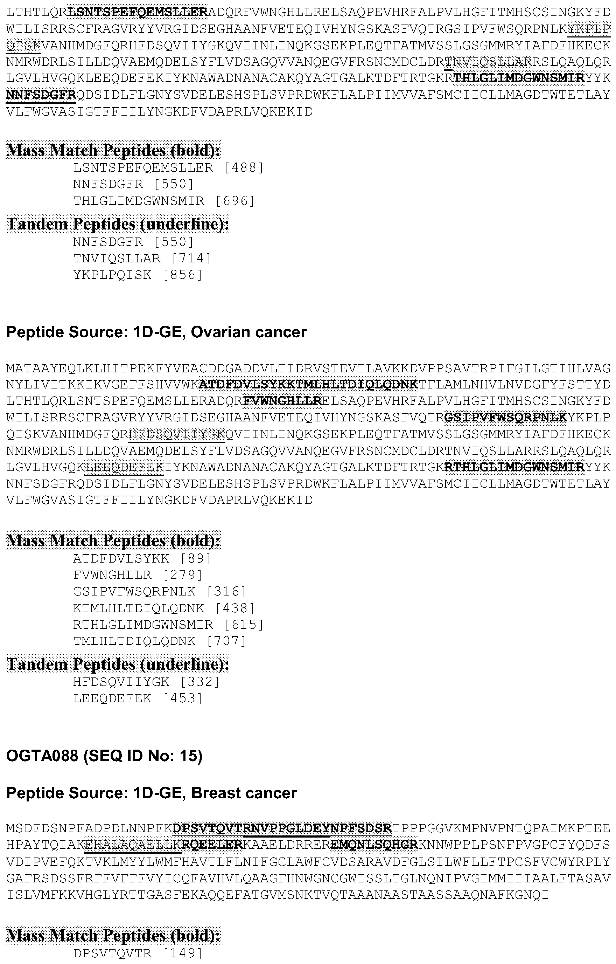

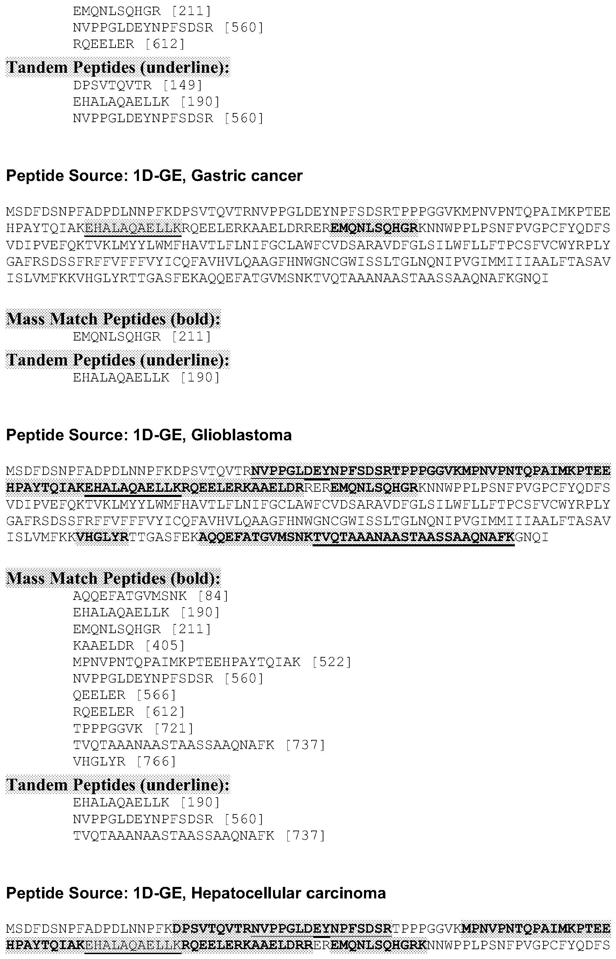

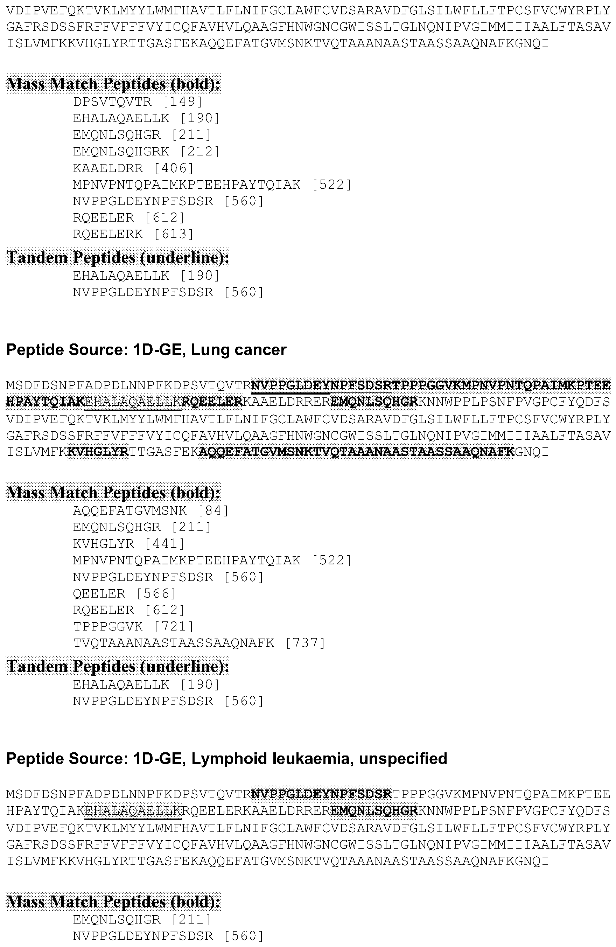

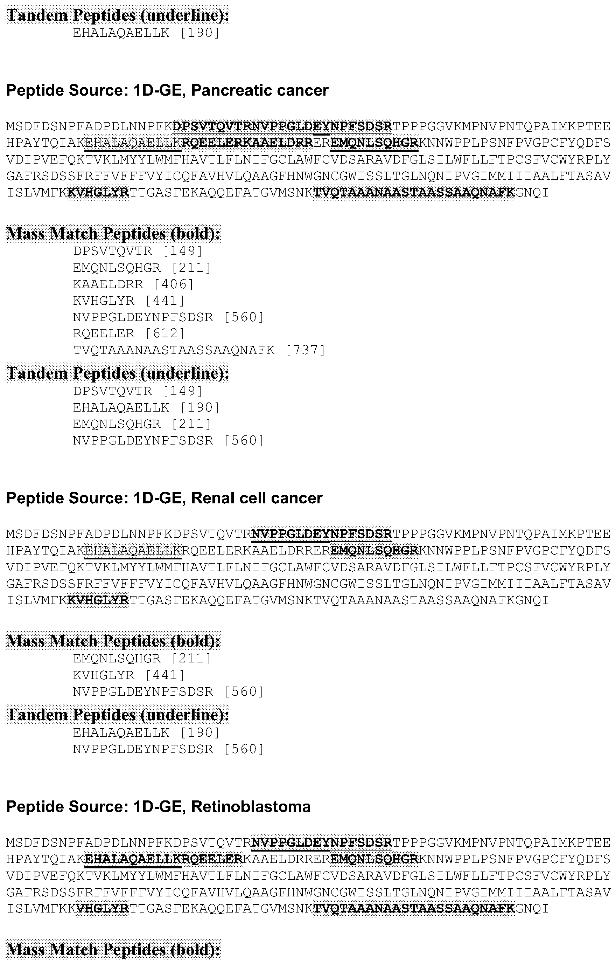

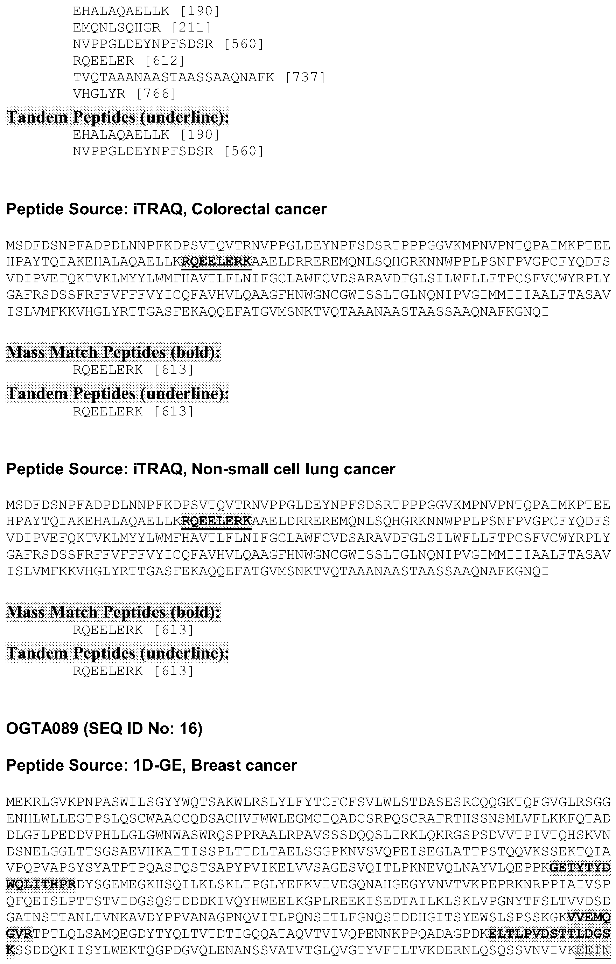

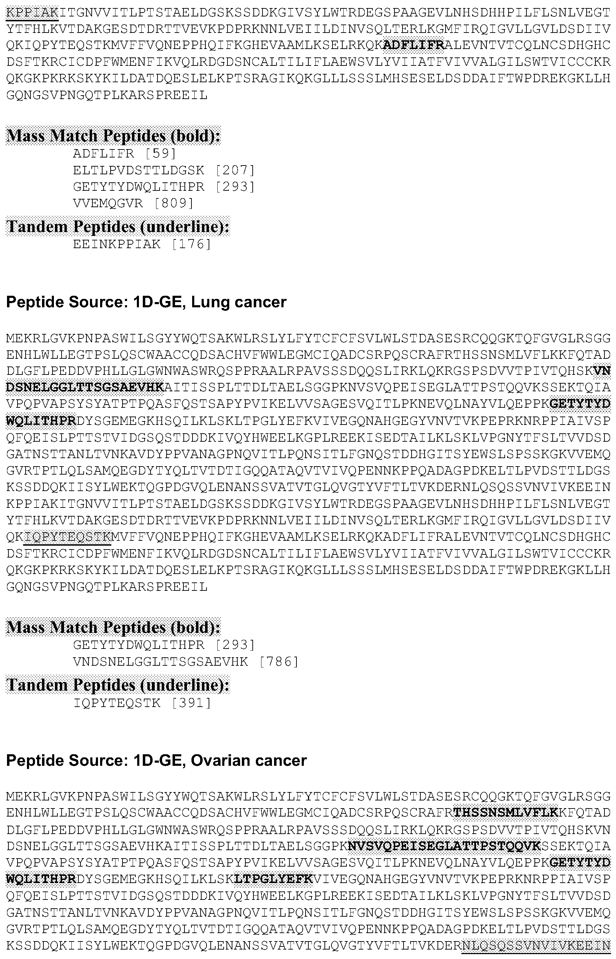

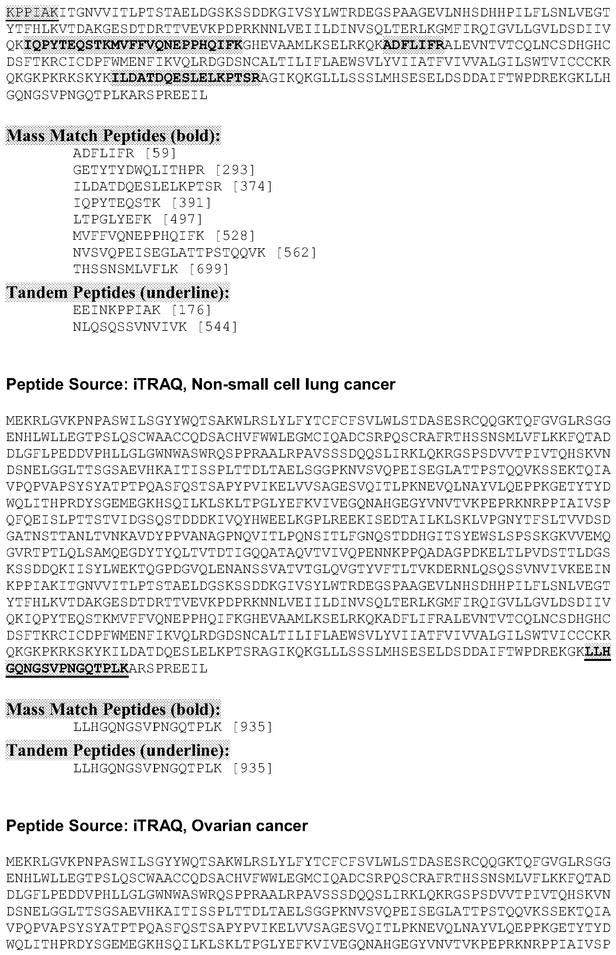

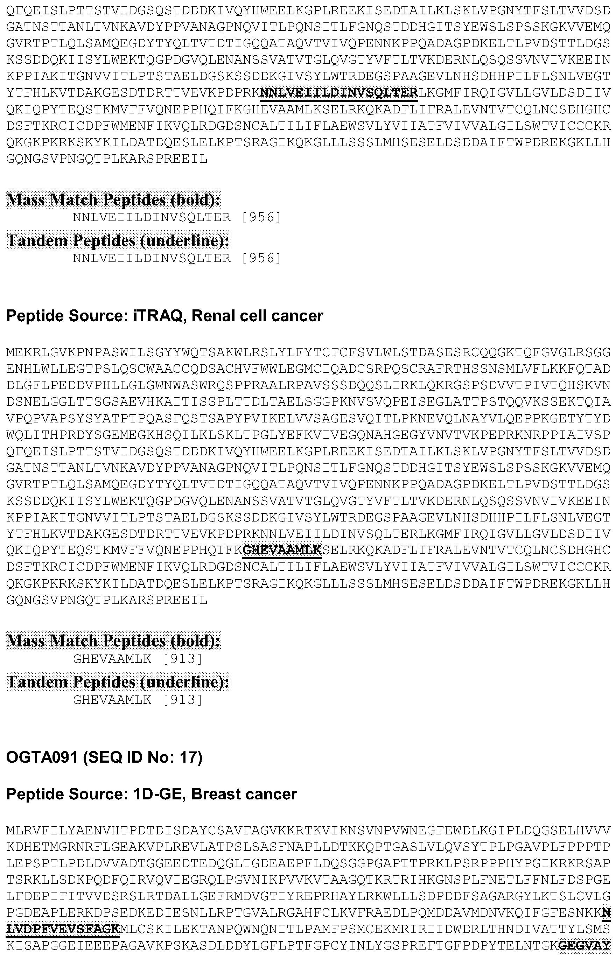

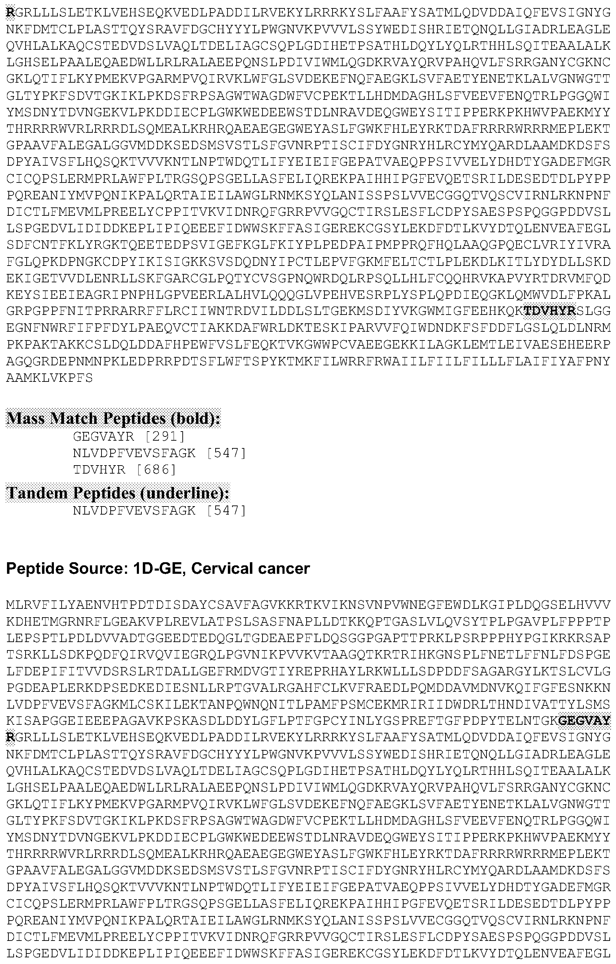

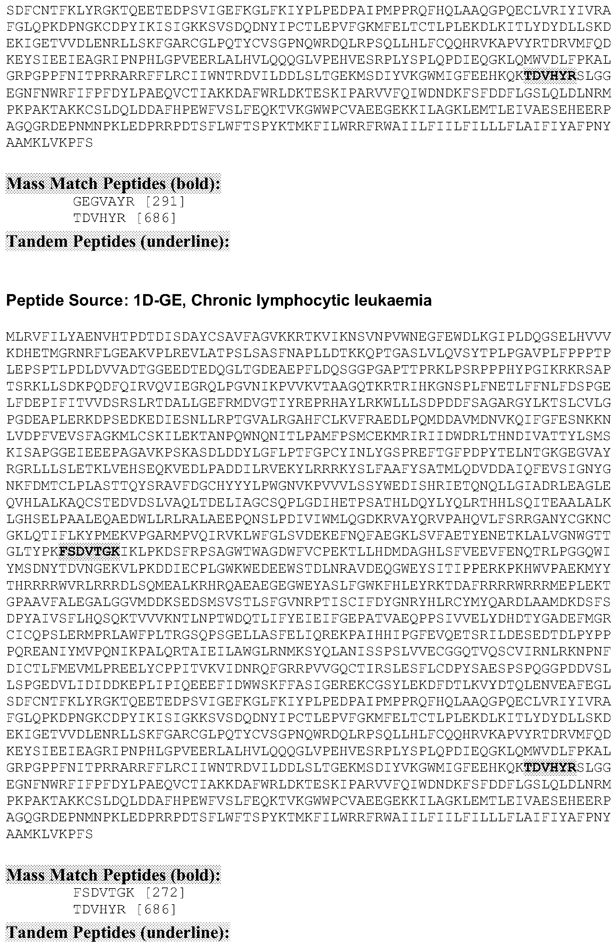

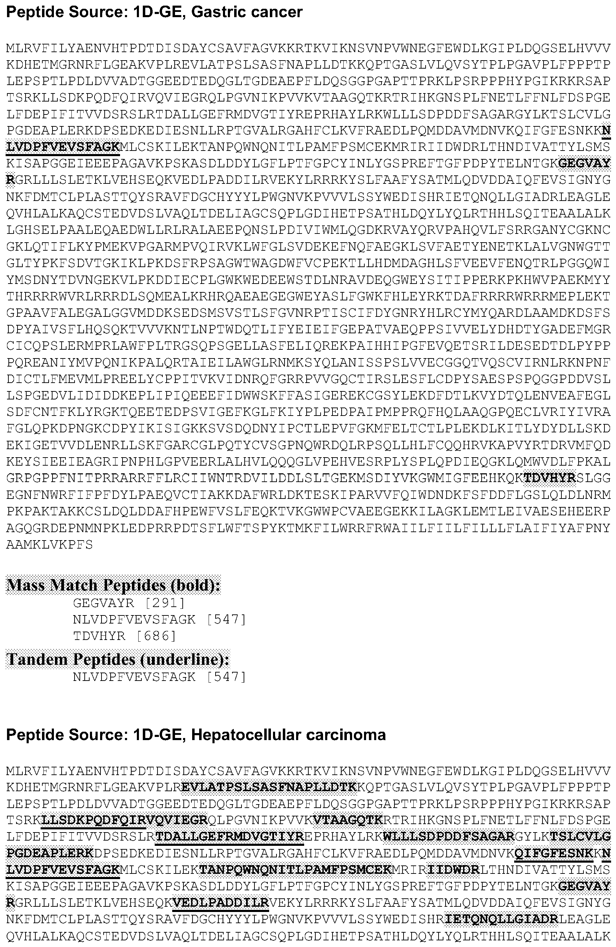

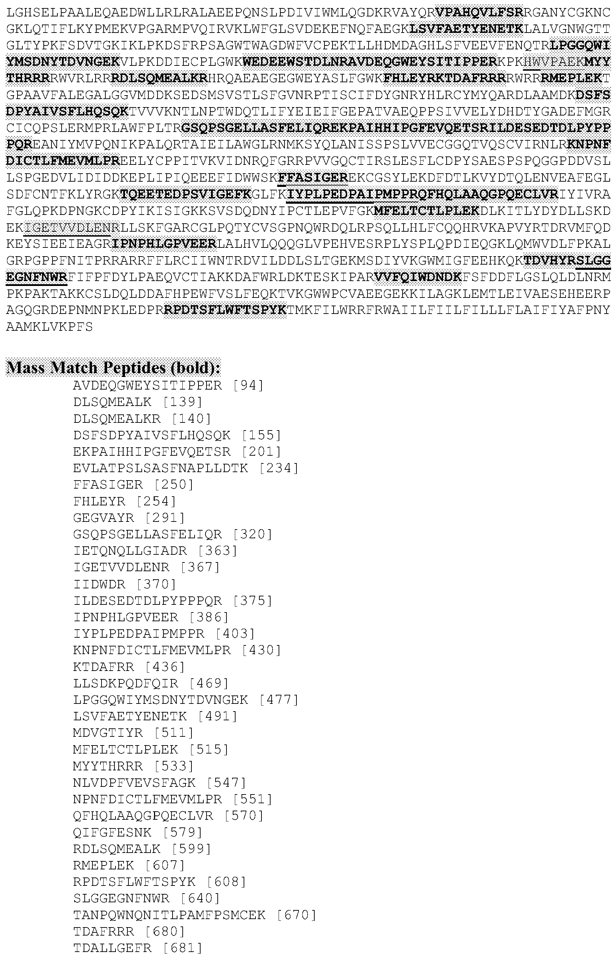

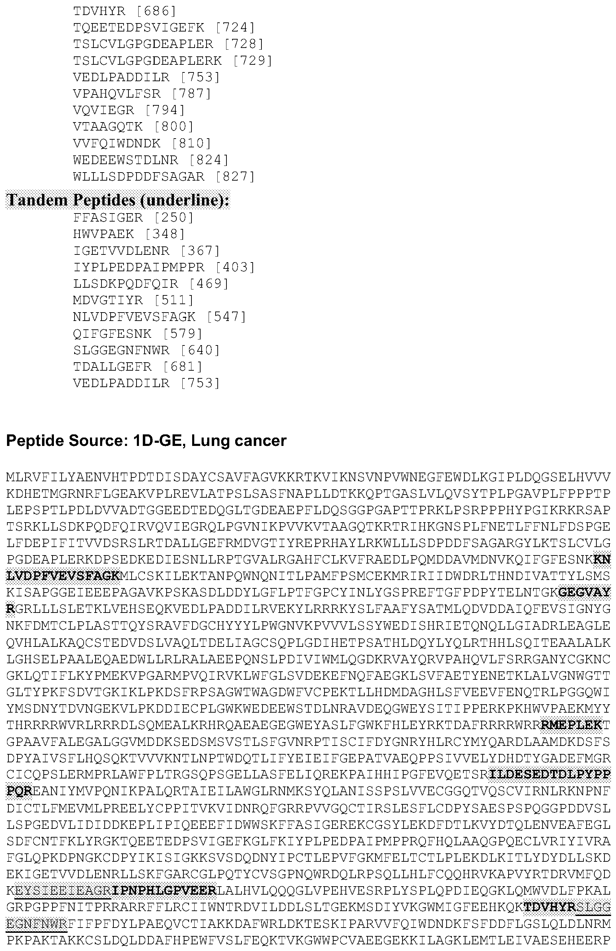

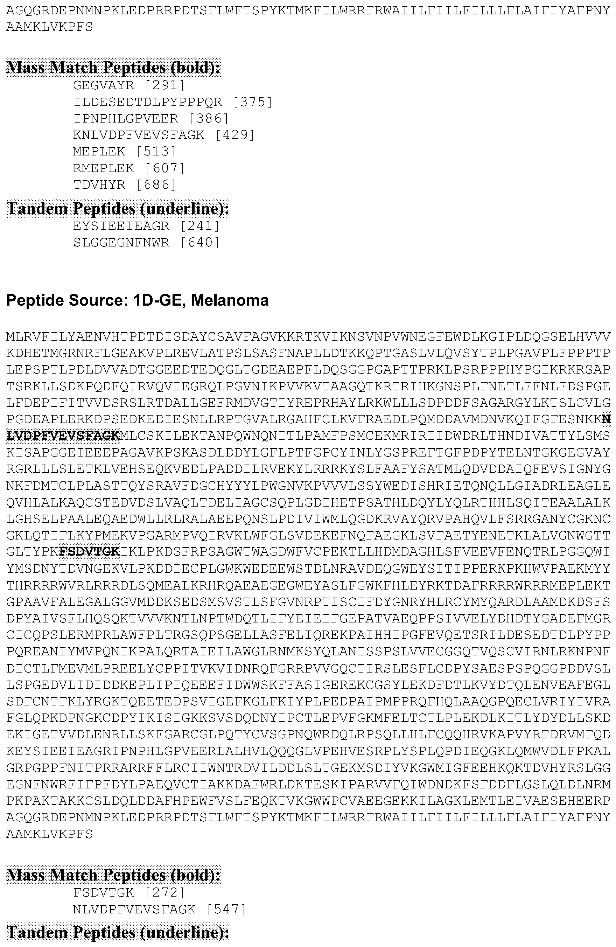

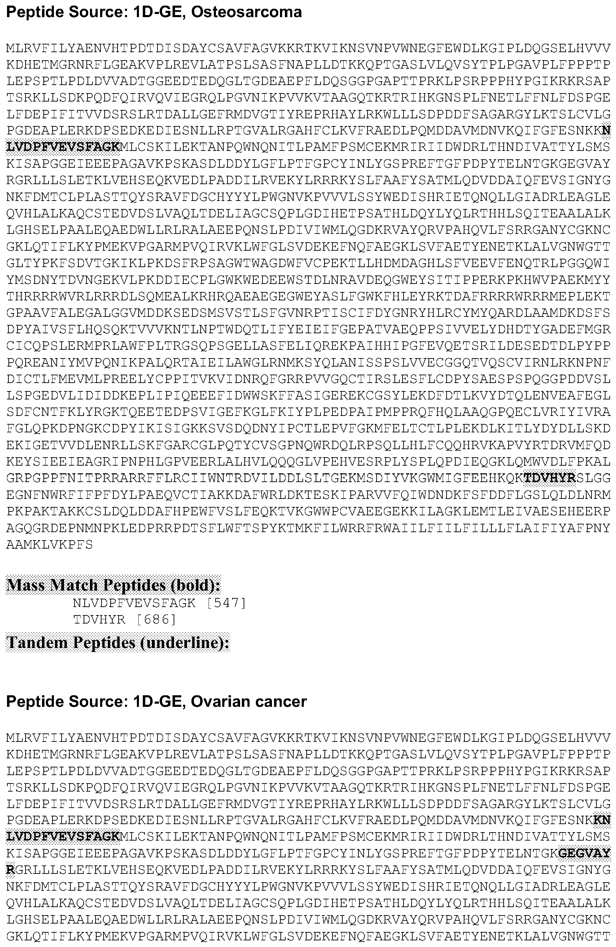

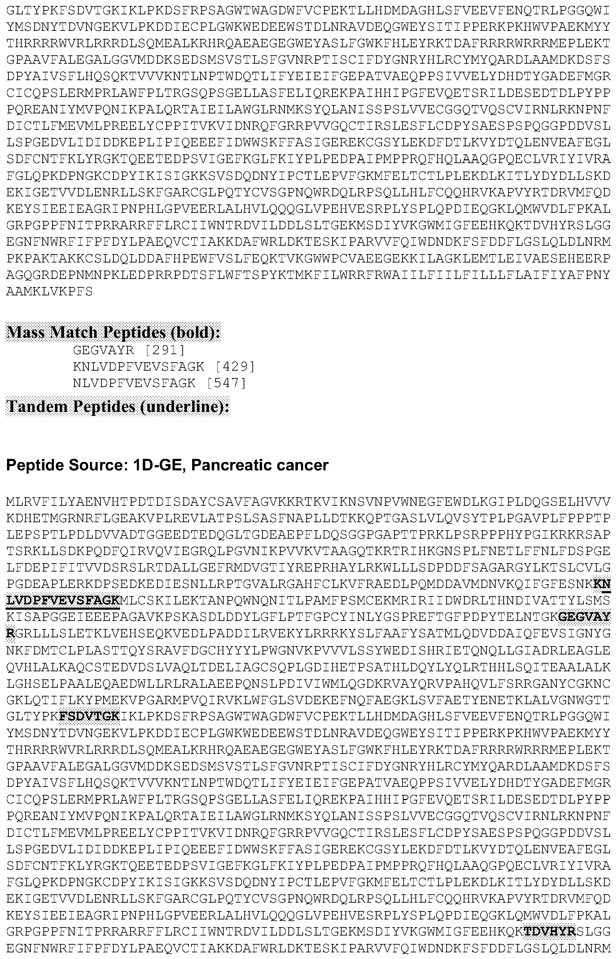

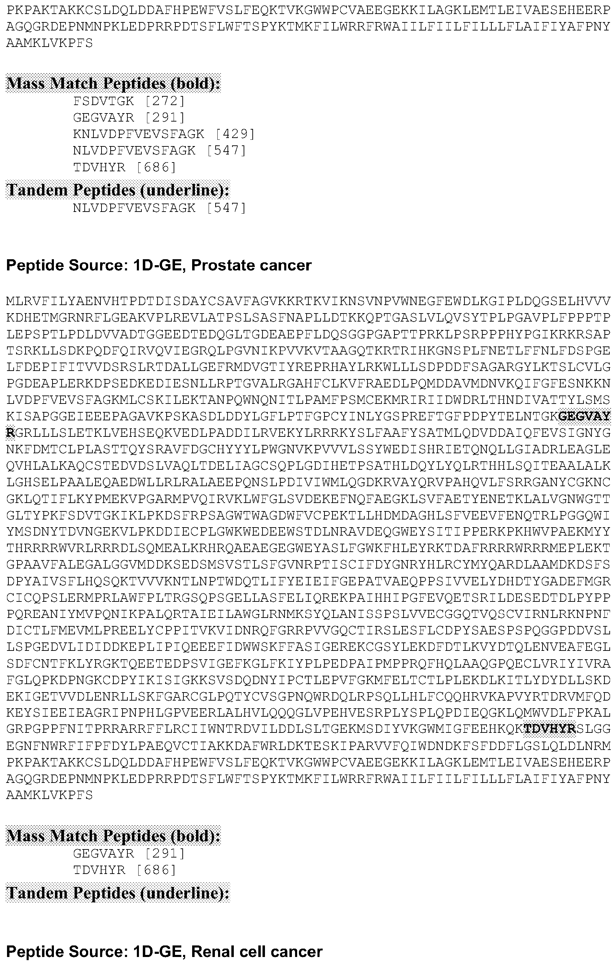

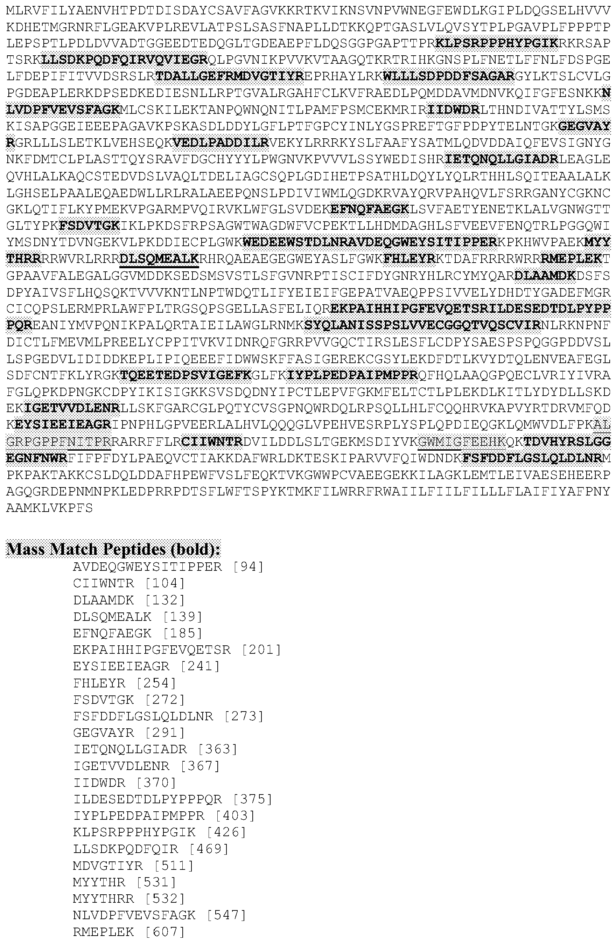

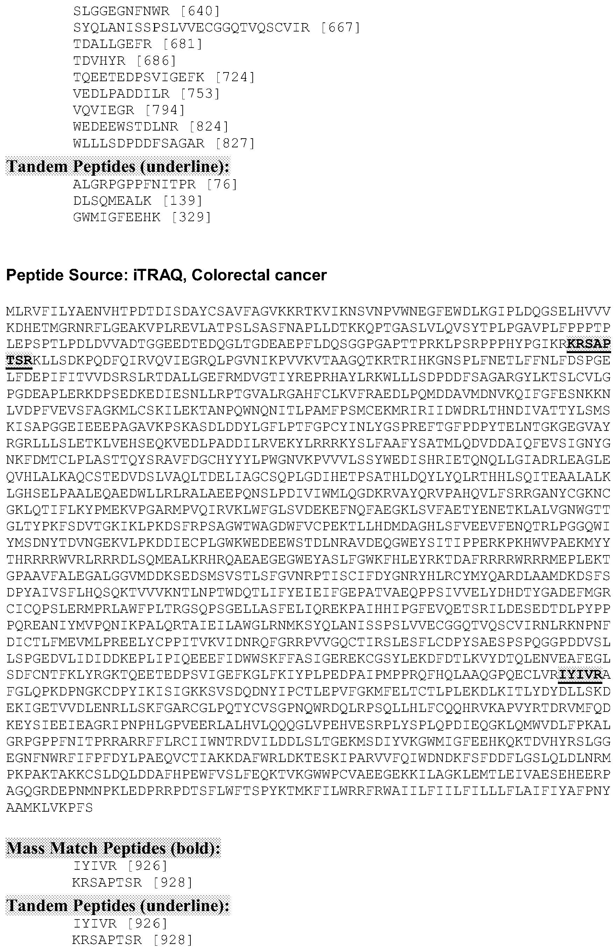

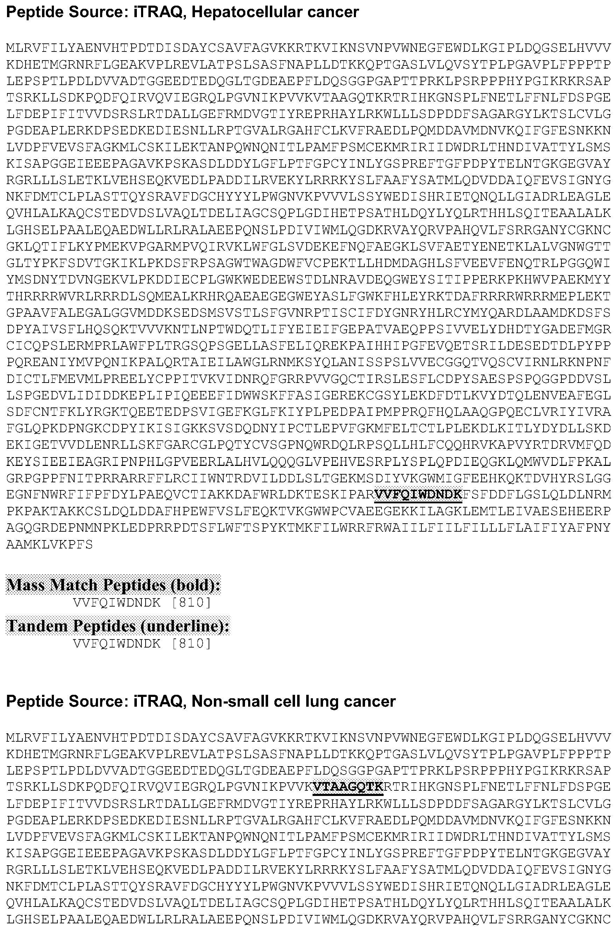

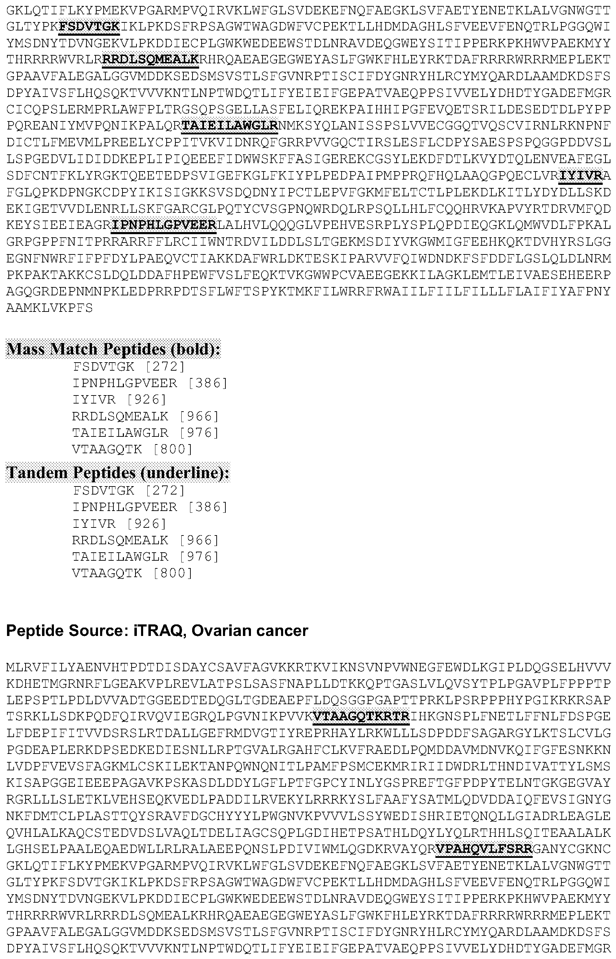

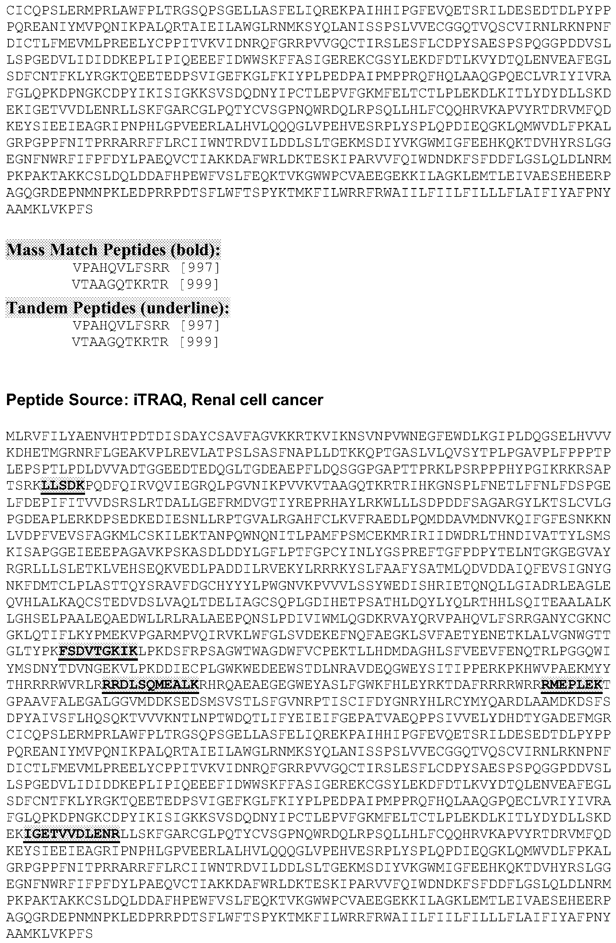

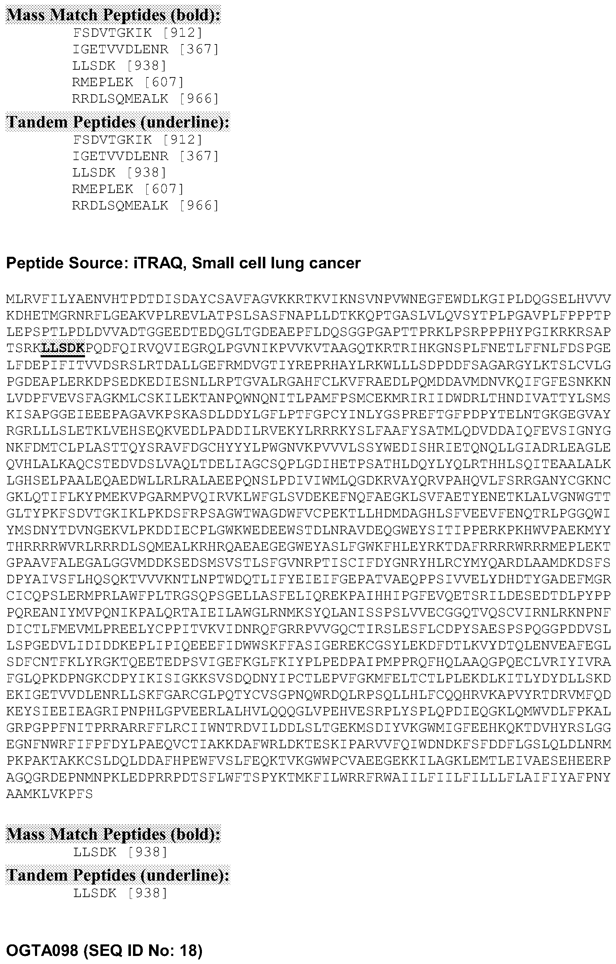

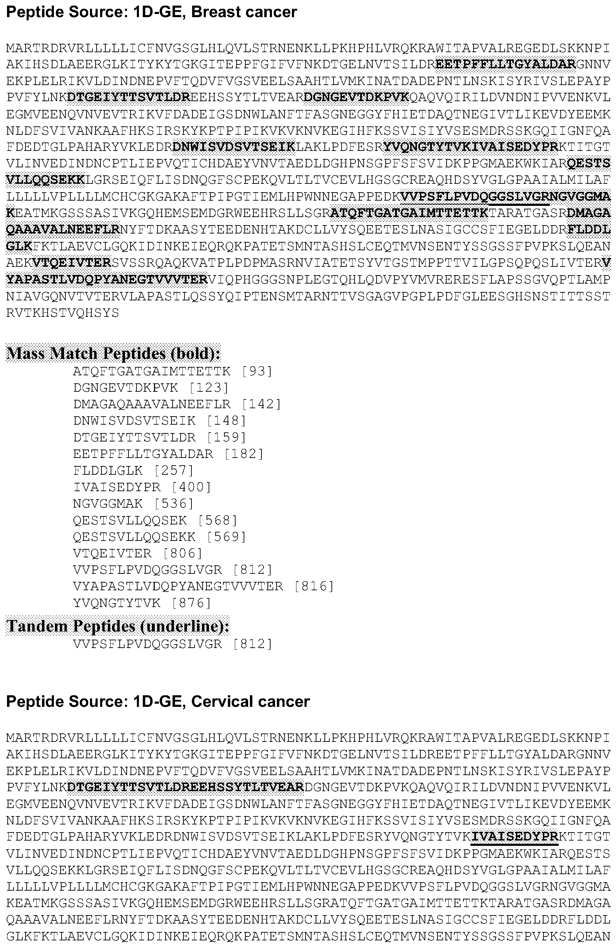

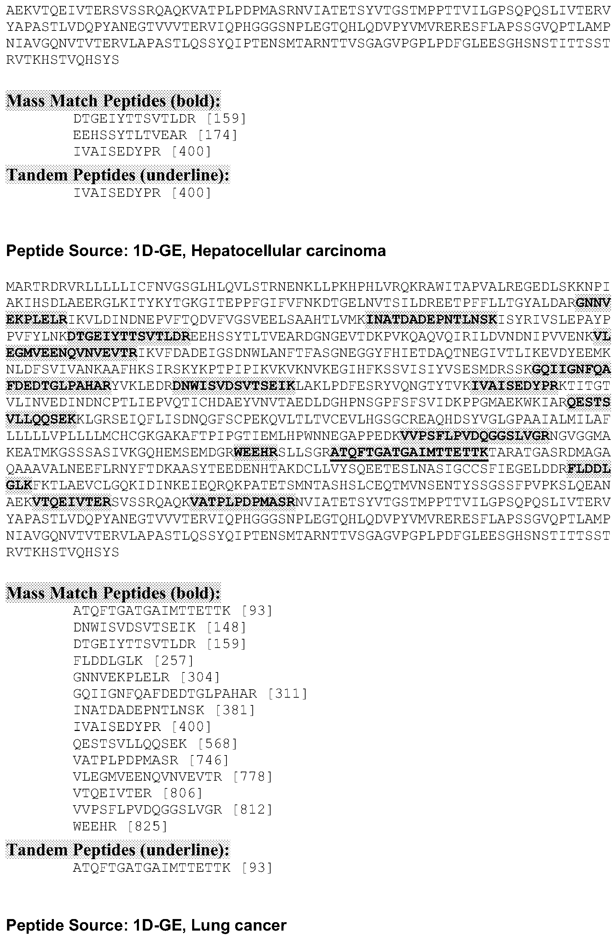

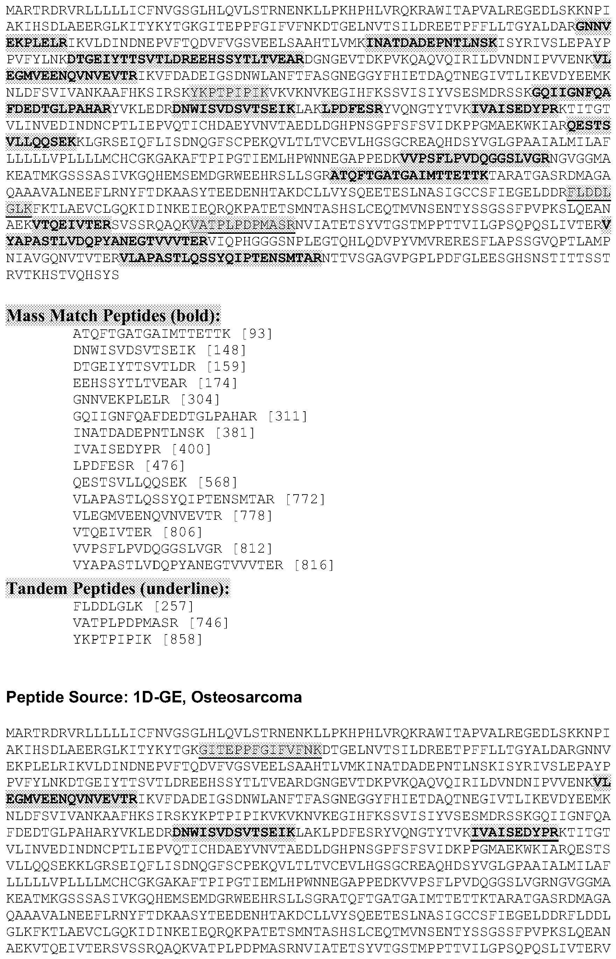

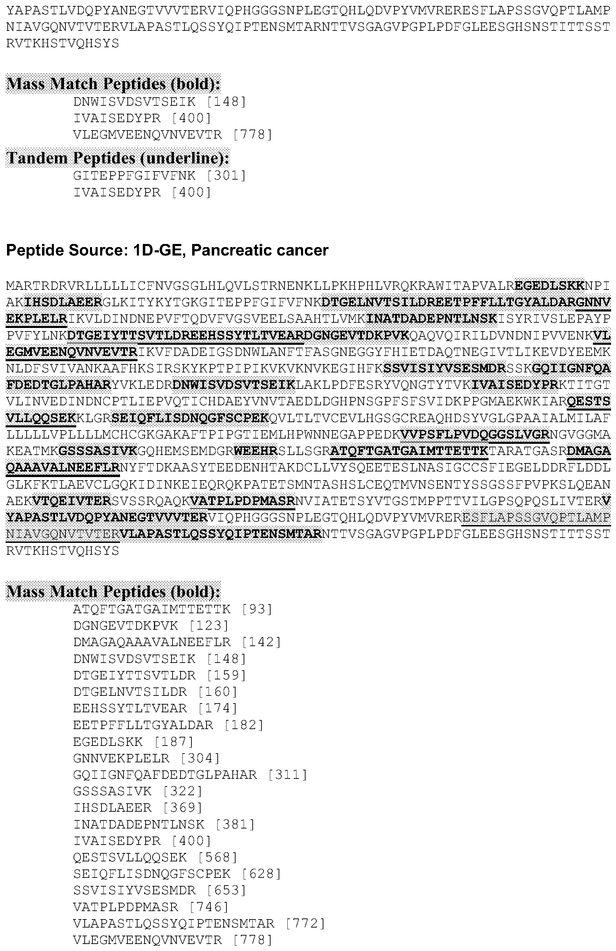

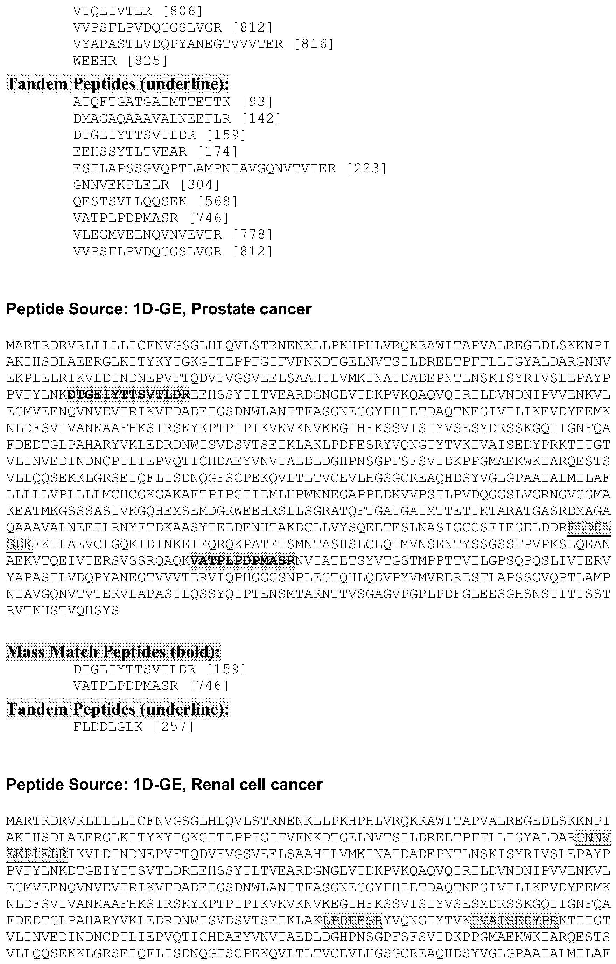

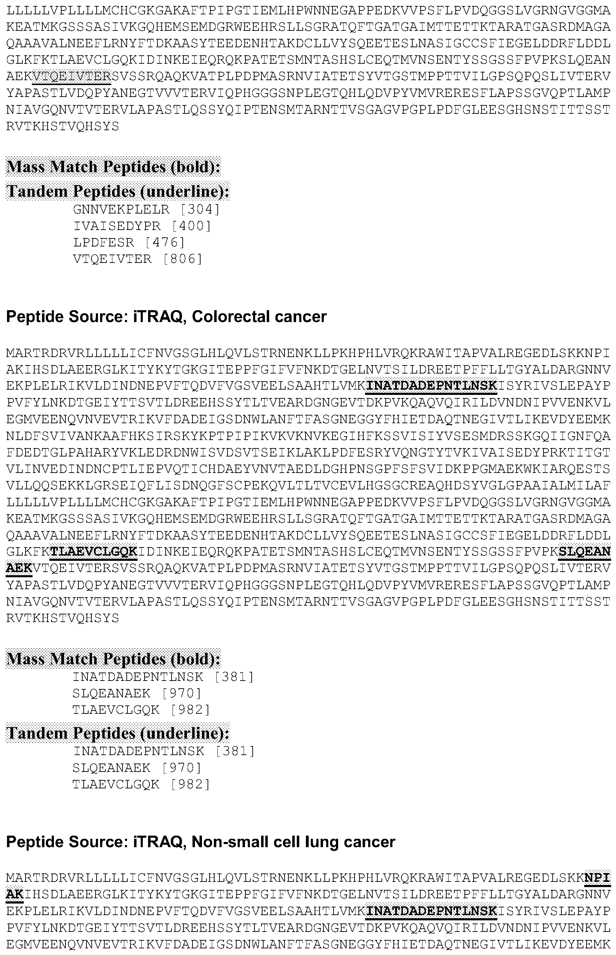

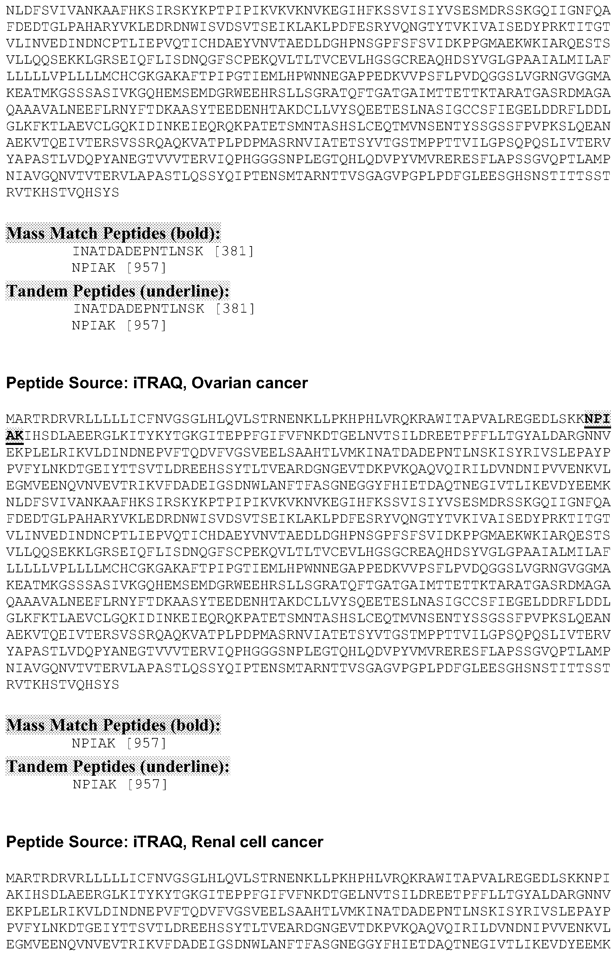

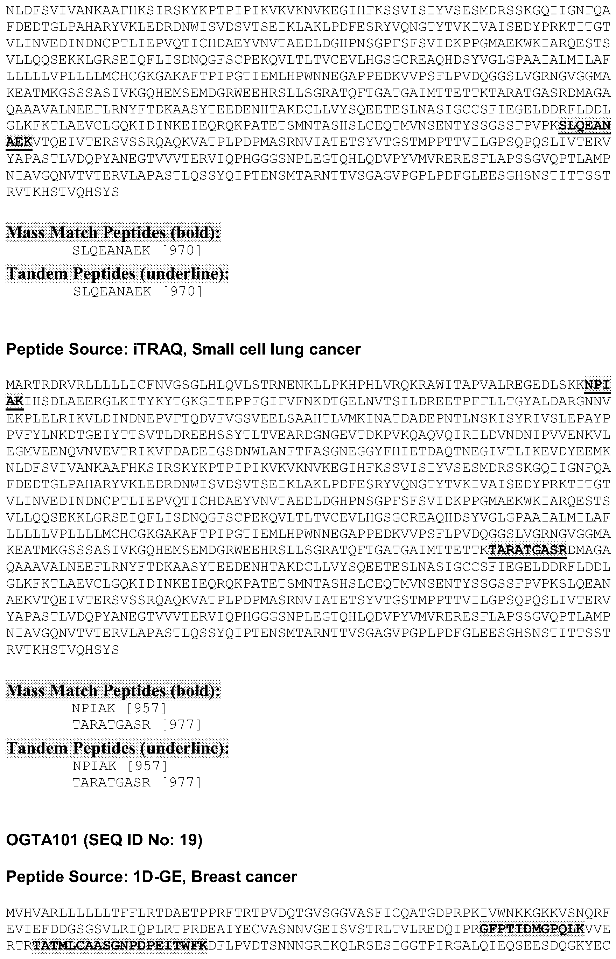

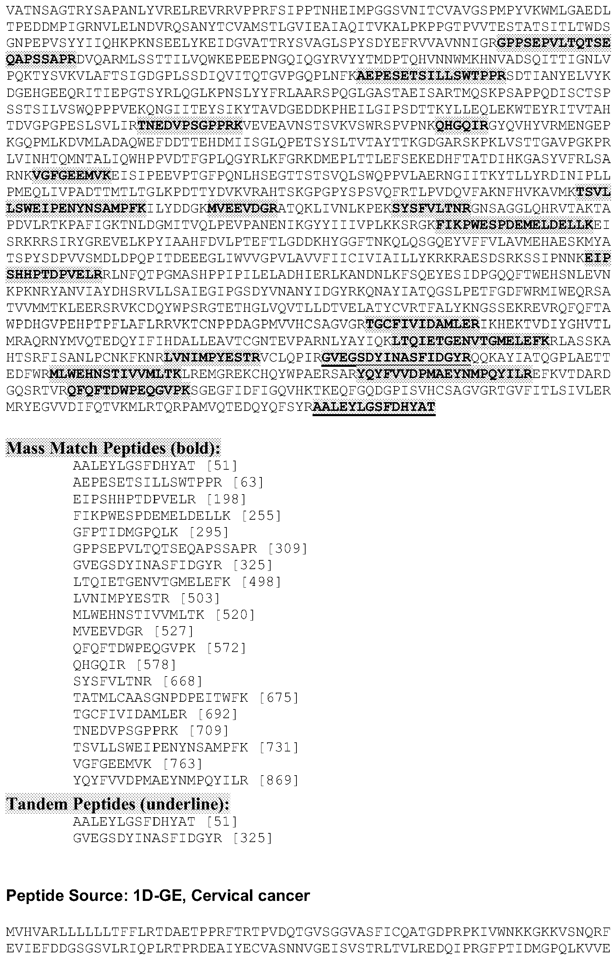

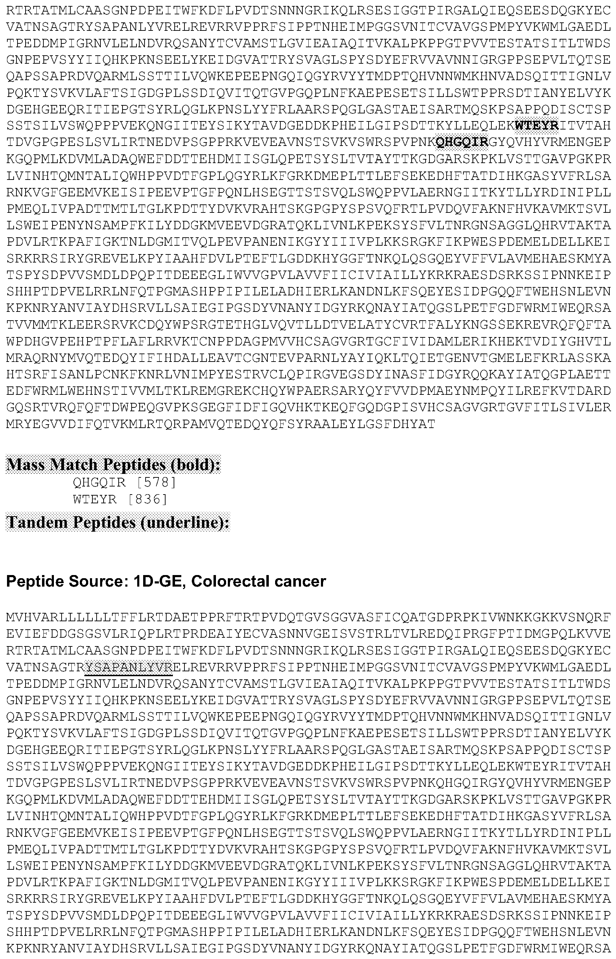

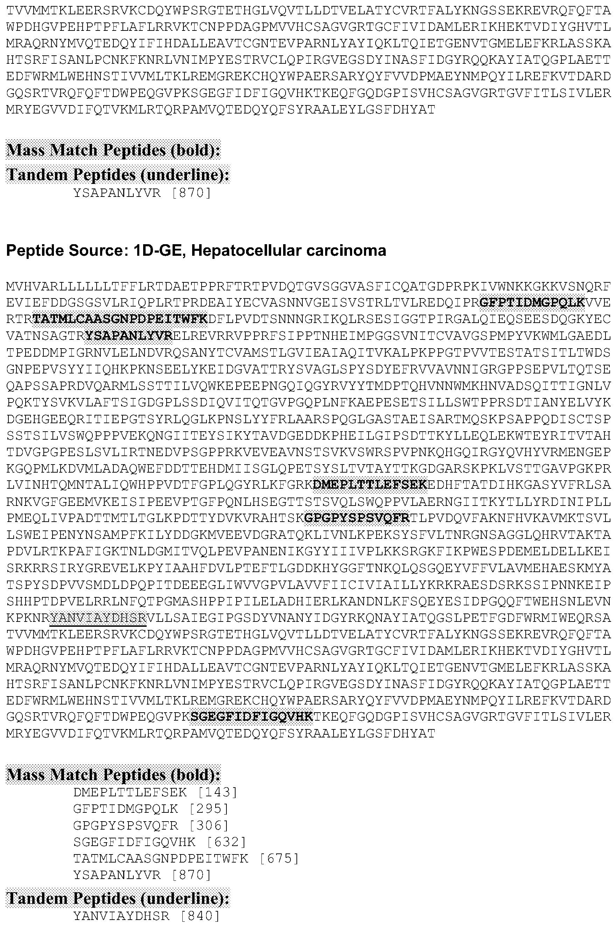

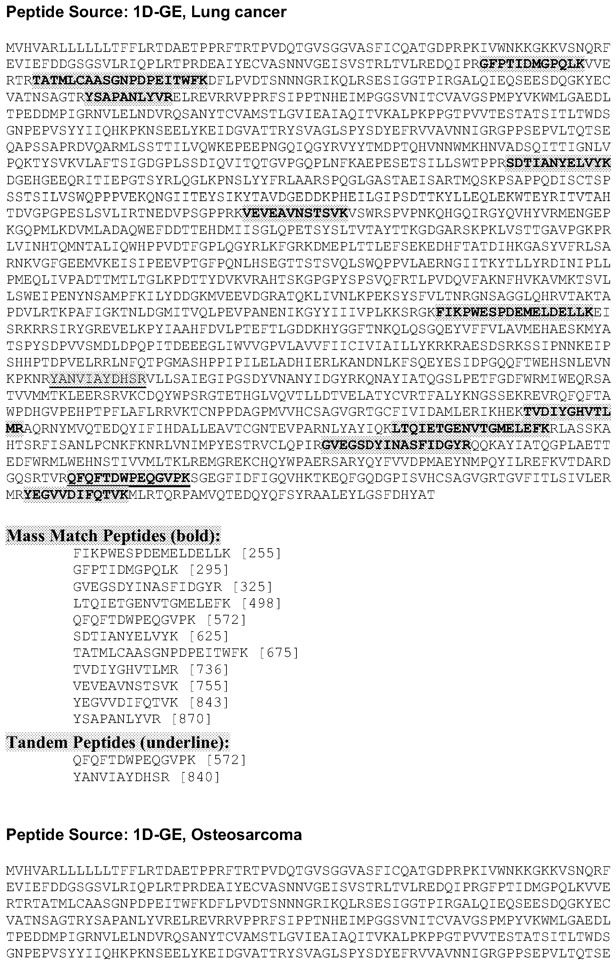

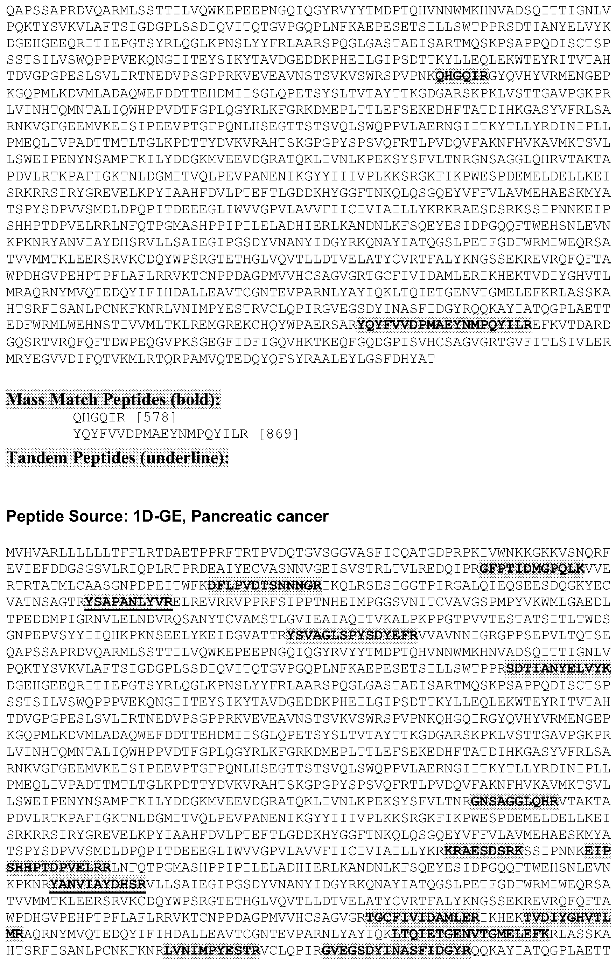

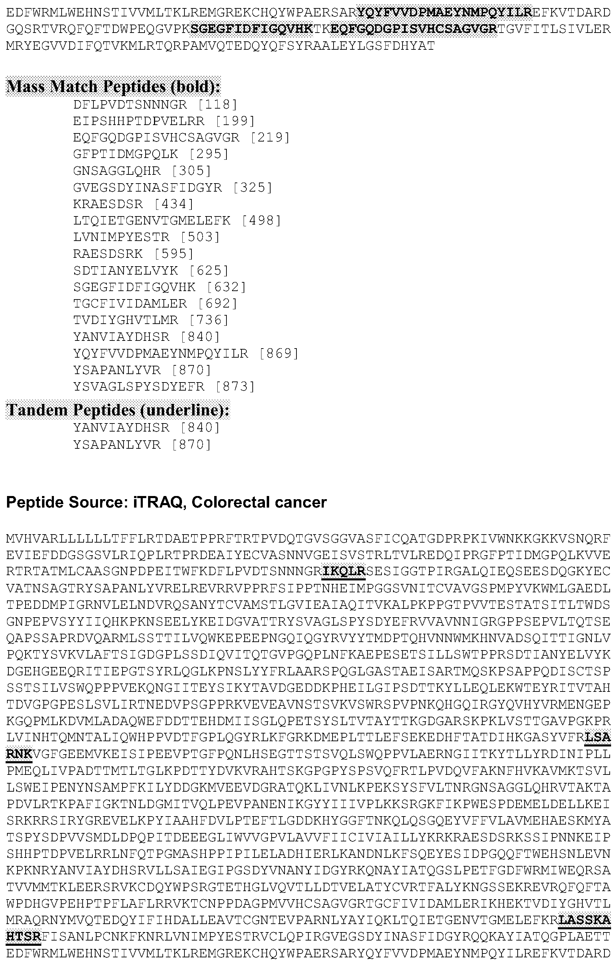

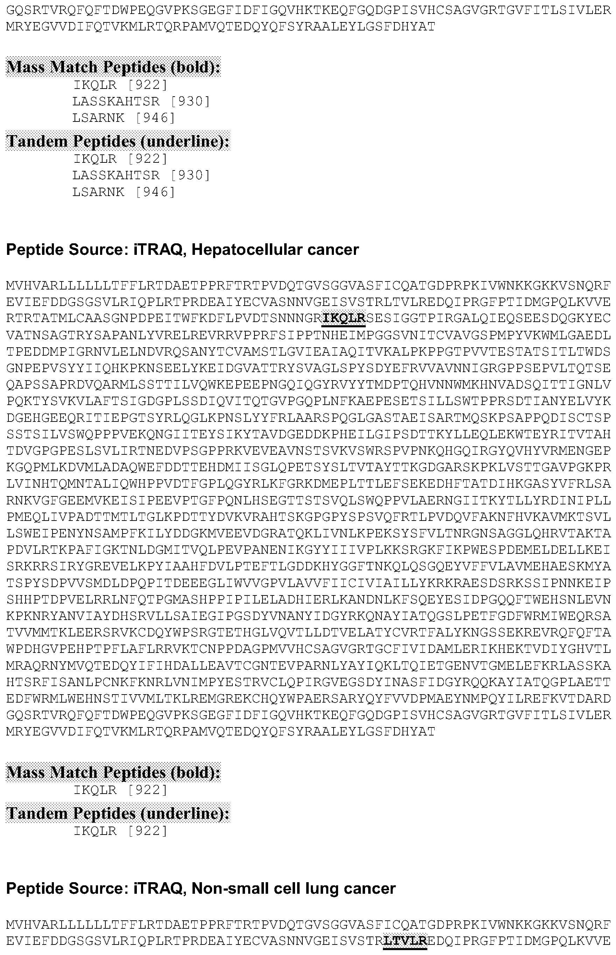

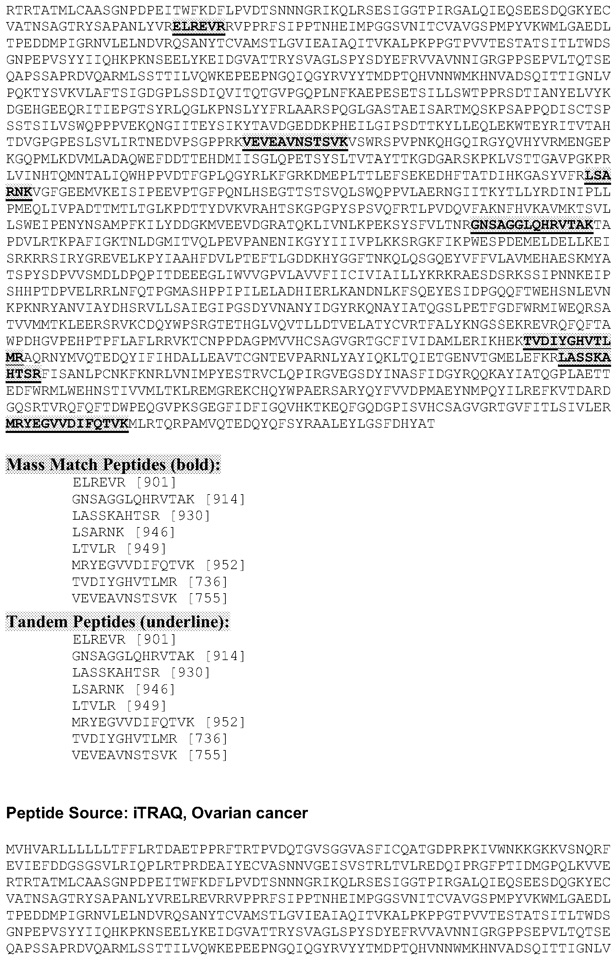

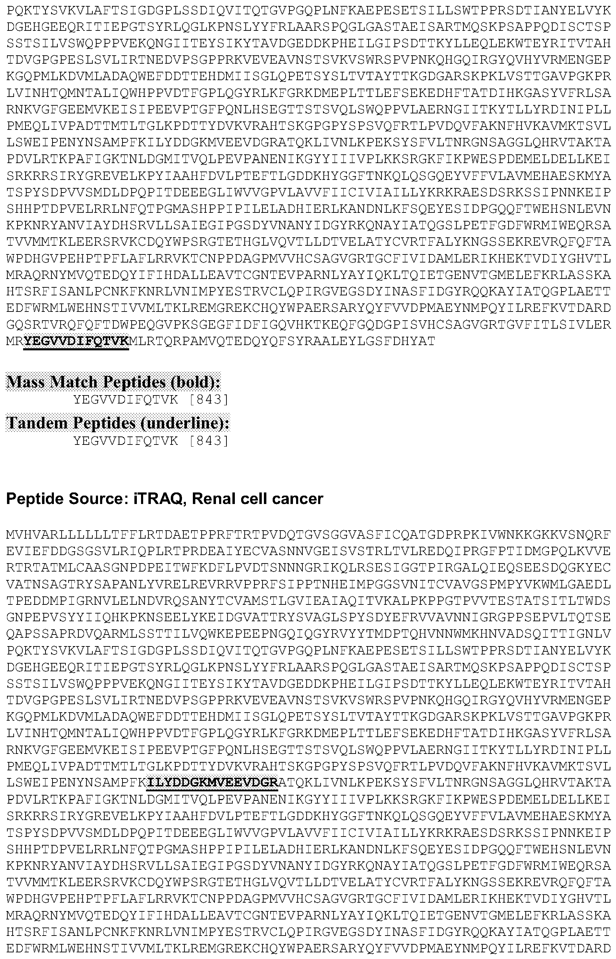

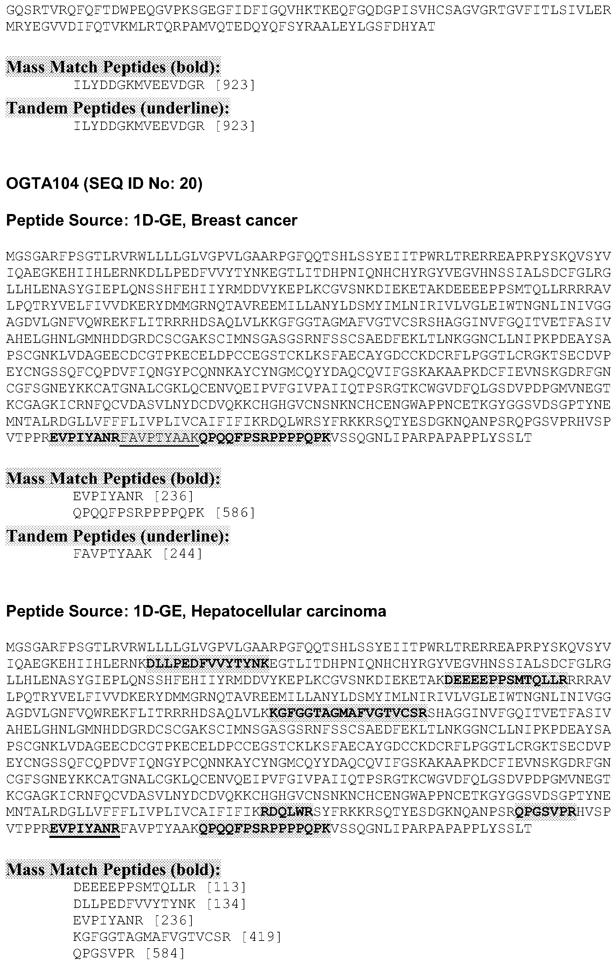

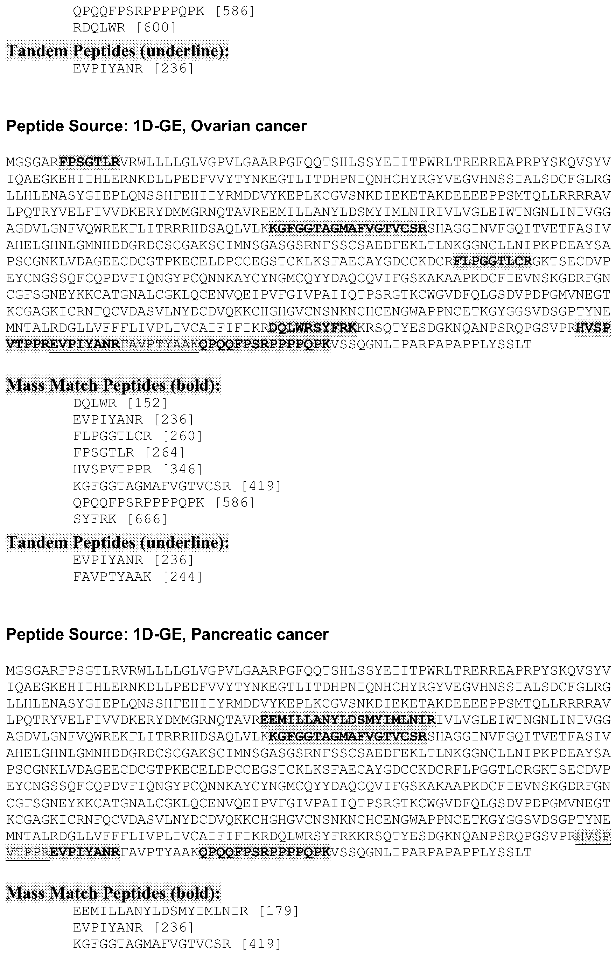

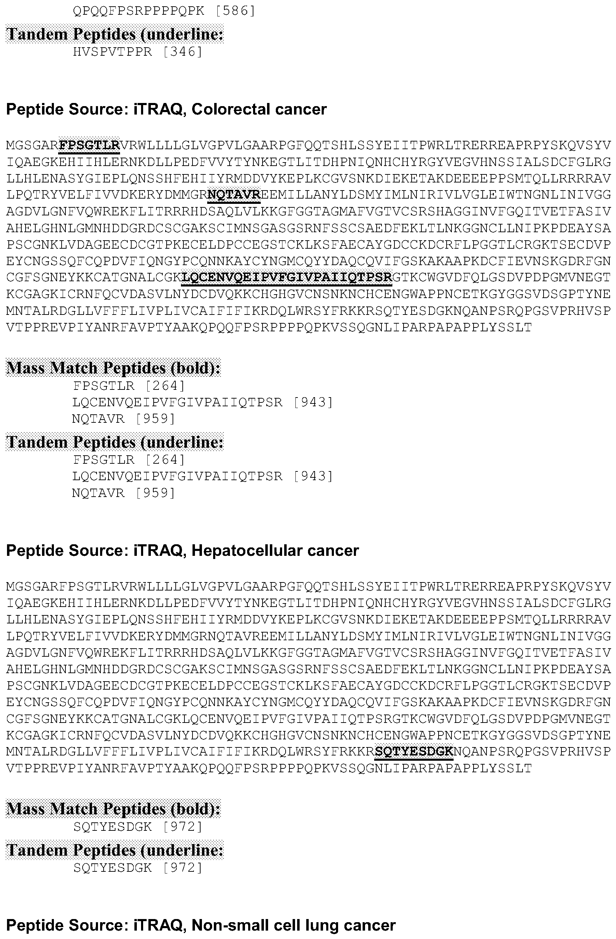

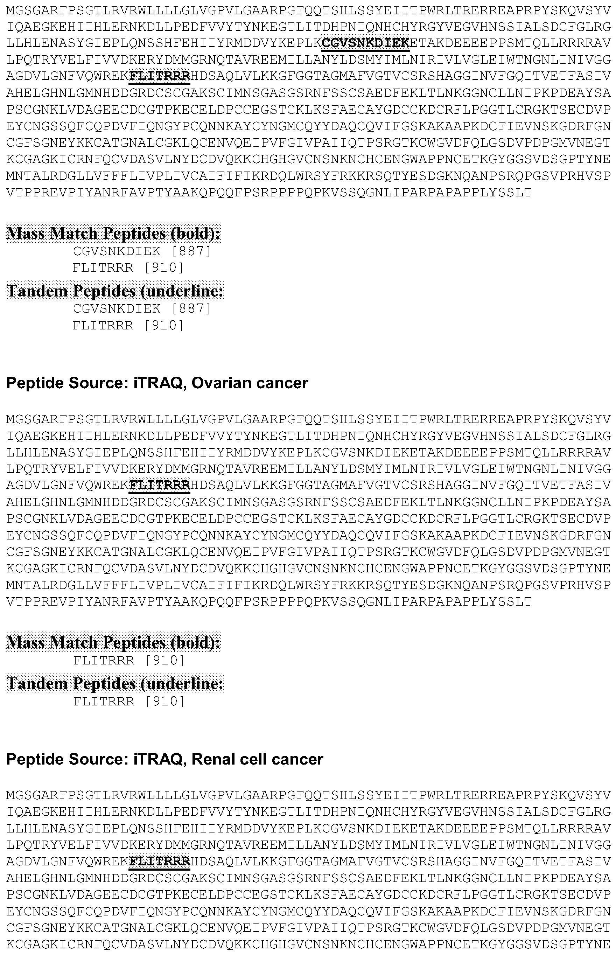

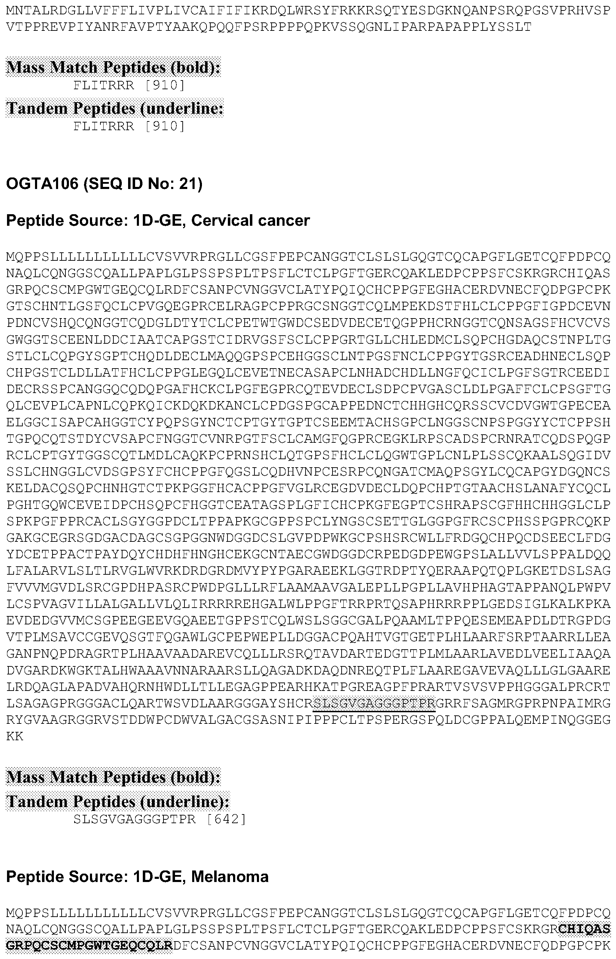

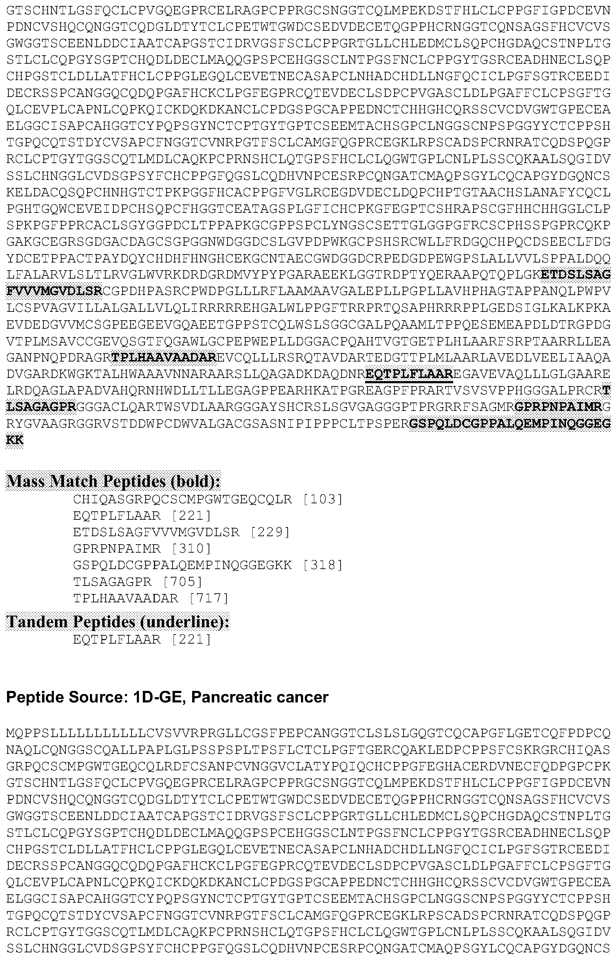

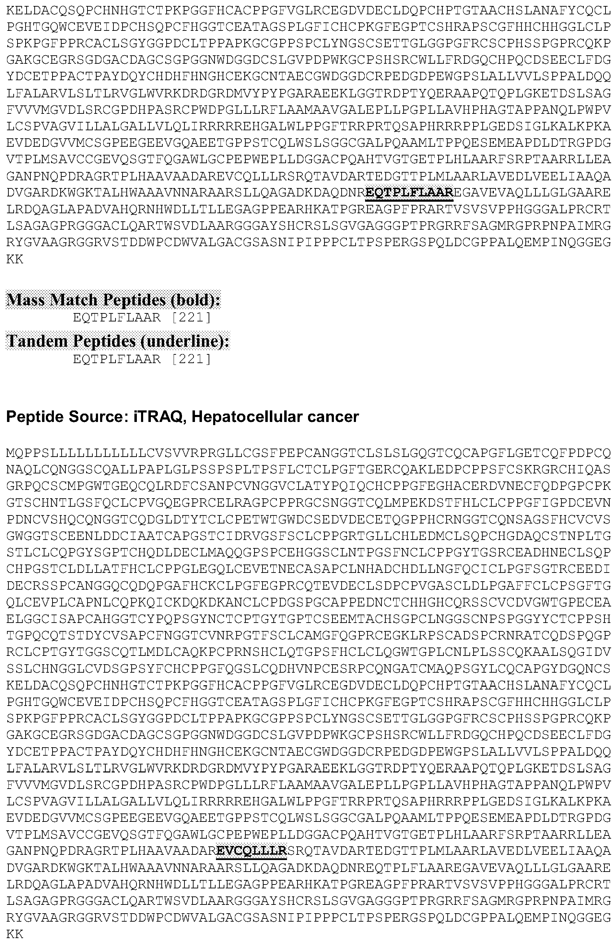

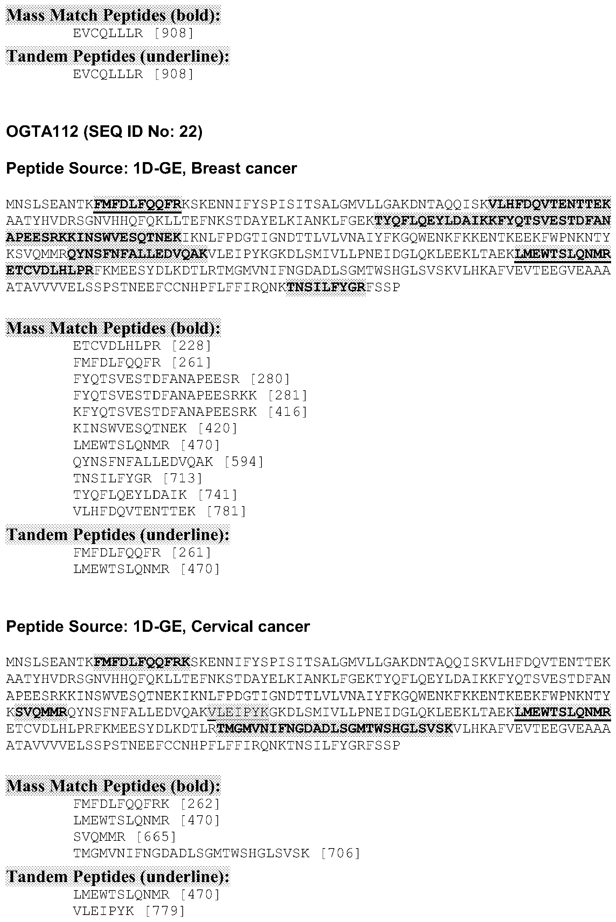

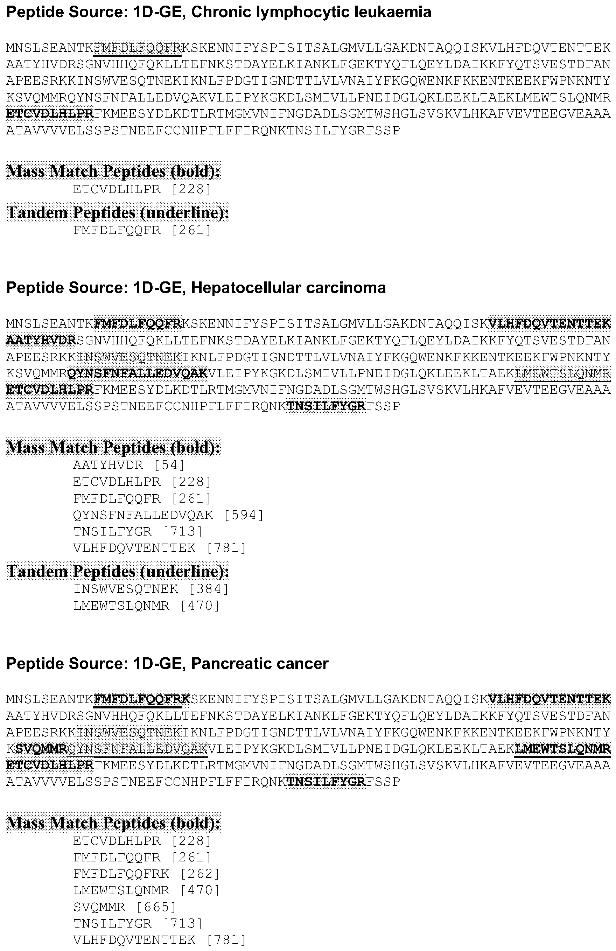

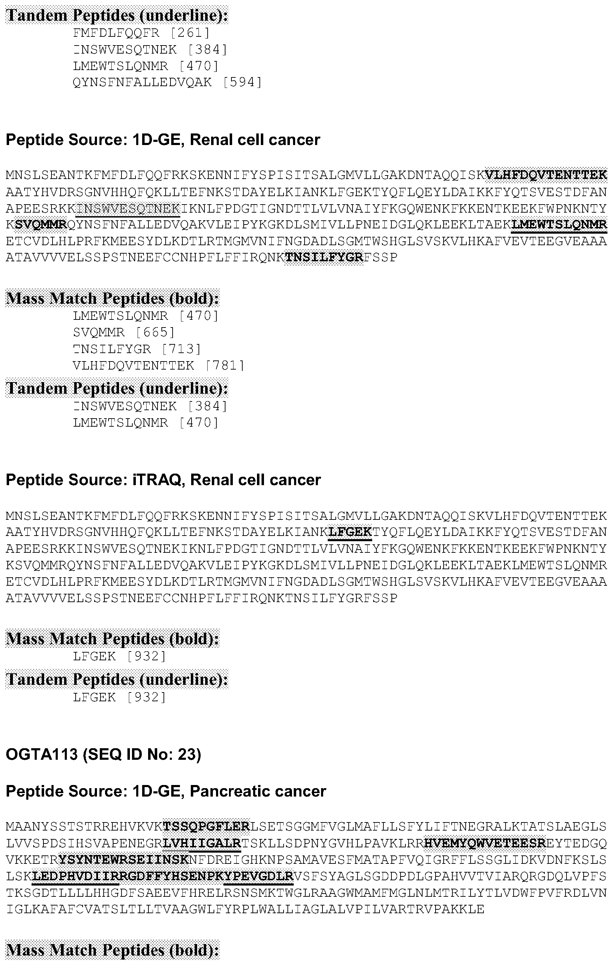

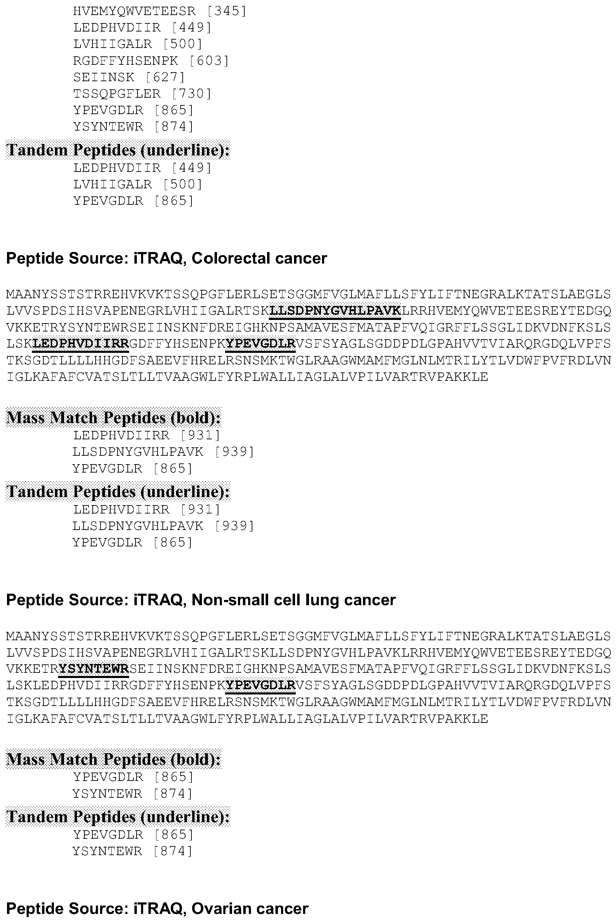

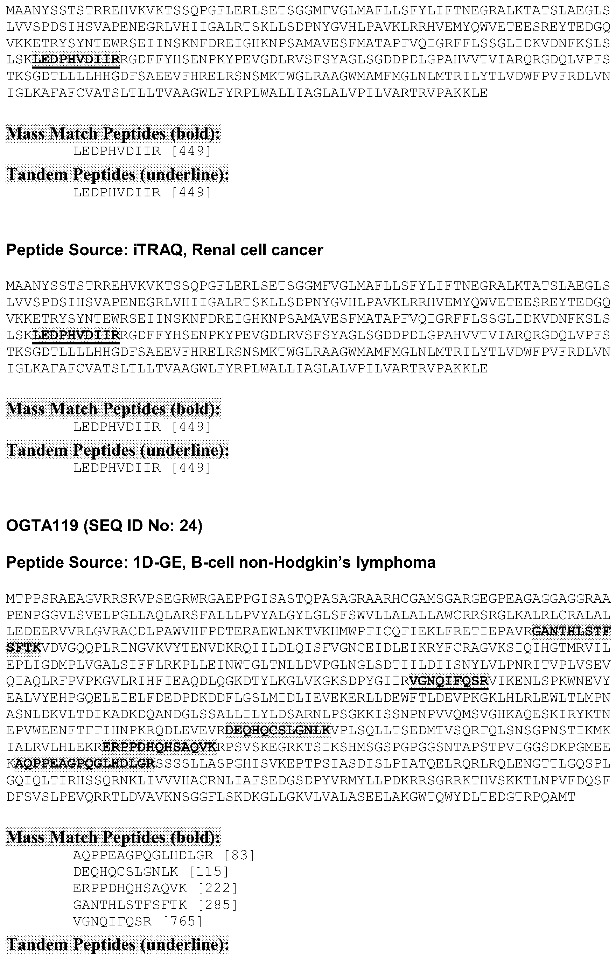

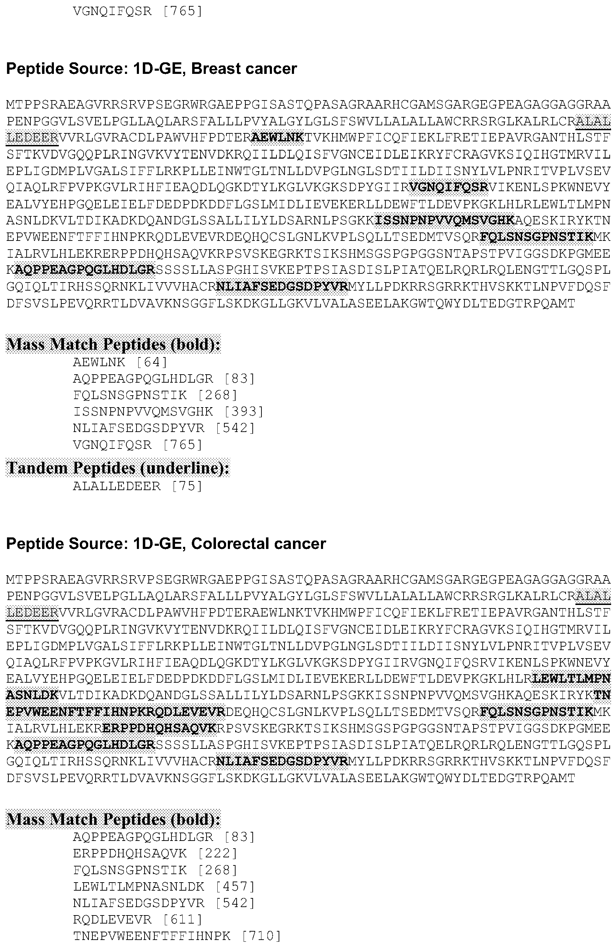

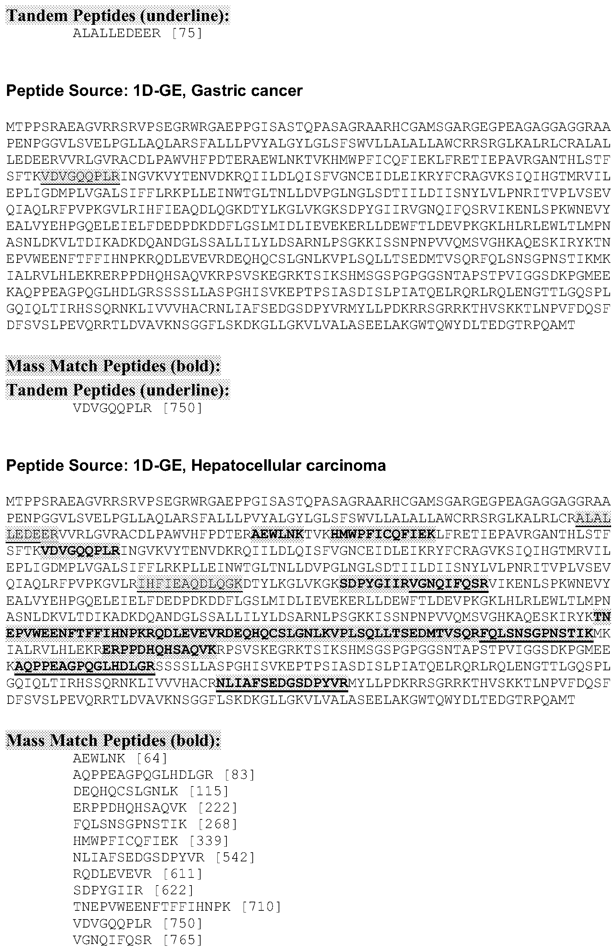

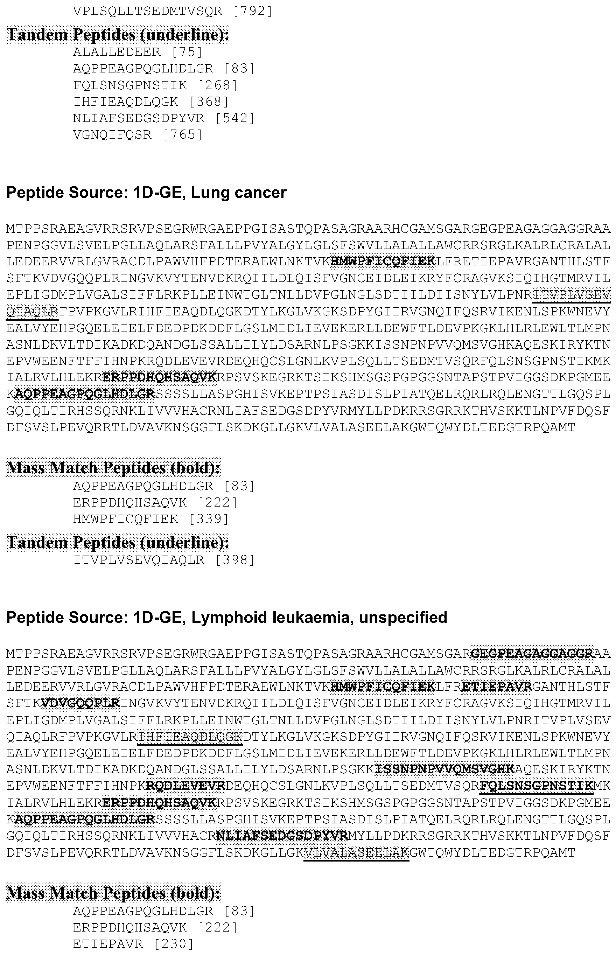

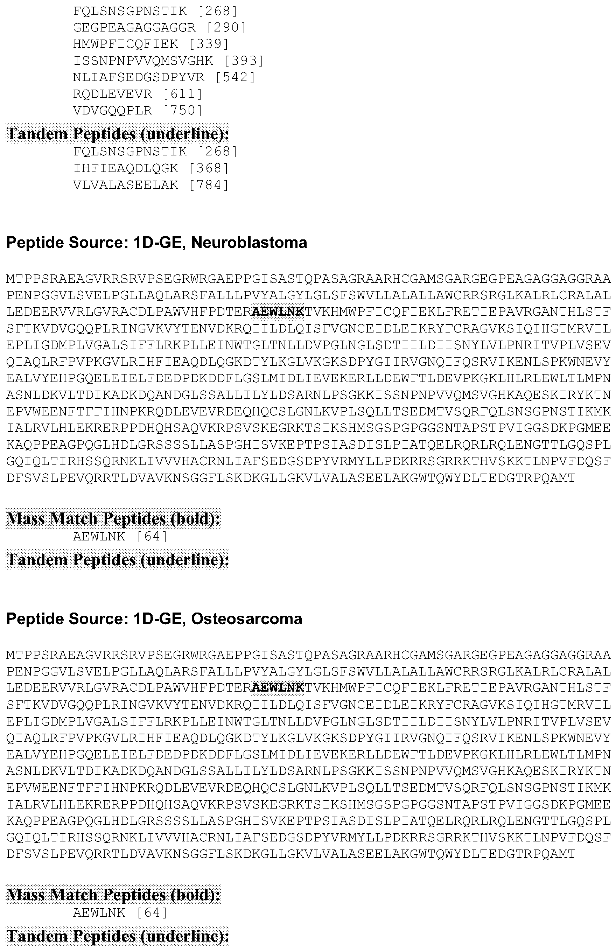

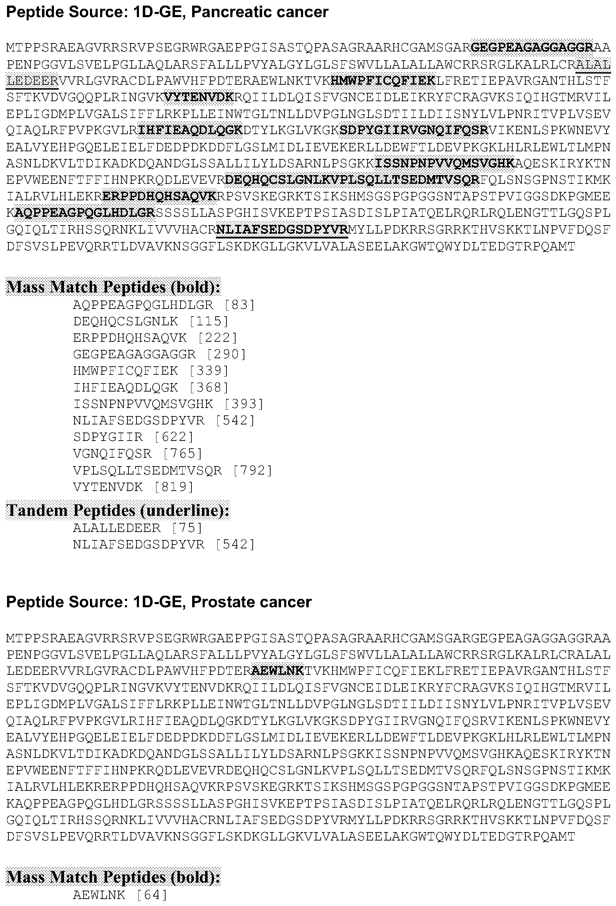

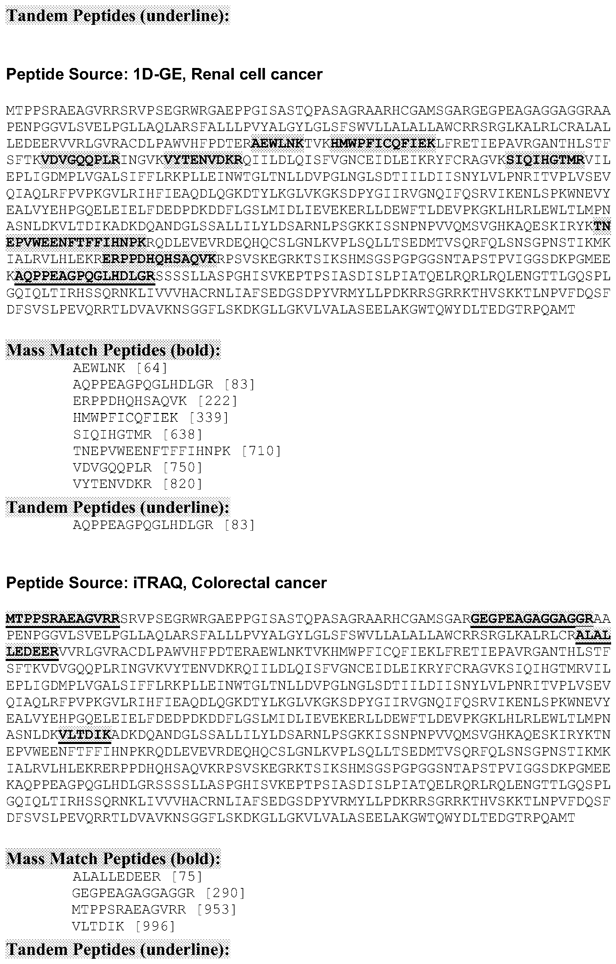

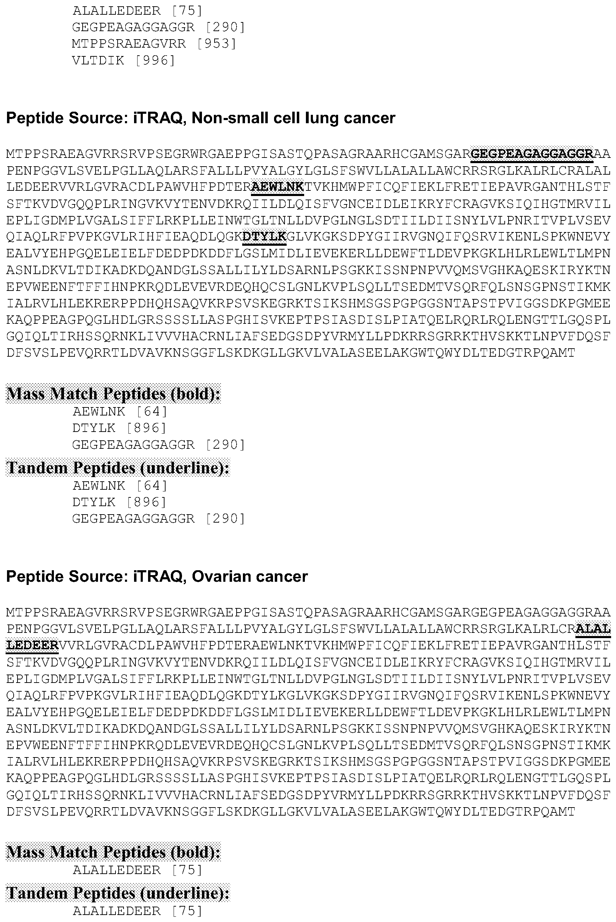

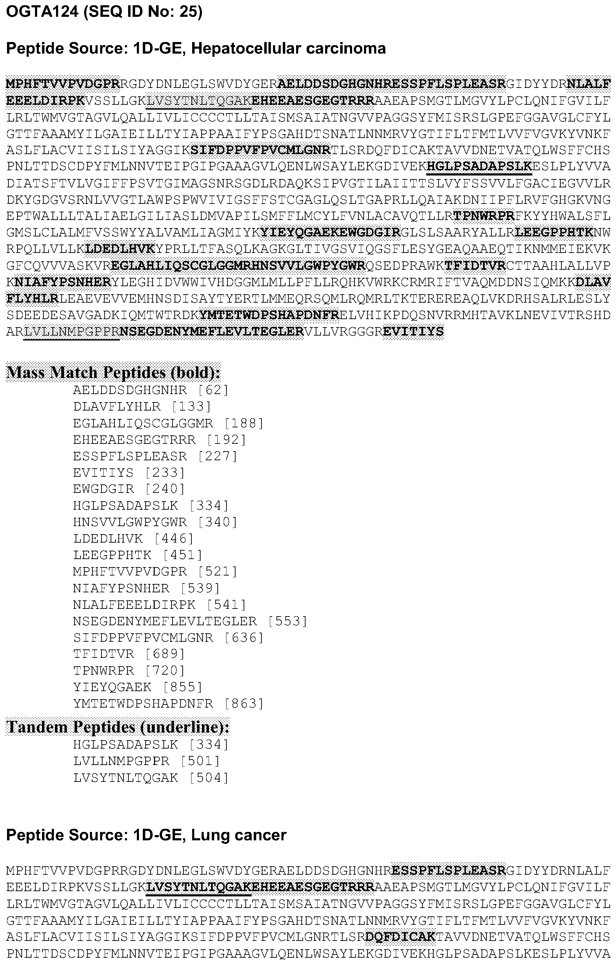

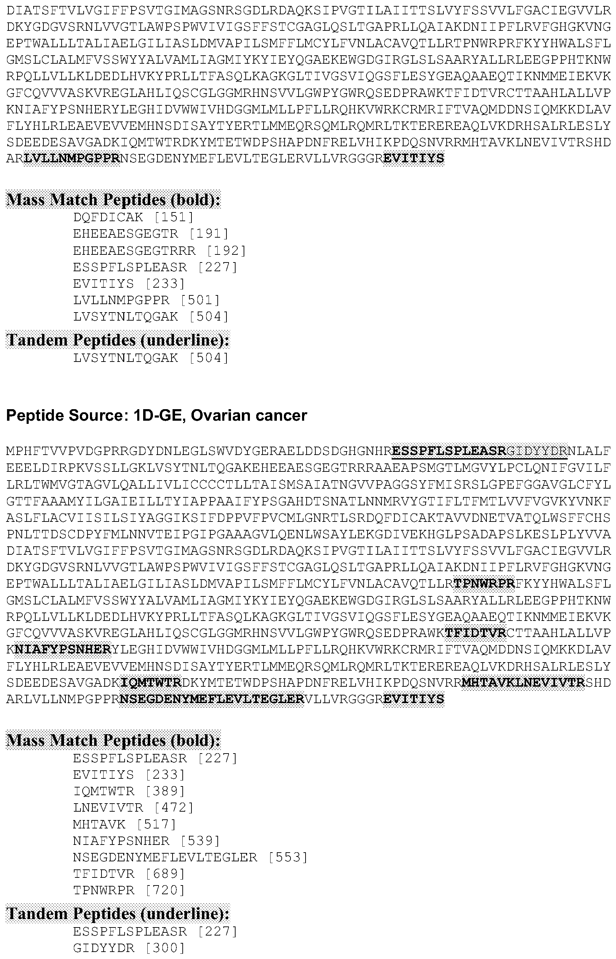

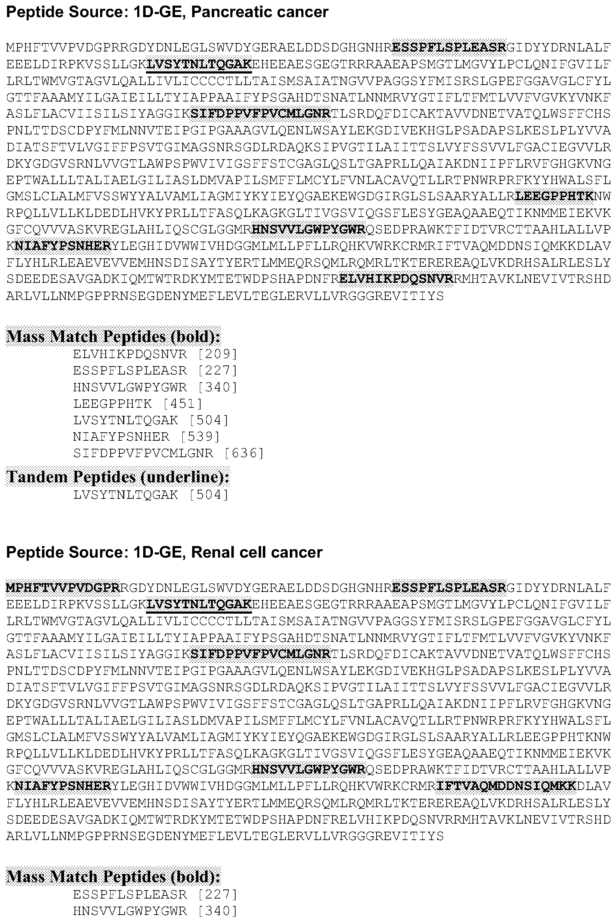

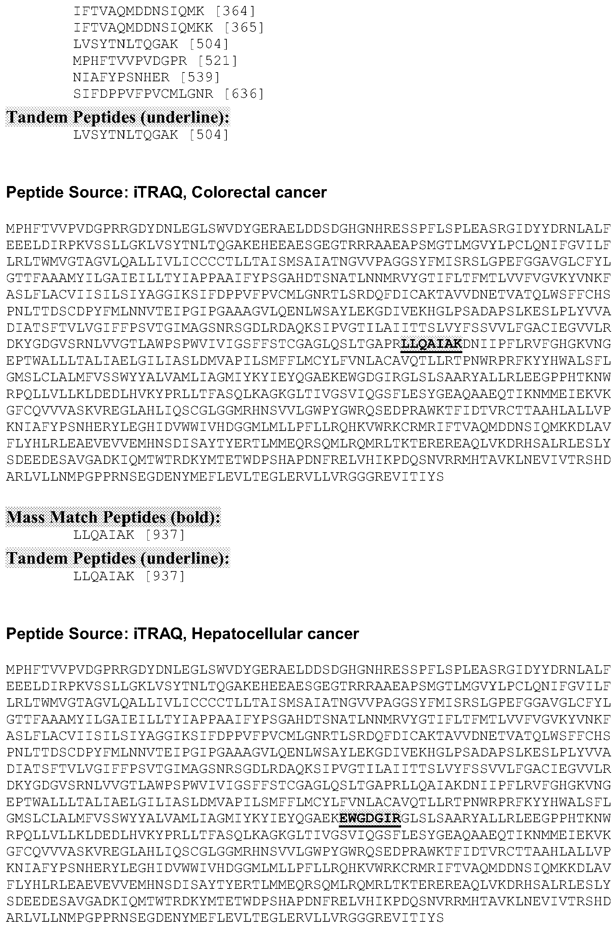

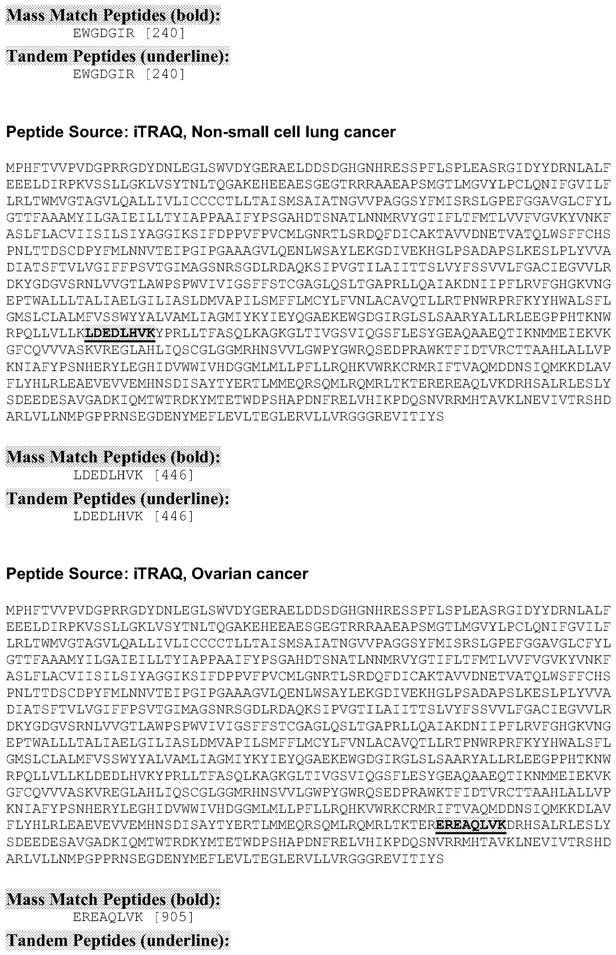

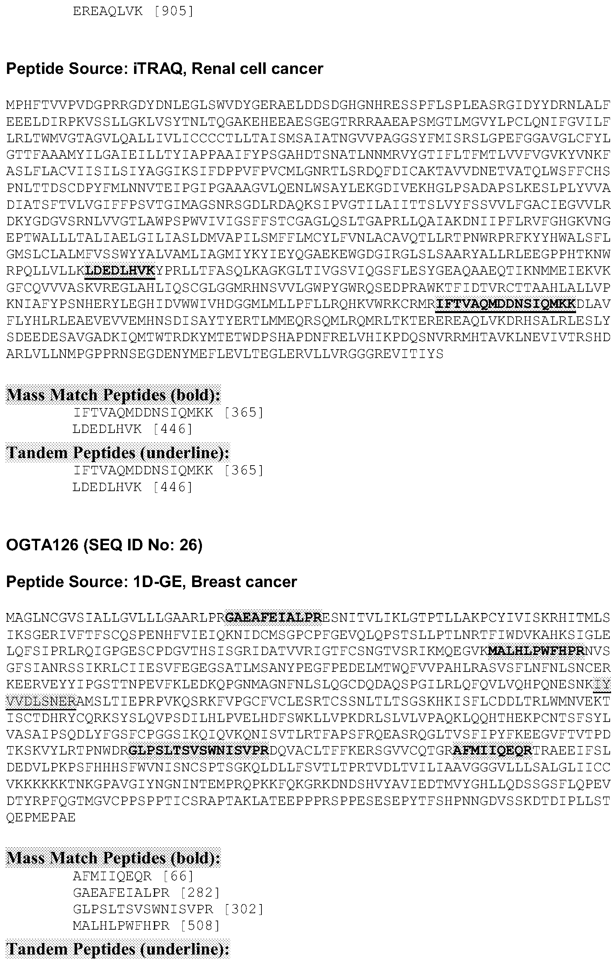

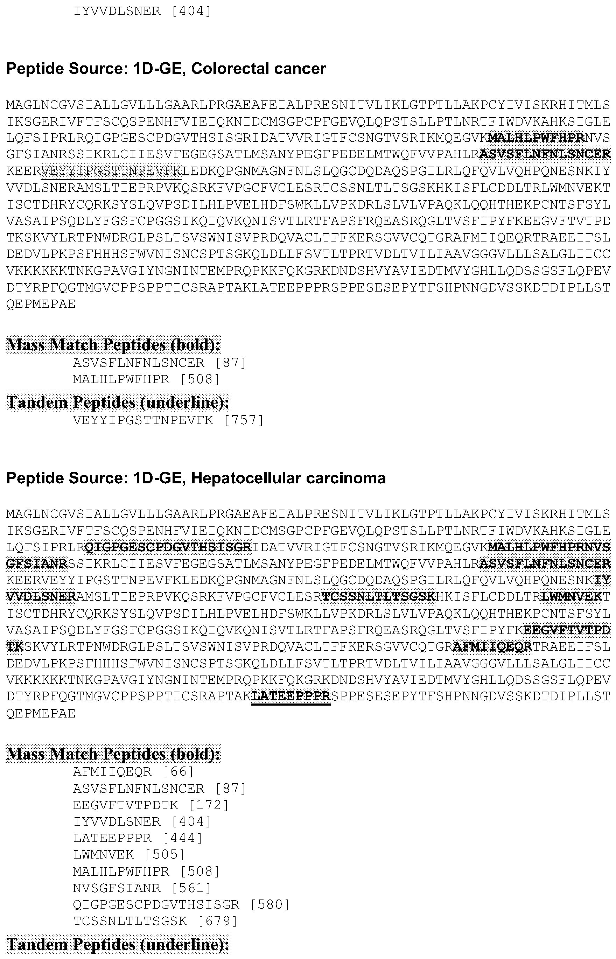

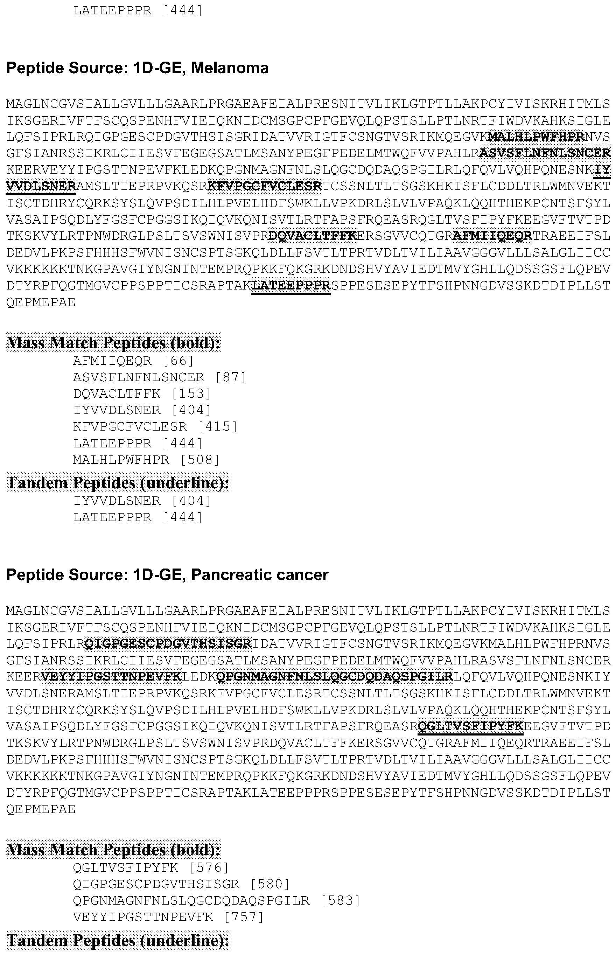

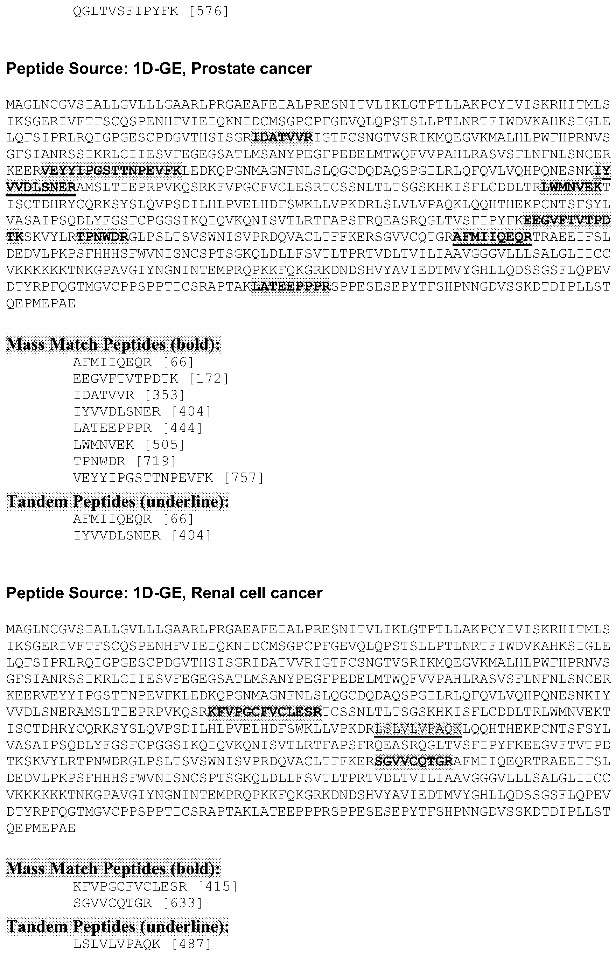

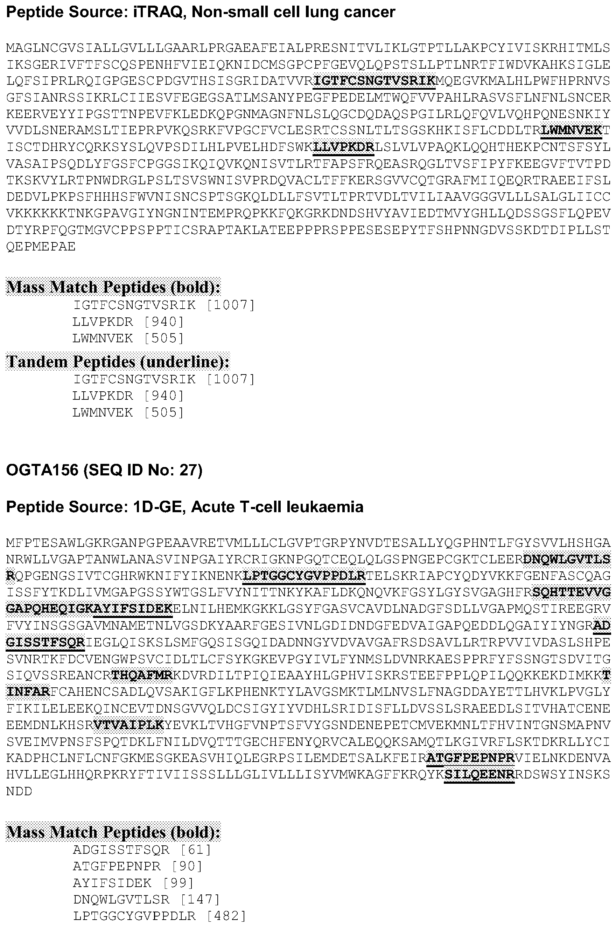

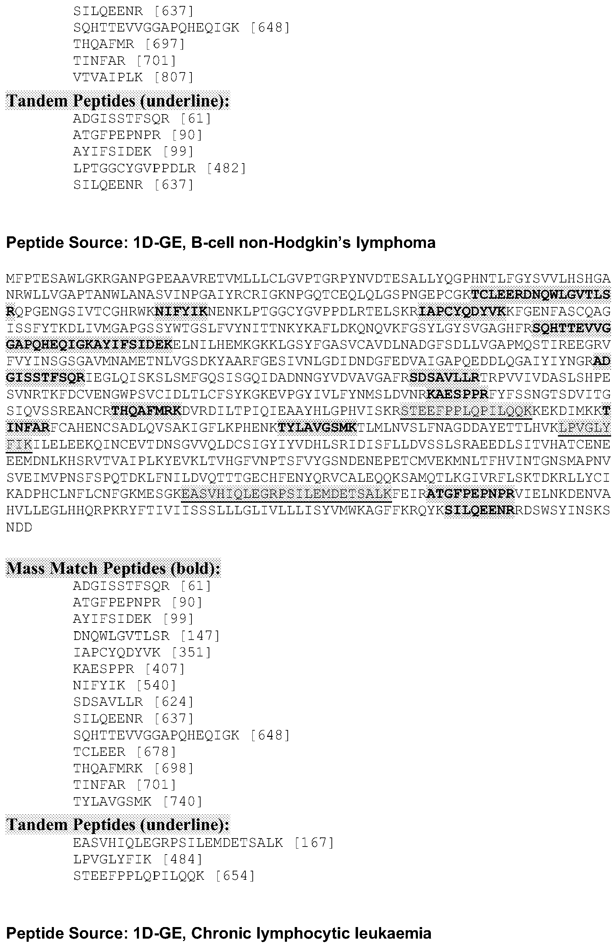

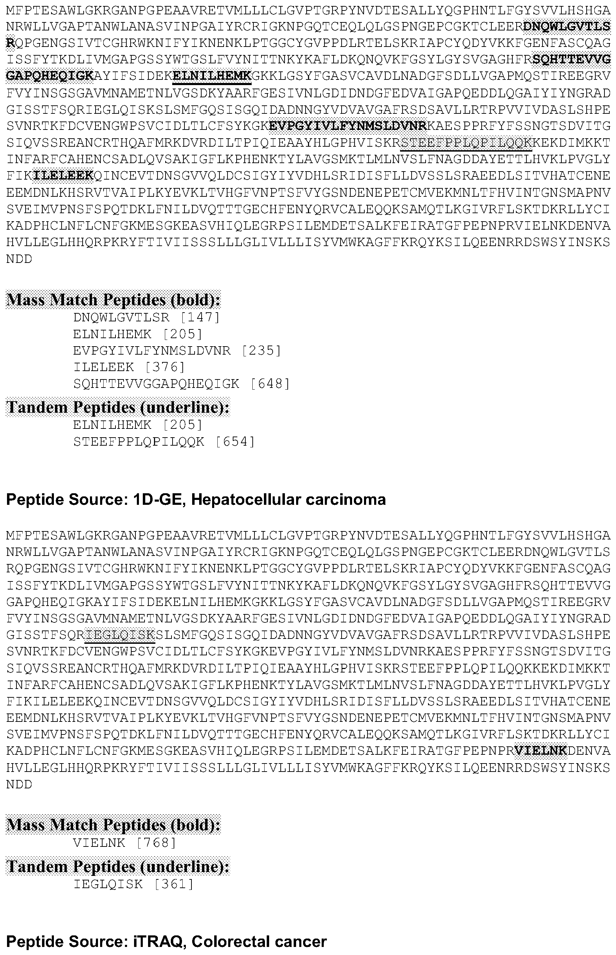

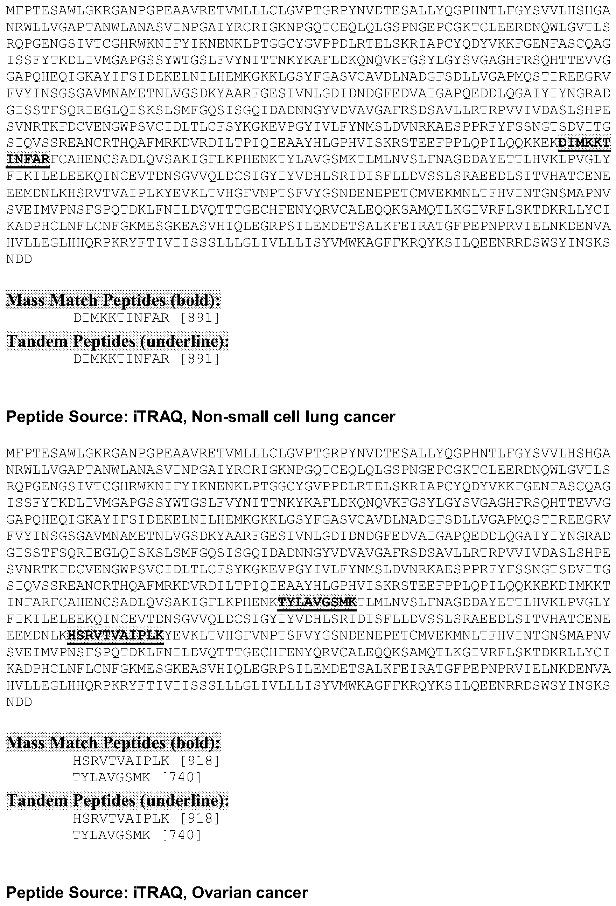

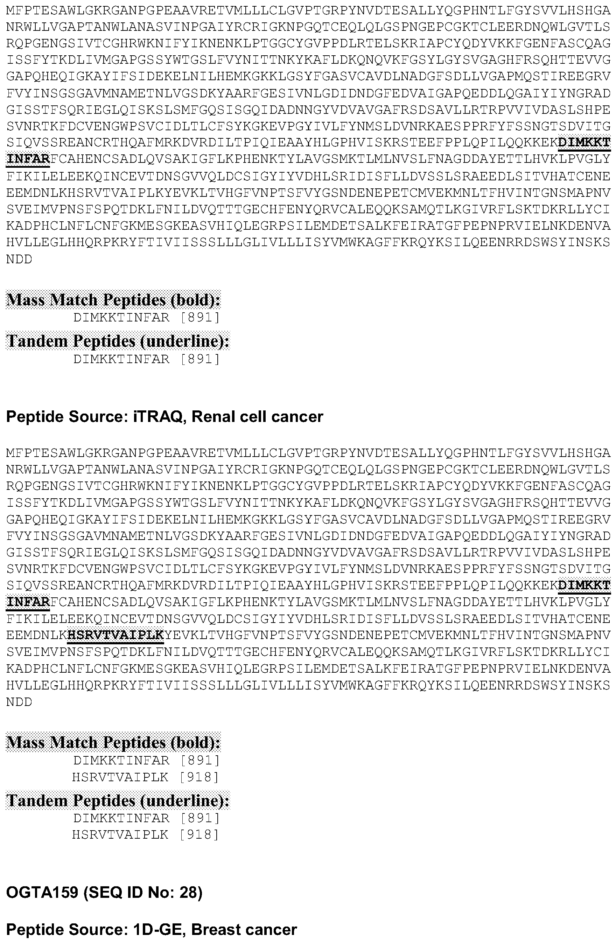

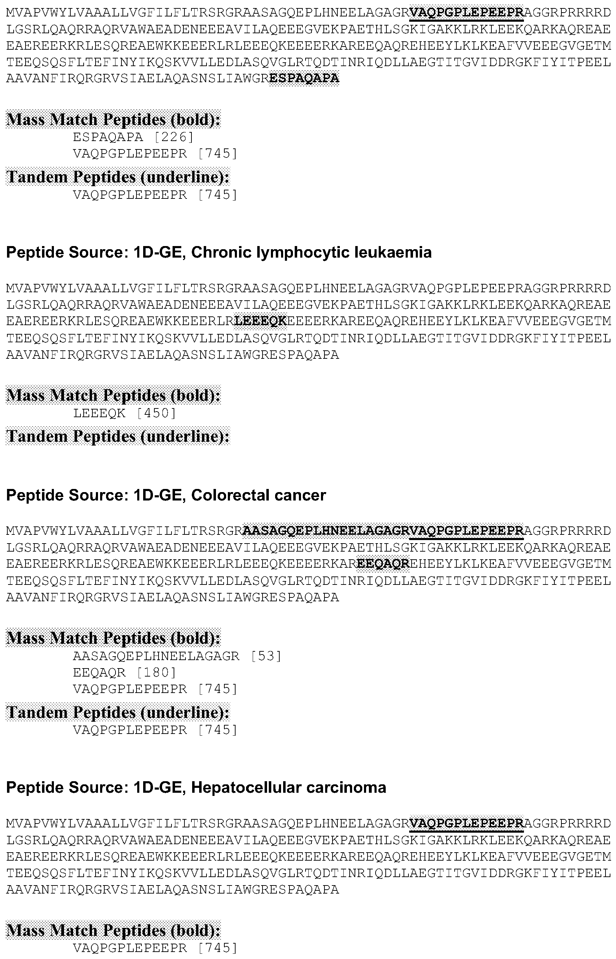

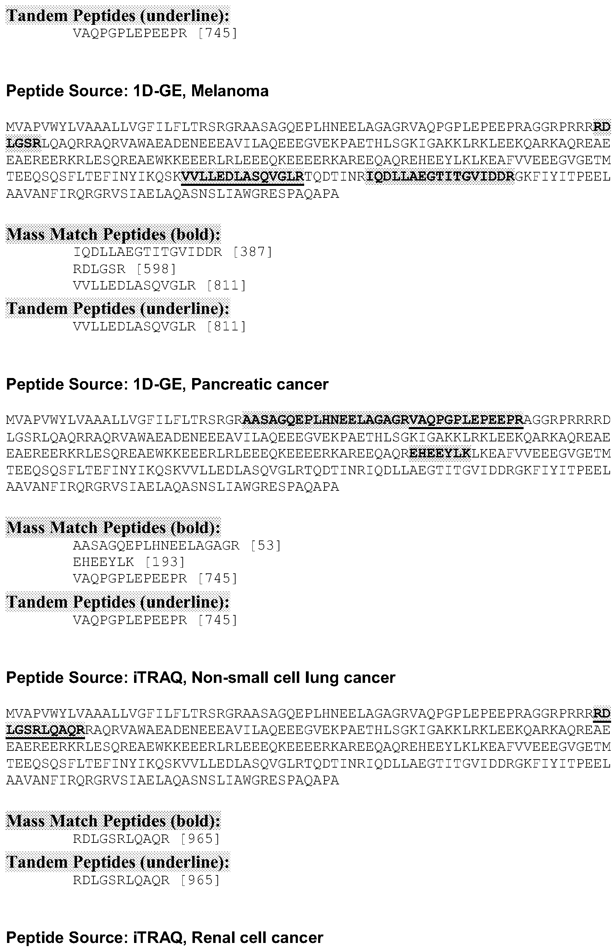

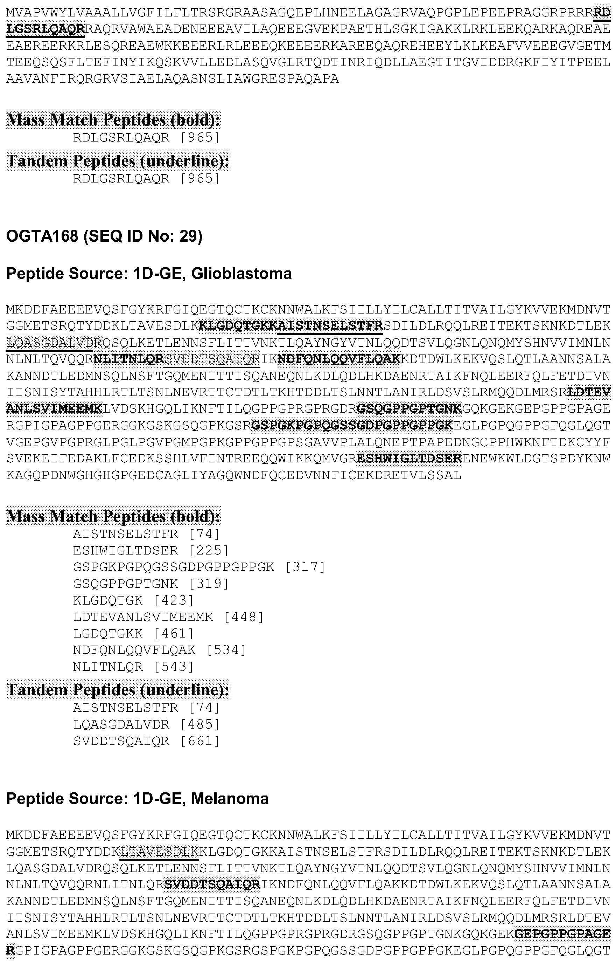

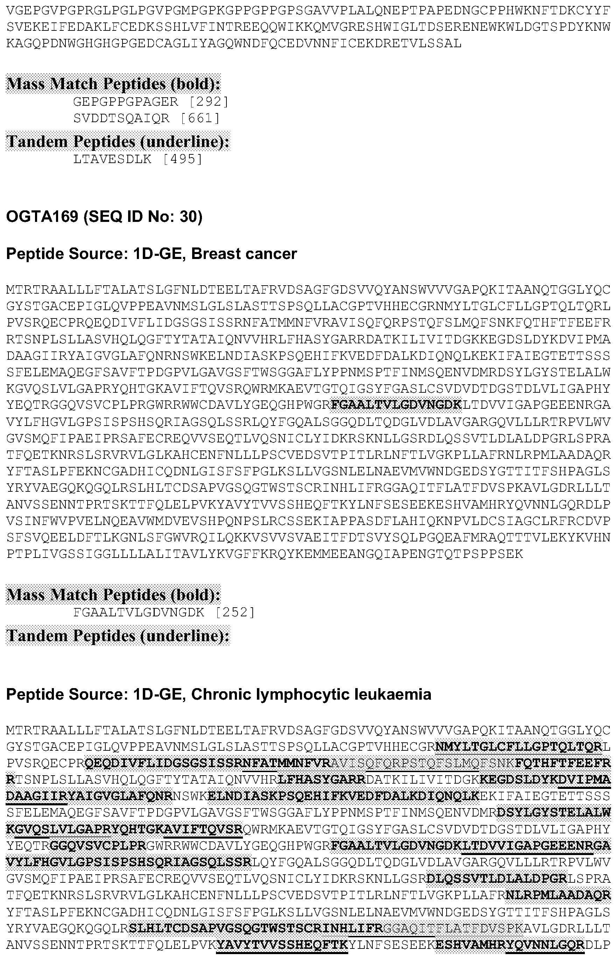

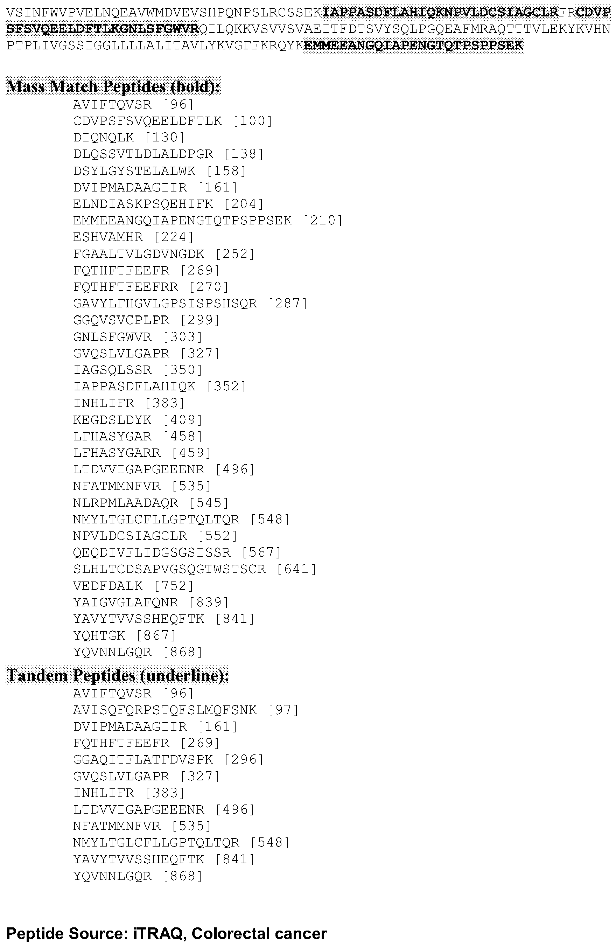

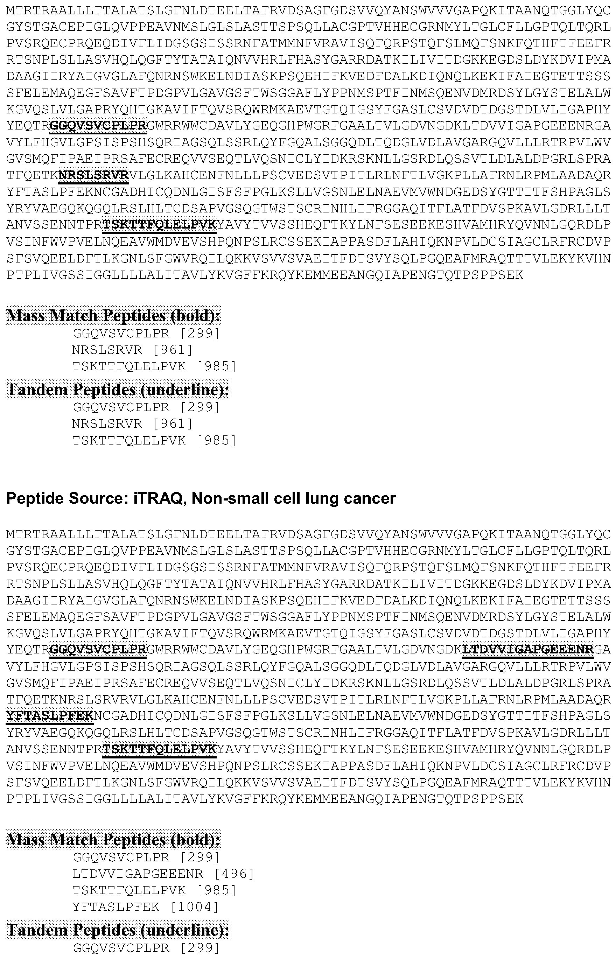

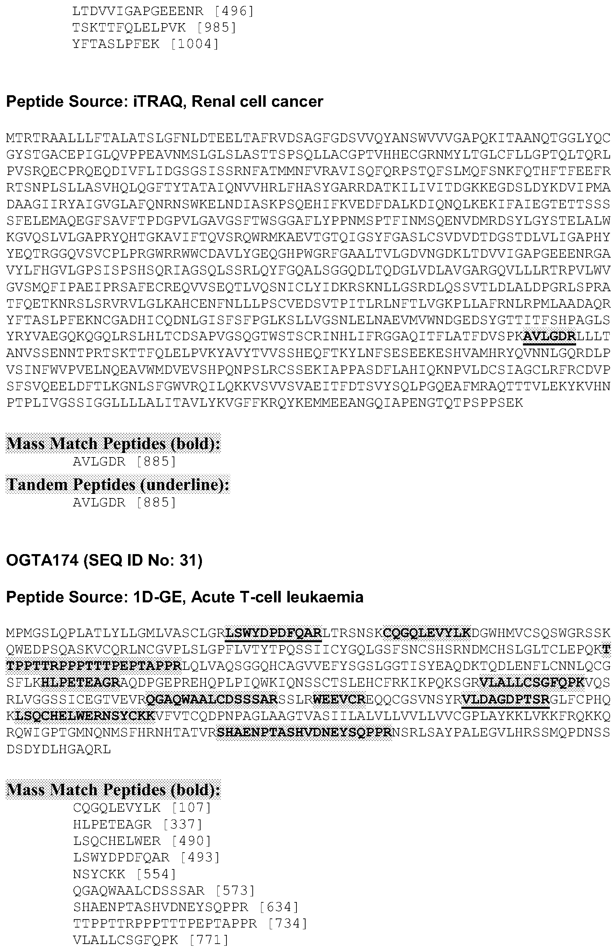

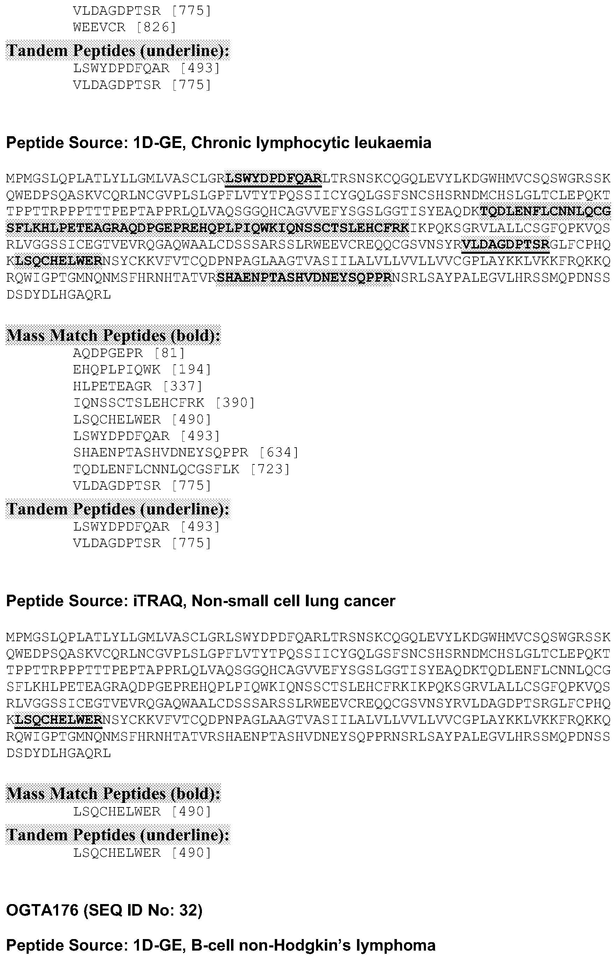

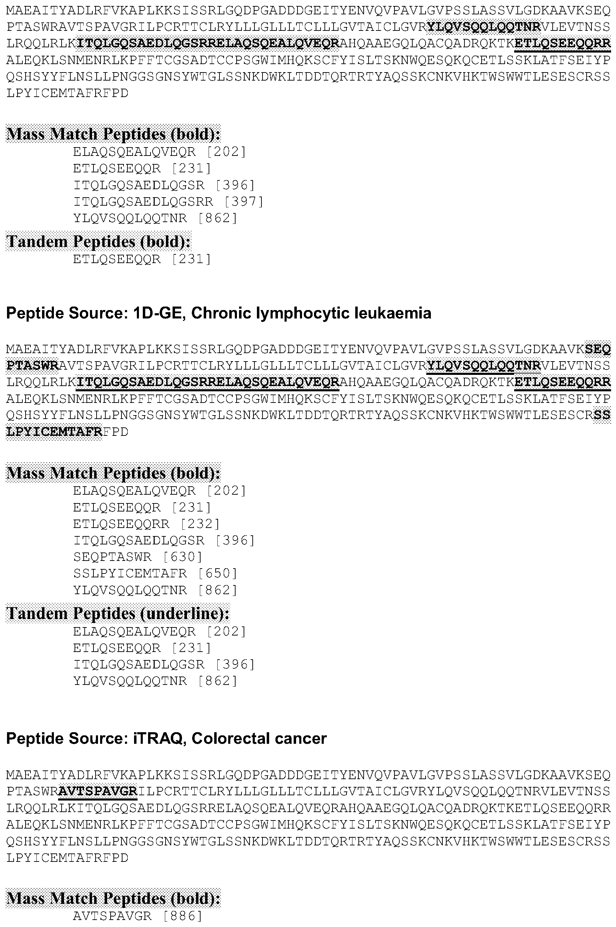

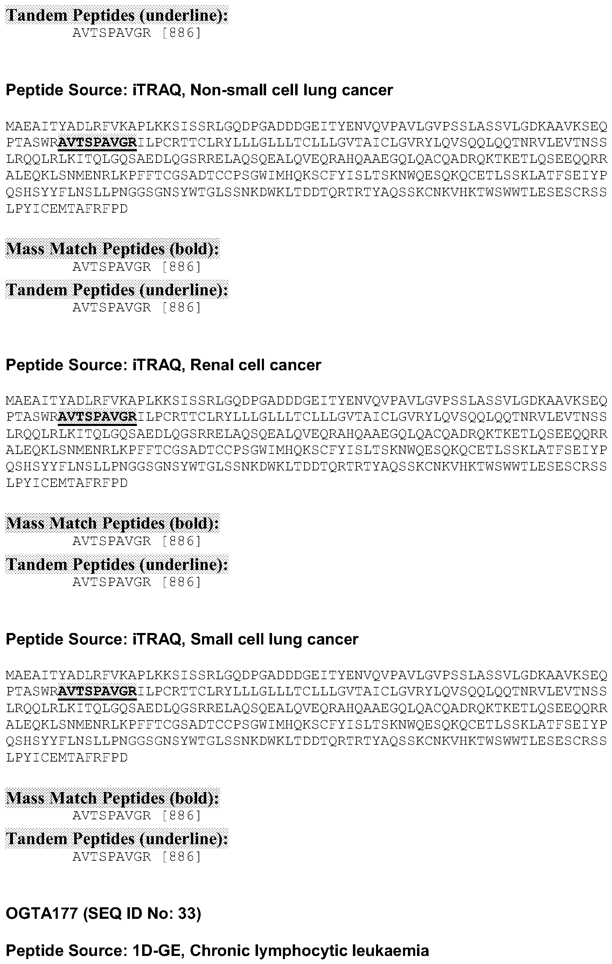

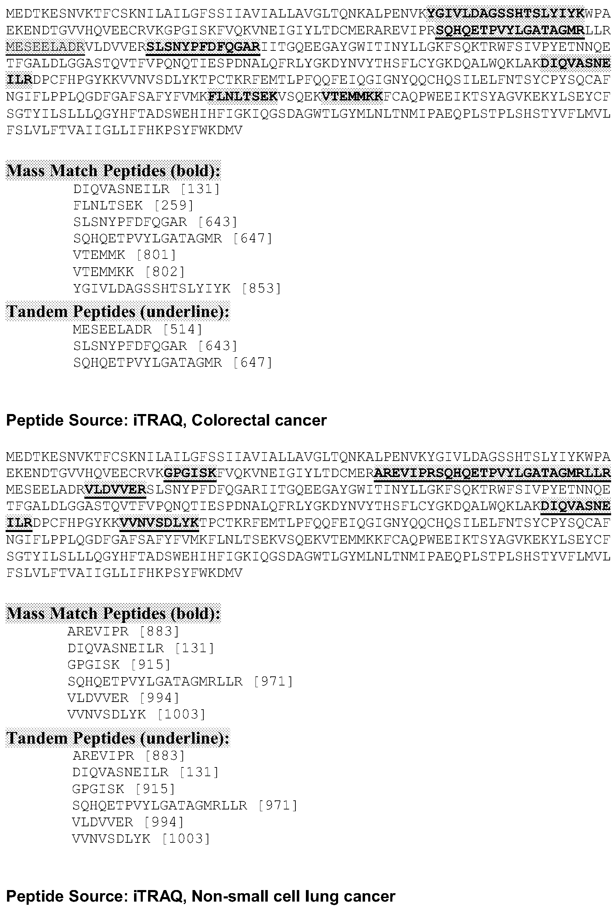

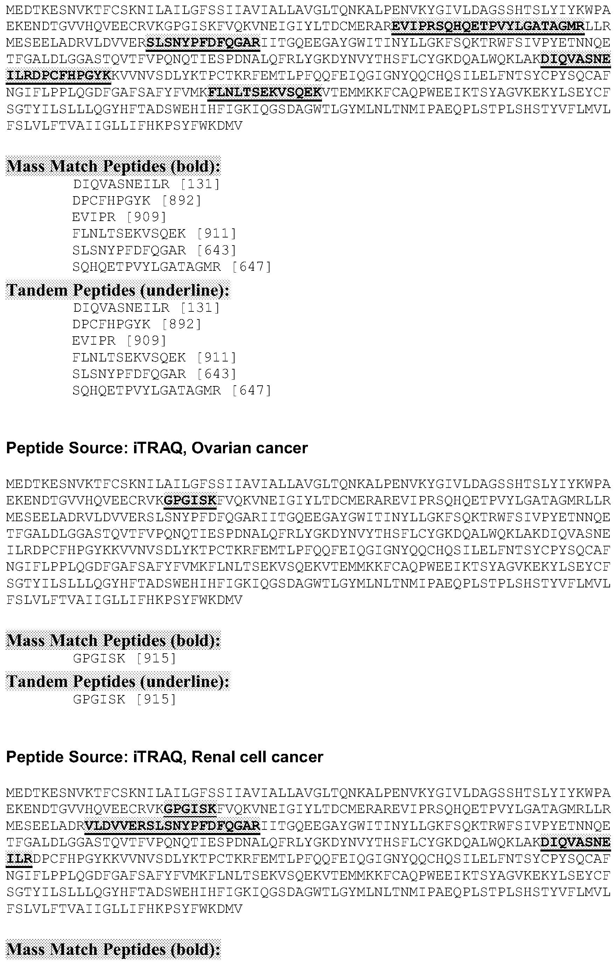

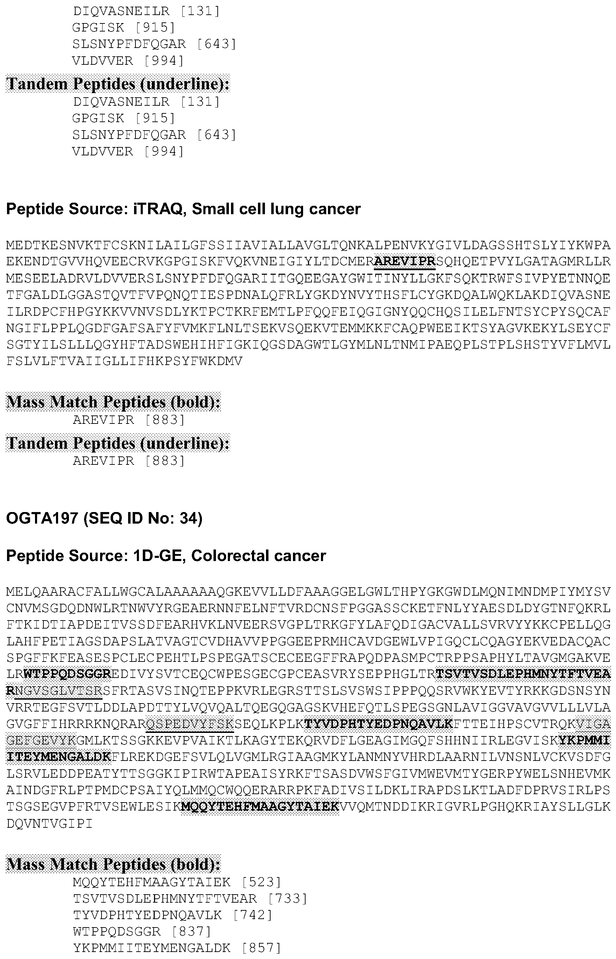

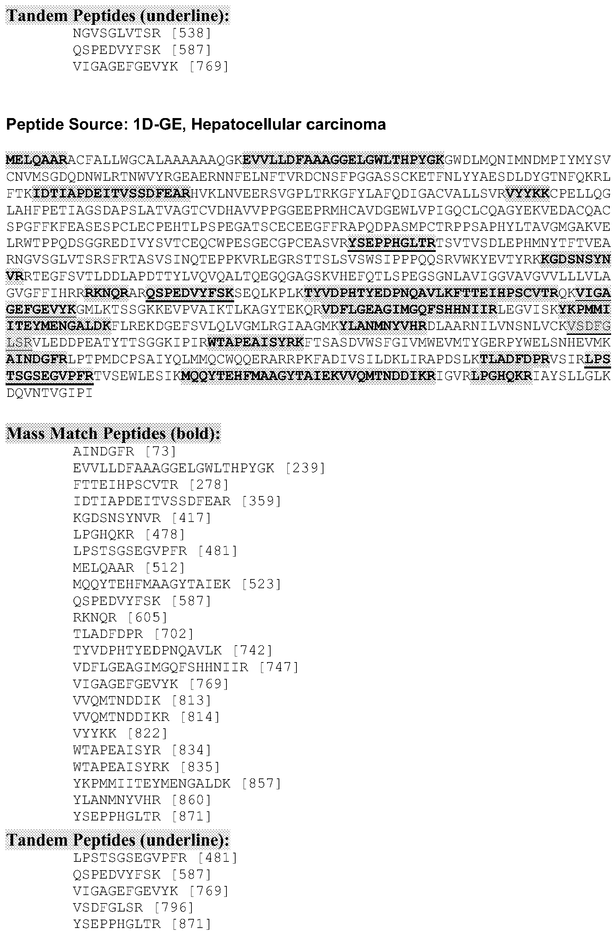

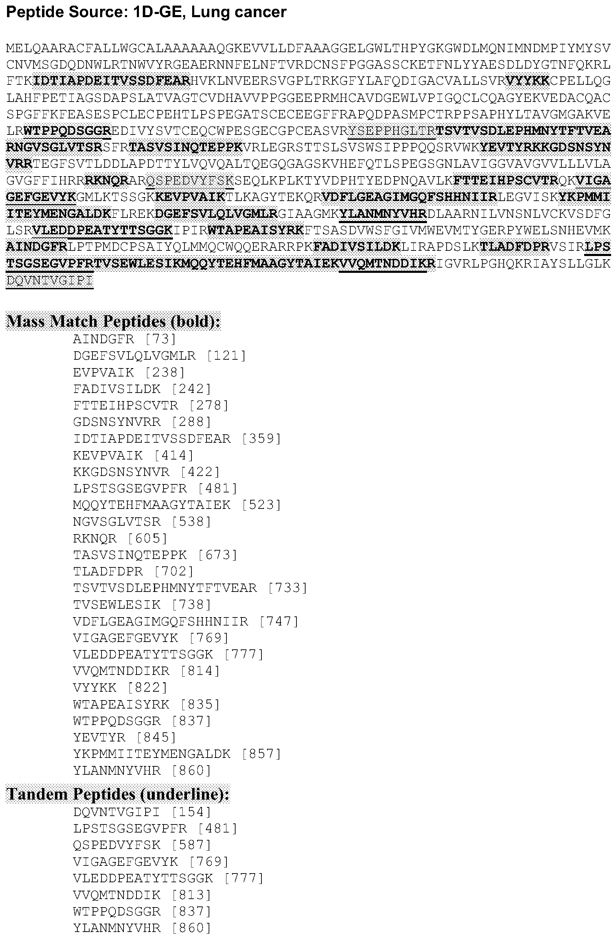

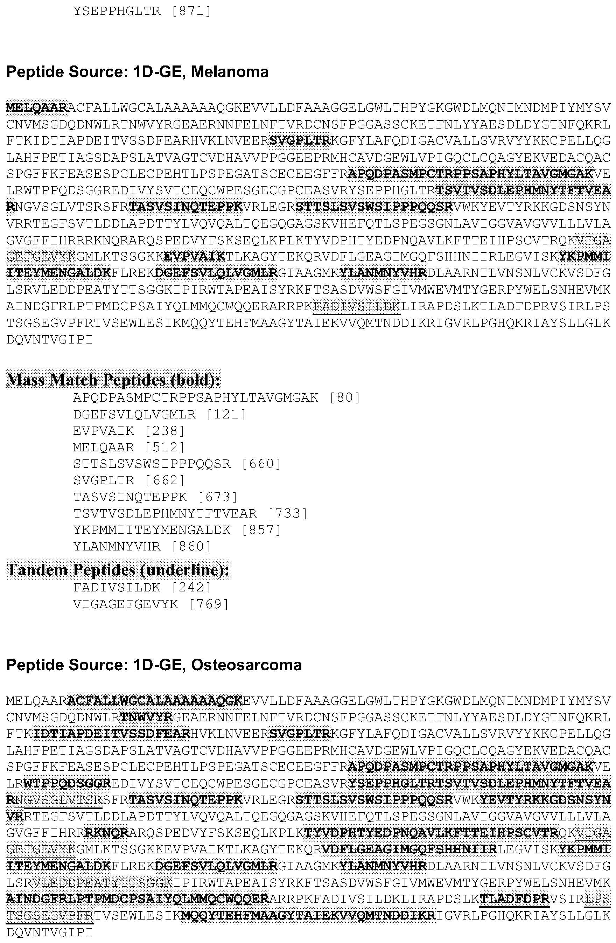

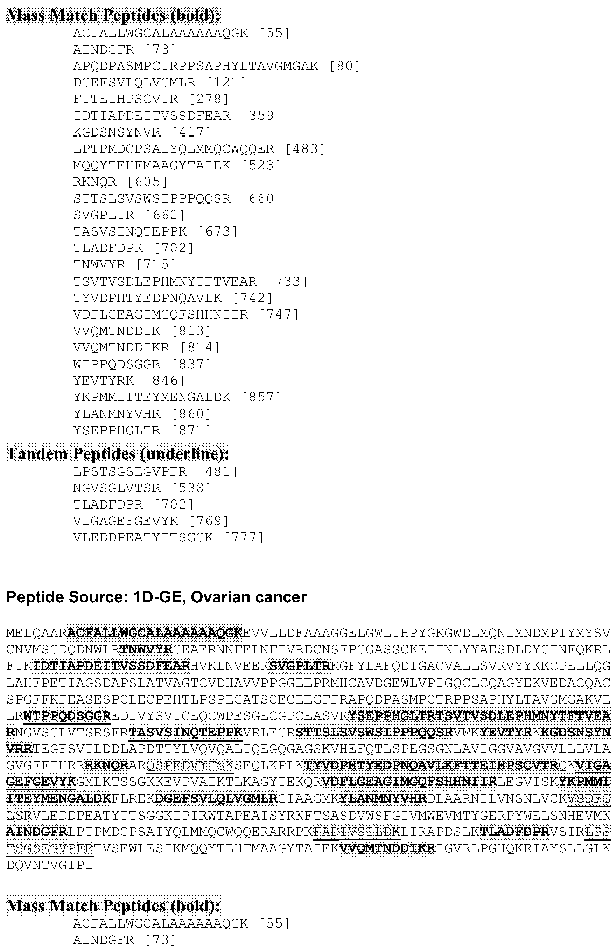

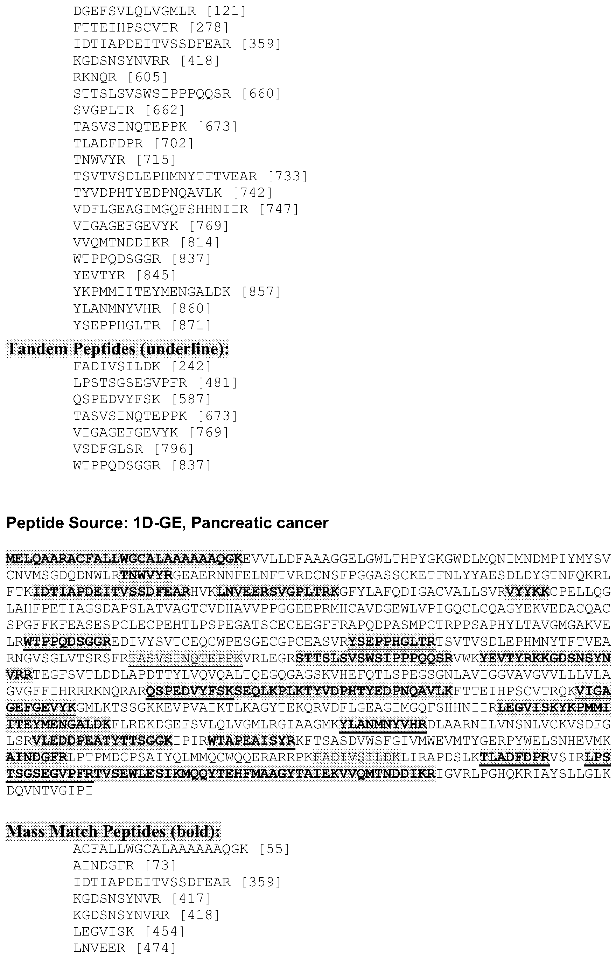

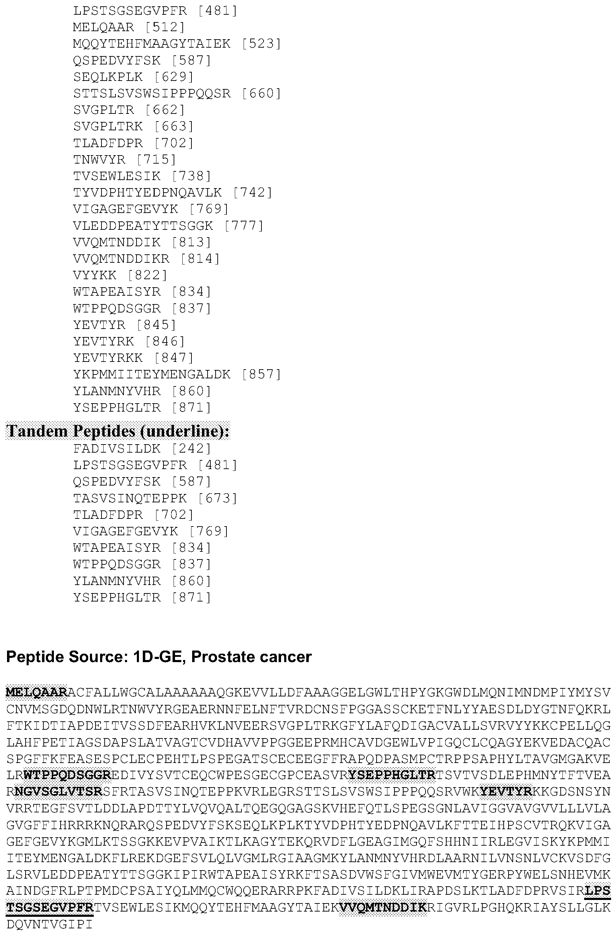

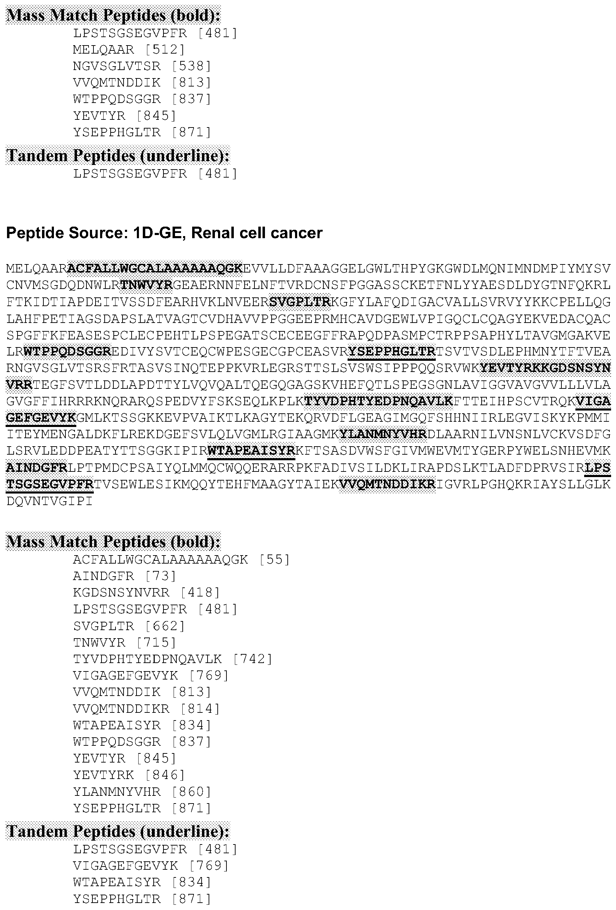

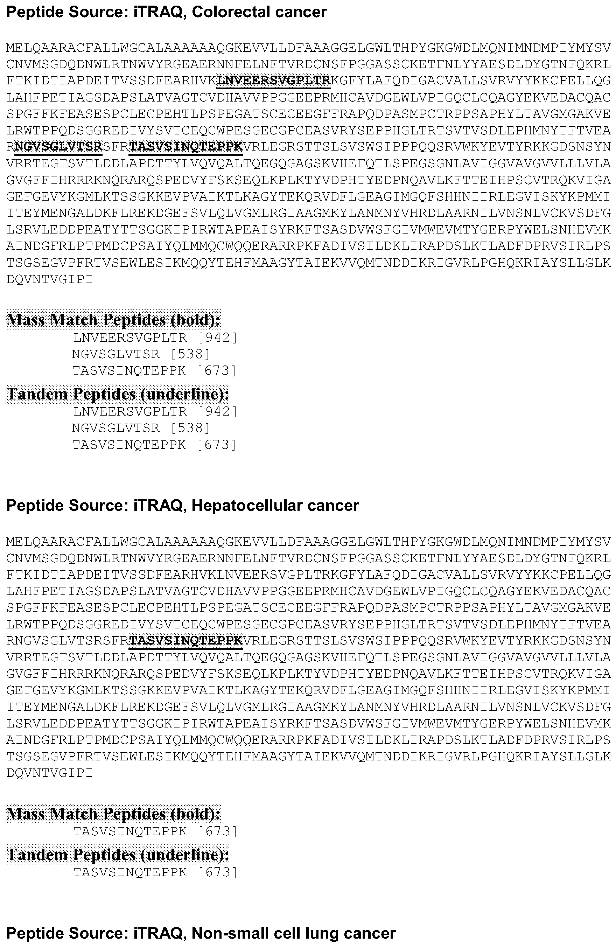

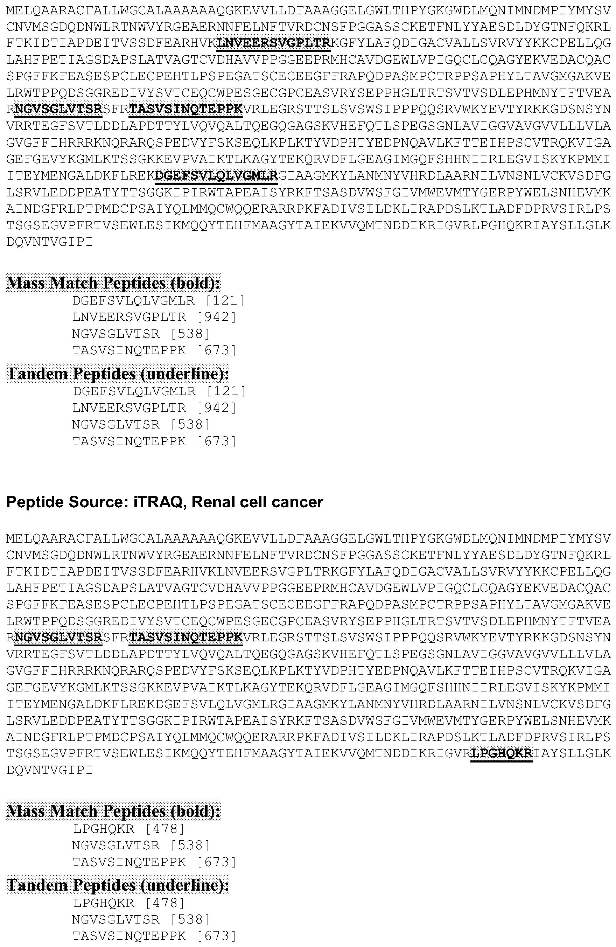

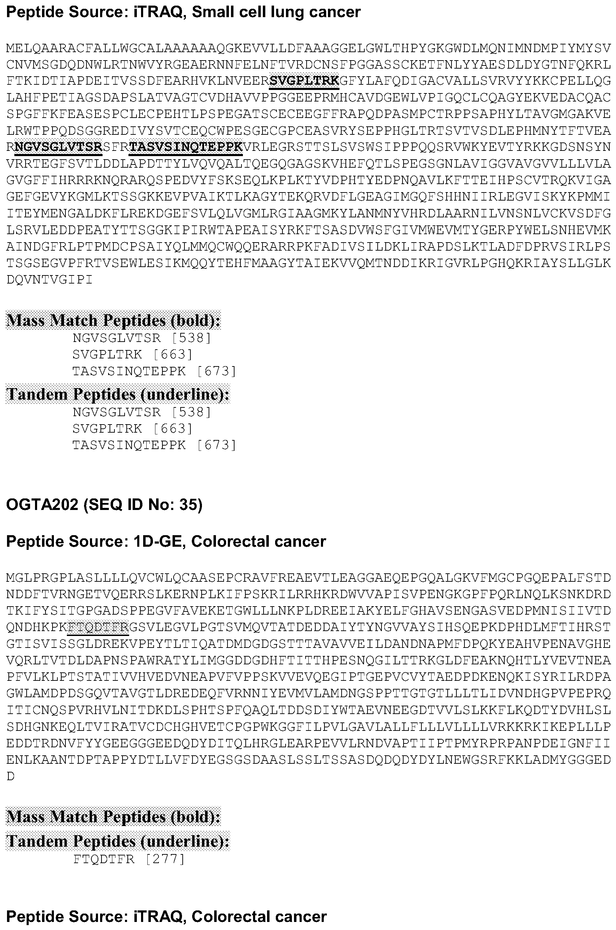

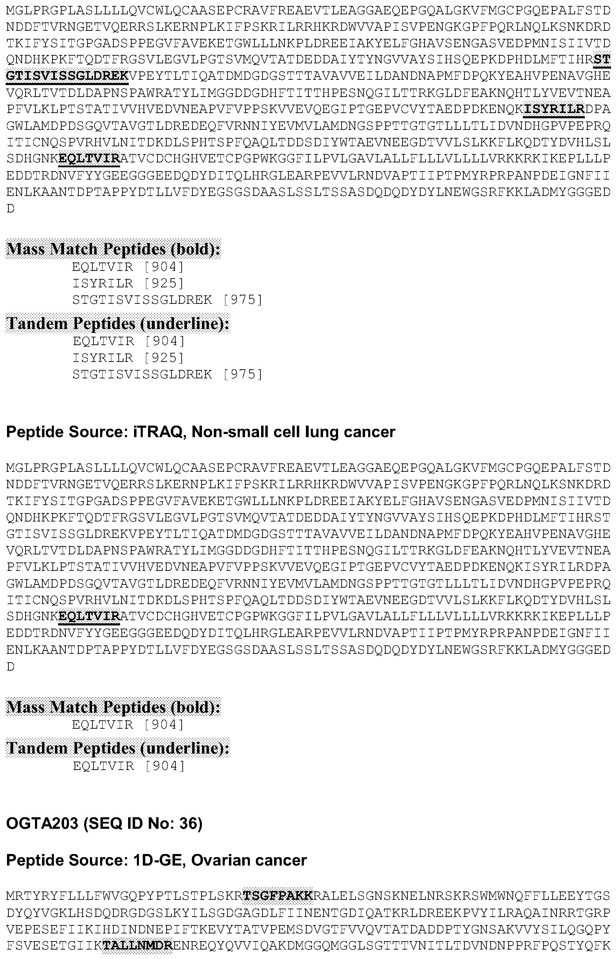

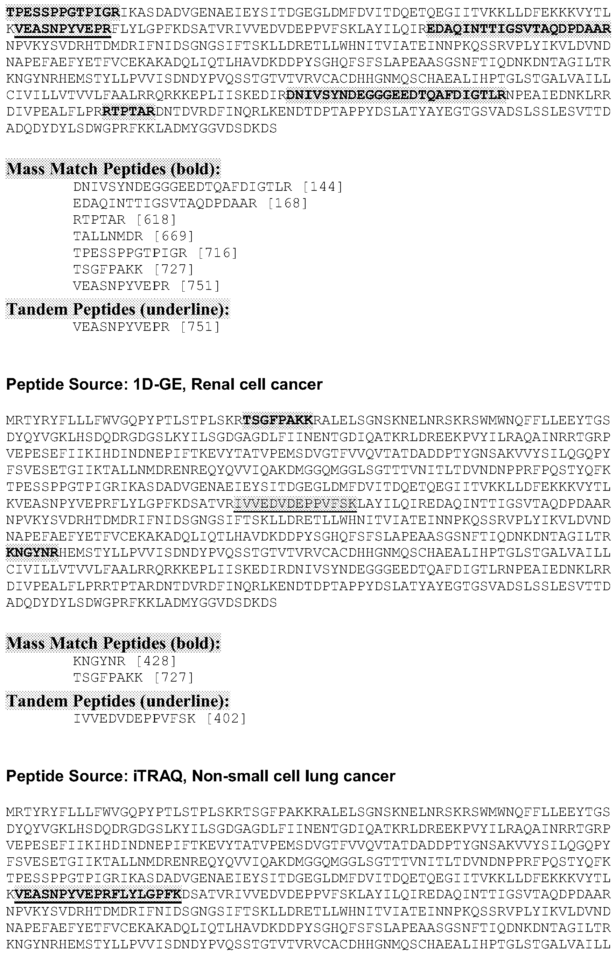

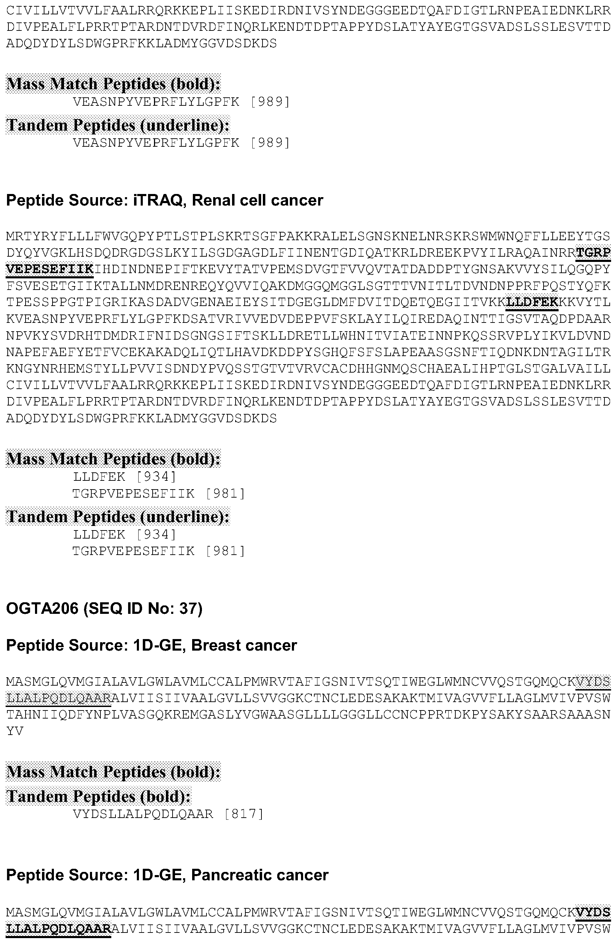

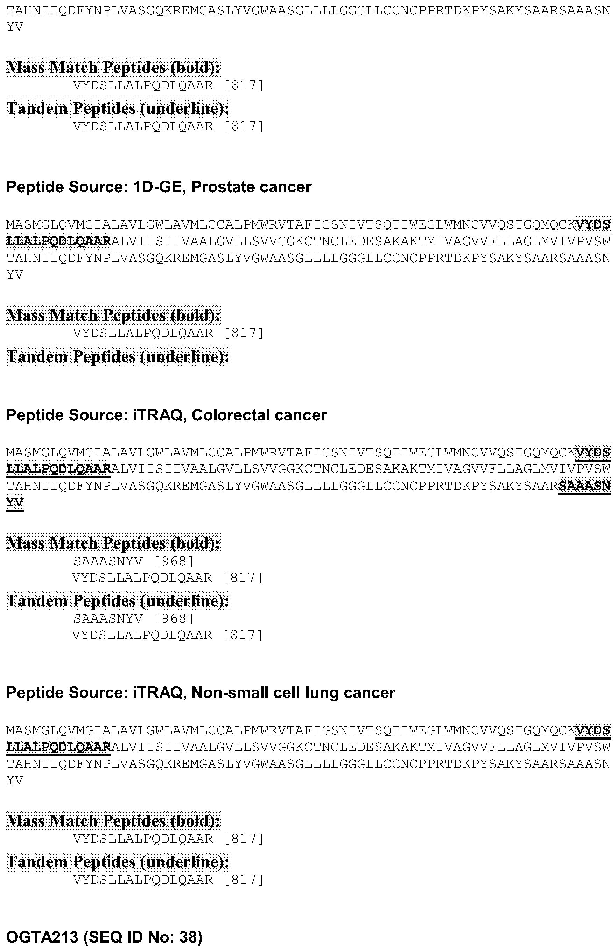

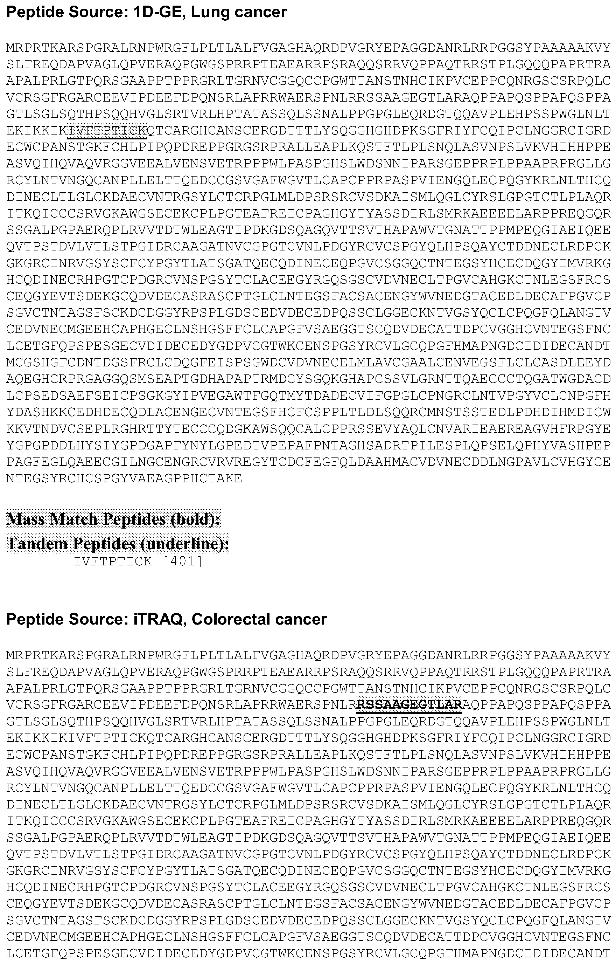

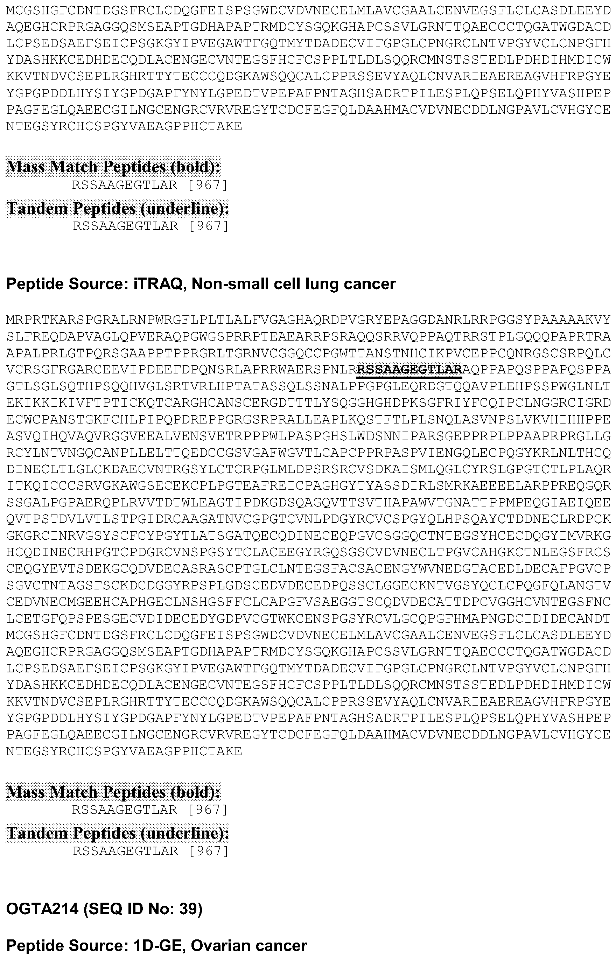

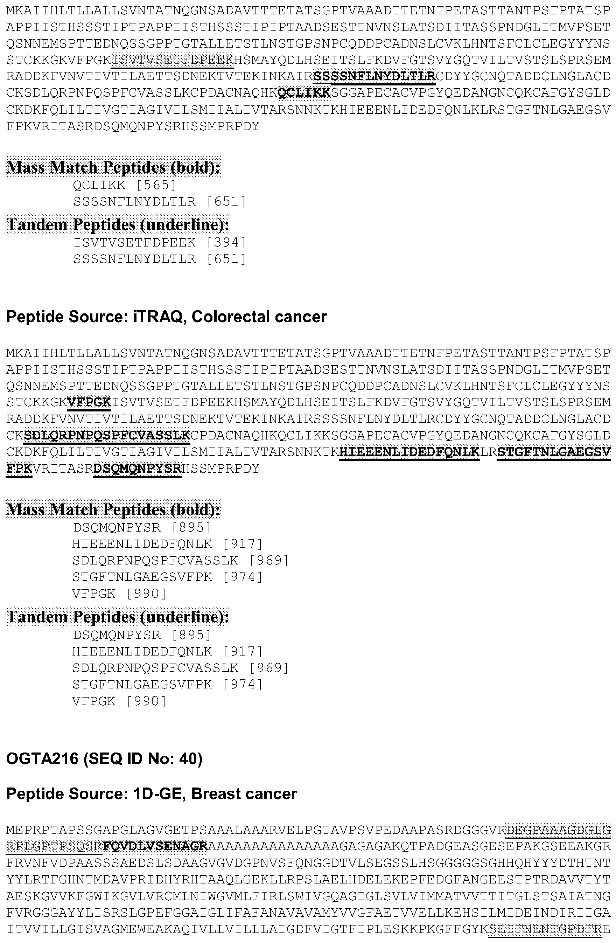

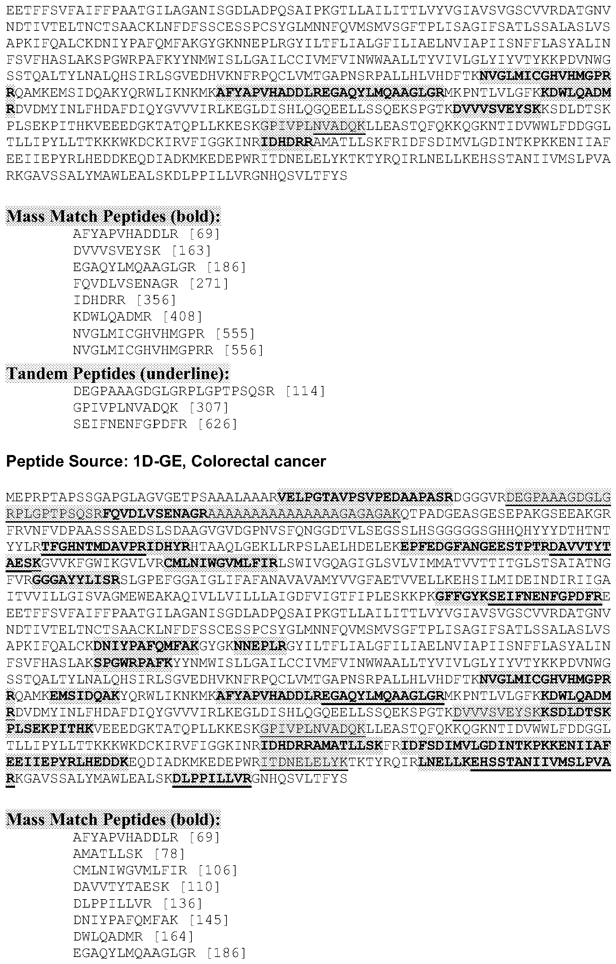

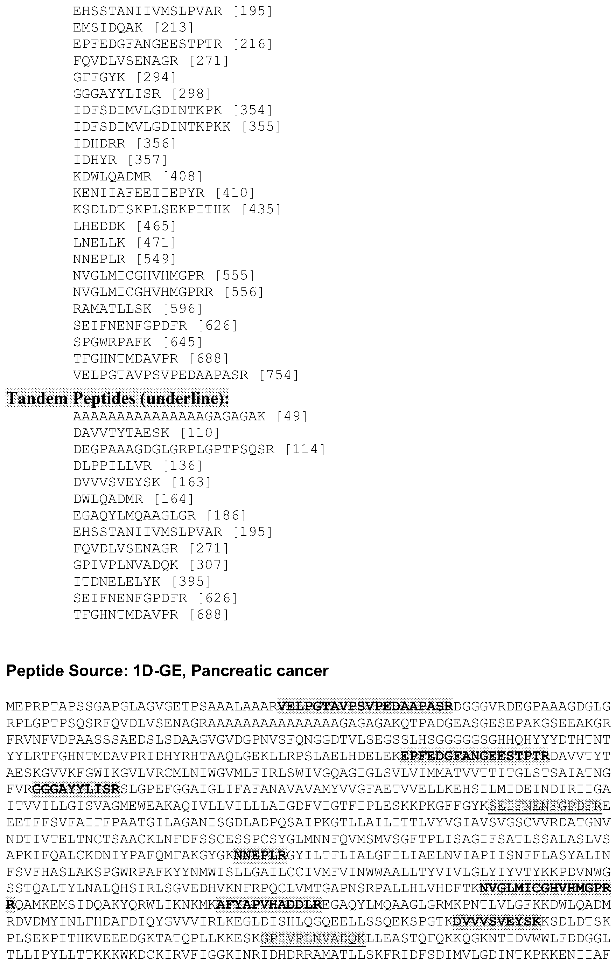

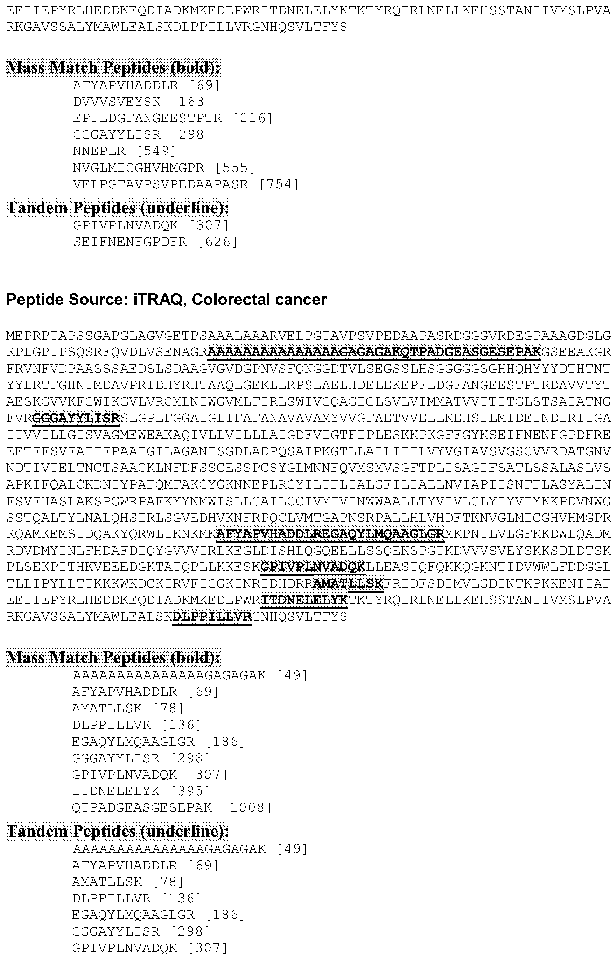

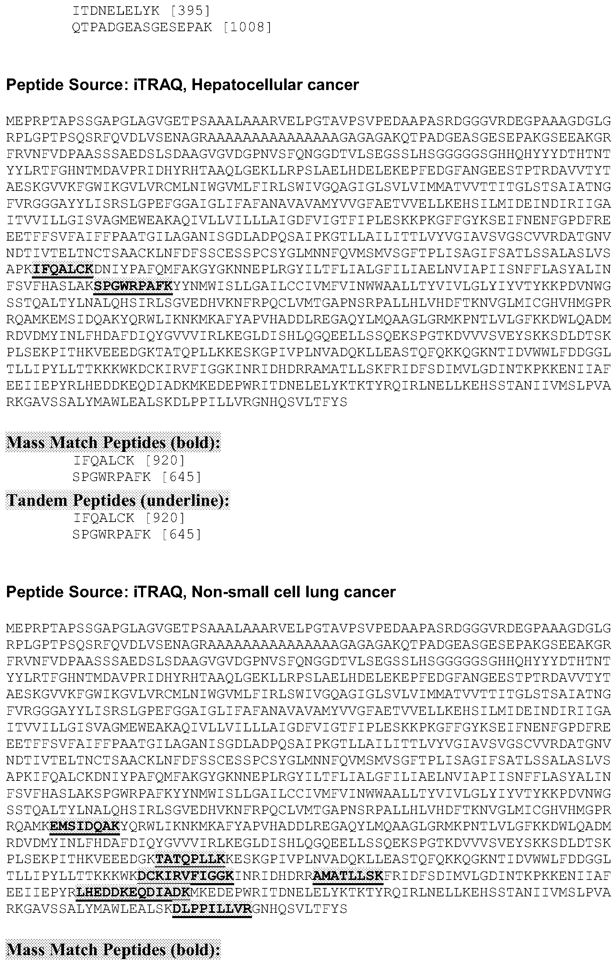

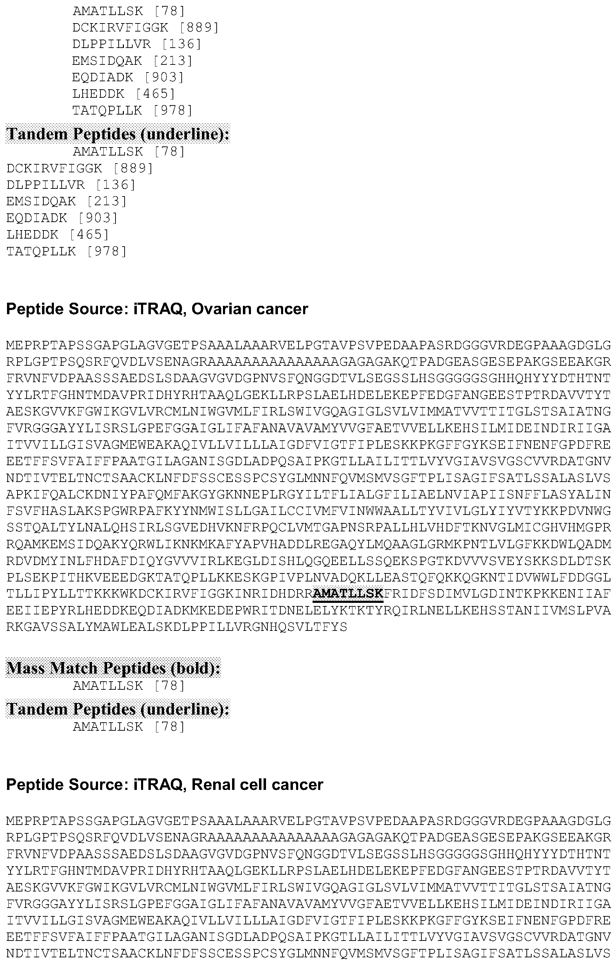

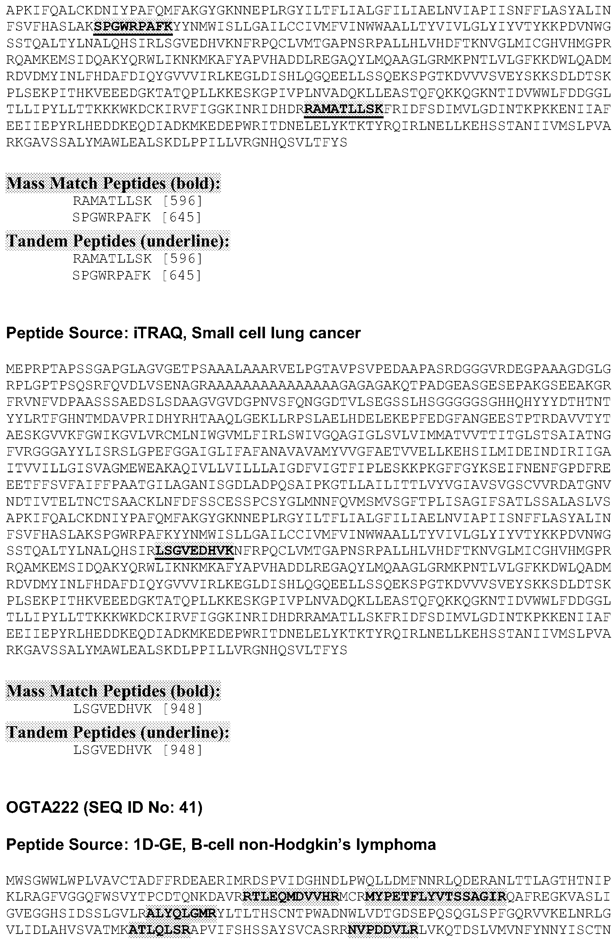

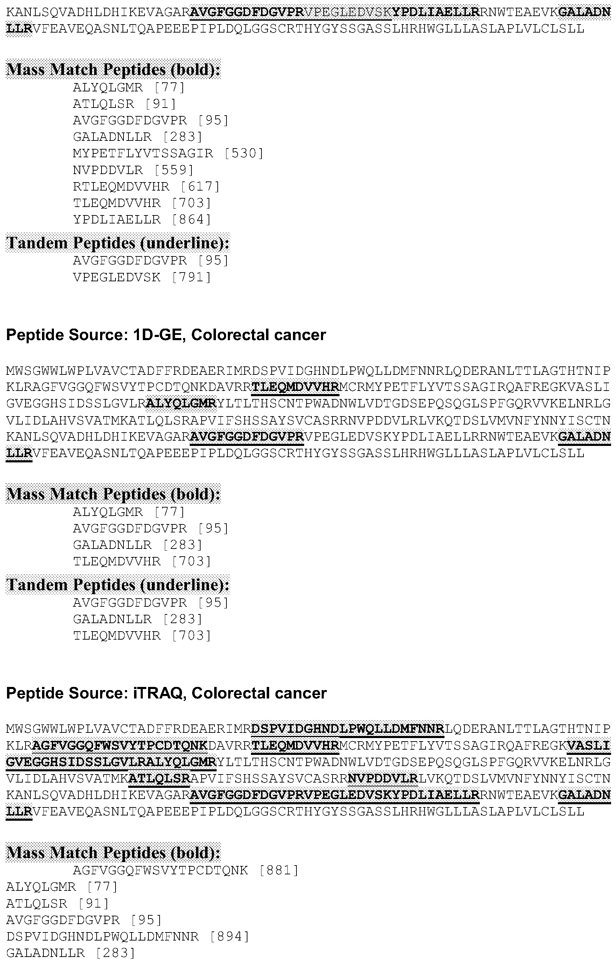

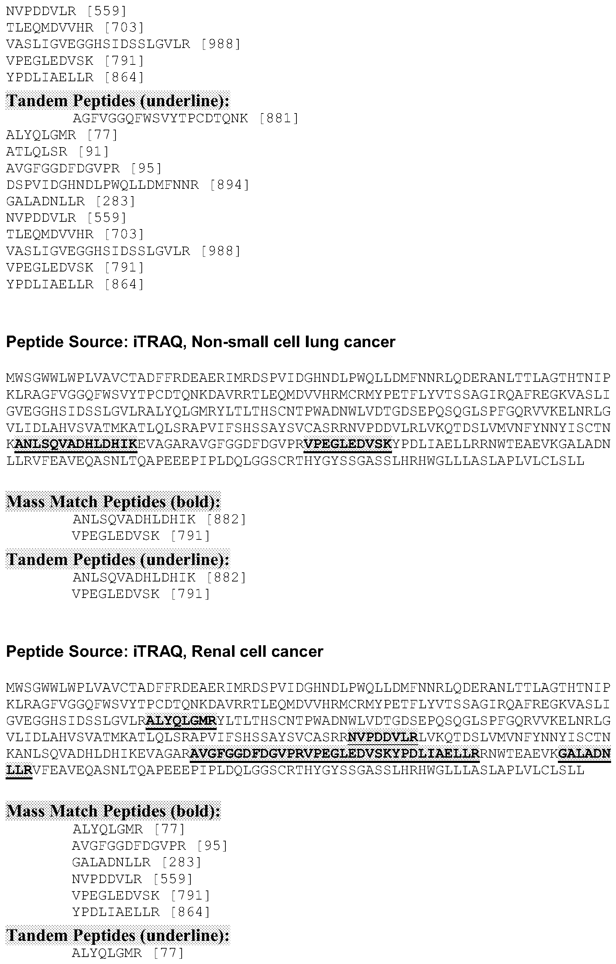

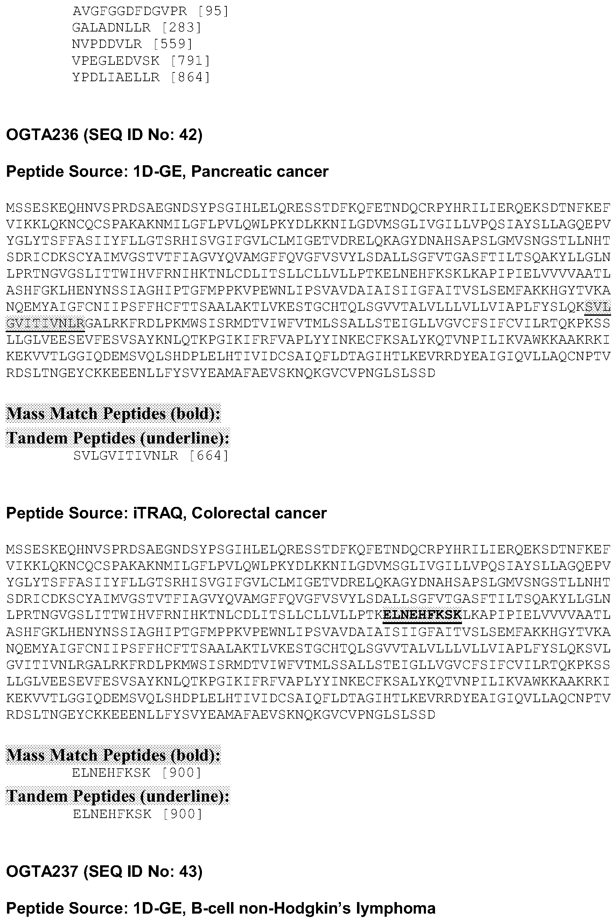

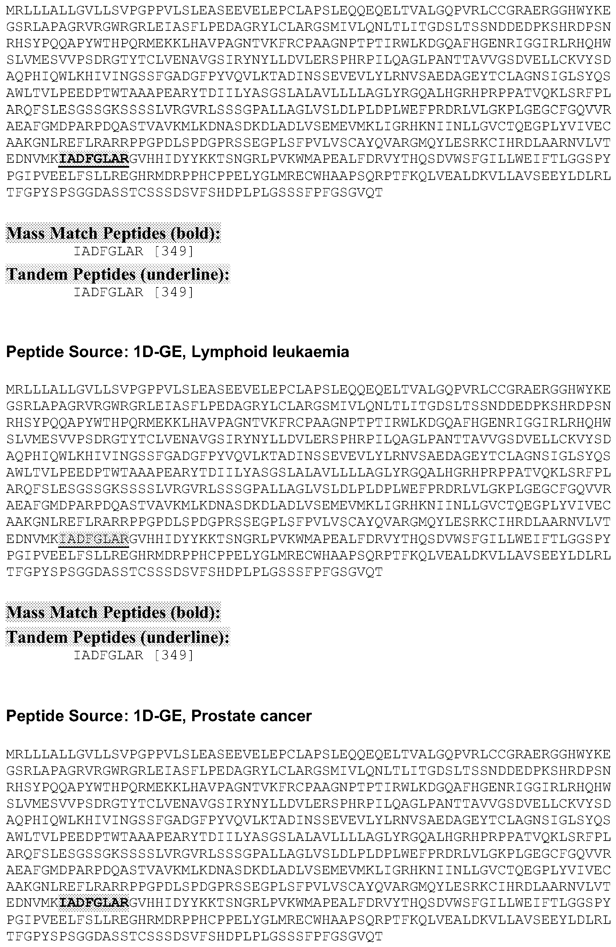

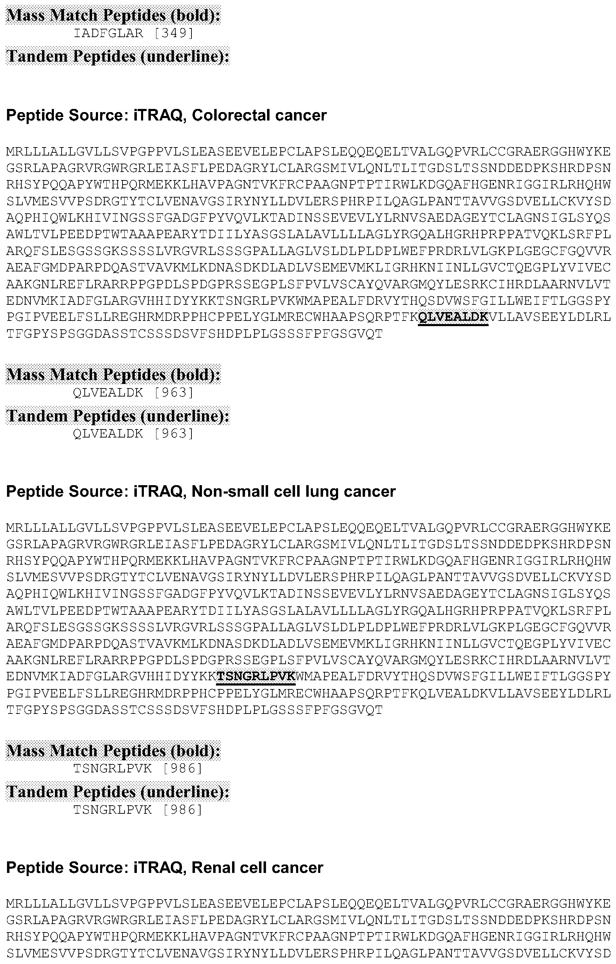

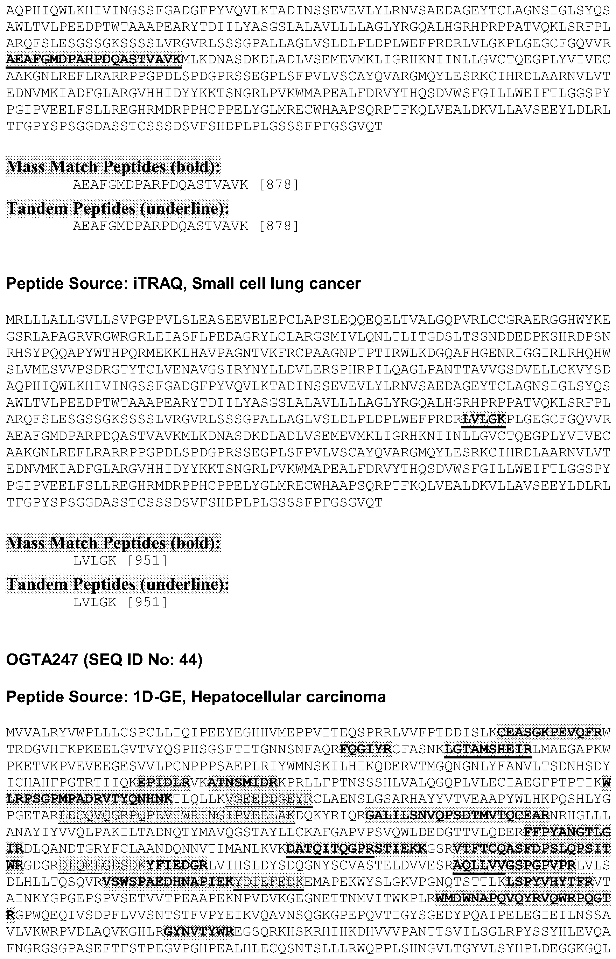

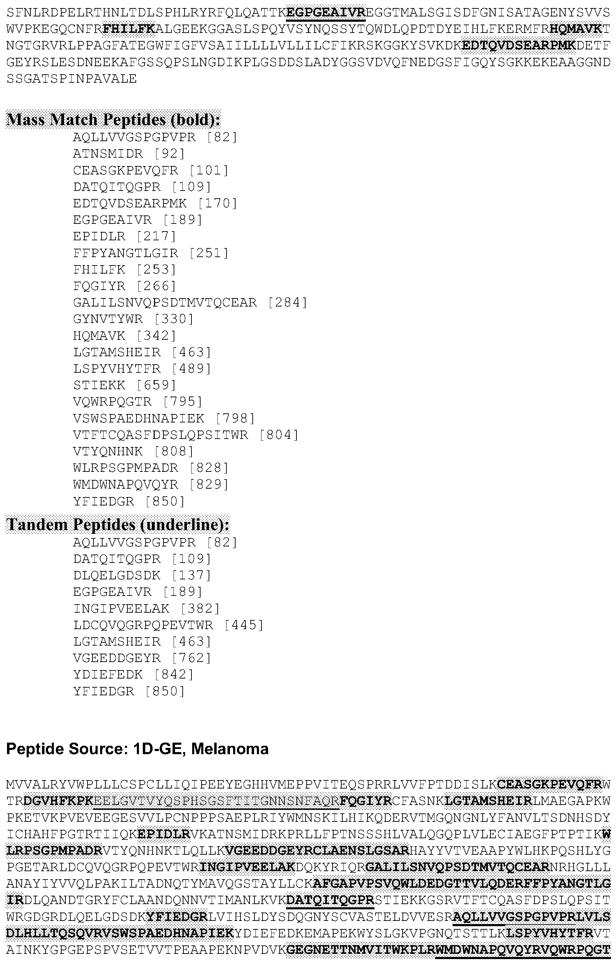

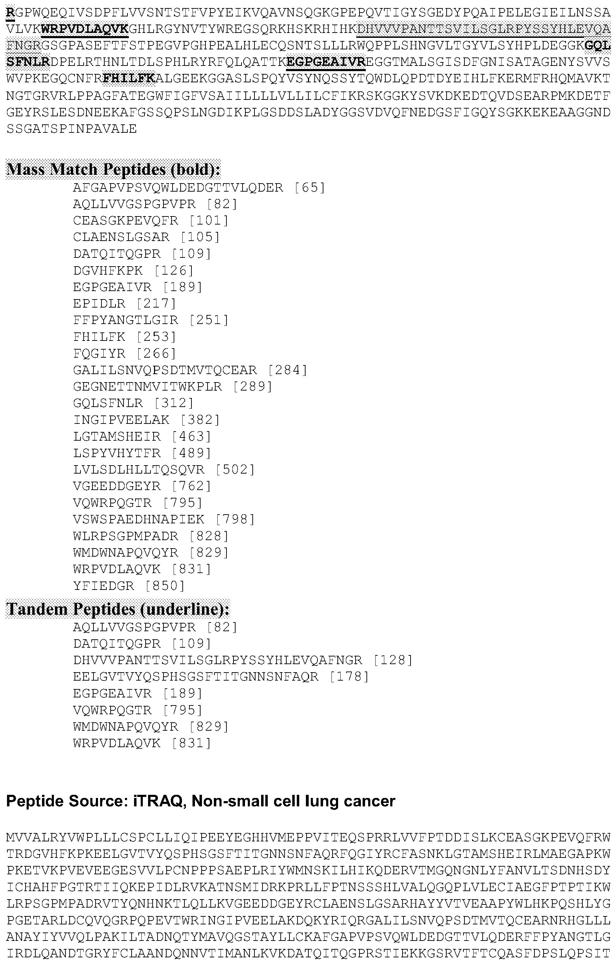

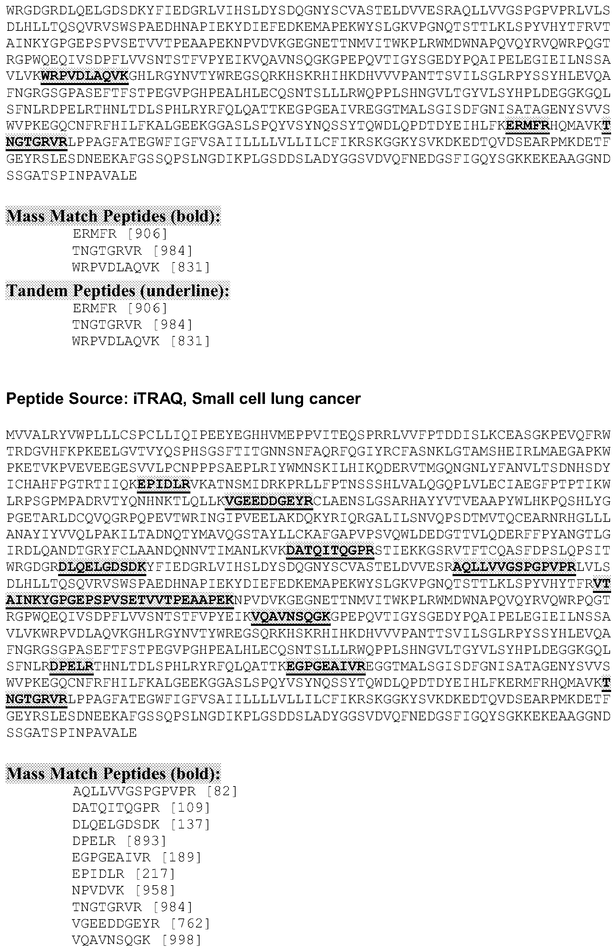

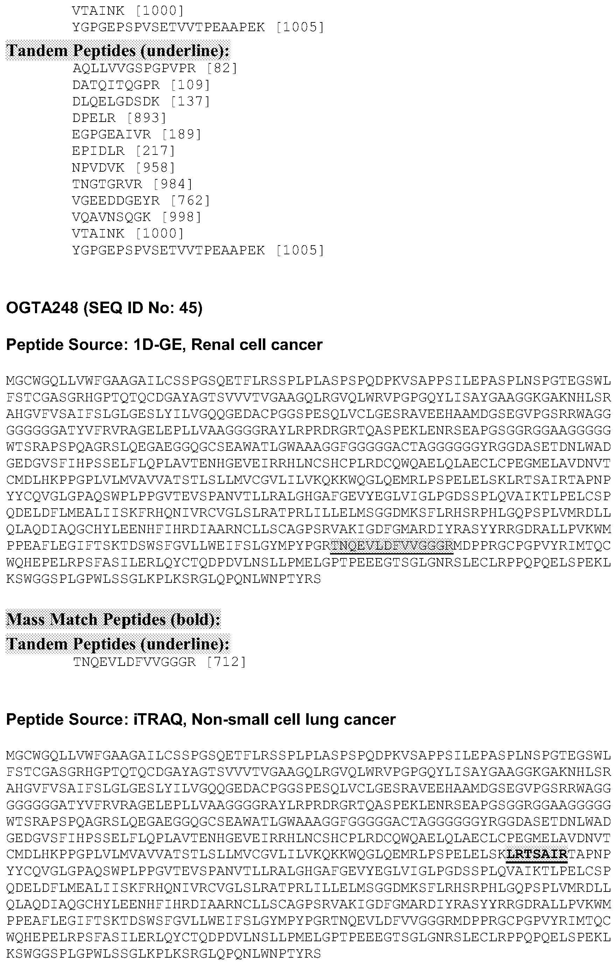

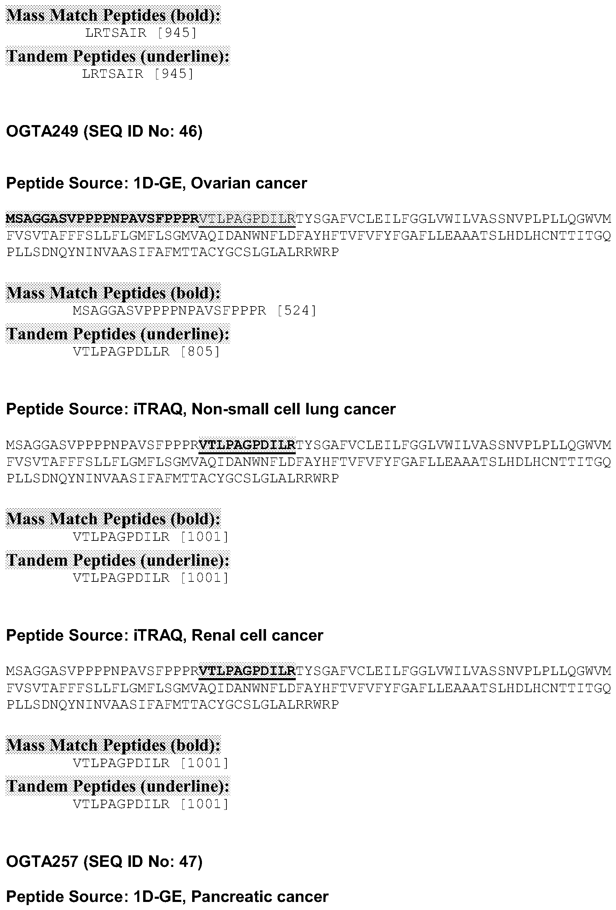

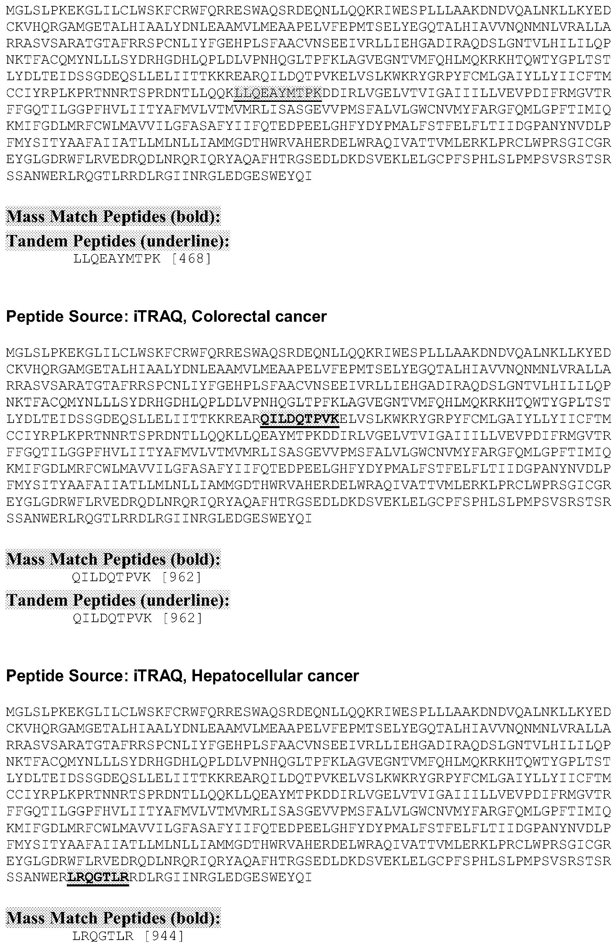

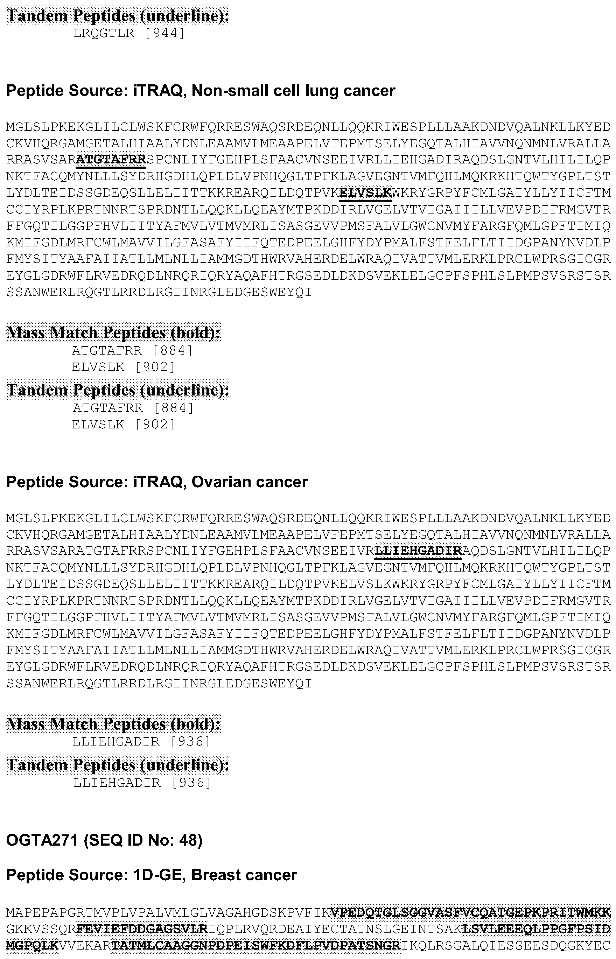

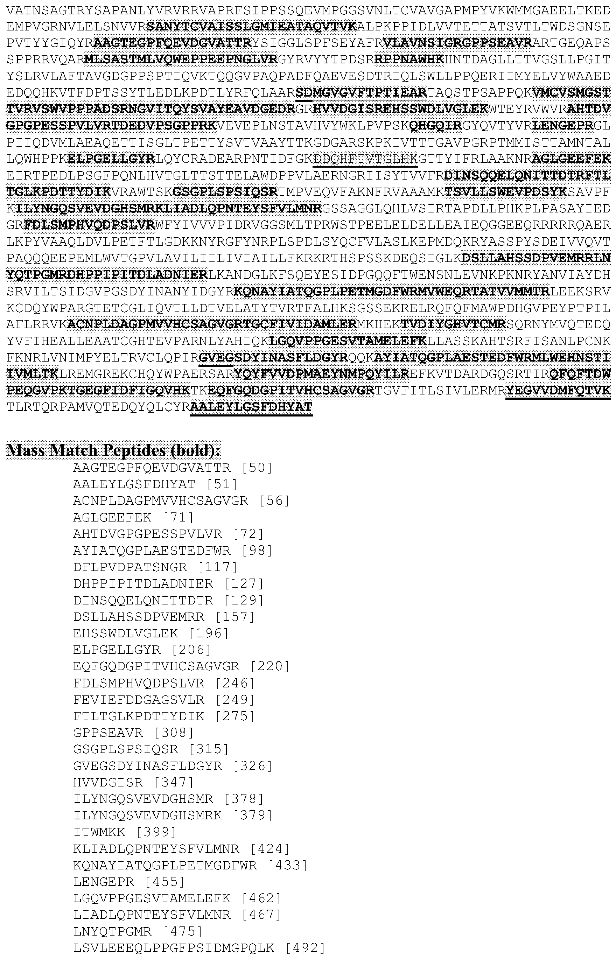

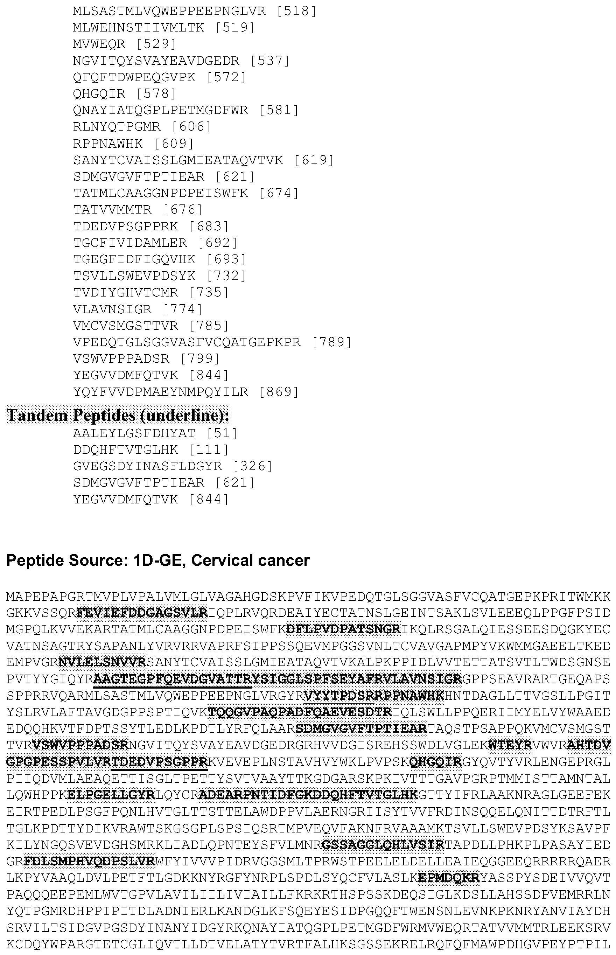

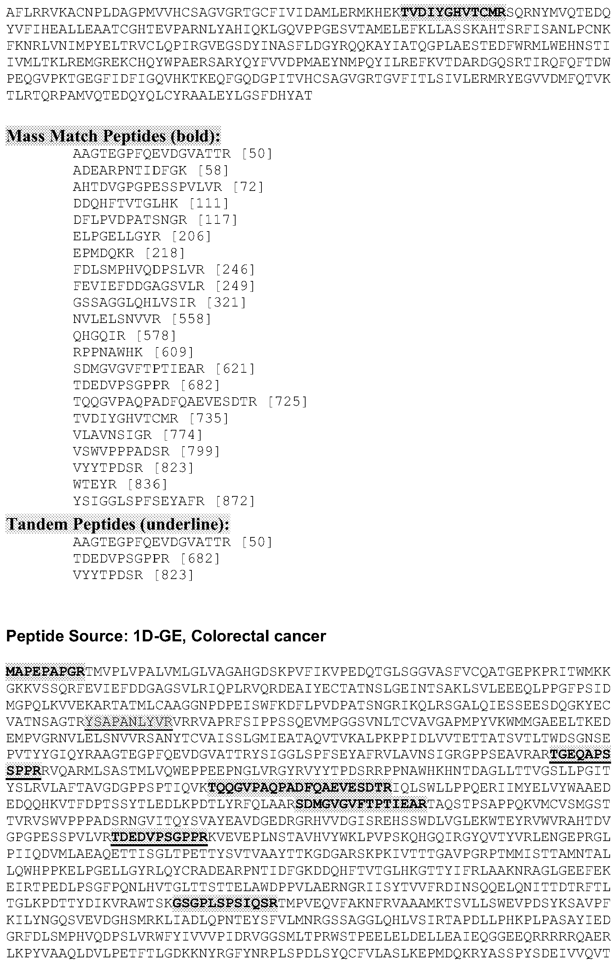

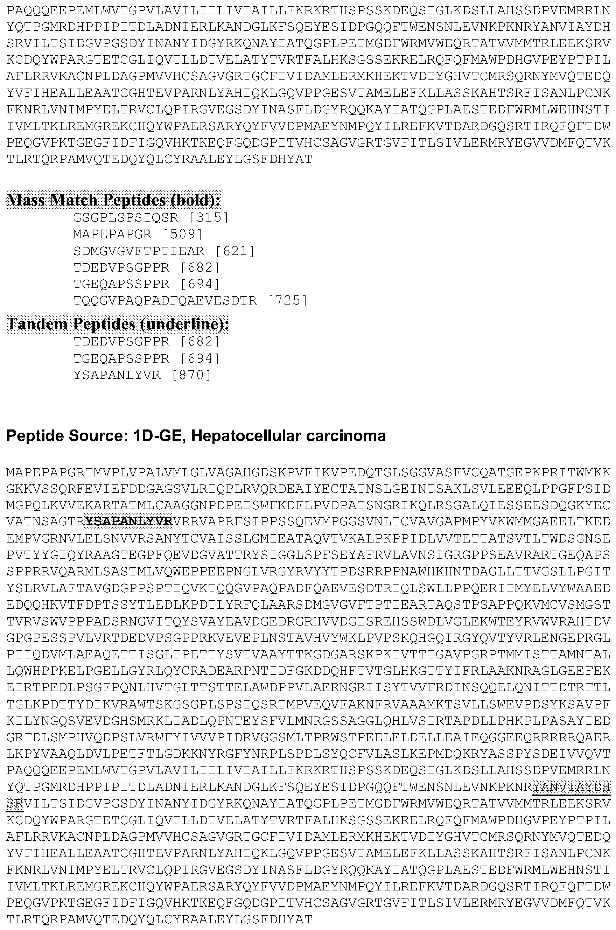

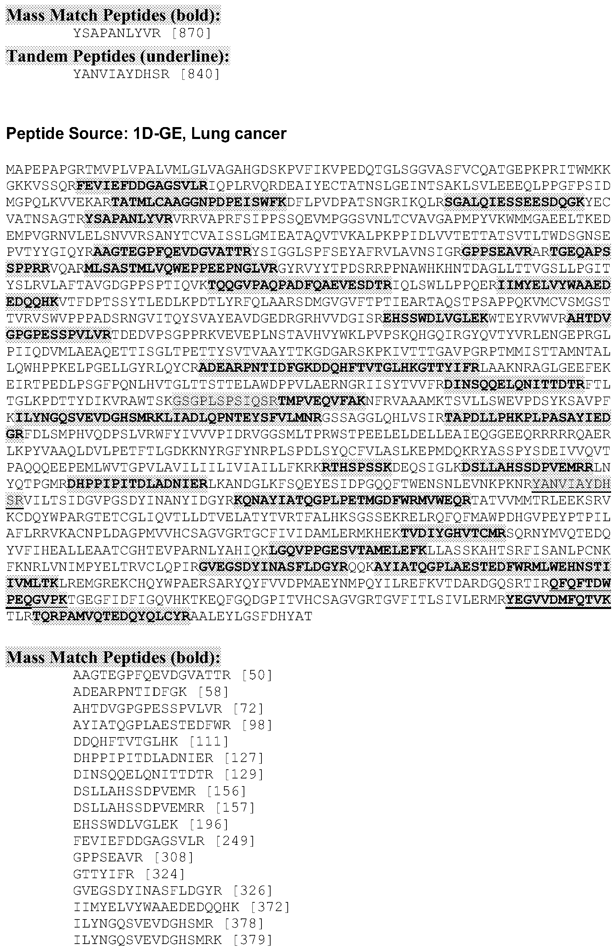

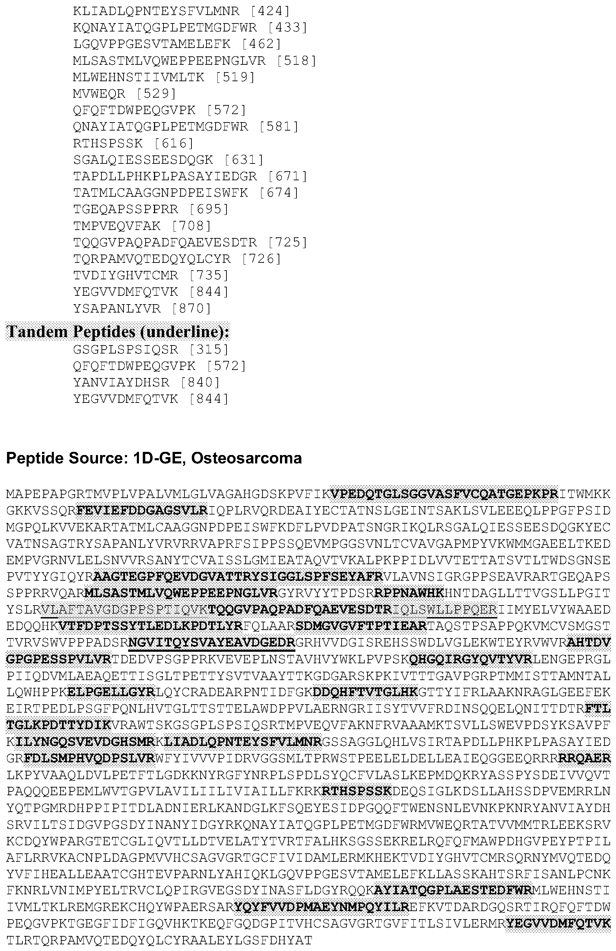

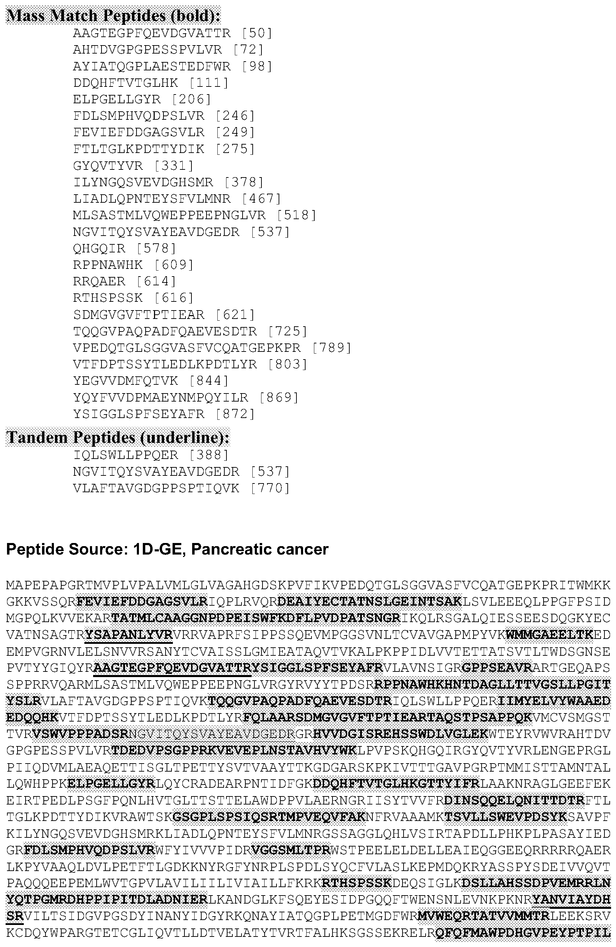

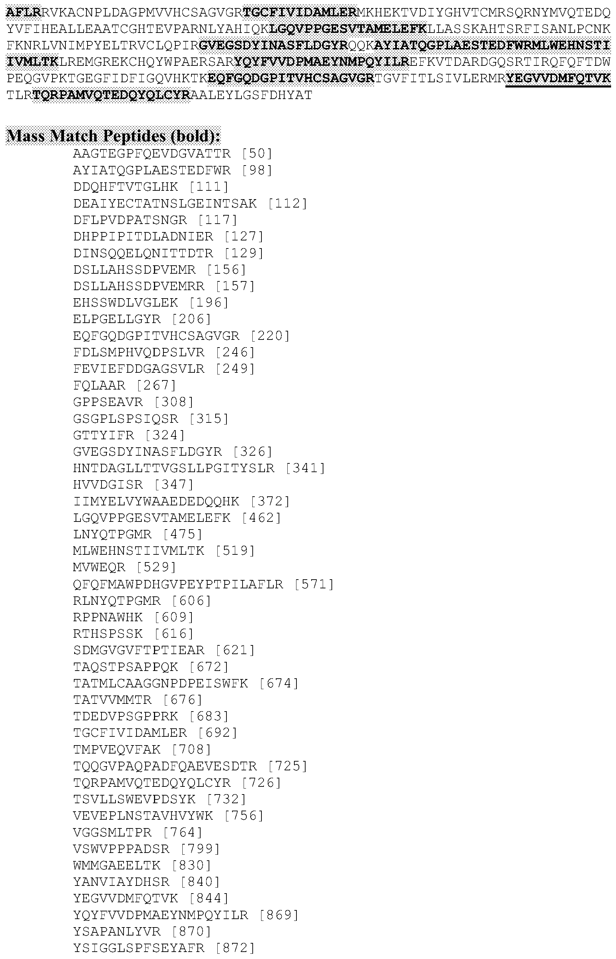

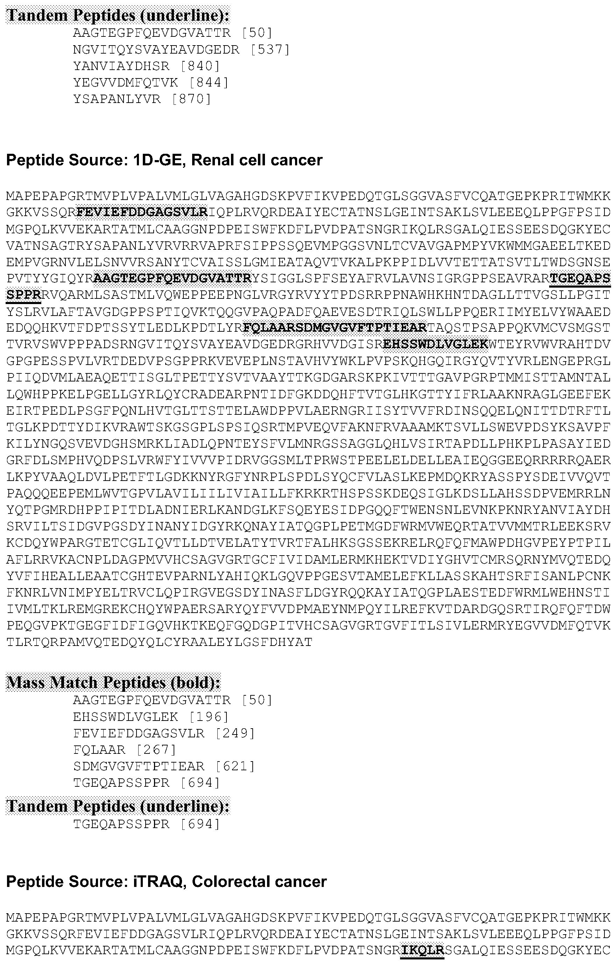

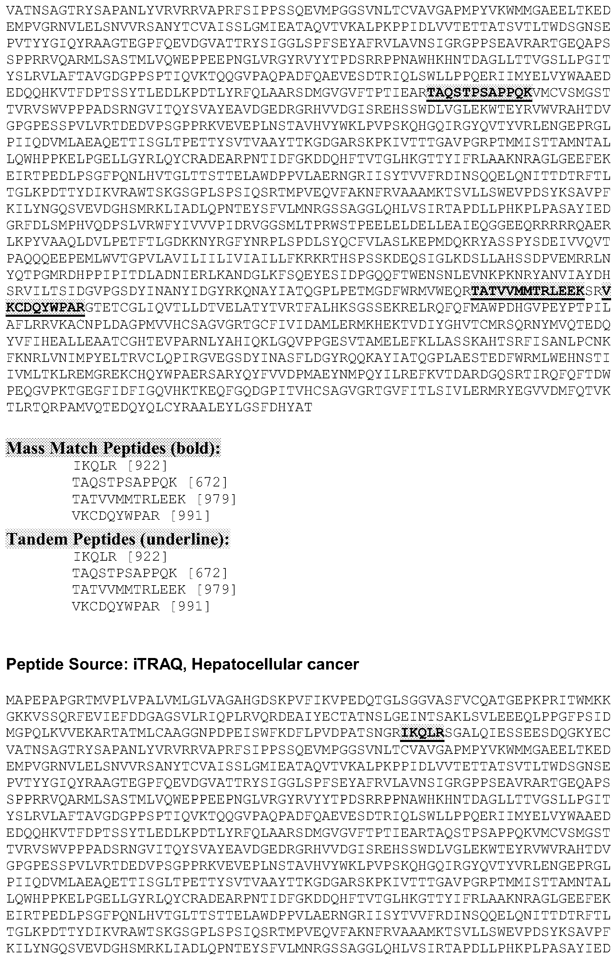

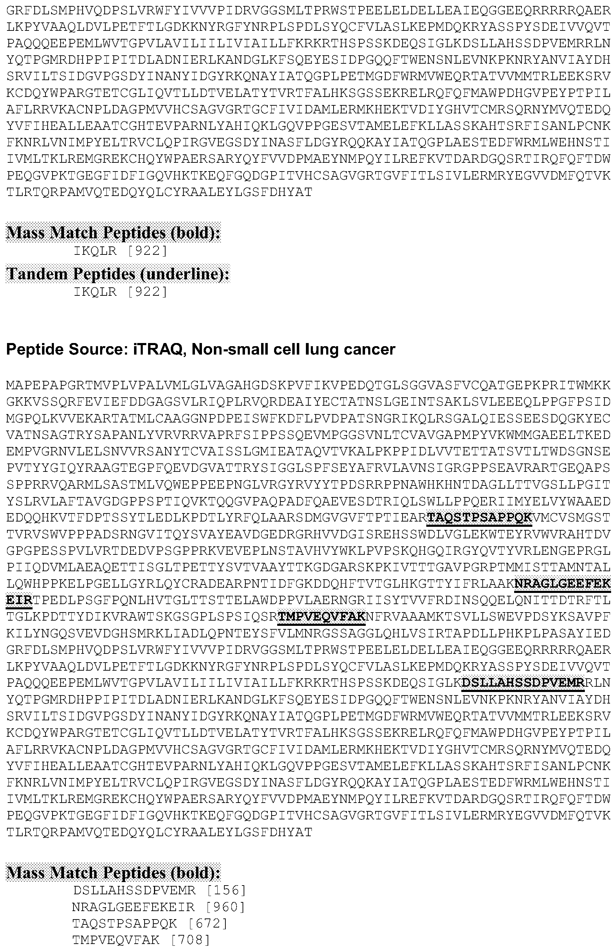

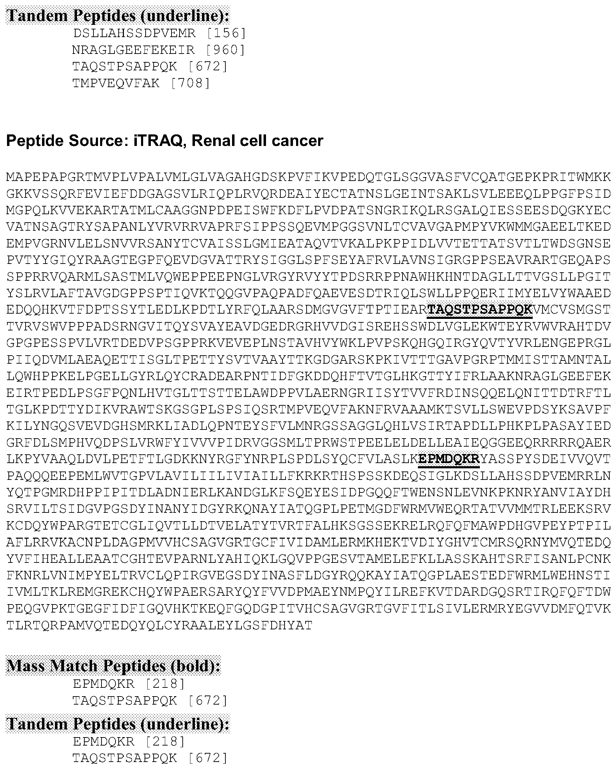

As used herein, the terms:
- OGTA(s), or
- OGTA according to the invention, or
- OGTA employed in the invention or
- "Proteins of the invention",
relates to OGTA 126. Other proteins disclosed herein relate to "OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and/or OGTA271"as illustrated in Figure 1 detected experimentally by ID gel electrophoresis and iTRAQ analysis of lymphoid, breast, cervical, colorectal, gastric, brain, liver, lung, skin, neuronal, osteoblast, ovarian, pancreatic, prostate, kidney and/or eye tissue samples. These terms are used interchangeably in this specification.

OGTA002 has been identified in membrane protein extracts of breast, colorectal, liver, skin, pancreatic, lung and kidney tissue samples from breast cancer, colorectal cancer, heaptocellular carcinoma, melanoma, ovarian cancer, pancreatic cancer, lung cancer and kidney cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P54760, Ephrin type-B receptor 4, was identified.

Ephrin type-B receptor 4 is known to be abundantly expressed in placenta and in a range of primary tissues and malignant cell lines. It is expressed in fetal, but not adult, brain, and in primitive and myeloid, but not lymphoid, hematopoietic cells. It is a receptor for members of the ephrin-B family. It binds to ephrin-B2. It may have a role in events mediating differentiation and development.

OGTA009 has been identified in membrane protein extracts of breast, pancreatic, prostate, kidney, colorectal, lung and ovarian tissue samples from breast cancer, pancreatic cancer, prostate cancer, renal cell cancer, colorectal cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 015551, Claudin-3, was identified.
Claudin-3 plays a major role in tight junction-specific obliteration of the intercellular space, through calcium-independent cell-adhesion activity.

OGTA016 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P01833, Polymeric-immunoglobulin receptor, was identified.
Polymeric-immunoglobulin receptor binds polymeric IgA and IgM at the basolateral surface of epithelial cells. The complex is then transported across the cell to be secreted at the apical surface. During this process a cleavage occurs that separates the extracellular (known as the secretory component) from the transmembrane segment.

OGTA028 has been identified in membrane protein extracts of ovarian, colorectal, kidney, liver and lung samples from ovarian cancer, colorectal cancer, kidney cancer, liver cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T7N2, FLJ10884, was identified.

OGTA037 has been identified in membrane protein extracts of gastric, colorectal, lung and ovarian tissue samples from gastric cancer, colorectal cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9HA72, Protein FAM26B, was identified.

OGTA041 has been identified in membrane protein extracts of liver, lung and pancreatic tissue samples from hepatocellular carcinoma, lung cancer and pancreatic cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P40189, Interleukin-6 receptor subunit beta, was identified.
Interleukin-6 receptor subunit beta is expressed in all the tissues and cell lines examined. Expression is not restricted to IL6 responsive cells. It is a signal-transducing molecule. The receptor systems for IL6, LIF, OSM, CNTF, IL11, CTF1 and BSF3 can utilize gp130 for initiating signal transmission. It binds to IL6/IL6R (alpha chain) complex, resulting in the formation of high-affinity IL6 binding sites, and transduces the signal. It does not bind IL6. It may have a role in embryonic development. The type I OSM receptor is capable of transducing OSM-specific signaling events.

OGTA053 has been identified in membrane protein extracts of pancreatic, colorectal, kidney, liver and lung tissue samples from pancreatic cancer, colorectal cancer, kidney cancer, liver cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9Y4D2, Sn1-specific diacylglycerol lipase alpha, was identified. Sn1-specific diacylglycerol lipase alpha is known to be highly expressed in the brain and pancreas. It catalyses the hydrolysis of diacylglycerol (DAG) to 2-arachidonoyl-glycerol (2-AG), the most abundant endocannabinoid in tissues. It is required for axonal growth during development and for retrograde synaptic signaling at mature synapses.

OGTA054 has been identified in membrane protein extracts of pancreatic, colorectal and lung tissue samples from pancreatic cancer, colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P13164, Interferon-induced transmembrane protein 1, was identified.
Interferon-induced transmembrane protein 1 is implicated in the control of cell growth. It is a component of a multimeric complex involved in the transduction of antiproliferative and homotypic adhesion signals.

OGTA066 has been identified in membrane protein extracts of colorectal, pancreatic, kidney and lung tissue samples from colorectal cancer, pancreatic cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q8TD06, Anterior gradient protein 3 homolog, was identified.

OGTA072 has been identified in membrane protein extracts of colorectal tissue samples from colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q16186, Adhesion-regulating molecule 1, was identified. Adhesion-regulating molecule 1 promotes cell adhesion.

OGTA074 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: O00508, Latent TGF-beta binding protein-4, was identified.

OGTA076 has been identified in membrane protein extracts of lymphoid, colorectal, pancreatic, kidney, liver, lung and ovarian tissue samples from chronic lymphocytic leukaemia, colorectal cancer, pancreatic cancer, kidney cancer, liver cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: O60449, Lymphocyte antigen 75, was identified.
Lymphocyte antigen 75 is known to be predominantly expressed in the spleen, thymus, colon and peripheral blood lymphocytes. It is detected in myeloid and B lymphoid cell lines. It is also expressed in malignant Hodgkin's lymphoma cells called Hodgkin's and Reed-Sternberg (HRS) cells. It acts as an endocytic receptor to direct captured antigens from the extracellular space to a specialized antigen-processing compartment. It causes reduced proliferation of B-lymphocytes.

OGTA085 has been identified in membrane protein extracts of lung, skin, eye and colorectal tissue samples from lung cancer, melanoma, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14318, 38 kDa FK506-binding protein homologue, was identified. 38 kDa FK506-binding protein homologue is known to be widely expressed, with the highest levels seen in the brain. It has no PPIase/rotamase activity.

OGTA087 has been identified in membrane protein extracts of lymphoid, breast, colorectal, lung, stomach and ovarian tissue samples from B-cell non-Hodgkin's lymphoma, breast cancer, colorectal cancer, gastric cancer, lung cancer, lymphoid leukaemia and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: O94935, KIAA0851 protein, was identified.

OGTA088 has been identified in membrane protein extracts of breast, gastric, brain, liver, lung, lymphoid, pancreatic, kidney, eye and colorectal tissue samples from breast cancer, gastric cancer, glioblastoma, hepatocellular carcinoma, lung cancer, lymphoid leukaemia, pancreatic cancer, renal cell cancer, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 015126, Secretory carrier-associated membrane protein 1, was identified.
Secretory carrier-associated membrane protein 1 is known to be widely expressed, with the highest levels seen in the brain. It functions in post-Golgi recycling pathways. It acts as a recycling carrier to the cell surface.

OGTA089 has been identified in membrane protein extracts of breast, lung, ovarian and kidney tissue samples from breast cancer, lung cancer, ovarian cancer and kidney cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T225, Polycystic kidney disease 1-like, was identified.

OGTA091 has been identified in membrane protein extracts of breast, cervical, lymphoid, stomach, liver, lung, skin, bone, ovarian, pancreatic, prostate, kidney, eye and colorectal tissue samples from breast cancer, cervical cancer, chronic lymphocytic leukaemia, gastric cancer, hepatocellular carcinoma, lung cancer, melanoma, neuroblastoma, osteosarcoma, ovarian cancer, pancreatic cancer, prostate cancer, renal cell cancer, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: O75923, Dysferlin, was identified.
Dysferlin is known to be highly expressed in skeletal muscle. It is also found in heart, placenta and at lower levels in liver, lung, kidney and pancreas.

OGTA098 has been identified in membrane protein extracts of breast, cervical, liver, lung, bone, pancreatic, prostate, kidney, colorectal and ovarian tissue samples from breast cancer, cervical cancer, hepatocellular carcinoma, lung cancer, osteosarcoma, pancreatic cancer, prostate cancer, renal cell cancer, colorectal cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14126, Desmoglein-2, was identified.
Desmoglein-2 is found in all of the tissues tested and carcinomas. It is a component of intercellular desmosome junctions. It is involved in the interaction of plaque proteins and intermediate filaments mediating cell-cell adhesion.

OGTA101 has been identified in membrane protein extracts of breast, cervical, colorectal, liver, lung, bone, pancreatic, kidney and ovarian tissue samples from breast cancer, cervical cancer, colorectal cancer, gastric cancer, hepatocellular carcinoma, lung cancer, osteosarcoma, pancreatic cancer, kidney cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P23468, Receptor-type tyrosine-protein phosphatase delta, was identified.

OGTA104 has been identified in membrane protein extracts of breast, liver, ovarian, pancreatic, colorectal, kidney and lung tissue samples from breast cancer, hepatocellular carcinoma, ovarian cancer, pancreatic cancer, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q13443, ADAM 9, was identified.
ADAM 9 is known to be widely expressed. It is expressed in chondrocytes, liver and heart. It is a probable zinc protease. It may mediate cell-cell or cell-matrix interactions. It displays alpha-secretase activity for APP.

OGTA106 has been identified in membrane protein extracts of cervical, skin, pancreatic and liver tissue samples from cervical cancer, melanoma, pancreatic cancer and liver cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q99466, Neurogenic locus notch homologue protein 4, was identified.
Neurogenic locus notch homologue protein 4 is known to be highly expressed in the heart, moderately expressed in the lung and placenta and at low levels in the liver, skeletal muscle, kidney, pancreas, spleen, lymph node, thymus, bone marrow and fetal liver. No expression was seen in adult brain or peripheral blood leukocytes. It functions as a receptor for membrane-bound ligands Jagged1, Jagged2 and Delta1 to regulate cell-fate determination. Upon ligand activation through the released notch intracellular domain (NICD) it forms a transcriptional activator complex with RBP-J kappa and activates genes of the enhancer of split locus. It affects the implementation of differentiation, proliferation and apoptotic programs. It may regulate branching morphogenesis in the developing vascular system.

OGTA112 has been identified in membrane protein extracts of breast, cervical, lymphoid, liver, pancreatic and kidney tissue samples from breast cancer, cervical cancer, chronic lymphocytic leukaemia, hepatocellular carcinoma, pancreatic cancer and renal cell cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P48594, Serpin B4, was identified.
Serpin B4 is known to be predominantly expressed in squamous cells. It may act as a protease inhibitor to modulate the host immune response against tumour cells.

OGTA113 has been identified in membrane protein extracts of pancreatic, colorectal, kidney, lung and ovarian tissue samples from pancreatic cancer, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9BTV4, Transmembrane protein 43, was identified.

OGTA119 has been identified in membrane protein extracts of lymphoid, breast, colorectal, stomach, liver, lung, brain, bone, pancreatic, prostate, kidney and ovarian tissue samples from B-cell non-Hodgkin's lymphoma, breast cancer, colorectal cancer, gastric cancer, hepatocellular carcinoma, lung cancer, lymphoid leukaemia, neuroblastoma, osteosarcoma, pancreatic cancer, prostate cancer, renal cell cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q6UKI4, KIAA1228 protein, was identified.

OGTA124 has been identified in membrane protein extracts of liver, lung, ovarian, pancreatic, kidney and colorectal tissue samples from hepatocellular carcinoma, lung cancer, ovarian cancer, pancreatic cancer, renal cell cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9UP95, Solute carrier family 12 member 4, was identified.
Solute carrier family 12 member 4 is known to be ubiquitous. Levels are much higher in erythrocytes from patients with Hb SC and Hb SS compared to normal AA erythrocytes. This may contribute to red blood cell dehydration and to the manifestation of sickle cell disease by increasing the intracellular concentration of HbS. It mediates electroneutral potassium-chloride cotransport when activated by cell swelling. It may contribute to cell volume homeostasis in single cells. It may be involved in the regulation of basolateral Cl(-) exit in NaCl absorbing epithelia.

OGTA126 has been identified in membrane protein extracts of breast, colorectal, liver, skin, pancreatic, prostate, kidney and lung tissue samples from breast cancer, colorectal cancer, hepatocellular carcinoma, melanoma, pancreatic cancer, prostate cancer, renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H5V8, CUB domain-containing protein 1, was identified.
CUB domain-containing protein 1 is known to be highly expressed in mitotic cells with low expression during interphase. It is detected at highest levels in skeletal muscle and colon with lower levels in kidney, small intestine, placenta and lung. It is expressed in a number of human tumour cell lines as well as in colorectal cancer, breast carcinoma and lung cancer. It is also expressed in cells with phenotypes reminiscent of mesenchymal stem cells and neural stem cells. It may be involved in cell adhesion and cell matrix association. It may play a role in the regulation of anchorage versus migration or proliferation versus differentiation via its phosphorylation. It may be a novel marker for leukaemia diagnosis and for immature hematopoietic stem cell subsets. It belongs to the tetraspanin web involved in tumour progression and metastasis.

OGTA156 has been identified in membrane protein extracts of lymphoid, liver, colorectal, kidney, lung and ovarian tissue samples from acute T-cell leukaemia, B-cell non-Hodgkin's lymphoma, chronic lymphocytic leukaemia, hepatocellular carcinoma, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P13612, Integrin alpha-4, was identified. Integrins alpha-4/beta-1 (VLA-4) and alpha-4/beta-7 are receptors for fibronectin. They recognize one or more domains within the alternatively spliced CS-1 and CS-5 regions of fibronectin. They are also receptors for VCAM1. Integrin alpha-4/beta-1 recognizes the sequence Q-I-D-S in VCAM1. Integrin alpha-4/beta-7 is also a receptor for MADCAM1. It recognizes the sequence L-D-T in MADCAM1. On activated endothelial cells integrin VLA-4 triggers homotypic aggregation for most VLA-4-positive leukocyte cell lines. It may also participate in cytolytic T-cell interactions with target cells.

OGTA159 has been identified in membrane protein extracts of breast, lymphoid, colorectal, liver, skin, pancreatic, eye, kidney and lung tissue samples from breast cancer, chronic lymphocytic leukaemia, colorectal cancer, hepatocellular carcinoma, melanoma, pancreatic cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q96HY6, Uncharacterized protein C20orf116, was identified.

OGTA168 has been identified in membrane protein extracts of brain and skin tissue samples from glioblastoma and melanoma patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q8WZA4, Collectin placenta 1, was identified.

OGTA169 has been identified in membrane protein extracts of breast, lymphoid, colorectal, kidney and lung tissue samples from breast cancer, chronic lymphocytic leukaemia, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P20702, Integrin alpha-X, was identified.
Integrin alpha-X is known to be predominantly expressed in monocytes and granulocytes. Integrin alpha-X/beta-2 is a receptor for fibrinogen. It recognizes the sequence G-P-R in fibrinogen. It mediates cell-cell interaction during inflammatory responses. It is especially important in monocyte adhesion and chemotaxis.

OGTA174 has been identified in membrane protein extracts of lymphoid and lung tissue samples from acute T-cell leukaemia, chronic lymphocytic leukaemia and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P06127, T-cell surface glycoprotein CD5, was identified.
T-cell surface glycoprotein CD5 may act as a receptor in regulating T-cell proliferation. CD5 interacts with CD72/LYB-2.

OGTA176 has been identified in membrane protein extracts of lymphoid, colorectal, kidney and lung tissue samples from B-cell non-Hodgkin's lymphoma, chronic lymphocytic leukaemia, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P21854, B-cell differentiation antigen CD72 homolog, was identified.
B-cell differentiation antigen CD72 is known to be predominantly expressed in pre-B-cells and B-cells but not terminally differentiated plasma cells. It plays a role in B-cell proliferation and differentiation. It associates with CD5.

OGTA177 has been identified in membrane protein extracts of lymphoid, colorectal, kidney, lung and ovarian tissue samples from chronic lymphocytic leukaemia, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P49961, Ectonucleoside triphosphate diphosphohydrolase 1, was identified.
Ectonucleoside triphosphate diphosphohydrolase 1 is known to be expressed primarily on activated lymphoid cells. It is also expressed in endothelial tissues. It is present in both placenta and umbilical vein. In the nervous system, it could hydrolyze ATP and other nucleotides to regulate purinergic neurotransmission. It could also be implicated in the prevention of platelet aggregation. It hydrolyses ATP and ADP equally well.

OGTA197 has been identified in membrane protein extracts of colorectal, liver, lung, skin, bone, ovarian, pancreatic, prostate and kidney tissue samples from colorectal cancer, hepatocellular carcinoma, lung cancer, melanoma, neuroblastoma, osteosarcoma, ovarian cancer, pancreatic cancer, prostate cancer, renal cell cancer and retinoblastoma patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P29317, Ephrin type-A receptor 2, was identified.
Ephrin type-A receptor 2 is known to be expressed most highly in tissues that contain a high proportion of epithelial cells e.g. skin, intestine, lung, and ovary. It is a receptor for members of the ephrin-A family. It binds to ephrin-Al, -A3, -A4 and -A5.

OGTA202 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P22223, Cadherin-3, was identified. Cadherin-3 is known to be expressed in some normal epithelial tissues and in some carcinoma cell lines. Cadherins are calcium dependent cell adhesion proteins. They preferentially interact with themselves in a homophilic manner in connecting cells; cadherins may thus contribute to the sorting of heterogeneous cell types.

OGTA203 has been identified in membrane protein extracts of ovarian, kidney and lung tissue samples from ovarian cancer, renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P55285, Cadherin-6, was identified.
Cadherin-6 is known to be highly expressed in brain, cerebellum, and kidney. Lung, pancreas, and gastric mucosa show a weak expression. It is also expressed in certain liver and kidney carcinomas. Cadherins are calcium dependent cell adhesion proteins. They preferentially interact with themselves in a homophilic manner in connecting cells; cadherins may thus contribute to the sorting of heterogeneous cell types.

OGTA206 has been identified in membrane protein extracts of breast, pancreatic, prostate, colorectal and lung tissue samples from breast cancer, pancreatic cancer, prostate cancer, colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 014493, Claudin-4, was identified.
Claudin-4 plays a major role in tight junction-specific obliteration of the intercellular space.

OGTA213 has been identified in membrane protein extracts of lung and colorectal tissue samples from lung cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14767, Latent-transforming growth factor beta-binding protein 2, was identified.
Latent-transforming growth factor beta-binding protein 2 is known to be expressed in lung and weakly expressed in heart, placenta, liver and skeletal muscle. It may play an integral structural role in elastic-fiber architectural organization and/or assembly.

OGTA214 has been identified in membrane protein extracts of ovarian and colorectal tissue samples from ovarian cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H3R2, Mucin-13, was identified. Mucin-13 is known to be highly expressed in epithelial tissues, particularly those of the gastrointestinal and respiratory tracts, such as large intestine and trachea, followed by kidney, small intestine, appendix and stomach. It is an epithelial and hemopoietic transmembrane mucin that may play a role in cell signaling.

OGTA216 has been identified in membrane protein extracts of breast, pancreatic, colorectal, kidney, liver, lung and ovarian tissue samples from breast cancer, pancreatic cancer, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P55011, Solute carrier family 12 member 2, was identified.
Solute carrier family 12 member 2 is known to be expressed in many tissues. It is an electrically silent transporter system. It mediates sodium and chloride reabsorption. It plays a vital role in the regulation of ionic balance and cell volume.

OGTA222 has been identified in membrane protein extracts of lymphoid and colorectal tissue samples from B-cell non-Hodgkin's lymphoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P16444, Dipeptidase 1, was identified.
Dipeptidase 1 hydrolyses a wide range of dipeptides. It is implicated in the renal metabolism of glutathione and its conjugates. It converts leukotriene D4 to leukotriene E4; it may play an important role in the regulation of leukotriene activity.

OGTA236 has been identified in membrane protein extracts of pancreatic and colorectal tissue samples from pancreatic cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P50443, Sulfate transporter, was identified. Sulfate transporter is known to be ubiquitously expressed. It is a sulfate transporter. It may play a role in endochondral bone formation.

OGTA237 has been identified in membrane protein extracts of lymphoid, prostate, colorectal, kidney and lung tissue samples from B-cell non-Hodgkin's lymphoma, lymphoid leukaemia, prostate cancer, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P22455, Fibroblast growth factor receptor 4, was identified.
Fibroblast growth factor receptor 4 is a receptor for acidic fibroblast growth factor. It does not bind to basic fibroblast growth factor. It binds FGF19.

OGTA247 has been identified in membrane protein extracts of liver, skin and lung tissue samples from hepatocellular carcinoma, melanoma and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P32004, Neural cell adhesion molecule L1, was identified.
Neural cell adhesion molecule L1 is a cell adhesion molecule with an important role in the development of the nervous system. It is involved in neuron-neuron adhesion, neurite fasciculation, outgrowth of neurites, etc. It binds to axonin on neurons.

OGTA248 has been identified in membrane protein extracts of kidney and lung tissue samples from renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P29376, Leukocyte tyrosine kinase receptor, was identified.
Leukocyte tyrosine kinase receptor is known to be expressed in non-hematopoietic cell lines and T- and B-cell lines. The exact function of this protein is not known. It is probably a receptor with a tyrosine-protein kinase activity.

OGTA249 has been identified in membrane protein extracts of ovarian, kidney and lung tissue samples from ovarian cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q969L2, Protein MAL2, was identified.

Protein MAL2 is known to be predominantly expressed in kidney, lung, and liver. It is also found in thyroid gland, stomach and at lower levels in testis and small intestine. It is a member of the machinery of polarized transport. It is required for the indirect transcytotic route at the step of the egress of the transcytosing cargo from perinuclear endosomes in order for it to travel to the apical surface via a raft-dependent pathway.

OGTA257 has been identified in membrane protein extracts of pancreatic, colorectal, liver, lung and ovarian tissue samples from pancreatic cancer, colorectal cancer, liver cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H1D0, Transient receptor potential cation channel subfamily V member 6, was identified.
Transient receptor potential cation channel subfamily V member 6 is known to be expressed at high levels in the gastrointestinal tract, including esophagus, stomach, duodenum, jejunum, ileum and colon, and in pancreas, placenta, prostate and salivary gland. It is expressed at moderate levels in liver, kidney and testis. It is expressed in locally advanced prostate cancer, metastatic and androgen-insensitive prostatic lesions but not detected in healthy prostate tissue and benign prostatic hyperplasia. It is a calcium selective cation channel probably involved in Ca(2+) uptake in various tissues, including Ca(2+) reabsorption in intestine. The channel is activated by low internal calcium level, probably including intracellular calcium store depletion, and the current exhibits an inward rectification. Inactivation includes both a rapid Ca(2+)-dependent and a slower Ca(2+)-calmodulin-dependent mechanism, the latter may be regulated by phosphorylation. In vitro, is slowly inhibited by Mg(2+) in a voltage-independent manner. Heteromeric assembly with TRPV5 seems to modify channel properties. TRPV5-TRPV6 heteromultimeric concatemers exhibit voltage-dependent gating.

OGTA271 has been identified in membrane protein extracts of breast, cervical, colorectal, liver, lung, osteoblast, pancreatic and kidney tissue samples from breast cancer, cervical cancer, colorectal cancer, hepatocellular carcinoma, lung cancer, osteosarcoma, pancreatic cancer and renal cell cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T021, Protein tyrosine phosphatase, receptor type, F, was identified. Proteins of the disclosure are useful as are fragments e.g. antigenic or immunogenic fragments thereof and derivatives thereof. Epitope containing fragments including antigenic or immunogenic fragments will typically be of length 12 amino acids or more e.g. 20 amino acids or more e.g. 50 or 100 amino acids or more. Fragments may be 95% or more of the length of the full protein e.g. 90% or more e.g. 75% or 50% or 25% or 10% or more of the length of the full protein.
Eptiope containing fragments including antigenic or immunogenic fragments will be capable of eliciting a relevant immune response in a patient. DNA encoding proteins of the disclosure is also useful as are fragments thereof e.g. DNA encoding fragments of proteins of the disclosure such as immunogenic fragments thereof. Fragments of nucleic acid (e.g. DNA) encoding Proteins of the disclosure may be 95% or more of the length of the full coding region e.g. 90% or more e.g. 75% or 50% or 25% or 10% or more of the length of the full coding region. Fragments of nucleic acid (e.g. DNA) may be 36 nucleotides or more e.g. 60 nucleotides or more e.g. 150 or 300 nucleotides or more in length.

Derivatives of proteins of the disclosure include variants on the sequence in which one or more (e.g. 1-20 such as 15, or up to 20% such as up to 10% or 5% or 1% by number based on the total length of the protein) deletions, insertions or substitutions have been made. Substitutions may typically be conservative substitutions. Derivatives of proteins of the disclosure include variants on the sequence which have a sequence identity over the entire length thereof of typically at least 60%, 70%, 80%, 90 or 95%. Derivatives will typically have essentially the same biological function as the protein from which they are derived. Derivatives will typically be comparably antigenic or immunogenic to the protein from which they are derived. Derivatives will typically have either the ligand-binding activity, or the active receptor-complex forming ability, or preferably both, of the protein from which they are derived.

Tables 1-48 below illustrate the different occurrences of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 as detected by ID gel electrophoresis and mass spectrometry of membrane protein extracts of lymphoid, breast, cervical, colorectal, gastric, brain, liver, lung, skin, neuronal, osteoblast, ovarian, pancreatic, prostate, kidney and eye tissue samples from relevant cancer patients. The first column provides the molecular weight, the second column gives information on the sub fractionation protocol used, if any (see Example 1 below), and the last column provides a list of the sequences observed by mass spectrometry and the corresponding SEQ ID Nos.

Tables 49-93 illustrate the different occurrences of OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 as detected by iTRAQ and mass spectrometry of membrane protein extracts of colorectal, kidney, liver, lung and ovarian tissue samples from colorectal cancer, kidney cancer, liver cancer, lung cancer and ovarian cancer patients respectively. The first column provides the samples batch number, the second column gives the iTRAQ experiment number and the last column provides a list of the sequences observed by mass spectrometry and the corresponding SEQ ID Nos.

### OGTA002

**Table 1a - Breast cancer**

| MW (Da) | Subfractionation | Tryptics identified [SEQ ID No] |
|---|---|---|
| 88475 | | LNGSSLHLEWSAPLESGGR [473], QPHYSAFGSVGEWLR [585], SQAKPGTPGGTGGPAPQY [646], VDTVAAEHLTR [749] |

**Table 1b - Colorectal cancer**

| MW (Da) | Subfractionation | Tryptics identified [SEQ ID No] |
|---|---|---|
| | Heparin Binding | DLVEPWVVVR [141], EFLSEASIMGQFEHPNIIR [184], EVPPAVSDIR [237], LETADLK [456], VDTVAAEHLTR [749] |
| 46639 | Nucleotide Binding | FPQVVSALDK [263], ILASVQHMK [373], VYIDPFTYEDPNEAVR [818], WTAPEAIAFR [833], YLAEMSYVHR [859] |
| 119837 | Nucleotide Binding | FPQVVSALDK [263], GAPCTTPPSAPR [286] |

**Table 1c - Hepatocellular carcinoma**

| MW (Da) | Subfractionation | Tryptics identified [SEQ ID No] |
|---|---|---|
| 88432 | | VSDFGLSR [796] |

**Table 1d - Melanoma**

| MW (Da) | Subfractionation | Tryptics identified [SEQ ID No] |
|---|---|---|
| 81352 | | ENGGASHPLLDQR [214], FLEENSSDPTYTSSLGGK [258], FTMLECLSLPR [276], IEEVIGAGEFGEVCR [360], KESCVAIK [413], LPPPPDCPTSLHQLMLDCWQK [479], VDTVAAEHLTR [749] |

**Table 1e - Ovarian cancer**

| MW (Da) | Subfractionation | Tryptics identified [SEQ ID No] |
|---|---|---|
| 69811 | | VSDFGLSR [796] |

**Table 1f - Pancreatic cancer**

| MW (Da) | Subfractionation | Tryptics identified [SEQ ID No] |
|---|---|---|
| 116302 | | FPQVVSALDK [263], HGQYLIGHGTK [335], TYEVCDVQR [739] |

### OGTA009

**Table 2a - Breast cancer**

| MW (Da) | Sub fractionation | Tryptics identified [SEQ ID No] |
|---|---|---|
| 23489 | Vesicles | DFYNPVVPEAQK [119], DFYNPVVPEAQKR [120], STGPGASLGTGYDR [657] |
| 23739 | Vesicles | DFYNPVVPEAQK [119] |
| 26156 | Vesicles | DFYNPVVPEAQK [119], STGPGASLGTGYDRK [658] |
| 27751 | Vesicles | VYDSLLALPQDLQAAR [817] |

**Table 2b - Colorectal cancer**

| MW (Da) | Subfractionation | Tryptics identified [SEQ ID No] |
|---|---|---|
| 20920 | | DFYNPVVPEAQKR [120], STGPGASLGTGYDR [657] |
| 21129 | | DFYNPVVPEAQK [119], STGPGASLGTGYDR [657] |
| 21235 | | STGPGASLGTGYDR [657] |
| 21342 | | STGPGASLGTGYDR [657] |
| 21450 | | STGPGASLGTGYDR [657] |
| 21560 | | STGPGASLGTGYDR [657] |
| 21671 | | STGPGASLGTGYDR [657] |

**Table 2c - Pancreatic cancer**

| MW (Da) | Subfractionation | Tryptics identified [SEQ ID No] |
|---|---|---|
| 26738 | | VYDSLLALPQDLQAAR [817] |
| 27019 | | VYDSLLALPQDLQAAR [817] |
| 27306 | | STGPGASLGTGYDR [657] |

**Table 2d - Prostate cancer**

| MW (Da) | Subfractionation | Tryptics identified [SEQ ID No] |
|---|---|---|
| | Digitonin | DFYNPVVPEAQK [120], STGPGASLGTGYDR [657], VVYSAPR [815], VYDSLLALPQDLQAAR [817] |

**Table 2e - Renal cell cancer**

| MW (Da) | Subfractionation | Tryptics identified [SEQ ID No] |
|---|---|---|
| 19493 | | STGPGASLGTGYDR [657] |

### OGTA016

**Table 3 - Colorectal cancer**

| MW (Da) | Subfractionation | Tryptics identified [SEQ ID No] |
|---|---|---|
| | Heparin Binding | ADAAPDEK [57], DGSFSVVITGLR [125] |
| | Heparin Binding | AFVNCDENSR [68], ASVDSGSSEEQGGSSR [86], DGSFSVVITGLR [125], ILLNPQDK [377], QGHFYGETAAVYVAVEER [574] |
| | Heparin Binding | ANLTNFPENGTFVVNIAQLSQDDSGR [79], AQYEGR [85], DGSFSVVITGLR [125], EEFVATTESTTETK [171], QGHFYGETAAVYVAVEER [574], VYTVDLGR [821] |
| | Heparin Binding | DGSFSVVITGLR [125], GSVTFHCALGPEVANVAK [323], ILLNPQDK [377], QGHFYGETAAVYVAVEER [574], VYTVDLGR [821] |
| | Heparin Binding | DGSFSVVITGLR [125], QGHFYGETAAVYVAVEER [574] |
| | Heparin Binding | DGSFSVVITGLR [125], DQADGSR [150], IIEGEPNLK [371], ILLNPQDK [377], QGHFYGETAAVYVAVEER [574], TDISMSDFENSR [684], VPCHFPCK [788], VYTVDLGR [821] |
| 70703 | Nucleotide Binding | AQYEGR [85], EEFVATTESTTETK [171] |
| 84772 | Nucleotide Binding | DGSFSVVITGLR [125], FSSYEK [274], KYWCR [443], QGHFYGETAAVYVAVEER [574] |
| 88695 | Nucleotide Binding | DGSFSVVITGLR [125], FSSYEK [274], QGHFYGETAAVYVAVEER [574], YLCGAHSDGQLQEGSPIQAWQLFVNEESTIPR [861] |
| 91099 | | DFLLQSSTVAAEAQDGPQEA [116], DGSFSVVITGLR [125], EEFVATTESTTETK [171], QGHFYGETAAVYVAVEER [574], TDISMSDFENSR [684], VYTVDLGR [821] |
| 91554 | Nucleotide Binding | ILLNPQDK [377], QGHFYGETAAVYVAVEER [574], RAPAFEGR [597] |
| 93409 | | EEFVATTESTTETK [171], QGHFYGETAAVYVAVEER [574], QSSGENCDVVVNTLGK [588], QSSGENCDVVVNTLGKR [589], VLDSGFR [776], TDISMSDFENSR [684] |
| 95846 | | EEFVATTESTTETK [171], QGHFYGETAAVYVAVEER [574], QGHFYGETAAVYVAVEERK [575], QSSGENCDVVVNTLGK [588] |
| 107634 | Nucleotide Binding | DGSFSVVITGLR [125], EEFVATTESTTETK [171], ILLNPQDK [377], QGHFYGETAAVYVAVEER [574], VYTVDLGR [821] |
| 109842 | Nucleotide Binding | ASVDSGSSEEQGGSSR [86], DGSFSVVITGLR [125], EEFVATTESTTETK [171], FSSYEK [274], ILLNPQDK [377], LSLLEEPGNGTFTVILNQLTSR [486], QGHFYGETAAVYVAVEER [574], TDISMSDFENSR [684], VYTVDLGR [821] |
| 117144 | Nucleotide Binding | ASVDSGSSEEQGGSSR [86], DGSFSVVITGLR [125], GGCITLISSEGYVSSK [297], QGHFYGETAAVYVAVEER [574], TDISMSDFENSR [684] |
| 125678 | Nucleotide Binding | EEFVATTESTTETK [171] |

### OGTA028

**Table 4 - Ovarian cancer**

| MW (Da) | Subfractionation | Tryptics identified [SEQ ID No] |
|---|---|---|
| 22364 | | HQVVHK [343], KENITYMKR [412], MSDVSTSVQSK [525], TEFQQIINLALQK [687], TFSDLQSLRK [691], VAKPEEMK [743], VLMEIQDLMFEEMR [783] |
| 22505 | | EADLTEETEENLR [166], IDSLEDQIEEFSK [358], KENITYMKR [412], VAKPEEMK [743], VFLSIEEFR [761] |
| 24585 | | EITYQGTR [200], KKENITYMK [421], KLPQGESR [425], MDILEER [510], TFSDLQSLR [690], VFLSIEEFR [761], VLMEIQDLMFEEMR [783] |
| 26031 | | KENITYMKR [412], KKENITYMK [421], KLPQGESR [425], QWSNVFNILR [592], REQLTETDK [602], VLMEIQDLMFEEMR [783] |
| 26256 | | EEINQGGR [177], FLCEVK [256], KKENITYMK [421], LPQGESR [480], QWSNVFNILR [592], VLMEIQDLMFEEMR [783] |
| 26487 | | DYVLHMPTLR [165], EEINQGGR [177], IGDDNENLTFK [366], KENITYMKR [412], KKENITYMK [421], LPQGESR [480], NVHLEFTER [557], QWSNVFNILR [592], TEFQQIINLALQK [687], VAKPEEMK [743], VLMEIQDLMFEEMR [783], YGIQEK [852] |
| 26724 | | EEINQGGR [177], IGDDNENLTFK [366], KENITYMKR [412], KKENITYMK [421], LPQGESR [480], QWSNVFNILR [592], TEFQQIINLALQK [687], TLHTEELTSK [704], VLMEIQDLMFEEMR [783], YGIQEK [852] |
| 26966 | | EEIGNLK [175], EIIDENFAELK [197], ELLGNNIP [203], KENITYMK [411], KKENITYMK [421], LPQGESR [480], QWSNVFNILR [592], TLHTEELTSK [704], VLMDEGAVLTLVADLSSATLDISK [782], VLMEIQDLMFEEMR [783] |
| 27470 | | AGEITSDGLSFLFLK [70], DYVLHMPTLR [165], ELLGNNIP [203], KKENITYMK [421], KLPQGESR [425], YGIQEK [852] |
| 28000 | | AGEITSDGLSFLFLK [70], KKENITYMK [421] |
| 47275 | | DVSAIMNK [162], DYVLHMPTLR [165], EIIDENFAELK [197], EITYQGTR [200], HSHTNLSISTGVTK [344], KKENITYMK [421], KLPQGESR [425], KTEEKK [437], LPQGESR [480], LTADLSLDTLDAR [494], REITYQGTR [601], SSHSGVLEIENSVDDLSSR [649], SSVINSIR [652], TFSDLQSLR [690], VFLSIEEFR [761], VLMEIQDLMFEEMR [783] |
| 48293 | | DYVLHMPTLR [165], EIIDENFAELK [197], EITYQGTR [200], HSHTNLSISTGVTK [344], KLPQGESR [425], KTEEKK [437], LTADLSLDTLDAR [494], MAFDFR [507], SLEMSHDEHKK [639], VFLSIEEFR [761], WSNVFK [832] |

### OGTA037

**Table 5 - Gastric cancer**

| MW (Da) | Subfractionation | Tryptics identified [SEQ ID No] |
|---|---|---|
| 24774 | | ENPDNLSDFR [215] |

### OGTA041

**Table 6a - Hepatocellular carcinoma**

| MW (Da) | Subfractionation | Tryptics identified [SEQ ID No] |
|---|---|---|
| 90607 | | DGPEFTFTTPK [124], KPFPEDLK [431] |

**Table 6b - Lung cancer**

| MW (Da) | Subfractionation | Tryptics identified [SEQ ID No] |
|---|---|---|
| 122412 | | DNMLWVEWTTPR [146], QNCSQHESSPDISHFER [582], QVSSVNEEDFVR [591], SHLQNYTVNATK [635] |

**Table 6c - Pancreatic cancer**

| MW (Da) | Subfractionation | Tryptics identified [SEQ ID No] |
|---|---|---|
| 104240 | | HIWPNVPDPSK [336], NYTIFYR [564], SHLQNYTVNATK [635], TNHFTIPK [711] |

### OGTA053

**Table 7 - Pancreatic cancer**

| MW (Da) | Subfractionation | Tryptics identified [SEQ ID No] |
|---|---|---|
| 67953 | | FAPGVTIEEDNCCGCNAIAIR [243], LEEGQATSWSR [452], VLENYNK [780] |

### OGTA054

**Table 8 - Pancreatic cancer**

| MW (Da) | Subfractionation | Tryptics identified [SEQ ID No] |
|---|---|---|
| 9213 | | EEHEVAVLGAPPSTILPR [173] |

### OGTA066

**Table 9a - Colorectal cancer**

| MW (Da) | Subfractionation | Tryptics identified [SEQ ID No] |
|---|---|---|
| 19046 | | IMFVDPSLTVR [380] |
| 19131 | | IMFVDPSLTVR [380] |
| 19217 | | IMFVDPSLTVR [380] |
| 19304 | | IMFVDPSLTVR [380] |

**Table 9c - Pancreatic cancer**

| MW (Da) | Subfractionation | Tryptics identified [SEQ ID No] |
|---|---|---|
| 24387 | | IMFVDPSLTVR [380] |
| 24611 | | IMFVDPSLTVR [380], KPLMVIHHLEDCQYSQALK [432] |

In one or more examples of the disclosure OGTA does not relate to, for example use/methods of diagnosis for colorectal cancer.

### OGTA072

**Table 10 - Colorectal cancer**

| MW (Da) | Subfractionation | Tryptics identified [SEQ ID No] |
|---|---|---|
| | Heparin Binding | MTTSGALFPSLVPGSR [526], TDQDEEHCR [685] |

### OGTA074

**Table 11 - Colorectal cancer**

| MW (Da) | Sub fractionation | Tryptics identified [SEQ ID No] |
|---|---|---|
| 36031 | Nucleotide Binding | EDGYSDASGFGYCFR [169] |
| | Heparin Binding | EDGYSDASGFGYCFR [169], QGPVGSGRR [577], VPEGFTCR [790] |

### OGTA076

**Table 12a - Chronic lymphocytic leukaemia**

| MW (Da) | Subfractionation | Tryptics identified [SEQ ID No] |
|---|---|---|
| 198008 | Chloroform Extracted | AANDPFTIVHGNTGK [52], CEHHSLYGAAR [102], CSMLIASNETWKK [108], EFIYLRPFACDTK [183], ELTYSNFHPLLVSGR [208], FCQALGAHLSSFSHVDEIK [245], FEQEYLNDLMK [247], GWHFYDDR [328], IPENFFEEESR [385], ISEWPIDDHFTYSR [392], KYFWTGLR [442], LFHLHSQK [460], SPDLQGSWQWSDR [644], TPLSYTHWR [718], TPVSTIIMPNEFQQDYDIR [722], VFHRPWR [760], VQCSEQWIPFQNK [793], WVSQHR [838], YFWTGLR [851], YPWHR [866] |
| 223568 | | AANDPFTIVHGNTGK [52], CSMLIASNETWKK [108], ELTYSNFHPLLVSGR [208], FEQEYLNDLMKK [248], FPVTFGEECLYMSAK [265], GWHFYDDR [328], HFVSLCQK [333], IPENFFEEESR [385], ISEWPIDDHFTYSR [392], KVECEHGFGR [439], NWEEAER [563], SDQALHSFSEAK [623], SPDLQGSWQWSDR [644], TPLSYTHWR [718], VFHAER [759], VQCSEQWIPFQNK [793], WVSQHR [838], YPWHR [866] |
| 238644 | | KVECEHGFGR [439], SDQALHSFSEAK [623], TPLSYTHWR [718], VFHAER [759] |

**Table 12b - Colorectal cancer**

| MW (Da) | Subfractionation | Tryptics identified [SEQ ID No] |
|---|---|---|
| 167535 | | IPENFFEEESR [385], TPVSTIIMPNEFQQDYDIR [722] |

**Table 12c - Pancreatic cancer**

| MW (Da) | Subfractionation | Tryptics identified [SEQ ID No] |
|---|---|---|
| 161314 | | AANDPFTIVHGNTGK [52], EFIYLRPFACDTK [183], ELTYSNFHPLLVSGR [208], IEMVDYK [362], IPENFFEEESR [385], ISEWPIDDHFTYSR [392], LTYSSR [499], RHGETCYK [604], SPDLQGSWQWSDR [644], TPLSYTHWR [718], VFHAER [759], VFHRPWR [760], VQCSEQWIPFQNK [793], WVSQHR [838], YFWTGLR [851], YPWHR [866], YVCKRK [875] |
| 261976 | | AFSSDLISIHSLADVEVVVTK [67], CSMLIASNETWKK [108], DGHGTAISNASDVWKK [122], EFIYLRPFACDTK [183], HMATTQDEVHTK [338], IPENFFEEESR [385], ISEWPIDDHFTYSR [392], KYFWTGLR [442], SPDLQGSWQWSDR [644], TPLSYTHWR [718], VFHRPWR [760], VIEEAVYFHQHSILACK [767], VQCSEQWIPFQNK [793], YFWTGLR [851], YPWHR [866], YVCKRK [875] |

### OGTA085

**Table 13a - Lung cancer**

| MW (Da) | Subfractionation | Tryptics identified [SEQ ID No] |
|---|---|---|
| 31005 | | KMLGNPSR [427], STETALYR [655], TIHAELSK [700], VLAQQGEYSEAIPILR [773] |
| 64045 | | TAVDGPDLEMLTGQER [677], VLAQQGEYSEAIPILR [773] |
| 69488 | | TAVDGPDLEMLTGQER [677], VLAQQGEYSEAIPILR [773] |

**Table 13b - Melanoma**

| MW (Da) | Subfractionation | Tryptics identified [SEQ ID No] |
|---|---|---|
| 22017 | | KMLGNPSR [427], MGQPPAEEAEQPGALAR [516], STETALYR [655], TAVDGPDLEMLTGQER [677], VDMTFEEEAQLLQLK [748] |
| 22380 | | STETALYRK [656], TAVDGPDLEMLTGQER [677], VDMTFEEEAQLLQLK [748] |
| 22505 | | SCSLVLEHQPDNIK [620], STETALYRK [656], TAVDGPDLEMLTGQER [677] |
| 22632 | | SCSLVLEHQPDNIK [620], TAVDGPDLEMLTGQER [677], VDMTFEEEAQLLQLK [748] |
| 27711 | | ADFVLAANSYDLAIK [60], GQVVTVHLQTSLENGTR [313], KMLGNPSR [427], LDHYR [447], TAVDGPDLEMLTGQER [677] |

**Table 13c - Retinoblastoma**

| MW (Da) | Subfractionation | Tryptics identified [SEQ ID No] |
|---|---|---|
| 50346 | | KMLGNPSR [427], MGQPPAEEAEQPGALAR [516], VLAQQGEYSEAIPILR [773] |

### OGTA087

**Table 14a - B-cell non-Hodgkin's lymphoma**

| MW (Da) | Subfractionation | Tryptics identified [SEQ ID No] |
|---|---|---|
| 54163 | | FVWNGHLLR [279], LGVLHVGQK [464], LHITPEK [466], LSNTSPEFQEMSLLER [488], NNFSDGFR [550], YIAFDFHK [854], YKPLPQISK [856] |
| 55443 | | ATDFDVLSYKK [89], FVWNGHLLR [279], KTMLHLTDIQLQDNK [438], LHITPEK [466], NNFSDGFR [550], QVIINLINQK [590], QYAGTGALK [593], VANHMDGFQR [744], YKPLPQISK [856] |
| 75222 | | LHITPEK [466], NNFSDGFR [550], QVIINLINQK [590], QYAGTGALK [593], VANHMDGFQR [744], YKPLPQISK [856] |

**Table 14b - Breast cancer**

| MW (Da) | Subfractionation | Tryptics identified [SEQ ID No] |
|---|---|---|
| 52857 | | ATDFDVLSYKK [89], FVWNGHLLR [279], TNVIQSLLAR [714] |

**Table 14c - Colorectal cancer**

| MW (Da) | Subfractionation | Tryptics identified [SEQ ID No] |
|---|---|---|
| | Heparin Binding | LHITPEK [466], YIAFDFHK [854] |

**Table 14d - Gastric cancer**

| MW (Da) | Subfractionation | Tryptics identified [SEQ ID No] |
|---|---|---|
| 50390 | | TNVIQSLLAR [714] |
| 50979 | | TNVIQSLLAR [714] |

**Table 14e - Lung cancer**

| MW (Da) | Subfractionation | Tryptics identified [SEQ ID No] |
|---|---|---|
| 34397 | | LSNTSPEFQEMSLLER [488], NNFSDGFR [550], YFDWILISR [848], YFDWILISRR [849], YIAFDFHK [854], YKPLPQISK [856] |
| 63738 | | HFDSQVIIYGK [332], LHITPEK [466], NNFSDGFR [550], RTHLGLIMDGWNSMIR [615], TNVIQSLLAR [714] |
| 66502 | | GSEKPLEQTFATMVSSLGSGMMR [314], HFDSQVIIYGK [332], LHITPEK [466], NNFSDGFR [550], QYAGTGALK [593], VANHMDGFQR [744], YIAFDFHK [854] |
| 69051 | | ATDFDVLSYK [88], GSEKPLEQTFATMVSSLGSGMMR [314], LEEQDEFEK [453], THLGLIMDGWNSMIR [696], VSTEVTLAVK [797] |

**Table 14f - Lymphoid leukaemia, unspecified**

| MW (Da) | Subfractionation | Tryptics identified [SEQ ID No] |
|---|---|---|
| 56356 | | TNVIQSLLAR [714], YKPLPQISK [856] |
| 58422 | | LSNTSPEFQEMSLLER [488], NNFSDGFR [550], THLGLIMDGWNSMIR [696] |

**Table 14g - Ovarian cancer**

| MW (Da) | Subfractionation | Tryptics identified [SEQ ID No] |
|---|---|---|
| 42632 | | ATDFDVLSYKK [89], FVWNGHLLR [279], GSIPVFWSQRPNLK [316], HFDSQVIIYGK [332], KTMLHLTDIQLQDNK [438], LEEQDEFEK [453], RTHLGLIMDGWNSMIR [615], TMLHLTDIQLQDNK [707] |

### OGTA088

**Table 15a - Breast cancer**

| MW (Da) | Subfractionation | Tryptics identified [SEQ ID No] |
|---|---|---|
| 29702 | | EHALAQAELLK [190], EMQNLSQHGR [211], NVPPGLDEYNPFSDSR [560], RQEELER [612] |
| 29901 | | DPSVTQVTR [149], EMQNLSQHGR [211], NVPPGLDEYNPFSDSR [560], RQEELER [612] |
| 30103 | | EHALAQAELLK [190], EMQNLSQHGR [211], NVPPGLDEYNPFSDSR [560], RQEELER [612] |
| 30309 | | EMQNLSQHGR [211], NVPPGLDEYNPFSDSR [560], RQEELER [612] |

**Table 15b - Gastric cancer**

| MW (Da) | Subfractionation | Tryptics identified [SEQ ID No] |
|---|---|---|
| 29509 | | EHALAQAELLK [190], EMQNLSQHGR [211] |

**Table 15c - Glioblastoma**

| MW (Da) | Subfractionation | Tryptics identified [SEQ ID No] |
|---|---|---|
| 29544 | | EHALAQAELLK [190], EMQNLSQHGR [211], KAAELDR [405], MPNVPNTQPAIMKPTEEHPAYTQIAK [522], NVPPGLDEYNPFSDSR [560], QEELER [566], RQEELER [612], TPPPGGVK [721], TVQTAAANAASTAASSAAQNAFK [737], VHGLYR [766] |
| 29779 | | EHALAQAELLK [190], EMQNLSQHGR [211], NVPPGLDEYNPFSDSR [560], RQEELER [612] |
| 30027 | | AQQEFATGVMSNK [84], EHALAQAELLK [190], EMQNLSQHGR [211], NVPPGLDEYNPFSDSR [560], RQEELER [612], TVQTAAANAASTAASSAAQNAFK [737], VHGLYR [766] |

**Table 15d - Hepatocellular carcinoma**

| MW (Da) | Subfractionation | Tryptics identified [SEQ ID No] |
|---|---|---|
| 26264 | | DPSVTQVTR [149], EHALAQAELLK [190], EMQNLSQHGR [211], KAAELDRR [406], NVPPGLDEYNPFSDSR [560], RQEELER [612] |
| 26522 | | EMQNLSQHGR [211], NVPPGLDEYNPFSDSR [560] |
| 27053 | | EMQNLSQHGR [211], EMQNLSQHGRK [212], NVPPGLDEYNPFSDSR [560], RQEELERK [613] |
| 28538 | | EHALAQAELLK [190], EMQNLSQHGR [211], MPNVPNTQPAIMKPTEEHPAYTQIAK [522], NVPPGLDEYNPFSDSR [560] |

**Table 15e - Lung cancer**

| MW (Da) | Subfractionation | Tryptics identified [SEQ ID No] |
|---|---|---|
| 31584 | | KVHGLYR [441], NVPPGLDEYNPFSDSR [560] |
| 31835 | | EMQNLSQHGR [211], NVPPGLDEYNPFSDSR [560] |
| 33009 | | EMQNLSQHGR [211], NVPPGLDEYNPFSDSR [560], QEELER [566] |
| 33422 | | EHALAQAELLK [190], EMQNLSQHGR [211], MPNVPNTQPAIMKPTEEHPAYTQIAK [522], NVPPGLDEYNPFSDSR [560], RQEELER [612] |
| 33847 | | EMQNLSQHGR [211], NVPPGLDEYNPFSDSR [560] |
| 34135 | | AQQEFATGVMSNK [84], EMQNLSQHGR [211], NVPPGLDEYNPFSDSR [560] |
| 35643 | | EMQNLSQHGR [211], NVPPGLDEYNPFSDSR [560], TPPPGGVK [721], TVQTAAANAASTAASSAAQNAFK [737] |

**Table 15f- Lymphoid leukaemia, unspecified**

| MW (Da) | Subfractionation | Tryptics identified [SEQ ID No] |
|---|---|---|
| 28206 | | EHALAQAELLK [190], EMQNLSQHGR [211], NVPPGLDEYNPFSDSR [560] |

**Table 15g - Pancreatic cancer**

| MW (Da) | Subfractionation | Tryptics identified [SEQ ID No] |
|---|---|---|
| 26742 | | EHALAQAELLK [190], NVPPGLDEYNPFSDSR [560] |
| 31738 | | EHALAQAELLK [190], EMQNLSQHGR [211], NVPPGLDEYNPFSDSR [560] |
| 32245 | | EHALAQAELLK [190], EMQNLSQHGR [211], NVPPGLDEYNPFSDSR [560] |
| 34059 | | DPSVTQVTR [149], EMQNLSQHGR [211], KVHGLYR [441], NVPPGLDEYNPFSDSR [560], TVQTAAANAASTAASSAAQNAFK [737] |
| 34270 | | DPSVTQVTR [149], EMQNLSQHGR [211], NVPPGLDEYNPFSDSR [560] |
| 36671 | | EHALAQAELLK [190], EMQNLSQHGR [211], KAAELDRR [406], NVPPGLDEYNPFSDSR [560], RQEELER [612] |
| 36974 | | DPSVTQVTR [149], EHALAQAELLK [190], EMQNLSQHGR [211], NVPPGLDEYNPFSDSR [560], RQEELER [612] |

**Table 15h - Renal cell cancer**

| MW (Da) | Subfractionation | Tryptics identified [SEQ ID No] |
|---|---|---|
| 30318 | | EHALAQAELLK [190], EMQNLSQHGR [211], NVPPGLDEYNPFSDSR [560] |
| 33762 | | EHALAQAELLK [190], EMQNLSQHGR [211], KVHGLYR [441], NVPPGLDEYNPFSDSR [560] |

**Table 15i - Retinoblastoma**

| MW (Da) | Subfractionation | Tryptics identified [SEQ ID No] |
|---|---|---|
| 30816 | | EHALAQAELLK [190], EMQNLSQHGR [211], NVPPGLDEYNPFSDSR [560], RQEELER [612], TVQTAAANAASTAASSAAQNAFK [737], VHGLYR [766] |

### OGTA089

**Table 16a - Breast cancer**

| MW (Da) | Subfractionation | Tryptics identified [SEQ ID No] |
|---|---|---|
| 110512 | | ADFLIFR [59], EEINKPPIAK [176], ELTLPVDSTTLDGSK [207], GETYTYDWQLITHPR [293], VVEMQGVR [809] |

**Table 16b - Lung cancer**

| MW (Da) | Subfractionation | Tryptics identified [SEQ ID No] |
|---|---|---|
| 72110 | | GETYTYDWQLITHPR [293], IQPYTEQSTK [391], VNDSNELGGLTTSGSAEVHK [786] |

**Table 16c - Ovarian cancer**

| MW (Da) | Subfractionation | Tryptics identified [SEQ ID No] |
|---|---|---|
| 77125 | | GETYTYDWQLITHPR [293], NLQSQSSVNVIVK [544] |
| 89348 | | ADFLIFR [59], EEINKPPIAK [176], GETYTYDWQLITHPR [293], ILDATDQESLELKPTSR [374], IQPYTEQSTK [391], LTPGLYEFK [497], MVFFVQNEPPHQIFK [528], NVSVQPEISEGLATTPSTQQVK [562], THSSNSMLVFLK [699] |

### OGTA091

**Table 17a - Breast cancer**

| MW (Da) | Subfractionation | Tryptics identified [SEQ ID No] |
|---|---|---|
| 24993 | Vesicles | NLVDPFVEVSFAGK [547] |
| 114969 | | TDVHYR [686] |
| 120658 | | TDVHYR [686] |
| 124535 | PNGase F Deglycosylation | GEGVAYR [291] |
| 142847 | | GEGVAYR [291] |
| 164389 | | GEGVAYR [291], NLVDPFVEVSFAGK [547] |
| 165165 | | GEGVAYR [291], NLVDPFVEVSFAGK [547] |
| 171988 | | GEGVAYR [291], NLVDPFVEVSFAGK [547], TDVHYR [686] |
| 176984 | | GEGVAYR [291], TDVHYR [686] |
| 179325 | | GEGVAYR [291], NLVDPFVEVSFAGK [547] |
| 210126 | PNGase F Deglycosylation | TDVHYR [686] |
| 230530 | PNGase F Deglycosylation | TDVHYR [686] |

**Table 17b - Cervical cancer**

| MW (Da) | Subfractionation | Tryptics identified [SEQ ID No] |
|---|---|---|
| | | GEGVAYR [291], TDVHYR [686] |
| | | GEGVAYR [291], TDVHYR [686] |
| | | TDVHYR [686] |

**Table 17c - Chronic lymphocytic leukaemia**

| MW (Da) | Subfractionation | Tryptics identified [SEQ ID No] |
|---|---|---|
| 223568 | | FSDVTGK [272], TDVHYR [686] |

**Table 17d - Gastric cancer**

| MW (Da) | Subfractionation | Tryptics identified [SEQ ID No] |
|---|---|---|
| 95956 | | TDVHYR [686] |
| 125740 | | TDVHYR [686] |
| 129831 | | NLVDPFVEVSFAGK [547] |
| 138914 | | GEGVAYR [291], NLVDPFVEVSFAGK [547], TDVHYR [686] |
| 143975 | | GEGVAYR [291], TDVHYR [686] |

**Table 17e - Hepatocellular carcinoma**

| MW (Da) | Subfractionation | Tryptics identified [SEQ ID No] |
|---|---|---|
| 98239 | | GEGVAYR [291] |
| 102119 | | GEGVAYR [291], NLVDPFVEVSFAGK [547] |
| 106345 | | GEGVAYR [291], TDVHYR [686] |
| 107073 | | GEGVAYR [291] |
| 110964 | | GEGVAYR [291], TDVHYR [686] |
| 114377 | | GEGVAYR [291] |
| 116037 | | GEGVAYR [291], TDVHYR [686] |
| 118513 | | GEGVAYR [291], NLVDPFVEVSFAGK [547], TDVHYR [686] |
| 121636 | | FFASIGER [250], FHLEYR [254], GEGVAYR [291], IGETVVDLENR [367], IIDWDR [370], IPNPHLGPVEER [386], KTDAFRR [436], LLSDKPQDFQIR [469], QIFGFESNK [579], SLGGEGNFNWR [640], TDALLGEFR [681], TDVHYR [686], VEDLPADDILR [753], VQVIEGR [794], VTAAGQTK [800] |
| 123033 | | NLVDPFVEVSFAGK [547], TDVHYR [686] |
| 127849 | | GEGVAYR [291] |
| 128000 | | TDVHYR [686] |
| 133487 | | A VDEQGWEYSITIPPER [94], DLSQMEALK [139], DSFSDPYAIVSFLHQSQK [155], EKPAIHHIPGFEVQETSR [201], EVLATPSLSASFNAPLLDTK [234], FHLEYR [254], GEGVAYR [291], GSQPSGELLASFELIQR [320], HWVPAEK [348], IETQNQLLGIADR [363], IGETVVDLENR [367], ILDESEDTDLPYPPPQR [375], IPNPHLGPVEER [386], IYPLPEDPAIPMPPR [403], LPGGQWIYMSDNYTDVNGEK [477], LSVFAETYENETK [491], MDVGTIYR [511], MFELTCTLPLEK [515], MYYTHRRR [533], RMEPLEK [607], RPDTSFLWFTSPYK [608], SLGGEGNFNWR [640], TDALLGEFR [681], TDVHYR [686], TSLCVLGPGDEAPLERK [729], VEDLPADDILR [753], VPAHQVLFSR [787], VQVIEGR [794], VVFQIWDNDK [810], WEDEEWSTDLNR [824], WLLLSDPDDFSAGAR [827] |
| 139587 | | A VDEQGWEYSITIPPER [94], DLSQMEALK [139], DLSQMEALKR [140], FFASIGER [250], ILDESEDTDLPYPPPQR [375], KNPNFDICTLFMEVMLPR [430], LPGGQWIYMSDNYTDVNGEK [477], MFELTCTLPLEK [515], NPNFDICTLFMEVMLPR [551], QFHQLAAQGPQECLVR [570], RDLSQMEALK [599], RMEPLEK [607], TANPQWNQNITLPAMFPSMCEK [670], TDAFRRR [680], TDALLGEFR [681], TQEETEDPSVIGEFK [724], TSLCVLGPGDEAPLER [728], VEDLPADDILR [753], VVFQIWDNDK [810] |
| 192580 | | TDVHYR [686] |
| 200565 | | GEGVAYR [291], TDVHYR [686] |
| 209214 | | TDVHYR [686] |

**Table 17f - Lung cancer**

| MW (Da) | Subfractionation | Tryptics identified [SEQ ID No] |
|---|---|---|
| 195217 | | GEGVAYR [291], TDVHYR [686] |
| 202986 | | GEGVAYR [291], KNLVDPFVEVSFAGK [429], TDVHYR [686] |
| 211353 | | GEGVAYR [291], KNLVDPFVEVSFAGK [429], TDVHYR [686] |
| 220388 | | GEGVAYR [291], KNLVDPFVEVSFAGK [429], TDVHYR [686] |
| 230173 | | EYSIEEIEAGR [241], ILDESEDTDLPYPPPQR [375], IPNPHLGPVEER [386], MEPLEK [513], RMEPLEK [607], SLGGEGNFNWR [640], TDVHYR [686] |

**Table 17g - Melanoma**

| MW (Da) | Subfractionation | Tryptics identified [SEQ ID No] |
|---|---|---|
| 20629 | | FSDVTGK [272], NLVDPFVEVSFAGK [547] |

**Table 17h - Osteosarcoma**

| MW (Da) | Subfractionation | Tryptics identified [SEQ ID No] |
|---|---|---|
| 151810 | | NLVDPFVEVSFAGK [547] |
| 159457 | | TDVHYR [686] |
| 181827 | | NLVDPFVEVSFAGK [547] |

**Table 17i - Ovarian cancer**

| MW (Da) | Subfractionation | Tryptics identified [SEQ ID No] |
|---|---|---|
| 70973 | | KNLVDPFVEVSFAGK [429] |
| 74967 | | KNLVDPFVEVSFAGK [429] |
| 110436 | | NLVDPFVEVSFAGK [547] |
| 115528 | | NLVDPFVEVSFAGK [547] |
| 127159 | | GEGVAYR [291] |

**Table 17j - Pancreatic cancer**

| MW (Da) | Subfractionation | Tryptics identified [SEQ ID No] |
|---|---|---|
| 55323 | | TDVHYR [686] |
| 70820 | | GEGVAYR [291] |
| 88202 | | FSDVTGK [272], KNLVDPFVEVSFAGK [429] |
| 100099 | | GEGVAYR [291] |
| 132648 | | TDVHYR [686] |
| 133778 | | TDVHYR [686] |
| 142218 | | GEGVAYR [291] |
| 142690 | | NLVDPFVEVSFAGK [547] |
| 154726 | | GEGVAYR [291], NLVDPFVEVSFAGK [547] |
| 158113 | | GEGVAYR [291] |
| 168430 | | GEGVAYR [291], TDVHYR [686] |
| 174222 | | NLVDPFVEVSFAGK [547] |
| 184505 | | FSDVTGK [272], TDVHYR [686] |
| 189061 | | KNLVDPFVEVSFAGK [429] |
| 192682 | | TDVHYR [686] |
| 208929 | | GEGVAYR [291], NLVDPFVEVSFAGK [547], TDVHYR [686] |
| 216690 | | GEGVAYR [291], NLVDPFVEVSFAGK [547], TDVHYR [686] |
| 217481 | | TDVHYR [686] |
| 222673 | | TDVHYR [686] |
| 229012 | | GEGVAYR [291], TDVHYR [686] |
| 230839 | | GEGVAYR [291], TDVHYR [686] |
| 261976 | | TDVHYR [686] |

**Table 17k - Prostate cancer**

| MW (Da) | Subfractionation | Tryptics identified [SEQ ID No] |
|---|---|---|
| | | GEGVAYR [291], TDVHYR [686] |
| | | TDVHYR [686] |

**Table 17l - Renal cell cancer**

| MW (Da) | Subfractionation | Tryptics identified [SEQ ID No] |
|---|---|---|
| 62841 | | TDVHYR [686] |
| 83490 | | GEGVAYR [291] |
| 122161 | | FSDVTGK [272], GEGVAYR [291] |
| 126235 | | GEGVAYR [291] |
| 135291 | | GEGVAYR [291] |
| 143822 | | GEGVAYR [291], TDVHYR [686] |
| 148249 | | TDVHYR [686] |
| 151721 | | GEGVAYR [291], TDVHYR [686] |
| 158158 | | ALGRPGPPFNITPR [76], AVDEQGWEYSITIPPER [94], CIIWNTR [104], DLAAMDK [132], DLSQMEALK [139], EFNQFAEGK [185], EKPAIHHIPGFEVQETSR [201], EYSIEEIEAGR [241], FHLEYR [254], FSFDDFLGSLQLDLNR [273], GEGVAYR [291], GWMIGFEEHK [329], IETQNQLLGIADR [363], IGETVVDLENR [367], IIDWDR [370], ILDESEDTDLPYPPPQR [375], IYPLPEDPAIPMPPR [403], KLPSRPPPHYPGIK [426], LLSDKPQDFQIR [469], MDVGTIYR [511], MYYTHR [531], MYYTHRR [532], RMEPLEK [607], SLGGEGNFNWR [640], SYQLANISSPSLVVECGGQTVQSCVIR [667], TDALLGEFR [681], TQEETEDPSVIGEFK [724], VEDLPADDILR [753], VQVIEGR [794], WEDEEWSTDLNR [824], WLLLSDPDDFSAGAR [827] |
| 200334 | | NLVDPFVEVSFAGK [547] |
| 221217 | | GEGVAYR [291], NLVDPFVEVSFAGK [547], TDVHYR [686] |
| 233732 | | TDVHYR [686] |

### OGTA098

**Table 18a - Breast cancer**

| MW (Da) | Subfractionation | Tryptics identified [SEQ ID No] |
|---|---|---|
| 49867 | | VVPSFLPVDQGGSLVGR [812] |
| 50576 | | DGNGEVTDKPVK [123], DMAGAQAAAVALNEEFLR [142], DNWISVDSVTSEIK [148], DTGEIYTTSVTLDR [159], EETPFFLLTGYALDAR [182], NGVGGMAK [536], QESTSVLLQQSEKK [569], VTQEIVTER [806], VVPSFLPVDQGGSLVGR [812], VYAPASTLVDQPYANEGTVVVTER [816], YVQNGTYTVK [876] |
| 115607 | | ATQFTGATGAIMTTETTK [93], DGNGEVTDKPVK [123], FLDDLGLK [257], IVAISEDYPR [400], QESTSVLLQQSEK [568], VVPSFLPVDQGGSLVGR [812] |

**Table 18b - Cervical cancer**

| MW (Da) | Subfractionation | Tryptics identified [SEQ ID No] |
|---|---|---|
| 82244 | MonoQ Aqueous | DTGEIYTTSVTLDR [159], EEHSSYTLTVEAR [174], IVAISEDYPR [400] |

**Table 18c - Hepatocellular carcinoma**

| MW (Da) | Subfractionation | Tryptics identified [SEQ ID No] |
|---|---|---|
| 83238 | | ATQFTGATGAIMTTETTK [93], GNNVEKPLELR [304], INATDADEPNTLNSK [381], IVAISEDYPR [400], QESTSVLLQQSEK [568] |
| 112208 | | ATQFTGATGAIMTTETTK [93], DNWISVDSVTSEIK [148], DTGEIYTTSVTLDR [159], FLDDLGLK [257], GNNVEKPLELR [304], GQIIGNFQAFDEDTGLPAHAR [311], IVAISEDYPR [400], VATPLPDPMASR [746], VLEGMVEENQVNVEVTR [778], VTQEIVTER [806], VVPSFLPVDQGGSLVGR [812], WEEHR [825] |
| 134896 | | ATQFTGATGAIMTTETTK [93] |

**Table 18d - Lung cancer**

| MW (Da) | Subfractionation | Tryptics identified [SEQ ID No] |
|---|---|---|
| | | ATQFTGATGAIMTTETTK [93], DTGEIYTTSVTLDR [159], EEHSSYTLTVEAR [174], FLDDLGLK [257], GNNVEKPLELR [304], GQIIGNFQAFDEDTGLPAHAR [311], INATDADEPNTLNSK [381], IVAISEDYPR [400], LPDFESR [476], QESTSVLLQQSEK [568], VATPLPDPMASR [746], VLEGMVEENQVNVEVTR [778], VTQEIVTER [806], VVPSFLPVDQGGSLVGR [812] |
| | | DNWISVDSVTSEIK [148], DTGEIYTTSVTLDR [159], EEHSSYTLTVEAR [174], GNNVEKPLELR [304], GQIIGNFQAFDEDTGLPAHAR [311], INATDADEPNTLNSK [381], IVAISEDYPR [400], LPDFESR [476], VLAPASTLQSSYQIPTENSMTAR [772], VLEGMVEENQVNVEVTR [778], VVPSFLPVDQGGSLVGR [812], VYAPASTLVDQPYANEGTVVVTER [816], YKPTPIPIK [858] |

**Table 18e - Osteosarcoma**

| MW (Da) | Subfractionation | Tryptics identified [SEQ ID No] |
|---|---|---|
| 24158 | | GITEPPFGIFVFNK [301] |
| 41065 | | DNWISVDSVTSEIK [148], IVAISEDYPR [400], VLEGMVEENQVNVEVTR [778] |

**Table 18f - Pancreatic cancer**

| MW (Da) | Subfractionation | Tryptics identified [SEQ ID No] |
|---|---|---|
| 47024 | | ATQFTGATGAIMTTETTK [93], DGNGEVTDKPVK [123], EGEDLSKK [187], ESFLAPSSGVQPTLAMPNIAVGQNVTVTER [223], GSSSASIVK [322], VATPLPDPMASR [746], VTQEIVTER [806], VVPSFLPVDQGGSLVGR [812], VYAPASTLVDQPYANEGTVVVTER [816] |
| 47767 | | ATQFTGATGAIMTTETTK [93], DTGEIYTTSVTLDR [159], ESFLAPSSGVQPTLAMPNIAVGQNVTVTER [223], GSSSASIVK [322], VATPLPDPMASR [746], VLAPASTLQSSYQIPTENSMTAR [772], VTQEIVTER [806], VVPSFLPVDQGGSLVGR [812], VYAPASTLVDQPYANEGTVVVTER [816], WEEHR [825] |
| 48531 | | ATQFTGATGAIMTTETTK [93], DGNGEVTDKPVK [123], VTQEIVTER [806], VVPSFLPVDQGGSLVGR [812], VYAPASTLVDQPYANEGTVVVTER [816], WEEHR [825] |
| 57915 | | ATQFTGATGAIMTTETTK [93], DGNGEVTDKPVK [123], DMAGAQAAAVALNEEFLR [142], DTGELNVTSILDR [160], EEHSSYTLTVEAR [174], EETPFFLLTGYALDAR [182], ESFLAPSSGVQPTLAMPNIAVGQNVTVTER [223], GNNVEKPLELR [304], INATDADEPNTLNSK [381], SSVISIYVSESMDR [653], VTQEIVTER [806], VVPSFLPVDQGGSLVGR [812], VYAPASTLVDQPYANEGTVVVTER [816], WEEHR [825] |
| 58846 | | ATQFTGATGAIMTTETTK [93], DGNGEVTDKPVK [123], EGEDLSKK [187], ESFLAPSSGVQPTLAMPNIAVGQNVTVTER [223], IHSDLAEER [369], INATDADEPNTLNSK [381], VTQEIVTER [806], VVPSFLPVDQGGSLVGR [812], VYAPASTLVDQPYANEGTVVVTER [816], WEEHR [825] |
| 119656 | | DGNGEVTDKPVK [123], DMAGAQAAAVALNEEFLR [142], GNNVEKPLELR [304], INATDADEPNTLNSK [381], IVAISEDYPR [400], SEIQFLISDNQGFSCPEK [628], SSVISIYVSESMDR [653] |
| 134980 | | ATQFTGATGAIMTTETTK [93], DNWISVDSVTSEIK [148], DTGEIYTTSVTLDR [159], EEHSSYTLTVEAR [174], GNNVEKPLELR [304], GQIIGNFQAFDEDTGLPAHAR [311], VTQEIVTER [806], VYAPASTLVDQPYANEGTVVVTER [816] |
| 142690 | | ATQFTGATGAIMTTETTK [93], DTGEIYTTSVTLDR [159], EEHSSYTLTVEAR [174], EETPFFLLTGYALDAR [182], GNNVEKPLELR [304], GQIIGNFQAFDEDTGLPAHAR [311], QESTSVLLQQSEK [568], VLEGMVEENQVNVEVTR [778] |

**Table 18g - Prostate cancer**

| MW (Da) | Subfractionation | Tryptics identified [SEQ ID No] |
|---|---|---|
| | | DTGEIYTTSVTLDR [159], FLDDLGLK [257], VATPLPDPMASR [746] |

**Table 18h - Renal cell cancer**

| MW (Da) | Subfractionation | Tryptics identified [SEQ ID No] |
|---|---|---|
| 111421 | | GNNVEKPLELR [304], IVAISEDYPR [400], LPDFESR [476], VTQEIVTER [806] |

### OGTA101

**Table 19a - Breast cancer**

| MW (Da) | Subfractionation | Tryptics identified [SEQ ID No] |
|---|---|---|
| 24841 | Vesicles | QFQFTDWPEQGVPK [572], YQYFVVDPMAEYNMPQYILR [869] |
| 24993 | Vesicles | AALEYLGSFDHYAT [51], QFQFTDWPEQGVPK [572], YQYFVVDPMAEYNMPQYILR [869] |
| 55008 | | GVEGSDYINASFIDGYR [325], YQYFVVDPMAEYNMPQYILR [869] |
| 55921 | | AALEYLGSFDHYAT [51], QFQFTDWPEQGVPK [572] |
| 56324 | | QHGQIR [578] |
| 56872 | | AEPESETSILLSWTPPR [63], EIPSHHPTDPVELR [198], FIKPWESPDEMELDELLK [255], GFPTIDMGPQLK [295], GPPSEPVLTQTSEQAPSSAPR [309], GVEGSDYINASFIDGYR [325], LTQIETGENVTGMELEFK [498], LVNIMPYESTR [503], MLWEHNSTIVVMLTK [520], MVEEVDGR [527], SYSFVLTNR [668], TATMLCAASGNPDPEITWFK [675], TNEDVPSGPPRK [709], TSVLLSWEIPENYNSAMPFK [731], VGFGEEMVK [763] |
| 105325 | | TGCFIVIDAMLER [692] |

**Table 19b - Cervical cancer**

| MW (Da) | Subfractionation | Tryptics identified [SEQ ID No] |
|---|---|---|
| 118858 | Integral | QHGQIR [578], WTEYR [836] |
| 124604 | Integral | WTEYR [836] |

**Table 19c - Colorectal cancer**

| MW (Da) | Subfractionation | Tryptics identified [SEQ ID No] |
|---|---|---|
| | Heparin Binding | YSAPANLYVR [870] |

**Table 19d - Hepatocellular carcinoma**

| MW (Da) | Subfractionation | Tryptics identified [SEQ ID No] |
|---|---|---|
| 72397 | | DMEPLTTLEFSEK [143], GFPTIDMGPQLK [295], GPGPYSPSVQFR [306], SGEGFIDFIGQVHK [632], TATMLCAASGNPDPEITWFK [675], YSAPANLYVR [870], YANVIAYDHSR [840] |

**Table 19e - Lung cancer**

| MW (Da) | Subfractionation | Tryptics identified [SEQ ID No] |
|---|---|---|
| | | YSAPANLYVR [870] |
| 77419 | | FIKPWESPDEMELDELLK [255], GFPTIDMGPQLK [295], GVEGSDYINASFIDGYR [325], LTQIETGENVTGMELEFK [498], QFQFTDWPEQGVPK [572], SDTIANYELVYK [625], TATMLCAASGNPDPEITWFK [675], TVDIYGHVTLMR [736], VEVEAVNSTSVK [755], YEGVVDIFQTVK [843] |
| 78771 | | YANVIAYDHSR [840] |

**Table 19f - Osteosarcoma**

| MW (Da) | Subfractionation | Tryptics identified [SEQ ID No] |
|---|---|---|
| 63624 | | QHGQIR [578], YQYFVVDPMAEYNMPQYILR [869] |

**Table 19g - Pancreatic cancer**

| MW (Da) | Subfractionation | Tryptics identified [SEQ ID No] |
|---|---|---|
| 74307 | | DFLPVDTSNNNGR [118], EIPSHHPTDPVELRR [199], EQFGQDGPISVHCSAGVGR [219], GFPTIDMGPQLK [295], GNSAGGLQHR [305], GVEGSDYINASFIDGYR [325], KRAESDSR [434], LTQIETGENVTGMELEFK [498], LVNIMPYESTR [503], RAESDSRK [595], SDTIANYELVYK [625], SGEGFIDFIGQVHK [632], TGCFIVIDAMLER [692], TVDIYGHVTLMR [736], YANVIAYDHSR [840], YSVAGLSPYSDYEFR [873] |
| 115291 | | TGCFIVIDAMLER [692], YQYFVVDPMAEYNMPQYILR [869], YSAPANLYVR [870] |
| 124451 | | TGCFIVIDAMLER [692], YSAPANLYVR [870] |
| 126215 | | YSAPANLYVR [870] |
| 129890 | | YSAPANLYVR [870] |

### OGTA104

**Table 20a - Breast cancer**

| MW (Da) | Subfractionation | Tryptics identified [SEQ ID No] |
|---|---|---|
| 78181 | | EVPIYANR [236], FAVPTYAAK [244], QPQQFPSRPPPPQPK [586] |

**Table 20b - Hepatocellular carcinoma**

| MW (Da) | Subfractionation | Tryptics identified [SEQ ID No] |
|---|---|---|
| 63302 | | DEEEEPPSMTQLLR [113], DLLPEDFVVYTYNK [134], EVPIYANR [236], KGFGGTAGMAFVGTVCSR [419], QPGSVPR [584], QPQQFPSRPPPPQPK [586], RDQLWR [600] |

**Table 20c - Ovarian cancer**

| MW (Da) | Subfractionation | Tryptics identified [SEQ ID No] |
|---|---|---|
| 49732 | | EVPIYANR [236], KGFGGTAGMAFVGTVCSR [419], QPQQFPSRPPPPQPK [586] |
| 50782 | | DQLWR [152], FAVPTYAAK [244], HVSPVTPPR [346], KGFGGTAGMAFVGTVCSR [419], QPQQFPSRPPPPQPK [586], SYFRK [666] |
| 54191 | | FAVPTYAAK [244], FLPGGTLCR [260], FPSGTLR [264], QPQQFPSRPPPPQPK [586], SYFRK [666] |

**Table 20d - Pancreatic cancer**

| MW (Da) | Subfractionation | Tryptics identified [SEQ ID No] |
|---|---|---|
| 73243 | | EEMILLANYLDSMYIMLNIR [179], EVPIYANR [236], HVSPVTPPR [346], KGFGGTAGMAFVGTVCSR [419], QPQQFPSRPPPPQPK [586] |

### OGTA106

**Table 21a - Cervical cancer**

| MW (Da) | Subfractionation | Tryptics identified [SEQ ID No] |
|---|---|---|
| 61458 | MonoQ Aqueous | SLSGVGAGGGPTPR [642] |

**Table 21b - Melanoma**

| MW (Da) | Subfractionation | Tryptics identified [SEQ ID No] |
|---|---|---|
| 75458 | | CHIQASGRPQCSCMPGWTGEQCQLR [103], EQTPLFLAAR [221], ETDSLSAGFVVVMGVDLSR [229], GPRPNPAIMR [310], GSPQLDCGPPALQEMPINQGGEGKK [318], TLSAGAGPR [705], TPLHAAVAADAR [717] |

**Table 21c - Pancreatic cancer**

| MW (Da) | Subfractionation | Tryptics identified [SEQ ID No] |
|---|---|---|
| 88318 | | EQTPLFLAAR [221] |

### OGTA112

**Table 22a - Breast cancer**

| MW (Da) | Subfractionation | Tryptics identified [SEQ ID No] |
|---|---|---|
| | Triton X114 | ETCVDLHLPR [228], FMFDLFQQFR [261], FYQTSVESTDFANAPEESR [280], FYQTSVESTDFANAPEESRKK [281], KFYQTSVESTDFANAPEESRK [416], KINSWVESQTNEK [420], LMEWTSLQNMR [470], QYNSFNFALLEDVQAK [594], TNSILFYGR [713], TYQFLQEYLDAIK [741], VLHFDQVTENTTEK [781] |

**Table 22b - Cervical cancer**

| MW (Da) | Subfractionation | Tryptics identified [SEQ ID No] |
|---|---|---|
| 133844 | MonoQ Aqueous | FMFDLFQQFRK [262], LMEWTSLQNMR [470], SVQMMR [665], TMGMVNIFNGDADLSGMTWSHGLSVSK [706], VLEIPYK [779] |

**Table 22c - Chronic lymphocytic leukaemia**

| MW (Da) | Subfractionation | Tryptics identified [SEQ ID No] |
|---|---|---|
| 34501 | | ETCVDLHLPR [228], FMFDLFQQFR [261] |

**Table 22d - Hepatocellular carcinoma**

| MW (Da) | Subfractionation | Tryptics identified [SEQ ID No] |
|---|---|---|
| 26010 | | AATYHVDR [54], ETCVDLHLPR [228], FMFDLFQQFR [261], INSWVESQTNEK [384], LMEWTSLQNMR [470], QYNSFNFALLEDVQAK [594], TNSILFYGR [713], VLHFDQVTENTTEK [781] |

**Table 22e - Pancreatic cancer**

| MW (Da) | Subfractionation | Tryptics identified [SEQ ID No] |
|---|---|---|
| 37971 | | ETCVDLHLPR [228], FMFDLFQQFR [261], FMFDLFQQFRK [262], INSWVESQTNEK [384], LMEWTSLQNMR [470], QYNSFNFALLEDVQAK [594], SVQMMR [665], TNSILFYGR [713], VLHFDQVTENTTEK [781] |
| 77017 | | ETCVDLHLPR [228], FMFDLFQQFR [261], INSWVESQTNEK [384], LMEWTSLQNMR [470], QYNSFNFALLEDVQAK [594], SVQMMR [665] |

**Table 22f - Renal cell cancer**

| MW (Da) | Subfractionation | Tryptics identified [SEQ ID No] |
|---|---|---|
| 34256 | | INSWVESQTNEK [384], LMEWTSLQNMR [470], SVQMMR [665], TNSILFYGR [713], VLHFDQVTENTTEK [781] |

### OGTA113

**Table 23 - Pancreatic cancer**

| MW (Da) | Subfractionation | Tryptics identified [SEQ ID No] |
|---|---|---|
| 32378 | | LEDPHVDIIR [449], LVHIIGALR [500], RGDFFYHSENPK [603], SEIINSK [627], TSSQPGFLER [730], YSYNTEWR [874] |
| 32754 | | LEDPHVDIIR [449], LVHIIGALR [500], RGDFFYHSENPK [603], TSSQPGFLER [730], YPEVGDLR [865], YSYNTEWR [874] |
| 42556 | | HVEMYQWVETEESR [345], LEDPHVDIIR [449], RGDFFYHSENPK [603], TSSQPGFLER [730], YPEVGDLR [865], YSYNTEWR [874] |
| 43294 | | HVEMYQWVETEESR [345], LEDPHVDIIR [449], LVHIIGALR [500], RGDFFYHSENPK [603], TSSQPGFLER [730], YSYNTEWR [874] |

### OGTA119

**Table 24a - B-cell non-Hodgkin's lymphoma**

| MW (Da) | Subfractionation | Tryptics identified [SEQ ID No] |
|---|---|---|
| 115552 | | AQPPEAGPQGLHDLGR [83], DEQHQCSLGNLK [115], ERPPDHQHSAQVK [222], GANTHLSTFSFTK [285], VGNQIFQSR [765] |

**Table 24b - Breast cancer**

| MW (Da) | Subfractionation | Tryptics identified [SEQ ID No] |
|---|---|---|
| | | AEWLNK [64] |
| 70772 | | ALALLEDEER [75], AQPPEAGPQGLHDLGR [83], FQLSNSGPNSTIK [268], ISSNPNPVVQMSVGHK [393], NLIAFSEDGSDPYVR [542], VGNQIFQSR [765] |
| 98013 | | AEWLNK [64] |
| 121313 | | AEWLNK [64] |

**Table 24c - Colorectal cancer**

| MW (Da) | Subfractionation | Tryptics identified [SEQ ID No] |
|---|---|---|
| 96268 | Nucleotide Binding | ALALLEDEER [75], AQPPEAGPQGLHDLGR [83], ERPPDHQHSAQVK [222], FQLSNSGPNSTIK [268], LEWLTLMPNASNLDK [457], NLIAFSEDGSDPYVR [542], RQDLEVEVR [611], TNEPVWEENFTFFIHNPK [710] |

**Table 24d - Gastric cancer**

| MW (Da) | Subfractionation | Tryptics identified [SEQ ID No] |
|---|---|---|
| 56270 | | VDVGQQPLR [750] |

**Table 24e - Hepatocellular carcinoma**

| MW (Da) | Subfractionation | Tryptics identified [SEQ ID No] |
|---|---|---|
| 59358 | | ALALLEDEER [75], ERPPDHQHSAQVK [222] |
| 60649 | | ALALLEDEER [75], AQPPEAGPQGLHDLGR [83], RQDLEVEVR [611], VDVGQQPLR [750], VGNQIFQSR [765] |
| 73339 | | AEWLNK [64], AQPPEAGPQGLHDLGR [83], ERPPDHQHSAQVK [222], NLIAFSEDGSDPYVR [542], SDPYGIIR [622], VGNQIFQSR [765], VPLSQLLTSEDMTVSQR [792] |
| 84442 | | AEWLNK [64] |
| 87814 | | AQPPEAGPQGLHDLGR [83], DEQHQCSLGNLK [115], ERPPDHQHSAQVK [222], FQLSNSGPNSTIK [268], HMWPFICQFIEK [339], IHFIEAQDLQGK [368], NLIAFSEDGSDPYVR [542], TNEPVWEENFTFFIHNPK [710], VDVGQQPLR [750] |
| 98646 | | AEWLNK [64] |

**Table 24f- Lung cancer**

| MW (Da) | Subfractionation | Tryptics identified [SEQ ID No] |
|---|---|---|
| 92181 | | AQPPEAGPQGLHDLGR [83], ERPPDHQHSAQVK [222], HMWPFICQFIEK [339], ITVPLVSEVQIAQLR [398] |

**Table 24g - Lymphoid leukaemia unspecified**

| MW (Da) | Subfractionation | Tryptics identified [SEQ ID No] |
|---|---|---|
| 85026 | | ETIEPAVR [230], FQLSNSGPNSTIK [268], GEGPEAGAGGAGGR [290], RQDLEVEVR [611], VDVGQQPLR [750] |
| 86516 | | AQPPEAGPQGLHDLGR [83], ERPPDHQHSAQVK [222], GEGPEAGAGGAGGR [290], HMWPFICQFIEK [339], IHFIEAQDLQGK [368], ISSNPNPVVQMSVGHK [393], NLIAFSEDGSDPYVR [542], RQDLEVEVR [611], VDVGQQPLR [750], VLVALASEELAK [784] |

**Table 24h - Neuroblastoma**

| MW (Da) | Subfractionation | Tryptics identified [SEQ ID No] |
|---|---|---|
| | Sodium Carbonate Scrub | AEWLNK [64] |

**Table 24i - Osteosarcoma**

| MW (Da) | Subfractionation | Tryptics identified [SEQ ID No] |
|---|---|---|
| 73285 | | AEWLNK [64] |

**Table 24j - Pancreatic cancer**

| MW (Da) | Subfractionation | Tryptics identified [SEQ ID No] |
|---|---|---|
| 80573 | | AQPPEAGPQGLHDLGR [83], ERPPDHQHSAQVK [222], GEGPEAGAGGAGGR [290], HMWPFICQFIEK [339], IHFIEAQDLQGK [368], NLIAFSEDGSDPYVR [542], SDPYGIIR [622], VGNQIFQSR [765], VYTENVDK [819] |
| 93931 | | ALALLEDEER [75], DEQHQCSLGNLK [115], ERPPDHQHSAQVK [222], GEGPEAGAGGAGGR [290], ISSNPNPVVQMSVGHK [393], VPLSQLLTSEDMTVSQR [792] |

**Table 24k - Prostate cancer**

| MW (Da) | Subfractionation | Tryptics identified [SEQ ID No] |
|---|---|---|
| | Digitonin Insoluble, Triton X114, Aqueous | AEWLNK [64] |

**Table 24l - Renal cell cancer**

| MW (Da) | Subfractionation | Tryptics identified [SEQ ID No] |
|---|---|---|
| 76269 | | AEWLNK [64], AQPPEAGPQGLHDLGR [83], ERPPDHQHSAQVK [222], HMWPFICQFIEK [339], SIQIHGTMR [638], TNEPVWEENFTFFIHNPK [710], VDVGQQPLR [750], VYTENVDKR [820] |

### OGTA124

**Table 25a - Hepatocellular carcinoma**

| MW (Da) | Subfractionation | Tryptics identified [SEQ ID No] |
|---|---|---|
| 98003 | | DLAVFLYHLR [133], EGLAHLIQSCGLGGMR [188], ESSPFLSPLEASR [227], EWGDGIR [240], HGLPSADAPSLK [334], HNSVVLGWPYGWR [340], LDEDLHVK [446], LEEGPPHTK [451], LVLLNMPGPPR [501], MPHFTVVPVDGPR [521], NIAFYPSNHER [539], NLALFEEELDIRPK [541], SIFDPPVFPVCMLGNR [636] |
| 116154 | | ESSPFLSPLEASR [227], EWGDGIR [240], HGLPSADAPSLK [334], LEEGPPHTK [451], NIAFYPSNHER [539], TPNWRPR [720], YIEYQGAEK [855] |
| 126143 | | EHEEAESGEGTRRR [192], ESSPFLSPLEASR [227], EWGDGIR [240], HNSVVLGWPYGWR [340], LVSYTNLTQGAK [504], MPHFTVVPVDGPR [521], NSEGDENYMEFLEVLTEGLER [553], TFIDTVR [689], YMTETWDPSHAPDNFR [863] |
| 137186 | | AELDDSDGHGNHR [62], EVITIYS [233], LVSYTNLTQGAK [504], MPHFTVVPVDGPR [521], NIAFYPSNHER [539] |

**Table 25b - Lung cancer**

| MW (Da) | Subfractionation | Tryptics identified [SEQ ID No] |
|---|---|---|
| 153348 | | DQFDICAK [151], EHEEAESGEGTR [191], EHEEAESGEGTRRR [192], ESSPFLSPLEASR [227], EVITIYS [233], LVLLNMPGPPR [501], LVSYTNLTQGAK [504] |

**Table 25c - Ovarian cancer**

| MW (Da) | Subfractionation | Tryptics identified [SEQ ID No] |
|---|---|---|
| 69811 | | ESSPFLSPLEASR [227], EVITIYS [233], IQMTWTR [389], LNEVIVTR [472], MHTAVK [517], NIAFYPSNHER [539], NSEGDENYMEFLEVLTEGLER [553], TPNWRPR [720] |
| 74557 | | ESSPFLSPLEASR [227], GIDYYDR [300], NIAFYPSNHER [539], TFIDTVR [689], TPNWRPR [720] |

**Table 25d - Pancreatic cancer**

| MW (Da) | Subfractionation | Tryptics identified [SEQ ID No] |
|---|---|---|
| 137609 | | ELVHIKPDQSNVR [209], ESSPFLSPLEASR [227], HNSVVLGWPYGWR [340], LEEGPPHTK [451], LVSYTNLTQGAK [504], NIAFYPSNHER [539], SIFDPPVFPVCMLGNR [636] |

**Table 25e - Renal cell cancer**

| MW (Da) | Subfractionation | Tryptics identified [SEQ ID No] |
|---|---|---|
| 135833 | | HNSVVLGWPYGWR [340], IFTVAQMDDNSIQMK [364], LVSYTNLTQGAK [504], MPHFTVVPVDGPR [521], NIAFYPSNHER [539] |
| 154919 | | ESSPFLSPLEASR [227], HNSVVLGWPYGWR [340], IFTVAQMDDNSIQMKK [365], LVSYTNLTQGAK [504], SIFDPPVFPVCMLGNR [636] |

### OGTA126

**Table 26a - Breast cancer**

| MW (Da) | Subfractionation | Tryptics identified [SEQ ID No] |
|---|---|---|
| 105325 | | AFMIIQEQR [66], GAEAFEIALPR [282], GLPSLTSVSWNISVPR [302], IYVVDLSNER [404], MALHLPWFHPR [508] |

**Table 26b - Colorectal cancer**

| MW (Da) | Subfractionation | Tryptics identified [SEQ ID No] |
|---|---|---|
| 104028 | | ASVSFLNFNLSNCER [87], MALHLPWFHPR [508], VEYYIPGSTTNPEVFK [757] |

**Table 26c - Hepatocellular carcinoma**

| MW (Da) | Subfractionation | Tryptics identified [SEQ ID No] |
|---|---|---|
| 82606 | | AFMIIQEQR [66], ASVSFLNFNLSNCER [87], EEGVFTVTPDTK [172], LATEEPPPR [444], LWMNVEK [505], QIGPGESCPDGVTHSISGR [580], TCSSNLTLTSGSK [679] |
| 86381 | | AFMIIQEQR [66], LATEEPPPR [444] |
| 88432 | | AFMIIQEQR [66], ASVSFLNFNLSNCER [87], IYVVDLSNER [404], LATEEPPPR [444], LWMNVEK [505], MALHLPWFHPR [508], NVSGFSIANR [561], QIGPGESCPDGVTHSISGR [580] |
| 90607 | | AFMIIQEQR [66], LATEEPPPR [444], NVSGFSIANR [561] |

**Table 26d - Melanoma**

| MW (Da) | Subfractionation | Tryptics identified [SEQ ID No] |
|---|---|---|
| 81352 | | DQVACLTFFK [153], IYVVDLSNER [404], KFVPGCFVCLESR [415] |
| 87990 | | AFMIIQEQR [66], DQVACLTFFK [153], IYVVDLSNER [404], MALHLPWFHPR [508] |
| 102001 | | AFMIIQEQR [66], ASVSFLNFNLSNCER [87], IYVVDLSNER [404], LATEEPPPR [444] |

**Table 26e - Pancreatic cancer**

| MW (Da) | Subfractionation | Tryptics identified [SEQ ID No] |
|---|---|---|
| 57018 | | QGLTVSFIPYFK [576], QIGPGESCPDGVTHSISGR [580], QPGNMAGNFNLSLQGCDQDAQSPGILR [583], VEYYIPGSTTNPEVFK [757] |

**Table 26f- Prostate cancer**

| MW (Da) | Subfractionation | Tryptics identified [SEQ ID No] |
|---|---|---|
| | | AFMIIQEQR [66], EEGVFTVTPDTK [172], IDATVVR [353], IYVVDLSNER [404], LWMNVEK [505], TPNWDR [719] |
| | | AFMIIQEQR [66], IYVVDLSNER [404], LATEEPPPR [444], TPNWDR [719] |
| | | AFMIIQEQR [66], EEGVFTVTPDTK [172], IYVVDLSNER [404], LWMNVEK [505], TPNWDR [719], VEYYIPGSTTNPEVFK [757] |

**Table 26g - Renal cell cancer**

| MW (Da) | Subfractionation | Tryptics identified [SEQ ID No] |
|---|---|---|
| 125611 | | KFVPGCFVCLESR [415], LSLVLVPAQK [487], SGVVCQTGR [633] |

### OGTA156

**Table 27a - Acute T-cell leukaemia**

| MW (Da) | Subfractionation | Tryptics identified [SEQ ID No] |
|---|---|---|
| | | ADGISSTFSQR [61], ATGFPEPNPR [90], AYIFSIDEK [99], SILQEENR [637], THQAFMR [697], TINFAR [701] |
| | | ADGISSTFSQR [61], ATGFPEPNPR [90], AYIFSIDEK [99], DNQWLGVTLSR [147], LPTGGCYGVPPDLR [482], SILQEENR [637], SQHTTEVVGGAPQHEQIGK [648], TINFAR [701] |
| | | ATGFPEPNPR [90], AYIFSIDEK [99], SILQEENR [637], THQAFMR [697], TINFAR [701], VTVAIPLK [807] |
| | | ATGFPEPNPR [90], AYIFSIDEK [99], SILQEENR [637], SQHTTEVVGGAPQHEQIGK [648], THQAFMR [697] |
| | | ATGFPEPNPR [90], AYIFSIDEK [99], SILQEENR [637] |

**Table 27b - B-cell non-Hodgkin's lymphoma**

| MW (Da) | Subfractionation | Tryptics identified [SEQ ID No] |
|---|---|---|
| 112390 | | ADGISSTFSQR [61], ATGFPEPNPR [90], AYIFSIDEK [99], DNQWLGVTLSR [147], EASVHIQLEGRPSILEMDETSALK [167], LPVGLYFIK [484], NIFYIK [540], SDSAVLLR [624], SILQEENR [637], SQHTTEVVGGAPQHEQIGK [648], TINFAR [701], TYLAVGSMK [740] |
| 114990 | | ADGISSTFSQR [61], ATGFPEPNPR [90], AYIFSIDEK [99], DNQWLGVTLSR [147], IAPCYQDYVK [351], KAESPPR [407], NIFYIK [540], SDSAVLLR [624], SILQEENR [637], SQHTTEVVGGAPQHEQIGK [648], STEEFPPLQPILQQK [654], TCLEER [678], THQAFMRK [698], TINFAR [701], TYLAVGSMK [740] |

**Table 27c - Chronic lymphocytic leukaemia**

| MW (Da) | Subfractionation | Tryptics identified [SEQ ID No] |
|---|---|---|
| 95173 | | DNQWLGVTLSR [147], ELNILHEMK [205], SQHTTEVVGGAPQHEQIGK [648] |
| 168205 | | EVPGYIVLFYNMSLDVNR [235], ILELEEK [376], SQHTTEVVGGAPQHEQIGK [648], STEEFPPLQPILQQK [654] |

**Table 27d - Hepatocellular carcinoma**

| MW (Da) | Subfractionation | Tryptics identified [SEQ ID No] |
|---|---|---|
| 66260 | | IEGLQISK [361], VIELNK [768] |

### OGTA159

**Table 28a - Breast cancer**

| MW (Da) | Subfractionation | Tryptics identified [SEQ ID No] |
|---|---|---|
| Unknown | | ESPAQAPA [226], VAQPGPLEPEEPR [745] |

**Table 28b - Chronic lymphocytic leukaemia**

| MW (Da) | Subfractionation | Tryptics identified [SEQ ID No] |
|---|---|---|
| 24836 | | LEEEQK [450] |

**Table 28c - Colorectal cancer**

| MW (Da) | Subfractionation | Tryptics identified [SEQ ID No] |
|---|---|---|
| 42137 | | AASAGQEPLHNEELAGAGR [53], EEQAQR [180], VAQPGPLEPEEPR [745] |
| 42590 | | AASAGQEPLHNEELAGAGR [53], VAQPGPLEPEEPR [745] |

**Table 28d - Hepatocellular carcinoma**

| MW (Da) | Subfractionation | Tryptics identified [SEQ ID No] |
|---|---|---|
| 44183 | | VAQPGPLEPEEPR [745] |

**Table 28e - Melanoma**

| MW (Da) | Subfractionation | Tryptics identified [SEQ ID No] |
|---|---|---|
| 21554 | | IQDLLAEGTITGVIDDR [387], VVLLEDLASQVGLR [811] |
| 21899 | | RDLGSR [598], VVLLEDLASQVGLR [811] |

**Table 28f - Pancreatic cancer**

| MW (Da) | Subfractionation | Tryptics identified [SEQ ID No] |
|---|---|---|
| 33531 | | AASAGQEPLHNEELAGAGR [53], EHEEYLK [193], VAQPGPLEPEEPR [745] |
| 33932 | | AASAGQEPLHNEELAGAGR [53], VAQPGPLEPEEPR [745] |

### OGTA168

**Table 29a - Glioblastoma**

| MW (Da) | Subfractionation | Tryptics identified [SEQ ID No] |
|---|---|---|
| 82431 | MonoQ/Digitonin | AISTNSELSTFR [74], ESHWIGLTDSER [225], GSPGKPGPQGSSGDPGPPGPPGK [317], LQASGDALVDR [485], NDFQNLQQVFLQAK [534], NLITNLQR [543] |
| 99352 | MonoQ/Digitonin | GSPGKPGPQGSSGDPGPPGPPGK [317], GSQGPPGPTGNK [319], KLGDQTGK [423], LDTEVANLSVIMEEMK [448], LGDQTGKK [461], LQASGDALVDR [485], NDFQNLQQVFLQAK [534], SVDDTSQAIQR [661] |

**Table 29b - Melanoma**

| MW (Da) | Subfractionation | Tryptics identified [SEQ ID No] |
|---|---|---|
| 33451 | | GEPGPPGPAGER [292], LTAVESDLK [495], SVDDTSQAIQR [661] |

### OGTA169

**Table 30a - Breast cancer**

| MW (Da) | Subfractionation | Tryptics identified [SEQ ID No] |
|---|---|---|
| 100968 | PNGase F Deglycosylation | FGAALTVLGDVNGDK [252] |

**Table 30b - Chronic lymphocytic leukaemia**

| MW (Da) | Subfractionation | Tryptics identified [SEQ ID No] |
|---|---|---|
| | Triton X114, Detergent Soluble | AVIFTQVSR [96], DLQSSVTLDLALDPGR [138], ELNDIASKPSQEHIFK [204], ESHVAMHR [224], FQTHFTFEEFR [269], FQTHFTFEEFRR [270], GAVYLFHGVLGPSISPSHSQR [287], GVQSLVLGAPR [327], IAGSQLSSR [350], IAPPASDFLAHIQK [352], INHLIFR [383], LFHASYGAR [458], LTDVVIGAPGEEENR [496], NFATMMNFVR [535], NLRPMLAADAQR [545], QEQDIVFLIDGSGSISSR [567], SLHLTCDSAPVGSQGTWSTSCR [641], VEDFDALK [752], YAVYTVVSSHEQFTK [841] |
| 108778 | | AVIFTQVSR [96], DVIPMADAAGIIR [161], ESHVAMHR [224], FQTHFTFEEFR [269], GVQSLVLGAPR [327], INHLIFR [383], LFHASYGAR [458], LTDVVIGAPGEEENR [496], NLRPMLAADAQR [545], QEQDIVFLIDGSGSISSR [567], YAVYTVVSSHEQFTK [841] |
| 111501 | | AVIFTQVSR [96], DVIPMADAAGIIR [161], ELNDIASKPSQEHIFK [204], ESHVAMHR [224], FGAALTVLGDVNGDK [252], FQTHFTFEEFR [269], FQTHFTFEEFRR [270], GAVYLFHGVLGPSISPSHSQR [287], GVQSLVLGAPR [327], IAGSQLSSR [350], INHLIFR [383], LFHASYGAR [458], LTDVVIGAPGEEENR [496], NFATMMNFVR [535], NLRPMLAADAQR [545], NMYLTGLCFLLGPTQLTQR [548], QEQDIVFLIDGSGSISSR [567], YAIGVGLAFQNR [839], YAVYTVVSSHEQFTK [841], YQVNNLGQR [868] |
| 114387 | | AVIFTQVSR [96], DVIPMADAAGIIR [161], EMMEEANGQIAPENGTQTPSPPSEK [210], ESHVAMHR [224], FQTHFTFEEFR [269], FQTHFTFEEFRR [270], GGAQITFLATFDVSPK [296], IAGSQLSSR [350], INHLIFR [383], KEGDSLDYK [409], LFHASYGAR [458], LTDVVIGAPGEEENR [496], NFATMMNFVR [535], NLRPMLAADAQR [545], VEDFDALK [752], YAIGVGLAFQNR [839], YAVYTVVSSHEQFTK [841], YQVNNLGQR [868] |
| 117454 | | DVIPMADAAGIIR [161], FQTHFTFEEFR [269], GGQVSVCPLPR [299], IAGSQLSSR [350], INHLIFR [383], KEGDSLDYK [409], LFHASYGAR [458], LTDVVIGAPGEEENR [496], NMYLTGLCFLLGPTQLTQR [548], QEQDIVFLIDGSGSISSR [567], YQVNNLGQR [868] |
| 136634 | | AVIFTQVSR [96], CDVPSFSVQEELDFTLK [100], FQTHFTFEEFR [269], FQTHFTFEEFRR [270], GVQSLVLGAPR [327], INHLIFR [383], KEGDSLDYK [409], LTDVVIGAPGEEENR [496], NFATMMNFVR [535], NLRPMLAADAQR [545], NPVLDCSIAGCLR [552], SLHLTCDSAPVGSQGTWSTSCR [641], YQHTGK [867] |
| 139470 | | AVIFTQVSR [96], DSYLGYSTELALWK [158], FQTHFTFEEFR [269], GVQSLVLGAPR [327], INHLIFR [383], KEGDSLDYK [409], LTDVVIGAPGEEENR [496], NFATMMNFVR [535], NLRPMLAADAQR [545], QEQDIVFLIDGSGSISSR [567] |
| 142364 | | GGQVSVCPLPR [299] |
| 152450 | | AVIFTQVSR [96], AVISQFQRPSTQFSLMQFSNK [97], DIQNQLK [130], DVIPMADAAGIIR [161], ELNDIASKPSQEHIFK [204], ESHVAMHR [224], FQTHFTFEEFR [269], GNLSFGWVR [303], GVQSLVLGAPR [327], IAPPASDFLAHIQK [352], INHLIFR [383], KEGDSLDYK [409], LFHASYGAR [458], LFHASYGARR [459], LTDVVIGAPGEEENR [496], NLRPMLAADAQR [545], QEQDIVFLIDGSGSISSR [567], YAVYTVVSSHEQFTK [841], YQVNNLGQR [868] |
| 159984 | | AVIFTQVSR [96], DIQNQLK [130], DVIPMADAAGIIR [161], ESHVAMHR [224], FQTHFTFEEFR [269], GAVYLFHGVLGPSISPSHSQR [287], GVQSLVLGAPR [327], INHLIFR [383], KEGDSLDYK [409], LFHASYGAR [458], LTDVVIGAPGEEENR [496], NLRPMLAADAQR [545], NMYLTGLCFLLGPTQLTQR [548], QEQDIVFLIDGSGSISSR [567], YAVYTVVSSHEQFTK [841], YQVNNLGQR [868] |

### OGTA174

**Table 31a - Acute T-cell leukaemia**

| MW (Da) | Subfractionation | Tryptics identified [SEQ ID No] |
|---|---|---|
| | | CQGQLEVYLK [107], HLPETEAGR [337], LSQCHELWER [490], LSWYDPDFQAR [493], NSYCKK [554], QGAQWAALCDSSSAR [573], SHAENPTASHVDNEYSQPPR [634], TTPPTTRPPPTTTPEPTAPPR [734], VLALLCSGFQPK [771], WEEVCR [826] |
| | | HLPETEAGR [337], NSYCKK [554], QGAQWAALCDSSSAR [573], VLDAGDPTSR [775] |
| | | VLDAGDPTSR [775] |

**Table 31b - Chronic lymphocytic leukaemia**

| MW (Da) | Subfractionation | Tryptics identified [SEQ ID No] |
|---|---|---|
| 52248 | | HLPETEAGR [337], LSWYDPDFQAR [493], TQDLENFLCNNLQCGSFLK [723] |
| 53229 | | LSWYDPDFQAR [493] |
| 70807 | | AQDPGEPR [81], HLPETEAGR [337], IQNSSCTSLEHCFRK [390], LSWYDPDFQAR [493], SHAENPTASHVDNEYSQPPR [634] |
| 72277 | | EHQPLPIQWK [194], HLPETEAGR [337], LSQCHELWER [490], LSWYDPDFQAR [493], SHAENPTASHVDNEYSQPPR [634], VLDAGDPTSR [775] |
| 73777 | | HLPETEAGR [337], LSWYDPDFQAR [493], SHAENPTASHVDNEYSQPPR [634], VLDAGDPTSR [775] |

### OGTA176

**Table 32a - B-cell non-Hodgkin's lymphoma**

| MW (Da) | Subfractionation | Tryptics identified [SEQ ID No] |
|---|---|---|
| 33825 | | ELAQSQEALQVEQR [202], ETLQSEEQQR [231], ITQLGQSAEDLQGSR [396], ITQLGQSAEDLQGSRR [397], YLQVSQQLQQTNR [862] |

**Table 32b - Chronic lymphocytic leukaemia**

| MW (Da) | Subfractionation | Tryptics identified [SEQ ID No] |
|---|---|---|
| 38233 | | ELAQSQEALQVEQR [202], ETLQSEEQQR [231], ETLQSEEQQRR [232], ITQLGQSAEDLQGSR [396], SEQPTASWR [630], SSLPYICEMTAFR [650], YLQVSQQLQQTNR [862] |
| 39836 | | ELAQSQEALQVEQR [202], ITQLGQSAEDLQGSR [396] |
| 40663 | | ELAQSQEALQVEQR [202], ITQLGQSAEDLQGSR [396] |

### OGTA177

**Table 33 - Chronic lymphocytic leukaemia**

| MW (Da) | Subfractionation | Tryptics identified [SEQ ID No] |
|---|---|---|
| 81514 | | FLNLTSEK [259], MESEELADR [514], SLSNYPFDFQGAR [643] |
| 81757 | | SLSNYPFDFQGAR [643] |
| 83454 | | DIQVASNEILR [131], FLNLTSEK [259], SLSNYPFDFQGAR [643], SQHQETPVYLGATAGMR [647], YGIVLDAGSSHTSLYIYK [853], VTEMMK [801] |
| 83957 | | DIQVASNEILR [131], SLSNYPFDFQGAR [643], SQHQETPVYLGATAGMR [647], VTEMMKK [802] |
| 85186 | | SLSNYPFDFQGAR [643] |
| 86534 | | SQHQETPVYLGATAGMR [647] |
| 86954 | | DIQVASNEILR [131], SLSNYPFDFQGAR [643] |

### OGTA197

**Table 34a - Colorectal cancer**

| MW (Da) | Subfractionation | Tryptics identified [SEQ ID No] |
|---|---|---|
| 93409 | | QSPEDVYFSK [587], TYVDPHTYEDPNQAVLK [742], WTPPQDSGGR [837], YKPMMIITEYMENGALDK [857] |
| 95846 | | MQQYTEHFMAAGYTAIEK [523], NGVSGLVTSR [538], VIGAGEFGEVYK [769], WTPPQDSGGR [837], YKPMMIITEYMENGALDK [857] |
| 101142 | | QSPEDVYFSK [587], TSVTVSDLEPHMNYTFTVEAR [733], YKPMMIITEYMENGALDK [857] |

**Table 34b - Hepatocellular carcinoma**

| MW (Da) | Subfractionation | Tryptics identified [SEQ ID No] |
|---|---|---|
| 75736 | | AINDGFR [73], LPSTSGSEGVPFR [481], TLADFDPR [702], YLANMNYVHR [860], YSEPPHGLTR [871] |
| 82606 | | AINDGFR [73], EVVLLDFAAAGGELGWLTHPYGK [239], IDTIAPDEITVSSDFEAR [359], LPSTSGSEGVPFR [481], RKNQR [605], TYVDPHTYEDPNQAVLK [742], VDFLGEAGIMGQFSHHNIIR [747], VVQMTNDDIK [813], YLANMNYVHR [860], YSEPPHGLTR [871] |
| 84442 | | AINDGFR [73], FTTEIHPSCVTR [278], IDTIAPDEITVSSDFEAR [359], LPSTSGSEGVPFR [481], MQQYTEHFMAAGYTAIEK [523], TYVDPHTYEDPNQAVLK [742], VVQMTNDDIKR [814], YKPMMIITEYMENGALDK [857], YLANMNYVHR [860], YSEPPHGLTR [871] |
| 86381 | | AINDGFR [73], QSPEDVYFSK [587] |
| 88432 | | AINDGFR [73], LPGHQKR [478], QSPEDVYFSK [587], VIGAGEFGEVYK [769], VSDFGLSR [796], VVQMTNDDIK [813], VVQMTNDDIKR [814], WTAPEAISYR [834], WTAPEAISYRK [835], YKPMMIITEYMENGALDK [857], YSEPPHGLTR [871] |
| 92956 | | KGDSNSYNVR [417], QSPEDVYFSK [587], RKNQR [605], VIGAGEFGEVYK [769], VVQMTNDDIK [813], WTAPEAISYR [834], YLANMNYVHR [860], YSEPPHGLTR [871] |
| 116695 | | LPSTSGSEGVPFR [481], MELQAAR [512], MQQYTEHFMAAGYTAIEK [523], TLADFDPR [702], VYYKK [822], YKPMMIITEYMENGALDK [857] |

**Table 34c - Lung cancer**

| MW (Da) | Subfractionation | Tryptics identified [SEQ ID No] |
|---|---|---|
| | | DQVNTVGIPI [154], EVPVAIK [238], FTTEIHPSCVTR [278], IDTIAPDEITVSSDFEAR [359], KKGDSNSYNVR [422], VIGAGEFGEVYK [769], VLEDDPEATYTTSGGK [777], VVQMTNDDIK [813], VVQMTNDDIKR [814], YKPMMIITEYMENGALDK [857] |
| | | KEVPVAIK [414], LPSTSGSEGVPFR [481], TSVTVSDLEPHMNYTFTVEAR [733], VLEDDPEATYTTSGGK [777], WTPPQDSGGR [837], YEVTYR [845], YLANMNYVHR [860], YSEPPHGLTR [871] |
| 113648 | | DGEFSVLQLVGMLR [121], LPSTSGSEGVPFR [481], MQQYTEHFMAAGYTAIEK [523], QSPEDVYFSK [587], RKNQR [605], VDFLGEAGIMGQFSHHNIIR [747], VIGAGEFGEVYK [769], VVQMTNDDIK [813], WTAPEAISYRK [835], YKPMMIITEYMENGALDK [857], YLANMNYVHR [860] |
| 116436 | | AINDGFR [73], FADIVSILDK [242], FTTEIHPSCVTR [278], GDSNSYNVRR [288], LPSTSGSEGVPFR [481], MQQYTEHFMAAGYTAIEK [523], NGVSGLVTSR [538], TASVSINQTEPPK [673], TLADFDPR [702], TVSEWLESIK [738], VIGAGEFGEVYK [769], WTPPQDSGGR [837], YEVTYR [845], YKPMMIITEYMENGALDK [857], YLANMNYVHR [860] |
| 122412 | | LPSTSGSEGVPFR [481], VYYKK [822], YLANMNYVHR [860], QSPEDVYFSK [587] |

**Table 34d - Melanoma**

| MW (Da) | Subfractionation | Tryptics identified [SEQ ID No] |
|---|---|---|
| 78319 | | APQDPASMPCTRPPSAPHYLTAVGMGAK [80], DGEFSVLQLVGMLR [121], EVPVAIK [238], FADIVSILDK [242], MELQAAR [512], STTSLSVSWSIPPPQQSR [660], SVGPLTR [662], TASVSINQTEPPK [673], TSVTVSDLEPHMNYTFTVEAR [733], VIGAGEFGEVYK [769], YKPMMIITEYMENGALDK [857], YLANMNYVHR [860] |

**Table 34e - Osteosarcoma**

| MW (Da) | Subfractionation | Tryptics identified [SEQ ID No] |
|---|---|---|
| 30076 | | LPSTSGSEGVPFR [481], VVQMTNDDIK [813], VVQMTNDDIKR [814], YEVTYRK [846], YKPMMIITEYMENGALDK [857], YLANMNYVHR [860] |
| 55008 | | AINDGFR [73], APQDPASMPCTRPPSAPHYLTAVGMGAK [80], DGEFSVLQLVGMLR [121], IDTIAPDEITVSSDFEAR [359], RKNQR [605], STTSLSVSWSIPPPQQSR [660], SVGPLTR [662], TASVSINQTEPPK [673], TNWVYR [715], TSVTVSDLEPHMNYTFTVEAR [733], TYVDPHTYEDPNQAVLK [742], VLEDDPEATYTTSGGK [777], WTPPQDSGGR [837], YKPMMIITEYMENGALDK [857] |
| 67385 | | AINDGFR [73], IDTIAPDEITVSSDFEAR [359], KGDSNSYNVR [417], LPTPMDCPSAIYQLMMQCWQQER [483], MQQYTEHFMAAGYTAIEK [523], NGVSGLVTSR [538], STTSLSVSWSIPPPQQSR [660], SVGPLTR [662], TASVSINQTEPPK [673], TLADFDPR [702], TNWVYR [715], TSVTVSDLEPHMNYTFTVEAR [733], TYVDPHTYEDPNQAVLK [742], VDFLGEAGIMGQFSHHNIIR [747], WTPPQDSGGR [837], YSEPPHGLTR [871] |
| 93496 | | ACFALLWGCALAAAAAAQGK [55], AINDGFR [73], FTTEIHPSCVTR [278], IDTIAPDEITVSSDFEAR [359], RKNQR [605], STTSLSVSWSIPPPQQSR [660], SVGPLTR [662], TASVSINQTEPPK [673], TNWVYR [715], TYVDPHTYEDPNQAVLK [742], VIGAGEFGEVYK [769], VVQMTNDDIKR [814], YEVTYRK [846], YLANMNYVHR [860], YSEPPHGLTR [871] |

**Table 34f - Ovarian cancer**

| MW (Da) | Subfractionation | Tryptics identified [SEQ ID No] |
|---|---|---|
| 63557 | | AINDGFR [73], FTTEIHPSCVTR [278], LPSTSGSEGVPFR [481], STTSLSVSWSIPPPQQSR [660], TASVSINQTEPPK [673], TNWVYR [715], VVQMTNDDIKR [814], WTPPQDSGGR [837], YEVTYR [845], YLANMNYVHR [860], YSEPPHGLTR [871] |
| 67618 | | ACFALLWGCALAAAAAAQGK [55], QSPEDVYFSK [587], RKNQR [605], SVGPLTR [662], TASVSINQTEPPK [673], TLADFDPR [702], TSVTVSDLEPHMNYTFTVEAR [733], VDFLGEAGIMGQFSHHNIIR [747], WTPPQDSGGR [837], YKPMMIITEYMENGALDK [857] |
| 69811 | | FADIVSILDK [242], KGDSNSYNVRR [418], RKNQR [605], TASVSINQTEPPK [673], TYVDPHTYEDPNQAVLK [742], VSDFGLSR [796] |
| 79417 | | ACFALLWGCALAAAAAAQGK [55], DGEFSVLQLVGMLR [121], IDTIAPDEITVSSDFEAR [359], SVGPLTR [662], TSVTVSDLEPHMNYTFTVEAR [733], TYVDPHTYEDPNQAVLK [742], VIGAGEFGEVYK [769], YKPMMIITEYMENGALDK [857], YLANMNYVHR [860], YSEPPHGLTR [871] |

**Table 34g - Pancreatic cancer**

| MW (Da) | Subfractionation | Tryptics identified [SEQ ID No] |
|---|---|---|
| 53057 | | AINDGFR [73], IDTIAPDEITVSSDFEAR [359], LEGVISK [454], LNVEER [474], LPSTSGSEGVPFR [481], SVGPLTR [662], VIGAGEFGEVYK [769], VVQMTNDDIK [813], YEVTYRKK [847] |
| 53737 | | IDTIAPDEITVSSDFEAR [359], LPSTSGSEGVPFR [481], SEQLKPLK [629], SVGPLTRK [663], YLANMNYVHR [860] |
| 93964 | | LPSTSGSEGVPFR [481], TVSEWLESIK [738], WTPPQDSGGR [837], YSEPPHGLTR [871] |
| 102286 | | VIGAGEFGEVYK [769], VVQMTNDDIKR [814], WTPPQDSGGR [837], YSEPPHGLTR [871] |
| 107693 | | KGDSNSYNVR [417], QSPEDVYFSK [587], TNWVYR [715], TYVDPHTYEDPNQAVLK [742], VIGAGEFGEVYK [769], VVQMTNDDIKR [814], WTPPQDSGGR [837], YLANMNYVHR [860], YSEPPHGLTR [871] |
| 110489 | | TASVSINQTEPPK [673], TLADFDPR [702], TYVDPHTYEDPNQAVLK [742], VIGAGEFGEVYK [769], WTPPQDSGGR [837] |
| 113324 | | FADIVSILDK [242], IDTIAPDEITVSSDFEAR [359], KGDSNSYNVR [417], SVGPLTR [662], TLADFDPR [702], TYVDPHTYEDPNQAVLK [742], VYYKK [822], WTPPQDSGGR [837], YEVTYR [845], YEVTYRK [846], YLANMNYVHR [860], YSEPPHGLTR [871] |
| 116302 | | IDTIAPDEITVSSDFEAR [359], TYVDPHTYEDPNQAVLK [742], VVQMTNDDIK [813], WTPPQDSGGR [837], YEVTYR [845], YLANMNYVHR [860], YSEPPHGLTR [871] |
| 116866 | | ACFALLWGCALAAAAAAQGK [55], AINDGFR [73], IDTIAPDEITVSSDFEAR [359], LPSTSGSEGVPFR [481], STTSLSVSWSIPPPQQSR [660], TYVDPHTYEDPNQAVLK [742], VIGAGEFGEVYK [769], VLEDDPEATYTTSGGK [777], WTAPEAISYR [834], WTPPQDSGGR [837], YKPMMIITEYMENGALDK [857], YLANMNYVHR [860], YSEPPHGLTR [871] |
| 122219 | | ACFALLWGCALAAAAAAQGK [55], AINDGFR [73], IDTIAPDEITVSSDFEAR [359], KGDSNSYNVR [417], LPSTSGSEGVPFR [481], MQQYTEHFMAAGYTAIEK [523], QSPEDVYFSK [587], STTSLSVSWSIPPPQQSR [660], TNWVYR [715], VIGAGEFGEVYK [769], WTAPEAISYR [834], WTPPQDSGGR [837], YKPMMIITEYMENGALDK [857], YSEPPHGLTR [871] |
| 180074 | | KGDSNSYNVR [417], KGDSNSYNVRR [418], LPSTSGSEGVPFR [481], MELQAAR [512], QSPEDVYFSK [587], TNWVYR [715], WTAPEAISYR [834], WTPPQDSGGR [837], YEVTYR [845], YSEPPHGLTR [871] |
| 184373 | | AINDGFR [73], LPSTSGSEGVPFR [481], QSPEDVYFSK [587], TNWVYR [715], WTAPEAISYR [834], WTPPQDSGGR [837], YEVTYR [845], YLANMNYVHR [860], YSEPPHGLTR [871] |
| 188775 | | LPSTSGSEGVPFR [481], TNWVYR [715], VIGAGEFGEVYK [769], VVQMTNDDIK [813], WTAPEAISYR [834], WTPPQDSGGR [837], YLANMNYVHR [860], YSEPPHGLTR [871] |

**Table 34h - Prostate cancer**

| MW (Da) | Subfractionation | Tryptics identified [SEQ ID No] |
|---|---|---|
| | | LPSTSGSEGVPFR [481], MELQAAR [512], YEVTYR [845] |
| | | LPSTSGSEGVPFR [481], NGVSGLVTSR [538], VVQMTNDDIK [813], WTPPQDSGGR [837], YSEPPHGLTR [871] |

**Table 34i - Renal cell cancer**

| MW (Da) | Subfractionation | Tryptics identified [SEQ ID No] |
|---|---|---|
| 88378 | | AINDGFR [73], KGDSNSYNVRR [418], LPSTSGSEGVPFR [481], TNWVYR [715], WTAPEAISYR [834], WTPPQDSGGR [837], YSEPPHGLTR [871] |
| 90442 | | AINDGFR [73], LPSTSGSEGVPFR [481], SVGPLTR [662], TNWVYR [715], TYVDPHTYEDPNQAVLK [742], VIGAGEFGEVYK [769], VVQMTNDDIKR [814], WTAPEAISYR [834], WTPPQDSGGR [837], YEVTYR [845], YLANMNYVHR [860], YSEPPHGLTR [871] |
| 117578 | | AINDGFR [73], TNWVYR [715], VIGAGEFGEVYK [769], VVQMTNDDIK [813], VVQMTNDDIKR [814], WTAPEAISYR [834], YLANMNYVHR [860], YSEPPHGLTR [871] |
| 122597 | | ACFALLWGCALAAAAAAQGK [55], TYVDPHTYEDPNQAVLK [742], VIGAGEFGEVYK [769], VVQMTNDDIKR [814], WTAPEAISYR [834], YEVTYRK [846], YLANMNYVHR [860], YSEPPHGLTR [871] |

### OGTA202

**Table 35 - Colorectal cancer**

| MW (Da) | Subfractionation | Tryptics identified [SEQ ID No] |
|---|---|---|
| 51531 | Nucleotide Binding | FTQDTFR [277] |

### OGTA203

**Table 36a - Ovarian cancer**

| MW (Da) | Subfractionation | Tryptics identified [SEQ ID No] |
|---|---|---|
| 69811 | | DNIVSYNDEGGGEEDTQAFDIGTLR [144], EDAQINTTIGSVTAQDPDAAR [168], RTPTAR [618], TALLNMDR [669], TPESSPPGTPIGR [716], TSGFPAKK [727], VEASNPYVEPR [751] |

**Table 36b - Renal cell cancer**

| MW (Da) | Subfractionation | Tryptics identified [SEQ ID No] |
|---|---|---|
| 82732 | | IVVEDVDEPPVFSK [402], KNGYNR [428], TSGFPAKK [727] |

### OGTA206

**Table 37a - Breast cancer**

| MW (Da) | Subfractionation | Tryptics identified [SEQ ID No] |
|---|---|---|
| 27751 | Vesicles | VYDSLLALPQDLQAAR [817] |

**Table 37b - Pancreatic cancer**

| MW (Da) | Subfractionation | Tryptics identified [SEQ ID No] |
|---|---|---|
| 26738 | | VYDSLLALPQDLQAAR [817] |
| 27019 | | VYDSLLALPQDLQAAR [817] |

| Table 37c - Prostate cancer | | |
|---|---|---|
| MW (Da) | Subfractionation | Tryptics identified [SEQ ID No] |
| | Digitonin | VYDSLLALPQDLQAAR [817] |

### OGTA213

**Table 38 - Lung cancer**

| MW (Da) | Subfractionation | Tryptics identified [SEQ ID No] |
|---|---|---|
| 27644 | | IVFTPTICK [401] |

### OGTA214

**Table 39 - Ovarian cancer**

| MW (Da) | Subfractionation | Tryptics identified [SEQ ID No] |
|---|---|---|
| 65600 | | QCLIKK [565], SSSSNFLNYDLTLR [651] |
| 79331 | | ISVTVSETFDPEEK [394], SSSSNFLNYDLTLR [651] |
| 89348 | | ISVTVSETFDPEEK [394], SSSSNFLNYDLTLR [651] |

### OGTA216

**Table 40a - Breast cancer**

| MW (Da) | Subfractionation | Tryptics identified [SEQ ID No] |
|---|---|---|
| 196008 | | AFYAPVHADDLR [69], EGAQYLMQAAGLGR [186], FQVDLVSENAGR [271], GPIVPLNVADQK [307], IDHDRR [356], KDWLQADMR [408], NVGLMICGHVHMGPR [555], SEIFNENFGPDFR [626] |
| 248063 | | DEGPAAAGDGLGRPLGPTPSQSR [114], DVVVSVEYSK [163], EGAQYLMQAAGLGR [186], FQVDLVSENAGR [271], NVGLMICGHVHMGPRR [556], SEIFNENFGPDFR [626] |

**Table 40b - Colorectal cancer**

| MW (Da) | Subfractionation | Tryptics identified [SEQ ID No] |
|---|---|---|
| | Heparin Binding | DAVVTYTAESK [110], DVVVSVEYSK [163], SEIFNENFGPDFR [626] |
| | Heparin Binding | KENIIAFEEIIEPYR [410], SEIFNENFGPDFR [626] |
| | Heparin Binding | KENIIAFEEIIEPYR [410], LNELLK [471], SEIFNENFGPDFR [626] |
| | Heparin Binding | AFYAPVHADDLR [69], EHSSTANIIVMSLPVAR [195], FQVDLVSENAGR [271], SEIFNENFGPDFR [626], SPGWRPAFK [645] |
| | Heparin Binding | DEGPAAAGDGLGRPLGPTPSQSR [114], GFFGYK [294], KENIIAFEEIIEPYR [410], SEIFNENFGPDFR [626] |
| | Heparin Binding | FQVDLVSENAGR [271], GGGAYYLISR [298], KENIIAFEEIIEPYR [410], SEIFNENFGPDFR [626] |
| | Heparin Binding | AAAAAAAAAAAAAAAGAGAGAK [49], DLPPILLVR [136], DNIYPAFQMFAK [145], EGAQYLMQAAGLGR [186], EHSSTANIIVMSLPVAR [195], EPFEDGFANGEESTPTR [216], FQVDLVSENAGR [271], IDFSDIMVLGDINTKPK [354], IDHYR [357], KENIIAFEEIIEPYR [410], SEIFNENFGPDFR [626] |
| | Heparin Binding | CMLNIWGVMLFIR [106], IDHDRR [356], ITDNELELYK [395], SEIFNENFGPDFR [626] |
| | Heparin Binding | DLPPILLVR [136], NVGLMICGHVHMGPR [555], SEIFNENFGPDFR [626] |
| | Heparin Binding | AMATLLSK [78], EGAQYLMQAAGLGR [186], GGGAYYLISR [298], GPIVPLNVADQK [307], NVGLMICGHVHMGPRR [556], SPGWRPAFK [645] |
| 121506 | | DEGPAAAGDGLGRPLGPTPSQSR [114], DLPPILLVR [136], DNIYPAFQMFAK [145], DWLQADMR [164], EGAQYLMQAAGLGR [186], EPFEDGFANGEESTPTR [216], IDFSDIMVLGDINTKPKK [355], IDHYR [357], KENIIAFEEIIEPYR [410], KSDLDTSKPLSEKPITHK [435], LNELLK [471], NVGLMICGHVHMGPRR [556], SEIFNENFGPDFR [626], TFGHNTMDAVPR [688], VELPGTAVPSVPEDAAPASR [754] |
| 125772 | | DLPPILLVR [136], DWLQADMR [164], EPFEDGFANGEESTPTR [216], KDWLQADMR [408], SEIFNENFGPDFR [626], SPGWRPAFK [645], TFGHNTMDAVPR [688], VELPGTAVPSVPEDAAPASR [754] |
| 140688 | | DLPPILLVR [136], DWLQADMR [164], EGAQYLMQAAGLGR [186], EPFEDGFANGEESTPTR [216], KDWLQADMR [408], KSDLDTSKPLSEKPITHK [435], NVGLMICGHVHMGPR [555], TFGHNTMDAVPR [688], VELPGTAVPSVPEDAAPASR [754] |
| 146522 | | DLPPILLVR [136], DWLQADMR [164], EGAQYLMQAAGLGR [186], EPFEDGFANGEESTPTR [216], IDHDRR [356], KDWLQADMR [408], KENIIAFEEIIEPYR [410], KSDLDTSKPLSEKPITHK [435], SEIFNENFGPDFR [626], TFGHNTMDAVPR [688], VELPGTAVPSVPEDAAPASR [754] |
| 167535 | | DWLQADMR [164], EPFEDGFANGEESTPTR [216], GPIVPLNVADQK [307], IDHDRR [356], SEIFNENFGPDFR [626], TFGHNTMDAVPR [688], VELPGTAVPSVPEDAAPASR [754] |
| 175447 | Nucleotide Binding | NNEPLR [549], SEIFNENFGPDFR [626], TFGHNTMDAVPR [688], VELPGTAVPSVPEDAAPASR [754] |
| 190098 | Nucleotide Binding | DAVVTYTAESK [110], EGAQYLMQAAGLGR [186], EMSIDQAK [213], LHEDDK [465], LNELLK [471], RAMATLLSK [596], SEIFNENFGPDFR [626], SPGWRPAFK [645], TFGHNTMDAVPR [688] |
| 218400 | Nucleotide Binding | DEGPAAAGDGLGRPLGPTPSQSR [114], EGAQYLMQAAGLGR [186], KENIIAFEEIIEPYR [410], KSDLDTSKPLSEKPITHK [435], SEIFNENFGPDFR [626] |
| 221484 | | DEGPAAAGDGLGRPLGPTPSQSR [114], EGAQYLMQAAGLGR [186], EPFEDGFANGEESTPTR [216], IDHDRR [356], KDWLQADMR [408], NVGLMICGHVHMGPRR [556], SEIFNENFGPDFR [626], TFGHNTMDAVPR [688] |
| 255000 | | EGAQYLMQAAGLGR [186], EPFEDGFANGEESTPTR [216], SEIFNENFGPDFR [626], TFGHNTMDAVPR [688], VELPGTAVPSVPEDAAPASR [754] |
| 301246 | | DEGPAAAGDGLGRPLGPTPSQSR [114], EGAQYLMQAAGLGR [186], EPFEDGFANGEESTPTR [216], IDHDRR [356], IDHYR [357], SEIFNENFGPDFR [626], TFGHNTMDAVPR [688] |

**Table 40c - Pancreatic cancer**

| MW (Da) | Subfractionation | Tryptics identified [SEQ ID No] |
|---|---|---|
| 148609 | | AFYAPVHADDLR [69], DVVVSVEYSK [163], GGGAYYLISR [298], NNEPLR [549], SEIFNENFGPDFR [626], VELPGTAVPSVPEDAAPASR [754] |
| 154726 | | DVVVSVEYSK [163], EPFEDGFANGEESTPTR [216], GPIVPLNVADQK [307], NVGLMICGHVHMGPR [555], SEIFNENFGPDFR [626] |

### OGTA222

**Table 41a - B-cell non-Hodgkin's lymphoma**

| MW (Da) | Subfractionation | Tryptics identified [SEQ ID No] |
|---|---|---|
| 45662 | | AVGFGGDFDGVPR [95], TLEQMDVVHR [703] |
| 48028 | | ALYQLGMR [77], AVGFGGDFDGVPR [95], GALADNLLR [283], MYPETFLYVTSSAGIR [530], NVPDDVLR [559], RTLEQMDVVHR [617], TLEQMDVVHR [703], YPDLIAELLR [864], VPEGLEDVSK [791] |
| 48530 | | ALYQLGMR [77], AVGFGGDFDGVPR [95], GALADNLLR [283], MYPETFLYVTSSAGIR [530], TLEQMDVVHR [703], VPEGLEDVSK [791] |
| 49042 | | ALYQLGMR [77], AVGFGGDFDGVPR [95], GALADNLLR [283], MYPETFLYVTSSAGIR [530], TLEQMDVVHR [703], VPEGLEDVSK [791] |
| 66777 | | ALYQLGMR [77], AVGFGGDFDGVPR [95], GALADNLLR [283], TLEQMDVVHR [703], ATLQLSR [91] |
| 68315 | | ALYQLGMR [77], AVGFGGDFDGVPR [95], GALADNLLR [283], TLEQMDVVHR [703] |
| 69923 | | ALYQLGMR [77], ATLQLSR [91], AVGFGGDFDGVPR [95], GALADNLLR [283], TLEQMDVVHR [703] |
| 71607 | | ALYQLGMR [77], AVGFGGDFDGVPR [95], GALADNLLR [283], TLEQMDVVHR [703], VPEGLEDVSK [791] |

**Table 41b - Colorectal cancer**

| MW (Da) | Subfractionation | Tryptics identified [SEQ ID No] |
|---|---|---|
| 52940 | | ALYQLGMR [77], AVGFGGDFDGVPR [95], GALADNLLR [283], TLEQMDVVHR [703] |
| 55204 | | AVGFGGDFDGVPR [95] |
| 58558 | | AVGFGGDFDGVPR [95], TLEQMDVVHR [703] |

### OGTA236

**Table 42 - Pancreatic cancer**

| MW (Da) | Subfractionation | Tryptics identified [SEQ ID No] |
|---|---|---|
| 73411 | | SVLGVITIVNLR [664] |

### OGTA237

**Table 43a - B-cell non-Hodkin's lymphoma**

| MW (Da) | Subfractionation | Tryptics identified [SEQ ID No] |
|---|---|---|
| 47536 | | IADFGLAR [349] |
| 48028 | | IADFGLAR [349] |
| 48530 | | IADFGLAR [349] |
| 49565 | | IADFGLAR [349] |
| 50098 | | IADFGLAR [349] |
| 51766 | | IADFGLAR [349] |
| 52346 | | IADFGLAR [349] |

**Table 43b - Lymphoid leukaemia, unspecified**

| MW (Da) | Subfractionation | Tryptics identified [SEQ ID No] |
|---|---|---|
| 52041 | | IADFGLAR [349] |
| 52618 | | IADFGLAR [349] |
| 55054 | | IADFGLAR [349] |

**Table 43c - Prostate cancer**

| MW (Da) | Subfractionation | Tryptics identified [SEQ ID No] |
|---|---|---|
| | | IADFGLAR [349] |
| | | IADFGLAR [349] |

### OGTA247

**Table 44a - Hepatocellular carcinoma**

| MW (Da) | Subfractionation | Tryptics identified [SEQ ID No] |
|---|---|---|
| 102119 | | DATQITQGPR [109], EDTQVDSEARPMK [170], GYNVTYWR [330], STIEKK [659], YDIEFEDK [842], YFIEDGR [850] |
| 114377 | | DATQITQGPR [109], FFPYANGTLGIR [251], FHILFK [253], GYNVTYWR [330], LGTAMSHEIR [463], LSPYVHYTFR [489], STIEKK [659], VSWSPAEDHNAPIEK [798], VTFTCQASFDPSLQPSITWR [804], VTYQNHNK [808], WLRPSGPMPADR [828], WMDWNAPQVQYR [829], YDIEFEDK [842] |
| 116037 | | DATQITQGPR [109], EPIDLR [217], FQGIYR [266], LDCQVQGRPQPEVTWR [445], LGTAMSHEIR [463], YFIEDGR [850] |
| 118513 | | AQLLVVGSPGPVPR [82], ATNSMIDR [92], DATQITQGPR [109], EGPGEAIVR [189], FFPYANGTLGIR [251], LGTAMSHEIR [463], LSPYVHYTFR [489], VTYQNHNK [808], WLRPSGPMPADR [828], YFIEDGR [850] |
| 121636 | | DATQITQGPR [109], EPIDLR [217], FHILFK [253], FQGIYR [266], YDIEFEDK [842], YFIEDGR [850] |
| 123033 | | AQLLVVGSPGPVPR [82], CEASGKPEVQFR [101], DATQITQGPR [109], EGPGEAIVR [189], FFPYANGTLGIR [251], LSPYVHYTFR [489], VGEEDDGEYR [762], VQWRPQGTR [795], YFIEDGR [850] |
| 128000 | | AQLLVVGSPGPVPR [82], DATQITQGPR [109], LGTAMSHEIR [463], LSPYVHYTFR [489], YFIEDGR [850] |
| 200565 | | CEASGKPEVQFR [101], EPIDLR [217], INGIPVEELAK [382], VSWSPAEDHNAPIEK [798], WLRPSGPMPADR [828] |
| 209214 | | ATNSMIDR [92], DATQITQGPR [109], EGPGEAIVR [189], EPIDLR [217], GALILSNVQPSDTMVTQCEAR [284], HQMAVK [342] |
| 218613 | | AQLLVVGSPGPVPR [82], CEASGKPEVQFR [101], DLQELGDSDK [137], FFPYANGTLGIR [251], LGTAMSHEIR [463], WLRPSGPMPADR [828] |

**Table 44b - Melanoma**

| MW (Da) | Subfractionation | Tryptics identified [SEQ ID No] |
|---|---|---|
| 146479 | | AQLLVVGSPGPVPR [82], CEASGKPEVQFR [101], CLAENSLGSAR [105], DATQITQGPR [109], DGVHFKPK [126], EGPGEAIVR [189], EPIDLR [217], FQGIYR [266], GQLSFNLR [312], INGIPVEELAK [382], LGTAMSHEIR [463], LSPYVHYTFR [489], LVLSDLHLLTQSQVR [502], VGEEDDGEYR [762], VSWSPAEDHNAPIEK [798], WLRPSGPMPADR [828], WMDWNAPQVQYR [829], WRPVDLAQVK [831], YFIEDGR [850] |
| 152349 | | AQLLVVGSPGPVPR [82], CLAENSLGSAR [105], DATQITQGPR [109], DGVHFKPK [126], EGPGEAIVR [189], EPIDLR [217], FQGIYR [266], LGTAMSHEIR [463], LSPYVHYTFR [489], LVLSDLHLLTQSQVR [502], WLRPSGPMPADR [828], WMDWNAPQVQYR [829], WRPVDLAQVK [831], YFIEDGR [850] |
| 163936 | | AFGAPVPSVQWLDEDGTTVLQDER [65], AQLLVVGSPGPVPR [82], CEASGKPEVQFR [101], FQGIYR [266], GQLSFNLR [312], LGTAMSHEIR [463], LVLSDLHLLTQSQVR [502], VSWSPAEDHNAPIEK [798], WLRPSGPMPADR [828], WMDWNAPQVQYR [829], WRPVDLAQVK [831], YFIEDGR [850] |
| 172991 | | AFGAPVPSVQWLDEDGTTVLQDER [65], AQLLVVGSPGPVPR [82], CEASGKPEVQFR [101], CLAENSLGSAR [105], DHVVVPANTTSVILSGLRPYSSYHLEVQAFNGR [128], EELGVTVYQSPHSGSFTITGNNSNFAQR [178], EGPGEAIVR [189], EPIDLR [217], FHILFK [253], FQGIYR [266], GQLSFNLR [312], LVLSDLHLLTQSQVR [502], VGEEDDGEYR [762], VQWRPQGTR [795], VSWSPAEDHNAPIEK [798], WLRPSGPMPADR [828], WMDWNAPQVQYR [829], WRPVDLAQVK [831], YFIEDGR [850] |
| 193221 | | AFGAPVPSVQWLDEDGTTVLQDER [65], AQLLVVGSPGPVPR [82], FFPYANGTLGIR [251], FHILFK [253], FQGIYR [266], GALILSNVQPSDTMVTQCEAR [284], GEGNETTNMVITWKPLR [289], GQLSFNLR [312], INGIPVEELAK [382], LGTAMSHEIR [463], LVLSDLHLLTQSQVR [502], WLRPSGPMPADR [828], WMDWNAPQVQYR [829], WRPVDLAQVK [831] |

### OGTA248

**Table 45 - Renal cell cancer**

| MW (Da) | Subfractionation | Tryptics identified [SEQ ID No] |
|---|---|---|
| 34832 | | TNQEVLDFVVGGGR [712] |

### OGTA249

**Table 46 - Ovarian cancer**

| MW (Da) | Subfractionation | Tryptics identified [SEQ ID No] |
|---|---|---|
| | | VTLPAGPDLLR [805] |
| | | MSAGGASVPPPPNPAVSFPPPR [524] |

### OGTA257

**Table 47 - Pancreatic cancer**

| MW (Da) | Subfractionation | Tryptics identified [SEQ ID No] |
|---|---|---|
| 176135 | | LLQEAYMTPK [468] |

### OGTA271

**Table 48a - Breast cancer**

| MW (Da) | Subfractionation | Tryptics identified [SEQ ID No] |
|---|---|---|
| 24841 | Vesicles | AHTDVGPGPESSPVLVR [72], AYIATQGPLAESTEDFWR [98], DHPPIPITDLADNIER [127], DSLLAHSSDPVEMRR [157], GVEGSDYINASFLDGYR [326], LGQVPPGESVTAMELEFK [462], LIADLQPNTEYSFVLMNR [467], MLWEHNSTIIVMLTK [519], QFQFTDWPEQGVPK [572], QNAYIATQGPLPETMGDFWR [581], SDMGVGVFTPTIEAR [621], YEGVVDMFQTVK [844], YQYFVVDPMAEYNMPQYILR [869] |
| 24993 | Vesicles | AALEYLGSFDHYAT [51], AYIATQGPLAESTEDFWR [98], DHPPIPITDLADNIER [127], DINSQQELQNITTDTR [129], DSLLAHSSDPVEMRR [157], FDLSMPHVQDPSLVR [246], GVEGSDYINASFLDGYR [326], HVVDGISR [347], ITWMKK [399], KQNAYIATQGPLPETMGDFWR [433], LGQVPPGESVTAMELEFK [462], LIADLQPNTEYSFVLMNR [467], LSVLEEEQLPPGFPSIDMGPQLK [492], MLSASTMLVQWEPPEEPNGLVR [518], MLWEHNSTIIVMLTK [519], NGVITQYSVAYEAVDGEDR [537], QFQFTDWPEQGVPK [572], TGEGFIDFIGQVHK [693], VPEDQTGLSGGVASFVCQATGEPKPR [789], YQYFVVDPMAEYNMPQYILR [869] |
| 55008 | | MVWEQR [529], YQYFVVDPMAEYNMPQYILR [869] |
| 55921 | | AALEYLGSFDHYAT [51], AYIATQGPLAESTEDFWR [98], DFLPVDPATSNGR [117], DHPPIPITDLADNIER [127], EHSSWDLVGLEK [196], GVEGSDYINASFLDGYR [326], HVVDGISR [347], ILYNGQSVEVDGHSMRK [379], KLIADLQPNTEYSFVLMNR [424], LENGEPR [455], LGQVPPGESVTAMELEFK [462], LNYQTPGMR [475], MLWEHNSTIIVMLTK [519], MVWEQR [529], NGVITQYSVAYEAVDGEDR [537], QFQFTDWPEQGVPK [572], RLNYQTPGMR [606], TGEGFIDFIGQVHK [693], YEGVVDMFQTVK [844] |
| 56324 | | DFLPVDPATSNGR [117], DHPPIPITDLADNIER [127], ILYNGQSVEVDGHSMR [378], LNYQTPGMR [475], MLWEHNSTIIVMLTK [519], QHGQIR [578], RLNYQTPGMR [606], TDEDVPSGPPRK [683], TVDIYGHVTCMR [735], VMCVSMGSTTVR [785], YEGVVDMFQTVK [844] |
| 56872 | | AGLGEEFEK [71], AYIATQGPLAESTEDFWR [98], DHPPIPITDLADNIER [127], FDLSMPHVQDPSLVR [246], FTLTGLKPDTTYDIK [275], GSGPLSPSIQSR [315], GVEGSDYINASFLDGYR [326], LNYQTPGMR [475], MVWEQR [529], RPPNAWHK [609], SDMGVGVFTPTIEAR [621], TVDIYGHVTCMR [735], VLAVNSIGR [774] |
| 105325 | | AAGTEGPFQEVDGVATTR [50], ACNPLDAGPMVVHCSAGVGR [56], AYIATQGPLAESTEDFWR [98], DFLPVDPATSNGR [117], DINSQQELQNITTDTR [129], FDLSMPHVQDPSLVR [246], FEVIEFDDGAGSVLR [249], FTLTGLKPDTTYDIK [275], GPPSEAVR [308], GSGPLSPSIQSR [315], ILYNGQSVEVDGHSMR [378], MVWEQR [529], RPPNAWHK [609], SANYTCVAISSLGMIEATAQVTVK [619], SDMGVGVFTPTIEAR [621], TATMLCAAGGNPDPEISWFK [674], TATVVMMTR [676], TDEDVPSGPPRK [683], TGCFIVIDAMLER [692], TGEGFIDFIGQVHK [693], TSVLLSWEVPDSYK [732], VSWVPPPADSR [799] |
| 109891 | | AAGTEGPFQEVDGVATTR [50], ACNPLDAGPMVVHCSAGVGR [56], AYIATQGPLAESTEDFWR [98], DDQHFTVTGLHK [111], ELPGELLGYR [206], EQFGQDGPITVHCSAGVGR [220], FEVIEFDDGAGSVLR [249], ILYNGQSVEVDGHSMRK [379], KQNAYIATQGPLPETMGDFWR [433], SDMGVGVFTPTIEAR [621], YEGVVDMFQTVK [844] |

**Table 48b - Cervical cancer**

| MW (Da) | Subfractionation | Tryptics identified [SEQ ID No] |
|---|---|---|
| 118858 | Integral | AAGTEGPFQEVDGVATTR [50], AHTDVGPGPESSPVLVR [72], DDQHFTVTGLHK [111], DFLPVDPATSNGR [117], ELPGELLGYR [206], EPMDQKR [218], FEVIEFDDGAGSVLR [249], GSSAGGLQHLVSIR [321], QHGQIR [578], RPPNAWHK [609], TDEDVPSGPPR [682], TQQGVPAQPADFQAEVESDTR [725], TVDIYGHVTCMR [735], VSWVPPPADSR [799], WTEYR [836], YSIGGLSPFSEYAFR [872] |
| 124604 | Integral | AAGTEGPFQEVDGVATTR [50], ADEARPNTIDFGK [58], AHTDVGPGPESSPVLVR [72], DDQHFTVTGLHK [111], ELPGELLGYR [206], EPMDQKR [218], FDLSMPHVQDPSLVR [246], FEVIEFDDGAGSVLR [249], GSSAGGLQHLVSIR [321], NVLELSNVVR [558], RPPNAWHK [609], SDMGVGVFTPTIEAR [621], TQQGVPAQPADFQAEVESDTR [725], VLAVNSIGR [774], VSWVPPPADSR [799], VYYTPDSR [823], WTEYR [836], YSIGGLSPFSEYAFR [872] |

**Table 48c - Colorectal cancer**

| MW (Da) | Subfractionation | Tryptics identified [SEQ ID No] |
|---|---|---|
| | Heparin Binding | GSGPLSPSIQSR [315], MAPEPAPGR [509], SDMGVGVFTPTIEAR [621], TDEDVPSGPPR [682], TGEQAPSSPPR [694], TQQGVPAQPADFQAEVESDTR [725], YSAPANLYVR [870] |

**Table 48d - Hepatocellular carcinoma**

| MW (Da) | Subfractionation | Tryptics identified [SEQ ID No] |
|---|---|---|
| 72397 | | YANVIAYDHSR [840], YSAPANLYVR [870] |

**Table 48e - Lung cancer**

| MW (Da) | Subfractionation | Tryptics identified [SEQ ID No] |
|---|---|---|
| | | AAGTEGPFQEVDGVATTR [50], ADEARPNTIDFGK [58], AHTDVGPGPESSPVLVR [72], DSLLAHSSDPVEMR [156], EHSSWDLVGLEK [196], FEVIEFDDGAGSVLR [249], GSGPLSPSIQSR [315], GTTYIFR [324], ILYNGQSVEVDGHSMR [378], ILYNGQSVEVDGHSMRK [379], KLIADLQPNTEYSFVLMNR [424], KQNAYIATQGPLPETMGDFWR [433], SGALQIESSEESDQGK [631], TAPDLLPHKPLPASAYIEDGR [671], TATMLCAAGGNPDPEISWFK [674], TMPVEQVFAK [708], TQQGVPAQPADFQAEVESDTR [725], TQRPAMVQTEDQYQLCYR [726], TVDIYGHVTCMR [735], YEGVVDMFQTVK [844], YSAPANLYVR [870] |
| 77419 | | AYIATQGPLAESTEDFWR [98], DDQHFTVTGLHK [111], GPPSEAVR [308], GVEGSDYINASFLDGYR [326], IIMYELVYWAAEDEDQQHK [372], KQNAYIATQGPLPETMGDFWR [433], LGQVPPGESVTAMELEFK [462], QFQFTDWPEQGVPK [572], RTHSPSSK [616], YEGVVDMFQTVK [844] |
| 78771 | | DHPPIPITDLADNIER [127], DINSQQELQNITTDTR [129], DSLLAHSSDPVEMRR [157], EHSSWDLVGLEK [196], GVEGSDYINASFLDGYR [326], ILYNGQSVEVDGHSMRK [379], LGQVPPGESVTAMELEFK [462], MLSASTMLVQWEPPEEPNGLVR [518], MLWEHNSTIIVMLTK [519], MVWEQR [529], QNAYIATQGPLPETMGDFWR [581], TAPDLLPHKPLPASAYIEDGR [671], TGEQAPSSPPRR [695], YANVIAYDHSR [840], YEGVVDMFQTVK [844] |

**Table 48f - Osteosarcoma**

| MW (Da) | Subfractionation | Tryptics identified [SEQ ID No] |
|---|---|---|
| 63624 | | AAGTEGPFQEVDGVATTR [50], AHTDVGPGPESSPVLVR [72], AYIATQGPLAESTEDFWR [98], DDQHFTVTGLHK [111], ELPGELLGYR [206], FDLSMPHVQDPSLVR [246], FEVIEFDDGAGSVLR [249], FTLTGLKPDTTYDIK [275], GYQVTYVR [331], ILYNGQSVEVDGHSMR [378], IQLSWLLPPQER [388], LIADLQPNTEYSFVLMNR [467], MLSASTMLVQWEPPEEPNGLVR [518], NGVITQYSVAYEAVDGEDR [537], QHGQIR [578], RPPNAWHK [609], RRQAER [614], RTHSPSSK [616], SDMGVGVFTPTIEAR [621], TQQGVPAQPADFQAEVESDTR [725], VLAFTAVGDGPPSPTIQVK [770], VPEDQTGLSGGVASFVCQATGEPKPR [789], VTFDPTSSYTLEDLKPDTLYR [803], YEGVVDMFQTVK [844], YQYFVVDPMAEYNMPQYILR [869], YSIGGLSPFSEYAFR [872] |

**Table 48g - Pancreatic cancer**

| MW (Da) | Subfractionation | Tryptics identified [SEQ ID No] |
|---|---|---|
| 73243 | | DDQHFTVTGLHK [111], DSLLAHSSDPVEMRR [157], GVEGSDYINASFLDGYR [326], LGQVPPGESVTAMELEFK [462], RLNYQTPGMR [606], TATMLCAAGGNPDPEISWFK [674], TMPVEQVFAK [708], VGGSMLTPR [764], YEGVVDMFQTVK [844] |
| 74307 | | AYIATQGPLAESTEDFWR [98], DEAIYECTATNSLGEINTSAK [112], DSLLAHSSDPVEMRR [157], FDLSMPHVQDPSLVR [246], GPPSEAVR [308], GVEGSDYINASFLDGYR [326], IIMYELVYWAAEDEDQQHK [372], LNYQTPGMR [475], MVWEQR [529], SDMGVGVFTPTIEAR [621], TAQSTPSAPPQK [672], TDEDVPSGPPRK [683], TGCFIVIDAMLER [692], YANVIAYDHSR [840], YEGVVDMFQTVK [844], YSIGGLSPFSEYAFR [872] |
| 115291 | | AAGTEGPFQEVDGVATTR [50], DINSQQELQNITTDTR [129], DSLLAHSSDPVEMRR [157], EHSSWDLVGLEK [196], EQFGQDGPITVHCSAGVGR [220], FQLAAR [267], LNYQTPGMR [475], RPPNAWHK [609], RTHSPSSK [616], SDMGVGVFTPTIEAR [621], TDEDVPSGPPRK [683], TGCFIVIDAMLER [692], TQQGVPAQPADFQAEVESDTR [725], YEGVVDMFQTVK [844], YQYFVVDPMAEYNMPQYILR [869], YSAPANLYVR [870] |
| 124451 | | DDQHFTVTGLHK [111], DEAIYECTATNSLGEINTSAK [112], DFLPVDPATSNGR [117], DHPPIPITDLADNIER [127], FDLSMPHVQDPSLVR [246], HNTDAGLLTTVGSLLPGITYSLR [341], LNYQTPGMR [475], MLWEHNSTIIVMLTK [519], QFQFMAWPDHGVPEYPTPILAFLR [571], RLNYQTPGMR [606], RTHSPSSK [616], TATVVMMTR [676], TGCFIVIDAMLER [692], TQRPAMVQTEDQYQLCYR [726], TSVLLSWEVPDSYK [732], VEVEPLNSTAVHVYWK [756], YEGVVDMFQTVK [844], YSAPANLYVR [870] |
| 126215 | | DDQHFTVTGLHK [111], DSLLAHSSDPVEMR [156], EHSSWDLVGLEK [196], ELPGELLGYR [206], GTTYIFR [324], LNYQTPGMR [475], MVWEQR [529], TATVVMMTR [676], VGGSMLTPR [764], WMMGAEELTK [830], YSAPANLYVR [870] |
| 129890 | | AAGTEGPFQEVDGVATTR [50], DSLLAHSSDPVEMR [156], ELPGELLGYR [206], FEVIEFDDGAGSVLR [249], GPPSEAVR [308], GSGPLSPSIQSR [315], HVVDGISR [347], LNYQTPGMR [475], NGVITQYSVAYEAVDGEDR [537], RLNYQTPGMR [606], RTHSPSSK [616], VSWVPPPADSR [799], YEGVVDMFQTVK [844], YSAPANLYVR [870], YSIGGLSPFSEYAFR [872] |

**Table 48h - Renal cell cancer**

| MW (Da) | Subfractionation | Tryptics identified [SEQ ID No] |
|---|---|---|
| 135833 | | AAGTEGPFQEVDGVATTR [50], EHSSWDLVGLEK [196], FEVIEFDDGAGSVLR [249], FQLAAR [267], SDMGVGVFTPTIEAR [621], TGEQAPSSPPR [694] |

### OGTA002

**Table 49a - Colorectal cancer, iTRAQ**

| Samples batch no. | Experiment no. | Tryptics identified [SEQ ID No] |
|---|---|---|
| Samples 1 | Experiment 1 | AELRGLK [880], TLRLGPLSK [983] |
| Samples 2 | Experiment 1 | ILASVQHMK [373] |
| Samples 2 | Experiment 2 | ILASVQHMK [373] |

**Table 49b - Kidney cancer, iTRAQ**

| Sample no. | Experiment no. | Tryptics identified [SEQ ID No] |
|---|---|---|
| Samples 1 | Experiment 1 | ILASVQHMK [373] |

**Table 49c - Liver cancer, iTRAQ**

| Sample no. | Experiment no. | Tryptics identified [SEQ ID No] |
|---|---|---|
| Samples 1 | Experiment 1 | EIDVSYVK [899] |

**Table 49d - Non-small cell lung cancer, iTRAQ**

| Sample no. | Experiment no. | Tryptics identified [SEQ ID No] |
|---|---|---|
| Samples 1 | Experiment 1 | AELRGLK [880], KCAQLTVNLTR [927] |
| Samples 2 | Experiment 1 | QPHYSAFGSVGEWLRAIK [964] |

### OGTA009

**Table 50a - Colorectal cancer, iTRAQ**

| Samples batch no. | Experiment no. | Tryptics identified [SEQ ID No] |
|---|---|---|
| Samples 1 | Experiment 1 | STGPGASLGTGYDR [657] |
| Samples 1 | Experiment 2 | DFYNPVVPEAQK [119] |
| Samples 2 | Experiment 1 | VYDSLLALPQDLQAAR [817] |
| Samples 2 | Experiment 2 | VYDSLLALPQDLQAAR [817] |
| Samples 2 | Experiment 3 | VYDSLLALPQDLQAAR [817] |
| Samples 2 | Experiment 4 | VYDSLLALPQDLQAAR [817] |

**Table 50b - Non-small cell lung cancer, iTRAQ**

| Sample no. | Experiment no. | Tryptics identified [SEQ ID No] |
|---|---|---|
| Samples 1 | Experiment 1 | VYDSLLALPQDLQAAR [817] |
| Samples 2 | Experiment 1 | VYDSLLALPQDLQAAR [817] |
| Samples 2 | Experiment 2 | VYDSLLALPQDLQAAR [817] |

**Table 50c - Ovarian cancer, iTRAQ**

| Sample no. | Experiment no. | Tryptics identified [SEQ ID No] |
|---|---|---|
| Samples 1 | Experiment 1 | DFYNPVVPEAQK [119] |
| Samples 1 | Experiment 2 | DFYNPVVPEAQK [119] |

### OGTA016

**Table 51a - Colorectal cancer iTRAQ**

| Samples batch no. | Experiment no. | Tryptics identified [SEQ ID No] |
|---|---|---|
| Samples 1 | Experiment 1 | ADAAPDEK [57], AFVNCDENSR [68], ASVDSGSSEEQGGSSR [86], LSDAGQYLCQAGDDSNSNKK [947], NADLQVLKPEPELVYEDLR [955], QSSGENCDVVVNTLGK [588], VYTVDLGR [821] |
| Samples 1 | Experiment 2 | ADAAPDEK [57] |
| Samples 2 | Experiment 1 | ADAAPDEK [57], ASVDSGSSEEQGGSSR [86] |
| Samples 2 | Experiment 2 | AAGSRDVSLAKADAAPDEK [877] |
| Samples 2 | Experiment 3 | ADAAPDEK [57], IIEGEPNLK [371], ILLNPQDK [377], VYTVDLGR [821] |
| Samples 2 | Experiment 4 | ASVDSGSSEEQGGSSR [86] |
| Samples 2 | Experiment 5 | ASVDSGSSEEQGGSSR [86] |
| Samples 2 | Experiment 6 | ASVDSGSSEEQGGSSR [86], IIEGEPNLK [371], ILLNPQDK [377], QSSGENCDVVVNTLGK [588] |

**Table 51b - Non-small cell lung cancer, iTRAQ**

| Sample no. | Experiment no. | Tryptics identified [SEQ ID No] |
|---|---|---|
| Samples 1 | Experiment 1 | ADAAPDEK [57], ASVDSGSSEEQGGSSR [86], ILLNPQDK [377] |
| Samples 2 | Experiment 1 | IIEGEPNLK [371] |
| Samples 2 | Experiment 2 | ESKSIK [907], TVTINCPFK [987] |

### OGTA028

**Table 52a - Colorectal cancer, iTRAQ**

| Samples batch no. | Experiment no. | Tryptics identified [SEQ ID No] |
|---|---|---|
| Samples 1 | Experiment 1 | AEDIIK [879], DIAPVLDLK [890] |
| Samples 2 | Experiment 1 | DIAPVLDLK [890] |
| Samples 2 | Experiment 2 | DIAPVLDLK [890], EEINQGGR [177], IDEKR [919] |
| Samples 2 | Experiment 3 | DIAPVLDLK [890], SSVKELLK [973] |
| Samples 2 | Experiment 4 | DIAPVLDLK [890] |
| Samples 2 | Experiment 5 | DIAPVLDLK [890] |
| Samples 2 | Experiment 6 | DIAPVLDLK [890] |

**Table 52b - Kidney cancer, iTRAQ**

| Sample no. | Experiment no. | Tryptics identified [SEQ ID No] |
|---|---|---|
| Samples 1 | Experiment 1 | DIAPVLDLK [890], EEINQGGR [177] |
| Samples 1 | Experiment 2 | DIAPVLDLK [890], EEINQGGR [177] |
| Samples 1 | Experiment 3 | DIAPVLDLK [890] |

**Table 52c - Liver cancer, iTRAQ**

| Sample no. | Experiment no. | Tryptics identified [SEQ ID No] |
|---|---|---|
| Samples 1 | Experiment 1 | VKTTSLTEKK [992] |
| Samples 1 | Experiment 2 | DIAPVLDLK [890], WSNVFK [832] |

**Table 52d - Non-small cell lung cancer, iTRAQ**

| Sample no. | Experiment no. | Tryptics identified [SEQ ID No] |
|---|---|---|
| Samples 1 | Experiment 1 | DIAPVLDLK [890], LKNKEEVLK [933], VLLEK [995] |
| Samples 1 | Experiment 2 | DIAPVLDLK [890] |
| Samples 2 | Experiment 1 | DIAPVLDLK [890] |
| Samples 2 | Experiment 2 | DIAPVLDLK [890] |
| Samples 2 | Experiment 3 | DIAPVLDLK [890] |
| Samples 2 | Experiment 4 | DIAPVLDLK [890], DVLPQK [897] |
| Samples 2 | Experiment 5 | DIAPVLDLK [890], EEINQGGR [177] |

**Table 52e - Small cell lung cancer, iTRAQ**

| Sample no. | Experiment no. | Tryptics identified [SEQ ID No] |
|---|---|---|
| Samples 1 | Experiment 1 | DIAPVLDLK [890] |
| Samples 1 | Experiment 2 | DIAPVLDLK [890], EEINQGGR [177] |

**Table 52f - Ovarian cancer, iTRAQ**

| Sample no. | Experiment no. | Tryptics identified [SEQ ID No] |
|---|---|---|
| Samples 1 | Experiment 1 | DIAPVLDLK [890] |
| Samples 1 | Experiment 2 | DIAPVLDLK [890] |
| Samples 1 | Experiment 3 | DIAPVLDLK [890] |
| Samples 1 | Experiment 4 | DIAPVLDLK [890] |

### OGTA037

**Table 53a - Colorectal cancer, iTRAQ**

| Samples batch no. | Experiment no. | Tryptics identified [SEQ ID No] |
|---|---|---|
| Samples 1 | Experiment 1 | VLAANNVR [993] |

**Table 53b - Non-small cell lung cancer, iTRAQ**

| Sample no. | Experiment no. | Tryptics identified [SEQ ID No] |
|---|---|---|
| Samples 1 | Experiment 1 | VLAANNVR [993] |

**Table 53c - Ovarian cancer, iTRAQ**

| Sample no. | Experiment no. | Tryptics identified [SEQ ID No] |
|---|---|---|
| Samples 1 | Experiment 1 | VLAANNVR [993] |

### OGTA041

**Table 54 - Non-small cell lung cancer, iTRAQ**

| Sample no. | Experiment no. | Tryptics identified [SEQ ID No] |
|---|---|---|
| Samples 1 | Experiment 1 | LTWTNPSIK [950] |
| Samples 2 | Experiment 1 | LTWTNPSIK [950] |

### OGTA053

**Table 55a - Colorectal cancer, iTRAQ**

| Samples batch no. | Experiment no. | Tryptics identified [SEQ ID No] |
|---|---|---|
| Samples 1 | Experiment 1 | VVISIR [1002] |
| Samples 2 | Experiment 1 | TELLAADSLSK [980] |
| Samples 2 | Experiment 2 | VVISIR [1002] |
| Samples 2 | Experiment 3 | IRTSTPTGHGASPAK [924] |
| Samples 2 | Experiment 4 | TELLAADSLSK [980] |
| Samples 2 | Experiment 5 | IRTSTPTGHGASPAK [924], TELLAADSLSK [980] |

**Table 55b - Kidney cancer, iTRAQ**

| Sample no. | Experiment no. | Tryptics identified [SEQ ID No] |
|---|---|---|
| Samples 1 | Experiment 1 | TELLAADSLSK [980] |
| Samples 1 | Experiment 2 | IRTSTPTGHGASPAK [924], TELLAADSLSK [980] |
| Samples 1 | Experiment 3 | IRTSTPTGHGASPAK [924] |

**Table 55c - Liver cancer, iTRAQ**

| Sample no. | Experiment no. | Tryptics identified [SEQ ID No] |
|---|---|---|
| Samples 1 | Experiment 1 | IRTSTPTGHGASPAK [924] |

**Table 55d - Non-small cell lung cancer, iTRAQ**

| Sample no. | Experiment no. | Tryptics identified [SEQ ID No] |
|---|---|---|
| Samples 1 | Experiment 1 | IRTSTPTGHGASPAK [924], TELLAADSLSK [980] |
| Samples 1 | Experiment 2 | GTLSPKDALTDLTGDAER [916], IRTSTPTGHGASPAK [924], TELLAADSLSK [980] |
| Samples 2 | Experiment 1 | IRTSTPTGHGASPAK [924] |
| Samples 2 | Experiment 2 | IRTSTPTGHGASPAK [924], TELLAADSLSK [980] |

**Table 55e - Small cell lung cancer, iTRAQ**

| Sample no. | Experiment no. | Tryptics identified [SEQ ID No] |
|---|---|---|
| Samples 1 | Experiment 1 | TELLAADSLSK [980] |
| Samples 1 | Experiment 2 | TELLAADSLSK [980] |

### OGTA054

**Table 56a - Colorectal cancer iTRAQ**

| Samples batch no. | Experiment no. | Tryptics identified [SEQ ID No] |
|---|---|---|
| Samples 1 | Experiment 1 | EEHEVAVLGAPPSTILPR [173], MVGDVTGAQAYASTAK [954] |
| Samples 2 | Experiment 1 | MVGDVTGAQAYASTAK [954] |

**Table 56b - Non-small cell lung cancer, iTRAQ**

| Sample no. | Experiment no. | Tryptics identified [SEQ ID No] |
|---|---|---|
| Samples 1 | Experiment 1 | MVGDVTGAQAYASTAK [954] |
| Samples 1 | Experiment 2 | MVGDVTGAQAYASTAK [954] |

### OGTA066

**Table 57a -Colorectal cancer iTRAQ**

| Samples batch no. | Experiment no. | Tryptics identified [SEQ ID No] |
|---|---|---|
| Samples 1 | Experiment 1 | IMFVDPSLTVR [380] [3] |
| Samples 1 | Experiment 2 | IMFVDPSLTVR [380] [3] |
| Samples 2 | Experiment 1 | IMFVDPSLTVR [380] [3] |
| Samples 2 | Experiment 2 | DLPLLIENMK [135] [2], IMFVDPSLTVR [380] [3] |
| Samples 2 | Experiment 3 | IMFVDPSLTVR [380] [3] |
| Samples 2 | Experiment 4 | DLPLLIENMK [135] [2], IMFVDPSLTVR [380] [3] |
| Samples 2 | Experiment 5 | IMFVDPSLTVR [380] [3] |

**Table 57b - Kidney cancer iTRAQ**

| Sample no. | Experiment no. | Tryptics identified [SEQ ID No] |
|---|---|---|
| Samples 1 | Experiment 1 | DLPLLIENMK [135] [2] |

**Table 57c -Non-small cell lung cancer iTRAQ**

| Sample no. | Experiment no. | Tryptics identified [SEQ ID No] |
|---|---|---|
| Samples 1 | Experiment 1 | DLPLLIENMK [135] [2], IMFVDPSLTVR [380] [3], NLSPDGQYVPR [546] [5] |
| Samples 2 | Experiment 1 | IMFVDPSLTVR [380] [3] |
| Samples 2 | Experiment 2 | IMFVDPSLTVR [380] [3] |
| Samples 2 | Experiment 3 | IMFVDPSLTVR [380] [3] |
| Samples 2 | Experiment 4 | IMFVDPSLTVR [380] [3] |

### OGTA074

**Table 58 - Non-small cell lung cancer, iTRAQ**

| Sample no. | Experiment no. | Tryptics identified [SEQ ID No] |
|---|---|---|
| Samples 1 | Experiment 1 | CLNPVPAVPSPSPSVRK [888] |

### OGTA076

**Table 59a- Colorectal cancer iTRAQ**

| Samples batch no. | Experiment no. | Tryptics identified [SEQ ID No] |
|---|---|---|
| Samples 1 | Experiment 1 | EGIAK [898] [14], IIPK [921] [28], LALK [929] [35], LNPK [941] [40] |
| Samples 1 | Experiment 2 | EGIAK [898] [14], LALK [929] [35] |
| Samples 1 | Experiment 3 | EGIAK [898] [14], IIPK [921] [28], LALK [929] [35] |
| Samples 1 | Experiment 4 | LNPK [941] [40] |

**Table 59b - Kidney cancer iTRAQ**

| Sample no. | Experiment no. | Tryptics identified [SEQ ID No] |
|---|---|---|
| Samples 1 | Experiment 1 | EGIAK [898] [14], LALK [929] [35], LNPK [941] [40], YLNNLYK [1006] [66] |
| Samples 1 | Experiment 2 | LALK [929] [35] |

**Table 59c - Liver cancer iTRAQ**

| Sample no. | Experiment no. | Tryptics identified [SEQ ID No] |
|---|---|---|
| Samples 1 | Experiment 1 | YLNNLYK [1006] [66] |

**Table 59d - Non-small cell lung cancer iTRAQ**

| Sample no. | Experiment no. | Tryptics identified [SEQ ID No] |
|---|---|---|
| Samples 1 | Experiment 1 | YLNNLYK [1006] [66] |
| Samples 1 | Experiment 2 | LALK [929] [35] |
| Samples 2 | Experiment 1 | IIPK [921] [28], LALK [929] [35], YLNNLYK [1006] [66] |
| Samples 2 | Experiment 2 | YLNNLYK [1006] [66] |

**Table 59e- Small cell lung cancer iTRAQ**

| Sample no. | Experiment no. | Tryptics identified [SEQ ID No] |
|---|---|---|
| Samples 1 | Experiment 1 | EGIAK [898] [14] |
| Samples 1 | Experiment 2 | EGIAK [898] [14] |

**Table 59f- Ovarian cancer iTRAQ**

| Sample no. | Experiment no. | Tryptics identified [SEQ ID No] |
|---|---|---|
| Samples 1 | Experiment 1 | LALK [929] [35] |
| Samples 1 | Experiment 2 | LALK [929] [35], YLNNLYK [1006] [66] |
| Samples 1 | Experiment 3 | LALK [929] [35] |

### OGTA085

**Table 60a - Colorectal cancer, iTRAQ**

| Samples batch no. | Experiment no. | Tryptics identified [SEQ ID No] |
|---|---|---|
| Samples 1 | Experiment 1 | KMLGNPSR [427] |

**Table 60b - Non-small cell lung cancer, iTRAQ**

| Sample no. | Experiment no. | Tryptics identified [SEQ ID No] |
|---|---|---|
| Samples 1 | Experiment 1 | KMLGNPSR [427] |

### OGTA088

**Table 61a - Colorectal cancer, iTRAQ**

| Samples batch no. | Experiment no. | Tryptics identified [SEQ ID No] |
|---|---|---|
| Samples 1 | Experiment 1 | RQEELERK [613] |

**Table 61b - Non-small cell lung cancer, iTRAQ**

| Sample no. | Experiment no. | Tryptics identified [SEQ ID No] |
|---|---|---|
| Samples 1 | Experiment 1 | RQEELERK [613] |

### OGTA089

**Table 62a - Kidney cancer, iTRAQ**

| Sample no. | Experiment no. | Tryptics identified [SEQ ID No] |
|---|---|---|
| Samples 1 | Experiment 1 | GHEVAAMLK [913] |

**Table 62b - Non-small cell lung cancer, iTRAQ**

| Sample no. | Experiment no. | Tryptics identified [SEQ ID No] |
|---|---|---|
| Samples 1 | Experiment 1 | LLHGQNGSVPNGQTPLK [935] |

**Table 62c - Ovarian cancer, iTRAQ**

| Sample no. | Experiment no. | Tryptics identified [SEQ ID No] |
|---|---|---|
| Samples 1 | Experiment 1 | NNLVEIILDINVSQLTER [956] |

### OGTA091

**Table 63a - Colorectal cancer, iTRAQ**

| Samples batch no. | Experiment no. | Tryptics identified [SEQ ID No] |
|---|---|---|
| Samples 1 | Experiment 1 | IYIVR [926] |
| Samples 2 | Experiment 1 | KRSAPTSR [928] |

**Table 63b - Kidney cancer, iTRAQ**

| Sample no. | Experiment no. | Tryptics identified [SEQ ID No] |
|---|---|---|
| Samples 1 | Experiment 1 | LLSDK [938], RMEPLEK [607] |
| Samples 1 | Experiment 2 | FSDVTGKIK [912], IGETVVDLENR [367], RRDLSQMEALK [966] |

**Table 63c - Liver cancer, iTRAQ**

| Sample no. | Experiment no. | Tryptics identified [SEQ ID No] |
|---|---|---|
| Samples 1 | Experiment 1 | VVFQIWDNDK [810] |

**Table 63d - Non-small cell lung cancer, iTRAQ**

| Sample no. | Experiment no. | Tryptics identified [SEQ ID No] |
|---|---|---|
| Samples 1 | Experiment 1 | IPNPHLGPVEER [386], IYIVR [926], TAIEILAWGLR [976] |
| Samples 1 | Experiment 2 | FSDVTGK [272], VTAAGQTK [800] |
| Samples 2 | Experiment 1 | RRDLSQMEALK [966] |

**Table 63e - Small cell lung cancer, iTRAQ**

| Sample no. | Experiment no. | Tryptics identified [SEQ ID No] |
|---|---|---|
| Samples 1 | Experiment 1 | LLSDK [938] |

**Table 63f - Ovarian cancer, iTRAQ**

| Sample no. | Experiment no. | Tryptics identified [SEQ ID No] |
|---|---|---|
| Samples 1 | Experiment 1 | VTAAGQTKRTR [999] |
| Samples 1 | Experiment 2 | VPAHQVLFSRR [997], VTAAGQTKRTR [999] |

### OGTA098

**Table 64a - Colorectal cancer, iTRAQ**

| Samples batch no. | Experiment no. | Tryptics identified [SEQ ID No] |
|---|---|---|
| Samples 1 | Experiment 1 | SLQEANAEK [970], TLAEVCLGQK [982] |
| Samples 2 | Experiment 1 | INATDADEPNTLNSK [381], SLQEANAEK [970] |

**Table 64b - Kidney cancer, iTRAQ**

| Sample no. | Experiment no. | Tryptics identified [SEQ ID No] |
|---|---|---|
| Samples 1 | Experiment 1 | SLQEANAEK [970] |

**Table 64c - Non-small cell lung cancer, iTRAQ**

| Sample no. | Experiment no. | Tryptics identified [SEQ ID No] |
|---|---|---|
| Samples 1 | Experiment 1 | INATDADEPNTLNSK [381] |
| Samples 1 | Experiment 2 | NPIAK [957] |
| Samples 2 | Experiment 1 | NPIAK [957] |

**Table 64d - Small cell lung cancer, iTRAQ**

| Sample no. | Experiment no. | Tryptics identified [SEQ ID No] |
|---|---|---|
| Samples 1 | Experiment 1 | NPIAK [957], TARATGASR [977] |

**Table 64e - Ovarian cancer, iTRAQ**

| Sample no. | Experiment no. | Tryptics identified [SEQ ID No] |
|---|---|---|
| Samples 1 | Experiment 1 | NPIAK [957] |

### OGTA101

**Table 65a - Colorectal cancer, iTRAQ**

| Samples batch no. | Experiment no. | Tryptics identified [SEQ ID No] |
|---|---|---|
| Samples 1 | Experiment 1 | LSARNK [946] |
| Samples 2 | Experiment 1 | IKQLR [922] |
| Samples 2 | Experiment 2 | LASSKAHTSR [930] |

**Table 65b - Kidney cancer, iTRAQ**

| Sample no. | Experiment no. | Tryptics identified [SEQ ID No] |
|---|---|---|
| Samples 1 | Experiment 1 | ILYDDGKMVEEVDGR [923] |

**Table 65c - Liver cancer, iTRAQ**

| Sample no. | Experiment no. | Tryptics identified [SEQ ID No] |
|---|---|---|
| Samples 1 | Experiment 1 | IKQLR [922] |

**Table 65d - Non-small cell lung cancer, iTRAQ**

| Sample no. | Experiment no. | Tryptics identified [SEQ ID No] |
|---|---|---|
| Samples 1 | Experiment 1 | ELREVR [901], LTVLR [949], MRYEGVVDIFQTVK [952], VEVEAVNSTSVK [755] |
| Samples 1 | Experiment 2 | LASSKAHTSR [930], LTVLR [949] |
| Samples 2 | Experiment 1 | GNSAGGLQHRVTAK [914], LSARNK [946] |
| Samples 2 | Experiment 2 | TVDIYGHVTLMR [736] |

**Table 65e - Ovarian cancer, iTRAQ**

| Sample no. | Experiment no. | Tryptics identified [SEQ ID No] |
|---|---|---|
| Samples 1 | Experiment 1 | YEGVVDIFQTVK [843] |

### OGTA104

**Table 66a - Colorectal cancer, iTRAQ**

| Samples batch no. | Experiment no. | Tryptics identified [SEQ ID No] |
|---|---|---|
| Samples 1 | Experiment 1 | FPSGTLR [264], NQTAVR [959] |
| Samples 2 | Experiment 1 | LQCENVQEIPVFGIVPAIIQTPSR [943] |
| Samples 2 | Experiment 2 | FPSGTLR [264] |

**Table 66b - Kidney cancer, iTRAQ**

| Sample no. | Experiment no. | Tryptics identified [SEQ ID No] |
|---|---|---|
| Samples 1 | Experiment 1 | FLITRRR [910] |

**Table 66c - Liver cancer, iTRAQ**

| Sample no. | Experiment no. | Tryptics identified [SEQ ID No] |
|---|---|---|
| Samples 1 | Experiment 1 | SQTYESDGK [972] |

**Table 66d - Non-small cell lung cancer, iTRAQ**

| Sample no. | Experiment no. | Tryptics identified [SEQ ID No] |
|---|---|---|
| Samples 1 | Experiment 1 | FLITRRR [910] |
| Samples 1 | Experiment 2 | CGVSNKDIEK [887], FLITRRR [910] |

**Table 66e - Ovarian cancer, iTRAQ**

| Sample no. | Experiment no. | Tryptics identified [SEQ ID No] |
|---|---|---|
| Samples 1 | Experiment 1 | FLITRRR [910] |

### OGTA106

**Table 67 - Liver cancer, iTRAQ**

| Sample no. | Experiment no. | Tryptics identified [SEQ ID No] |
|---|---|---|
| Samples 1 | Experiment 1 | EVCQLLLR [908] |

### OGTA112

**Table 68 - Kidney cancer, iTRAQ**

| Sample no. | Experiment no. | Tryptics identified [SEQ ID No] |
|---|---|---|
| Samples 1 | Experiment 1 | LFGEK [932] |

### OGTA113

**Table 69a - Colorectal cancer, iTRAQ**

| Samples batch no. | Experiment no. | Tryptics identified [SEQ ID No] |
|---|---|---|
| Samples 1 | Experiment 1 | LLSDPNYGVHLPAVK [939] |
| Samples 2 | Experiment 1 | YPEVGDLR [865] |
| Samples 2 | Experiment 2 | LEDPHVDIIRR [931] |

**Table 69b - Kidney cancer, iTRAQ**

| Sample no. | Experiment no. | Tryptics identified [SEQ ID No] |
|---|---|---|
| Samples 1 | Experiment 1 | LEDPHVDIIR [449] |

**Table 69c - Non-small cell lung cancer, iTRAQ**

| Sample no. | Experiment no. | Tryptics identified [SEQ ID No] |
|---|---|---|
| Samples 1 | Experiment 1 | YPEVGDLR [865], YSYNTEWR [874] |

**Table 69d - Ovarian cancer, iTRAQ**

| Sample no. | Experiment no. | Tryptics identified [SEQ ID No] |
|---|---|---|
| Samples 1 | Experiment 1 | LEDPHVDIIR [449] |

### OGTA119

**Table 70a - Colorectal cancer, iTRAQ**

| Samples batch no. | Experiment no. | Tryptics identified [SEQ ID No] |
|---|---|---|
| Samples 1 | Experiment 1 | ALALLEDEER [75], GEGPEAGAGGAGGR [290] |
| Samples 2 | Experiment 1 | VLTDIK [996] |
| Samples 2 | Experiment 2 | MTPPSRAEAGVRR [953] |

**Table 70b - Non-small cell lung cancer, iTRAQ**

| Sample no. | Experiment no. | Tryptics identified [SEQ ID No] |
|---|---|---|
| Samples 1 | Experiment 1 | AEWLNK [64], DTYLK [896] |
| Samples 1 | Experiment 2 | GEGPEAGAGGAGGR [290] |
| Samples 2 | Experiment 1 | DTYLK [896] |

**Table 70c - Ovarian cancer, iTRAQ**

| Sample no. | Experiment no. | Tryptics identified [SEQ ID No] |
|---|---|---|
| Samples 1 | Experiment 1 | ALALLEDEER [75] |
| Samples 1 | Experiment 2 | ALALLEDEER [75] |

### OGTA124

**Table 71a - Colorectal cancer, iTRAQ**

| Samples batch no. | Experiment no. | Tryptics identified [SEQ ID No] |
|---|---|---|
| Samples 1 | Experiment 1 | LLQAIAK [937] |

**Table 71b - Kidney cancer, iTRAQ**

| Sample no. | Experiment no. | Tryptics identified [SEQ ID No] |
|---|---|---|
| Samples 1 | Experiment 1 | IFTVAQMDDNSIQMKK [365], LDEDLHVK [446] |

**Table 71c - Liver cancer, iTRAQ**

| Sample no. | Experiment no. | Tryptics identified [SEQ ID No] |
|---|---|---|
| Samples 1 | Experiment 1 | EWGDGIR [240] |

**Table 71d - Non-small cell lung cancer, iTRAQ**

| Sample no. | Experiment no. | Tryptics identified [SEQ ID No] |
|---|---|---|
| Samples 1 | Experiment 1 | LDEDLHVK [446] |
| Samples 1 | Experiment 2 | LDEDLHVK [446] |

**Table 71e - Ovarian cancer, iTRAQ**

| Sample no. | Experiment no. | Tryptics identified [SEQ ID No] |
|---|---|---|
| Samples 1 | Experiment 1 | EREAQLVK [905] |

### OGTA126

**Table 72 - Non-small cell lung cancer, iTRAQ**

| Sample no. | Experiment no. | Tryptics identified [SEQ ID No] |
|---|---|---|
| Samples 1 | Experiment 1 | IGTFCSNGTVSRIK [1007], LLVPKDR [940], LWMNVEK [505] |
| Samples 2 | Experiment 1 | LLVPKDR [940] |

### OGTA156

**Table 73a - Colorectal cancer, iTRAQ**

| Samples batch no. | Experiment no. | Tryptics identified [SEQ ID No] |
|---|---|---|
| Samples 1 | Experiment 1 | DIMKKTINFAR [891] |

**Table 73b - Kidney cancer, iTRAQ**

| Sample no. | Experiment no. | Tryptics identified [SEQ ID No] |
|---|---|---|
| Samples 1 | Experiment 1 | DIMKKTINFAR [891], HSRVTVAIPLK [918] |

**Table 73c - Non-small cell lung cancer, iTRAQ**

| Sample no. | Experiment no. | Tryptics identified [SEQ ID No] |
|---|---|---|
| Samples 1 | Experiment 1 | HSRVTVAIPLK [918] |
| Samples 2 | Experiment 1 | HSRVTVAIPLK [918], TYLAVGSMK [740] |

**Table 73d - Ovarian cancer, iTRAQ**

| Sample no. | Experiment no. | Tryptics identified [SEQ ID No] |
|---|---|---|
| Samples 1 | Experiment 1 | DIMKKTINFAR [891] |

### OGTA159

**Table 74a - Kidney cancer, iTRAQ**

| Sample no. | Experiment no. | Tryptics identified [SEQ ID No] |
|---|---|---|
| Samples 1 | Experiment 1 | RDLGSRLQAQR [965] |
| Samples 1 | Experiment 2 | RDLGSRLQAQR [965] |

**Table 74b - Non-small cell lung cancer, iTRAQ**

| Sample no. | Experiment no. | Tryptics identified [SEQ ID No] |
|---|---|---|
| Samples 1 | Experiment 1 | RDLGSRLQAQR [965] |
| Samples 1 | Experiment 2 | RDLGSRLQAQR [965] |

### OGTA169

**Table 75a - Colorectal cancer, iTRAQ**

| Samples batch no. | Experiment no. | Tryptics identified [SEQ ID No] |
|---|---|---|
| Samples 1 | Experiment 1 | GGQVSVCPLPR [299], NRSLSRVR [961] |
| Samples 2 | Experiment 1 | GGQVSVCPLPR [299] |
| Samples 2 | Experiment 2 | TSKTTFQLELPVK [985] |

**Table 75b - Kidney cancer, iTRAQ**

| Sample no. | Experiment no. | Tryptics identified [SEQ ID No] |
|---|---|---|
| Samples 1 | Experiment 1 | AVLGDR [885] |

**Table 75c - Non-small cell lung cancer, iTRAQ**

| Sample no. | Experiment no. | Tryptics identified [SEQ ID No] |
|---|---|---|
| Samples 1 | Experiment 1 | GGQVSVCPLPR [299] |
| Samples 2 | Experiment 1 | TSKTTFQLELPVK [985] |
| Samples 2 | Experiment 2 | GGQVSVCPLPR [299], LTDVVIGAPGEEENR [496], YFTASLPFEK [1004] |

### OGTA174

**Table 76 - Non-small cell lung cancer, iTRAQ**

| Sample no. | Experiment no. | Tryptics identified [SEQ ID No] |
|---|---|---|
| Samples 1 | Experiment 1 | LSQCHELWER [490] |

### OGTA176

**Table 77a - Colorectal cancer, iTRAQ**

| Samples batch no. | Experiment no. | Tryptics identified [SEQ ID No] |
|---|---|---|
| Samples 1 | Experiment 1 | AVTSPAVGR [886] |
| Samples 1 | Experiment 2 | AVTSPAVGR [886] |

**Table 77b - Kidney cancer, iTRAQ**

| Sample no. | Experiment no. | Tryptics identified [SEQ ID No] |
|---|---|---|
| Samples 1 | Experiment 1 | AVTSPAVGR [886] |

**Table 77c - Non-small cell lung cancer, iTRAQ**

| Sample no. | Experiment no. | Tryptics identified [SEQ ID No] |
|---|---|---|
| Samples 1 | Experiment 1 | AVTSPAVGR [886] |

**Table 77d - Small cell lung cancer, iTRAQ**

| Sample no. | Experiment no. | Tryptics identified [SEQ ID No] |
|---|---|---|
| Samples 1 | Experiment 1 | AVTSPAVGR [886] |
| Samples 1 | Experiment 2 | AVTSPAVGR [886] |

### OGTA177

**Table 78a - Colorectal cancer, iTRAQ**

| Samples batch no. | Experiment no. | Tryptics identified [SEQ ID No] |
|---|---|---|
| Samples 1 | Experiment 1 | VLDVVER [994], VVNVSDLYK [1003] |
| Samples 2 | Experiment 1 | AREVIPR [883] |
| Samples 2 | Experiment 2 | DIQVASNEILR [131], SQHQETPVYLGATAGMRLLR [971] |
| Samples 2 | Experiment 3 | GPGISK [915], SQHQETPVYLGATAGMRLLR [971] |

**Table 78b - Kidney cancer, iTRAQ**

| Sample no. | Experiment no. | Tryptics identified [SEQ ID No] |
|---|---|---|
| Samples 1 | Experiment 1 | DIQVASNEILR [131], GPGISK [915], SLSNYPFDFQGAR [643], VLDVVER [994] |

**Table 78c - Non-small cell lung cancer, iTRAQ**

| Sample no. | Experiment no. | Tryptics identified [SEQ ID No] |
|---|---|---|
| Samples 1 | Experiment 1 | DIQVASNEILR [131], DPCFHPGYK [892], SLSNYPFDFQGAR [643], SQHQETPVYLGATAGMR [647] |
| Samples 1 | Experiment 2 | EVIPR [909], SLSNYPFDFQGAR [643] |
| Samples 2 | Experiment 1 | FLNLTSEKVSQEK [911] |

**Table 78d - Small cell lung cancer, iTRAQ**

| Sample no. | Experiment no. | Tryptics identified [SEQ ID No] |
|---|---|---|
| Samples 1 | Experiment 1 | AREVIPR [883] |

**Table 78e - Ovarian cancer, iTRAQ**

| Sample no. | Experiment no. | Tryptics identified [SEQ ID No] |
|---|---|---|
| Samples 1 | Experiment 1 | GPGISK [915] |

### OGTA197

**Table 79a - Colorectal cancer, iTRAQ**

| Samples batch no. | Experiment no. | Tryptics identified [SEQ ID No] |
|---|---|---|
| Samples 1 | Experiment 1 | LNVEERSVGPLTR [942] |
| Samples 2 | Experiment 1 | NGVSGLVTSR [538], TASVSINQTEPPK [673] |
| Samples 2 | Experiment 2 | NGVSGLVTSR [538] |

**Table 79b - Kidney cancer, iTRAQ**

| Sample no. | Experiment no. | Tryptics identified [SEQ ID No] |
|---|---|---|
| Samples 1 | Experiment 1 | LPGHQKR [478], NGVSGLVTSR [538], TASVSINQTEPPK [673] |

**Table 79c - Liver cancer, iTRAQ**

| Sample no. | Experiment no. | Tryptics identified [SEQ ID No] |
|---|---|---|
| Samples 1 | Experiment 1 | TASVSINQTEPPK [673] |

**Table 79d - Non-small cell lung cancer, iTRAQ**

| Sample no. | Experiment no. | Tryptics identified [SEQ ID No] |
|---|---|---|
| Samples 1 | Experiment 1 | TASVSINQTEPPK [673] |
| Samples 1 | Experiment 2 | DGEFSVLQLVGMLR [121], LNVEERSVGPLTR [942] |
| Samples 2 | Experiment 1 | NGVSGLVTSR [538] |

**Table 79e - Small cell lung cancer, iTRAQ**

| Sample no. | Experiment no. | Tryptics identified [SEQ ID No] |
|---|---|---|
| Samples 1 | Experiment 1 | SVGPLTRK [663], TASVSINQTEPPK [673] |
| Samples 1 | Experiment 2 | NGVSGLVTSR [538], SVGPLTRK [663], TASVSINQTEPPK [673] |

### OGTA202

**Table 80a - Colorectal cancer iTRAQ**

| Samples batch no. | Experiment no. | Tryptics identified [SEQ ID No] |
|---|---|---|
| Samples 1 | Experiment 1 | STGTISVISSGLDREK [975] |
| Samples 2 | Experiment 1 | EQLTVIR [904], ISYRILR [925] |

**Table 80b - Non-small cell lung cancer, iTRAQ**

| Sample no. | Experiment no. | Tryptics identified [SEQ ID No] |
|---|---|---|
| Samples 1 | Experiment 1 | EQLTVIR [904] |
| Samples 1 | Experiment 2 | EQLTVIR [904] |
| Samples 2 | Experiment 1 | EQLTVIR [904] |

### OGTA203

**Table 81a - Kidney cancer, iTRAQ**

| Sample no. | Experiment no. | Tryptics identified [SEQ ID No] |
|---|---|---|
| Samples 1 | Experiment 1 | LLDFEK [934], TGRPVEPESEFIIK [981] |

**Table 81b - Non-small cell lung cancer, iTRAQ**

| Sample no. | Experiment no. | Tryptics identified [SEQ ID No] |
|---|---|---|
| Samples 1 | Experiment 1 | VEASNPYVEPRFLYLGPFK [989] |

### OGTA206

**Table 82a - Colorectal cancer, iTRAQ**

| Samples batch no. | Experiment no. | Tryptics identified [SEQ ID No] |
|---|---|---|
| Samples 1 | Experiment 1 | VYDSLLALPQDLQAAR [817] |
| Samples 2 | Experiment 1 | SAAASNYV [968], VYDSLLALPQDLQAAR [817] |
| Samples 2 | Experiment 2 | VYDSLLALPQDLQAAR [817] |
| Samples 2 | Experiment 3 | VYDSLLALPQDLQAAR [817] |
| Samples 2 | Experiment 4 | VYDSLLALPQDLQAAR [817] |

**Table 82b - Non-small cell lung cancer, iTRAQ**

| Sample no. | Experiment no. | Tryptics identified [SEQ ID No] |
|---|---|---|
| Samples 1 | Experiment 1 | VYDSLLALPQDLQAAR [817] |
| Samples 2 | Experiment 1 | VYDSLLALPQDLQAAR [817] |
| Samples 2 | Experiment 2 | VYDSLLALPQDLQAAR [817] |

### OGTA213

**Table 83a - Colorectal cancer, iTRAQ**

| Samples batch no. | Experiment no. | Tryptics identified [SEQ ID No] |
|---|---|---|
| Samples 1 | Experiment 1 | RSSAAGEGTLAR [967] |
| Samples 2 | Experiment 1 | RSSAAGEGTLAR [967] |
| Samples 2 | Experiment 2 | RSSAAGEGTLAR [967] |

**Table 83b - Non-small cell lung cancer, iTRAQ**

| Sample no. | Experiment no. | Tryptics identified [SEQ ID No] |
|---|---|---|
| Samples 1 | Experiment 1 | RSSAAGEGTLAR [967] |
| Samples 1 | Experiment 2 | RSSAAGEGTLAR [967] |
| Samples 2 | Experiment 1 | RSSAAGEGTLAR [967] |

### OGTA214

**Table 84 - Colorectal cancer, iTRAQ**

| Samples batch no. | Experiment no. | Tryptics identified [SEQ ID No] |
|---|---|---|
| Samples 1 | Experiment 1 | DSQMQNPYSR [895], HIEEENLIDEDFQNLK [917], SDLQRPNPQSPFCVASSLK [969], STGFTNLGAEGSVFPK [974] |
| Samples 1 | Experiment 2 | STGFTNLGAEGSVFPK [974] |
| Samples 2 | Experiment 1 | HIEEENLIDEDFQNLK [917] |
| Samples 2 | Experiment 2 | VFPGK [990] |

### OGTA216

**Table 85a - Colorectal cancer, iTRAQ**

| Samples batch no. | Experiment no. | Tryptics identified [SEQ ID No] |
|---|---|---|
| Samples 1 | Experiment 1 | AAAAAAAAAAAAAAAGAGAGAK [49], AFYAPVHADDLR [69], GGGAYYLISR [298], GPIVPLNVADQK [307], ITDNELELYK [395] |
| Samples 1 | Experiment 2 | DLPPILLVR [136], EGAQYLMQAAGLGR [186] |
| Samples 2 | Experiment 1 | AMATLLSK [78], EGAQYLMQAAGLGR [186] |
| Samples 2 | Experiment 2 | DLPPILLVR [136] |
| Samples 2 | Experiment 3 | QTPADGEASGESEPAK [1008] |

**Table 85b - Kidney cancer, iTRAQ**

| Sample no. | Experiment no. | Tryptics identified [SEQ ID No] |
|---|---|---|
| Samples 1 | Experiment 1 | RAMATLLSK [596] |
| Samples 1 | Experiment 2 | RAMATLLSK [596], SPGWRPAFK [645] |

**Table 85c - Liver cancer, iTRAQ**

| Sample no. | Experiment no. | Tryptics identified [SEQ ID No] |
|---|---|---|
| Samples 1 | Experiment 1 | IFQALCK [920] |
| Samples 1 | Experiment 2 | SPGWRPAFK [645] |

**Table 85d - Non-small cell lung cancer, iTRAQ**

| Sample no. | Experiment no. | Tryptics identified [SEQ ID No] |
|---|---|---|
| Samples 1 | Experiment 1 | AMATLLSK [78], DCKIRVFIGGK [889], TATQPLLK [978] |
| Samples 1 | Experiment 2 | EMSIDQAK [213], LHEDDK [465] |
| Samples 2 | Experiment 1 | EQDIADK [903] |
| Samples 2 | Experiment 2 | DLPPILLVR [136] |

**Table 85e - Small cell lung cancer, iTRAQ**

| Sample no. | Experiment no. | Tryptics identified [SEQ ID No] |
|---|---|---|
| Samples 1 | Experiment 1 | LSGVEDHVK [948] |

**Table 85f- Ovarian cancer, iTRAQ**

| Sample no. | Experiment no. | Tryptics identified [SEQ ID No] |
|---|---|---|
| Samples 1 | Experiment 1 | AMATLLSK [78] |

### OGTA222

**Table 86a - Colorectal cancer, iTRAQ**

| Samples batch no. | Experiment no. | Tryptics identified [SEQ ID No] |
|---|---|---|
| Samples 1 | Experiment 1 | AGFVGGQFWSVYTPCDTQNK [881], ATLQLSR [91], AVGFGGDFDGVPR [95], GALADNLLR [283], NVPDDVLR [559], TLEQMDVVHR [703] |
| Samples 1 | Experiment 2 | AGFVGGQFWSVYTPCDTQNK [881], ALYQLGMR [77], AVGFGGDFDGVPR [95], DSPVIDGHNDLPWQLLDMFNNR [894]. GALADNLLR [283], TLEQMDVVHR [703], VASLIGVEGGHSIDSSLGVLR [988], VPEGLEDVSK [791], YPDLIAELLR [864] |
| Samples 2 | Experiment 1 | AVGFGGDFDGVPR [95] |

**Table 86b - Kidney cancer, iTRAQ**

| Sample no. | Experiment no. | Tryptics identified [SEQ ID No] |
|---|---|---|
| Samples 1 | Experiment 1 | AVGFGGDFDGVPR [95], VPEGLEDVSK [791] |
| Samples 1 | Experiment 2 | ALYQLGMR [77], AVGFGGDFDGVPR [95], GALADNLLR [283], NVPDDVLR [559], VPEGLEDVSK [791], YPDLIAELLR [864] |
| Samples 1 | Experiment 3 | AVGFGGDFDGVPR [95] |

**Table 86c - Non-small cell lung cancer, iTRAQ**

| Sample no. | Experiment no. | Tryptics identified [SEQ ID No] |
|---|---|---|
| Samples 1 | Experiment 1 | ANLSQVADHLDHIK [882] |
| Samples 1 | Experiment 2 | VPEGLEDVSK [791] |

### OGTA236

**Table 87 - Colorectal cancer, iTRAQ**

| Samples batch no. | Experiment no. | Tryptics identified [SEQ ID No] |
|---|---|---|
| Samples 1 | Experiment 1 | ELNEHFKSK [900] |

### OGTA237

**Table 88a - Colorectal cancer, iTRAQ**

| Samples batch no. | Experiment no. | Tryptics identified [SEQ ID No] |
|---|---|---|
| Samples 1 | Experiment 1 | QLVEALDK [963] |

**Table 88b - Kidney cancer, iTRAQ**

| Sample no. | Experiment no. | Tryptics identified [SEQ ID No] |
|---|---|---|
| Samples 1 | Experiment 1 | AEAFGMDPARPDQASTVAVK [878] |

**Table 88c - Non-small cell lung cancer, iTRAQ**

| Sample no. | Experiment no. | Tryptics identified [SEQ ID No] |
|---|---|---|
| Samples 1 | Experiment 1 | TSNGRLPVK [986] |

**Table 88d - Small cell lung cancer, iTRAQ**

| Sample no. | Experiment no. | Tryptics identified [SEQ ID No] |
|---|---|---|
| Samples 1 | Experiment 1 | LVLGK [951] |

### OGTA247

**Table 89a - Non-small cell lung cancer, iTRAQ**

| Sample no. | Experiment no. | Tryptics identified [SEQ ID No] |
|---|---|---|
| Samples 1 | Experiment 1 | TNGTGRVR [984], WRPVDLAQVK [831] |
| Samples 1 | Experiment 2 | ERMFR [906] |

**Table 89b - Small cell lung cancer, iTRAQ**

| Sample no. | Experiment no. | Tryptics identified [SEQ ID No] |
|---|---|---|
| Samples 1 | Experiment 1 | DATQITQGPR [109], DLQELGDSDK [137], DPELR [893], EGPGEAIVR [189], NPVDVK [958], TNGTGRVR [984], VGEEDDGEYR [762], VTAINK [1000], YGPGEPSPVSETVVTPEAAPEK [1005] |
| Samples 1 | Experiment 2 | AQLLVVGSPGPVPR [82], DATQITQGPR [109], DPELR [893], EPIDLR [217], NPVDVK [958], TNGTGRVR [984], VGEEDDGEYR [762], VQAVNSQGK [998], VTAINK [1000], YGPGEPSPVSETVVTPEAAPEK [1005] |

### OGTA248

**Table 90 - Non-small cell lung cancer, iTRAQ**

| Sample no. | Experiment no. | Tryptics identified [SEQ ID No] |
|---|---|---|
| Samples 1 | Experiment 1 | LRTSAIR [945] |

### OGTA249

**Table 91a - Kidney cancer, iTRAQ**

| Sample no. | Experiment no. | Tryptics identified [SEQ ID No] |
|---|---|---|
| Samples 1 | Experiment 1 | VTLPAGPDILR [1001] |

**Table 91b - Non-small cell lung cancer, iTRAQ**

| Sample no. | Experiment no. | Tryptics identified [SEQ ID No] |
|---|---|---|
| Samples 1 | Experiment 1 | VTLPAGPDILR [1001] |
| Samples 2 | Experiment 1 | VTLPAGPDILR [1001] |
| Samples 2 | Experiment 2 | VTLPAGPDILR [1001] |
| Samples 2 | Experiment 3 | VTLPAGPDILR [1001] |

### OGTA257

**Table 92a - Colorectal cancer, iTRAQ**

| Samples batch no. | Experiment no. | Tryptics identified [SEQ ID No] |
|---|---|---|
| Samples 1 | Experiment 1 | QILDQTPVK [962] |

**Table 92b - Liver cancer, iTRAQ**

| Sample no. | Experiment no. | Tryptics identified [SEQ ID No] |
|---|---|---|
| Samples 1 | Experiment 1 | LRQGTLR [944] |

**Table 92c - Non-small cell lung cancer, iTRAQ**

| Sample no. | Experiment no. | Tryptics identified [SEQ ID No] |
|---|---|---|
| Samples 1 | Experiment 1 | ATGTAFRR [884], ELVSLK [902] |

**Table 92d - Ovarian cancer, iTRAQ**

| Sample no. | Experiment no. | Tryptics identified [SEQ ID No] |
|---|---|---|
| Samples 1 | Experiment 1 | LLIEHGADIR [936] |

### OGTA271

**Table 93a - Colorectal cancer, iTRAQ**

| Samples batch no. | Experiment no. | Tryptics identified [SEQ ID No] |
|---|---|---|
| Samples 1 | Experiment 1 | IKQLR [922], TAQSTPSAPPQK [672], TATVVMMTRLEEK [979] |
| Samples 1 | Experiment 2 | VKCDQYWPAR [991] |

**Table 93b - Kidney cancer, iTRAQ**

| Sample no. | Experiment no. | Tryptics identified [SEQ ID No] |
|---|---|---|
| Samples 1 | Experiment 1 | TAQSTPSAPPQK [672] |
| Samples 1 | Experiment 2 | EPMDQKR [218] |

**Table 93c - Liver cancer, iTRAQ**

| Sample no. | Experiment no. | Tryptics identified [SEQ ID No] |
|---|---|---|
| Samples 1 | Experiment 1 | IKQLR [922] |

**Table 93d - Non-small cell lung cancer, iTRAQ**

| Sample no. | Experiment no. | Tryptics identified [SEQ ID No] |
|---|---|---|
| Samples 1 | Experiment 1 | NRAGLGEEFEKEIR [960], TAQSTPSAPPQK [672] |
| Samples 2 | Experiment 1 | DSLLAHSSDPVEMR [156], TMPVEQVFAK [708] |

For proteins of the disclosure the detected level obtained upon analyzing tissue from subjects having a relevant cancer relative to the detected level obtained upon analyzing tissue from subjects free from said cancers will depend upon the particular analytical protocol and detection technique that is used. Accordingly, the present disclosure contemplates that each laboratory will establish a reference range in subjects free from said cancers according to the analytical protocol and detection technique in use, as is conventional in the diagnostic art. For example, at least one control positive tissue sample from a subject known to have a relevant cancer or at least one control negative tissue sample from a subject known to be free from said cancer (and more preferably both positive and negative control samples) are included in each batch of test samples analysed.

Proteins of the disclosure can be used for detection, prognosis, diagnosis, or monitoring of a relevant cancer or for drug development. In one example of the disclosure, tissue from a subject (*e*.*g*., a subject suspected of having a relevant cancer) is analysed by ID electrophoresis or iTRAQ for detection of a protein according to the disclosure. An increased abundance of of a protein according to the disclosure in the tissue from the subject relative to tissue from a subject or subjects free from said cancer (*e*.*g*., a control sample) or a previously determined reference range indicates the presence of a relevant cancer.

In particular, OGTA002 can be used for detection, prognosis, diagnosis, or monitoring of breast cancer, colorectal cancer, hepatocellular carcinoma, lung cancer, melanoma, ovarian cancer, pancreatic cancer and renal cell cancer.

In particular, OGTA009 can be used for detection, prognosis, diagnosis, or monitoring of breast cancer, colorectal cancer, lung cancer, ovarian cancer, pancreatic cancer, prostate cancer and renal cell cancer.

In particular, OGTA016 can be used for detection, prognosis, diagnosis, or monitoring of colorectal cancer and lung cancer.

In particular, OGTA028 can be used for detection, prognosis, diagnosis, or monitoring of colorectal cancer, hepatocellular carcinoma, lung cancer, ovarian cancer and renal cell cancer. In particular, OGTA037 can be used for detection, prognosis, diagnosis, or monitoring of colorectal cancer, lung cancer, gastric cancer and ovarian cancer.

In particular, OGTA041 can be used for detection, prognosis, diagnosis, or monitoring of hepatocellular carcinoma, lung cancer and pancreatic cancer.

In particular, OGTA053 can be used for detection, prognosis, diagnosis, or monitoring of colorectal cancer, hepatocellular carcinoma, lung cancer, pancreatic cancer and renal cell cancer.

In particular, OGTA054 can be used for detection, prognosis, diagnosis, or monitoring of colorectal cancer, lung cancer and pancreatic cancer.

In particular, OGTA066 can be used for detection, prognosis, diagnosis, or monitoring of colorectal cancer, lung cancer, pancreatic cancer and renal cell cancer.

In particular, OGTA072 can be used for detection, prognosis, diagnosis, or monitoring of colorectal cancer.

In particular, OGTA074 can be used for detection, prognosis, diagnosis, or monitoring of colorectal cancer and lung cancer.

In particular, OGTA076 can be used for detection, prognosis, diagnosis, or monitoring of chronic lymphocytic leukaemia, colorectal cancer, hepatocellular carcinoma, lung cancer, ovarian cancer, pancreatic cancer and renal cell cancer.

In particular, OGTA085 can be used for detection, prognosis, diagnosis, or monitoring of colorectal cancer, lung cancer, melanoma and retinoblastoma.

In particular, OGTA087 can be used for detection, prognosis, diagnosis, or monitoring of B-cell non-Hodgkin's lymphoma, breast cancer, colorectal cancer, gastric cancer, lung cancer, lymphoid leukaemia and ovarian cancer.

In particular, OGTA088 can be used for detection, prognosis, diagnosis, or monitoring of breast cancer, colorectal cancer, gastric cancer, glioblastoma, hepatocellular carcinoma, lung cancer, lymphoid leukaemia, pancreatic cancer, renal cell cancer and retinoblastoma.

In particular, OGTA089 can be used for detection, prognosis, diagnosis, or monitoring of breast cancer, lung cancer, ovarian cancer and renal cell cancer.

In particular, OGTA091 can be used for detection, prognosis, diagnosis, or monitoring of breast cancer, cervical cancer, chronic lymphocytic leukaemia, colorectal cancer, gastric cancer, hepatocellular carcinoma, lung cancer, melanoma, osteosarcoma, ovarian cancer, pancreatic cancer, prostate cancer and renal cell cancer.

In particular, OGTA098 can be used for detection, prognosis, diagnosis, or monitoring of breast cancer, cervical cancer, colorectal cancer, hepatocellular carcinoma, lung cancer, osteosarcoma, ovarian cancer, pancreatic cancer, prostate cancer and renal cell cancer.

In particular, OGTA101 can be used for detection, prognosis, diagnosis, or monitoring of breast cancer, cervical cancer, colorectal cancer, hepatocellular carcinoma, lung cancer, osteosarcoma, ovarian cancer, pancreatic cancer and renal cell cancer.

In particular, OGTA104 can be used for detection, prognosis, diagnosis, or monitoring of breast cancer, colorectal cancer, hepatocellular carcinoma, lung cancer, ovarian cancer, pancreatic cancer and renal cell cancer.

In particular, OGTA106 can be used for detection, prognosis, diagnosis, or monitoring of cervical cancer, hepatocellular carcinoma, melanoma and pancreatic cancer.

In particular, OGTA112 can be used for detection, prognosis, diagnosis, or monitoring of breast cancer, cervical cancer, chronic lymphocytic leukaemia, hepatocellular carcinoma, pancreatic cancer and renal cell cancer.

In particular, OGTA113 can be used for detection, prognosis, diagnosis, or monitoring of colorectal cancer, lung cancer, ovarian cancer, pancreatic cancer and renal cell cancer.

In particular, OGTA119 can be used for detection, prognosis, diagnosis, or monitoring of B-cell non-Hodgkin's lymphoma, breast cancer, colorectal cancer, gastric cancer, hepatocellular carcinoma, lung cancer, lymphoid leukaemia, neuroblastoma, osteosarcoma, ovarian cancer, pancreatic cancer, prostate cancer and renal cell cancer.

In particular, OGTA124 can be used for detection, prognosis, diagnosis, or monitoring of colorectal cancer, hepatocellular carcinoma, lung cancer, ovarian cancer, pancreatic cancer and renal cell cancer.

In particular, OGTA126 can be used for detection, prognosis, diagnosis, or monitoring of breast cancer, colorectal cancer, hepatocellular carcinoma, lung cancer, melanoma, pancreatic cancer, prostate cancer and renal cell cancer.

In particular, OGTA156 can be used for detection, prognosis, diagnosis, or monitoring of acute T-cell leukaemia, B-cell non-Hodgkin's lymphoma, chronic lymphocytic leukaemia, colorectal cancer, hepatocellular carcinoma, lung cancer, ovarian cancer and renal cell cancer.

In particular, OGTA159 can be used for detection, prognosis, diagnosis, or monitoring of breast cancer, chronic lymphocytic leukaemia, colorectal cancer, hepatocellular carcinoma, lung cancer, melanoma, pancreatic cancer and renal cell cancer.

In particular, OGTA168 can be used for detection, prognosis, diagnosis, or monitoring of glioblastoma and melanoma.

In particular, OGTA169 can be used for detection, prognosis, diagnosis, or monitoring of breast cancer, chronic lymphocytic leukaemia, colorectal cancer, lung cancer and renal cell cancer.

In particular, OGTA174 can be used for detection, prognosis, diagnosis, or monitoring of acute T-cell leukaemia, chronic lymphocytic leukaemia and lung cancer.

In particular, OGTA176 can be used for detection, prognosis, diagnosis, or monitoring of B-cell non-Hodgkin's lymphoma, chronic lymphocytic leukaemia, colorectal cancer, lung cancer and renal cell cancer.

In particular, OGTA177 can be used for detection, prognosis, diagnosis, or monitoring of chronic lymphocytic leukaemia, colorectal cancer, lung cancer, ovarian cancer and renal cell cancer.

In particular, OGTA197 can be used for detection, prognosis, diagnosis, or monitoring of colorectal cancer, hepatocellular carcinoma, lung cancer, melanoma, osteosarcoma, ovarian cancer, pancreatic cancer, prostate cancer and renal cell cancer.

In particular, OGTA202 can be used for detection, prognosis, diagnosis, or monitoring of colorectal cancer and lung cancer.

In particular, OGTA203 can be used for detection, prognosis, diagnosis, or monitoring of lung cancer, ovarian cancer and renal cell cancer.

In particular, OGTA206 can be used for detection, prognosis, diagnosis, or monitoring of breast cancer, colorectal cancer, lung cancer, pancreatic cancer and prostate cancer.

In particular, OGTA213 can be used for detection, prognosis, diagnosis, or monitoring of colorectal cancer and lung cancer.

In particular, OGTA214 can be used for detection, prognosis, diagnosis, or monitoring of colorectal cancer and ovarian cancer.

In particular, OGTA216 can be used for detection, prognosis, diagnosis, or monitoring of breast cancer, colorectal cancer, hepatocellular carcinoma, lung cancer, ovarian cancer, pancreatic cancer and renal cell cancer.

In particular, OGTA222 can be used for detection, prognosis, diagnosis, or monitoring of B-cell non-Hodgkin's lymphoma, colorectal cancer, lung cancer and renal cell cancer.

In particular, OGTA236 can be used for detection, prognosis, diagnosis, or monitoring of colorectal cancer and pancreatic cancer.

In particular, OGTA237 can be used for detection, prognosis, diagnosis, or monitoring of B-cell non-Hodgkin's lymphoma, colorectal cancer, lung cancer, lymphoid leukaemia, prostate cancer and renal cell cancer.

In particular, OGTA247 can be used for detection, prognosis, diagnosis, or monitoring of hepatocellular carcinoma, lung cancer and melanoma.

In particular, OGTA248 can be used for detection, prognosis, diagnosis, or monitoring of lung cancer and renal cell cancer.

In particular, OGTA249 can be used for detection, prognosis, diagnosis, or monitoring of gastric cancer, lung cancer, ovarian cancer and renal cell cancer.

In particular, OGTA257 can be used for detection, prognosis, diagnosis, or monitoring of colorectal cancer, hepatocellular carcinoma, lung cancer, ovarian cancer and pancreatic cancer. In particular, OGTA271 can be used for detection, prognosis, diagnosis, or monitoring of breast cancer, cervical cancer, colorectal cancer, hepatocellular carcinoma, lung cancer, osteosarcoma, pancreatic cancer and renal cell cancer.

In one or more examples of the disclosure:
OGTA002 may, in particular, be characterized as an isoform having a MW substantially as recited (e.g. +/- 10%, particularly +/-5% of the value) in column 1 of any of the rows of Tables 1a-1f
OGTA009 may, in particular, be characterized as an isoform having a MW substantially as recited (e.g. +/- 10%, particularly +/-5% of the value) in column 1 of any of the rows of Tables 2a-2e
OGTA016 may, in particular, be characterized as an isoform having a MW substantially as recited (e.g. +/- 10%, such as +/-5% of the value) in column 1 of any of the rows of Table 3 OGTA028 may, in particular, be characterized as an isoform having a MW substantially as recited (e.g. +/- 10%, such as +/-5% of the value) in column 1 of any of the rows of Table 4 OGTA037 may, in particular, be characterized as an isoform having a MW substantially as recited (e.g. +/- 10%, such as +/-5% of the value) in column 1 of any of the rows of Table 5 OGTA041 may, in particular, be characterized as an isoform having a MW substantially as recited (e.g. +/- 10%, particularly +/-5% of the value) in column 1 of any of the rows of Tables 6a-6c
OGTA053 may, in particular, be characterized as an isoform having a MW substantially as recited (e.g. +/- 10%, such as +/-5% of the value) in column 1 of any of the rows of Table 7 OGTA054 may, in particular, be characterized as an isoform having a MW substantially as recited (e.g. +/- 10%, such as +/-5% of the value) in column 1 of any of the rows of Table 8 OGTA066 may, in particular, be characterized as an isoform having a MW substantially as recited (e.g. +/- 10%, particularly +/-5% of the value) in column 1 of any of the rows of Tables 9a-9b
OGTA072 may, in particular, be characterized as an isoform having a MW substantially as recited (e.g. +/- 10%, such as +/-5% of the value) in column 1 of any of the rows of Table 10 OGTA074 may, in particular, be characterized as an isoform having a MW substantially as recited (e.g. +/- 10%, such as +/-5% of the value) in column 1 of any of the rows of Table 11 OGTA076 may, in particular, be characterized as an isoform having a MW substantially as recited (e.g. +/- 10%, particularly +/-5% of the value) in column 1 of any of the rows of Tables 12a-12c
OGTA085 may, in particular, be characterized as an isoform having a MW substantially as recited (e.g. +/- 10%, particularly +/-5% of the value) in column 1 of any of the rows of Tables 13a-13c
OGTA087 may, in particular, be characterized as an isoform having a MW substantially as recited (e.g. +/- 10%, particularly +/-5% of the value) in column 1 of any of the rows of Tables 14a-14g
OGTA088 may, in particular, be characterized as an isoform having a MW substantially as recited (e.g. +/- 10%, particularly +/-5% of the value) in column 1 of any of the rows of Tables 15a-15i
OGTA089 may, in particular, be characterized as an isoform having a MW substantially as recited (e.g. +/- 10%, particularly +/-5% of the value) in column 1 of any of the rows of Tables 16a-16c
OGTA091 may, in particular, be characterized as an isoform having a MW substantially as recited (e.g. +/- 10%, particularly +/-5% of the value) in column 1 of any of the rows of Tables 17a-171
OGTA098 may, in particular, be characterized as an isoform having a MW substantially as recited (e.g. +/- 10%, particularly +/-5% of the value) in column 1 of any of the rows of Tables 18a-18h
OGTA101 may, in particular, be characterized as an isoform having a MW substantially as recited (e.g. +/- 10%, particularly +/-5% of the value) in column 1 of any of the rows of Tables 19a-19g
OGTA104 may, in particular, be characterized as an isoform having a MW substantially as recited (e.g. +/- 10%, particularly +/-5% of the value) in column 1 of any of the rows of Tables 20a-20d
OGTA106 may, in particular, be characterized as an isoform having a MW substantially as recited (e.g. +/- 10%, particularly +/-5% of the value) in column 1 of any of the rows of Tables 21a-21c
OGTA112 may, in particular, be characterized as an isoform having a MW substantially as recited (e.g. +/- 10%, particularly +/-5% of the value) in column 1 of any of the rows of Tables 22a-22f
OGTA113 may, in particular, be characterized as an isoform having a MW substantially as recited (e.g. +/- 10%, particularly +/-5% of the value) in column 1 of any of the rows of Table 23
OGTA119 may, in particular, be characterized as an isoform having a MW substantially as recited (e.g. +/- 10%, particularly +/-5% of the value) in column 1 of any of the rows of Tables 24a-24l
OGTA124 may, in particular, be characterized as an isoform having a MW substantially as recited (e.g. +/- 10%, particularly +/-5% of the value) in column 1 of any of the rows of Tables 25a-25e
OGTA126 may, in particular, be characterized as an isoform having a MW substantially as recited (e.g. +/- 10%, particularly +/-5% of the value) in column 1 of any of the rows of Tables 26a-26g
OGTA156 may, in particular, be characterized as an isoform having a MW substantially as recited (e.g. +/- 10%, particularly +/-5% of the value) in column 1 of any of the rows of Tables 27a-27d
OGTA159 may, in particular, be characterized as an isoform having a MW substantially as recited (e.g. +/- 10%, particularly +/-5% of the value) in column 1 of any of the rows of Tables 28a-28f
OGTA168 may, in particular, be characterized as an isoform having a MW substantially as recited (e.g. +/- 10%, particularly +/-5% of the value) in column 1 of any of the rows of Tables 29a-29b
OGTA169 may, in particular, be characterized as an isoform having a MW substantially as recited (e.g. +/- 10%, particularly +/-5% of the value) in column 1 of any of the rows of Tables 30a-30b
OGTA174 may, in particular, be characterized as an isoform having a MW substantially as recited (e.g. +/- 10%, particularly +/-5% of the value) in column 1 of any of the rows of Tables 31a-31b
OGTA176 may, in particular, be characterized as an isoform having a MW substantially as recited (e.g. +/- 10%, particularly +/-5% of the value) in column 1 of any of the rows of Tables 32a-32b
OGTA177 may, in particular, be characterized as an isoform having a MW substantially as recited (e.g. +/- 10%, such as +/-5% of the value) in column 1 of any of the rows of Table 33 OGTA197 may, in particular, be characterized as an isoform having a MW substantially as recited (e.g. +/- 10%, particularly +/-5% of the value) in column 1 of any of the rows of Tables 34a-34i
OGTA202 may, in particular, be characterized as an isoform having a MW substantially as recited (e.g. +/- 10%, particularly +/-5% of the value) in column 1 of any of the rows of Table 35
OGTA203 may, in particular, be characterized as an isoform having a MW substantially as recited (e.g. +/- 10%, particularly +/-5% of the value) in column 1 of any of the rows of Tables 36a-36b
OGTA206 may, in particular, be characterized as an isoform having a MW substantially as recited (e.g. +/- 10%, particularly +/-5% of the value) in column 1 of any of the rows of Tables 37a-37c
OGTA213 may, in particular, be characterized as an isoform having a MW substantially as recited (e.g. +/- 10%, such as +/-5% of the value) in column 1 of any of the rows of Table 38 OGTA214 may, in particular, be characterized as an isoform having a MW substantially as recited (e.g. +/- 10%, particularly +/-5% of the value) in column 1 of any of the rows of Table 39
OGTA216 may, in particular, be characterized as an isoform having a MW substantially as recited (e.g. +/- 10%, particularly +/-5% of the value) in column 1 of any of the rows of Tables 40a-40b
OGTA222 may, in particular, be characterized as an isoform having a MW substantially as recited (e.g. +/- 10%, particularly +/-5% of the value) in column 1 of any of the rows of Tables 41a-41b
OGTA236 may, in particular, be characterized as an isoform having a MW substantially as recited (e.g. +/- 10%, such as +/-5% of the value) in column 1 of any of the rows of Table 42 OGTA237 may, in particular, be characterized as an isoform having a MW substantially as recited (e.g. +/- 10%, particularly +/-5% of the value) in column 1 of any of the rows of Tables 43a-43c
OGTA247 may, in particular, be characterized as an isoform having a MW substantially as recited (e.g. +/- 10%, particularly +/-5% of the value) in column 1 of any of the rows of Tables 44a-44b
OGTA248 may, in particular, be characterized as an isoform having a MW substantially as recited (e.g. +/- 10%, such as +/-5% of the value) in column 1 of any of the rows of Table 45 OGTA249 may, in particular, be characterized as an isoform having a MW substantially as recited (e.g. +/- 10%, particularly +/-5% of the value) in column 1 of any of the rows of Table 46.
OGTA257 may, in particular, be characterized as an isoform having a MW substantially as recited (e.g. +/- 10%, such as +/-5% of the value) in column 1 of any of the rows of Table 47 OGTA271 may, in particular, be characterized as an isoform having a MW substantially as recited (e.g. +/- 10%, particularly +/-5% of the value) in column 1 of any of the rows of Tables 48a-48h

In relation to fragments, immunogenic fragments or antigenic fragments of OGTA002:
- for breast cancer applications: for example these comprise one or more of the sequences identified as tryptic sequences in the 3^{rd} column of Table 1a;
- for colorectal cancer applications: for example these comprise one or more of the sequences identified as tryptic sequences in the 3^{rd} column of Table 1b and Table 49a;
- for hepatocellular carcinoma applications: , for example these comprise one or more of the sequences identified as tryptic sequences in the 3^{rd} column of Table 1c and Table 49c;
- for lung cancer applications: for example these comprise one or more of the sequences identified as tryptic sequences in the 3^{rd} column of Table 49d;
- for melanoma applications: for example these comprise one or more of the sequences identified as tryptic sequences in the 3^{rd} column of Table 1d;
- for ovarian cancer applications: for example these comprise one or more of the sequences identified as tryptic sequences in the 3^{rd} column of Table 1e;
- for pancreatic cancer applications: for example these comprise one or more of the sequences identified as tryptic sequences in the 3^{rd} column of Table 1f;
- for renal cell cancer applications: for example these comprise one or more of the sequences identified as tryptic sequences in the 3^{rd} column of Table 49b.

In relation to fragments, immunogenic fragments or antigenic fragments of OGTA009:
- for breast cancer applications: for example these comprise one or more of the sequences identified as tryptic sequences in the 3^{rd} column of Table 2a;
- for colorectal cancer applications: for example these comprise one or more of the sequences identified as tryptic sequences in the 3^{rd} column of Table 2b and Table 50a;
- for lung cancer applications: for example these comprise one or more of the sequences identified as tryptic sequences in the 3^{rd} column of Table 50b;
- for ovarian cancer applications: for example these comprise one or more of the sequences identified as tryptic sequences in the 3^{rd} column of Table 50c;
- for pancreatic cancer applications: for example these comprise one or more of the sequences identified as tryptic sequences in the 3^{rd} column of Table 2c;
- for prostate cancer applications: for example these comprise one or more of the sequences identified as tryptic sequences in the 3^{rd} column of Table 2d;
- for renal cell cancer applications: for example these comprise one or more of the sequences identified as tryptic sequences in the 3^{rd} column of Table 2e;

In relation to fragments, immunogenic fragments or antigenic fragments of OGTA016:
- for colorectal cancer applications: for example these comprise one or more of the sequences identified as tryptic sequences in the 3^{rd} column of Table 3 and Table 51a;
- for lung cancer applications: for example these comprise one or more of the sequences identified as tryptic sequences in the 3^{rd} column of Table 51b.

In relation to fragments, immunogenic fragments or antigenic fragments of OGTA028:
- for colorectal cancer applications: for example these comprise one or more of the sequences identified as tryptic sequences in the 3^{rd} column of Table 52a;
- for hepatocellular carcinoma applications: for example these comprise one or more of the sequences identified as tryptic sequences in the 3^{rd} column of Table 52c;
- for lung cancer applications: for example these comprise one or more of the sequences identified as tryptic sequences in the 3^{rd} column of Table 52d and Table 52e;
- for ovarian cancer applications: for example these comprise one or more of the sequences identified as tryptic sequences in the 3^{rd} column of Table 4 and Table 52f;
- for renal cell cancer applications: for example these comprise one or more of the sequences identified as tryptic sequences in the 3^{rd} column of Table 52b.

In relation to fragments, immunogenic fragments or antigenic fragments of OGTA037:
- for colorectal cancer applications: for example these comprise one or more of the sequences identified as tryptic sequences in the 3^{rd} column of Table 53a;
- for lung cancer applications: for example these comprise one or more of the sequences identified as tryptic sequences in the 3^{rd} column of Table 53b;
- for gastric cancer applications: for example these comprise one or more of the sequences identified as tryptic sequences in the 3^{rd} column of Table 5;
- for ovarian cancer applications: for example these comprise one or more of the sequences identified as tryptic sequences in the 3^{rd} column of Table 53c.

In relation to fragments, immunogenic fragments or antigenic fragments of OGTA041:
- for hepatocellular carcinoma applications: for example these comprise one or more of the sequences identified as tryptic sequences in the 3^{rd} column of Table 6a;
- for lung cancer applications: for example these comprise one or more of the sequences identified as tryptic sequences in the 3^{rd} column of Table 6b and Table 54;
- for pancreatic cancer applications: for example these comprise one or more of the sequences identified as tryptic sequences in the 3^{rd} column of Table 6c.

In relation to fragments, immunogenic fragments or antigenic fragments of OGTA053:
- for colorectal cancer applications: for example these comprise one or more of the sequences identified as tryptic sequences in the 3^{rd} column of Table 55a;
- for hepatocellular carcinoma applications: for example these comprise one or more of the sequences identified as tryptic sequences in the 3^{rd} column of Table 55c;
- for lung cancer applications: for example these comprise one or more of the sequences identified as tryptic sequences in the 3^{rd} column of Table 55d and Table 55e;
- for pancreatic cancer applications: for example these comprise one or more of the sequences identified as tryptic sequences in the 3^{rd} column of Table 7;
- for renal cell cancer applications: for example these comprise one or more of the sequences identified as tryptic sequences in the 3^{rd} column of Table 55b.

In relation to fragments, immunogenic fragments or antigenic fragments of OGTA054:
- for colorectal cancer applications: for example these comprise one or more of the sequences identified as tryptic sequences in the 3^{rd} column of Table 56a;
- for lung cancer applications: for example these comprise one or more of the sequences identified as tryptic sequences in the 3^{rd} column of Table 56b;
- for pancreatic cancer applications: for example these comprise one or more of the sequences identified as tryptic sequences in the 3^{rd} column of Table 8.

In relation to fragments, immunogenic fragments or antigenic fragments of OGTA066:
- for colorectal cancer applications: for example these comprise one or more of the sequences identified as tryptic sequences in the 3^{rd} column of Table 9a and Table 57a;
- for lung cancer applications: for example these comprise one or more of the sequences identified as tryptic sequences in the 3^{rd} column of Table 57c;
- for pancreatic cancer applications: for example these comprise one or more of the sequences identified as tryptic sequences in the 3^{rd} column of Table 9b;
- for renal cell cancer applications: for example these comprise one or more of the sequences identified as tryptic sequences in the 3^{rd} column of Table 57b.

In relation to fragments, immunogenic fragments or antigenic fragments of OGTA072:
- for colorectal cancer applications: for example these comprise one or more of the sequences identified as tryptic sequences in the 3^{rd} column of Table 10.

In relation to fragments, immunogenic fragments or antigenic fragments of OGTA074:
- for colorectal cancer applications: for example these comprise one or more of the sequences identified as tryptic sequences in the 3^{rd} column of Table 11;
- for lung cancer applications: for example these comprise one or more of the sequences identified as tryptic sequences in the 3^{rd} column of Table 58.

In relation to fragments, immunogenic fragments or antigenic fragments of OGTA076:
- for chronic lymphocytic leukaemia applications: for example these comprise one or more of the sequences identified as tryptic sequences in the 3^{rd} column of Table 12a;
- for colorectal cancer applications: for example these comprise one or more of the sequences identified as tryptic sequences in the 3^{rd} column of Table 12b and Table 59a;
- for hepatocellular carcinoma applications: for example these comprise one or more of the sequences identified as tryptic sequences in the 3^{rd} column of Table 59c;
- for lung cancer applications: for example these comprise one or more of the sequences identified as tryptic sequences in the 3^{rd} column of Table 59d and Table 59e;
- for ovarian cancer applications: for example these comprise one or more of the sequences identified as tryptic sequences in the 3^{rd} column of Table 59f;
- for pancreatic cancer applications: for example these comprise one or more of the sequences identified as tryptic sequences in the 3^{rd} column of Table 12c;
- for renal cell cancer applications: for example these comprise one or more of the sequences identified as tryptic sequences in the 3^{rd} column of Table 59b.

In relation to fragments, immunogenic fragments or antigenic fragments of OGTA085:
- for colorectal cancer applications: for example these comprise one or more of the sequences identified as tryptic sequences in the 3^{rd} column of Table 60a;
- for lung cancer applications: for example these comprise one or more of the sequences identified as tryptic sequences in the 3^{rd} column of Table 13a and Table 60b;
- for melanoma applications: for example these comprise one or more of the sequences identified as tryptic sequences in the 3^{rd} column of Table 13b;
- for retinoblastoma applications: for example these comprise one or more of the sequences identified as tryptic sequences in the 3^{rd} column of Table 13c.

In relation to fragments, immunogenic fragments or antigenic fragments of OGTA087:
- for B-cell non-Hodgkin's lymphoma applications: for example these comprise one or more of the sequences identified as tryptic sequences in the 3^{rd} column of Table 14a;
- for breast cancer applications: for example these comprise one or more of the sequences identified as tryptic sequences in the 3^{rd} column of Table 14b;
- for colorectal cancer applications: for example these comprise one or more of the sequences identified as tryptic sequences in the 3^{rd} column of Table 14c;
- for gastric cancer applications: for example these comprise one or more of the sequences identified as tryptic sequences in the 3^{rd} column of Table 14d;
- for lung cancer applications: for example these comprise one or more of the sequences identified as tryptic sequences in the 3^{rd} column of Table 14e;
- for lymphoid leukaemia applications: for example these comprise one or more of the sequences identified as tryptic sequences in the 3^{rd} column of Table 14f;
- for ovarian cancer applications: for example these comprise one or more of the sequences identified as tryptic sequences in the 3^{rd} column of Table 14g.

In relation to fragments, immunogenic fragments or antigenic fragments of OGTA088:
- for breast cancer applications: for example these comprise one or more of the sequences identified as tryptic sequences in the 3^{rd} column of Table 15a;
- for colorectal cancer applications: for example these comprise one or more of the sequences identified as tryptic sequences in the 3^{rd} column of Table 61a;
- for gastric cancer applications: for example these comprise one or more of the sequences identified as tryptic sequences in the 3^{rd} column of Table 15b;
- for glioblastoma applications: for example these comprise one or more of the sequences identified as tryptic sequences in the 3^{rd} column of Table 15c;
- for hepatocellular carcinoma applications: for example these comprise one or more of the sequences identified as tryptic sequences in the 3^{rd} column of Table 15d;
- for lung cancer applications: for example these comprise one or more of the sequences identified as tryptic sequences in the 3^{rd} column of Table 15e and Table 61b;
- for lymphoid leukaemia applications: for example these comprise one or more of the sequences identified as tryptic sequences in the 3^{rd} column of Table 15f;
- for pancreatic cancer applications: for example these comprise one or more of the sequences identified as tryptic sequences in the 3^{rd} column of Table 15g;
- for renal cell cancer applications: for example these comprise one or more of the sequences identified as tryptic sequences in the 3^{rd} column of Table 15h;
- for retinoblastoma applications: for example these comprise one or more of the sequences identified as tryptic sequences in the 3^{rd} column of Table 15i.

In relation to fragments, immunogenic fragments or antigenic fragments of OGTA089:
- for breast cancer applications: for example these comprise one or more of the sequences identified as tryptic sequences in the 3^{rd} column of Table 16a;
- for lung cancer applications: for example these comprise one or more of the sequences identified as tryptic sequences in the 3^{rd} column of Table 16b and Table 62b;
- for ovarian cancer applications: for example these comprise one or more of the sequences identified as tryptic sequences in the 3^{rd} column of Table 16c and Table 62c;
- for renal cell cancer applications: for example these comprise one or more of the sequences identified as tryptic sequences in the 3^{rd} column of Table 62a.

In relation to fragments, immunogenic fragments or antigenic fragments of OGTA091:
- for breast cancer applications: for example these comprise one or more of the sequences identified as tryptic sequences in the 3^{rd} column of Table 17a;
- for cervical cancer applications: for example these comprise one or more of the sequences identified as tryptic sequences in the 3^{rd} column of Table 17b;
- for chronic lymphocytic leukaemia applications: for example these comprise one or more of the sequences identified as tryptic sequences in the 3^{rd} column of Table 17c;
- for colorectal cancer applications: for example these comprise one or more of the sequences identified as tryptic sequences in the 3^{rd} column of Table 63a;
- for gastric cancer applications: for example these comprise one or more of the sequences identified as tryptic sequences in the 3^{rd} column of Table 17d;
- for hepatocellular carcinoma applications: for example these comprise one or more of the sequences identified as tryptic sequences in the 3^{rd} column of Table 17e and Table 63c;
- for lung cancer applications: for example these comprise one or more of the sequences identified as tryptic sequences in the 3^{rd} column of Table 17f, Table 63d and Table 63e;
- for melanoma applications: for example these comprise one or more of the sequences identified as tryptic sequences in the 3^{rd} column of Table 17g;
- for osteosarcoma applications: for example these comprise one or more of the sequences identified as tryptic sequences in the 3^{rd} column of Table 17h;
- for ovarian cancer applications: for example these comprise one or more of the sequences identified as tryptic sequences in the 3^{rd} column of Table 17i and Table 63f;
- for pancreatic cancer applications: for example these comprise one or more of the sequences identified as tryptic sequences in the 3^{rd} column of Table 17j;
- for prostate cancer applications: for example these comprise one or more of the sequences identified as tryptic sequences in the 3^{rd} column of Table 17k;
- for renal cell cancer applications: for example these comprise one or more of the sequences identified as tryptic sequences in the 3^{rd} column of Table 171 and Table 63b.

In relation to fragments, immunogenic fragments or antigenic fragments of OGTA098:
- for breast cancer applications: for example these comprise one or more of the sequences identified as tryptic sequences in the 3^{rd} column of Table 18a;
- for cervical cancer applications: for example these comprise one or more of the sequences identified as tryptic sequences in the 3^{rd} column of Table 18b;
- for colorectal cancer applications: for example these comprise one or more of the sequences identified as tryptic sequences in the 3^{rd} column of Table 64a;
- for hepatocellular carcinoma applications: for example these comprise one or more of the sequences identified as tryptic sequences in the 3^{rd} column of Table 18c;
- for lung cancer applications: for example these comprise one or more of the sequences identified as tryptic sequences in the 3^{rd} column of Table 18d, Table 64c and Table 64d;
- for osteosarcoma applications: for example these comprise one or more of the sequences identified as tryptic sequences in the 3^{rd} column of Table 18e;
- for ovarian cancer applications: for example these comprise one or more of the sequences identified as tryptic sequences in the 3^{rd} column of Table 64e;
- for pancreatic cancer applications: for example these comprise one or more of the sequences identified as tryptic sequences in the 3^{rd} column of Table 18f;
- for prostate cancer applications: for example these comprise one or more of the sequences identified as tryptic sequences in the 3^{rd} column of Table 18g;
- for renal cell cancer applications: for example these comprise one or more of the sequences identified as tryptic sequences in the 3^{rd} column of Table 18h and Table 64b.

In relation to fragments, immunogenic fragments or antigenic fragments of OGTA101:
- for breast cancer applications: for example these comprise one or more of the sequences identified as tryptic sequences in the 3^{rd} column of Table 19a;
- for cervical cancer applications: for example these comprise one or more of the sequences identified as tryptic sequences in the 3^{rd} column of Table 19b;
- for colorectal cancer applications: for example these comprise one or more of the sequences identified as tryptic sequences in the 3^{rd} column of Table 19c and Table 65a;
- for hepatocellular carcinoma applications: for example these comprise one or more of the sequences identified as tryptic sequences in the 3^{rd} column of Table 19d and Table 65c;
- for lung cancer applications: for example these comprise one or more of the sequences identified as tryptic sequences in the 3^{rd} column of Table 19e and Table 65d;
- for osteosarcoma applications: for example these comprise one or more of the sequences identified as tryptic sequences in the 3^{rd} column of Table 19f;
- for ovarian cancer applications: for example these comprise one or more of the sequences identified as tryptic sequences in the 3^{rd} column of Table 65e;
- for pancreatic cancer applications: for example these comprise one or more of the sequences identified as tryptic sequences in the 3^{rd} column of Table 19g;
- for renal cell cancer applications: for example these comprise one or more of the sequences identified as tryptic sequences in the 3^{rd} column of Table 65b.

In relation to fragments, immunogenic fragments or antigenic fragments of OGTA104:
- for breast cancer applications: for example these comprise one or more of the sequences identified as tryptic sequences in the 3^{rd} column of Table 20a;
- for colorectal cancer applications: for example these comprise one or more of the sequences identified as tryptic sequences in the 3^{rd} column of Table 66a;
- for hepatocellular carcinoma applications: for example these comprise one or more of the sequences identified as tryptic sequences in the 3^{rd} column of Table 20b and Table 66c;
- for lung cancer applications: for example these comprise one or more of the sequences identified as tryptic sequences in the 3^{rd} column of Table 66d;
- for ovarian cancer applications: for example these comprise one or more of the sequences identified as tryptic sequences in the 3^{rd} column of Table 20c and Table 66e;
- for pancreatic cancer applications: for example these comprise one or more of the sequences identified as tryptic sequences in the 3^{rd} column of Table 20d;
- for renal cell cancer applications: for example these comprise one or more of the sequences identified as tryptic sequences in the 3^{rd} column of Table 66b.

In relation to fragments, immunogenic fragments or antigenic fragments of OGTA106:
- for cervical cancer applications: for example these comprise one or more of the sequences identified as tryptic sequences in the 3^{rd} column of Table 21a;
- for hepatocellular carcinoma applications: for example these comprise one or more of the sequences identified as tryptic sequences in the 3^{rd} column of Table 67;
- for melanoma applications: for example these comprise one or more of the sequences identified as tryptic sequences in the 3^{rd} column of Table 21b;
- for pancreatic cancer applications: for example these comprise one or more of the sequences identified as tryptic sequences in the 3^{rd} column of Table 21c.

In relation to fragments, immunogenic fragments or antigenic fragments of OGTA112:
- for breast cancer applications: for example these comprise one or more of the sequences identified as tryptic sequences in the 3^{rd} column of Table 22a;
- for cervical cancer applications: for example these comprise one or more of the sequences identified as tryptic sequences in the 3^{rd} column of Table 22b;
- for chronic lymphocytic leukaemia applications: for example these comprise one or more of the sequences identified as tryptic sequences in the 3^{rd} column of Table 22c;
- for hepatocellular carcinoma applications: for example these comprise one or more of the sequences identified as tryptic sequences in the 3^{rd} column of Table 22d;
- for pancreatic cancer applications: for example these comprise one or more of the sequences identified as tryptic sequences in the 3^{rd} column of Table 22e;
- for renal cell cancer applications: for example these comprise one or more of the sequences identified as tryptic sequences in the 3^{rd} column of Table 22f and Table 68.

In relation to fragments, immunogenic fragments or antigenic fragments of OGTA113:
- for colorectal cancer applications: for example these comprise one or more of the sequences identified as tryptic sequences in the 3^{rd} column of Table 69a;
- for lung cancer applications: for example these comprise one or more of the sequences identified as tryptic sequences in the 3^{rd} column of Table 69c;
- for ovarian cancer applications: for example these comprise one or more of the sequences identified as tryptic sequences in the 3^{rd} column of Table 69d;
- for pancreatic cancer applications: for example these comprise one or more of the sequences identified as tryptic sequences in the 3^{rd} column of Table 23;
- for renal cell cancer applications: for example these comprise one or more of the sequences identified as tryptic sequences in the 3^{rd} column of Table 69b.

In relation to fragments, immunogenic fragments or antigenic fragments of OGTA119:
- for B-cell non-Hodgkin's lymphoma applications: for example these comprise one or more of the sequences identified as tryptic sequences in the 3^{rd} column of Table 24a;
- for breast cancer applications: for example these comprise one or more of the sequences identified as tryptic sequences in the 3^{rd} column of Table 24b;
- for colorectal cancer applications: for example these comprise one or more of the sequences identified as tryptic sequences in the 3^{rd} column of Table 24c and Table 70a;
- for gastric cancer applications: for example these comprise one or more of the sequences identified as tryptic sequences in the 3^{rd} column of Table 24d;
- for hepatocellular carcinoma applications: for example these comprise one or more of the sequences identified as tryptic sequences in the 3^{rd} column of Table 24e;
- for lung cancer applications: for example these comprise one or more of the sequences identified as tryptic sequences in the 3^{rd} column of Table 24f and Table 70b;
- for lymphoid leukaemia applications: for example these comprise one or more of the sequences identified as tryptic sequences in the 3^{rd} column of Table 24g;
- for neuroblastoma applications: for example these comprise one or more of the sequences identified as tryptic sequences in the 3^{rd} column of Table 24h;
- for osteosarcoma applications: for example these comprise one or more of the sequences identified as tryptic sequences in the 3^{rd} column of Table 24i;
- for ovarian cancer applications: for example these comprise one or more of the sequences identified as tryptic sequences in the 3^{rd} column of Table 70c;
- for pancreatic cancer applications: for example these comprise one or more of the sequences identified as tryptic sequences in the 3^{rd} column of Table 24j;
- for prostate cancer applications: for example these comprise one or more of the sequences identified as tryptic sequences in the 3^{rd} column of Table 24k;
- for renal cell cancer applications: for example these comprise one or more of the sequences identified as tryptic sequences in the 3^{rd} column of Table 24l.

In relation to fragments, immunogenic fragments or antigenic fragments of OGTA124:
- for colorectal cancer applications: for example these comprise one or more of the sequences identified as tryptic sequences in the 3^{rd} column of Table 71a;
- for hepatocellular carcinoma applications: for example these comprise one or more of the sequences identified as tryptic sequences in the 3^{rd} column of Table 25a and Table 71c;

- for lung cancer applications: for example these comprise one or more of the sequences identified as tryptic sequences in the 3^{rd} column of Table 25b and Table 71d;
- for ovarian cancer applications: for example these comprise one or more of the sequences identified as tryptic sequences in the 3^{rd} column of Table 25c and Table 71e;
- for pancreatic cancer applications: for example these comprise one or more of the sequences identified as tryptic sequences in the 3^{rd} column of Table 25d;
- for renal cell cancer applications: for example these comprise one or more of the sequences identified as tryptic sequences in the 3^{rd} column of Table 25e and Table 71b.

In relation to fragments, immunogenic fragments or antigenic fragments of OGTA126:
- for breast cancer applications: for example these comprise one or more of the sequences identified as tryptic sequences in the 3^{rd} column of Table 26a;
- for colorectal cancer applications: for example these comprise one or more of the sequences identified as tryptic sequences in the 3^{rd} column of Table 26b;
- for hepatocellular carcinoma applications: for example these comprise one or more of the sequences identified as tryptic sequences in the 3^{rd} column of Table 26c;
- for lung cancer applications: for example these comprise one or more of the sequences identified as tryptic sequences in the 3^{rd} column of Table 72;
- for melanoma applications: for example these comprise one or more of the sequences identified as tryptic sequences in the 3^{rd} column of Table 26d;
- for pancreatic cancer applications: for example these comprise one or more of the sequences identified as tryptic sequences in the 3^{rd} column of Table 26e;
- for prostate cancer applications: for example these comprise one or more of the sequences identified as tryptic sequences in the 3^{rd} column of Table 26f;
- for renal cell cancer applications: for example these comprise one or more of the sequences identified as tryptic sequences in the 3^{rd} column of Table 26g.

In relation to fragments, immunogenic fragments or antigenic fragments of OGTA156:
- for acute T-cell leukaemia applications: for example these comprise one or more of the sequences identified as tryptic sequences in the 3^{rd} column of Table 27a;
- for B-cell non-Hodgkin's lymphoma applications: for example these comprise one or more of the sequences identified as tryptic sequences in the 3^{rd} column of Table 27b;
- for chronic lymphocytic leukaemia applications: for example these comprise one or more of the sequences identified as tryptic sequences in the 3^{rd} column of Table 27c;
- for colorectal cancer applications: for example these comprise one or more of the sequences identified as tryptic sequences in the 3^{rd} column of Table 73a;
- for hepatocellular carcinoma applications: for example these comprise one or more of the sequences identified as tryptic sequences in the 3^{rd} column of Table 27d;
- for lung cancer applications: for example these comprise one or more of the sequences identified as tryptic sequences in the 3^{rd} column of Table 73c;
- for ovarian cancer applications: for example these comprise one or more of the sequences identified as tryptic sequences in the 3^{rd} column of Table 73d;
- for renal cell cancer applications: for example these comprise one or more of the sequences identified as tryptic sequences in the 3^{rd} column of Table 73b.

In relation to fragments, immunogenic fragments or antigenic fragments of OGTA159:
- for breast cancer applications: for example these comprise one or more of the sequences identified as tryptic sequences in the 3^{rd} column of Table 28a;
- for chronic lymphocytic leukaemia applications: for example these comprise one or more of the sequences identified as tryptic sequences in the 3^{rd} column of Table 28b;
- for colorectal cancer applications: for example these comprise one or more of the sequences identified as tryptic sequences in the 3^{rd} column of Table 28c;
- for hepatocellular carcinoma applications: for example these comprise one or more of the sequences identified as tryptic sequences in the 3^{rd} column of Table 28d;
- for lung cancer applications: for example these comprise one or more of the sequences identified as tryptic sequences in the 3^{rd} column of Table 74b;
- for melanoma applications: for example these comprise one or more of the sequences identified as tryptic sequences in the 3^{rd} column of Table 28e;
- for pancreatic cancer applications: for example these comprise one or more of the sequences identified as tryptic sequences in the 3^{rd} column of Table 28f;
- for renal cell cancer applications: for example these comprise one or more of the sequences identified as tryptic sequences in the 3^{rd} column of Table 74a.

In relation to fragments, immunogenic fragments or antigenic fragments of OGTA168:
- for glioblastoma applications: for example these comprise one or more of the sequences identified as tryptic sequences in the 3^{rd} column of Table 29a;
- for melanoma applications: for example these comprise one or more of the sequences identified as tryptic sequences in the 3^{rd} column of Table 29b.

In relation to fragments, immunogenic fragments or antigenic fragments of OGTA169:
- for breast cancer applications: for example these comprise one or more of the sequences identified as tryptic sequences in the 3^{rd} column of Table 30a;
- for chronic lymphocytic leukaemia applications: for example these comprise one or more of the sequences identified as tryptic sequences in the 3^{rd} column of Table 30b;
- for colorectal cancer applications: for example these comprise one or more of the sequences identified as tryptic sequences in the 3^{rd} column of Table 75a;
- for lung cancer applications: for example these comprise one or more of the sequences identified as tryptic sequences in the 3^{rd} column of Table 75c;
- for renal cell cancer applications: for example these comprise one or more of the sequences identified as tryptic sequences in the 3^{rd} column of Table 75b.

In relation to fragments, immunogenic fragments or antigenic fragments of OGTA174:
- for acute T-cell leukaemia applications: for example these comprise one or more of the sequences identified as tryptic sequences in the 3^{rd} column of Table 31a;
- for chronic lymphocytic leukaemia applications: for example these comprise one or more of the sequences identified as tryptic sequences in the 3^{rd} column of Table 31b;
- for lung cancer applications: for example these comprise one or more of the sequences identified as tryptic sequences in the 3^{rd} column of Table 76.

In relation to fragments, immunogenic fragments or antigenic fragments of OGTA176:
- for B-cell non-Hodgkin's lymphoma applications: for example these comprise one or more of the sequences identified as tryptic sequences in the 3^{rd} column of Table 32a;
- for chronic lymphocytic leukaemia applications: for example these comprise one or more of the sequences identified as tryptic sequences in the 3^{rd} column of Table 32b;
- for colorectal cancer applications: for example these comprise one or more of the sequences identified as tryptic sequences in the 3^{rd} column of Table 77a;
- for lung cancer applications: for example these comprise one or more of the sequences identified as tryptic sequences in the 3^{rd} column of Table 77c and Table 77d;
- for renal cell cancer applications: for example these comprise one or more of the sequences identified as tryptic sequences in the 3^{rd} column of Table 77b.

In relation to fragments, immunogenic fragments or antigenic fragments of OGTA177:
- for chronic lymphocytic leukaemia applications: for example these comprise one or more of the sequences identified as tryptic sequences in the 3^{rd} column of Table 33;
- for colorectal cancer applications: for example these comprise one or more of the sequences identified as tryptic sequences in the 3^{rd} column of Table 78a;
- for lung cancer applications: for example these comprise one or more of the sequences identified as tryptic sequences in the 3^{rd} column of Table 78c and Table 78d;
- for ovarian cancer applications: for example these comprise one or more of the sequences identified as tryptic sequences in the 3^{rd} column of Table 78e;
- for renal cell cancer applications: for example these comprise one or more of the sequences identified as tryptic sequences in the 3^{rd} column of Table 78b;

In relation to fragments, immunogenic fragments or antigenic fragments of OGTA197:
- for colorectal cancer applications: for example these comprise one or more of the sequences identified as tryptic sequences in the 3^{rd} column of Table 34a and Table 79a;
- for hepatocellular carcinoma applications: for example these comprise one or more of the sequences identified as tryptic sequences in the 3^{rd} column of Table 34b and Table 79c;
- for lung cancer applications: for example these comprise one or more of the sequences identified as tryptic sequences in the 3^{rd} column of Table 34c, Table 79d and Table 79e;
- for melanoma applications: for example these comprise one or more of the sequences identified as tryptic sequences in the 3^{rd} column of Table 34d;
- for osteosarcoma applications: for example these comprise one or more of the sequences identified as tryptic sequences in the 3^{rd} column of Table 34e;
- for ovarian cancer applications: for example these comprise one or more of the sequences identified as tryptic sequences in the 3^{rd} column of Table 34f;
- for pancreatic cancer applications: for example these comprise one or more of the sequences identified as tryptic sequences in the 3^{rd} column of Table 34g;
- for prostate cancer applications: for example these comprise one or more of the sequences identified as tryptic sequences in the 3^{rd} column of Table 34h;
- for renal cell cancer applications: for example these comprise one or more of the sequences identified as tryptic sequences in the 3^{rd} column of Table 34i and Table 79b.

In relation to fragments, immunogenic fragments or antigenic fragments of OGTA0202:
- for colorectal cancer applications: for example these comprise one or more of the sequences identified as tryptic sequences in the 3^{rd} column of Table 35 and Table 80a;
- for lung cancer applications: for example these comprise one or more of the sequences identified as tryptic sequences in the 3^{rd} column of Table 80b.

In relation to fragments, immunogenic fragments or antigenic fragments of OGTA203:
- for lung cancer applications: for example these comprise one or more of the sequences identified as tryptic sequences in the 3^{rd} column of Table 81b;
- for ovarian cancer applications: for example these comprise one or more of the sequences identified as tryptic sequences in the 3^{rd} column of Table 36a;
- for renal cell cancer applications: for example these comprise one or more of the sequences identified as tryptic sequences in the 3^{rd} column of Table 36b and Table 81a.

In relation to fragments, immunogenic fragments or antigenic fragments of OGTA206:
- for breast cancer applications: for example these comprise one or more of the sequences identified as tryptic sequences in the 3^{rd} column of Table 37a;
- for colorectal cancer applications: for example these comprise one or more of the sequences identified as tryptic sequences in the 3^{rd} column of Table 82a;
- for lung cancer applications: for example these comprise one or more of the sequences identified as tryptic sequences in the 3^{rd} column of Table 82b;
- for pancreatic cancer applications: for example these comprise one or more of the sequences identified as tryptic sequences in the 3^{rd} column of Table 37b;
- for prostate cancer applications: for example these comprise one or more of the sequences identified as tryptic sequences in the 3^{rd} column of Table 37c.

In relation to fragments, immunogenic fragments or antigenic fragments of OGTA213:
- for colorectal cancer applications: for example these comprise one or more of the sequences identified as tryptic sequences in the 3^{rd} column of Table 83a;
- for lung cancer applications: for example these comprise one or more of the sequences identified as tryptic sequences in the 3^{rd} column of Table 38 and Table 83b.

In relation to fragments, immunogenic fragments or antigenic fragments of OGTA214:
- for colorectal cancer applications: for example these comprise one or more of the sequences identified as tryptic sequences in the 3^{rd} column of Table 84;
- for ovarian cancer applications: for example these comprise one or more of the sequences identified as tryptic sequences in the 3^{rd} column of Table 39.

In relation to fragments, immunogenic fragments or antigenic fragments of OGTA216:
- for breast cancer applications: for example these comprise one or more of the sequences identified as tryptic sequences in the 3^{rd} column of Table 40a;
- for colorectal cancer applications: for example these comprise one or more of the sequences identified as tryptic sequences in the 3^{rd} column of Table 40b and Table 85a;
- for hepatocellular carcinoma applications: for example these comprise one or more of the sequences identified as tryptic sequences in the 3^{rd} column of Table 85c;
- for lung cancer applications: for example these comprise one or more of the sequences identified as tryptic sequences in the 3^{rd} column of Table 85d and Table 85e;
- for ovarian cancer applications: for example these comprise one or more of the sequences identified as tryptic sequences in the 3^{rd} column of Table 85f;
- for pancreatic cancer applications: for example these comprise one or more of the sequences identified as tryptic sequences in the 3^{rd} column of Table 40c;
- for renal cell cancer applications: for example these comprise one or more of the sequences identified as tryptic sequences in the 3^{rd} column of Table 85b.

In relation to fragments, immunogenic fragments or antigenic fragments of OGTA222:
- for B-cell non-Hodgkin's lymphoma applications: for example these comprise one or more of the sequences identified as tryptic sequences in the 3^{rd} column of Table 41a;
- for colorectal cancer applications: for example these comprise one or more of the sequences identified as tryptic sequences in the 3^{rd} column of Table 41b and Table 86a;
- for lung cancer applications: for example these comprise one or more of the sequences identified as tryptic sequences in the 3^{rd} column of Table 86c;
- for renal cell applications: for example these comprise one or more of the sequences identified as tryptic sequences in the 3^{rd} column of Table 86b.

In relation to fragments, immunogenic fragments or antigenic fragments of OGTA236:
- for colorectal cancer applications: for example these comprise one or more of the sequences identified as tryptic sequences in the 3^{rd} column of Table 87;
- for pancreatic cancer applications: for example these comprise one or more of the sequences identified as tryptic sequences in the 3^{rd} column of Table 42.

In relation to fragments, immunogenic fragments or antigenic fragments of OGTA237:
- for B-cell non-Hodgkin's lymphoma applications: for example these comprise one or more of the sequences identified as tryptic sequences in the 3^{rd} column of Table 43a;
- for colorectal cancer applications: for example these comprise one or more of the sequences identified as tryptic sequences in the 3^{rd} column of Table 88a;
- for lung cancer applications: for example these comprise one or more of the sequences identified as tryptic sequences in the 3^{rd} column of Table 88c and table 88d;
- for lymphoid leukaemia applications: for example these comprise one or more of the sequences identified as tryptic sequences in the 3^{rd} column of Table 43b;
- for prostate cancer applications: for example these comprise one or more of the sequences identified as tryptic sequences in the 3^{rd} column of Table 43c;
- for renal cell cancer applications: for example these comprise one or more of the sequences identified as tryptic sequences in the 3^{rd} column of Table 88b.

In relation to fragments, immunogenic fragments or antigenic fragments of OGTA247:
- for hepatocellular carcinoma applications: for example these comprise one or more of the sequences identified as tryptic sequences in the 3^{rd} column of Table 44a;
- for lung cancer applications: for example these comprise one or more of the sequences identified as tryptic sequences in the 3^{rd} column of Table 89a and Table 89b;
- for melanoma applications: for example these comprise one or more of the sequences identified as tryptic sequences in the 3^{rd} column of Table 44b.

In relation to fragments, immunogenic fragments or antigenic fragments of OGTA248:
- for lung cancer applications: for example these comprise one or more of the sequences identified as tryptic sequences in the 3^{rd} column of Table 90.
- for renal cell cancer applications: for example these comprise one or more of the sequences identified as tryptic sequences in the 3^{rd} column of Table 45.

In relation to fragments, immunogenic fragments or antigenic fragments of OGTA249:
- for lung cancer applications: for example these comprise one or more of the sequences identified as tryptic sequences in the 3^{rd} column of Table 91b;
- for ovarian cancer applications: for example these comprise one or more of the sequences identified as tryptic sequences in the 3^{rd} column of Table 46;
- for renal cell cancer applications: for example these comprise one or more of the sequences identified as tryptic sequences in the 3^{rd} column of Table 91a.

In relation to fragments, immunogenic fragments or antigenic fragments of OGTA257:
- for colorectal cancer applications: for example these comprise one or more of the sequences identified as tryptic sequences in the 3^{rd} column of Table 92a;
- for hepatocellular carcinoma applications: for example these comprise one or more of the sequences identified as tryptic sequences in the 3^{rd} column of Table 92b;
- for lung cancer applications: for example these comprise one or more of the sequences identified as tryptic sequences in the 3^{rd} column of Table 92c;
- for ovarian cancer applications: for example these comprise one or more of the sequences identified as tryptic sequences in the 3^{rd} column of Table 92d;
- for pancreatic cancer applications: for example these comprise one or more of the sequences identified as tryptic sequences in the 3^{rd} column of Table 47.

In relation to fragments, immunogenic fragments or antigenic fragments of OGTA271:
- for breast cancer applications: for example these comprise one or more of the sequences identified as tryptic sequences in the 3^{rd} column of Table 48a;
- for cervical cancer applications: for example these comprise one or more of the sequences identified as tryptic sequences in the 3^{rd} column of Table 48b;
- for colorectal cancer applications: for example these comprise one or more of the sequences identified as tryptic sequences in the 3^{rd} column of Table 48c and Table 93a;
- for hepatocellular carcinoma applications: for example these comprise one or more of the sequences identified as tryptic sequences in the 3^{rd} column of Table 48d and table 93c;
- for lung cancer applications: for example these comprise one or more of the sequences identified as tryptic sequences in the 3^{rd} column of Table 48e and Table 93d;
- for osteosarcoma applications: for example these comprise one or more of the sequences identified as tryptic sequences in the 3^{rd} column of Table 48f;
- for pancreatic cancer applications: for example these comprise one or more of the sequences identified as tryptic sequences in the 3^{rd} column of Table 48g;
- for renal cell cancer applications: for example these comprise one or more of the sequences identified as tryptic sequences in the 3^{rd} column of Table 48f and table 93b.

The present disclosure additionally provides: (a) preparations comprising an isolated OGTA according to the disclosure; (b) preparations comprising one or more fragments of an OGTA according to the disclosure; and (c) antibodies or other affinity reagents that bind to an OGTA according to the disclosure, to fragments fragments thereof, or both. As used herein, OGTA(s) are "isolated" when they are present in preparations that are substantially free of contaminating proteins, *i.e*., preparations in which less than 10% by weight (for example less than 5%, such as less than 1%) of the total protein present is contaminating protein(s). A contaminating protein is a protein having a significantly different amino acid sequence from that of an isolated OGTA according to the disclosure, as determined by mass spectral analysis. As used herein, a "significantly different" sequence is one that permits the contaminating protein to be resolved from an OGTA of the disclosure by mass spectral analysis, performed according to the Reference Protocols.

An OGTA according to the disclosure can be assayed by any method known to those skilled in the art, including but not limited to, the Preferred Technologies described herein in Examples 1 and 2, kinase assays, enzyme assays, binding assays and other functional assays, immunoassays, and western blotting. In one example, the OGTAs according to the disclosure are separated on 1-D gels by virtue of their MW and visualized by staining the gel. In one example, OGTA(s) are stained with a fluorescent dye and imaged with a fluorescence scanner. Sypro Red (Molecular Probes, Inc., Eugene, Oregon) is a suitable dye for this purpose. A preferred fluorescent dye is disclosed in U.S. Application No. 09/412,168, filed on October 5, 1999. In another example, OGTA(s) are analysed using isobaric tags for relative and absolute quantification (iTRAQ).

Alternatively, OGTAs according to the disclosure can be detected in an immunoassay. In one example, an immunoassay is performed by contacting a sample from a subject to be tested with an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody (or other affinity reagent) under conditions such that immunospecific binding can occur if the relevant OGTA is present, and detecting or measuring the amount of any immunospecific binding by the affinity reagent. Anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 and anti-OGTA271 affinity reagents can be produced by the methods and techniques taught herein.

Proteins according to the disclosure may be detected by virtue of the detection of a fragment thereof e.g. an immunogenic or antigenic fragment thereof. Fragments may have a length of at least 10, more typically at least 20 amino acids e.g. at least 50 or 100 amino acids e.g. at least 200 or 500 amino acids e.g. at least 1000 amino acids e.g. at least 1500 amino acids e.g. at least 2000 amino acids.

In one example one or more for example 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 proteins according to the disclosure are dectected using an appropriate array analysis.

In one example, binding of antibody (or other affinity reagent) in tissue sections can be used to detect aberrant OGTA(s) localization or an aberrant level of OGTA(s). In a specific example, an antibody (or other affinity reagent) to an OGTA according to the disclosure can be used to assay a patient tissue (*e*.*g*., a lymphoid, breast, cervical, colorectal, gastric, brain, liver, lung, skin, neuronal, osteoblast, ovarian, pancreatic, prostate, kidney or eye tissue) for the level of OGTA(s) where an aberrant level of OGTA(s) is indicative of a relevant cancer. As used herein, an "aberrant level" means a level that is increased compared with the level in a subject free from said cancer or a reference level. This example may be suitable for detecting elevated levels of OGTA066 in cancer tissue, such as colorectal cancer, kidney cancer, lung cancer and/or pancreatic cancer tissue.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, a protein according to the disclosure can be detected in a fluid sample (e.g., blood, urine, or saliva) by means of a two-step sandwich assay. In the first step, a capture reagent (e.g., an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody or other affinity reagent) is used to capture the relevant OGTA(s). The capture reagent can optionally be immobilized on a solid phase. In the second step, a directly or indirectly labeled detection reagent is used to detect the captured OGTA(s). In one example, the detection reagent is a lectin. Any lectin can be used for this purpose that preferentially binds to an OGTA according to the disclosure rather than to other isoforms that have the same core protein as an OGTA according to the disclosure or to other proteins that share the antigenic determinant recognized by the antibody. In one example, the chosen lectin binds an OGTA according to the disclosure with at least 2-fold greater affinity, more for example at least 5-fold greater affinity, still more preferably at least 10-fold greater affinity, than to said other isoforms that have the same core protein as an OGTA according to the disclosure or to said other proteins that share the antigenic determinant recognized by the affinity reagent.

Whilst not wishing to be bound by theory it is thought that in patients with a relevant cancer such as colorectal cancer, kidney cancer, lung cancer and/or pancreatic cancer that the levels of OGTA066 are reduced in fluid samples such as serum samples in comparison to a subject free of said cancer. This is thought to be because the protein seems to be recruited to the cancerous tissue and thus the levels found in the cancerous tissue are higher in patients with the relevant cancer than subjects free from same.

Based on the present description, a lectin that is suitable for detecting an OGTA according to the disclosure can readily be identified by methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174. In an alternative example, the detection reagent is an antibody (or other affinity reagent), e.g. an antibody that immunospecifically detects other post-translational modifications, such as an antibody that immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding a protein according to the disclosure a related gene, or related nucleic acid sequences or subsequences, including complementary sequences, can also be used in hybridization assays. A nucleotide encoding a protein according to the disclosure, or subsequences thereof comprising at least 8 nucleotides, for example at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of the gene encoding a protein according to the disclosure, or for differential diagnosis of subjects with signs or symptoms suggestive of a relevant cancer. In particular, such a hybridization assay can be carried out by a method comprising contacting a subject's sample containing nucleic acid with a nucleic acid probe capable of hybridizing to a DNA or RNA that encodes a protein according to the disclosure under conditions such that hybridization can occur, and detecting or measuring any resulting hybridization.

Hence nucleic acid encoding OGTA(s) (e.g. DNA or more suitably RNA) may be detected, for example, using a hybridizing agent capable of hybridizing to nucleic acid encoding OGTA.

One such exemplary method comprises:
(a) contacting one or more oligonucleotide probes comprising 10 or more consecutive nucleotides complementary to a nucleotide sequence encoding OGTA, with an RNA obtained from a biological sample from the subject or with cDNA copied from the RNA, wherein said contacting occurs under conditions that permit hybridization of the probe to the nucleotide sequence if present;
(b) detecting hybridization, if any, between the probe and the nucleotide sequence; and
(c) comparing the hybridization, if any, detected in step (b) with the hybridization detected in a control sample, or with a previously determined reference range.

Thus in one example the analysis for differential expression of the gene or proteins in performed using microarrays comprising probes sets, allowing simulateous analysis in relation to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 proteins according to the disclosure. This technology is well known in the relevant field.

The disclosure also provides diagnostic kits, comprising an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody (or other affinity reagent).

In addition, such a kit may optionally comprise one or more of the following: (1) instructions for using the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent for diagnosis, prognosis, therapeutic monitoring or any combination of these applications; (2) a labeled binding partner to the affinity reagent; (3) a solid phase (such as a reagent strip) upon which the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent is immobilized; and (4) a label or insert indicating regulatory approval for diagnostic, prognostic or therapeutic use or any combination thereof.

If no labeled binding partner to the affinity reagent is provided, the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent itself can be labeled with a detectable marker, *e.g.* a chemiluminescent, enzymatic, fluorescent, or radioactive moiety.

The disclosure also provides a kit comprising a nucleic acid probe capable of hybridizing to RNA encoding a protein according to the disclosure. In a specific example, a kit comprises in one or more containers a pair of primers (*e*.*g*. each in the size range of 6-30 nucleotides, more preferably 10-30 nucleotides and still more preferably 10-20 nucleotides) that under appropriate reaction conditions can prime amplification of at least a portion of a nucleic acid encoding a protein according to the disclosure, such as by polymerase chain reaction (see, *e.g.,* Innis et al., 1990, PCR Protocols, Academic Press, Inc., San Diego, CA), ligase chain reaction (see EP 320,308) use of Qβ replicase, cyclic probe reaction, or other methods known in the art.

A kit can optionally further comprise a predetermined amount of an OGTA according to the disclosure or a nucleic acid encoding same, *e.g.* for use as a standard or control.

### Use in Clinical Studies

The diagnostic methods and compositions of the present disclosure can assist in monitoring a clinical study, *e.g.* to evaluate drugs for therapy of a relevant cancer. In one example, candidate molecules are tested for their ability to restore levels of a protein(s) according to the disclosure in a subject having a relevant cancer to levels found in subjects free from said cancer or, in a treated subject, to preserve said levels at or near non-B-cell non-Hodgkin's lymphoma, non-breast cancer, non-cervical cancer, non-colorectal cancer, non-gastric cancer, non-glioblastoma, non-hepatocellular carcinoma, non-lung cancer, non-lymphoid leukaemia (particularly acute T-cell leukaemia and chronic lymphocytic leukaemia), non-melanoma, non-neuroblastoma, non-osteosarcoma, non-ovarian cancer, non-pancreatic cancer, non-prostate cancer, non-renal cell cancer or non-retinoblastoma values.

In another example, the methods and compositions of the present disclosure are used to screen candidates for a clinical study to identify individuals having a relevant cancer; such individuals can then be excluded from the study or can be placed in a separate cohort for treatment or analysis.

### Production of Proteins of the Invention and Corresponding Nucleic Acid

A DNA of the present disclosure can be obtained by isolation as a cDNA fragment from cDNA libraries using as starter materials commercial mRNAs and determining and identifying the nucleotide sequences thereof. That is, specifically, clones are randomly isolated from cDNA libraries, which are prepared according to Ohara et al's method (DNA Research Vol.4, 53-59 (1997)). Next, through hybridization, duplicated clones (which appear repeatedly) are removed and then in vitro transcription and translation are carried out. Nucleotide sequences of both termini of clones, for which products of 50 kDa or more are confirmed, are determined. Furthermore, databases of known genes are searched for homology using the thus obtained terminal nucleotide sequences as queries. The entire nucleotide sequence of a clone revealed to be novel as a result is determined. In addition to the above screening method, the 5' and 3' terminal sequences of cDNA are related to a human genome sequence. Then an unknown long-chain gene is confirmed in a region between the sequences, and the full-length of the cDNA is analyzed. In this way, an unknown gene that is unable to be obtained by a conventional cloning method that depends on known genes can be systematically cloned.

Moreover, all of the regions of a human-derived gene containing a DNA of the present disclosure can also be prepared using a PCR method such as RACE while paying sufficient attention to prevent artificial errors from taking place in short fragments or obtained sequences. As described above, clones having DNA of the present disclosure can be obtained.
In another means for cloning DNA of the present disclosure, a synthetic DNA primer having an appropriate nucleotide sequence of a portion of a polypeptide of the present disclosure is produced, followed by amplification by the PCR method using an appropriate library.

Alternatively, selection can be carried out by hybridization of the DNA of the present disclosure with a DNA that has been incorporated into an appropriate vector and labeled with a DNA fragment or a synthetic DNA encoding some or all of the regions of the polypeptide of the present disclosure. Hybridization can be carried out by, for example, the method described in Current Protocols in Molecular Biology (edited by Frederick M. Ausubel et al., 1987). DNA of the present disclosure may be any DNA, as long as they contain nucleotide sequences encoding the polypeptides of the present disclosure as described above. Such a DNA may be a cDNA identified and isolated from cDNA libraries or the like that are derived from lymphoid, breast, cervical, colorectal, gastric, brain, liver, lung, skin, neuronal, osteoblast, ovarian, pancreatic, prostate, kidney or eye tissue. Such a DNA may also be a synthetic DNA or the like. Vectors for use in library construction may be any of bacteriophages, plasmids, cosmids, phargemids, or the like. Furthermore, by the use of a total RNA fraction or a mRNA fraction prepared from the above cells and/or tissues, amplification can be carried out by a direct reverse transcription coupled polymerase chain reaction (hereinafter abbreviated as "RT-PCR method").

DNA encoding the above polypeptides consisting of amino acid sequences that are substantially identical to the amino acid sequences of OGTA(s) according to the disclosure or DNA encoding the above polypeptides consisting of amino acid sequences derived from the amino acid sequences of OGTA(s) by deletion, substitution, or addition of one or more amino acids composing a portion of the amino acid sequence can be easily produced by an appropriate combination of, for example, a site-directed mutagenesis method, a gene homologous recombination method, a primer elongation method, and the PCR method known by persons skilled in the art. In addition, at this time, a possible method for causing a polypeptide to have substantially equivalent biological activity is substitution of homologous amino acids (e.g. polar and nonpolar amino acids, hydrophobic and hydrophilic amino acids, positively-charged and negatively charged amino acids, and aromatic amino acids) among amino acids composing the polypeptide. Furthermore, to maintain substantially equivalent biological activity, amino acids within functional domains contained in each polypeptide of the present disclosure are for example conserved.

Furthermore, examples of DNA of the present disclosure include DNA comprising nucleotide sequences that encode the amino acid sequences of an OGTA according to the disclosure and DNA hybridizing under stringent conditions to the DNA and encoding polypeptides (proteins) having biological activity (functions) equivalent to the functions of the polypeptides consisting of the amino acid sequence of an OGTA according to the disclosure. Under such conditions, an example of such DNA capable of hybridizing to DNA comprising the nucleotide sequences that encode the amino acid sequence of a protein according to the disclosure is DNA comprising nucleotide sequences that have a degree of overall mean homology with the entire nucleotide sequence of the DNA, such as approximately 80% or more, for example approximately 90% or more, and more preferably approximately 95% or more. Hybridization can be carried out according to a method known in the art such as a method described in Current Protocols in Molecular Biology (edited by Frederick M. Ausubel et al., 1987) or a method according thereto. Here, "stringent conditions" are, for example, conditions of approximately "1*SSC, 0.1% SDS, and 37°C, more stringent conditions of approximately "0.5*SSC, 0.1% SDS, and 42°C, or even more stringent conditions of approximately "0.2*SSC, 0.1% SDS, and 65°C. With more stringent hybridization conditions, the isolation of a DNA having high homology with a probe sequence can be expected. The above combinations of SSC, SDS, and temperature conditions are given for illustrative purposes. Stringency similar to the above can be achieved by persons skilled in the art using an appropriate combination of the above factors or other factors (for example, probe concentration, probe length, and reaction time for hybridization) for determination of hybridization stringency.

A cloned DNA of the present disclosure can be directly used or used, if desired, after digestion with a restriction enzyme or addition of a linker, depending on purposes. The DNA may have ATG as a translation initiation codon at the 5' terminal side and have TAA, TGA, or TAG as a translation termination codon at the 3' terminal side. These translation initiation and translation termination codons can also be added using an appropriate synthetic DNA adapter.

In methods of the disclosure the OGTA employed is, for example provided in isolated form, such as a form where the polypeptide has been purified to at least to some extent. The polypeptide may be provided in substantially pure form, that is to say free, to a substantial extent, from other proteins. The polypeptide can also be produced using recombinant methods, synthetically produced or produced by a combination of these methods. OGTA(s) according to the disclosure can be easily prepared by any method known by persons skilled in the art, which involves producing an expression vector containing a DNA of the present disclosure or a gene containing a DNA of the present disclosure, culturing a transformant transformed using the expression vector, generating and accumulating a polypeptide of the present disclosure or a recombinant protein containing the polypeptide, and then collecting the resultant.

Recombinant OGTA polypeptide may be prepared by processes well known in the art from genetically engineered host cells comprising expression systems. Accordingly, the present disclosure also relates to expression systems which comprise an OGTA polypeptide or nucleic acid, to host cells which are genetically engineered with such expression systems and to the production of OGTA polypeptide by recombinant techniques. For recombinant polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis et al., Basic Methods in Molecular Biology, 1986 and Sambrook et al. , Molecular Cloning: A Laboratory Manual, 2nd Ed., Cold Spring Harbour laboratory Press, Cold Spring Harbour, NY, 1989).

As host cells, for example, bacteria of the genus Escherichia, Streptococci, Staphylococci, Streptomyces, bacteria of the genus Bacillus, yeast, Aspergillus cells, insect cells, insects, and animal cells are used. Specific examples of bacteria of the genus Escherichia, which are used herein, include Escherichia coli K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, Saccaromyces cerevisiae AH22, AH22R-, NA87-11A, DKD-5D, and 20B-12, Schizosaccaromyces pombe NCYC1913 and NCYC2036, and Pichia pastoris are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 E. Coli HY transcription and translation kits from Roche Diagnostics Ltd., Lewes, UK and the TNT Quick coupled Transcription/Translation System from Promega UK, Southampton, UK).
The expression vector can be produced according to a method known in the art. For example, the vector can be produced by (1) excising a DNA fragment containing a DNA of the present disclosure or a gene containing a DNA of the present disclosure and (2) ligating the DNA fragment downstream of the promoter in an appropriate expression vector. A wide variety of expression systems can be used, such as and without limitation, chromosomal, episomal and virus-derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present disclosure may be any promoters as long as they are appropriate for hosts to be used for gene expression. For example, when a host is Escherichia coli, a trp promoter, a lac promoter, a recA promoter, a pL promoter, an 1pp promoter, and the like are preferred. When a host is Bacillus subtilis, an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook et al., supra. Appropriate secretion signals may be incorporated into the OGTA polypeptide to allow secretion of the translated protein into the lumen of the endoplasmic reticulum, the periplasmic space or the extracellular environment. These signals may be endogenous to the OGTA polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA of the present disclosure or a gene containing a DNA of the present disclosure can be cultured according to a method known in the art. For example, when hosts are bacteria of the genus Escherichia, the bacteria are generally cultured at approximately 15°C to 43°C for approximately 3 to 24 hours. If necessary, aeration or agitation can also be added. When hosts are bacteria of the genus Bacillus, the bacteria are generally cultured at approximately 30°C to 40°C for approximately 6 to 24 hours. If necessary, aeration or agitation can also be added. When transformants whose hosts are yeast are cultured, culture is generally carried out at approximately 20°C to 35°C for approximately 24 to 72 hours using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 hours using media with the pH adjusted to be approximately 6 to 8. If necessary, aeration or agitation can also be added.
If an OGTA(s) polypeptide is to be expressed for use in cell-based screening assays, it is preferred that the polypeptide be produced at the cell surface. In this event, the cells may be harvested prior to use in the screening assay. If the OGTA polypeptide is secreted into the medium, the medium can be recovered in order to isolate said polypeptide. If produced intracellularly, the cells must first be lysed before the OGTA polypeptide is recovered. OGTA polypeptide can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one example, a combination of these methods is used. In another example, high performance liquid chromatography is used. In a further example, an antibody which specifically binds to an OGTA polypeptide according to the disclosure can be used to deplete a sample comprising an a relevant OGTA polypeptide of said polypeptide or to purify said polypeptide.

To separate and purify a polypeptide or a protein of the present disclosure from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptide (protein) of the present disclosure can be converted into a salt by a known method or a method according thereto. Conversely, when the polypeptide (protein) of the present disclosure is obtained in the form of a salt, it can be converted into a free protein or peptide or another salt by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of a polypeptide (protein) of the present disclosure or a salt thereof can be measured by various binding assays, enzyme immunoassays using specific antibodies, and the like.
Techniques well known in the art, may be used for refolding to regenerate native or active conformations of the OGTA polypeptide when the polypeptide has been denatured during isolation and or purification. In the context of the present disclosure, OGTA polypeptide can be obtained from a biological sample from any source, such as and without limitation, a blood sample or tissue sample, e.g. lymphoid, breast, cervical, colorectal, gastric, brain, liver, lung, skin, neuronal, osteoblast, ovarian, pancreatic, prostate, kidney or eye tissue sample.
OGTA polypeptide may be in the form of a "mature protein" or may be part of a larger protein such as a fusion protein. It is often advantageous to include an additional amino acid sequence which contains secretory or leader sequences, a pre-, pro- or prepro- protein sequence, or a sequence which aids in purification such as an affinity tag, for example, but without limitation, multiple histidine residues, a FLAG tag, HA tag or myc tag.
An additional sequence that may provide stability during recombinant production may also be used. Such sequences may be optionally removed as required by incorporating a cleavable sequence as an additional sequence or part thereof. Thus, an OGTA polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

In one example one or more proteins according to the disclosure or fragments thereof, for example one or more sequences described herein may be fused with a heterologous fusion partner such as the surface protein, known as protein D from Haemophilus Influenza B, a non-structural protein from influenzae virus such as NS1, the S antigen from Hepatitis B or a protein known as LYTA such as the C terminal thereof.

### Production of Affinity Reagents to the Proteins of the Invention

According to those in the art, there are three main types of immunoaffinity reagent - monoclonal antibodies, phage display antibodies and smaller antibody-derived molecules such as Affibodies, Domain Antibodies (dAbs), Nanobodies or UniBodies. In general in applications according to the present invention where the use of antibodies is stated, other affinity reagents (e.g. Affibodies, domain antibodies, Nanobodies or UniBodies) may be employed. Such substances may be said to be capable of immunospecific binding to the proteins of the invention. Where appropriate the term "affinity agent" shall be construed to embrace immunoaffinity reagents and other substances capable of specific binding to the proteins of the invention including but not limited to ligands, lectins, streptavidins, antibody mimetics and synthetic binding agents.

### Production of Antibodies to the Proteins of the Invention

According to the disclosure OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, an analog of any of same, a related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody." Antibodies of the invention include, monoclonal antibodies, these may be bispecific, humanized or chimeric antibodies, single chain antibodies, Fab fragments and F(ab')₂ fragments, fragments produced by a Fab expression library, anti-idiotypic (anti-Id) antibodies, and epitope-binding fragments of any of the above. The immunoglobulin molecules of the invention can be of any class (*e*.*g*., IgG, IgE, IgM, IgD and IgA) or subclass of immunoglobulin molecule.

The term "specifically binds" (or "immunospecifically binds") is not intended to indicate that an antibody binds exclusively to its intended target. Rather, an antibody "specifically binds" if its affinity for its intended target is about 5-fold greater when compared to its affinity for a non-target molecule. For example the affinity of the antibody will be at least about 5 fold, for example 10 fold, such as 25-fold, particularly 50-fold, and especially 100-fold or more, greater for a target molecule than its affinity for a non-target molecule. In some examples, specific binding between an antibody or other binding agent and an antigen means a binding affinity of at least 10⁶ M⁻¹. Antibodies may bind with affinities of at least about 10⁷ M⁻¹, such as between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labeled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and
n = number of ligand binding sites per receptor molecule
By graphical analysis, r/c is plotted on the Y-axis versus r on the X-axis thus producing a Scatchard plot. The affinity is the negative slope of the line. k_{off} can be determined by competing bound labeled ligand with unlabeled excess ligand (see, e.g., U.S. Pat No. 6,316,409). The affinity of a targeting agent for its target molecule is for example at least about 1 x 10⁻⁶ moles/liter, is more preferably at least about 1 x 10⁻⁷ moles/liter, is even more preferably at least about 1 x 10⁻⁸ moles/liter, is yet even more preferably at least about 1 x 10⁻⁹ moles/liter, and is most preferably at least about 1 x 10⁻¹⁰ moles/liter. Antibody affinity measurement by Scatchard analysis is well known in the art. *See*, *e.g*., van Erp et al., J. Immunoassay 12: 425-43, 1991; Nelson and Griswold, Comput. Methods Programs Biomed. 27: 65-8, 1988.

In one example, antibodies that recognize gene products of genes encoding a protein of the disclosure are publicly available. In another example, methods known to those skilled in the art are used to produce antibodies that recognize OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, an analog thereof, a related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one example of the disclosure, antibodies to a specific domain of an OGTA according to the disclosure are produced. In a specific example, hydrophilic fragments of protein according to the disclosure are used as immunogens for antibody production.

In the production of antibodies, screening for the desired antibody can be accomplished by techniques known in the art, *e.g.* ELISA (enzyme-linked immunosorbent assay). For example, to select antibodies which recognize a specific domain of a protein according to the disclosure, one may assay generated hybridomas for a product which binds to an OGTA fragment containing such domain. For selection of an antibody that specifically binds a first OGTA homolog but which does not specifically bind to (or binds less avidly to) a second OGTA homolog, one can select on the basis of positive binding to the first OGTA homolog and a lack of binding to (or reduced binding to) the second OGTA homolog. Similarly, for selection of an antibody that specifically binds an OGTA according to the disclosure but which does not specifically bind to (or binds less avidly to) a different isoform of the same protein (such as a different glycoform having the same core peptide as an OGTA according to the disclosure, one can select on the basis of positive binding to an OGTA according to the disclosure and a lack of binding to (or reduced binding to) the different isoform (*e*.*g*., a different glycoform). Thus, the present disclosure provides an antibody (for example a monoclonal antibody) that binds with greater affinity (for example at least 2-fold, such as at least 5-fold, particularly at least 10-fold greater affinity) to OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271than to a different isoform or isoforms (*e*.*g*., glycoforms) of same.

Polyclonal antibodies which may be used in the methods of the disclosure are heterogeneous populations of antibody molecules derived from the sera of immunized animals. Unfractionated immune serum can also be used. Various procedures known in the art may be used for the production of polyclonal antibodies to OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, a fragment thereof, a related polypeptide, or a fragment of an OGTA related polypeptide. For example, one way is to purify polypeptides of interest or to synthesize the polypeptides of interest using, e.g., solid phase peptide synthesis methods well known in the art. See, e.g., Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. The Preferred Technology described herein in Example 1 provides isolated OGTA suitable for such immunization. If an OGTA is purified by gel electrophoresis, it can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward proteins of the disclosure, a fragment thereof, a related polypeptide, or a fragment of a related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the disclosure may be cultivated *in vitro* or *in vivo.* In an additional example of the disclosure, monoclonal antibodies can be produced in germ-free animals utilizing known technology (PCT/US90/02545,).

The monoclonal antibodies include but are not limited to human monoclonal antibodies and chimeric monoclonal antibodies (*e*.*g*., human-mouse chimeras). A chimeric antibody is a molecule in which different portions are derived from different animal species, such as those having a human immunoglobulin constant region and a variable region derived from a murine mAb. (See, *e*.*g*., Cabilly et al., U.S. Patent No. 4,816,567; and Boss et al., U.S. Patent No. 4,816397.) Humanized antibodies are antibody molecules from non-human species having one or more complementarity determining regions (CDRs) from the non-human species and a framework region from a human immunoglobulin molecule. (See, *e*.*g*., Queen, U.S. Patent No. 5,585,089.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are for example desirable for therapeutic treatment of human subjects.

Antibodies can be produced using transgenic mice which are incapable of expressing endogenous immunoglobulin heavy and light chain genes, but which can express human heavy and light chain genes. The transgenic mice are immunized in the normal fashion with a selected antigen, e.g., all or a portion of a protein according to the disclosure. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce useful (including therapeutically useful) IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see*, *e*.*g*., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g*., a mouse antibody, is used to guide the selection of a completely human antibody recognizing the same epitope. (Jespers et al. (1994) Bio/technology 12:899-903).
The antibodies of the present disclosure can also be generated by the use of phage display technology to produce and screen libraries of polypeptides for binding to a selected target. See, e.g., Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098. In particular, such phage can be utilized to display antigen binding domains expressed from a repertoire or combinatorial antibody library (*e*.*g*., human or murine). Phage expressing an antigen binding domain that binds the antigen of interest can be selected or identified with antigen, *e*.*g*., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present disclosure include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The disclosure further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in examples when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred example of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

The disclosure provides functionally active fragments, derivatives or analogs of anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 immunoglobulin molecules.

Functionally active means that the fragment, derivative or analog is able to elicit anti-anti-idiotype antibodies (*i*.*e*., tertiary antibodies) that recognize the same antigen that is recognized by the antibody from which the fragment, derivative or analog is derived. Specifically, in a preferred example the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present disclosure provides antibody fragments such as, but not limited to, F(ab')₂ fragments and Fab fragments. Antibody fragments which recognize specific epitopes may be generated by known techniques. F(ab')₂ fragments consist of the variable region, the light chain constant region and the CH1 domain of the heavy chain and are generated by pepsin digestion of the antibody molecule. Fab fragments are generated by reducing the disulfide bridges of the F(ab')₂ fragments. The disclosure also provides heavy chain and light chain dimers of the antibodies of the disclosure, or any minimal fragment thereof such as Fvs or single chain antibodies (SCAs) *(e.g.,* as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the disclosure. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

In other examples, the disclosure provides fusion proteins of the immunoglobulins of the disclosure (or functionally active fragments thereof), for example in which the immunoglobulin is fused via a covalent bond (*e*.*g*., a peptide bond), at either the N-terminus or the C-terminus to an amino acid sequence of another protein (or portion thereof, for example at least 10, 20 or 50 amino acid portion of the protein) that is not the immunoglobulin. For example the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins of the disclosure include analogs and derivatives that are modified, *i.e*., by the covalent attachment of any type of molecule as long as such covalent attachment does not impair immunospecific binding. For example, but not by way of limitation, the derivatives and analogs of the immunoglobulins include those that have been further modified, *e.g.,* by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of an OGTA*, e*.*g*., for imaging this protein, measuring levels thereof in appropriate physiological samples, in diagnostic methods, etc.

### Production of Affibodies to the Proteins of the Invention

Affibody molecules represent a new class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (Nord K, Gunneriusson E, Ringdahl J, Stahl S, Uhlen M, Nygren PA, Binding proteins selected from combinatorial libraries of an α-helical bacterial receptor domain, Nat Biotechnol 1997;15:772-7. Ronmark J, Gronlund H, Uhlen M, Nygren PA, Human immunoglobulin A (IgA)-specific ligands from combinatorial engineering of protein A, Eur J Biochem 2002;269:2647-55.). The simple, robust structure of Affibody molecules in combination with their low molecular weight (6 kDa), make them suitable for a wide variety of applications, for instance, as detection reagents (Ronmark J, Hansson M, Nguyen T, et al, Construction and characterization of affibody-Fc chimeras produced in Escherichia coli, J Immunol Methods 2002;261:199-211) and to inhibit receptor interactions (Sandstorm K, Xu Z, Forsberg G, Nygren PA, Inhibition of the CD28-CD80 co-stimulation signal by a CD28-binding Affibody ligand developed by combinatorial protein engineering, Protein Eng 2003;16:691-7). Further details of Affibodies and methods of production thereof may be obtained by reference to US Patent No 5831012.
Labelled Affibodies or the like may also be useful in imaging applications for determining abundance of Isoforms.

### Production of Domain Antibodies to the Proteins of the Invention

References to antibodies herein embrace references to Domain Antibodies. Domain Antibodies (dAbs) are the smallest functional binding units of antibodies, corresponding to the variable regions of either the heavy (V_{H}) or light (V_{L}) chains of human antibodies. Domain Antibodies have a molecular weight of approximately 13 kDa. Domantis has developed a series of large and highly functional libraries of fully human V_{H} and V_{L} dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 & 0616640; WO05/035572, WO04/101790, WO04/081026, WO04/058821, WO04/003019 and WO03/002609.

### Production of Nanobodies to the Proteins of the Invention

Nanobodies are antibody-derived therapeutic proteins that contain the unique structural and functional properties of naturally-occurring heavy-chain antibodies. These heavy-chain antibodies contain a single variable domain (VHH) and two constant domains (C_{H}2 and C_{H}3). Importantly, the cloned and isolated VHH domain is a perfectly stable polypeptide harbouring the full antigen-binding capacity of the original heavy-chain antibody. Nanobodies have a high homology with the VH domains of human antibodies and can be further humanised without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see e.g. WO 04/041867) and are easy to manufacture. Other advantages of Nanobodies include recognising uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts e.g. *E. coli* (see e.g. US 6,765,087), moulds (for example *Aspergillus* or *Trichoderma*) and yeast (for example *Saccharomyces, Kluyveromyces, Hansenula* or *Pichia*) (see e.g. US 6,838,254). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see e.g. WO 06/079372) is a proprietary method for generating Nanobodies against a desired target, based on automated high-throughout selection of B-cells.

### Production of UniBodies to the Proteins of the Disclosure

UniBody is a new proprietary antibody technology that creates a stable, smaller antibody format with an anticipated longer therapeutic window than current small antibody formats. IgG4 antibodies are considered inert and thus do not interact with the immune system. Genmab modified fully human IgG4 antibodies by eliminating the hinge region of the antibody. Unlike the full size IgG4 antibody, the half molecule fragment is very stable and is termed a UniBody. Halving the IgG4 molecule left only one area on the UniBody that can bind to disease targets and the UniBody therefore binds univalently to only one site on target cells. This univalent binding does not stimulate cancer cells to grow like bivalent antibodies might and opens the door for treatment of some types of cancer which ordinary antibodies cannot treat.

The UniBody is about half the size of a regular IgG4 antibody. This small size can be a great benefit when treating some forms of cancer, allowing for better distribution of the molecule over larger solid tumors and potentially increasing efficacy.

Fabs typically do not have a very long half-life. UniBodies, however, were cleared at a similar rate to whole IgG4 antibodies and were able to bind as well as whole antibodies and antibody fragments in pre-clinical studies. Other antibodies primarily work by killing the targeted cells whereas UniBodies only inhibit or silence the cells.

Further details of UniBodies may be obtained by reference to patent WO2007/059782,.

### Expression of Affinity Reagents

### Expression of Antibodies

The antibodies of the disclosure can be produced by any method known in the art for the synthesis of antibodies, in particular, by chemical synthesis or by recombinant expression, and are for example produced by recombinant expression techniques.
Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (*e.g*., as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (*e.g.,* an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (*e.g.*, as described in Huse et al., 1989, Science 246:1275-1281) for clones of Fab fragments that bind the specific antigen or by screening antibody libraries (See, *e.g*., Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, *e.g.,* PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra*, a chimeric antibody is a molecule in which different portions are derived from different animal species, such as those having a variable region derived from a murine mAb and a human antibody constant region, *e.g*., humanized antibodies.

Once a nucleic acid encoding an antibody molecule of the disclosure has been obtained, the vector for the production of the antibody molecule may be produced by recombinant DNA technology using techniques well known in the art. Thus, methods for preparing the proteins of the disclosure by expressing nucleic acid containing the antibody molecule sequences are described herein. Methods which are well known to those skilled in the art can be used to construct expression vectors containing antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the disclosure.

The host cells used to express a recombinant antibody of the disclosure may be either bacterial cells such as *Escherichia coli,* or, for example, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, Bio/Technology 8:2). A variety of host-expression vector systems may be utilized to express an antibody molecule of the disclosure. Such host-expression systems represent vehicles by which the coding sequences of interest may be produced and subsequently purified, but also represent cells which may, when transformed or transfected with the appropriate nucleotide coding sequences, express the antibody molecule of the disclosure *in situ.* These include but are not limited to microorganisms such as bacteria (*e.g., E. coli, B. subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g*., baculovirus) containing the antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BHK, 293, 3T3 cells) harboring recombinant expression constructs containing promoters derived from the genome of mammalian cells (*e.g.,* metallothionein promoter) or from mammalian viruses (*e.g.,* the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for example for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther et al., 1983, EMBO J. 2:1791), in which the antibody coding sequence may be ligated individually into the vector in frame with the *lac* Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (*e.g*., an adenovirus expression system) may be utilized.

As discussed above, a host cell strain may be chosen which modulates the expression of the inserted sequences, or modifies and processes the gene product in the specific fashion desired. Such modifications (*e.g.,* glycosylation) and processing (*e.g.,* cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (*e.g*., neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the disclosure, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once an antibody molecule of the disclosure has been recombinantly expressed, it may be purified by any method known in the art for purification of an antibody molecule, for example, by chromatography (*e.g*., ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.
The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, etc., assay performance of an antibody may be a more important measure than absolute affinity and specificity of an antibody.
Those skilled in the art will recognise that many approaches can be taken in producing antibodies or binding fragments and screening and selecting for affinity and specificity for the various polypeptides, but these approaches do not change the scope of the disclosure.

For some applications (including therpeutica applications), antibodies (particularly monoclonal antibodies) may suitably be human or humanized animal (e.g. mouse) antibodies. Animal antibodies may be raised in animals using the human protein (e.g. OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271) as immunogen. Humanisation typically involves grafting CDRs identified thereby into human framework regions. Normally some subsequent retromutation to optimize the conformation of chains is required. Such processes are known to persons skilled in the art.

### Expression of Affibodies

The construction of affibodies has been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.), including the construction of affibody phage display libraries (Nord, K., Nilsson, J., Nilsson, B., Uhle' n, M. & Nygren, P.A, A combinatorial library of an a-helical bacterial receptor domain, 1995, Protein Eng. 8, 601-608. Nord, K., Gunneriusson, E., Ringdahl, J., Sta° hl, S., Uhle' n, M. & Nygren, P.A°, Binding proteins selected from combinatorial libraries of an a-helical bacterial receptor domain, 1997, Nat. Biotechnol.15, 772-777.)

The biosensor analyses to investigate the optimal affibody variants using biosensor binding studies has also been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.).

### Affinity Reagent Modifications

In a preferred example, anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 and/or anti-OGTA271 affinity reagents such as antibodies or fragments thereof are conjugated to a diagnostic or therapeutic moiety.

Anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 antibodies or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumour necrosis factor, □-interferon, □-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor

The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present disclosure. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc. ⁶⁸Ga may also be employed.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

The disclosure also provides for fully human, or humanised antibodies that induce antibody-directed cell-mediated cytotoxicity (ADCC). A fully human antibody is one in which the protein sequences are encoded by naturally occurring human immunoglobulin sequences, either from isolated antibody-producing human B-lymphocytes, or from transgenic murine B-lymphocytes of mice in which the murine immunoglobulin coding chromosomal regions have been replaced by orthologous human sequences. Transgenic antibodies of the latter type include, but are not restricted to, HuMab (Medarex, Inc, CA) and Xenomouse (Abgenix Inc., CA). A humanised antibody is one in which the constant region of a non-human antibody molecule of appropriate antigen specificity, is replaced by the constant region of a human antibody, preferably of the IgG subtype, with appropriate effector functions (Morrison et al., 1984, Proc. Natl. Acad. Sci. 81:851-855; Neuberger et al., 1984, Nature 312:604-608; Takeda et al., 1985, Nature 314:452-454). Appropriate effector functions include ADCC, which is a natural process by which fully-human antibodies or humanized antibodies, when bound to targets on the surface of cancer cells, switch on the cell killing properties of lymphocytes that are part of the normal immune system. These active lymphocytes, called Natural Killer (NK) cells, use a cytotoxic process to destroy living cells to which the antibodies are bound. ADCC activity may be detected and quantified by measuring release of Europium (Eu3+) from Eu3+ labelled, living cells in the presence of an antigen-specific antibody and peripheral blood mononuclear cells extracted from an immunocompetent, living human subject. The ADCC process is described in detail in Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947. Suitable methods for the detection and quantification of ADCC can be found in Blomberg et al., Journal of Immunological Methods. 1986, 86:p225-9; Blomberg et al., Journal of Immunological Methods. 1986, 21;92:p117-23 and Patel & Boyd, Journal of Immunological Methods. 1995, 184:p29-38.

ADCC typically involves activation of NK cells and is dependent on the recognition of antibody-coated cells by Fc receptors on the surface of the NK cell. The Fc receptors recognize the Fc (crystalline) portion of antibodies such as IgG, bound specifically to the surface of a target cell. The Fc receptor that triggers activation of the NK cell is called CD16 or FcyRIIIa. Once the FcγRIIIa receptor is bound to the IgG Fc, the NK cell releases cytokines such as IFN-γ, and cytotoxic granules containing perforin and granzymes that enter the target cell and promote cell death by triggering apoptosis.

The induction of antibody-dependent cellular cytotoxicity (ADCC) by an antibody can be enhanced by modifications that alter interactions between the antibody constant region (Fc) and various receptors that are present on the surface of cells of the immune system. Such modifications include the reduction or absence of alpha1,6-linked fucose moieties in the complex oligosaccharide chains that are normally added to the Fc of antibodies during natural or recombinant synthesis in mammalian cells. In a preferred example, non-fucosylated anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 affinity reagents such as antibodies or fragments thereof are produced for the purpose of enhancing their ability to induce the ADCC response.

Techniques for reducing or ablating alpha1,6-linked fucose moieties in the oligosaccharide chains of the Fc are well established. In one example, the recombinant antibody is synthesized in a cell line that is impaired in its ability to add fucose in an alpha 1,6 linkage to the innermost N-acetylglucosamine of the N-linked biantennary complex-type Fc oligosaccharides. Such cell lines include, but are not limited to, the rat hybridoma YB2/0, which expresses a reduced level of the alpha 1,6-fucosyltransferase gene, FUT8. Preferably, the antibody is synthesized in a cell line that is incapable of adding alpha 1,6-linked fucosyl moieties to complex oligosaccharide chains, due to the deletion of both copies of the FUT8 gene. Such cell lines include, but are not limited to, FUT8-/- CHO/DG44 cell lines. Techniques for synthesizing partially fucosylated, or non-fucosylated antibodies and affinity reagents are described in Shinkawa et al., J. Biol. Chem. 278:3466-34735 (2003); Yamane-Ohnuki et al., Biotechnology and Bioengineering 87: 614-22 (2004) and in WO00/61739 A1, WO02/31140 A1 and WO03/085107 A1. In a second example, the fucosylation of a recombinant antibody is reduced or abolished by synthesis in a cell line that has been genetically engineered to overexpress a glycoprotein-modifying glycosyl transferase at a level that maximizes the production of complex N-linked oligosaccharides carrying bisecting N-acetylglucosamine. For example, the antibody is synthesized in a Chinese Hamster Ovary cell line expressing the enzyme N-acetyl glucosamine transferase III (GnT III). Cell lines stably transfected with suitable glycoprotein-modifying glycosyl transferases, and methods of synthesizing antibodies using these cells are described in WO9954342.

A non-fucosylated antibody or affinity reagent can be used as a therapeutic that is administered alone or in combination with cytotoxic factor(s) and/or cytokine(s).

In a further modification, the amino acid sequences of the antibody Fc are altered in a way that enhances ADCC activation, without affecting ligand affinity. Examples of such modifications are described in Lazar et al., Proceedings of the National Academy of Sciences 2006, 103:p4005-4010; WO03074679 and WO2007039818. In these examples, substitution of amino acids in the antibody Fc, such as aspartate for serine at position 239, and isoleucine for glutamate at position 332, altered the binding affinity of an antibody for Fc receptors, leading to an increase in ADCC activation.

An antibody reagent with enhanced ADCC activation due to amino acid substitutions can be used as a therapeutic that is administered alone or in combination with cytotoxic factor(s) and/or cytokine(s).

### Therapeutic Use of the Proteins of the Invention

The disclosure provides for treatment or prevention of various diseases and disorders by administration of a therapeutic compound. Such compounds include but are not limited to: OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, analogs thereof, related polypeptides and derivatives (including fragments) thereof; antibodies (or other affinity reagents) to the foregoing; nucleic acids encoding an OGTA, analogs of the latter or a related polypeptides and fragments thereof; antisense nucleic acids to a gene encoding an OGTA, analogs of the latter or a related polypeptides and fragments thereof; and modulator (e.g., agonists and antagonists) of a gene encoding an OGTA or a related polypeptide. An important feature of the present disclosure is the identification of genes encoding OGTA(s) involved in a relevant cancer. B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma can be treated (e.g. to ameliorate symptoms or to retard onset or progression) or prevented by administration of a therapeutic compound that reduces function or expression of an OGTA in the serum or tissue of subjects having said cancer.

In one embodiment, one or more antibodies (or other affinity reagents) each specifically binding to OGTA126 are administered alone or in combination with one or more additional therapeutic compounds or treatments.

In one embodiment a biological product such as an antibody (or other affinity reagent) is allogeneic to the subject to which it is administered. In another example, a human OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 or a related polypeptide, a nucleotide sequence encoding any of the aforementioned moieties, or an antibody (or other affinity reagent) to a human OGTA or a related polypeptide, is administered to a human subject for therapy (e.g. to ameliorate symptoms or to retard onset or progression) or prophylaxis.

Without being limited by theory, it is conceived that the therapeutic activity of antibodies (or other affinity reagents) which specifically bind to the proteins of the disclosure may be achieved through the phenomenon of Antibody -Dependent Cell-mediated Cytotoxicity (ADCC) (see e.g. Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947).

### Treatment and Prevention and/or diagnosis of B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, Pancreatic Cancer, Prostate Cancer, Renal Cell Cancer and Retinoblastoma

Relevant cancers are treated or prevented by administration to a subject, suspected of having or known to have or to be at risk of developing said cancer, of a compound that modulates (*i.e.,* increases or decreases) the level or activity (*i.e.,* function or expression) of an OGTA according to the disclosure that is differentially present in the serum or tissue of subjects having a relevant cancer listed herein compared with serum or tissue of subjects free from said cancer.

In one example, the cancer/cancers is/are treated or prevented by administering to a subject suspected having or known to have or to be at risk of developing a relevant cancer a compound that upregulates (*i.e.,* increases) the level or activity (*i.e.,* function or expression) of an OGTA according to the disclosure that are decreased in the serum or tissue of subjects having a relevant cancer. Examples of such a compound include, but are not limited to, OGTA antisense oligonucleotides, ribozymes, antibodies (or other affinity reagents) directed against the proteins of the disclosure, and compounds that inhibit the enzymatic activity of the proteins of the disclosure. Other useful compounds e.g. OGTA antagonists and small molecule OGTA antagonists, can be identified using *in vitro* assays.

A relevant cancer/cancers is/are also treated or prevented by administration to a subject suspected of having or known to have said cancer or to be at risk of developing said cancer of a compound that downregulates the level or activity (i.e. function) of an OGTA that are increased in the serum or tissue of subjects having said cancer. Examples of such a compound include but are not limited to: OGTAs according to the disclosure, OGTA fragments and OGTA-related polypeptides; nucleic acids encoding an OGTA, an OGTA fragment and an OGTA-related polypeptide (e.g. for use in gene therapy); and, for those OGTA or OGTA-related polypeptides with enzymatic activity, compounds or molecules known to modulate that enzymatic activity. Other compounds that can be used, e.g. OGTA agonists, can be identified using in *in vitro* assays.

In one example, therapy or prophylaxis is tailored to the needs of an individual subject. Thus, in specific examples, compounds that promote the level or function of an OGTA according to the disclosure are therapeutically or prophylactically administered to a subject suspected of having or known to have a relevant cancer, in whom the levels or functions of an OGTA according to the disclosure are absent or are decreased relative to a control or normal reference range. In further examples, compounds that promote the level or function of an OGTA according to the disclosure are therapeutically or prophylactically administered to a subject suspected of having or known to have a relevant cancer in whom the levels or functions of an OGTA according to the disclosure are increased relative to a control or to a reference range. In further examples, compounds are employed that decrease the level or function of an OGTA according to the disclosure, for example in subjects in whom said levels or functions are increased relative to a control or to a reference range.
In further examples, compounds that decrease the level or function of OGTA according to the disclosure are therapeutically or prophylactically administered to a subject suspected of having or known to have a relevant cancer in whom said levels or functions are increased relative to a control or to a reference range.

In further examples, compounds that decrease the level or function of an OGTA according to the disclosure are therapeutically or prophylactically administered to a subject suspected of having or known to have a relevant cancer in whom said levels or functions are decreased relative to a control or to a reference range.

The change in OGTA function or level due to the administration of such compounds can be readily detected, *e.g.,* by obtaining a sample (*e.g.,* blood or urine) and assaying *in vitro* the levels or activities of an OGTA according to the disclosure, or the levels of mRNAs encoding same, or any combination of the foregoing. Such assays can be performed before and after the administration of the compound as described herein.

The compounds of the disclosure include but are not limited to any compound, *e.g*., a small organic molecule, protein, peptide, antibody (or other affinity reagent), nucleic acid, etc. that restores the relevant OGTAs profile towards normal. The compounds of the disclosure may be given in combination with any other chemotherapy drugs.

In accordance with the present disclosure, test samples of lymphoid, breast, cervical, colorectal, gastric, brain, liver, lung, skin, neuronal, osteoblast, ovarian, pancreatic, prostate, kidney or eye tissue, serum, plasma or urine obtained from a subject suspected of having or known to have a relevant cancer can be used for diagnosis or monitoring. In one example, a change in the abundance of an OGTA according to the disclosure in a test sample relative to a control sample (from a subject or subjects free from said cancer or a previously determined reference range indicates the presence of the relevant cancer.
In another example, the relative abundance of an OGTA according to the disclosure in a test sample compared to a control sample or a previously determined reference range indicates a subtype of the relevant cancer (*e.g*. pre-T-cell or mature T-cell acute lymphocytic leukaemia, diffuse large B-cell lymphoma, inflammatory breast cancer, squamous cell cervical carcinoma, T-cell chronic lymphocytic leukaemia, familial or sporadic colorectal cancer, gastrointestinal stromal tumours, fibrolamellar hepatocellular carcinoma, squamous cell lung carcinoma, ganglioneuroblastoma or familial neuroblastoma, parosteal or periosteal osteosarcoma, malignant papillary serous adenocarcinoma, endocrine tumours of the pancreas or bilateral, multifocal retinoblastoma or unilateral, unifocal retinoblastoma). In yet another example, the relative abundance of OGTA(s) in a test sample relative to a control sample or a previously determined reference range indicates the degree or severity of the relevant cancer (e.g., the likelihood for metastasis). In any of the aforesaid methods, detection of OGTA(s) may optionally be combined with detection of one or more of additional biomarkers for a relevant cancer. Any suitable method in the art can be employed to measure the level of OGTA(s), including but not limited to the Preferred Technologies described in Examples 1 and 2 described herein, kinase assays, immunoassays to detect and/or visualize OGTA(s) (*e.g.,* Western blot, immunoprecipitation followed by sodium dodecyl sulfate polyacrylamide gel electrophoresis, immunocytochemistry, etc.). In a further example, a change in the abundance of mRNA encoding an OGTA according to the disclosure in a test sample relative to a control sample or a previously determined reference range indicates the presence of a relevant cancer. Any suitable hybridization assay can be used to detect expression of an OGTA according to the disclosure by detecting and/or visualizing mRNA encoding an OGTA according to the disclosure (*e.g.,* Northern assays, dot blots, *in situ* hybridization, etc.).

In another example of the disclosure, labeled antibodies (or other affinity reagents), derivatives and analogs thereof, which specifically bind to an OTGA according to the disclosure can be used for diagnostic purposes to detect, diagnose, or monitor a relevant cancer. For example a relevant cancer is detected in an animal, such as in a mammal and particularly in a human.

### Identification of Compounds that Inhibit the Proteins of the Disclosure to Treat B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, Pancreatic Cancer, Prostate Cancer, Renal Cell Cancer and Retinoblastoma

In one example of the disclosure, B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma is treated or prevented by administration of a compound that antagonizes (inhibits) the level(s) and/or function(s) of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 which are elevated in the serum or tissue of subjects having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma as compared with serum or tissue of subjects free from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma.

Compounds useful for this purpose include but are not limited to anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 antibodies (or other affinity reagents, and fragments and derivatives containing the binding region thereof), OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 antisense or ribozyme nucleic acids, and nucleic acids encoding dysfunctional OGTAs that are used to "knockout" endogenous OGTA function by homologous recombination (see, *e.g.,* Capecchi, 1989, Science 244:1288-1292). Other compounds that inhibit OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function can be identified by use of known *in vitro* assays, *e.g.,* assays for the ability of a test compound to inhibit binding of an OGTA according to the disclosure to another protein or a binding partner, or to inhibit a known OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function. Such inhibition may, for example be assayed *in vitro* or in cell culture, but genetic assays may also be employed. The Preferred Technologies described in Examples 1 and 2 can also be used to detect levels of an OGTA according to the disclosure before and after the administration of the compound. Preferably, suitable *in vitro* or *in vivo* assays are utilized to determine the effect of a specific compound and whether its administration is indicated for treatment of the affected tissue.

In a specific example, a compound that inhibits OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function is administered therapeutically or prophylactically to a subject in whom an increased serum or tissue level or functional activity of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 (*e.g*., greater than the normal level or desired level) is detected as compared with serum or tissue of subjects free from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma or a predetermined reference range.

Methods standard in the art can be employed to measure the increase in the level or function of an OGTA according to the disclosure, as outlined above.

In one example OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 inhibitor compositions include small molecules, i.e. molecules of 1000 daltons or less. Such small molecules can be identified by the screening methods described herein.

### Therapeutic and Prophylactic Compositions and their Use

The disclosure provides methods of treatment (and prophylaxis) comprising administering to a subject an effective amount of a compound of the disclosure. In a preferred example, the compound is substantially purified (*e.g*., substantially free from substances that limit its effect or produce undesired side-effects). The subject is preferably an animal, including but not limited to animals such as cows, pigs, horses, chickens, cats, dogs, etc., and is preferably a mammal, and most preferably human. In a specific example, a non-human mammal is the subject.

Formulations and methods of administration that can be employed when the compound comprises a nucleic acid are described above; additional appropriate formulations and routes of administration are described below.
Various delivery systems are known and can be used to administer a compound of the invention, *e.g.*, encapsulation in liposomes, microparticles, microcapsules, recombinant cells capable of expressing the compound, receptor-mediated endocytosis (see, *e.g.,* Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local. In addition, it may be desirable to introduce the pharmaceutical compositions of the disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g*., by use of an inhaler or nebulizer, and formulation with an aerosolizing agent.

In a specific example, it may be desirable to administer the pharmaceutical compositions of the disclosure locally to the area in need of treatment; this may be achieved, for example, and not by way of limitation, by local infusion during surgery, topical application, *e.g*., by injection, by means of a catheter, or by means of an implant, said implant being of a porous, non-porous, or gelatinous material, including membranes, such as sialastic membranes, or fibers. In one example, administration can be by direct injection into lymphoid, breast, cervical, colorectal, gastric, brain, liver, lung, skin, neuronal, osteoblast, ovarian, pancreatic, prostate, kidney and eye tissue or at the site (or former site) of a malignant tumour or neoplastic or pre-neoplastic tissue.
In another example, the compound can be delivered in a vesicle, in particular a liposome (*see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, *ibid.,* pp. 317-327; *see* generally *ibid*.).

In yet another example, the compound can be delivered in a controlled release system. In one example, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another example, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another example, a controlled release system can be placed in proximity of the therapeutic target, *e.g.* the breast, cervix, colon, stomach, brain, liver, lung, skin, bone, ovary, pancreas, prostate, kidney or eye, thus requiring only a fraction of the systemic dose (see, *e.g.,* Goodson, in Medical Applications of Controlled Release, supra, vol. 2, pp. 115-138 (1984)).
Other controlled release systems are discussed in the review by Langer (1990, Science 249:1527-1533).

In a specific example where the compound of the disclosure is a nucleic acid encoding a protein, the nucleic acid can be administered *in vivo* to promote expression of its encoded protein, by constructing it as part of an appropriate nucleic acid expression vector and administering it so that it becomes intracellular, *e.g.,* by use of a retroviral vector (see U.S. Patent No. 4,980,286), or by direct injection, or by use of microparticle bombardment (*e.g*., a gene gun; Biolistic, Dupont), or coating with lipids or cell-surface receptors or transfecting agents, or by administering it in linkage to a homeobox-like peptide which is known to enter the nucleus (see *e.g.,* Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The present disclosure also provides pharmaceutical compositions. Such compositions comprise a therapeutically effective amount of a compound, and a pharmaceutically acceptable carrier. In a specific example, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water may be a suitable carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

In one embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the disclosure can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.
The amount of the compound of the disclosure which will be effective in the treatment of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight or more such as 10 or 100mg/kg. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The disclosure also provides a pharmaceutical pack or kit comprising one or more containers filled with one or more of the ingredients of the pharmaceutical compositions of the disclosure. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

### Diagnosis and Treatment of B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, Pancreatic Cancer, Prostate Cancer, Renal Cell Cancer and Retinoblastoma using Immunohistochemistry

Immunohistochemistry is an excellent detection technique and may therefore be very useful in the diagnosis and treatment of a relevant cancer. Immunohistochemistry may be used to detect, diagnose, or monitor a relevant cancer through the localization of OGTA antigens in tissue sections by the use of labeled antibodies (or other affinity reagents), derivatives and analogs thereof, which specifically bind to the proteins of the disclosure, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutical approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr;2(4):371-93.

### EXAMPLE 1:

### IDENTIFICATION OF MEMBRANE PROTEINS EXPRESSED IN B-CELL NON-HODGKIN'S LYMPHOMA, BREAST CANCER, CERVICAL CANCER, COLORECTAL CANCER, GASTRIC CANCER, GLIOBLASTOMA, HEPATOCELLULAR CARCINOMA, LUNG CANCER, LYMPHOID LEUKAEMIA (PARTICULARLY ACUTE T-CELL LEUKAEMIA AND CHRONIC LYMPHOCYTIC LEUKAEMIA), MELANOMA, NEUROBLASTOMA, OSTEOSARCOMA, OVARIAN CANCER, PANCREATIC CANCER, PROSTATE CANCER, RENAL CELL CANCER AND RETINOBLASTOMA BLOOD AND TISSUE SAMPLES

Using the following Reference Protocol, membrane proteins extracted from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma tissue samples were separated by ID gel and analysed.

### 1.1 MATERIALS AND METHODS

### 1.1.1 - Plasma Membrane Fractionation

The cells recovered from a B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma were lysed and submitted to centrifugation at 1000G. The supernatant was taken, and it was subsequently centrifuged at 3000G. Once again, the supernatant was taken, and it was then centrifuged at 100 000G.

The resulting pellet was recovered and put on 15-60% sucrose gradient.

A Western blot was used to identify sub cellular markers, and the Plasma Membrane fractions were pooled.

The pooled solution was either run directly on ID gels (see section 1.1.4 below), or further fractionated into heparin binding and nucleotide binding fractions as described below.

### 1.1.2 - Plasma Membrane Heparin-binding Fraction

The pooled solution from 1.1.1 above was applied to a Heparin column, eluted from column and run on ID gels (see section 1.1.4 below).

### 1.1.3 - Plasma Nucleotide-binding Fraction

The pooled solution from 1.1.1 above was applied to a Cibacrom Blue 3GA column, eluted from column and run on ID gels (see section 1.1.4 below).

### 1.1.4 - 1D gel technology

Protein or membrane pellets were solubilised in ID sample buffer (1-2 µg/µl). The sample buffer and protein mixture was then heated to 95 °C for 3 min.

A 9-16% acrylamide gradient gel was cast with a stacking gel and a stacking comb according to the procedure described in Ausubel F.M. et al., eds., 1989, Current Protocols in Molecular Biology, Vol. II, Green Publishing Associates, Inc., and John Wiley & Sons, Inc., New York, section 10.2.

30-50 micrograms of the protein mixtures obtained from detergent and the molecular weight standards (66, 45 ,31, 21,14 kDa) were added to the stacking gel wells using a 10 microlitre pipette tip and the samples run at 40mA for 5 hours.

The plates were then prised open, the gel placed in a tray of fixer (10% acetic acid, 40% ethanol, 50% water) and shaken overnight. Following this, the gel was primed by 30 minutes shaking in a primer solution (7.5% acetic acid (75ml), 0.05% SDS (5ml of 10%)). The gel was then incubated with a fluorescent dye (7.5% acetic acid, 0.06% OGS in-house dye (600µl)) with shaking for 3 hrs. Sypro Red (Molecular Probes, Inc., Eugene, Oregon) is a suitable dye for this purpose. A preferred fluorescent dye is disclosed in U.S. Application No. 09/412,168, filed on October 5, 1999.

A computer-readable output was produced by imaging the fluorescently stained gels with an Apollo 3 scanner (Oxford Glycosciences, Oxford, UK). This scanner is developed from the scanner described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686. The latest embodiment of this instrument includes the following improvements: The gel is transported through the scanner on a precision lead-screw drive system. This is preferable to laying the glass plate on the belt-driven system that is defined in the Basiji thesis as it provides a reproducible means of accurately transporting the gel past the imaging optics.

The gel is secured into the scanner against three alignment stops that rigidly hold the glass plate in a known position. By doing this in conjunction with the above precision transport system and the fact that the gel is bound to the glass plate, the absolute position of the gel can be predicted and recorded. This ensures that accurate co-ordinates of each feature on the gel can be communicated to the cutting robot for excision. This cutting robot has an identical mounting arrangement for the glass plate to preserve the positional accuracy.

The carrier that holds the gel in place has integral fluorescent markers (Designated M1, M2, M3) that are used to correct the image geometry and are a quality control feature to confirm that the scanning has been performed correctly.

The optical components of the system have been inverted. The laser, mirror, waveguide and other optical components are now above the glass plate being scanned. The embodiment of the Basiji thesis has these underneath. The glass plate is therefore mounted onto the scanner gel side down, so that the optical path remains through the glass plate. By doing this, any particles of gel that may break away from the glass plate will fall onto the base of the instrument rather than into the optics.

In scanning the gels, they were removed from the stain, rinsed with water and allowed to air dry briefly and imaged on the Apollo 3. After imaging, the gels were sealed in polyethylene bags containing a small volume of staining solution, and then stored at 4°C.

Apparent molecular weights were calculated by interpolation from a set of known molecular weight markers run alongside the samples.

### 1.1.5 - Recovery and analysis of selected proteins

Proteins were robotically excised from the gels by the process described in U.S. Patent No. 6,064,754, Sections 5.4 and 5.6, 5.7, 5.8, as is applicable to 1D-electrophoresis, with modification to the robotic cutter as follows: the cutter begins at the top of the lane, and cuts a gel disc 1.7mm in diameter from the left edge of the lane. The cutter then moves 2mm to the right, and 0.7mm down and cuts a further disc. This is then repeated. The cutter then moves back to a position directly underneath the first gel cut, but offset by 2.2mm downwards, and the pattern of three diagonal cuts are repeated. This is continued for the whole length of the gel.

NOTE: If the lane is observed to broaden significantly then a correction can be made also sideways i.e. instead of returning to a position directly underneath a previous gel cut, the cut can be offset slightly to the left (on the left of the lane) and/or the right (on the right of the lane). The proteins contained within the gel fragments were processed to generate tryptic peptides; partial amino acid sequences of these peptides were determined by mass spectroscopy as described in WO98/53323 and Application No. 09/094,996, filed June 15, 1998.

Proteins were processed to generate tryptic digest peptides. Tryptic peptides were analyzed by mass spectrometry using a PerSeptive Biosystems Voyager- DETM STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflowTM electrospray Z-spray source. For partial amino acid sequencing and identification of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was a database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662).

### 1.1.6- Discrimination of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma associated proteins

The process to identify OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 uses the peptide sequences obtained experimentally by mass spectrometry described above of naturally occurring human proteins to identify and organise coding exons in the published human genome sequence.

Recent dramatic advances in defining the chemical sequence of the human genome have led to the near completion of this immense task (Venter, J.C. et al. (2001). The sequence of the human genome. Science 16: 1304-51; International Human Genome Sequencing Consortium. (2001). Initial sequencing and analysis of the human genome Nature 409: 860-921). There is little doubt that this sequence information will have a substantial impact on our understanding of many biological processes, including molecular evolution, comparative genomics, pathogenic mechanisms and molecular medicine. For the full medical value inherent in the sequence of the human genome to be realised, the genome needs to be 'organised' and annotated. By this, is meant at least the following three things: (i) The assembly of the sequences of the individual portions of the genome into a coherent, continuous sequence for each chromosome. (ii) The unambiguous identification of those regions of each chromosome that contain genes. (iii) Determination of the fine structure of the genes and the properties of its mRNA and protein products. While the definition of a 'gene' is an increasingly complex issue (H Pearson: What is a gene? Nature (2006) 24: 399 - 401), what is of immediate interest for drug discovery and development is a catalogue of those genes that encode functional, expressed proteins. A subset of these genes will be involved in the molecular basis of most if not all pathologies. Therefore an important and immediate goal for the pharmaceutical industry is to identify all such genes in the human genome and describe their fine structure.

### Processing and integration of peptide masses, peptide signatures, ESTs and Public Domain Genomic Sequence Data to form OGAP® database

Discrete genetic units (exons, transcripts and genes) were identified using the following sequential steps:
1. A 'virtual transcriptome' is generated, containing the tryptic peptides which map to the human genome by combining the gene identifications available from Ensembl and various gene prediction programs. This also incorporates SNP data (from dbSNP) and all alternate splicing of gene identifications. Known contaminants were also added to the virtual transcriptome.
2. All tandem spectra in the OGeS Mass Spectrometry Database are interpreted in order to produce a peptide that can be mapped to one in the virtual transcriptome. A set of automated spectral interpretation algorithms were used to produce the peptide identifications.
3. The set of all mass-matched peptides in the OGeS Mass Spectrometry Database is generated by searching all peptides from transcripts hit by the tandem peptides using a tolerance based on the mass accuracy of the mass spectrometer, typically 20ppm.
4. All tandem and mass-matched peptides are combined in the form of "protein clusters". This is done using a recursive process which groups sequences into clusters based on common peptide hits. Biological sequences are considered to belong to the same cluster if they share one or more tandem or mass-matched peptide.
5. After initial filtering to screen out incorrectly identified peptides, the resulting clusters are then mapped on the human genome.
6. The protein clusters are then aggregated into regions that define preliminary gene boundaries using their proximity and the co-observation of peptides within protein clusters. Proximity is defined as the peptide being within 80,000 nucleotides on the same strand of the same chromosome. Various elimination rules, based on cluster observation scoring and multiple mapping to the genome are used to refine the output. The resulting 'confirmed genes' are those which best account for the peptides and masses observed by mass spectrometry in each cluster. Nominal co-ordinates for the gene are also an output of this stage.
7. The best set of transcripts for each confirmed gene are created from the protein clusters, peptides, ESTs, candidate exons and molecular weight of the original protein spot.
8. Each identified transcript was linked to the sample providing the observed peptides.
9. Use of an application for viewing and mining the data. The result of steps 1 - 8 was a database containing genes, each of which consisted of a number of exons and one or more transcripts. An application was written to display and search this integrated genome / proteome data. Any features (OMIM disease locus, InterPro etc.) that had been mapped to the same Golden Path co-ordinate system by Ensembl could be cross-referenced to these genes by coincidence of location and fine structure.

The process was used to generate approximately 1 million peptide sequences to identify protein-coding genes and their exons resulted in the identification of protein sequences for 18083 genes across 67 different tissues and 57 diseases including 2,025 genes in acute T-cell leukaemia, 501 genes in B-cell non-Hodgkin's lymphoma, 4,713 genes in breast cancer, 1,371 genes in cervical cancer, 2,424 genes in chronic lymphocytic leukaemia, 949 genes in colorectal cancer, 524 genes in gastric cancer, 1,544 genes in glioblastoma, 1,782 genes in hepatocellular carcinoma, 978 genes in lung cancer, 373 genes in lymphoid leukaemia (unspecified), 1,764 genes in melanoma, 1,391 genes in neuroblastoma, 1,324 genes in osteosarcoma, 1,033 genes in ovarian cancer, 2,961 genes in pancreatic cancer, 3,307 genes in prostate cancer, 1005 genes in renal cell cancer and 1,783 genes in retinoblastoma, illustrated here by OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 isolated and identified from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma samples. Following comparison of the experimentally determined sequences with sequences in the OGAP® database, OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 showed a high degree of specificity to B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma indicative of the prognostic and diagnostic nature.

### 1.2 RESULTS

These experiments identified OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, as further described herein.
The full-length OGTA002 was detected in the plasma membrane of breast cancer, colorectal cancer, hepatocellular carcinoma, melanoma, ovarian cancer and pancreatic cancer samples. The full-length OGTA009 was detected in the plasma membrane of breast cancer, colorectal cancer, pancreatic cancer, prostate cancer and renal cell cancer samples. The full-length OGTA016 was detected in the plasma membrane of colorectal cancer samples. The full-length OGTA028 was detected in the plasma membrane of ovarian cancer samples. The full-length OGTA037 was detected in the plasma membrane of gastric cancer samples. The full-length OGTA041 was detected in the plasma membrane of hepatocellular carcinoma, lung cancer and pancreatic cancer samples. The full-length OGTA053 was detected in the plasma membrane of pancreatic cancer samples. The full-length OGTA054 was detected in the plasma membrane of pancreatic cancer samples. The full-length OGTA066 was detected in the plasma membrane of colorectal cancer and pancreatic cancer samples. The full-length OGTA072 was detected in the plasma membrane of colorectal cancer samples. The full-length OGTA074 was detected in the plasma membrane of colorectal cancer samples. The full-length OGTA076 was detected in the plasma membrane of chronic lymphocytic leukaemia, colorectal cancer and pancreatic cancer samples. The full-length OGTA085 was detected in the plasma membrane of lung cancer, melanoma and retinoblastoma samples. The full-length OGTA087 was detected in the plasma membrane of B-cell non-Hodgkin's lymphoma, breast cancer, colorectal cancer, gastric cancer, lung cancer, lymphoid leukaemia and ovarian cancer samples. The full-length OGTA088 was detected in the plasma membrane of breast cancer, gastric cancer, glioblastoma, hepatocellular carcinoma, lung cancer, lymphoid leukaemia, pancreatic cancer, renal cell cancer and retinoblastoma samples. The full-length OGTA089 was detected in the plasma membrane of breast cancer, lung cancer and ovarian cancer samples. The full-length OGTA091 was detected in the plasma membrane of breast cancer, cervical cancer, chronic lymphocytic leukaemia, gastric cancer, hepatocellular carcinoma, lung cancer, melanoma, osteosarcoma, ovarian cancer, pancreatic cancer, prostate cancer and renal cell cancer samples. The full-length OGTA098 was detected in the plasma membrane of breast cancer, cervical cancer, hepatocellular carcinoma, lung cancer, osteosarcoma, pancreatic cancer, prostate cancer and renal cell cancer samples. The full-length OGTA101 was detected in the plasma membrane of breast cancer, cervical cancer, colorectal cancer, hepatocellular carcinoma, lung cancer, osteosarcoma and pancreatic cancer samples. The full-length OGTA104 was detected in the plasma membrane of breast cancer, hepatocellular carcinoma, ovarian cancer and pancreatic cancer samples. The full-length OGTA106 was detected in the plasma membrane of cervical cancer, melanoma and pancreatic cancer samples. The full-length OGTA112 was detected in the plasma membrane of breast cancer, cervical cancer, chronic lymphocytic leukaemia, hepatocellular carcinoma, pancreatic cancer and renal cell cancer samples. The full-length OGTA113 was detected in the plasma membrane of pancreatic cancer samples. The full-length OGTA119 was detected in the plasma membrane of B-cell non-Hodgkin's lymphoma, breast cancer, colorectal cancer, gastric cancer, hepatocellular carcinoma, lung cancer, lymphoid leukaemia, neuroblastoma, osteosarcoma, pancreatic cancer, prostate cancer and renal cell cancer samples. The full-length OGTA124 was detected in the plasma membrane of hepatocellular carcinoma, lung cancer, ovarian cancer, pancreatic cancer and renal cell cancer samples. The full-length OGTA126 was detected in the plasma membrane of breast cancer, colorectal cancer, hepatocellular carcinoma, melanoma, pancreatic cancer, prostate cancer and renal cell cancer samples. The full-length OGTA156 was detected in the plasma membrane of acute T-cell leukaemia, B-cell non-Hodgkin's lymphoma, chronic lymphocytic leukaemia and hepatocellular carcinoma samples. The full-length OGTA159 was detected in the plasma membrane of breast cancer, chronic lymphocytic leukaemia, colorectal cancer, hepatocellular carcinoma, melanoma and pancreatic cancer samples. The full-length OGTA168 was detected in the plasma membrane of glioblastoma and melanoma samples. The full-length OGTA169 was detected in the plasma membrane of breast cancer and chronic lymphocytic leukaemia samples. The full-length OGTA174 was detected in the plasma membrane of acute T-cell leukaemia and chronic lymphocytic leukaemia samples. The full-length OGTA176 was detected in the plasma membrane of B-cell non-Hodgkin's lymphoma and chronic lymphocytic leukaemia samples. The full-length OGTA177 was detected in the plasma membrane of chronic lymphocytic leukaemia samples. The full-length OGTA197 was detected in the plasma membrane of colorectal cancer, hepatocellular carcinoma, lung cancer, melanoma, osteosarcoma, ovarian cancer, pancreatic cancer, prostate cancer and renal cell cancer samples. The full-length OGTA202 was detected in the plasma membrane of colorectal cancer samples. The full-length OGTA203 was detected in the plasma membrane of ovarian cancer and renal cell cancer samples. The full-length OGTA206 was detected in the plasma membrane of breast cancer, pancreatic cancer and prostate cancer samples. The full-length OGTA213 was detected in the plasma membrane of lung cancer samples. The full-length OGTA214 was detected in the plasma membrane of ovarian cancer samples. The full-length OGTA216 was detected in the plasma membrane of breast cancer, colorectal cancer and pancreatic cancer samples. The full-length OGTA222 was detected in the plasma membrane of B-cell non-Hodgkin's lymphoma and colorectal cancer samples. The full-length OGTA236 was detected in the plasma membrane of pancreatic cancer samples. The full-length OGTA237 was detected in the plasma membrane of B-cell non-Hodgkin's lymphoma, lymphoid leukaemia and prostate cancer samples. The full-length OGTA247 was detected in the plasma membrane of hepatocellular carcinoma and melanoma samples. The full-length OGTA248 was detected in the plasma membrane of renal cell cancer samples. The full-length OGTA249 was detected in the plasma membrane of ovarian cancer samples. The full-length OGTA257 was detected in the plasma membrane of pancreatic cancer samples. The full-length OGTA271 was detected in the plasma membrane of breast cancer, cervical cancer, colorectal cancer, hepatocellular carcinoma, lung cancer, osteosarcoma, pancreatic cancer and renal cell cancer samples.

### EXAMPLE 2: IDENTIFICATION OF MEMBRANE PROTEINS EXPRESSED IN COLORECTAL CANCER, KIDNEY CANCER, LIVER CANCER, LUNG CANCER OR OVARIAN CANCER BLOOD AND TISSUE SAMPLES USING ISOTOPE TAGGING FOR ABSOLUTE AND RELATIVE QUANTITATION (iTRAQ)

Using the following Reference Protocol, membrane proteins extracted from colorectal cancer, kidney cancer, liver cancer, lung cancer and ovarian cancer tissue and normal adjacent colorectal and lung tissue samples were digested, labelled with Isotope Tagging for Absolute & Relative Quantitation reagents (iTRAQ; Applied Biosystems, Foster City, CA, USA) and resulting peptides sequenced by tandem mass spectrometry.

### 2.1 MATERIALS AND METHODS

### 2.1.1 - Plasma Membrane Fractionation

The cells recovered from a colorectal cancer, kidney cancer, liver cancer, lung cancer or ovarian cancer or normal adjacent colorectal, kidney, liver, lung or ovarian tissue were lysed and submitted to centrifugation at 1000G. The supernatant was taken, and it was subsequently centrifuged at 3000G. Once again, the supernatant was taken, and it was then centrifuged at 100 000G.

The resulting pellet was recovered and put on 15-60% sucrose gradient.

A Western blot was used to identify sub cellular markers, and the Plasma Membrane fractions were pooled.

The pooled solution was then analysed directly by iTRAQ (see section 2.1.2 below).

### 2.1.2 - iTRAQ methodology

Membrane protein pellets from colorectal cancer, kidney cancer, liver cancer, lung cancer or ovarian cancer and normal adjacent colorectal, kidney, liver, lung or ovarian tissue were solubilised in sample buffer (2-4 µg/µl in 0.5% SDS) by the addition of buffer and then heating to 95°C for 3 min.

To a volume of each protein solution equating to 50µg, 150µl of 0.5M triethylammonium bicarbonate (TEAB) solution was added. To each sample, 3µl of 50mM tris-(2-carboxyethyl)phosphine was added and the mixture was incubated at 60°C for 1 hour. 1µl of cysteine blocking reagent, 200mM methyl methanethiosulphonate (MMTS) in isopropanol, was then added. After incubation at room temperature for 10 minutes, 15µl of 1µg/µl trypsin was added to each sample followed by incubation at 37°C overnight.

The digested samples were dried under a vacuum and re-constituted with 30µl of 0.5M TEAB solution. 70µl ethanol was added to each of the four iTRAQ reagents (114/115/116/117) and one reagent added to each of the four samples analysed (two colorectal cancer, kidney cancer, liver cancer, lung cancer or ovarian cancer samples and two corresponding normal adjacent tissue samples) and left at room temperature for 1 hour. The specific reagent added to each sample was recorded. The four labeled samples were combined & vortexed.

The combined sample was reduced to dryness under a vacuum and de-salted by loading onto a C18 spin column, washing with aqueous solvent and then eluting with 70% acetonitrile. The sample fraction was again reduced to dryness and then re-dissolved in 40µl of solvent A (97.9 water, 2% acetonitrile, 0.1% formic acid) prior to ion exchange fractionation.

### 2.1.3 - Fractionation and analysis of labeled peptides

The sample was fractionated by strong cation exchange chromatography using an Agilent 1200 chromatograph (Agilent, Santa Clara, CA, USA). Samples were eluted off an Agilent Zorbax Bio-SCXII column (3.5µm; 50 x 0.8mm) using a 20µl/min gradient of 0-100mM sodium acetate over 20 minutes and then to 1M over 10 minutes. 1 minute fractions were collected over the 30 minute run.

Each fraction was analysed by liquid chromatography/mass spectrometry using an Agilent 1200 chromatograph fitted with a Zorbax 300SB-C18 (150mm x 75µm) and an Agilent 6510 quadrupole - time-of-flight instrument (Agilent, Santa Clara, CA, USA). Peptides were eluted with a 300nl/min gradient increasing from 15% to 45% acetonitrile in 60 minutes. Data was acquired in auto MS/MS mode such that up to 3 precursor ions above the intensity threshold were selected and product ion spectra accumulated to facilitate the sequencing of the labeled peptides. Raw was processed to create peak lists using Spectrum Mill software (Agilent, Santa Clara, CA, USA).

### 2.1.4 - Amino acid sequence analysis of labeled peptides

For partial amino acid sequencing and identification of OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, uninterpreted tandem mass spectra of tryptic peptides were searched using the SEQUEST search program (Eng et al., 1994, J. Am. Soc. Mass Spectrom. 5:976-989). Criteria for database identification included: the cleavage specificity of trypsin; the detection of a suite of a, b and y ions in peptides returned from the database, and a mass increment for all cysteine residues to account for modification with methyl methanethiosulphonate and the addition of iTRAQ labels to free amines (N-terminus & lysine). The data was searched through IPI Human v3.23 (www.ebi.ac.uk/IPI/IPIhuman.html).

### 2.1.5 - Discrimination of colorectal cancer, kidney cancer, liver cancer, lung cancer and ovarian cancer associated proteins

The process described in Example 1 section 1.1.6 was employed to discriminate the colorectal cancer, kidney cancer, liver cancer, lung cancer and ovarian cancer associated proteins in the experimental samples.

### 2.2 RESULTS

These experiments identified OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, as further described herein.

The full-length OGTA002 was detected in the plasma membrane of colorectal cancer, hepatocellular carcinoma, lung cancer and renal cell cancer samples. The full-length OGTA009 was detected in the plasma membrane of colorectal cancer, lung cancer and ovarian cancer samples. The full-length OGTA016 was detected in the plasma membrane of colorectal cancer and lung cancer samples. The full-length OGTA028 was detected in the plasma membrane of colorectal cancer, hepatocellular carcinoma, lung cancer, ovarian cancer and renal cell cancer samples. The full-length OGTA037 was detected in the plasma membrane of colorectal cancer, lung cancer and ovarian cancer samples. The full-length OGTA041 was detected in the plasma membrane of lung cancer samples. The full-length OGTA053 was detected in the plasma membrane of colorectal cancer, hepatocellular carcinoma, lung cancer and renal cell cancer samples. The full-length OGTA054 was detected in the plasma membrane of colorectal cancer and lung cancer samples. The full-length OGTA066 was detected in the plasma membrane of colorectal cancer, lung cancer and renal cell cancer samples. The full-length OGTA074 was detected in the plasma membrane of lung cancer samples. The full-length OGTA076 was detected in the plasma membrane of colorectal cancer, hepatocellular carcinoma, lung cancer, ovarian cancer and renal cell cancer samples. The full-length OGTA085 was detected in the plasma membrane of colorectal cancer and lung cancer samples. The full-length OGTA088 was detected in the plasma membrane of colorectal cancer and lung cancer samples. The full-length OGTA089 was detected in the plasma membrane of lung cancer, ovarian cancer and renal cell cancer samples. The full-length OGTA091 was detected in the plasma membrane of breast colorectal cancer, hepatocellular carcinoma, lung cancer, ovarian cancer and renal cell cancer samples. The full-length OGTA098 was detected in the plasma membrane of colorectal cancer, lung cancer, ovarian cancer and renal cell cancer samples. The full-length OGTA101 was detected in the plasma membrane of colorectal cancer, hepatocellular carcinoma, lung cancer, ovarian cancer and renal cell cancer samples. The full-length OGTA104 was detected in the plasma membrane of colorectal cancer, hepatocellular carcinoma, lung cancer, ovarian cancer and renal cell cancer samples. The full-length OGTA106 was detected in the plasma membrane of hepatocellular carcinoma samples. The full-length OGTA112 was detected in the plasma membrane of renal cell cancer samples. The full-length OGTA113 was detected in the plasma membrane of colorectal cancer, lung cancer, ovarian cancer and renal cell cancer samples. The full-length OGTA119 was detected in the plasma membrane of colorectal cancer, lung cancer, and ovarian cancer samples. The full-length OGTA124 was detected in the plasma membrane of colorectal cancer, hepatocellular carcinoma, lung cancer, ovarian cancer and renal cell cancer samples. The full-length OGTA126 was detected in the plasma membrane of lung cancer samples. The full-length OGTA156 was detected in the plasma membrane of colorectal cancer, lung cancer, ovarian cancer and renal cell cancer samples. The full-length OGTA159 was detected in the plasma membrane of lung cancer and renal cell cancer samples. The full-length OGTA169 was detected in the plasma membrane of colorectal cancer, lung cancer and renal cell cancer samples. The full-length OGTA174 was detected in the plasma membrane of lung cancer samples. The full-length OGTA176 was detected in the plasma membrane of colorectal cancer, lung cancer and renal cell cancer samples. The full-length OGTA177 was detected in the plasma membrane of colorectal cancer, lung cancer, ovarian cancer and renal cell cancer samples. The full-length OGTA197 was detected in the plasma membrane of colorectal cancer, hepatocellular carcinoma, lung cancer and renal cell cancer samples. The full-length OGTA202 was detected in the plasma membrane of colorectal cancer and lung cancer samples. The full-length OGTA203 was detected in the plasma membrane of lung cancer and renal cell cancer samples. The full-length OGTA206 was detected in the plasma membrane of colorectal cancer and lung cancer samples. The full-length OGTA213 was detected in the plasma membrane of colorectal cancer and lung cancer samples. The full-length OGTA214 was detected in the plasma membrane of colorectal cancer samples. The full-length OGTA216 was detected in the plasma membrane of colorectal cancer, hepatocellular carcinoma, lung cancer, ovarian cancer and renal cell cancer samples. The full-length OGTA222 was detected in the plasma membrane of colorectal cancer, lung cancer and renal cell cancer samples. The full-length OGTA236 was detected in the plasma membrane of colorectal cancer samples. The full-length OGTA237 was detected in the plasma membrane of colorectal cancer, lung cancer and renal cell cancer samples. The full-length OGTA247 was detected in the plasma membrane of lung cancer samples. The full-length OGTA248 was detected in the plasma membrane of lung cancer samples. The full-length OGTA249 was detected in the plasma membrane of lung cancer and renal cell cancer samples. The full-length OGTA257 was detected in the plasma membrane of colorectal cancer, hepatocellular carcinoma, lung cancer and ovarian cancer samples. The full-length OGTA271 was detected in the plasma membrane of colorectal cancer, hepatocellular carcinoma, lung cancer and renal cell cancer samples.

### EXAMPLE 3:

### EVALUATION OF COLORECTAL CANCER MARKER PROTEINS IN SANDWICH ELISA

Using the following Reference Protocol, colorectal cancer marker proteins, including OGTA066 were evaluated in a sandwich ELISA.

### 3.1 MATERIALS AND METHODS

Antibodies for the sandwich ELISAs were developed at Biosite. Biotinylated antibody (primary antibody) was diluted into assay buffer (10 mM Tris, 150 mM NaCl, 1% BSA) to 2 ug/ml and added to 384 well neutravidin coated plate (Pierce Chemical Company, Rockford IL) and allowed to incubate at room temperature for 1 hour. Wells were then washed with wash buffer (20mM Borate, 150 mM NaCl, 0.2% Tween 20). Samples and standards were added and allowed to incubate at room temperature for 1 hour. Wells again were washed. An antibody conjugated to fluorscein (secondary antibody) was diluted into assay buffer to 2 ug/ml and was then added to the plate and allowed to incubate at room temperature for 1 hour. Wells again were washed. Anti-fluorscein antibody conjugated to alkaline phosphatase, diluted 1/2338 into assay buffer, was added and allowed to incubate at room temperature for 1hour. Final wash was then performed. Finally substrate (Promega Attophos Product#S1011, Promega Corporation, Madison, WI) was added and the plate was read immediately. All additions were 10 ul/well. The plate was washed 3 times between each addition and final wash was 9 times prior to the addition of substrate. Standards were prepared by spiking specific antigen into a normal serum patient pool. Reading was performed using a Tecan Spectrafluor plus (Tecan Inc, Mannedorf, Switzerland) in kinetic mode for 6 read cycles with excitation filter of 430nm and an emission filter 570nm emission. Slope of RFU/seconds was determined.

Final Box and ROC results were analyzed using Analyse-it General + Clinical Laboratory 1.73 (Analyse-it Software Ltd., Leeds England).

### 3.2 RESULTS

These experiments identified proteins of particular interest including, but not limited to, OGTA066 (SEQ ID No: 9).

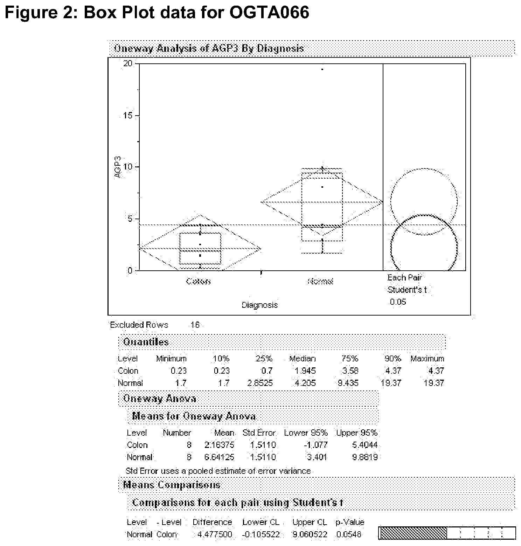

Figure 2 shows Box plot data for OGTA066. The vertical axis on this graph is concentration of OGTA066 in ng/ml. These data show decreased concentration of OGTA066 in colorectal cancer samples compared to normal samples, with an almost significant p value, thereby indicating that OGTA066 discriminates well between colorectal cancer and normal, making it a good potential marker for colorectal cancer.

### EXAMPLE 4:

### EVALUATION OF COLORECTAL CANCER MARKER PROTEINS IN MULTIPLEX ASSAY USING LUMINEX TECHNOLOGY

Using the following Reference Protocol, OGTA066 was evaluated in a multiplex assay using the Luminex technology.

### 4.1 MATERIALS AND METHODS

Each primary antibody was conjugated to a unique Luminex magnetic microsphere (Mug beads, Luminex Corporation, Austin, TX). Mag bead cocktail (50ul) was added to a 96 black well round bottom Costar plate (Corning Incorporated, Corning NY). Using a 96 well magnetic ring stand, the Mag beads were pulled down for 1 minute and washed with wash/assay buffer (PBS with 1% BSA and 0.02% Tween 20). 50ul of sample or standard was added along with an additional 50ul of wash/assay buffer and allowed to incubate on a shaker for 1 hour at room temperature. Plate was placed on magnetic ring stand and allowed to sit for 1 minute. Mag beads were then washed again. Biotin labeled antibody was then added at 50ul per well with an additional 50ul of wash/assay buffer and allowed to incubate on a shaker for 1 hour at room temperature. The plate again was placed on a magnetic stand and the Mag beads were washed. Streptavidin-RPE (Prozyme, San Leandro, CA, Phycolin, Code#PJ31S) was diluted to 1ug/ml in wash/assay buffer and 50ul was added to each well along with an additional 50ul of wash/assay buffer and allowed to incubate on a shaker for 1 hour at room temperature. Final wash was performed and the beads were re-suspended with 100 ul of wash/assay buffer and each well was then read in a Luminex 200 reader using Xponent software 3.0. All reagent dilutions were made in wash/assay buffer. Biotin-antibody varied for each assay to optimal concentration. Initial Mag bead amounts added were approximately 50,000 for each assay. Magnetic beads were allowed 1 minute pull down time prior to each wash. Each wash step was 3 times washed with 100ul of wash/assay buffer. Assay standard curves were made in a normal donor patient serum pool. Luminex reader and Mag beads were used and prepared according to manufacturer guidelines. Standard curves were calculated using a 5 parameter log-logistic fit and each sample concentration was determined from this curve fit.

Final Box and ROC results were analyzed using Analyse-it General + Clinical Laboratory 1.73 (Analyse-it Software Ltd., Leeds England).

### 4.2 RESULTS

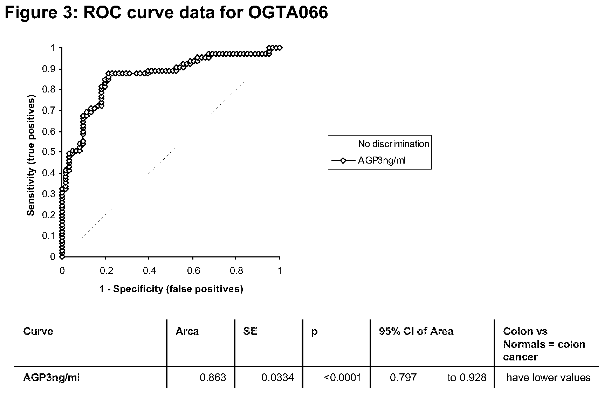

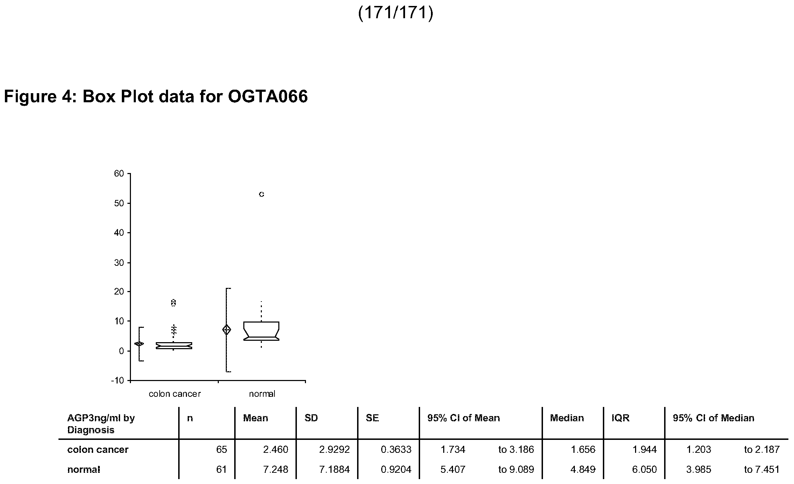

Experiments using 61 normal samples and 65 colorectal cancer samples resulted in further evidence for some of the proteins of interest identified in Example 3 above, including, but not limited to, OGTA066. Figure 4 shows Box plot data for OGTA066. Figure 3 shows ROC curve data for OGTA066.
The ROC curves plot sensitivity (true positives) against 1-specificity (false positives). The area under the ROC curve is a measure of the probability that the measured marker level will allow correct identification of a disease or condition. An area of greater than 0.5 indicates that the marker can discriminate between disease and normal. This is the case in the data shown in Figure 3, with OGTA066 having a high area under the curve and a very low p value therefore indicating that OGTA066 is a good potential marker to discriminate between colorectal cancer and normal.
The vertical axes on the box plot in Figure 4 is concentration of the OGTA066 in ng/ml. Figure 4 shows lower concentration in serum samples of OGTA066 in colorectal cancer samples than in normal samples. OGTA066 shows good discrimination between colorectal cancer and normal, indicating it may be useful as a marker for colorectal cancer.

## Claims

1. An isolated monoclonal antibody, or a fragment thereof which comprises the binding domain, capable of immunospecific binding to CUB domain-containing protein 1, for use in treating breast cancer, wherein the antibody or fragment thereof is conjugated to a cytotoxic moiety or a drug moiety.

2. The isolated monoclonal antibody for use according to claim 1, wherein:
(A) said antibody is selected from the group consisting of
a chimeric antibody
a human antibody
a humanised antibody
a single chain antibody
a bispecific antibody, and
a non-fucosylated antibody;
(B) the fragment is a domain antibody.

## Patentansprüche

1. Isolierter monoklonaler Antikörper oder ein Fragment davon, das die Bindungsdomäne umfasst, der bzw. das immunspezifisch an CUB Domain-Containing Protein 1 binden kann, zur Verwendung in der Behandlung von Brustkrebs, wobei der Antikörper oder das Fragment davon an einen zytotoxischen Teil oder einen Arzneimittelteil konjugiert ist.

2. Isolierter monoklonaler Antikörper zur Verwendung nach Anspruch 1, wobei:
(A) der Antikörper ausgewählt ist aus der Gruppe bestehend aus:
einem chimären Antikörper,
einem humanen Antikörper,
einem humanisierten Antikörper,
einem einkettigen Antikörper,
einem bispezifischen Antikörper und
einem nicht fukosylierten Antikörper;
(B) das Fragment ein Domänen-Antikörper ist.

## Revendications

1. Anticorps monoclonal isolé, ou un fragment de celui-ci, qui comprend le domaine de liaison, capable de se lier immunospécifiquement à la protéine 1 contenant le domaine CUB, destiné à être utilisé dans le traitement du cancer du sein, l'anticorps ou le fragment de celui-ci étant conjugué à un fragment cytotoxique ou à un fragment de médicament.

2. Anticorps monoclonal destiné à être utilisé selon la revendication 1,
(A) ledit anticorps étant choisi dans le groupe constitué par un anticorps chimérique
un anticorps humain
un anticorps humanisé
un anticorps à chaîne unique
un anticorps bispécifique, et
un anticorps non fucosylé ;
(B) le fragment étant un anticorps à domaine.
